# EUROPEAN PATENT APPLICATION

(11) **EP 3 133 163 A1**
(43) Date of publication of application: **22.02.2017**
(21) Application number: 16183781.0
(22) Date of filing: 11.08.2016
(51) Int. Cl.: C12P 7/02, C12P 7/06, C12P 7/16

(54) **DESIGNER PHOTOAUTOTROPHIC AND HYDROGENOTROPHIC PRODUCTION OF ALCOHOLS AND BIODIESEL**

(30) Priority: 21.08.2015 US 201514832476
(71) Applicant: Lee, James Weifu, Chesapeake, VA 23322 (US)
(72) Inventor: Lee, James Weifu, Chesapeake, VA 23322 (US)
(74) Representative: Phillips & Leigh

(57) **Abstract**

Designer Calvin-cycle-channeled and hydrogenotrophic biofuel-production pathways, the associated designer genes and designer transgenic organisms for autotrophic production of alcohols and biodiesel from carbon dioxide, hydrogen, and/or water are disclosed. The alcohols include methanol, ethanol, propanol, 1-butanol, 2-methyl-1-butanol, isobutanol, 3-methyl-1-butanol, 1-hexanol, 1-octanol, 1-pentanol, 1-heptanol, 3-methyl-1-pentanol, 4-methyl-1-hexanol, 5-methyl-1-heptanol, 4-methyl-1-pentanol, 5-methyl-1-hexanol, and 6-methyl-1-heptanol. The designer autotrophic organisms such as designer transgenic oxyphotobacteria and algae comprise designer Calvin-cycle-channeled and hydrogenotrophic pathway gene(s) and biosafety-guarding technology for enhanced autotrophic production of alcohols and biodiesel from carbon dioxide and water; wherein the designer transgenic cells in their mass liquid culture can inducibly self-flocculate for enhanced harvesting of their biomass upon the expression of the designer cell surface-linked positively charged polypeptides.

## Description

### FIELD OF THE INVENTION

The present invention generally relates to biosafety-guarded biofuel energy production technology. More specifically, the present invention provides an autotrophic advanced-biofuels production methodology based on designer transgenic plants, such as transgenic algae, blue-green algae (cyanobacteria and oxychlorobacteria), plant cells or bacterial cells that are created to use the reducing power (NADPH) or Hydrogen (H₂), and energy (ATP) acquired from the photosynthetic and/or hydrogenotrophic process for autotrophic synthesis of alcohols and biodiesel from carbon dioxide (CO₂) and water (H₂O).

### REFERENCE TO SEQUENCE LISTING

The present invention contains references to sequences which have been submitted concurrently herewith as the sequence listing text file
"JWL_004_US3_SeqListingFull_ST25.txt".

### BACKGROUND OF THE INVENTION

Butanol, related higher alcohols and biodiesel can be used as a liquid fuel to run engines such as cars. Butanol can replace gasoline and the energy contents of the two fuels are nearly the same (110,000 Btu per gallon for butanol; 115,000 Btu per gallon for gasoline). Butanol has many superior properties as an alternative fuel when compared to ethanol as well. These include: 1) Butanol has higher energy content (110,000 Btu per gallon butanol) than ethanol (84,000 Btu per gallon ethanol); 2) Butanol is six times less "evaporative" than ethanol and 13.5 times less evaporative than gasoline, making it safer to use as an oxygenate and thereby eliminating the need for very special blends during the summer and winter seasons; 3) Butanol can be transported through the existing fuel infrastructure including the gasoline pipelines whereas ethanol must be shipped via rail, barge or truck; and 4) Butanol can be used as replacement for gasoline gallon for gallon e.g. 100% or any other percentage, whereas ethanol can only be used as an additive to gasoline up to about 85% (E-85) and then only after significant modification to the engine (while butanol can work as a 100% replacement fuel without having to modify the current car engine).

A significant potential market for butanol, related higher alcohols and biodiesel as a liquid fuel already exists in the current transportation and energy systems. Butanol is also used as an industrial solvent. In the United States, currently, butanol is manufactured primarily from petroleum. Historically (1900s-1950s), biobutanol was manufactured from corn and molasses in a fermentation process that also produced acetone and ethanol and was known as an ABE (acetone, butanol, ethanol) fermentation typically with certain butanol-producing bacteria such as *Clostridium acetobutylicum* and *Clostridium beijerinckii.* When the USA lost its low-cost sugar supply from Cuba around 1954, however, butanol production by fermentation declined mainly because the price of petroleum dropped below that of sugar. Recently, there is renewed R&D interest in producing butanol and/or ethanol from biomass such as corn starch using Clostridia- and/or yeast-fermentation process. However, similarly to the situation of "cornstarch ethanol production," the "cornstarch butanol production" process also requires a number of energy-consuming steps including agricultural corn-crop cultivation, corn-grain harvesting, corn-grain starch processing, and starch-to-sugar-to-butanol fermentation. The "cornstarch butanol production" process could also probably cost nearly as much energy as the energy value of its product butanol. This is not surprising, understandably because the cornstarch that the current technology can use represents only a small fraction of the corn crop biomass that includes the corn stalks, leaves and roots. The cornstovers are commonly discarded in the agricultural fields where they slowly decompose back to CO₂, because they represent largely lignocellulosic biomass materials that the current biorefinery industry cannot efficiently use for ethanol or butanol production. There are research efforts in trying to make ethanol or butanol from lignocellulosic plant biomass materials - a concept called "cellulosic ethanol" or "cellulosic butanol". However, plant biomass has evolved effective mechanisms for resisting assault on its cell-wall structural sugars from the microbial and animal kingdoms. This property underlies a natural recalcitrance, creating roadblocks to the cost-effective transformation of lignocellulosic biomass to fermentable sugars. Therefore, one of its problems known as the "lignocellulosic recalcitrance" represents a formidable technical barrier to the cost-effective conversion of plant biomass to fermentable sugars. That is, because of the recalcitrance problem, lignocellulosic biomasses (such as cornstover, switchgrass, and woody plant materials) could not be readily converted to fermentable sugars to make ethanol or butanol without certain pretreatment, which is often associated with high processing cost. Despite more than 50 years of R&D efforts in lignocellulosic biomass pretreatment and fermentative butanol-production processing, the problem of recalcitrant lignocellulosics still remains as a formidable technical barrier that has not yet been eliminated so far. Furthermore, the steps of lignocellulosic biomass cultivation, harvesting, pretreatment processing, and cellulose-to-sugar-to-butanol fermentation all cost energy. Therefore, any new technology that could bypass these bottleneck problems of the biomass technology would be useful.

Oxyphotobacteria (also known as blue-green algae including cyanobacteria and oxychlorobacteria) and algae (such as *Chlamydomonas reinhardtii, Platymonas subcordiformis, Chlorellafusca*, *Dunaliella salina*, *Ankistrodesmus braunii*, and *Scenedesmus obliquus),* which can perform photosynthetic assimilation of CO₂ with O₂ evolution from water in a liquid culture medium with a maximal theoretical solar-to-biomass energy conversion of about 10%, have tremendous potential to be a clean and renewable energy resource. However, the wild-type oxygenic photosynthetic green plants, such as blue-green algae and eukaryotic algae, do not possess the ability to produce butanol directly from CO₂ and H₂O. The wild-type photosynthesis uses the reducing power (NADPH) and energy (ATP) from the photosynthetic water splitting and proton gradient-coupled electron transport process through the algal thylakoid membrane system to reduce CO₂ into carbohydrates (CH₂O)ₙ such as starch with a series of enzymes collectively called the "Calvin cycle" at the stroma region in an algal or green-plant chloroplast. The net result of the wild-type photosynthetic process is the conversion of CO₂ and H₂O into carbohydrates (CH₂O)ₙ and O₂ using sunlight energy according to the following process reaction:

nCO₂ + nH₂O → (CH₂O)ₙ + nO₂ [1]

The carbohydrates (CH₂O)ₙ are then further converted to all kinds of complicated cellular (biomass) materials including proteins, lipids, and cellulose and other cell-wall materials during cell metabolism and growth.

In certain alga such as *Chlamydomonas reinhardtii,* some of the organic reserves such as starch could be slowly metabolized to ethanol (but not to butanol) through a secondary fermentative metabolic pathway. The algal fermentative metabolic pathway is similar to the yeast-fermentation process, by which starch is breakdown to smaller sugars such as glucose that is, in turn, transformed into pyruvate by a glycolysis process. Pyruvate may then be converted to formate, acetate, and ethanol by a number of additional metabolic steps (Gfeller and Gibbs (1984) "Fermentative metabolism of Chlamydomonas reinhardtii," Plant Physiol. 75:212-218). The efficiency of this secondary metabolic process is quite limited, probably because it could use only a small fraction of the limited organic reserve such as starch in an algal cell. Furthermore, the native algal secondary metabolic process could not produce any butanol. As mentioned above, butanol (and/or related higher alcohols) has many superior physical properties to serve as a replacement for gasoline as a fuel. Therefore, a new photobiological and/or hydrogenotrophic butanol (and/or related alcohols and biodiesel)-producing mechanism with a high energy conversion efficiency is needed.

International Application No. PCT/US2009/034801 discloses a set of methods on designer photosynthetic organisms (such as designer transgenic plant, plant cells, algae and oxyphotobacteria) for photobiological production of butanol from carbon dioxide (CO₂) and water (H₂O).

### SUMMARY OF THE INVENTION

The present invention discloses designer photosynthetic and/or hydrogenotrophic pathways, the associated designer genes and designer transgenic organisms for autotrophic production of alcohols and/or biodiesel that are selected from the group that consists of: methanol, ethanol, propanol,1-butanol, 2-methyl-1-butanol, isobutanol, 3-methyl-1-butanol, 1-hexanol, 1-octanol, 1-pentanol, 1-heptanol, 3-methyl-1-pentanol, 4-methyl-1-hexanol, 5-methyl-1-heptanol, 4-methyl-1-pentanol, 5-methyl-1-hexanol, 6-methyl-1-heptanol, biodiesel, and combinations thereof.

The designer autotrophic organisms such as designer transgenic oxyphotobacteria and algae comprise designer photosynthetic and/or hydrogenotrophic pathway gene(s) and biosafety-guarding technology for autotrophic synthesis of methanol, ethanol, butanol and related higher alcohols from carbon dioxide and water, wherein the alcohol is simultaneously and/or subsequently utilized by a lipase in transesterification of triglyceride and fatty acids for production of biodiesel.

According to one of various embodiments, the transgenic autotrophic organism comprises a transgenic cell selected from the group consisting of blue-green algae (oxyphotobacteria including cyanobacteria and oxychlorobacteria), hydrogenotrophic bacteria, fermentative bacteria, methanogens, aquatic plants, plant cells, green algae, red algae, brown algae, diatoms, marine algae, freshwater algae, salt-tolerant algal strains, cold-tolerant algal strains, heat-tolerant algal strains, antenna-pigment-deficient mutants, butanol-tolerant algal strains, higher-alcohols-tolerant algal strains, butanol-tolerant oxyphotobacteria, butanol-tolerant hydrogenotrophic bacteria and methanogens, higher-alcohols-tolerant oxyphotobacteria, alcohol-tolerant hydrogenotrophic bacteria, alcohol-tolerant fermentative bacteria, biodiesel-tolerant algae, biodiesel-tolerant cyanobacteria, biodiesel-tolerant fermentative bacteria, and biodiesel-tolerant hydrogenotrophic bacteria, alcohol-tolerant and biodiesel-tolerant algae, alcohol-tolerant and biodiesel-tolerant cyanobacteria, alcohol-tolerant and biodiesel-tolerant fermentative bacteria, alcohol-tolerant and biodiesel-tolerant hydrogenotrophic bacteria, and alcohol-tolerant and biodiesel-tolerant methanogens, and combinations thereof.

According to another embodiment, a designer transgenic autotrophic organism comprises a set of designer genes that express a designer photo autotrophic methanol-biodiesel production pathway comprising: NADPH-dependent glyceraldehyde-3-phosphate dehydrogenase, NAD⁺-dependent glyceraldehyde-3-phosphate dehydrogenase, formate dehydrogenase, formaldehyde dehydrogenase, alcohol dehydrogenase, and lipase.

According to another embodiment, a designer transgenic autotrophic organism comprises a set of designer genes that express a designer hydrogenotrophic methanol-biodiesel production pathway comprising: NAD-reducing soluble hydrogenase, formate dehydrogenase, formaldehyde dehydrogenase, alcohol dehydrogenase, and lipase.

According to another embodiment, a designer transgenic autotrophic organism comprises a set of designer genes that express a designer photo autotrophic ethanol-biodiesel-production pathway comprising: NADPH-dependent glyceraldehyde-3-phosphate dehydrogenase, NAD⁺-dependent glyceraldehyde-3-phosphate dehydrogenase, phosphoglycerate mutase, enolase, pyruvate kinase, pyruvate decarboxylase, alcohol dehydrogenase, and lipase.

According to another embodiment, a designer transgenic autotrophic organism comprises a set of designer genes that express a designer photo autotrophic butanol-biodiesel-production pathway comprising: NAD⁺-dependent glyceraldehyde-3-phosphate dehydrogenase, phosphoglycerate mutase, enolase, pyruvate kinase, pyruvate-ferredoxin oxidoreductase, acetyl-CoA acetyltransferase, thiolase, 3-hydroxybutyryl-CoA dehydrogenase, crotonase, trans-enoyl-CoA reductase, butyryl-CoA dehydrogenase, butyraldehyde dehydrogenase, aldehyde/alcohol dehydrogenase (AdhE2), butanol dehydrogenase, and lipase.

According to another embodiment, a designer transgenic autotrophic organism comprises a set of designer genes that express a designer photo autotrophic butanol-biodiesel-production pathway comprising: NADPH-dependent glyceraldehyde-3-phosphate dehydrogenase, NAD-dependent glyceraldehyde-3-phosphate dehydrogenase, phosphoglycerate mutase, enolase, phosphoenolpyruvate carboxylase, aspartate aminotransferase, aspartokinase, aspartate-semialdehyde dehydrogenase, homoserine dehydrogenase, homoserine kinase, threonine synthase, threonine ammonia-lyase, 2-isopropylmalate synthase, isopropylmalate isomerase, 3-isopropylmalate dehydrogenase, 2-keto acid decarboxylase, NAD-dependent alcohol dehydrogenase, NADPH-dependent alcohol dehydrogenase, butanol dehydrogenase, and lipase.

According to another embodiment, a designer transgenic autotrophic organism comprises a set of designer genes that express a designer photo autotrophic isobutanol-biodiesel-production pathway comprising: NADPH-dependent glyceraldehyde-3-phosphate dehydrogenase, NAD-dependent glyceraldehyde-3-phosphate dehydrogenase, phosphoglycerate mutase, enolase, pyruvate kinase, acetolactate synthase, ketol-acid reductoisomerase, dihydroxy-acid dehydratase, 2-keto acid decarboxylase, NAD-dependent alcohol dehydrogenase, NADPH-dependent alcohol dehydrogenase, and lipase.

According to another embodiment, a designer transgenic autotrophic organism comprises a set of designer genes that express a designer photo autotrophic 3-methyl-1-butanol - biodiesel-production pathway comprising: NADPH-dependent glyceraldehyde-3-phosphate dehydrogenase, NAD-dependent glyceraldehyde-3-phosphate dehydrogenase, phosphoglycerate mutase, enolase, pyruvate kinase, acetolactate synthase, ketol-acid reductoisomerase, dihydroxy-acid dehydratase, 2-isopropylmalate synthase, 3-isopropylmalate dehydratase, 3-isopropylmalate dehydrogenase, 2-keto acid decarboxylase, NAD-dependent alcohol dehydrogenase, NADPH-dependent alcohol dehydrogenase, 3-methylbutanal reductase, and lipase.

According to another embodiment, a designer transgenic autotrophic organism comprises a set of designer genes that express a designer anaerobic hydrogenotrophic 1-butanol-biodiesel-production pathway comprising: energy converting hydrogenase, [NiFe]-hydrogenase, Coenzyme F₄₂₀-reducing hydrogenase, soluble hydrogenase, heterodissulfide reductase, formylmethanofuran dehydrogenase, formyl transferase, 10-methenyl-tetrahydromethanopterin cyclohydrolase, 10-methylene-H₄ methanopterin dehydrogenase, 10-methylene-H₄-methanopterin reductase, methyl-H₄-methanopterin: corrinoid iron-sulfur protein methyltransferase, corrinoid iron-sulfur protein, CO dehydrogenase/acetyl-CoA synthase, thiolase, 3-hydroxybutyryl-CoA dehydrogenase, crotonase, butyryl-CoA dehydrogenase, butyaldehyde dehydrogenase, butanol dehydrogenase, alcohol dehydrogenase, and lipase.

According to another embodiment, a designer transgenic autotrophic organism is made free of any antibiotic resistance genes for better biosafety by using nutrient-complementation selection with special authoxtrophs that are generated by deletion of an essential nutrient-gene selected from the group consisting of argininosuccinate lyase (*arg7*), nitrate reductase, ketol-acid reductoisomerase and dihydroxy-acid dehydratase.

According to another embodiment, a designer positively-charged polypeptides expressed on transgenic microbial cell surfaces are selected from the group consisting of polypeptides rich in lysine residuals, polypeptides rich in arginine residues, polypeptides rich in histidine residues, polypeptides rich in lysine and arginine residues, polypeptides rich in lysine and histidine residues, polypeptides rich in lysine and arginine and histidine residues, lipase-fused polylysine, polyamine-lipase-fused polylysine, lipase-fused positively-charged polypeptides, fluorescent protein-lipase-fused polylysine, and fluorescent protein-lipase-fused positively-charged polypeptides. Wherein the designer transgenic cells in their mass liquid culture inducibly self-flocculate for enhanced harvesting of their biomass upon the expression of the designer cell surface-linked positively charged polypeptides.

### BRIEF DESCRIPTION OF THE DRAWINGS

Fig. 1 presents designer butanol-production pathways branched from the Calvin cycle using the reducing power (NADPH) and energy (ATP) from the photosynthetic water splitting and proton gradient-coupled electron transport process to reduce carbon dioxide (CO₂) into butanol CH₃CH₂CH₂CH₂OH with a series of enzymatic reactions.
Fig. 2A presents a DNA construct for designer butanol-production-pathway gene(s).
Fig. 2B presents a DNA construct for NADPH/NADH-conversion designer gene for NADPH/NADH inter-conversion.
Fig. 2C presents a DNA construct for a designer iRNA starch/glycogen-synthesis inhibitor(s) gene.
Fig. 2D presents a DNA construct for a designer starch-degradation-glycolysis gene(s).
Fig. 2E presents a DNA construct of a designer butanol-production-pathway gene(s) for cytosolic expression.
Fig. 2F presents a DNA construct of a designer butanol-production-pathway gene(s) with two recombination sites for integrative genetic transformation in oxyphotobacteria.
Fig. 2G presents a DNA construct of a designer biosafety-control gene(s).
Fig. 2H presents a DNA construct of a designer proton-channel gene(s).
Fig. 3A illustrates a cell-division-controllable designer organism that contains two key functions: designer biosafety mechanism(s) and designer biofuel-production pathway(s).
Fig. 3B illustrates a cell-division-controllable designer organism for photobiological production of butanol (CH₃CH₂CH₂CH₂OH) from carbon dioxide (CO₂) and water (H₂O) with designer biosafety mechanism(s).
Fig. 3C illustrates a cell-division-controllable designer organism for biosafety-guarded photobiological production of other biofuels such as ethanol (CH₃CH₂OH) from carbon dioxide (CO₂) and water (H₂O).
Fig. 4 presents designer Calvin-cycle-channeled and photosynthetic NADPH-enhanced pathways using the reducing power (NADPH) and energy (ATP) from the photosynthetic water splitting and proton gradient-coupled electron transport process to reduce carbon dioxide (CO₂) into 1-butanol (CH₃CH₂CH₂CH₂OH) with a series of enzymatic reactions.
Fig. 5 presents designer Calvin-cycle-channeled and photosynthetic NADPH-enhanced pathways using NADPH and ATP from the photosynthetic water splitting and proton gradient-coupled electron transport process to reduce carbon dioxide (CO₂) into 2-methyl-1-butanol (CH₃CH₂CH(CH₃)CH₂OH) with a series of enzymatic reactions.
Fig. 6 presents designer Calvin-cycle-channeled and photosynthetic NADPH-enhanced pathways using NADPH and ATP from the photosynthetic water splitting and proton gradient-coupled electron transport process to reduce carbon dioxide (CO₂) into isobutanol ((CH₃)₂CHCH₂OH) and 3-methyl-1-butanol (CH₃CH(CH₃)CH₂CH₂OH) with a series of enzymatic reactions.
Fig. 7 presents designer Calvin-cycle-channeled and photosynthetic NADPH-enhanced pathways using NADPH and ATP from the photosynthetic water splitting and proton gradient-coupled electron transport process to reduce carbon dioxide (CO₂) into 1-hexanol (CH₃CH₂CH₂CH₂CH₂CH₂OH) and 1-octanol (CH₃CH₂CH₂CH₂CH₂CH₂CH₂CH₂OH) with a series of enzymatic reactions.
Fig. 8 presents designer Calvin-cycle-channeled and photosynthetic NADPH-enhanced pathways using NADPH and ATP from the photosynthetic water splitting and proton gradient-coupled electron transport process to reduce carbon dioxide (CO₂) into 1-pentanol (CH₃CH₂CH₂CH₂CH₂OH), 1-hexanol (CH₃CH₂CH₂CH₂CH₂CH₂OH), and 1-heptanol (CH₃CH₂CH₂CH₂CH₂CH₂CH₂OH) with a series of enzymatic reactions.
Fig. 9 presents designer Calvin-cycle-channeled and photosynthetic NADPH-enhanced pathways using NADPH and ATP from the photosynthetic water splitting and proton gradient-coupled electron transport process to reduce carbon dioxide (CO₂) into 3-methyl-1-pentanol (CH₃CH₂CH(CH₃)CH₂CH₂OH), 4-methyl-1-hexanol (CH₃CH₂CH(CH₃)CH₂CH₂CH₂OH), and 5-methyl-1-heptanol (CH₃CH₂CH(CH₃)CH₂CH₂CH₂CH₂OH) with a series of enzymatic reactions.
Fig. 10 presents designer Calvin-cycle-channeled and photosynthetic NADPH-enhanced pathways using NADPH and ATP from the photosynthetic water splitting and proton gradient-coupled electron transport process to reduce carbon dioxide (CO₂) into 4-methyl-1-pentanol (CH₃CH(CH₃)CH₂CH₂CH₂OH), 5-methyl-1-hexanol (CH₃CH(CH₃)CH₂CH₂CH₂CH₂OH), and 6-methyl-1-heptanol (CH₃CH(CH₃)CH₂CH₂CH₂CH₂CH₂OH) with a series of enzymatic reactions.
Fig. 11 illustrates a designer organism with designer oxygen-tolerant hydrogenases and Calvin-cycle-channeled biofuel-production pathway(s) for aerobic chemolithoautotrophic production of biofuels such as butanol (CH₃CH₂CH₂CH₂OH) from hydrogen (H₂), carbon dioxide (CO₂), and oxygen (O₂).
Fig. 12 illustrates a designer organism that comprises a designer anaerobic hydrogenotrophic system with reductive-acetyl-CoA biofuel-production pathway(s) for anaerobic chemolithotrophic production of 1-butanol (CH₃CH₂CH₂CH₂OH) from hydrogen (H₂) and carbon dioxide (CO₂).
Fig. 13 presents a designer reductive-acetyl-CoA biofuel-production pathway for anaerobic hydrogenotrophic production of 1-butanol (CH₃CH₂ CH₂CH₂OH) from carbon dioxide (CO₂) with a series of enzymatic reactions.
Fig. 14 presents a designer ATP-required reductive-acetyl-CoA biofuel-production pathway for anaerobic hydrogenotrophic production of 1-butanol (CH₃ CH₂ CH₂CH₂OH) from carbon dioxide (CO₂) with a series of enzymatic reactions.
Fig. 15 illustrates a designer organism that comprises a designer methanogenic hydrogenotrophic system with reductive-acetyl-CoA biofuel-production pathway(s) for anaerobic chemolithotrophic production of both 1-butanol (CH₃CH₂CH₂CH₂OH) and methane (CH₄) from hydrogen (H₂) and carbon dioxide (CO₂).
Fig. 16 presents designer reductive-acetyl-CoA biofuel-production pathways for anaerobic hydrogenotrophic production of both 1-butanol (CH₃CH₂CH₂CH₂OH) and methane (CH₄) from carbon dioxide (CO₂) with a series of enzymatic reactions.
Fig. 17 presents designer ATP-required reductive-acetyl-CoA biofuel-production pathways for anaerobic hydrogenotrophic production of both 1-butanol (CH₃CH₂CH₂CH₂OH) and methane (CH₄) from carbon dioxide (CO₂) and with a series of enzymatic reactions.
Fig. 18 presents a designer autotrophic methanol-biodiesel production pathway with an NADPH/NADH conversion process.
Fig. 19 presents a designer hydrogenotrophic cell for autotrophic synthesis of alcohol and biodiesel from carbon dioxide (CO₂) and molecular hydrogen (H₂).
Fig. 20 presents a designer autotrophic cell such as a transgenic algal cell with lipase and positively-charged polypeptide such as polylysine expressed on cell surface for autotrophic (such as photosynthetic) production of alcohol and biodiesel from carbon dioxide (CO₂) and water (H₂O) and for enabling self-flocculation to enhance harvesting of cells from liquid culture.

### DETAILED DESCRIPTION OF THE INVENTION

The present invention is directed to an autotrophic methanol, ethanol, butanol and related high alcohols, and biodiesel production technology based on designer autotrophic organisms such as designer transgenic plants (e.g., algae and oxyphotobacteria), plant cells, or bacteria. In this context throughout this specification, a "higher alcohol" or "related higher alcohol" refers to an alcohol that comprises at least four carbon atoms, which includes both straight and branched alcohols such as 1-butanol and 2-methyl-1-butanol. Conversely, a "related alcohol" refers to an alcohol that comprises at least one carbon atom. The Calvin-cycle-channeled and photosynthetic-NADPH-enhanced pathways are constructed with designer enzymes expressed through use of designer genes in host photosynthetic organisms such as algae and oxyphotobacteria (including cyanobacteria and oxychlorobacteria) organisms for photobiological production of butanol and related higher alcohols. The said butanol and related alcohols are selected from the group consisting of: methanol, ethanol, 1-butanol, 2-methyl-1-butanol, isobutanol, 3-methyl-1-butanol, 1-hexanol, 1-octanol, 1-pentanol, 1-heptanol, 3-methyl-1-pentanol, 4-methyl-1-hexanol, 5-methyl-1-heptanol, 4-methyl-1-pentanol, 5-methyl-1-hexanol, and 6-methyl-1-heptanol. The designer plants and plant cells are created using genetic engineering techniques such that the endogenous photosynthesis regulation mechanism is tamed, and the reducing power (NADPH) and energy (ATP) acquired from the photosynthetic water splitting and proton gradient-coupled electron transport process can be used for immediate synthesis of alcohols, such as 1-butanol (CH₃CH₂CH₂CH₂OH) and 2-methyl-1-butanol (CH₃CH₂CH(CH₃)CH₂OH), from carbon dioxide (CO₂) and water (H₂O) according to the following generalized process reaction (where *m, n*, *x* and *y* are its molar coefficients) in accordance of the present invention:

*m*(CO₂) + *n*(H₂O) → *x*(alcohols) +*y*(O₂) [2]

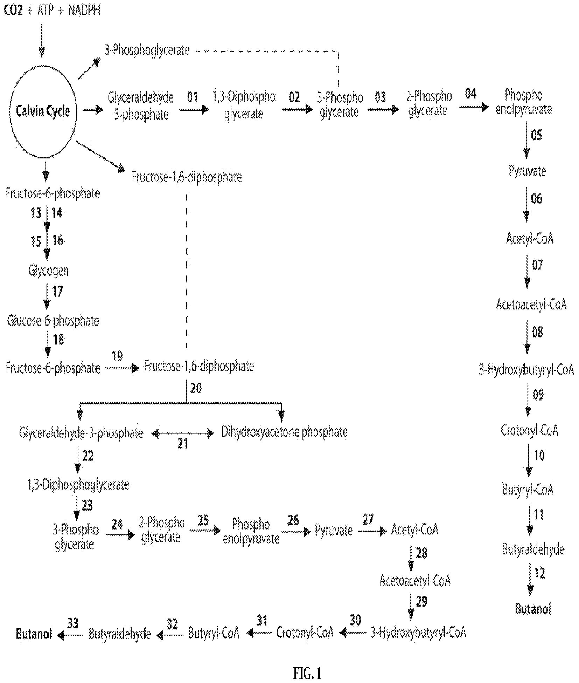

The photobiological alcohols-production methods of the present invention completely eliminate the problem of recalcitrant lignocellulosics by bypassing the bottleneck problem of the biomass technology. As shown in Fig. 1, for example, the photosynthetic process in a designer organism effectively uses the reducing power (NADPH) and energy (ATP) from the photosynthetic water splitting and proton gradient-coupled electron transport process for immediate synthesis of butanol (CH₃CH₂CH₂CH₂OH) directly from carbon dioxide (CO₂) and water (H₂O) without being drained into the other pathway for synthesis of the undesirable lignocellulosic materials that are very hard and often inefficient for the biorefinery industry to use. This approach is also different from the existing "cornstarch butanol production" process. In accordance with this invention, butanol can be produced directly from carbon dioxide (CO₂) and water (H₂O) without having to go through many of the energy consuming steps that the cornstarch butanol-production process has to go through, including corn crop cultivation, corn-grain harvesting, corn-grain cornstarch processing, and starch-to-sugar-to-butanol fermentation. As a result, the photosynthetic butanol-production technology of the present invention is expected to have a much (more than 10-times) higher solar-to-butanol energy-conversion efficiency than the current technology. Assuming a 10% solar energy conversion efficiency for the proposed photosynthetic butanol production process, the maximal theoretical productivity (yield) could be about 72,700 kg of butanol per acre per year, which could support about 70 cars (per year per acre). Therefore, this invention could bring a significant capability to the society in helping to ensure energy security. The present invention could also help protect the Earth's environment from the dangerous accumulation of CO₂ in the atmosphere, because the present methods convert CO₂ directly into clean butanol energy.

A fundamental feature of the present methodology is utilizing a plant (e.g., an alga or oxyphotobacterium) or plant cells, introducing into the plant or plant cells nucleic acid molecules encoding for a set of enzymes that can act on an intermediate product of the Calvin cycle and convert the intermediate product into butanol as illustrated in Figure 1, instead of making starch and other complicated cellular (biomass) materials as the end products by the wild-type photosynthetic pathway. Accordingly, the present invention provides, *inter alia*, methods for producing butanol and/or related alcohols based on a designer plant (such as a designer alga and a designer oxyphotobacterium), designer plant tissue, or designer plant cells, DNA constructs encoding genes of a designer butanol- and/or related higher alcohols-production pathway(s), as well as the designer algae, designer oxyphotobacteria (including designer cyanobacteria), designer fermentative bacteria, designer hydrogenotrophic bacteria, designer plants, designer plant tissues, and designer plant cells created. The various aspects of the present invention are described in further detail hereinbelow.

### Host Photosynthetic Organism

According to the present invention, a designer organism or cell for the photosynthetic butanol and/or related higher alcohols production of the invention can be created utilizing as host, any plant (including alga and oxyphotobacterium), plant tissue, or plant cells that have a photosynthetic capability, i.e., an active photosynthetic apparatus and enzymatic pathway that captures light energy through photosynthesis, using this energy to convert inorganic substances into organic matter. Preferably, the host organism should have an adequate photosynthetic CO₂ fixation rate, for example, to support photosynthetic butanol (and/or related higher alcohols) production from CO₂ and H₂O at least about 1,450 kg butanol per acre per year, more preferably, 7,270 kg butanol per acre per year, or even more preferably, 72,700 kg butanol per acre per year.

In a preferred embodiment, an aquatic plant is utilized to create a designer plant. Aquatic plants, also called hydrophytic plants, are plants that live in or on aquatic environments, such as in water (including on or under the water surface) or permanently saturated soil. As used herein, aquatic plants include, for example, algae, blue-green algae (cyanobacteria and oxychlorobacteria), submersed aquatic herbs (*Hydrilla verticillata, Elodea densa, Hippuris vulgaris, Aponogeton Boivinianus, Aponogeton Rigidifolius, Aponogeton Longiplumulosus, Didiplis Diandra, Vesicularia Dubyana, Hygrophilia Augustifolia, Micranthemum Umbrosum, Eichhornia Azurea, Saururus Cernuus, Cryptocoryne Lingua, Hydrotriche Hottoniiflora, Eustralis Stellata, Vallisneria Rubra, Hygrophila Salicifolia, Cyperus Helferi, Cryptocoryne Petchii, Vallisneria americana, Vallisneria Torta, Hydrotriche Hottoniiflora, Crassula Helmsii, Limnophila Sessiliflora, Potamogeton Perfoliatus, Rotala Wallichii, Cryptocoryne Becketii, Blyxa Aubertii, Hygrophila Difformmis),* duckweeds (*Spirodela polyrrhiza*, *Wolffia globosa*, *Lemna trisulca*, *Lemna gibba*, *Lemna minor*, *Landoltia punctata),* water cabbage (*Pistia stratiotes*), buttercups *(Ranunculus),* water caltrop (*Trapa natans* and *Trap bicornis*), water lily (*Nymphaea lotus,* Nymphaeaceae and Nelumbonaceae), water hyacinth (*Eichhornia crassipes*), *Bolbitis heudelotii*, *Cabomba* sp., seagrasses (*Heteranthera Zosterifolia,* Posidoniaceae, Zosteraceae, Hydrocharitaceae, and Cymodoceaceae). Butanol (and/or related higher alcohols) produced from an aquatic plant can diffuse into water, permitting normal growth of the plants and more robust production of butanol from the plants. Liquid cultures of aquatic plant tissues (including, but not limited to, multicellular algae) or cells (including, but not limited to, unicellular algae) are also highly preferred for use, since the butanol (and/or related higher alcohols) molecules produced from a designer butanol (and/or related higher alcohols) production pathway(s) can readily diffuse out of the cells or tissues into the liquid water medium, which can serve as a large pool to store the product butanol (and/or related higher alcohols) that can be subsequently harvested by filtration and/or distillation/evaporation techniques.

Although aquatic plants or cells are preferred host organisms for use in the methods of the present invention, tissue and cells of non-aquatic plants, which are photosynthetic and can be cultured in a liquid culture medium, can also be used to create designer tissue or cells for photosynthetic butanol (and/or related higher alcohols) production. For example, the following tissue or cells of non-aquatic plants can also be selected for use as a host organism in this invention: the photoautotrophic shoot tissue culture of wood apple tree *Feronia limonia*, the chlorophyllous callus-cultures of corn plant *Zea mays,* the green root cultures of Asteraceae and Solanaceae species, the tissue culture of sugarcane stalk parenchyma, the tissue culture of bryophyte *Physcomitrella patens*, the photosynthetic cell suspension cultures of soybean plant *(Glycine max),* the photo autotrophic and photomixotrophic culture of green Tobacco (*Nicofiana tabacum L.)* cells, the cell suspension culture of *Gisekia pharnaceoides* (a C₄ plant), the photosynthetic suspension cultured lines of Amaranthus powellii Wats., Datura innoxia Mill., Gossypium hirsutum L., and Nicotiana tabacum x Nicotiana glutinosa L. fusion hybrid.

By "liquid medium" is meant liquid water plus relatively small amounts of inorganic nutrients (e.g., N, P, K etc, commonly in their salt forms) for photoautotrophic cultures; and sometimes also including certain organic substrates (e.g., sucrose, glucose, or acetate) for photomixotrophic and/or photoheterotrophic cultures.

In an especially preferred embodiment, the plant utilized in the butanol (and/or related higher alcohols) production method of the present invention is an alga or a blue-green alga. The use of algae and/or blue-green algae has several advantages. They can be grown in an open pond at large amounts and low costs. Harvest and purification of butanol (and/or related higher alcohols) from the water phase is also easily accomplished by distillation/evaporation or membrane separation.

Algae suitable for use in the present invention include both unicellular algae and multi-unicellular algae. Multicellular algae that can be selected for use in this invention include, but are not limited to, seaweeds such as *Ulva latissima* (sea lettuce), *Ascophyllum nodosum, Codium fragile, Fucus vesiculosus, Eucheuma denticulatum, Gracilaria gracilis, Hydrodictyon reticulatum, Laminaria japonica, Undaria pinntifida, Saccharina japonica, Porphyra yezoensis*, and *Porphyry tenera.* Suitable algae can also be chosen from the following divisions of algae: green algae (Chlorophyta), red algae (Rhodophyta), brown algae (Phaeophyta), diatoms (Bacillariophyta), and blue-green algae (Oxyphotobacteria including Cyanophyta and Prochlorophytes). Suitable orders of green algae include Ulvales, Ulotrichales, Volvocales, Chlorellales, Schizogoniales, Oedogoniales, Zygnematales, Cladophorales, Siphonales, and Dasycladales. Suitable genera of Rhodophyta are Porphyra, Chondrus, Cyanidioschyzon, Porphyridium, Gracilaria, Kappaphycus, Gelidium and Agardhiella. Suitable genera of Phaeophyta are Laminaria, Undaria, Macrocystis, Sargassum and Dictyosiphon. Suitable genera of Cyanophyta (also known as Cyanobacteria) include (but not limited to) Phoridium, Synechocystis, Syncechococcus, Oscillatoria, and Anabaena. Suitable genera of Prochlorophytes (also known as oxychlorobacteria) include (but not limited to) Prochloron, Prochlorothrix, and Prochlorococcus. Suitable genera of Bacillariophyta are Cyclotella, Cylindrotheca, Navicula, Thalassiosira, and Phaeodactylum. Preferred species of algae for use in the present invention include *Chlamydomonas reinhardtii, Platymonas subcordiformis, Chlorella fusca*, *Chlorella sorokiniana, Chlorella vulgaris, 'Chlorella' ellipsoidea, Chlorella spp., Dunaliella salina, Dunaliella viridis, Dunaliella bardowil, Haematococcus pluvialis; Parachlorella kessleri, Betaphycus gelatinum, Chondrus crispus, Cyanidioschyzon merolae, Cyanidium caldarium, Galdieria sulphuraria, Gelidiella acerosa, Gracilaria changii, Kappaphycus alvarezii, Porphyra miniata, Ostreococcus tauri, Porphyra yezoensis, Porphyridium sp., Palmaria palmata, Gracilaria spp., Isochrysis galbana, Kappaphycus spp., Laminariajaponica, Laminaria spp., Monostroma spp., Nannochloris bacillaris,* Nannochloris sp., *Nannochloropsis oculata, Porphyra spp., Porphyridium spp., Undariapinnatifida, Ulva lactuca, Ulva spp., Undaria spp., Phaeodactylum Tricornutum, Navicula saprophila, Crypthecodinium cohnii, Cylindrothecafusiformis, Cyclotella cryptica, Euglena gracilis, Amphidinium sp., Symbiodinium microadriaticum, Macrocystispyrifera, Ankistrodesmus braunii*, *Ankistrodesmus convolutus, Ankistrodesmus falcatus, Ankistrodesmus stipitatus, Pavlova salina, Pavlova lutheri, Botryococcus braunii*, *Scenedesmus vacuolatus, Scenedesmus acutus, Scenedesmus rotundus, Scenedesmus dimorphus, Scenedesmus sp. Ki4, Scenedesmus sp. LU4, Scenedesmus quadricaudus,* and *Scenedesmus obliquus.*

Preferred species of blue-green algae (oxyphotobacteria including cyanobacteria and oxychlorobacteria) for use in the present invention include *Thermosynechococcus elongatus* BP-1, *Nostoc sp.* PCC 7120, *Synechococcus elongatus* PCC 6301, *Syncechococcus* sp. strain PCC 7942, *Syncechococcus* sp. strain PCC 7002, *Syncechocystis* sp. strain PCC 6803, *Prochlorococcus marinus* MED4, *Prochlorococcus marinus* MIT 9313, *Prochlorococcus marinus* NATL1A, *Prochlorococcus* SS120, *Spirulina platensis (Arthrospira platensis), Spirulinapacifica, Lyngbya majuscule, Anabaena sp., Synechocystis sp., Synechococcus elongates, Synechococcus (MC-A), Trichodesmium sp., Richelia intracellularis, Synechococcus* WH7803, *Synechococcus* WH8102, *Nostoc punctiforme, Syncechococcus* sp. strain PCC 7943, Synechocyitis PCC 6714 phycocyanin-deficient mutant PD-1, *Cyanothece* strain 51142, *Cyanothece sp.* CCY0110, *Oscillatoria limosa, Lyngbya majuscula, Symploca muscorum, Gloeobacter violaceus, Prochloron didemni, Prochlorothrix hollandica, Synechococcus (MC-A)*, *Trichodesmium sp., Richelia intracellularis*, *Prochlorococcus marinus, Prochlorococcus* SS120, *Synechococcus* WH8102, *Lyngbya majuscula, Symploca muscorum, Synechococcus bigranulatus,* cryophilic *Oscillatoria sp., Phormidium sp., Nostoc sp.-1, Calothrixparietina,* thermophilic *Synechococcus bigranulatus, Synechococcus lividus,* thermophilic *Mastigocladus laminosus, Chlorogloeopsis fritschii* PCC 6912, *Synechococcus vulcanus, Synechococcus sp.* strain MA4, *Synechococcus sp.* strain MA19, and *Thermosynechococcus elongatus.*

Proper selection of host photosynthetic organisms for their genetic backgrounds and certain special features is also beneficial. For example, a photosynthetic-butanol-producing designer alga created from cryophilic algae (psychrophiles) that can grow in snow and ice, and/or from cold-tolerant host strains such as *Chlamydomonas* cold strain CCMG1619, which has been characterized as capable of performing photosynthetic water splitting as cold as 4°C (Lee, Blankinship and Greenbaum (1995), "Temperature effect on production of hydrogen and oxygen by Chlamydomonas cold strain CCMP1619 and wild type 137c," Applied Biochemistry and Biotechnology 51/52:379-386), permits photobiological butanol production even in cold seasons or regions such as Canada. Meanwhile, a designer alga created from a thermophilic/thermotolerant photosynthetic organism such as thermophilic algae *Cyanidium caldarium* and *Galdieria sulphuraria* and/or thermophilic cyanobacteria (blue-green algae) such as *Thermosynechococcus elongatus* BP-1 and *Synechococcus bigranulatus* may permit the practice of this invention to be well extended into the hot seasons or areas such as Mexico and the Southwestern region of the United States including Nevada, California, Arizona, New Mexico and Texas, where the weather can often be hot. Furthermore, a photosynthetic-butanol-producing designer alga created from a marine alga, such as *Platymonas subcordiformis,* permits the practice of this invention using seawater, while the designer alga created from a freshwater alga such as *Chlamydomonas reinhardtii* can use freshwater. Additional optional features of a photosynthetic butanol (and/or related higher alcohols) producing designer alga include the benefits of reduced chlorophyll-antenna size, which has been demonstrated to provide higher photosynthetic productivity (Lee, Mets, and Greenbaum (2002). "Improvement of photosynthetic efficiency at high light intensity through reduction of chlorophyll antenna size," Applied Biochemistry and Biotechnology, 98-100: 37-48) and butanol-tolerance (and/or related higher alcohols- tolerance) that allows for more robust and efficient photosynthetic production of butanol (and/or related higher alcohols) from CO₂ and H₂O. By use of a phycocyanin-deficient mutant of *Synechocystis* PCC 6714, it has been experimentally demonstrated that photoinhibition can be reduced also by reducing the content of light-harvesting pigments (Nakajima, Tsuzuki, and Ueda (1999) "Reduced photoinhibition of a phycocyanin-deficient mutant of Synechocystis PCC 6714", Journal of Applied Phycology 10: 447-452). These optional features can be incorporated into a designer alga, for example, by use of a butanol-tolerant and/or chlorophyll antenna-deficient mutant (e.g., *Chlamydomonas reinhardtii* strain DS521) as a host organism, for gene transformation with the designer butanol-production-pathway genes. Therefore, in one of the various embodiments, a host organism is selected from the group consisting of aquatic plants, plant cells, green algae, red algae, brown algae, blue-green algae (oxyphotobacteria including cyanobacteria and oxychlorobacteria), hydrogenotrophic bacteria, fermentative bacteria, methanogens, diatoms, marine algae, freshwater algae, unicellular algae, multicellular algae, seaweeds, salt-tolerant algal strains, cold-tolerant algal strains, heat-tolerant algal strains, light-harvesting-antenna-pigment-deficient mutants, butanol-tolerant algal strains, higher-alcohols-tolerant algal strains, butanol-tolerant oxyphotobacteria, butanol-tolerant hydrogenotrophic bacteria and methanogens, higher-alcohols-tolerant oxyphotobacteria, alcohol-tolerant hydrogenotrophic bacteria, alcohol-tolerant fermentative bacteria, biodiesel-tolerant algae, biodiesel-tolerant cyanobacteria, biodiesel-tolerant fermentative bacteria, and biodiesel-tolerant hydrogenotrophic bacteria, alcohol-tolerant and biodiesel-tolerant algae, alcohol-tolerant and biodiesel-tolerant cyanobacteria, alcohol-tolerant and biodiesel-tolerant fermentative bacteria, alcohol-tolerant and biodiesel-tolerant hydrogenotrophic bacteria, and alcohol-tolerant and biodiesel-tolerant methanogens, and combinations thereof.

### Creating a Designer Butanol-Production Pathway in a Host

### Selecting appropriate designer enzymes

One of the key features in the present invention is the creation of a designer butanol-production pathway to tame and work with the natural photosynthetic mechanisms to achieve the desirable synthesis of butanol directly from CO₂ and H₂O. The natural photosynthetic mechanisms include (1) the process of photosynthetic water splitting and proton gradient-coupled electron transport through the thylakoid membrane, which produces the reducing power (NADPH) and energy (ATP), and (2) the Calvin cycle, which reduces CO₂ by consumption of the reducing power (NADPH) and energy (ATP).

In accordance with the present invention, a series of enzymes are used to create a designer butanol-production pathway that takes an intermediate product of the Calvin cycle and converts the intermediate product into butanol as illustrated in Figure 1. A "designer butanol-production-pathway enzyme" is hereby defined as an enzyme that serves as a catalyst for at least one of the steps in a designer butanol-production pathway. According to the present invention, a number of intermediate products of the Calvin cycle can be utilized to create designer butanol-production pathway(s); and the enzymes required for a designer butanol-production pathway are selected depending upon from which intermediate product of the Calvin cycle the designer butanol-production pathway branches off from the Calvin cycle.

In one example, a designer pathway is created that takes glyceraldehydes-3-phosphate and converts it into butanol by using, for example, a set of enzymes consisting of, as shown with the numerical labels **01-12** in Figure 1, glyceraldehyde-3-phosphate dehydrogenase **01,** phosphoglycerate kinase **02,** phosphoglycerate mutase **03,** enolase **04,** pyruvate kinase **05,** pyruvate-ferredoxin oxidoreductase **06,** thiolase (or acetyl-CoA acetyltransferase) **07,** 3-hydroxybutyryl-CoA dehydrogenase **08,** crotonase **09,** butyryl-CoA dehydrogenase (or trans-enoyl-CoA reductase) **10,** butyraldehyde dehydrogenase (or aldehyde/alcohol dehydrogenase (AdhE2)) **11,** and butanol dehydrogenase **12.** In this glyceraldehydes-3-phosphate-branched designer pathway, for conversion of two molecules of glyceraldehyde-3-phosphate to butanol, two NADH molecules are generated from NAD⁺ at the step from glyceraldehyde-3-phosphate to 1,3-diphosphoglycerate catalyzed by glyceraldehyde-3-phosphate dehydrogenase **01;** meanwhile two molecules of NADH are converted to NAD⁺: one at the step catalyzed by 3-hydroxybutyryl-CoA dehydrogenase **08** in reducing acetoacetyl-CoA to 3-hydroxybutyryl-CoA and another at the step catalyzed by butyryl-CoA dehydrogenase **10** in reducing crotonyl-CoA to butyryl-CoA. Consequently, in this glyceraldehydes-3-phosphate-branched designer pathway **(01-12),** the number of NADH molecules consumed is balanced with the number of NADH molecules generated. Furthermore, both the pathway step catalyzed by butyraldehyde dehydrogenase **11** (in reducing butyryl-CoA to butyraldehyde) and the terminal step catalyzed by butanol dehydrogenase **12** (in reducing butyraldehyde to butanol) can use NADPH, which can be regenerated by the photosynthetic water splitting and proton gradient-coupled electron transport process. Therefore, this glyceraldehydes-3-phosphate-branched designer butanol-production pathway can operate continuously.

In another example, a designer pathway is created that takes the intermediate product, 3-phosphoglycerate, and converts it into butanol by using, for example, a set of enzymes consisting of (as shown with the numerical labels **03-12** in Figure 1) phosphoglycerate mutase **03,** enolase **04,** pyruvate kinase **05,** pyruvate-ferredoxin oxidoreductase **06,** thiolase (or acetyl-CoA acetyltransferase) **07,** 3-hydroxybutyryl-CoA dehydrogenase **08,** crotonase **09,** butyryl-CoA dehydrogenase (or trans-enoyl-CoA reductase) **10,** butyraldehyde dehydrogenase (or aldehyde/alcohol dehydrogenase (AdhE2)) **11,** and butanol dehydrogenase **12.** It is worthwhile to note that the last ten enzymes **(03-12)** of the glyceraldehydes-3-phosphate-branched designer butanol-producing pathway **(01-12)** are identical with those utilized in the 3-phosphoglycerate-branched designer pathway **(03-12).** In other words, the designer enzymes **(01-12)** of the glyceraldehydes-3-phosphate-branched pathway permit butanol production from both the point of 3-phosphoglycerate and the point of glyceraldehydes-3-phosphate in the Calvin cycle. These two pathways, however, have different characteristics. Unlike the glyceraldehyde-3-phosphate-branched butanol-production pathway, the 3-phosphoglycerate-branched pathway which consists of the activities of only ten enzymes **(03-12)** could not itself generate any NADH that is required for use at two places: one at the step catalyzed by 3-hydroxybutyryl-CoA dehydrogenase **08** in reducing acetoacetyl-CoA to 3-hydroxybutyryl-CoA, and another at the step catalyzed by butyryl-CoA dehydrogenase **10** in reducing crotonyl-CoA to butyryl-CoA. That is, if (or when) a 3-hydroxybutyryl-CoA dehydrogenase and/or a butyryl-CoA dehydrogenase that can use strictly only NADH but not NADPH is employed, it would require a supply of NADH for the 3-phosphoglycerate-branched pathway **(03-12)** to operate. Consequently, in order for the 3-phosphoglycerate-branched butanol-production pathway to operate, it is important to use a 3-hydroxybutyryl-CoA dehydrogenase **08** and a butyryl-CoA dehydrogenase **10** that can use NADPH which can be supplied by the photo-driven electron transport process. Therefore, it is a preferred practice to use a 3-hydroxybutyryl-CoA dehydrogenase and a butyryl-CoA dehydrogenase that can use NADPH or both NADPH and NADH (i.e., NAD(P)H) for this 3-phosphoglycerate-branched designer butanol-production pathway **(03-12** in Figure 1). Alternatively, when a 3-hydroxybutyryl-CoA dehydrogenase and a butyryl-CoA dehydrogenase that can use only NADH are employed, it is preferably here to use an additional embodiment that can confer an NADPH/NADH conversion mechanism (to supply NADH by converting NADPH to NADH, see more detail later in the text) in the designer organism to facilitate photosynthetic production of butanol through the 3-phosphoglycerate-branched designer pathway.

In still another example, a designer pathway is created that takes fructose-1,6-diphosphate and converts it into butanol by using, as shown with the numerical labels **20-33** in Figure 1, a set of enzymes consisting of aldolase **20,** triose phosphate isomerase **21,** glyceraldehyde-3-phosphate dehydrogenase **22,** phosphoglycerate kinase **23,** phosphoglycerate mutase **24,** enolase **25,** pyruvate kinase **26,** pyruvate-NADP⁺ oxidoreductase (or pyruvate-ferredoxin oxidoreductase) **27,** thiolase **28,** 3-hydroxybutyryl-CoA dehydrogenase **29,** crotonase **30,** butyryl-CoA dehydrogenase **31,** butyraldehyde dehydrogenase **32,** and butanol dehydrogenase **33,** with aldolase **20** and triose phosphate isomerase **21** being the only two additional enzymes relative to the glyceraldehydes-3-phosphate-branched designer pathway. The use of a pyruvate-NADP⁺ oxidoreductase **27** (instead of pyruvate-ferredoxin oxidoreductase) in catalyzing the conversion of a pyruvate molecule to acetyl-CoA enables production of an NADPH, which can be used in some other steps of the butanol-production pathway. The addition of yet one more enzyme in the designer organism, phosphofructose kinase **19,** permits the creation of another designer pathway which branches off from the point of fructose-6-phosphate of the Calvin cycle for the production of butanol. Like the glyceraldehyde-3-phosphate-branched butanol-production pathway, both the fructose-1,6-diphosphate-branched pathway **(20-33)** and the fructose-6-phosphate-branched pathway **(19-33)** can themselves generate NADH for use in the pathway at the step catalyzed by 3-hydroxybutyryl-CoA dehydrogenase **29** to reduce acetoacetyl-CoA to 3-hydroxybutyryl-CoA and at the step catalyzed by butyryl-CoA dehydrogenase **31** to reduce crotonyl-CoA to butyryl-CoA. In each of these designer butanol-production pathways, the numbers of NADH molecules consumed are balanced with the numbers of NADH molecules generated; and both the butyraldehyde dehydrogenase **32** (catalyzing the step in reducing butyryl-CoA to butyraldehyde) and the butanol dehydrogenase **33** (catalyzing the terminal step in reducing butyraldehyde to butanol) can all use NADPH, which can be regenerated by the photosynthetic water splitting and proton gradient-coupled electron transport process. Therefore, these designer butanol-production pathways can operate continuously.

Table 1 lists examples of the enzymes including those identified above for construction of the designer butanol-production pathways. Throughout this specification, when reference is made to an enzyme, such as, for example, any of the enzymes listed in Table 1, it includes their isozymes, functional analogs, and designer modified enzymes and combinations thereof. These enzymes can be selected for use in construction of the designer butanol-production pathways (such as those illustrated in Figure 1). The "isozymes or functional analogs" refer to certain enzymes that have the same catalytic function but may or may not have exactly the same protein structures. The most essential feature of an enzyme is its active site that catalyzes the enzymatic reaction. Therefore, certain enzyme-protein fragment(s) or subunit(s) that contains such an active catalytic site may also be selected for use in this invention. For various reasons, some of the natural enzymes contain not only the essential catalytic structure but also other structure components that may or may not be desirable for a given application. With techniques of bioinformatics-assisted molecular designing, it is possible to select the essential catalytic structure(s) for use in construction of a designer DNA construct encoding a desirable designer enzyme. Therefore, in one of the various embodiments, a designer enzyme gene is created by artificial synthesis of a DNA construct according to bioinformatics-assisted molecular sequence design. With the computer-assisted synthetic biology approach, any DNA sequence (thus its protein structure) of a designer enzyme may be selectively modified to achieve more desirable results by design. Therefore, the terms "designer modified sequences" and "designer modified enzymes" are hereby defined as the DNA sequences and the enzyme proteins that are modified with bioinformatics-assisted molecular design. For example, when a DNA construct for a designer chloroplast-targeted enzyme is designed from the sequence of a mitochondrial enzyme, it is a preferred practice to modify some of the protein structures, for example, by selectively cutting out certain structure component(s) such as its mitochondrial transit-peptide sequence that is not suitable for the given application, and/or by adding certain peptide structures such as an exogenous chloroplast transit-peptide sequence (e.g., a 135-bp Rubisco small-subunit transit peptide (RbcS2)) that is needed to confer the ability in the chloroplast-targeted insertion of the designer protein. Therefore, one of the various embodiments flexibly employs the enzymes, their isozymes, functional analogs, designer modified enzymes, and/or the combinations thereof in construction of the designer butanol-production pathway(s).

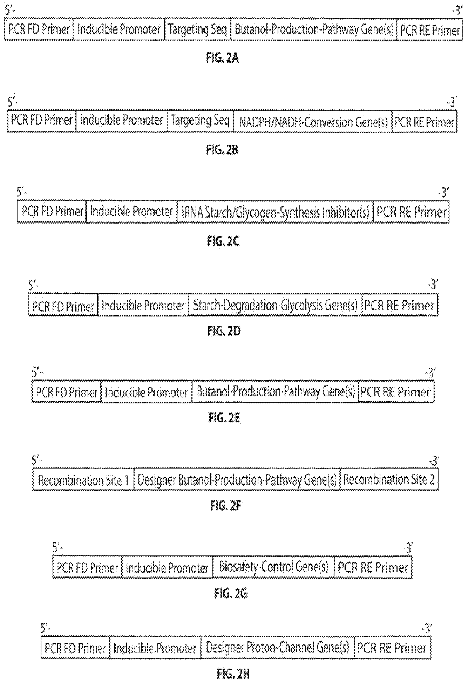

As shown in Table 1, many genes of the enzymes identified above have been cloned and/or sequenced from various organisms. Both genomic DNA and/or mRNA sequence data can be used in designing and synthesizing the designer DNA constructs for transformation of a host alga, oxyphotobacterium, plant, plant tissue or cells to create a designer organism for photobiological butanol production (Figure 1). However, because of possible variations often associated with various source organisms and cellular compartments with respect to a specific host organism and its chloroplast/thylakoid environment where the butanol-production pathway(s) is designed to work with the Calvin cycle, certain molecular engineering art work in DNA construct design including codon-usage optimization and sequence modification is often necessary for a designer DNA construct (Figure 2) to work well. For example, in creating a butanol-producing designer eukaryotic alga, if the source sequences are from cytosolic enzymes (sequences), a functional chloroplast-targeting sequence may be added to provide the capability for a designer unclear gene-encoded enzyme to insert into a host chloroplast to confer its function for a designer butanol-production pathway. Furthermore, to provide the switchability for a designer butanol-production pathway, it is also important to include a functional inducible promoter sequence such as the promoter of a hydrogenase (Hydl) or nitrate reductase (Nia1) gene, or nitrite reductase (nirA) gene in certain designer DNA construct(s) as illustrated in Fig. 2A to control the expression of designer gene(s). In addition, as mentioned before, certain functional derivatives or fragments of these enzymes (sequences), chloroplast-targeting transit peptide sequences, and inducible promoter sequences can also be selected for use in full, in part or in combinations thereof, to create the designer organisms according to various embodiments of this invention. The arts in creating and using the designer organisms are further described hereinbelow.

### Targeting the designer enzymes to the stroma region of chloroplasts

Some of the designer enzymes discussed above, such as, pyruvate-ferredoxin oxidoreductase, thiolase, 3-hydroxybutyryl-CoA dehydrogenase, crotonase, butyryl-CoA dehydrogenase, butyraldehyde dehydrogenase, and butanol dehydrogenase are known to function in certain special bacteria such as *Clostridium;* but wild-type plant chloroplasts generally do not possess these enzymes to function with the Calvin cycle. Therefore, in one of the various embodiments in creating a butanol-producing eukaryotic designer organism, designer nucleic acids encoding for these enzymes are expressed in the chloroplast(s) of a host cell. This can be accomplished by delivery of designer butanol-production-pathway gene(s) into the chloroplast genome of the eukaryotic host cell typically using a genegun. In certain extent, the molecular genetics of chloroplasts are similar to that of cyanobacteria. After being delivered into the chloroplast, a designer DNA construct that contains a pair of proper recombination sites as illustrated in Figure 2F can be incorporated into the chloroplast genome through a natural process of homologous DNA double recombination.

In another embodiment, nucleic acids encoding for these enzymes are genetically engineered such that the enzymes expressed are inserted into the chloroplasts to operate with the Calvin cycle there. Depending on the genetic background of a particular host organism, some of the designer enzymes discussed above such as phosphoglycerate mutase and enolase may exist at some background levels in its native form in a wild-type chloroplast. For various reasons including often the lack of their controllability, however, some of the chloroplast background enzymes may or may not be sufficient to serve as a significant part of the designer butanol-production pathway(s). Furthermore, a number of useful inducible promoters happen to function in the nuclear genome. For example, both the hydrogenase (Hydl) promoter and the nitrate reductase (Nia1) promoter that can be used to control the expression of the designer butanol-production pathways are located in the nuclear genome of *Chlamydomonas reinhardtii,* of which the genome has recently been sequenced. Therefore, in one of the various embodiments, it is preferred to use nuclear-genome-encodable designer genes to confer a switchable butanol-production pathway. Consequently, nucleic acids encoding for these enzymes also need to be genetically engineered with proper sequence modification such that the enzymes are controllably expressed and are inserted into the chloroplasts to create a designer butanol-production pathway.

According to one of the various embodiments, it is best to express the designer butanol-producing-pathway enzymes only into chloroplasts (at the stroma region), exactly where the action of the enzymes is needed to enable photosynthetic production of butanol. If expressed without a chloroplast-targeted insertion mechanism, the enzymes would just stay in the cytosol and not be able to directly interact with the Calvin cycle for butanol production. Therefore, in addition to the obvious distinctive features in pathway designs and associated approaches, another significant distinction is that one of the various embodiments innovatively employs a chloroplast-targeted mechanism for genetic insertion of many designer butanol-production-pathway enzymes into chloroplast to directly interact with the Calvin cycle for photobiological butanol production.

With a chloroplast stroma-targeted mechanism, the cells will not only be able to produce butanol but also to grow and regenerate themselves when they are returned to certain conditions under which the designer pathway is turned off, such as under aerobic conditions when designer hydrogenase promoter-controlled butanol-production-pathway genes are used. Designer algae, plants, or plant cells that contain normal mitochondria should be able to use the reducing power (NADH) from organic reserves (and/or some exogenous organic substrate such as acetate or sugar) to power the cells immediately after returning to aerobic conditions. Consequently, when the designer algae, plants, or plant cells are returned to aerobic conditions after use under anaerobic conditions for photosynthetic butanol production, the cells will stop making the butanol-producing-pathway enzymes and start to restore the normal photoautotrophic capability by synthesizing new and functional chloroplasts. Therefore, it is possible to use such genetically engineered designer alga/plant organisms for repeated cycles of photoautotrophic growth under normal aerobic conditions and efficient production of butanol directly from CO₂ and H₂O under certain specific designer butanol-producing conditions such as under anaerobic conditions and/or in the presence of nitrate when a Nia1 promoter-controlled butanol-production pathway is used.

The targeted insertion of designer butanol-production-pathway enzymes can be accomplished through use of a DNA sequence that encodes for a stroma "signal" peptide. A stroma-protein signal (transit) peptide directs the transport and insertion of a newly synthesized protein into stroma. In accordance with one of the various embodiments, a specific targeting DNA sequence is preferably placed in between the promoter and a designer butanol-production-pathway enzyme sequence, as shown in a designer DNA construct (Figure 2A). This targeting sequence encodes for a signal (transit) peptide that is synthesized as part of the apoprotein of an enzyme in the cytosol. The transit peptide guides the insertion of an apoprotein of a designer butanol-production-pathway enzyme from cytosol into the chloroplast. After the apoprotein is inserted into the chloroplast, the transit peptide is cleaved off from the apoprotein, which then becomes an active enzyme.

A number of transit peptide sequences are suitable for use for the targeted insertion of the designer butanol-production-pathway enzymes into chloroplast, including but not limited to the transit peptide sequences of: the hydrogenase apoproteins (such as HydA1 (Hydl) and HydA2, GenBank accession number AJ308413, AF289201, AY090770), ferredoxin apoprotein (Frx1, accession numbers L10349, P07839), thioredoxin m apoprotein (Trx2, X62335), glutamine synthase apoprotein (Gs2, Q42689), LhcII apoproteins (AB051210, AB051208, AB051205), PSII-T apoprotein (PsbT), PSII-S apoprotein (PsbS), PSII-W apoprotein (PsbW), CF₀CF₁ subunit-γ apoprotein (AtpC), CF₀CF₁ subunit-δ apoprotein (AtpD, U41442), CF₀CF₁ subunit-II apoprotein (AtpG), photosystem I (PSI) apoproteins (such as, of genes PsaD, PsaE, PsaF, PsaG, PsaH, and PsaK), Rubisco SSU apoproteins (such as RbcS2, X04472). Throughout this specification, when reference is made to a transit peptide sequence, such as, for example, any of the transit peptide sequence described above, it includes their functional analogs, modified designer sequences, and combinations thereof. A "functional analog" or "modified designer sequence" in this context refers to a peptide sequence derived or modified (by, e.g., conservative substitution, moderate deletion or addition of amino acids, or modification of side chains of amino acids) based on a native transit peptide sequence, such as those identified above, that has the same function as the native transit peptide sequence, i.e., effecting targeted insertion of a desired enzyme.

In certain specific embodiments, the following transit peptide sequences are used to guide the insertion of the designer butanol-production-pathway enzymes into the stroma region of the chloroplast: the Hyd1 transit peptide (having the amino acid sequence: msalvlkpca avsirgsscr arqvaprapl aastvrvala tleaparrlg nvacaa (SEQ ID NO: 54)), the RbcS2 transit peptides (having the amino acid sequence: maaviakssv saavarpars svrpmaalkp avkaapvaap aqanq (SEQ ID NO: 55)), ferredoxin transit peptide (having the amino acid sequence: mamamrs (SEQ ID NO: 56)), the CF₀CF₁ subunit-δ transit peptide (having the amino acid sequence: mlaaksiagp rafkasavra apkagrrtw vma (SEQ ID NO: 57)), their analogs, functional derivatives, designer sequences, and combinations thereof.

### Use of a genetic switch to control the expression of a designer butanol-producing pathway.

Another key feature of the invention is the application of a genetic switch to control the expression of the designer butanol-producing pathway(s), as illustrated in Figure 1. This switchability is accomplished through the use of an externally inducible promoter so that the designer transgenes are inducibly expressed under certain specific inducing conditions. Preferably, the promoter employed to control the expression of designer genes in a host is originated from the host itself or a closely related organism. The activities and inducibility of a promoter in a host cell can be tested by placing the promoter in front of a reporting gene, introducing this reporter construct into the host tissue or cells by any of the known DNA delivery techniques, and assessing the expression of the reporter gene.

In a preferred embodiment, the inducible promoter used to control the expression of designer genes is a promoter that is inducible by anaerobiosis, i.e., active under anaerobic conditions but inactive under aerobic conditions. A designer alga/plant organism can perform autotrophic photosynthesis using CO₂ as the carbon source under aerobic conditions, and when the designer organism culture is grown and ready for photosynthetic butanol production, anaerobic conditions will be applied to turn on the promoter and the designer genes that encode a designer butanol-production pathway(s).

A number of promoters that become active under anaerobic conditions are suitable for use in the present invention. For example, the promoters of the hydrogenase genes (HydA1 (Hydl) and HydA2, GenBank accession number: AJ308413, AF289201, AY090770) of *Chlamydomonas reinhardtii,* which is active under anaerobic conditions but inactive under aerobic conditions, can be used as an effective genetic switch to control the expression of the designer genes in a host alga, such as *Chlamydomonas reinhardtii.* In fact, *Chlamydomonas* cells contain several nuclear genes that are coordinately induced under anaerobic conditions. These include the hydrogenase structural gene itself (Hyd1), the Cyc6 gene encoding the apoprotein of Cytochrome C₆, and the Cpxl gene encoding coprogen oxidase. The regulatory regions for the latter two have been well characterized, and a region of about 100 bp proves sufficient to confer regulation by anaerobiosis in synthetic gene constructs (Quinn, Barraco, Ericksson and Merchant (2000). "Coordinate copper- and oxygen-responsive Cyc6 and Cpx1 expression in Chlamydomonas is mediated by the same element." JBiol Chem 275: 6080-6089). Although the above inducible algal promoters may be suitable for use in other plant hosts, especially in plants closely related to algae, the promoters of the homologous genes from these other plants, including higher plants, can be obtained and employed to control the expression of designer genes in those plants.

In another embodiment, the inducible promoter used in the present invention is an algal nitrate reductase (Nia1) promoter, which is inducible by growth in a medium containing nitrate and repressed in a nitrate-deficient but ammonium-containing medium (Loppes and Radoux (2002) "Two short regions of the promoter are essential for activation and repression of the nitrate reductase gene in Chlamydomonas reinhardtii," Mol Genet Genomics 268: 42-48). Therefore, the Nia1 (gene accession number AF203033) promoter can be selected for use to control the expression of the designer genes in an alga according to the concentration levels of nitrate and ammonium in a culture medium. Additional inducible promoters that can also be selected for use in the present invention include, for example, the heat-shock protein promoter HSP70A (accession number: DQ059999, AY456093, M98823; Schroda, Blocker, Beek (2000) The HSP70A promoter as a tool for the improved expression of transgenes in Chlamydomonas. Plant Journal 21:121-131), the promoter of CabII-1 gene (accession number M24072), the promoter of Ca1 gene (accession number P20507), and the promoter of Ca2 gene (accession number P24258).

In the case of blue-green algae (oxyphotobacteria including cyanobacteria and oxychlorobacteria), there are also a number of inducible promoters that can be selected for use in the present invention. For example, the promoters of the anaerobic-responsive bidirectional hydrogenase *hox* genes of *Nostoc sp.* PCC 7120 (GenBank: BA000019), *Prochlorothrix hollandica* (GenBank: U88400; *hoxUYH* operon promoter), *Synechocystis* sp. strain PCC 6803 (CyanoBase: sll1220 and sll1223), *Synechococcus elongatus* PCC 6301 (CyanoBase: syc1235_c), *Arthrospira platensis* (GenBank: ABC26906), *Cyanothece sp.* CCY0110 (GenBank: ZP_01727419) and *Synechococcus sp.* PCC 7002 (GenBank: AAN03566), which are active under anaerobic conditions but inactive under aerobic conditions (Sjoholm, Oliveira, and Lindblad (2007) "Transcription and regulation of the bidirectional hydrogenase in the Cyanobacterium Nostoc sp. strain PCC 7120," Applied and Environmental Microbiology, 73(17): 5435-5446), can be used as an effective genetic switch to control the expression of the designer genes in a host oxyphotobacterium, such as *Nostoc sp.* PCC 7120, *Synechocystis* sp. strain PCC 6803, *Synechococcus elongatus* PCC 6301, *Cyanothece sp.* CCY0110, *Arthrospira platensis,* or *Synechococcus sp.* PCC 7002.

In another embodiment in creating switchable butanol-production designer organisms such as switchable designer oxyphotobacteria, the inducible promoter selected for use is a nitrite reductase (*nirA*) promoter, which is inducible by growth in a medium containing nitrate and repressed in a nitrate-deficient but ammonium-containing medium (Qi, Hao, Ng, Slater, Baszis, Weiss, and Valentin (2005) "Application of the Synechococcus nirA promoter to establish an inducible expression system for engineering the Synechocystis tocopherol pathway," Applied and Environmental Microbiology, 71(10): 5678-5684; Maeda, Kawaguchi, Ohe, and Omata (1998) "cis-Acting sequences required for NtcB-dependent, nitrite-responsive positive regulation of the nitrate assimilation operon in the Cyanobacterium Synechococcus sp. strain PCC 7942," Journal of Bacteriology, 180(16):4080-4088). Therefore, the *nirA* promoter sequences can be selected for use to control the expression of the designer genes in a number of oxyphotobacteria according to the concentration levels of nitrate and ammonium in a culture medium. The *nirA* promoter sequences that can be selected and modified for use include (but not limited to) the *nirA* promoters of the following oxyphotobacteria: *Synechococcus elongatus* PCC 6301 (GenBank: AP008231, region 355890-255950), *Synechococcus sp.* (GenBank: X67680.1, D16303.1, D12723.1, and D00677), *Synechocystis sp.* PCC 6803 (GenBank: NP_442378, BA000022, AB001339, D63999-D64006, D90899-D90917), *Anabaena sp.* (GenBank: X99708.1), *Nostoc sp.* PCC 7120 (GenBank: BA000019.2 and AJ319648), *Plectonema boryanum* (GenBank: D31732.1), *Synechococcus elongatus* PCC 7942 (GenBank: P39661, CP000100.1), *Thermosynechococcus elongatus* BP-1 (GenBank: BAC08901, NP_682139), *Phormidium laminosum* (GenBank: CAA79655, Q51879), *Mastigocladus laminosus* (GenBank: ABD49353, ABD49351, ABD49349, ABD49347), *Anabaena variabilis* ATCC 29413 (GenBank: YP_325032), *Prochlorococcus marinus str.* MIT 9303 (GenBank: YP_001018981), *Synechococcus sp.* WH 8103 (GenBank: AAC17122), *Synechococcus sp.* WH 7805 (GenBank: ZP_01124915), and *Cyanothece sp.* CCY0110 (GenBank: ZP_01727861).

In yet another embodiment, an inducible promoter selected for use is the light- and heat-responsive chaperone gene *groE* promoter, which can be induced by heat and/or light [Kojima and Nakamoto (2007) "A novel light- and heat-responsive regulation of the groE transcription in the absence of HrcA or CIRCE in cyanobacteria," FEBS Letters 581:1871-1880). A number of *groE* promoters such as the *groES* and *groEL* (chaperones) promoters are available for use as an inducible promoter in controlling the expression of the designer butanol-production-pathway enzymes. The *groE* promoter sequences that can be selected and modified for use in one of the various embodiments include (but not limited to) the *groES* and/or *groEL* promoters of the following oxyphotobacteria: *Synechocystis sp.* (GenBank: D12677.1), *Synechocystis sp.* PCC 6803 (GenBank: BA000022.2), *Synechococcus elongatus* PCC 6301 (GenBank: AP008231.1), *Synechococcus sp* (GenBank: M58751.1), *Synechococcus elongatus* PCC 7942 (GenBank: CP000100.1), *Nostoc sp.* PCC 7120 (GenBank: BA000019.2), *Anabaena variabilis* ATCC 29413 (GenBank: CP000117.1), *Anabaena sp.* L-31 (GenBank: AF324500); *Thermosynechococcus elongatus* BP-1 (CyanoBase: t110185, t110186), *Synechococcus vulcanus* (GenBank: D78139), *Oscillatoria sp.* NKBG091600 (GenBank: AF054630), *Prochlorococcus marinus* MIT9313 (GenBank: BX572099), *Prochlorococcus marinus str.* MIT 9303 (GenBank: CP000554), *Prochlorococcus marinus str.* MIT 9211 (GenBank: ZP_01006613), *Synechococcus sp.* WH8102 (GenBank: BX569690), Synechococcus sp. CC9605 (GenBank: CP000110), *Prochlorococcus marinus subsp.* marinus str. CCMP1375 (GenBank: AE017126), and *Prochlorococcus marinus* MED4 (GenBank: BX548174).

Additional inducible promoters that can also be selected for use in the present invention include: for example, the metal (zinc)-inducible *smt* promoter of *Synechococcus* PCC 7942 (Erbe, Adams, Taylor and Hall (1996) "Cyanobacteria carrying an smt-lux transcriptional fusion as biosensors for the detection of heavy metal cations," Journal of Industrial Microbiology, 17:80-83); the iron-responsive *idiA* promoter of *Synechococcus elongatus* PCC 7942 (Michel, Pistorius, and Golden (2001) "Unusual regulatory elements for iron deficiency induction of the idiA gene of Synechococcus elongatus PCC 7942" Journal of Bacteriology, 183(17):5015-5024); the redox-responsive cyanobacterial *crhR* promoter (Patterson-Fortin, Colvin and Owttrim (2006) "A LexA-related protein regulates redox-sensitive expression of the cyanobacterial RNA helicase, crhR", Nucleic Acids Research, 34(12):3446-3454); the heat-shock gene *hsp16.6* promoter of *Synechocystis* sp. PCC 6803 (Fang and Barnum (2004) "Expression of the heat shock gene hsp16.6 and promoter analysis in the Cyanobacterium, Synechocystis sp. PCC 6803," Current Microbiology 49:192-198); the small heat-shock protein (Hsp) promoter such as *Synechococcus vulcanus* gene *hspA* promoter (Nakamoto, Suzuki, and Roy (2000) "Constitutive expression of a small heat-shock protein confers cellular thermotolerance and thermal protection to the photosynthetic apparatus in cyanobacteria," FEBS Letters 483:169-174); the CO₂-responsive promoters of oxyphotobacterial carbonic-anhydrase genes (GenBank: EAZ90903, EAZ90685, ZP_01624337, EAW33650, ABB17341, AAT41924, CA089711, ZP_00111671, YP_400464, AAC44830; and CyanoBase: all2929, PMT1568 slr0051, slr1347, and syc0167_c); the nitrate-reductase-gene (*narB*) promoters (such as GenBank accession numbers: BAC08907, NP_682145, AAO25121; ABI46326, YP_732075, BAB72570, NP_484656); the green/red light-responsive promoters such as the light-regulated *cpcB2A2* promoter of *Fremyella diplosiphon* (Casey and Grossman (1994) "In vivo and in vitro characterization of the light-regulated cpcB2A2 promoter of Fremyella diplosiphont" Journal of Bacteriology, 176(20):6362-6374); and the UV-light responsive promoters of cyanobacterial genes *lexA*, *reca* and *ruvB* (Domain, Houot, Chauvat, and Cassier-Chauvat (2004) "Function and regulation of the cyanobacterial genes lexA, recA and ruvB: LexA is critical to the survival of cells facing inorganic carbon starvation," Molecular Microbiology, 53(1):65-80).

Furthermore, in one of the various embodiments, certain "semi-inducible" or constitutive promoters can also be selected for use in combination of an inducible promoter(s) for construction of a designer butanol-production pathway(s) as well. For example, the promoters of oxyphotobacterial Rubisco operon such as the *rbcL* genes (GenBank: X65960, ZP_01728542, Q3M674, BAF48766, NP_895035, 0907262A; CyanoBase: PMT1205, PMM0550, Pro0551, tll1506, SYNW1718, glr2156, alr1524, slr0009), which have certain light-dependence but could be regarded almost as constitutive promoters, can also be selected for use in combination of an inducible promoter(s) such as the *nirA, hox,* and/or *groE* promoters for construction of the designer butanol-production pathway(s) as well.

Throughout this specification, when reference is made to inducible promoter, such as, for example, any of the inducible promoters described above, it includes their analogs, functional derivatives, designer sequences, and combinations thereof. A "functional analog" or "modified designer sequence" in this context refers to a promoter sequence derived or modified (by, e.g., substitution, moderate deletion or addition or modification of nucleotides) based on a native promoter sequence, such as those identified hereinabove, that retains the function of the native promoter sequence.

### DNA constructs and transformation into host organisms

DNA constructs are generated in order to introduce designer butanol-production-pathway genes to a host alga, plant, plant tissue or plant cells. That is, a nucleotide sequence encoding a designer butanol-production-pathway enzyme is placed in a vector, in an operable linkage to a promoter, preferably an inducible promoter, and in an operable linkage to a nucleotide sequence coding for an appropriate chloroplast-targeting transit-peptide sequence. In a preferred embodiment, nucleic acid constructs are made to have the elements placed in the following 5' (upstream) to 3' (downstream) orientation: an externally inducible promoter, a transit targeting sequence, and a nucleic acid encoding a designer butanol-production-pathway enzyme, and preferably an appropriate transcription termination sequence. One or more designer genes (DNA constructs) can be placed into one genetic vector. An example of such a construct is depicted in Figure 2A. As shown in the embodiment illustrated in Figure 2A, a designer butanol-production-pathway transgene is a nucleic acid construct comprising: a) a PCR forward primer; b) an externally inducible promoter; c) a transit targeting sequence; d) a designer butanol-production-pathway-enzyme-encoding sequence with an appropriate transcription termination sequence; and e) a PCR reverse primer.

In accordance with various embodiments, any of the components a) through e) of this DNA construct are adjusted to suit for certain specific conditions. In practice, any of the components a) through e) of this DNA construct are applied in full or in part, and/or in any adjusted combination to achieve more desirable results. For example, when an algal hydrogenase promoter is used as an inducible promoter in the designer butanol-production-pathway DNA construct, a transgenic designer alga that contains this DNA construct will be able to perform autotrophic photosynthesis using ambient-air CO₂ as the carbon source and grows normally under aerobic conditions, such as in an open pond. When the algal culture is grown and ready for butanol production, the designer transgene(s) can then be expressed by induction under anaerobic conditions because of the use of the hydrogenase promoter. The expression of designer gene(s) produces a set of designer butanol-production-pathway enzymes to work with the Calvin cycle for photobiological butanol production (Figure 1).

The two PCR primers are a PCR forward primer (PCR FD primer) located at the beginning (the 5' end) of the DNA construct and a PCR reverse primer (PCR RE primer) located at the other end (the 3' end) as shown in Fig. 2A. This pair of PCR primers is designed to provide certain convenience when needed for relatively easy PCR amplification of the designer DNA construct, which is helpful not only during and after the designer DNA construct is synthesized in preparation for gene transformation, but also after the designer DNA construct is delivered into the genome of a host alga for verification of the designer gene in the transformants. For example, after the transformation of the designer gene is accomplished in a *Chlamydomonas reinhardtii*-arg7 host cell using the techniques of electroporation and argininosuccinate lyase (*arg7*) complementation screening, the resulted transformants can be then analyzed by a PCR DNA assay of their nuclear DNA using this pair of PCR primers to verify whether the entire designer butanol-production-pathway gene (the DNA construct) is successfully incorporated into the genome of a given transformant. When the nuclear DNA PCR assay of a transformant can generate a PCR product that matches with the predicted DNA size and sequence according to the designer DNA construct, the successful incorporation of the designer gene(s) into the genome of the transformant is verified.

Therefore, the various embodiments also teach the associated method to effectively create the designer transgenic algae, plants, or plant cells for photobiological butanol production. This method, in one of embodiments, includes the following steps: a) Selecting an appropriate host alga, plant, plant tissue, or plant cells with respect to their genetic backgrounds and special features in relation to butanol production; b) Introducing the nucleic acid constructs of the designer genes into the genome of said host alga, plant, plant tissue, or plant cells; c) Verifying the incorporation of the designer genes in the transformed alga, plant, plant tissue, or plant cells with DNA PCR assays using the said PCR primers of the designer DNA construct; d) Measuring and verifying the designer organism features such as the inducible expression of the designer butanol-pathway genes for photosynthetic butanol production from carbon dioxide and water by assays of mRNA, protein, and butanol-production characteristics according to the specific designer features of the DNA construct(s) (Figure 2A).

The above embodiment of the method for creating the designer transgenic organism for photobiological butanol production can also be repeatedly applied for a plurality of operational cycles to achieve more desirable results. In various embodiments, any of the steps a) through d) of this method described above are adjusted to suit for certain specific conditions. In various embodiments, any of the steps a) through d) of the method are applied in full or in part, and/or in any adjusted combination.

Examples of designer butanol-production-pathway genes (DNA constructs) are shown in the sequence listings. SEQ ID NO: 1 presents a detailed DNA construct of a designer Butanol Dehydrogenase gene (1809 bp) that includes a PCR FD primer (sequence 1-20), a 262-bp nitrate reductase Nia1 promoter (21-282), a 135-bp *RbcS2* transit peptide (283-417), an enzyme-encoding sequence (418-1566) selected and modified from a *Clostridium saccharoperbutylacetonicum* Butanol Dehydrogenase sequence (AB257439), a 223-bp *RbcS2* terminator (1567-1789), and a PCR RE primer (1790-1809). The 262-bp Nia1 promoter (DNA sequence 21-282) is used as an example of an inducible promoter to control the expression of a designer butanol-production-pathway Butanol Dehydrogenase gene (DNA sequence 418-1566). The 135-bp *RbcS2* transit peptide (DNA sequence 283-417) is used as an example to guide the insertion of the designer enzyme (DNA sequence 418-1566) into the chloroplast of the host organism. The *RbcS2* terminator (DNA sequence 1567-1789) is employed so that the transcription and translation of the designer gene is properly terminated to produce the designer apoprotein (RbcS2 transit peptide-Butanol Dehydrogenase) as desired. Because the Nia1 promoter is a nuclear DNA that can control the expression only for nuclear genes, the synthetic butanol-production-pathway gene in this example is designed according to the codon usage of *Chlamydomonas* nuclear genome. Therefore, in this case, the designer enzyme gene is transcribed in nucleus. Its mRNA is naturally translocated into cytosol, where the mRNA is translated to an apoprotein that consists of the RbcS2 transit peptide (corresponding to DNA sequence 283-417) with its C-terminal end linked together with the N-terminal end of the Butanol Dehydrogenase protein (corresponding to DNA sequence 418-1566). The transit peptide of the apoprotein guides its transportation across the chloroplast membranes and into the stroma area, where the transit peptide is cut off from the apoprotein. The resulting Butanol Dehydrogenase then resumes its function as an enzyme for the designer butanol-production pathway in chloroplast. The two PCR primers (sequences 1-20 and 1790-1809) are selected and modified from the sequence of a Human actin gene and can be paired with each other. Blasting the sequences against *Chlamydomonas* GenBank found no homologous sequences of them. Therefore, they can be used as appropriate PCR primers in DNA PCR assays for verification of the designer gene in the transformed alga.

SEQ ID NO: 2 presents example 2 for a designer Butyraldehyde Dehydrogenase DNA construct (2067 bp) that includes a PCR FD primer (sequence 1-20), a 262-bp nitrate reductase Nia1 promoter (21-282), a 135-bp *RbcS2* transit peptide (283-417), a Butyraldehyde Dehydrogenase-encoding sequence (418-1824) selected and modified from a *Clostridium saccharoperbutylacetonicum* Butyraldehyde Dehydrogenase sequence (AY251646), a 223-bp *RbcS2* terminator (1825-2047), and a PCR RE primer (2048-2067). This DNA construct is similar to example 1, SEQ ID NO: 1, except that a Butyraldehyde Dehydrogenase-encoding sequence (418-1824) selected and modified from a *Clostridium saccharoperbutylacetonicum* Butyraldehyde Dehydrogenase sequence (AY251646) is used.

SEQ ID NO: 3 presents example 3 for a designer Butyryl-CoA Dehydrogenase construct (1815 bp) that includes a PCR FD primer (sequence 1-20), a 262-bp nitrate reductase promoter (21-282), a 9-bp Xho I NdeI site (283-291), a 135-bp *RbcS2* transit peptide (292-426), a Butyryl-CoA Dehydrogenase encoding sequence (427-1563) selected/modified from the sequences of a *Clostridium beijerinckii* Butyryl-CoA Dehydrogenase (AF494018), a 9-bp XbaI site (1564-1572), a 223-bp *RbcS2* terminator (1573-1795), and a PCR RE primer (1796-1815) at the 3' end. This DNA construct is similar to example 1, SEQ ID NO: 1, except that a Butyryl-CoA Dehydrogenase encoding sequence (427-1563) selected/modified from the sequences of a *Clostridium beijerinckii* Butyryl-CoA Dehydrogenase (AF494018) is used and restriction sites of Xho I NdeI and XbaI are added to make the key components such as the targeting sequence (292-426) and the designer enzyme sequence (427-1563) as a modular unit that can be flexible replaced when necessary to save cost of gene synthesis and enhance work productivity. Please note, the enzyme does not have to be *Clostridium beijerinckii* Butyryl-CoA Dehydrogenase; a number of butyryl-CoA dehydrogenase enzymes (such as those listed in Table 1) including their isozymes, designer modified enzymes, and functional analogs from other sources such as *Butyrivibrio fibrisolvens, Butyrate-producing bacterium L2-50, Thermoanaerobacterium thermosaccharolyticum,* can also be selected for use.

SEQ ID NO: 4 presents example 4 for a designer Crotonase DNA construct (1482 bp) that includes a PCR FD primer (sequence 1-20), a 262-bp nitrate reductase promoter (21-282), a 9-bp Xho I NdeI site (283-291) a 135-bp *RbcS2* transit peptide (292-426), a Crotonase-encoding sequence (427-1209) selected/modified from the sequences of a *Clostridium beijerinckii* Crotonase (GenBank: AF494018), a 21-bp Lumio-tag-encoding sequence (1210-1230), a 9-bp XbaI site (1231-1239) containing a stop codon, a 223-bp *RbcS2* terminator (1240-1462), and a PCR RE primer (1463-1482) at the 3' end. This DNA construct is similar to example 3, SEQ ID NO: 3, except that a Crotonase-encoding sequence (427-1209) selected/modified from the sequences of a *Clostridium beijerinckii* Crotonase (GenBank: AF494018) is used and a 21-bp Lumio-tag-encoding sequence (1210-1230) is added at the C-terminal end of the enolase sequence. The 21-bp Lumio-tag sequence (1210-1230) is employed here to encode a Lumio peptide sequence Gly-Cys-Cys-Pro-Gly-Cys-Cys, which can become fluorescent when treated with a Lumio reagent that is now commercially available from Invitrogen [https://catalog.invitrogen.com]. Lumio molecular tagging technology is based on an EDT (1,2-ethanedithiol) coupled biarsenical derivative (the Lumio reagent) of fluorescein that binds to an engineered tetracysteine sequence (Keppetipola, Coffman, and et al (2003). Rapid detection of in vitro expressed proteins using Lumio^{™} technology, *Gene Expression*, 25.3: 7-11). The tetracysteine sequence consists of Cys-Cys-Xaa-Xaa-Cys-Cys, where Xaa is any non-cysteine amino acid such as Pro or Gly in this example. The EDT-linked Lumio reagent allows free rotation of the arsenic atoms that quenches the fluorescence of fluorescein. Covalent bond formation between the thiols of the Lumio's arsenic groups and the tetracysteines prevents free rotation of arsenic atoms that releases the fluorescence of fluorescein (Griffin, Adams, and Tsien (1998), "Specific covalent labeling of recombinant protein molecules inside live cells", Science, 281:269-272). This also permits the visualization of the tetracysteine-tagged proteins by fluorescent molecular imaging. Therefore, use of the Lumio tag in this manner enables monitoring and/or tracking of the designer Crotonase when expressed to verify whether the designer butanol-production pathway enzyme is indeed delivered into the chloroplast of a host organism as designed. The Lumio tag (a short 7 amino acid peptide) that is linked to the C-terminal end of the Crotonase protein in this example should have minimal effect on the function of the designer enzyme, but enable the designer enzyme molecule to be visualized when treated with the Lumio reagent. Use of the Lumio tag is entirely optional. If the Lumio tag somehow affects the designer enzyme function, this tag can be deleted in the DNA sequence design.

SEQ ID NO: 5 presents example 5 for a designer 3-Hydroxybutyryl-CoA Dehydrogenase DNA construct (1367 bp) that includes a PCR FD primer (sequence 1-20), a 84-bp nitrate reductase promoter (21-104), a 9-bp Xho I NdeI site (105-113) a 135-bp *RbcS2* transit peptide (114-248), a 3-Hydroxybutyryl-CoA Dehydrogenase-encoding sequence (249-1094) selected/modified from a *Clostridium beijerinckii* 3-Hydroxybutyryl-CoA Dehydrogenase sequence (Genbank: AF494018), a 21-bp Lumio-tag sequence (1095-1115), a 9-bp XbaI site (1116-1124), a 223-bp *RbcS2* terminator (1125-1347), and a PCR RE primer (1348-1367). This DNA construct is similar to example 4, SEQ ID NO: 4, except that an 84-bp nitrate reductase promoter (21-104) and a 3-Hydroxybutyryl-CoA Dehydrogenase-encoding sequence (249-1094) selected/modified from a *Clostridium beijerinckii* 3-Hydroxybutyryl-CoA Dehydrogenase sequence (Genbank: AF494018) are used. The 84-bp nitrate-reductase promoter is artificially created by joining two partially homologous sequence regions (-231 to -201 and -77 to -25 with respect to the start site of transcription) of the native *Chlamydomonas reinhardtii* Nial promoter. Experimental studies have demonstrated that the 84-bp sequence is more active than the native Nial promoter (Loppes and Radoux (2002) "Two short regions of the promoter are essential for activation and repression of the nitrate reductase gene in Chlamydomonas reinhardtii," Mol Genet Genomics 268: 42-48). Therefore, this is also an example where functional synthetic sequences, analogs, functional derivatives and/or designer modified sequences such as the synthetic 84-bp sequence can be selected for use according to various embodiments in this invention.

SEQ ID NO: 6 presents example 6 for a designer Thiolase DNA construct (1721 bp) that includes a PCR FD primer (sequence 1-20), a 84-bp nitrate reductase promoter (21-104), a 9-bp Xho I NdeI site (105-113) a 135-bp *RbcS2* transit peptide (114-248), a Thiolase-encoding sequence (248-1448) selected/modified from a *Butyrivibrio fibrisolvens* Thiolase sequence (AB190764), a 21-bp Lumio-tag sequence (1449-1469), a 9-bp XbaI site (1470-1478), a 223-bp *RbcS2* terminator (1479-1701), and a PCR RE primer (1702-1721). This DNA construct is also similar to example 4, SEQ ID NO: 4, except that a Thiolase-encoding-encoding sequence (249-1448) and an 84-bp synthetic Nia1 promoter (21-104) are used. This is another example that functional synthetic sequences can also be selected for use in designer DNA constructs.

SEQ ID NO: 7 presents example 7 for a designer Pyruvate-Ferredoxin Oxidoreductase DNA construct (4211 bp) that includes a PCR FD primer (sequence 1-20), a 2x84-bp nitrate reductase promoter (21-188), a 9-bp Xho I NdeI site (189-197) a 135-bp *RbcS2* transit peptide (198-332), a Pyruvate-Ferredoxin Oxidoreductase-encoding sequence (333-3938) selected/modified from the sequences of a *Mastigamoeba balamuthi* Pyruvate-ferredoxin oxidoreductase (GenBank: AY101767), a 21-bp Lumio-tag sequence (3939-3959), a 9-bp XbaI site (3960-3968), a 223-bp *RbcS2* terminator (3969-4191), and a PCR RE primer (4192-4211). This DNA construct is also similar to example 4, SEQ ID NO: 4, except a designer 2x84-bp Nia1 promoter and a Pyruvate-Ferredoxin Oxidoreductase-encoding sequence (333-3938) selected/modified from the sequences of a *Mastigamoeba balamuthi* Pyruvate-ferredoxin oxidoreductase (GenBank: AY101767) are used. The 2x84-bp Nia1 promoter is constructed as a tandem duplication of the 84-bp synthetic Nia1 promoter sequence presented in SEQ ID NO: 6 above. Experimental tests have shown that the 2x84-bp synthetic Nia1 promoter is even more powerful than the 84-bp sequence which is more active than the native Nia1 promoter (Loppes and Radoux (2002) "Two short regions of the promoter are essential for activation and repression of the nitrate reductase gene in Chlamydomonas reinhardtii," Mol Genet Genomics 268: 42-48). Use of this type of inducible promoter sequences with various promoter strengths can also help in adjusting the expression levels of the designer enzymes for the butanol-production pathway(s).

SEQ ID NO: 8 presents example 8 for a designer Pyruvate Kinase DNA construct (2021 bp) that includes a PCR FD primer (sequence 1-20), a 84-bp nitrate reductase promoter (21-104), a 9-bp Xho I NdeI site (105-113) a 135-bp *RbcS2* transit peptide (114-248), a pyruvate kinase-encoding sequence (249-1748) selected/modified from a *Saccharomyces cerevisiae* Pyruvate Kinase sequence (GenBank: AY949876), a 21-bp Lumio-tag sequence (1749-1769), a 9-bp XbaI site (1770-1778), a 223-bp *RbcS2* terminator (1779-2001), and a PCR RE primer (2002-2021). This DNA construct is similar to example 6, SEQ ID NO: 6, except that a pyruvate kinase-encoding sequence (249-1748) is used.

SEQ ID NO: 9 presents example 9 for a designer Enolase gene (1815 bp) consisting of a PCR FD primer (sequence 1-20), a 262-bp nitrate reductase promoter (21-282), a 9-bp Xho I NdeI site (283-291) a 135-bp *RbcS2* transit peptide (292-426), a enolase-encoding sequence (427-1542) selected/modified from the sequences of a *Chlamydomonas reinhardtii* cytosolic enolase (Genbank: X66412, P31683), a 21-bp Lumio-tag-encoding sequence (1507-1527), a 9-bp XbaI site (1543-1551) containing a stop codon, a 223-bp *RbcS2* terminator (1552-1795), and a PCR RE primer (1796-1815) at the 3' end. This DNA construct is similar to example 3, SEQ ID NO: 3, except that an enolase-encoding sequence (427-1542) selected/modified from the sequences of a *Chlamydomonas reinhardtii* cytosolic enolase is used.

SEQ ID NO: 10 presents example 10 for a designer Phosphoglycerate-Mutase DNA construct (2349 bp) that includes a PCR FD primer (sequence 1-20), a 262-bp nitrate reductase promoter (21-282), a 9-bp Xho I NdeI site (283-291), a 135-bp *RbcS2* transit peptide (292-426), a phosphoglycerate-mutase encoding sequence (427-2097) selected/modified from the sequences of a *Chlamydomonas reinhardtii* cytosolic phosphoglycerate mutase (JGI Chlre2 protein ID 161689, Genbank: AF268078), a 9-bp XbaI site (2098-2106), a 223-bp *RbcS2* terminator (2107-2329), and a PCR RE primer (2330-2349) at the 3' end. This DNA construct is similar to example 3, SEQ ID NO: 3, except that a phosphoglycerate-mutase encoding sequence (427-2097) selected/modified from the sequences of a *Chlamydomonas reinhardtii* cytosolic phosphoglycerate mutase is used.

SEQ ID NO: 11 presents example 11 for a designer Phosphoglycerate Kinase DNA construct (1908 bp) that includes a PCR FD primer (sequence 1-20), a 262-bp nitrate reductase Nia1 promoter (21-282), a phosphoglycerate-kinase-encoding sequence (283-1665) selected from a *Chlamydomonas reinhardtii* chloroplast phosphoglycerate-kinase sequence including its chloroplast signal peptide and mature enzyme sequence (GenBank: U14912), a 223-bp *RbcS2* terminator (1666-1888), and a PCR RE primer (1889-1908). This DNA construct is similar to example 1, SEQ ID NO: 1, except a phosphoglycerate-kinase-encoding sequence (283-1665) selected from a *Chlamydomonas reinhardtii* chloroplast phosphoglycerate-kinase sequence including its chloroplast signal peptide and mature enzyme sequence is used. Therefore, this is also an example where the sequence of a nuclear-encoded chloroplast enzyme such as the *Chlamydomonas reinhardtii* chloroplast phosphoglycerate kinase can also be used in design and construction of a designer butanol-production pathway gene when appropriate with a proper inducible promoter such as the Nia1 promoter (DNA sequence 21-282).

SEQ ID NO: 12 presents example 12 for a designer Glyceraldehyde-3-Phosphate Dehydrogenase gene (1677 bp) that includes a PCR FD primer (sequence 1-20), a 262-bp nitrate reductase Nia1 promoter (21-282), a 135-bp *RbcS2* transit peptide (283-417), an enzyme-encoding sequence (418-1434) selected and modified from a *Mesostigma viride* cytosolic glyceraldehyde-3-phosphate dehydrogenase (mRNA) sequence (GenBank accession number DQ873404), a 223-bp *RbcS2* terminator (1435-1657), and a PCR RE primer (1658-1677). This DNA construct is similar to example 1, SEQ ID NO: 1, except that an enzyme-encoding sequence (418-1434) selected and modified from a *Mesostigma viride* cytosolic glyceraldehyde-3-phosphate dehydrogenase (mRNA) sequence (GenBank accession number DQ873404) is used.

SEQ ID NO: 13 presents example 13 for a designer HydA1-promoter-linked Phosphoglycerate Mutase DNA construct (2351 bp) that includes a PCR FD primer (sequence 1-20), a 282-bp HydA1 promoter (21-302), a 135-bp *RbcS2* transit peptide (303-437), a phosphoglycerate-mutase encoding sequence (438-2108) selected/modified from the sequences of a *Chlamydomonas reinhardtii* cytosolic phosphoglycerate mutase (JGI Chlre2 protein ID 161689, Genbank: AF268078), a 223-bp *RbcS2* terminator (2109-2331), and a PCR RE primer (2332-2351). This designer DNA construct is quite similar to example 1, SEQ ID NO:1, except that a 282-bp HydA1 promoter (21-302) and a phosphoglycerate-mutase encoding sequence (438-2108) selected/modified from the sequences of a *Chlamydomonas reinhardtii* cytosolic phosphoglycerate mutase are used. The 282-bp HydA1 promoter (21-302) has been proven active by experimental assays at the inventor's laboratory. Use of the HydA1 promoter (21-302) enables activation of designer enzyme expression by using anaerobic culture-medium conditions.

With the same principle of using an inducible anaerobic promoter and a chloroplast-targeting sequence as that shown in SEQ ID NO: 13 (example 13), SEQ ID NOS: 14-23 show designer-gene examples 14-23. Briefly, SEQ ID NO: 14 presents example 14 for a designer HydA1-promoter-linked Enolase DNA construct (1796 bp) that includes a PCR FD primer (sequence 1-20), a 282-bp HydA1 promoter (21-302), a 135-bp RbcS2 transit peptide (303-437), a Enolase-encoding sequence (438-1553) selected/modified from the sequences of a *Chlamydomonas reinhardtii* cytosolic enolase (Genbank: X66412, P31683), a 223-bp *RbcS2* terminator (1554-1776), and a PCR RE primer (1777-1796).

SEQ ID NO: 15 presents example 15 for a designer HydA1-promoter-controlled Pyruvate-Kinase DNA construct that includes a PCR FD primer (sequence 1-20), a 282-bp HydA1 promoter (21-302), a 135-bp RbcS2 transit peptide (303-437), a Pyruvate Kinase-encoding sequence (438-1589) selected/modified from a *Chlamydomonas reinhardtii* cytosolic pyruvate kinase sequence (JGI Chlre3 protein ID 138105), a 223-bp *RbcS2* terminator (1590-1812), and a PCR RE primer (1813-1832).

SEQ ID NO:16 presents example 16 for a designer HydA1-promoter-linked Pyruvate-ferredoxin oxidoreductase DNA construct (4376 bp) that includes a PCR FD primer (sequence 1-20), a 282-bp HydA1 promoter (21-302), a 135-bp *RbcS2* transit peptide (303-437), a Pyruvate-ferredoxin oxidoreductase-encoding sequence (438-4133) selected/modified from a *Desulfovibrio africanus* Pyruvate-ferredoxin oxidoreductase sequence (GenBank Accession Number Y09702), a 223-bp *RbcS2* terminator (4134-4356), and a PCR RE primer (4357-4376).

SEQ ID NO:17 presents example 17 for a designer HydA1-promoter-linked Pyruvate-NADP⁺ oxidoreductase DNA construct (6092 bp) that includes a PCR FD primer (sequence 1-20), a 282-bp HydA1 promoter (21-302), a 135-bp *RbcS2* transit peptide (303-437), a Pyruvate-NADP⁺ oxidoreductase-encoding sequence (438-5849) selected/modified from a *Euglena gracilis* Pyruvate-NADP⁺ oxidoreductase sequence (GenBank Accession Number AB021127), a 223-bp *RbcS2* terminator (5850-6072), and a PCR RE primer (6073-6092).

SEQ ID NO:18 presents example 18 for a designer HydA1-promoter-linked Thiolase DNA construct (1856 bp) that includes a PCR FD primer (sequence 1-20), a 282-bp HydA1 promoter (21-302), a 135-bp *RbcS2* transit peptide (303-437), a Thiolase-encoding sequence (438-1613) selected/modified from the sequences of a *Thermoanaerobacterium thermosaccharolyticum* Thiolase (GenBank Z92974), a 223-bp *RbcS2* terminator (1614-1836), and a PCR RE primer (1837-1856).

SEQ ID NO:19 presents example 19 for a designer HydA1-promoter-linked 3-Hydroxybutyryl-CoA dehydrogenase DNA construct (1550 bp) that includes a PCR FD primer (sequence 1-20), a 282-bp HydA1 promoter (21-302), a 135-bp *RbcS2* transit peptide (303-437), a 3-Hydroxybutyryl-CoA dehydrogenase-encoding sequence (438-1307) selected/modified from the sequences of a *Thermoanaerobacterium thermosaccharolyticum* 3-Hydroxybutyryl-CoA dehydrogenase (GenBank Z92974), a 223-bp *RbcS2* terminator (1308-1530), and a PCR RE primer (1531-1550).

SEQ ID NO:20 presents example 20 for a designer HydA1-promoter-linked Crotonase DNA construct (1457 bp) that includes a PCR FD primer (sequence 1-20), a 282-bp HydA1 promoter (21-302), a 135-bp *RbcS2* transit peptide (303-437), a Crotonase-encoding sequence (438-1214) selected/modified from the sequences of a *Thermoanaerobacterium thermosaccharolyticum* Crotonase (GenBank Z92974), a 223-bpRbcS2 terminator (1215-1437), and a PCR RE primer (1438-1457).

SEQ ID NO:21 presents example 21 for a designer HydA1-promoter-linked Butyryl-CoA dehydrogenase DNA construct (1817 bp) that includes a PCR FD primer (sequence 1-20), a 282-bp HydA1 promoter (21-302), a 135-bp *RbcS2* transit peptide (303-437), a Butyryl-CoA dehydrogenase-encoding sequence (438-1574) selected/modified from the sequences of a *Thermoanaerobacterium thermosaccharolyticum* Butyryl-CoA dehydrogenase (GenBank Z92974), a 223-bp *RbcS2* terminator (1575-1797), and a PCR RE primer (1798-1817).

SEQ ID NO: 22 presents example 22 for a designer HydA1-promoter-linked Butyraldehyde dehydrogenase DNA construct (2084 bp) that includes a PCR FD primer (sequence 1-20), a 282-bp HydA1 promoter (21-302), a 135-bp *RbcS2* transit peptide (303-437), a Butyraldehyde dehydrogenase-encoding sequence (438-1841) selected/modified from the sequences of a *Clostridium saccharoperbutylacetonicum* Butyraldehyde dehydrogenase (GenBank AY251646), a 223-bp *RbcS2* terminator (1842-2064), and a PCR RE primer (2065-2084).

SEQ ID NO: 23 presents example 23 for a designer HydA1-promoter-linked Butanol dehydrogenase DNA construct (1733 bp) that includes a PCR FD primer (sequence 1-20), a 282-bp HydA1 promoter (21-302), a 135-bp RbcS2 transit peptide (303-437), a Butanol dehydrogenase-encoding sequence (438-1490) selected/modified from the sequences of a *Clostridium beijerinckii* Butanol dehydrogenase (GenBank AF157307), a 223-bp *RbcS2* terminator (1491-1713), and a PCR RE primer (1714-1733).

With the same principle of using a 2x84 synthetic Nia1 promoter and a chloroplast-targeting mechanism as mentioned previously, SEQ ID NOS:24-26 show more examples of designer-enzyme DNA-constructs. Briefly, SEQ ID NO: 24 presents example 24 for a designer Fructose-Diphosphate-Aldolase DNA construct that includes a PCR FD primer (sequence 1-20), a 2 x 84-bp NR promoter (21-188), a Fructose-Diphosphate Aldolase-encoding sequence (189-1313) selected/modified from a C. *reinhardtii* chloroplast fructose-1,6-bisphosphate aldolase sequence (GenBank: X69969), a 223-bpRbcS2 terminator (1314-1536), and a PCR RE primer (1537-1556).

SEQ ID NO: 25 presents example 24 for a designer Triose-Phosphate-Isomerase DNA construct that includes a PCR FD primer (sequence 1-20), a 2 x 84-bp NR promoter (21-188), a Triose-Phosphate Isomerase-encoding sequence (189-1136) selected and modified from a *Arabidopsis thaliana* chloroplast triosephosphate-isomerase sequence (GenBank: AF247559), a 223-bp *RbcS2* terminator (1137-1359), and a PCR RE primer (1360-1379).

SEQ ID NO: 26 presents example 26 for a designer Phosphofructose-Kinase DNA construct that includes a PCR FD primer (sequence 1-20), a 2 x 84-bp NR promoter (21-188), a 135-bp *RbcS2* transit peptide (189-323), a Phosphofructose Kinase-encoding sequence (324-1913) selected/modified from *Arabidopsis thaliana* 6-phosphofructokinase sequence (GenBank: NM_001037043), a 223-bp *RbcS2* terminator (1914-2136), and a PCR RE primer (2137-2156).

The nucleic acid constructs, such as those presented in the examples above, may include additional appropriate sequences, for example, a selection marker gene, and an optional biomolecular tag sequence (such as the Lumio tag described in example 4, SEQ ID NO: 4). Selectable markers that can be selected for use in the constructs include markers conferring resistances to kanamycin, hygromycin, spectinomycin, streptomycin, sulfonyl urea, gentamycin, chloramphenicol, among others, all of which have been cloned and are available to those skilled in the art. Alternatively, the selective marker is a nutrition marker gene that can complement a deficiency in the host organism. For example, the gene encoding argininosuccinate lyase (arg7) can be used as a selection marker gene in the designer construct, which permits identification of transformants when *Chlamydomonas reinhardtii* arg7- (minus) cells are used as host cells.

Nucleic acid constructs carrying designer genes can be delivered into a host alga, blue-green alga, plant, or plant tissue or cells using the available gene-transformation techniques, such as electroporation, PEG induced uptake, and ballistic delivery of DNA, and Agrobacterium-mediated transformation. For the purpose of delivering a designer construct into algal cells, the techniques of electroporation, glass bead, and biolistic genegun can be selected for use as preferred methods; and an alga with single cells or simple thallus structure is preferred for use in transformation. Transformants can be identified and tested based on routine techniques.

The various designer genes can be introduced into host cells sequentially in a step-wise manner, or simultaneously using one construct or in one transformation. For example, the ten DNA constructs shown in SEQ ID NO: 13-16 (or 17) and 18-23 for the ten-enzyme 3-phosphoglycerate-branched butanol-production pathway can be placed into a genetic vector such as p389-Arg7 with a single selection marker (Arg7). Therefore, by use of a plasmid in this manner, it is possible to deliver all the ten DNA constructs (designer genes) into an arginine-requiring *Chlamydomonas reinhardtii*-arg7 host (CC-48) in one transformation for expression of the 3-phosphoglycerate-branched butanol-production pathway (**03-12** in Figure 1). When necessary, a transformant containing the ten DNA constructs can be further transformed to get more designer genes into its genomic DNA with an additional selection marker such as streptomycin. By using combinations of various designer-enzymes DNA constructs such as those presented in SEQ ID NO: 1-26 in genetic transformation with an appropriate host organism, various butanol-production pathways such as those illustrated in Figure 1 can be constructed. For example, the designer DNA constructs of SEQ ID NO: 1-12 can be selected for construction of the glyceraldehydes-3-phosphate-branched butanol-production pathway (01-12 in Figure 1); The designer DNA constructs of SEQ ID NO: 1-12, 24, and 25 can be selected for construction of the fructose-1,6-diphosphate-branched butanol-production pathway (**20-33**); and the designer DNA constructs of SEQ ID NO: 1-12 and 24-26 can be selected for construction of the fructose-6-phosphate-branched butanol-production pathway (**19-33**).

### Additional Host Modifications to Enhance Photosynthetic Butanol Production

### An NADPH/NADH conversion mechanism

According to the photosynthetic butanol production pathway(s), to produce one molecule of butanol from 4CO₂ and 5H₂O is likely to require 14 ATP and 12 NADPH, both of which are generated by photosynthetic water splitting and photophosphorylation across the thylakoid membrane. In order for the 3-phosphoglycerate-branched butanol-production pathway (**03-12** in Figure 1) to operate, it is a preferred practice to use a butanol-production-pathway enzyme(s) that can use NADPH that is generated by the photo-driven electron transport process. *Clostridium saccharoperbutylacetonicum* butanol dehydrogenase (GenBank accession number: AB257439) and butyaldehyde dehydrogenase (GenBank: AY251646) are examples of a butanol-production-pathway enzyme that is capable of accepting either NADP(H) or NAD(H). Such a butanol-production-pathway enzyme that can use both NADPH and NADH (i.e., NAD(P)H) can also be selected for use in this 3-phosphoglycerate-branched and any of the other designer butanol-production pathway(s) (Figures 1) as well. *Clostridium beijerinckii* Butyryl-CoA dehydrogenase (GenBank: AF494018) and 3-Hydroxybutyryl-CoA dehydrogenase (GenBank: AF494018) are examples of a butanol-production-pathway enzyme that can accept only NAD(H). When a butanol-production-pathway enzyme that can only use NADH is employed, it may require an NADPH/NADH conversion mechanism in order for this 3-phosphoglycerate-branched butanol-production pathway to operate well. However, depending on the genetic backgrounds of a host organism, a conversion mechanism between NADPH and NADH may exist in the host so that NADPH and NADH may be interchangeably used in the organism. In addition, it is known that NADPH could be converted into NADH by a NADPH-phosphatase activity (Pattanayak and Chatterjee (1998) "Nicotinamide adenine dinucleotide phosphate phosphatase facilitates dark reduction of nitrate: regulation by nitrate and ammonia," Biologia Plantarium 41(1):75-84) and that NAD can be converted to NADP by a NAD kinase activity (Muto, Miyachi, Usuda, Edwards and Bassham (1981) "Light-induced conversion of nicotinamide adenine dinucleotide to nicotinamide adenine dinucleotide phosphate in higher plant leaves," Plant Physiology 68(2):324-328; Matsumura-Kadota, Muto, Miyachi (1982) "Light-induced conversion of NAD+ to NADP+ in Chlorella cells," Biochimica Biophysica Acta 679(2):300-300). Therefore, when enhanced NADPH/NADH conversion is desirable, the host may be genetically modified to enhance the NADPH phosphatase and NAD kinase activities. Thus, in one of the various embodiments, the photosynthetic butanol-producing designer plant, designer alga or plant cell further contains additional designer transgenes (Figure 2B) to inducibly express one or more enzymes to facilitate the NADPH/NADH inter-conversion, such as the NADPH phosphatase and NAD kinase (GenBank: XM_001609395, XM_001324239), in the stroma of algal chloroplast.

Another embodiment that can provide an NADPH/NADH conversion mechanism is by properly selecting an appropriate branching point at the Calvin cycle for a designer butanol-production pathway to branch from. To confer this NADPH/NADH conversion mechanism by pathway design according to this embodiment, it is a preferred practice to branch a designer butanol-production pathway at or after the point of glyceraldehydes-3-phosphate of the Calvin cycle as shown in Figures 1. In these pathway designs, the NADPH/NADH conversion is achieved essentially by a two-step mechanism: 1) Use of the step with the Calvin-cycle's glyceraldehyde-3-phosphate dehydrogenase, which uses NADPH in reducing1,3-diphosphoglycerate to glyceraldehydes-3-phosphate; and 2) use of the step with the designer pathway's NAD⁺-dependent glyceraldehyde-3-phosphate dehydrogenase **01,** which produces NADH in oxidizing glyceraldehyde-3-phosphate to1,3-diphosphoglycerate. The net result of the two steps described above is the conversion of NADPH to NADH, which can supply the needed reducing power in the form of NADH for the designer butanol-production pathway(s). For step 1), use of the Calvin-cycle's NADPH-dependent glyceraldehyde-3-phosphate dehydrogenase naturally in the host organism is usually sufficient. Consequently, introduction of a designer NAD⁺-dependent glyceraldehyde-3-phosphate dehydrogenase **01** to work with the Calvin-cycle's NADPH-dependent glyceraldehyde-3-phosphate dehydrogenase may confer the function of an NADPH/NADH conversion mechanism, which is needed for the 3-phosphoglycerate-branched butanol-production pathway (**03-12** in Figure 1) to operate well. For this reason, the designer NAD⁺-dependent glyceraldehyde-3-phosphate-dehydrogenase DNA construct (example 12, SEQ ID NO: 12) is used also as an NADPH/NADH-conversion designer gene (Figure 2B) to support the 3-phosphoglycerate-branched butanol-production pathway (**03-12** in Figure 1) in one of the various embodiments. This also explains why it is important to use a NAD⁺-dependent glyceraldehyde-3-phosphate dehydrogenase **01** to confer this two-step NADPH/NADH conversion mechanism for the designer butanol-production pathway(s). Therefore, in one of the various embodiments, it is also a preferred practice to use a NAD⁺-dependent glyceraldehyde-3-phosphate dehydrogenase, its isozymes, functional derivatives, analogs, designer modified enzymes and/or combinations thereof in the designer butanol-production pathway(s) as illustrated in Figure 1.

### iRNA techniques to further tame photosynthesis regulation mechanism

In another embodiment of the present invention, the host plant or cell is further modified to tame the Calvin cycle so that the host can directly produce liquid fuel butanol instead of synthesizing starch (glycogen in the case of oxyphotobacteria), celluloses and lignocelluloses that are often inefficient and hard for the biorefinery industry to use. According to the one of the various embodiments, inactivation of starch-synthesis activity is achieved by suppressing the expression of any of the key enzymes, such as, starch synthase (glycogen synthase in the case of oxyphotobacteria) **13,** glucose-1-phosphate (G-1-P) adenylyltransferase **14,** phosphoglucomutase **15,** and hexose-phosphate-isomerase **16** of the starch-synthesis pathway which connects with the Calvin cycle (Figure 1).

Introduction of a genetically transmittable factor that can inhibit the starch-synthesis activity that is in competition with designer butanol-production pathway(s) for the Calvin-cycle products can further enhance photosynthetic butanol production. In a specific embodiment, a genetically encoded-able inhibitor (Figure 2C) to the competitive starch-synthesis pathway is an interfering RNA (iRNA) molecule that specifically inhibits the synthesis of a starch-synthesis-pathway enzyme, for example, starch synthase **16,** glucose-1-phosphate (G-1-P) adenylyltransferase **15,** phosphoglucomutase **14,** and/or hexose-phosphate-isomerase **13** as shown with numerical labels **13-16** in Figure 1. The DNA sequences encoding starch synthase iRNA, glucose-1-phosphate (G-1-P) adenylyltransferase iRNA, a phosphoglucomutase iRNA and/or a G-P-isomerase iRNA, respectively, can be designed and synthesized based on RNA interference techniques known to those skilled in the art (Liszewski (June 1, 2003) Progress in RNA interference, Genetic Engineering News, Vol. 23, number 11, pp. 1-59). Generally speaking, an interfering RNA (iRNA) molecule is anti-sense but complementary to a normal mRNA of a particular protein (gene) so that such iRNA molecule can specifically bind with the normal mRNA of the particular gene, thus inhibiting (blocking) the translation of the gene-specific mRNA to protein (Fire, Xu, Montgomery, Kostas, Driver, Mello (1998) "Potent and specific genetic interference by double-stranded RNA in Caenorhabditis elegans". Nature 391(6669):806-11; Dykxhoorn, Novina, Sharp (2003) "Killing the messenger: short RNAs that silence gene expression", Nat Rev Mol Cell Biol. 4(6):457-67).

Examples of a designer starch-synthesis iRNA DNA construct (Figure 2C) are shown in SEQ ID NO: 27 and 28 listed. Briefly, SEQ ID NO: 27 presents example 27 for a designer Nia1-promoter-controlled Starch-Synthase-iRNA DNA construct (860 bp) that includes a PCR FD primer (sequence 1-20), a 262-bp Nia1 promoter (21-282), a Starch-Synthase iRNA sequence (283 - 617) consisting of start codon atg and a reverse complement sequence of two unique sequence fragments of a *Chlamydomonas reinhardtii* starch-synthase-mRNA sequence (GenBank: AF026422), a 223-bp *RbcS2* terminator (618-850), and a PCR RE primer (851-860). Because of the use of a Nia1 promoter (21-282), this designer starch-synthesis iRNA gene is designed to be expressed only when needed to enhance photobiological butanol production in the presence of its specific inducer, nitrate (NO₃⁻), which can be added into the culture medium as a fertilizer for induction of the designer organisms. The Starch-Synthase iRNA sequence (283 - 617) is designed to bind with the normal mRNA of the starch synthase gene, thus blocking its translation into a functional starch synthase. The inhibition of the starch/glycogen synthase activity at **16** in this manner is to channel more photosynthetic products of the Calvin cycle into the Calvin-cycle-branched butanol-production pathway(s) such as the glyceraldehydes-3-phosphate-branched butanol-production pathway **01-12** as illustrated in Figure 1.

SEQ ID NO: 28 presents example 28 for a designer HydA1-promoter-controlled Starch-Synthase-iRNA DNA construct (1328 bp) that includes a PCR FD primer (sequence 1-20), a 282-bp HydA1 promoter (21-302), a designer Starch-Synthase iRNA sequence (303 - 1085), a 223-bp *RbcS2* terminator (1086-1308), and a PCR RE primer (1309-1328). The designer Starch-Synthase-iRNA sequence (303-1085) comprises of: a 300-bp sense fragment (303-602) selected from the first 300-bp unique coding sequence of a *Chlamydomonas reinhardtii* starch synthase mRNA sequence (GenBank: AF026422), a 183-bp designer intron-like loop (603-785), and a 300-bp antisense sequence (786-1085) complement to the first 300-bp coding sequence of a *Chlamydomonas reinhardtii* starch-synthase-mRNA sequence (GenBank: AF026422). This designer Starch-Synthase-iRNA sequence (303-1085) is designed to inhibit the synthesis of starch synthase by the following two mechanisms. First, the 300-bp antisense complement iRNA sequence (corresponding to DNA sequence 786-1085) binds with the normal mRNA of the starch synthase gene, thus blocking its translation into a functional starch synthase. Second, the 300-bp antisense complement iRNA sequence (corresponding to DNA sequence 786-1085) can also bind with the 300-bp sense counterpart (corresponding to DNA sequence 303-602) in the same designer iRNA molecule, forming a hairpin-like double-stranded RNA structure with the 183-bp designer intron-like sequence (603-785) as a loop. Experimental studies have shown that this type of hairpin-like double-stranded RNA can also trigger post-transcriptional gene silencing (Fuhrmann, Stahlberg, Govorunova, Rank and Hegemann (2001) Journal of Cell Science 114:3857-3863). Because of the use of a HydA1 promoter (21-302), this designer starch-synthesis-iRNA gene is designed to be expressed only under anaerobic conditions when needed to enhance photobiological butanol production by channeling more photosynthetic products of the Calvin cycle into the butanol-production pathway(s) such as **01-12, 03-12,** and/or **20-33** as illustrated in Figure 1.

### Designer starch-degradation and glycolysis genes

In yet another embodiment of the present invention, the photobiological butanol production is enhanced by incorporating an additional set of designer genes (Figure 2D) that can facilitate starch/glycogen degradation and glycolysis in combination with the designer butanol-production gene(s) (Figure 2A). Such additional designer genes for starch degradation include, for example, genes coding for 17: amylase, starch phosphorylase, hexokinase, phosphoglucomutase, and for **18:** glucose-phosphate-isomerase (G-P-isomerase) as illustrated in Figure 1. The designer glycolysis genes encode chloroplast-targeted glycolysis enzymes: glucosephosphate isomerase **18,** phosphofructose kinase **19,** aldolase **20,** triose phosphate isomerase **21,** glyceraldehyde-3-phosphate dehydrogenase **22,** phosphoglycerate kinase **23,** phosphoglycerate mutase **24,** enolase **25,** and pyruvate kinase **26.** The designer starch-degradation and glycolysis genes in combination with any of the butanol-production pathways shown in Figure 1 can form additional pathway(s) from starch/glycogen to butanol (**17-33**). Consequently, co-expression of the designer starch-degradation and glycolysis genes with the butanol-production-pathway genes can enhance photobiological production of butanol as well. Therefore, this embodiment represents another approach to tame the Calvin cycle for enhanced photobiological production of butanol. In this case, some of the Calvin-cycle products flow through the starch synthesis pathway **(13-16)** followed by the starch/glycogen-to-butanol pathway **(17-33)** as shown in Figure 1. In this case, starch/glycogen acts as a transient storage pool of the Calvin-cycle products before they can be converted to butanol. This mechanism can be quite useful in maximizing the butanol-production yield in certain cases. For example, at high sunlight intensity such as around noon, the rate of Calvin-cycle photosynthetic CO₂ fixation can be so high that may exceed the maximal rate capacity of a butanol-production pathway(s); use of the starch-synthesis mechanism allows temporary storage of the excess photosynthetic products to be used later for butanol production as well.

Figure 1 also illustrates the use of a designer starch/glycogen-to-butanol pathway with designer enzymes (as labeled from **17** to **33)** in combination with a Calvin-cycle-branched designer butanol-production pathway(s) such as the glyceraldehydes-3-phosphate-branched butanol-production pathway **01-12** for enhanced photobiological butanol production. Similar to the benefits of using the Calvin-cycle-branched designer butanol-production pathways, the use of the designer starch/glycogen-to-butanol pathway **(17-33)** can also help to convert the photosynthetic products to butanol before the sugars could be converted into other complicated biomolecules such as lignocellulosic biomasses which cannot be readily used by the biorefinery industries. Therefore, appropriate use of the Calvin-cycle-branched designer butanol-production pathway(s) (such as **01-12, 03-12,** and/or **20-33)** and/or the designer starch/glycogen-to-butanol pathway **(17-33)** may represent revolutionary *inter alia* technologies that can effectively bypass the bottleneck problems of the current biomass technology including the "lignocellulosic recalcitrance" problem.

Another feature is that a Calvin-cycle-branched designer butanol-production pathway activity (such as **01-12, 03-12,** and/or **20-33)** can occur predominantly during the days when there is light because it uses an intermediate product of the Calvin cycle which requires supplies of reducing power (NADPH) and energy (ATP) generated by the photosynthetic water splitting and the light-driven proton-translocation-coupled electron transport process through the thylakoid membrane system. The designer starch/glycogen-to-butanol pathway **(17-33)** which can use the surplus sugar that has been stored as starch/glycogen during photosynthesis can operate not only during the days, but also at nights. Consequently, the use of a Calvin-cycle-branched designer butanol-production pathway (such as **01-12, 03-12,** and/or **20-33)** together with a designer starch/glycogen-to-butanol pathway(s) **(17-33)** as illustrated in Figure 1 enables production of butanol both during the days and at nights.

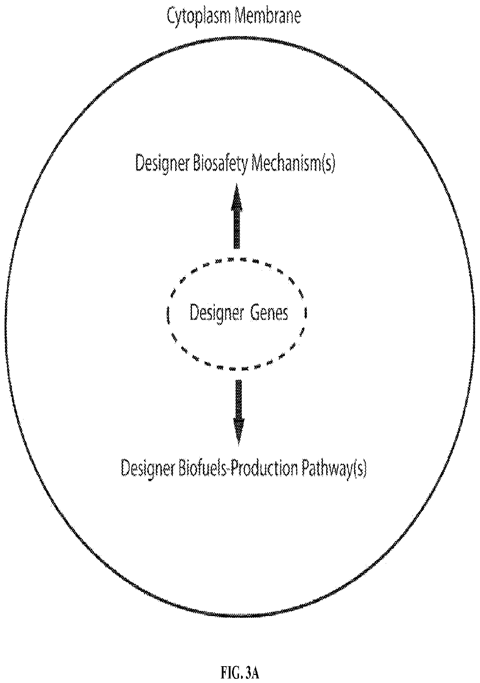

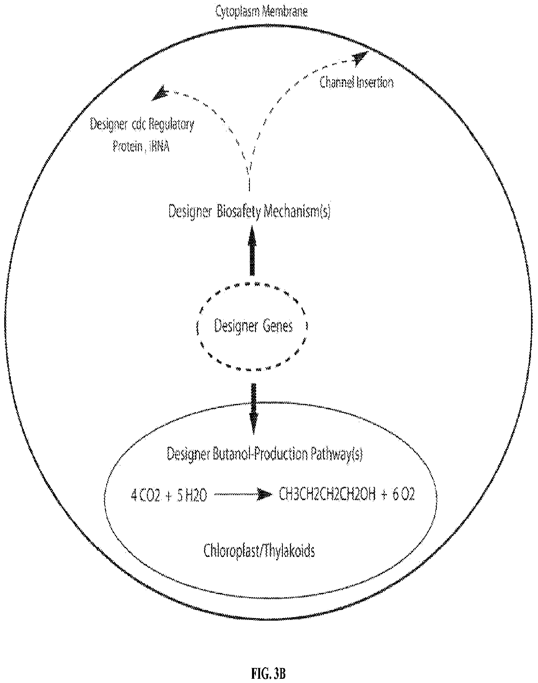

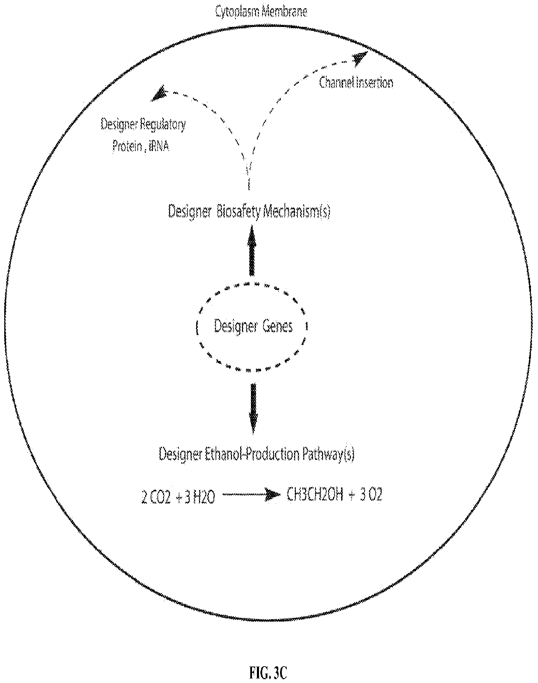

Because the expression for both the designer starch/glycogen-to-butanol pathway(s) and the Calvin-cycle-branched designer butanol-production pathway(s) is controlled by the use of an inducible promoter such as an anaerobic hydrogenase promoter, this type of designer organisms is also able to grow photoautotrophically under aerobic (normal) conditions. When the designer photosynthetic organisms are grown and ready for photobiological butanol production, the cells are then placed under the specific inducing conditions such as under anaerobic conditions [or an ammonium-to-nitrate fertilizer use shift, if designer Nia1/nirA promoter-controlled butanol-production pathway(s) is used] for enhanced butanol production, as shown in Figures 1 and 3.

Examples of designer starch (glycogen)-degradation genes are shown in SEQ ID NO: 29-33 listed. Briefly, SEQ ID NO:29 presents example 29 for a designer Amylase DNA construct (1889 bp) that includes a PCR FD primer (sequence 1-20), a 2 x 84-bp NR promoter (21-188), a 9-bp Xho I NdeI site (189-197), a 135-bp *RbcS2* transit peptide (198-332), an Amylase-encoding sequence (333-1616) selected and modified from a *Barley alpha-amylase* (GenBank: J04202A my46 expression tested in aleurone cells), a 21-bp Lumio-tag sequence (1617-1637), a 9-bp XbaI site (1638-1646), a 223-bp *RbcS2* terminator (1647-1869), and a PCR RE primer (1870-1889).

SEQ ID NO: 30 presents example 30 for a designer Starch-Phosphorylase DNA construct (3089 bp) that includes a PCR FD primer (sequence 1-20), a 2 x 84-bp NR promoter (21-188), a 135-bp *RbcS2* transit peptide (189-323), a Starch Phosphorylase-encoding sequence (324-2846) selected and modified from a *Citrus* root starch-phosphorylase sequence (GenBank: AY098895, expression tested in *citrus* root), a 223-bp *RbcS2* terminator (2847-3069), and a PCR RE primer (3070-3089).

SEQ ID NO: 31 presents example 31 for a designer Hexose-Kinase DNA construct (1949 bp) that includes a PCR FD primer (sequence 1-20), a 2 x 84-bp NR promoter (21-188), a 135-bp RbcS2 transit peptide (189-323), a Hexose Kinase-encoding sequence (324-1706) selected and modified from *Ajellomyces capsulatus* hexokinase mRNA sequence (Genbank: XM_001541513), a 223-bp *RbcS2* terminator (1707-1929), and a PCR RE primer (1930-1949).

SEQ ID NO: 32 presents example 32 for a designer Phosphoglucomutase DNA construct (2249 bp) that includes a PCR FD primer (sequence 1-20), a 2 x 84-bp NR promoter (21-188), a 135-bp *RbcS2* transit peptide (189-323), a Phosphoglucomutase-encoding sequence (324-2006) selected and modified from *Pichia stipitis* phosphoglucomutase sequence (GenBank: XM_001383281), a 223-bp *RbcS2* terminator (2007-2229), and a PCR RE primer (2230-2249).

SEQ ID NO: 33 presents example 33 for a designer Glucosephosphate-Isomerase DNA construct (2231 bp) that includes a PCR FD primer (sequence 1-20), a 2 x 84-bp NR promoter (21-188), a 135-bp *RbcS2* transit peptide (189-323), a Glucosephosphate Isomerase-encoding sequence (324-1988) selected and modified from a *S. cerevisiae* phosphoglucoisomerase sequence (GenBank: M21696), a 223-bp *RbcS2* terminator (1989-2211), and a PCR RE primer (2212-2231).

The designer starch-degradation genes such as those shown in SEQ ID NO: 29-33 can be selected for use in combination with various designer butanol-production-pathway genes for construction of various designer starch-degradation butanol-production pathways such as the pathways shown in Figure 1. For example, the designer genes shown in SEQ ID NOS: 1-12, 24-26, and 29-33 can be selected for construction of a Nia1 promoter-controlled starch-to-butanol production pathway that comprises of the following designer enzymes: amylase, starch phosphorylase, hexokinase, phosphoglucomutase, glucosephosphate isomerase, phosphofructose kinase, fructose diphosphate aldolase, triose phosphate isomerase, glyceraldehyde-3-phosphate dehydrogenase, phosphoglycerate kinase, phosphoglycerate mutase, enolase, pyruvate kinase, pyruvate-NADP⁺ oxidoreductase (or pyruvate-ferredoxin oxidoreductase), thiolase, 3-hydroxybutyryl-CoA dehydrogenase, crotonase, butyryl-CoA dehydrogenase, butyraldehyde dehydrogenase, and butanol dehydrogenase. This starch/glycogen-to-butanol pathway **17-33** may be used alone and/or in combinations with other butanol-production pathway(s) such as the 3-phosphoglycerate-branched butanol-production pathway **03-12** as illustrated in Figure 1.

### Distribution of designer butanol-production pathways between chloroplast and cytoplasm

In yet another embodiment of the present invention, photobiological butanol productivity is enhanced by a selected distribution of the designer butanol-production pathway(s) between chloroplast and cytoplasm in a eukaryotic plant cell. That is, not all the designer butanol-production pathway(s) (Figure 1) have to operate in the chloroplast; when needed, part of the designer butanol-production pathway(s) can operate in cytoplasm as well. For example, in one of the various embodiments, a significant part of the designer starch-to-butanol pathway activity from dihydroxyacetone phosphate to butanol **(21-33)** is designed to occur at the cytoplasm while the steps from starch to dihydroxyacetone phosphate **(17-20)** are in the chloroplast. In this example, the linkage between the chloroplast and cytoplasm parts of the designer pathway is accomplished by use of the triose phosphate-phosphate translocator, which facilitates translocation of dihydroxyacetone across the chloroplast membrane. By use of the triose phosphate-phosphate translocator, it also enables the glyceraldehyde-3-phospahte-branched designer butanol-production pathway to operate not only in chloroplast, but also in cytoplasm as well. The cytoplasm part of the designer butanol-production pathway can be constructed by use of designer butanol-production pathway genes (DNA constructs of Figure 2A) with their chloroplast-targeting sequence omitted as shown in Figure 2E.

### Designer oxyphotobacteria with designer butanol-production pathways in cytoplasm

In prokaryotic photosynthetic organisms such as blue-green algae (oxyphotobacteria including cyanobacteria and oxychlorobacteria), which typically contain photosynthetic thylakoid membrane but no chloroplast structure, the Calvin cycle is located in the cytoplasm. In this special case, the entire designer butanol-production pathway(s) (Figure 1) including (but not limited to) the glyceraldehyde-3-phosphate branched butanol-production pathway **(01-12),** the 3-phosphpglycerate-branched butanol-production pathway **(03-12),** the fructose-1,6-diphosphate-branched pathway **(20-33),** the fructose-6-phosphate-branched pathway **(19-33),** and the starch (or glycogen)-to-butanol pathways **(17-33)** are adjusted in design to operate with the Calvin cycle in the cytoplasm of a blue-green alga. The construction of the cytoplasm designer butanol-production pathways can be accomplished by use of designer butanol-production pathway genes (DNA construct of Figure 2A) with their chloroplast-targeting sequence all omitted. When the chloroplast-targeting sequence is omitted in the designer DNA construct(s) as illustrated in Figure 2E, the designer gene(s) is transcribed and translated into designer enzymes in the cytoplasm whereby conferring the designer butanol-production pathway(s). The designer gene(s) can be incorporated into the chromosomal and/or plasmid DNA in host blue-green algae (oxyphotobacteria including cyanobacteria and oxychlorobacteria) by using the techniques of gene transformation known to those skilled in the art. It is a preferred practice to integrate the designer genes through an integrative transformation into the chromosomal DNA that can usually provide better genetic stability for the designer genes. In oxyphotobacteria such as cyanobacteria, integrative transformation can be achieved through a process of homologous DNA double recombination into the host's chromosomal DNA using a designer DNA construct as illustrated in Fig. 2F, which typically, from the 5' upstream to the 3' downstream, consists of: recombination site 1, a designer butanol-production-pathway gene(s), and recombination site 2. This type of DNA constructs (Fig. 2F) can be delivered into oxyphotobacteria (blue-green algae) with a number of available genetic transformation techniques including electroporation, natural transformation, and/or conjugation. The transgenic designer organisms created from blue-green algae are also called designer blue-green algae (designer oxyphotobacteria including designer cyanobacteria and designer oxychlorobacteria).

Examples of designer oxyphotobacterial butanol-production-pathway genes are shown in SEQ ID NO: 34-45 listed. Briefly, SEQ ID NO:34 presents example 34 for a designer oxyphotobacterial Butanol Dehydrogenase DNA construct (1709 bp) that includes a PCR FD primer (sequence 1-20), a 400-bp nitrite reductase (nirA) promoter from *Thermosynechococcus elongatus* BP-1 (21-420), an enzyme-encoding sequence (421-1569) selected and modified from a *Clostridium saccharoperbutylacetonicum* Butanol Dehydrogenase sequence (AB257439), a 120-bp *rbcS* terminator from *Thermosynechococcus elongatus* BP-1 (1570-1689), and a PCR RE primer (1690-1709) at the 3' end.

SEQ ID NO:35 presents example 35 for a designer oxyphotobacterial Butyraldehyde Dehydrogenase DNA construct (1967 bp) that includes a PCR FD primer (sequence 1-20), a 400-bp *Thermosynechococcus elongatus* BP-1 nitrite reductase nirA promoter (21-420), an enzyme-encoding sequence (421-1827) selected and modified from a *Clostridium saccharoperbutylacetonicum* Butyraldehyde Dehydrogenase sequence (AY251646), a 120-bp *rbcS* terminator from *Thermosynechococcus* (1828-1947), and a PCR RE primer (1948-1967).

SEQ ID NO:36 presents example 36 for a designer oxyphotobacterial Butyryl-CoA Dehydrogenase DNA construct (1602 bp) that includes a PCR FD primer (sequence 1-20), a 305-bp *Thermosynechococcus elongatus* BP-1 nitrate reductase promoter (21-325), a Butyryl-CoA Dehydrogenase encoding sequence (326-1422) selected/modified from the sequences of a *Clostridium beijerinckii* Butyryl-CoA Dehydrogenase (AF494018), a 120-bp *Thermosynechococcus rbcS* terminator (1423-1582), and a PCR RE primer (1583-1602).

SEQ ID NO:37 presents example 37 for a designer oxyphotobacterial Crotonase DNA construct (1248 bp) that includes a PCR FD primer (sequence 1-20), a 305-bp *Thermosynechococcus elongatus* BP-1 nitrate reductase promoter (21-325), a Crotonase-encoding sequence (326-1108) selected/modified from the sequences of a *Clostridium beijerinckii* Crotonase (GenBank: AF494018), 120-bp *Thermosynechococcus elongatus* BP-1 *rbcS* terminator (1109-1228), and a PCR RE primer (1229-1248).

SEQ ID NO:38 presents example 38 for a designer oxyphotobacterial 3-Hydroxybutyryl-CoA Dehydrogenase DNA construct (1311 bp) that include of a PCR FD primer (sequence 1-20), a 305-bp nirA promoter from (21-325), a 3-Hydroxybutyryl-CoA Dehydrogenase-encoding sequence (326-1171) selected/modified from a *Clostridium beijerinckii* 3-Hydroxybutyryl-CoA Dehydrogenase sequence (GenBank: AF494018), a 120-bp *Thermosynechococcus rbcS* terminator (1172-1291), and a PCR RE primer (1292-1311).

SEQ ID NO:39 presents example 39 for a designer oxyphotobacterial Thiolase DNA construct (1665 bp) that includes a PCR FD primer (sequence 1-20), a 305-bp *Thermosynechococcus* nirA promoter (21-325), a Thiolase-encoding sequence (326-1525) selected from a *Butyrivibriofibrisolvens* Thiolase sequence (AB190764), a 120-bp *Thermosynechococcus rbcS* terminator (1526-1645), and a PCR RE primer (1646-1665).

SEQ ID NO:40 presents example 40 for a designer oxyphotobacterial Pyruvate-Ferredoxin Oxidoreductase DNA construct (4071 bp) that includes a PCR FD primer (sequence 1-20), a 305-bp nirA promoter from *Thermosynechococcus elongatus* BP-1 (21-325), a Pyruvate-Ferredoxin Oxidoreductase-encoding sequence (326-3931) selected/modified from the sequences of a *Mastigamoeba balamuthi* Pyruvate-ferredoxin oxidoreductase (GenBank: AY101767), a 120-bp *rbcS* terminator from *Thermosynechococcus elongatus* BP-1 (3932-4051), and a PCR RE primer (4052-4071).

SEQ ID NO:41 presents example 41 for a designer oxyphotobacterial Pyruvate Kinase DNA construct (1806 bp) that includes a PCR FD primer (sequence 1-20), a 305-bp nirA promoter from *Thermosynechococcus* (21-325), a pyruvate kinase-encoding sequence (326-1666) selected/modified from a *Thermoproteus tenax* pyruvate kinase (GenBank: AF065890), a 120-bp *Thermosynechococcus rbcS* terminator (1667-1786), and a PCR RE primer (1787-1806).

SEQ ID NO:42 presents example 42 for a designer oxyphotobacterial Enolase DNA construct (1696 bp) that includes a PCR FD primer (sequence 1-20), a 231-bp nirA promoter from *Thermosynechococcus* (21-251), a enolase-encoding sequence (252-1556) selected/modified from the sequences of a *Chlamydomonas* cytosolic enolase (GenBank: X66412, P31683), a 120-bp *rbcS* terminator from *Thermosynechococcus* (1557-1676), and a PCR RE primer (1677-1696).

SEQ ID NO:43 presents example 43 for a designer oxyphotobacterial Phosphoglycerate-Mutase DNA construct (2029 bp) that includes a PCR FD primer (sequence 1-20), a 231-bp nirA promoter from *Thermosynechococcus elongatus* BP-1 (21-251), a phosphoglycerate-mutase encoding sequence (252-1889) selected/modified from the sequences of a *Pelotomaculum thermopropionicum SI* phosphoglycerate mutase (GenBank: YP_001213270), a 120-bp *Thermosynechococcus rbcS* terminator (1890-2009), and a PCR RE primer (2010-2029).

SEQ ID NO:44 presents example 44 for a designer oxyphotobacterial Phosphoglycerate Kinase DNA construct (1687 bp) that includes a PCR FD primer (sequence 1-20), a 231-bp nirA promoter from *Thermosynechococcus elongatus* BP-1 (21-251), a phosphoglycerate-kinase-encoding sequence (252-1433) selected from *Pelotomaculum thermopropionicum SI* phosphoglycerate kinase (BAF60903), a 234-bp *Thermosynechococcus elongatus* BP-1 *rbcS* terminator (1434-1667), and a PCR RE primer (1668-1687).

SEQ ID NO:45 presents example 45 for a designer oxyphotobacterial Glyceraldehyde-3-Phosphate Dehydrogenase DNA construct (1514 bp) that includes a PCR FD primer (sequence 1-20), a 305-bp *Thermosynechococcus elongatus* BP-1 nirA promoter (21-325), an enzyme-encoding sequence (326-1260) selected and modified from *Blastochloris viridis* NAD-dependent Glyceraldehyde-3-phosphate dehydrogenase (CAC80993), a 234-bp *rbcS* terminator from *Thermosynechococcus elongatus* BP-1 (1261-1494), and a PCR RE primer (1495-1514).

The designer oxyphotobacterial genes such as those shown in SEQ ID NO: 34-45 can be selected for use in full or in part, and/or in combination with various other designer butanol-production-pathway genes for construction of various designer oxyphotobacterial butanol-production pathways such as the pathways shown in Figure 1. For example, the designer genes shown in SEQ ID NOS: 34-45 can be selected for construction of an oxyphotobacterial nirA promoter-controlled and glyceraldehyde-3-phosphate-branched butanol-production pathway **(01-12)** that comprises of the following designer enzymes: NAD-dependent glyceraldehyde-3-phosphate dehydrogenase **01,** phosphoglycerate kinase **02,** phosphoglycerate mutase **03,** enolase **04,** pyruvate kinase **05,** pyruvate-ferredoxin oxidoreductase (or pyruvate-NADP⁺ oxidoreductase) **06,** thiolase **07,** 3-hydroxybutyryl-CoA dehydrogenase **08,** crotonase **09,** butyryl-CoA dehydrogenase **10,** butyraldehyde dehydrogenase **11,** and butanol dehydrogenase **12.** Use of these designer oxyphotobacterial butanol-production-pathway genes (SEQ ID NOS: 34-45) in a thermophilic and/or thermotolerant cyanobacterium may represent a thermophilic and/or thermotolerant butanol-producing oxyphotobacterium. Fox example, use of these designer genes (SEQ ID NOS: 34-45) in a thermophilic/thermotolerant cyanobacterium such as *Thermosynechococcus elongatus* BP-1 may represent a designer thermophilic/thermotolerant butanol-producing cyanobacterium such as a designer butanol-producing *Thermosynechococcus.*

### Further Host Modifications to Help Ensure Biosafety

The present invention also provides biosafety-guarded photosynthetic biofuel (e.g., butanol and/or related higher alcohols) production methods based on cell-division-controllable designer transgenic plants (such as algae and oxyphotobacteria) or plant cells. For example, the cell-division-controllable designer photosynthetic organisms (Fig. 3) are created through use of a designer biosafety-control gene(s) (Fig. 2G) in conjunction with the designer butanol-production-pathway gene(s) (Figs. 2A-2F) such that their cell division and mating function can be controllably stopped to provide better biosafety features.

In one of the various embodiments, a fundamental feature is that a designer cell-division-controllable photosynthetic organism (such as an alga, plant cell, or oxyphotobacterium) contains two key functions (Fig. 3A): a designer biosafety mechanism(s) and a designer biofuel-production pathway(s). As shown in Fig. 3B, the designer biosafety feature(s) is conferred by a number of mechanisms including: (1) the inducible insertion of designer proton-channels into cytoplasm membrane to permanently disable any cell division and mating capability, (2) the selective application of designer cell-division-cycle regulatory protein or interference RNA (iRNA) to permanently inhibit the cell division cycle and preferably keep the cell at the G₁ phase or Go state, and (3) the innovative use of a high-CO₂-requiring host photosynthetic organism for expression of the designer biofuel-production pathway(s). Examples of the designer biofuel-production pathway(s) include the designer butanol-production pathway(s), which work with the Calvin cycle to synthesize biofuel such as butanol directly from carbon dioxide (CO₂) and water (H₂O). The designer cell-division-control technology can help ensure biosafety in using the designer organisms for photosynthetic biofuel production. Accordingly, this embodiment provides, *inter alia,* biosafety-guarded methods for producing biofuel (e.g., butanol and/or related higher alcohols) based on a cell-division-controllable designer biofuel-producing alga, cyanobacterium, oxychlorobacterium, plant or plant cells.

In one of the various embodiments, a cell-division-controllable designer butanol-producing eukaryotic alga or plant cell is created by introducing a designer proton-channel gene (Fig. 2H) into a host alga or plant cell (Fig. 3B). SEQ ID NO: 46 presents example 46 for a detailed DNA construct of a designer Nial-promoter-controlled proton-channel gene (609 bp) that includes a PCR FD primer (sequence 1-20), a 262-bp nitrate reductase Nia1 promoter (21-282), a Melittin proton-channel encoding sequence (283-366), a 223-bp *RbcS2* terminator (367-589), and a PCR RE primer (590-609).

The expression of the designer proton-channel gene (Fig. 2H) is controlled by an inducible promoter such as the nitrate reductase (Nia1) promoter, which can also be used to control the expression of a designer biofuel-production-pathway gene(s). Therefore, before the expression of the designer gene(s) is induced, the designer organism can grow photoautotrophically using CO₂ as the carbon source and H₂O as the source of electrons just like wild-type organism. When the designer organism culture is grown and ready for photobiological production of biofuels, the cell culture is then placed under a specific inducing condition (such as by adding nitrate into the culture medium if the nitrate reductase (Nia1) promoter is used as an inducible promoter) to induce the expression of both the designer proton-channel gene and the designer biofuel-production-pathway gene(s). The expression of the proton-channel gene is designed to occur through its transcription in the nucleus and its translation in the cytosol. Because of the specific molecular design, the expressed proton channels are automatically inserted into the cytoplasm membrane, but leave the photosynthetic thylakoid membrane intact. The insertion of the designer proton channels into cytoplasm membrane collapses the proton gradient across the cytoplasm membrane so that the cell division and mating function are permanently disabled. However, the photosynthetic thylakoid membrane inside the chloroplast is kept intact (functional) so that the designer biofuel-production-pathway enzymes expressed into the stroma region can work with the Calvin cycle for photobiological production of biofuels from CO₂ and H₂O. That is, when both the designer proton-channel gene and the designer biofuel-production-pathway gene(s) are turned on, the designer organism becomes a non-reproducible cell for dedicated photosynthetic production of biofuels. Because the cell division and mating function are permanently disabled (killed) at this stage, the designer-organism culture is no longer a living matter except its catalytic function for photochemical conversion of CO₂ and H₂O into a biofuel. It will no longer be able to mate or exchange any genetic materials with any other cells, even if it somehow comes in contact with a wild-type cell as it would be the case of an accidental release into the environments.

According to one of the various embodiments, the nitrate reductase (Nia1) promoter or nitrite reductase (nirA) promoter is a preferred inducible promoter for use to control the expression of the designer genes. In the presence of ammonium (but not nitrate) in culture medium, for example, a designer organism with Nia1-promoter-controlled designer proton-channel gene and biofuel-production-pathway gene(s) can grow photoauotrophically using CO₂ as the carbon source and H₂O as the source of electrons just like a wild-type organism. When the designer organism culture is grown and ready for photobiological production of biofuels, the expression of both the designer proton-channel gene and the designer biofuel-production-pathway gene(s) can then be induced by adding some nitrate fertilizer into the culture medium. Nitrate is widely present in soils and nearly all surface water on Earth. Therefore, even if a Nia1-promoter-controlled designer organism is accidentally released into the natural environment, it will soon die since the nitrate in the environment will trig the expression of a Nia1-promoter-controlled designer proton-channel gene which inserts proton-channels into the cytoplasm membrane thereby killing the cell. That is, a designer photosynthetic organism with Nia1-promoter-controlled proton-channel gene is programmed to die as soon as it sees nitrate in the environment. This characteristic of cell-division-controllable designer organisms with Nia1-promoter-controlled proton-channel gene provides an added biosafety feature.

The art in constructing proton-channel gene (Fig. 2H) with a thylakoid-membrane targeting sequence has recently been disclosed [James W. Lee (2007). Designer proton-channel transgenic algae for photobiological hydrogen production, PCT International Publication Number: WO 2007/134340 A2]. In the present invention of creating a cell-division-controllable designer organism, the thylakoid-membrane-targeting sequence must be omitted in the proton-channel gene design. For example, the essential components of a Nia1-promoter-controlled designer proton-channel gene can simply be a Nia1 promoter linked with a proton-channel-encoding sequence (without any thylakoid-membrane-targeting sequence) so that the proton channel will insert into the cytoplasm membrane but not into the photosynthetic thylakoid membrane.

According to one of the various embodiments, it is a preferred practice to use the same inducible promoter such as the Nial promoter to control the expression of both the designer proton-channel gene and the designer biofuel-production pathway genes. In this way, the designer biofuel-production pathway(s) can be inducibly expressed simultaneously with the expression of the designer proton-channel gene that terminates certain cellular functions including cell division and mating.

In one of the various embodiments, an inducible promoter that can be used in this designer biosafety embodiment is selected from the group consisting of the hydrogenase promoters [HydA1 (Hyd1) and HydA2, accession number: AJ308413, AF289201, AY090770], the Cyc6 gene promoter, the Cpxl gene promoter, the heat-shock protein promoter HSP70A, the CabII-1 gene (accession number M24072) promoter, the Ca1 gene (accession number P20507) promoter, the Ca2 gene (accession number P24258) promoter, the nitrate reductase (Nia1) promoter, the nitrite-reductase-gene (*nirA*) promoters, the bidirectional-hydrogenase-gene *hox* promoters, the light- and heat-responsive *groE* promoters, the Rubisco-operon *rbcL* promoters, the metal (zinc)-inducible *smt* promoter, the iron-responsive *idiA* promoter, the redox-responsive *crhR* promoter, the heat-shock-gene *hsp16.6* promoter, the small heat-shock protein (Hsp) promoter, the CO₂-responsive carbonic-anhydrase-gene promoters, the green/red light responsive *cpcB2A2* promoter, the UV-light responsive *lexA, recA* and *ruvB* promoters, the nitrate-reductase-gene (*narB*) promoters, and combinations thereof.

In another embodiment, a cell-division-controllable designer photosynthetic organism is created by use of a carbonic anhydrase deficient mutant or a high-CO₂-requiring mutant as a host organism to create the designer biofuel-production organism. High-CO₂-requiring mutants that can be selected for use in this invention include (but not limited to): *Chlamydomonas reinhardtii* carbonic-anhydrase-deficient mutant12-1C (CC-1219 cal mt-), *Chlamydomonas reinhardtii cia3* mutant (Plant Physiology 2003, 132:2267-2275), the high-CO₂-requiring mutant M3 of *Synechococcus sp.* Strain PCC 7942, or the carboxysome-deficient cells of *Synechocystis sp.* PCC 6803 (Plant biol (Stuttg) 2005, 7:342-347) that lacks the CO₂-concentrating mechanism can grow photoautotrophically only under elevated CO₂ concentration level such as 0.2-3% CO₂.

Under atmospheric CO₂ concentration level (380 ppm), the carbonic anhydrase deficient or high-CO₂-requiring mutants commonly cannot survive. Therefore, the key concept here is that a high-CO₂-requiring designer biofuel-production organism that lacks the CO₂ concentrating mechanism will be grown and used for photobiological production of biofuels always under an elevated CO₂ concentration level (0.2-5% CO₂) in a sealed bioreactor with CO₂ feeding. Such a designer transgenic organism cannot survive when it is exposed to an atmospheric CO₂ concentration level (380ppm = 0.038% CO₂) because its CO₂-concetrating mechanism (CCM) for effective photosynthetic CO₂ fixation has been impaired by the mutation. Even if such a designer organism is accidentally released into the natural environment, its cell will soon not be able to divide or mate, but die quickly of carbon starvation since it cannot effectively perform photosynthetic CO₂ fixation at the atmospheric CO₂ concentration (380 ppm). Therefore, use of such a high-CO₂-requiring mutant as a host organism for the genetic transformation of the designer biofuel-production-pathway gene(s) represents another way in creating the envisioned cell-division-controllable designer organisms for biosafety-guarded photobiological production of biofuels from CO₂ and H₂O. No designer proton-channel gene is required here.

In addition to the innovative use of a high-CO₂-requiring mutant, a highly thermophilic *Thermosynechococcus elongatus* (such as *T. elongatus* BP-1) which can grow inside a photobioreactor at a temperature as high as 55°C but cannot grow or survive outside the bioreactor below 30°C is used as a host organism as an additional approach to create biosafety-guarded transgenic photosynthetic organisms. Since this thermophilic organism can grow only inside the bioreactor at a temperature as high as 55°C but cannot grow or survive in the natural environment outside the reactor below 30°C, use of this type of highly thermophilic organism as a host organism for genetic transformation with designer biofuel-production-pathway genes in creating transgenic photosynthetic organisms provides an assured biosafety-guarded feature in accordance of the present invention.

In another embodiment, a cell-division-controllable designer organism (Fig. 3B) is created by use of a designer cell-division-cycle regulatory gene as a biosafety-control gene (Fig. 2G) that can control the expression of the cell-division-cycle (cdc) genes in the host organism so that it can inducibly turn off its reproductive functions such as permanently shutting off the cell division and mating capability upon specific induction of the designer gene.

Biologically, it is the expression of the natural cdc genes that controls the cell growth and cell division cycle in cyanobacteria, algae, and higher plant cells. The most basic function of the cell cycle is to duplicate accurately the vast amount of DNA in the chromosomes during the S phase (S for synthesis) and then segregate the copies precisely into two genetically identical daughter cells during the M phase (M for mitosis). Mitosis begins typically with chromosome condensation: the duplicated DNA strands, packaged into elongated chromosomes, condense into the much-more compact chromosomes required for their segregation. The nuclear envelope then breaks down, and the replicated chromosomes, each consisting of a pair of sister chromatids, become attached to the microtubules of the mitotic spindle. As mitosis proceeds, the cell pauses briefly in a state called metaphase, when the chromosomes are aligned at the equator of the mitotic spindle, poised for segregation. The sudden segregation of sister chromatids marks the beginning of anaphase during which the chromosomes move to opposite poles of the spindle, where they decondense and reform intact nuclei. The cell is then pinched into two by cytoplasmic division (cytokinesis) and the cell division is then complete. Note, most cells require much more time to grow and double their mass of proteins and organelles than they require to replicate their DNA (the S phase) and divide (the M phase). Therefore, there are two gap phases: a G₁ phase between M phase and S phase, and a G2 phase between S phase and mitosis. As a result, the eukaryotic cell cycle is traditionally divided into four sequential phases: G₁, S, G₂, and M. Physiologically, the two gap phases also provide time for the cell to monitor the internal and external environment to ensure that conditions are suitable and preparation are complete before the cell commits itself to the major upheavals of S phase and mitosis. The G₁ phase is especially important in this aspect. Its length can vary greatly depending on external conditions and extracellular signals from other cells. If extracellular conditions are unfavorable, for example, cells delay progress through G₁ and may even enter a specialized resting state known as Go (G zero), in which they remain for days, weeks, or even for years before resuming proliferation. Indeed, many cells remain permanently in Go state until they die.

In one of the various embodiments, a designer gene(s) that encodes a designer cdc-regulatory protein or a specific cdc-iRNA is used to inducibly inhibit the expression of certain cdc gene(s) to stop cell division and disable the mating capability when the designer gene(s) is trigged by a specific inducing condition. When the cell-division-controllable designer culture is grown and ready for photosynthetic production of biofuels, for example, it is a preferred practice to induce the expression of a specific designer cdc-iRNA gene(s) along with induction of the designer biofuel-production-pathway gene(s) so that the cells will permanently halt at the G₁ phase or Go state. In this way, the grown designer-organism cells become perfect catalysts for photosynthetic production of biofuels from CO₂ and H₂O while their functions of cell division and mating are permanently shut off at the G₁ phase or G₀ state to help ensure biosafety.

Use of the biosafety embodiments with various designer biofuel-production-pathways genes listed in SEQ ID NOS: 1-45 (and 58-165) can create various biosafety-guarded photobiological biofuel producers (Figs. 3A, 3B, and 3C). Note, SEQ ID NOS: 46 and 1-12 (examples 1-12) represent an example for a cell-division-controllable designer eukaryotic organism such as a cell-division-controllable designer alga (e.g., *Chlamydomonas*) that contains a designer Nial-promoter-controlled proton-channel gene (SEQ ID NO: 46) and a set of designer Nial-promoter-controlled butanol-production-pathway genes (SEQ ID NOS: 1-12). Because the designer proton-channel gene and the designer biofuel-production-pathway gene(s) are all controlled by the same Nial-promoter sequences, they can be simultaneously expressed upon induction by adding nitrate fertilizer into the culture medium to provide the biosafety-guarded photosynthetic biofuel-producing capability as illustrated in Fig. 3B. Use of the designer Nial-promoter-controlled butanol-production-pathway genes (SEQ ID NOS: 1-12) in a high CO₂-requiring host photosynthetic organism, such as *Chlamydomonas reinhardtii* carbonic-anhydrase-deficient mutant12-1C (CC-1219 cal mt-) or *Chlamydomonas reinhardtii cia3* mutant, represents another example in creating a designer cell-division-controllable photosynthetic organism to help ensure biosafety.

This designer biosafety feature may be useful to the production of other biofuels such as biodiesel, biohydrogen, ethanol, and intermediate products as well. For example, this biosafety embodiment in combination with a set of designer ethanol-production-pathway genes such as those shown SEQ ID NOS: 47-53 can represent a cell-division-controllable ethanol producer (Fig. 3C). Briefly, SEQ ID NO: 47 presents example 47 for a detailed DNA construct (1360 base pairs (bp)) of a *nirA*-promoter-controlled designer NAD-dependent Glyceraldehyde-3-Phosphate-Dehydrogenase gene including: a PCR FD primer (sequence 1-20), a 88-bp *nirA* promoter (21-108) selected from the *Synechococcus sp.* (freshwater cyanobacterium) nitrite-reductase-gene promoter sequence, an enzyme-encoding sequence (109-1032) selected and modified from a *Cyanidium caldarium* cytosolic NAD-dependent glyceraldehyde-3-phosphate-dehydrogenase sequence (GenBank accession number: CAC85917), a 308-bp *Synechococcus rbcS* terminator (1033-1340), and a PCR RE primer (1341-1360) at the 3' end.

SEQ ID NO: 48 presents example 48 for a designer *nirA*-promoter-controlled Phosphoglycerate Kinase DNA construct (1621 bp) that includes a PCR FD primer (sequence 1-20), a 88-bp *Synechococcus sp.* strain PCC 7942 nitrite-reductase *nirA* promoter (21-108), a phosphoglycerate-kinase-encoding sequence (109-1293) selected from a *Geobacillus kaustophilus* phosphoglycerate-kinase sequence (GenBank: BAD77342), a 308-bp *Synechococcus rbcS* terminator (1294-1601), and a PCR RE primer (1602-1621).

SEQ ID NO: 49 presents example 49 for a designer *nirA*-promoter-controlled Phosphoglycerate-Mutase DNA construct (1990 bp) that includes a PCR FD primer (sequence 1-20), a 88-bp *Synechococcus sp.* strain PCC 7942 nitrite-reductase *nirA* promoter (21-108), a 9-bp Xho I NdeI site (109-117), a phosphoglycerate-mutase encoding sequence (118-1653) selected from the sequences of a *Caldicellulosiruptor saccharolyticus* DSM 8903 phosphoglycerate mutase (GenBank: ABP67536), a 9-bp XbaI site (1654-1662), a 308-bp *Synechococcus sp.* strain PCC 7942 *rbcS* terminator (1663-1970), and a PCR RE primer (1971-1990).

SEQ ID NO: 50 presents example 50 for a designer *nirA*-promoter-controlled Enolase DNA construct (1765 bp) that includes a PCR FD primer (sequence 1-20), a 88-bp *Synechococcus sp.* strain PCC 7942 nitrite reductase *nirA* promoter (21-108), a 9-bp Xho I NdeI site (109-117), an enolase-encoding sequence (118-1407) selected from the sequence of a *Cyanothece sp.* CCY0110 enolase (GenBank: ZP_01727912), a 21-bp Lumio-tag-encoding sequence (1408-1428), a 9-bp XbaI site (1429-1437) containing a stop codon, a 308-bp *Synechococcus rbcS* terminator (1438-1745), and a PCR RE primer (1746-1765) at the 3' end.

SEQ ID NO: 51 presents example 51 for a designer *nirA*-promoter-controlledPyruvate Kinase DNA construct (1888 bp) that includes a PCR FD primer (sequence 1-20), a 88-bp *Synechococcus* nitrite reductase *nirA* promoter (21-108), a 9-bp Xho I NdeI site (109-117), a Pyruvate-Kinase-encoding sequence (118-1530) selected from a *Selenomonas ruminantium* Pyruvate Kinase sequence (GenBank: AB037182), a 21-bp Lumio-tag sequence (1531-1551), a 9-bp XbaI site (1552-1560), a 308-bp *Synechococcus rbcS* terminator (1561-1868), and a PCR RE primer (1869-1888).

SEQ ID NO: 52 presents example 52 for a designer *nirA*-promoter-controlledPyruvate Decarboxylase DNA construct (2188 bp) that includes a PCR FD primer (sequence 1-20), a 88-bp *Synechococcus* nitrite reductase *nirA* promoter (21-108), a 9-bp Xho I NdeI site (109-117), a Pyruvate-Decarboxylase-encoding sequence (118-1830) selected from the sequences of a *Pichia stipitis* pyruvate-decarboxylase sequence (GenBank: XM_001387668), a 21-bp Lumio-tag sequence (1831-1851), a 9-bp XbaI site (1852-1860), a 308-bp *Synechococcus rbcS terminator* (1861-2168), and a PCR RE primer (2169-2188) at the 3' end.

SEQ ID NO: 53 presents example 53 for a *nirA*-promoter-controlled designer NAD(P)H-dependent Alcohol Dehydrogenase DNA construct (1510 bp) that includes a PCR FD primer (sequence 1-20), a 88-bp *Synechococcus* nitrite-reductase *nirA* promoter (21-108), a NAD(P)H dependent Alcohol-Dehydrogenase-encoding sequence (109-1161) selected/modified (its mitochondrial signal peptide sequence removed) from the sequence of a *Kluyveromyces lactis* alcohol dehydrogenase (ADH3) gene (GenBank: X62766), a 21-bp Lumio-tag sequence (1162-1182), a 308-bp *Synechococcus rbcS* terminator (1183-1490), and a PCR RE primer (1491-1510) at the 3' end.

Note, SEQ ID NOS: 47-53 (DNA-construct examples 47-53) represent a set of designer *nirA*-promoter-controlledethanol-production-pathway genes that can be used in oxyphotobacteria such as *Synechococcus sp.* strain PCC 7942. Use of this set of designer ethanol-production-pathway genes in a high-CO₂-requiring cyanobacterium such as the *Synechococcus sp.* Strain PCC 7942 mutant M3 represents another example of cell-division-controllable designer cyanobacterium for biosafety-guarded photosynthetic production of biofuels from CO₂ and H₂O.

### More on Designer Calvin-Cycle-Channeled Production of Butanol and Related Higher Alcohols

The present invention further discloses designer Calvin-cycle-channeled and photosynthetic-NADPH (reduced nicotinamide adenine dinucleotide phosphate)-enhanced pathways, associated designer DNA constructs (designer genes) and designer transgenic photosynthetic organisms for photobiological production of butanol and related higher alcohols from carbon dioxide and water. In this context throughout this specification as mentioned before, a "higher alcohol" or "related higher alcohol" refers to an alcohol that comprises at least four carbon atoms, including both straight and branched higher alcohols such as 1-butanol and 2-methyl-1-butanol. The Calvin-cycle-channeled and photosynthetic-NADPH-enhanced pathways are constructed with designer enzymes expressed through use of designer genes in host photosynthetic organisms such as algae and oxyphotobacteria (including cyanobacteria and oxychlorobacteria) organisms for photobiological production of butanol and related higher alcohols. The said butanol and related higher alcohols are selected from the group consisting of: 1-butanol, 2-methyl-1-butanol, isobutanol, 3-methyl-1-butanol, 1-hexanol, 1-octanol, 1-pentanol, 1-heptanol, 3-methyl-1-pentanol, 4-methyl-1-hexanol, 5-methyl-1-heptanol, 4-methyl-1-pentanol, 5-methyl-1-hexanol, and 6-methyl-1-heptanol. The designer photosynthetic organisms such as designer transgenic algae and oxyphotobacteria (including cyanobacteria and oxychlorobacteria) comprise designer Calvin-cycle-channeled and photosynthetic NADPH-enhanced pathway gene(s) and biosafety-guarding technology for enhanced photobiological production of butanol and related higher alcohols from carbon dioxide and water.

Photosynthetic water splitting and its associated proton gradient-coupled electron transport process generates chemical energy intermediate in the form of adenosine triphosphate (ATP) and reducing power in the form of reduced nicotinamide adenine dinucleotide phosphate (NADPH). However, certain butanol-related metabolic pathway enzymes such as the NADH-dependent butanol dehydrogenase (GenBank accession numbers: YP_148778, NP_561774, AAG23613, ZP_05082669, ADO12118, ADC48983) can use only reduced nicotinamide adenine dinucleotide (NADH) but not NADPH. Therefore, to achieve a true coupling of a designer pathway with the Calvin cycle for photosynthetic production of butanol and related higher alcohols, it is a preferred practice to use an effective NADPH/NADH conversion mechanism and/or NADPH-using enzyme(s) (such as NADPH-dependent enzymes) in construction of a compatible designer pathway(s) to couple with the photosynthesis/Calvin-cycle process in accordance with the present invention.

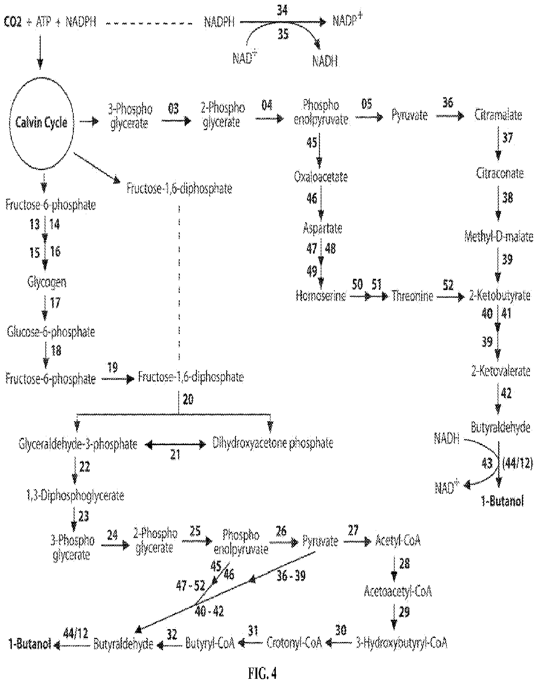

According to one of the various embodiments, a number of various designer Calvin-cycle-channeled pathways can be created by use of an NADPH/NADH conversion mechanism in combination with certain amino-acids-metabolic pathways for production of butanol and higher alcohols from carbon dioxide and water. The Calvin-cycle-channeled and photosynthetic-NADPH-enhanced pathways are constructed typically with designer enzymes that are selectively expressed through use of designer genes in a host photosynthetic organism such as a host alga or oxyphotobacterium for production of butanol and higher alcohols. A list of exemplary enzymes that can be selected for use in construction of the Calvin-cycle-channeled and photosynthetic-NADPH-enhanced pathways are presented in Table 1. As shown in Figures 4-10, the net results of the designer Calvin-cycle-channeled and photosynthetic NADPH-enhanced pathways in working with the Calvin cycle are production of butanol and related higher alcohols from carbon dioxide (CO₂) and water (H₂O) using photosynthetically generated ATP (Adenosine triphosphate) and NADPH (reduced nicotinamide adenine dinucleotide phosphate). A significant feature is the innovative utilization of an NADPH-dependent glyceraldehyde-3-phosphate dehydrogenase **34** and a nicotinamide adenine dinucleotide (NAD)-dependent glyceraldehyde-3-phosphate dehydrogenase **35** to serve as a NADPH/NADH conversion mechanism that can convert certain amount of photosynthetically generated NADPH to NADH which can then be used by NADH-requiring pathway enzymes such as an NADH-dependent alcohol dehydrogenase **43** (examples of its encoding gene with GenBank accession numbers are: BAB59540, CAA89136, NP_148480) for production of butanol and higher alcohols.

More specifically, an NADPH-dependent glyceraldehyde-3-phosphate dehydrogenase **34** (e.g., GenBank accession numbers: ADC37857, ADC87332, YP_003471459, ZP_04395517, YP_003287699, ZP_07004478, ZP_04399616) catalyzes the following reaction that uses NADPH in reducing 1,3-Diphosphoglycerate (1,3-DiPGA) to 3-Phosphoglyaldehyde (3-PGAld) and inorganic phosphate (Pi):

1,3-DiPGA + NADPH + H⁺ → 3-PGAld + NADP⁺ + Pi [3]

Meanwhile, an NAD-dependent glyceraldehyde-3-phosphate dehydrogenase **35** (e.g., GenBank: ADM41489, YP_003095198, ADC36961, ZP_07003925, ACQ61431, YP_002285269, ADN80469, ACI60574) catalyzes the oxidation of 3-PGAld by oxidized nicotinamide adenine dinucleotide (NAD⁺) back to 1,3-DiPGA:

3-PGAld + NAD⁺ + Pi → 1,3-DiPGA + NADH + H⁺ [4]

The net result of the enzymatic reactions [3] and [4] is the conversion of photosynthetically generated NADPH to NADH, which various NADH-requiring designer pathway enzymes such as NADH-dependent alcohol dehydrogenase **43** can use in producing butanol and related higher alcohols. When there is too much NADH, this NADPH/NADH conversion system can run also reversely to balance the supply of NADH and NADPH. Therefore, it is a preferred practice to innovatively utilize this NADPH/NADH conversion system under control of a designer switchable promoter such as *nirA* (or Nial for eukaryotic system) promoter when/if needed to achieve robust production of butanol and related higher alcohols. Various designer Calvin-cycle-channeled pathways in combination of a NADPH/NADH conversion mechanism with certain amino-acids-metabolism-related pathways for photobiological production of butanol and related higher alcohols are further described hereinbelow.

**Table 1 lists examples of enzymes for construction of designer Calvin-cycle-linked pathways for production of butanol and related higher alcohols.**

| Enzyme /callout number | Source (Organism) | GenBank Accession Number, JGI Protein ID or Citation |
|---|---|---|
| **03:** | *Oceanithermus profundus DSM 14977; 'Nostoc azollae' 0708; Thermotoga lettingae TMO; Syntrophothermus lipocalidus DSM 12680;* | ADR35708; ADI65627, YP_003722750; YP_001470593, ABV33529; ADI02216, YP_003702781; |
| Phosphoglycerate mutase (phosphoglyceromutase) | | |
| | *Pelotomaculum thermopropionicum SI; Fervidobacterium nodosum Rt17-B1; Caldicellulosiruptor bescii DSM 6725; Fervidobacterium nodosum Rt17-B1; Thermotoga petrophila RKU-1; Deferribacter desulfuricans SSM1; Cyanobium sp. PCC 7001; Cyanothece sp. PCC 8802; Chlamydomonas reinhardtii* cytoplasm; *Aspergillus fumigatus; Coccidioides immitis; Leishmania braziliensis; Ajellomyces capsulatus; Monocercomonoides sp.; Aspergillus clavatus; Arabidopsis thaliana; Zea mays* | YP_001212148; YP_001409891; YP_002573254, YP_002573195; ABS60234; ABQ47079, YP_001244998; YP_003496402, BAI80646; ZP_05046421; YP_003138980, YP_003138979; JGI Chlre2 protein ID 161689, GenBank: AF268078; XM_747847; XM_749597; XM_001248115; XM_001569263; XM_001539892; DQ665859; XM_001270940; NM_117020; M80912 |
| **04:** | *Syntrophothermus lipocalidus DSM 12680; 'Nostoc azollae' 0708; Thermotoga petrophila RKU-1; Spirochaeta thermophila DSM 6192; Cyanothece sp. PCC 7822; Hydrogenobacter thermophilus TK-6; Thermosynechococcus elongatus BP-1, Prochlorococcus marinus str. MIT* | ADI02602, YP_003703167; ADI63801; ABQ46079; YP_003875216, ADN02943 ; YP_003886899, ADN13624; YP_003432637, BAI69436 ; BAC08209; ABO16851; |
| Enolase | | |
| | *9301; Synechococcus sp. WH 5701; Trichodesmium erythraeum IMS101; Anabaena variabilis ATCC 29413; Nostoc sp. PCC 7120; Chlamydomonas reinhardtii* cytoplasm; *Arabidopsis thaliana; Leishmania Mexicana; Lodderomyces elongisporus; Babesia bovis; Sclerotinia sclerotiorum; Pichia guilliermondii; Spirotrichonympha leidyi; Oryza sativa; Trimastix pyriformis; Leuconostoc mesenteroides; Davidiella tassiana; Aspergillus oryzae; Schizosaccharomyces pombe; Brassica napus; Zea mays* | ZP_01083626; ABG51970; ABA23124; BAB75237; GenBank: X66412, P31683; AK222035;DQ221745; XM_001528071; XM_001611873; XM_001594215; XM_001483612; AB221057; EF122486, U09450; DQ845796; AB088633; U82438; D64113; U13799; AY307449; U17973; |
| **05:** | *Syntrophothermus lipocalidus DSM 12680; Cyanothece sp. PCC 8802; Thermotoga lettingae TMO; Caldicellulosiruptor bescii DSM 6725; Geobacillus kaustophilus HTA426; Thermosynechococcus elongatus BP-1; Thermosipho melanesiensis BI429; Thermotoga petrophila RKU-1; Caldicellulosiruptor saccharolyticus DSM 8903; Cyanothece sp. PCC 7425; Acaryochloris marina MBIC11017; Cyanothece sp.PCC 8801; Microcystis aeruginosa NIES-843; Cyanothece sp. PCC 7822; cyanobacterium UCYN-A; Arthrospira maxima CS-328; Synechococcus sp. PCC 7335; Chlamydomonas reinhardtii* cytoplasm; *Arabidopsis thaliana; Saccharomyces cerevisiae; Babesia bovis; Sclerotinia sclerotiorum; Trichomonas vaginalis; Pichia guilliermondii; Pichia stipitis; Lodderomyces elongisporus; Coccidioides immitis; Trimastix pyriformis; Glycine max (soybean)* | ADI02459, YP_003703024; YP_002372431; YP_001471580, ABV34516; YP_002573139; YP_148872; NP_681306, BAC08068; YP_001306168, ABR30783; YP_001244312, ABQ46736; ABP67416, YP_001180607; ACL43749, YP_002482578; YP_001514814; YP_003138017; YP_001655408; YP_003890281; YP_003422225; ZP_03273505; ZP_05035056; JGI Chlre3 protein ID 138105; GenBank: AK229638; AY949876, AY949890, AY949888; XM_001612087; XM_001594710; XM_001329865; XM_001487289; XM_001384591; XM_001528210; XM_001240868; DQ845797; L08632 |
| Pyruvate kinase | | |
| **06a:** | *Peranema trichophorum; Euglena gracilis* | GenBank: EF114757; AB021127, AJ278425; |
| Pyruvate-NADP⁺ oxidoreductase | | |
| **06b:** | *Mastigamoeba balamuthi; Desulfovibrio africanus; Entamoeba histolytica; Trichomonas vaginalis; Cryptosporidium parvum; Cryptosporidium baileyi; Giardia lamblia; Entamoeba histolytica; Hydrogenobacter thermophilus; Clostridium pasteurianum;* | GenBank: AY101767; Y09702; U30149; XM_001582310, XM_001313670, XM_001321286, XM_001307087, XM_001311860, XM_001314776, XM_001307250; EF030517; EF030516; XM_764947; XM_651927; AB042412; Y17727 |
| Pyruvate-ferredoxin oxidoreductase | | |
| **07**: | *Butyrivibrio fibrisolvens; Aeropyrum pernix K1 Bacillus subtilis; butyrate-producing bacterium L2-50;* | GenBank: AB190764; NP_148577, NP_147604; KFC31530, KFC30638; DQ987697; |
| Thiolase or acetyl-CoA acetyltransferase (EC 2.3.1.9) | | |
| | *Thermoanaerobacterium thermosaccharolyticum; Escherichia coli; Clostridium acetobutylicum ATCC 824; Clostridium beijerinckii NCIMB 8052; Clostridium sp. DL-VIII; Clostridium pasteurianum DSM 525; Clostridium cellulovorans 743B; Clostridium carboxidivorans P7; Clostridium haemolyticum NCTC 9693; Clostridium novyi B str. ATCC 27606; Clostridium aceticum; Clostridium pasteurianum NRRL B-598; Syntrophothermus lipocalidus DSM 12680; Sulfobacillus acidophilus DSM 10332; Kyrpidia tusciae DSM 2912; Geobacillus thermoleovorans; Exiguobacterium sp. AT1b; Anoxybacillus sp. BCO1; Thermotoga lettingae TMO; Kosmotoga olearia TBF 19.5.1; Fervidobacterium nodosum Rt17-B1; Archaeoglobus sulfaticallidus PM70-1; Methanothermobacter sp. CaT2; Methanocella conradii HZ254; Pyrococcus horikoshii OT3; Bacillus methanolicus MGA3; Geobacillus sp. JF8; Thermus sp. CCB_US3_UF1; Schleiferia thermophila str. Yellowstone;* | Z92974; EDV69072, EDV66074, YP_490465, EDX40880; NP_149242; ABR32599, ABR35750; EHI99795, EHI97451; KER14812, KER12654; ADL52748, ADL49976; ADO12109, EET85014; KEI18159; KEI13236; KJF28669; ETD70327, ETD69108; ADI01070, ADI02832, ADI02736, ADI01249; AEW06715, AEW06150; ADG07885, ADG05293; AEV21117, AEV18833; ACQ69746; KHF30322; ABV34112; ACR79316; ABS60179; AGK61581, AGK61418; BAM69958; YP_005381694, YP_005380728; NP_142626; AIE61589; AGT33759, AGT33346; AEV16506; KFD38991; |
| **08:** | *Clostridium beijerinckii; Butyrivibrio fibrisolvens; Ajellomyces capsulatus; Aspergillus fumigatus; Aspergillus clavatus; Neosartorya fischeri; Butyrate-producing bacterium L2-50; Arabidopsis thaliana; Thermoanaerobacterium thermosaccharolyticum; Zobellia galactanivorans; Clostridium pasteurianum NRRL B-598; Clostridium carboxidivorans P7; Pseudomonas batumici;* | GenBank: AF494018; AB190764; XM_001537366; XM_741533; XM_001274776; XM_001262361; DQ987697; BT001208; Z92974, WP_013298133; YP_004739166, CAZ98887; ETD65676, ETD67822; ADO12108; KIH82523 |
| 3-Hydroxybutyryl-CoA dehydrogenase | | |
| **09:** | *Clostridium beijerinckii; Butyrivibrio fibrisolvens; Butyrate-producing bacterium L2-50; Thermoanaerobacterium thermosaccharolyticum; Clostridium pasteurianum NRRL B-598; Clostridium perfringens str. 13; Geobacillus sp. JF8; Thermoanaerobacter sp. YS13;* | GenBank: AF494018; AB190764; DQ987697; Z92974, CAB07495, WP_01329813 7; ETD68658, ETD68657, ETD66584; BAB79801; AGT32321, AGT32312; KHO61061; |
| Crotonase | | |
| **10:** | *Clostridium beijerinckii; Butyrivibrio fibrisolvens; Butyrate-producing bacterium L2-50; Thermoanaerobacterium thermosaccharolyticum;* | GenBank: AF494018; AB190764; DQ987697; Z92974; |
| Butyryl-CoA dehydrogenase or *Trans*-enoyl-CoA reductase (Ter) (EC1.1.1.36) | | |
| | *Treponema denticola ATCC 35405;* | 4GGO_A, 4GGP_D, 4FBG_P, |
| | *Treponema denticola ATCC 35405; Treponema denticola; uncultured bacterium; Flavobacterium johnsoniae; Clostridium pasteurianum NRRL B-598; Fibrobacter succinogenes;* | 4FBG_O; NP_971211; WP_002669849, WP_002681770; AFH02847; WP_011921530, ABQ03048; ETD66981; WP 014545506, ACX74348; |
| **11:** | *Clostridiumsaccharoperbutylacetonicu;* | GenBank: AY251646, AAP42563; |
| Butyraldehyde dehydrogenase or aldehyde/alcohol dehydrogenase (AdhE2) | *Clostridium acetobutylicum ATCC 824* | YP_009076789, AAK09379; |
| **12**a: | *Geobacillus kaustophilus HTA426; Clostridium perfringens str. 13; Carboxydothermus hydrogenoformans; Pseudovibrio sp. JE062; Clostridium carboxidivorans P7; Bacillus pseudofirmus OF4; Oceanobacillus iheyensis HTE831; Slackia exigua ATCC 700122; Fusobacterium ulcerans ATCC 49185; Listeria monocytogenes FSL J1-175; Chlorobium chlorochromatii CaD3; Clostridium perfringens D str. JGS1721; Clostridium perfringens NCTC 8239; Clostridium perfringens CPE str. F4969; Clostridium perfringens B str. ATCC 3626; Clostridium botulinum NCTC 2916; Nostoc sp. PCC 7120; Clostridium botulinum CFSAN001628; Wolinella succinogenes;* | YP_148778, BAD77210 ; NP_561774, BAB80564; AAG23613; ZP_05082669, EEA96294 ; ADO12118; ADC48983, YP_003425875; NP_693981, BAC15015; ZP_06159969, EEZ61452; ZP_05633940; ZP_05388801; ABB28961; ZP_02952811; ZP_02641897; ZP_02638128; ZP_02634798; EDT24774; ZP_02614964, ZP_02614746; NP_488606, BAB76265; EKX78737; CAE10066; |
| NADH-dependent Butanol dehydrogenase | | |
| **12**b: | *Clostridium perfringens str. 13; Clostridium saccharobutylicum; Subdoligranulum variabile DSM 15176; Butyrivibrio crossotus DSM 2876; Oribacterium sp. oral taxon 078 str. F0262; Clostridium sp. M62*/*1; Clostridium hathewayi DSM 13479; Subdoligranulum variabile DSM 15176; Faecalibacterium prausnitzii A2-165; Blautia hansenii DSM 20583; Roseburia intestinalis L1-82, Bacillus cereus Rock3-28; Eubacterium rectale ATCC 33656; Clostridium sp. HGF2; Atopobium rimae ATCC 49626; Clostridium perfringens D str. JGS1721; Clostridium perfringens NCTC 8239; Clostridium butyricum 5521; Clostridium carboxidivorans P7; Clostridium botulinum E3 str. Alaska E43; Clostridium novyi NT; Clostridium botulinum B str. Eklund; Thermococcus sp. AM4; Fusobacterium sp. D11; Anaerococcus vaginalis ATCC 51170; Clostridium perfringens; Anaerostipes caccae DSM 14662; Amphritea japonica;* | NP_562172, BAB80962; AAA83520; EFB77036; EFF67629, ZP_05792927; ZP_06597730, EFE92592; EFE12215, ZP_06346636; EFC98086, ZP_06115415; ZP_05979561; ZP_05615704, EEU95840; ZP_05853889, EEX22072; ZP_04745071, EEU99657; ZP_04236939, EEL31374; YP_002938098, ACR75964; EFR36834; ZP_03568088; ZP_02952006; ZP_02642725; ZP_02950013, ZP_02950012; ZP_06856327; YP_001922606, YP_001922335, ACD52989; YP_878939; YP_001887401; EEB74113; EFD81183; ZP_05473100, EEU12061; EDT27639, EDT24389; EDR98218; WP 026340117; |
| NADPH-dependent Butanol dehydrogenase | | |
| | *Aquabacterium sp. NJ1;* | KGM39209; |
| **13**: | *Chlamydomonas reinhardtii; Phaseolus vulgaris; Oryza sativa; Arabidopsis thaliana; Colocasia esculenta; Amaranthus cruentus; Parachlorella kessleri; Triticum aestivum; Sorghum bicolor; Astragalus membranaceus; Perilla frutescens; Zea mays; Ipomoea batatas* | GenBank:AF026422, AF026421, DQ019314, AF433156; AB293998; D16202, AB115917, AY299404; AF121673, AK226881; NM_101044; AY225862, AY142712; DQ178026; AB232549; Y16340; AF168786; AF097922; AF210699; AF019297; AF068834; |
| Starch synthase | | |
| **14**: | *Arabidopsis thaliana; Zea mays; Chlamydia trachomatis; Solanum tuberosum (potato) ; Shigella flexneri; Lycopersicon esculentum* | GenBank: NM_127730, NM_124205, NM_121927, AY059862; EF694839, EF694838; AF087165; P55242; NP_709206; T07674 |
| Glucose-1-phosphate adenylyltransferase | | |
| **15:** | *Oryza sativa* plastid; *Ajellomyces capsulatus; Pichia stipitis; Lodderomyces elongisporus; Aspergillus fumigatus; Arabidopsis thaliana; Populus tomentosa; Oryza sativa; Zea mays* | GenBank: AC105932, AF455812; XM_001536436; XM_001383281; XM_001527445; XM_749345; NM_124561, NM_180508, AY128901; AY479974; AF455812; U89342, U89341 |
| Phosphoglucomutase | | |
| **16:** | *Staphylococcus carnosus subsp. carnosus TM300;* | YP_002633806, CAL27621; |
| Hexose-phosphate-isomerase | | |
| **17:** | *Hordeum vulgare* aleurone cells; *Trichomonas vaginalis; Phanerochaete chrysosporium; Chlamydomonas reinhardtii; Arabidopsis thaliana; Dictyoglomus thermophilum* heat-stable amylase gene; | GenBank: J04202; XM_001319100; EF143986; AY324649; NM_129551; X07896; |
| Alpha-amylase; | | |
| Beta-amylase; | *Arabidopsis thaliana; Hordeum vulgare; Musa acuminate;* | GenBank: NM 113297: D21349; DQ166026; |
| Starch phosphorylase; | *Citrus hybrid cultivar* root; *Solanum tuberosum* chloroplast; *Arabidopsis thaliana; Triticum aestivum; Ipomoea batatas;* | GenBank: AY098895; P53535; NM_113857, NM_114564; AF275551; M64362 |
| **18:** | *Chlamydomonas reinhardtii; Saccharomyces cerevisiae; Pichia stipitis; Ajellomyces capsulatus; Spinacia oleracea* cytosol; *Oryza sativa* cytoplasm; *Arabidopsis thaliana; Zea mays* | JGI Chlre3 protein ID 135202; GenBank: M21696; XM_001385873 ; XM_001537043; T09154; P42862; NM_123638, NM_118595; U17225 |
| Glucose-phosphate (glucose-6-phosphate) isomerase | | |
| **19:** | *Chlamydomonas reinhardtii; Arabidopsis thaliana; Ajellomyces capsulatus; Yarrowia lipolytica; Pichia stipitis; Dictyostelium discoideum; Tetrahymena thermophila; Trypanosoma brucei; Plasmodium falciparum; Spinacia oleracea;* | JGI Chlre2 protein ID 159495; GenBank: NM_001037043, NM_179694, NM_119066, NM_125551; XM_001537193; AY142710; M_001382359, XM_001383014; XM_639070; XM_001017610; XM_838827; XM_001347929; DQ437575; |
| Phosphofructose kinase | | |
| **20:** | *Chlamydomonas reinhardtii* chloroplast; *Fragaria x ananassa* cytoplasm; *Homo sapiens; Babesia bovis; Trichomonas* | GenBank: X69969; AF308587; NM_005165; XM_001609195; XM_001312327, XM_001312338; |
| Fructose-diphosphate aldolase | | |
| | *vaginalis; Pichia stipitis; Arabidopsis thaliana* | XM_001387466; NM_120057, NM 001036644 |
| **21:** | *Arabidopsis thaliana; Chlamydomonas reinhardtii; Sclerotinia sclerotiorum; Chlorella pyrenoidosa; Pichia guilliermondii; Euglena intermedia; Euglena longa; Spinacia oleracea; Solanum chacoense; Hordeum vulgare; Oryza sativa* | GenBank: NM_127687, AF247559; AY742323; XM_001587391; AB240149; XM_001485684; DQ459379; AY742325; L36387; AY438596; U83414; EF575877; |
| Triose phosphate isomerase | | |
| **34:** | *Staphylococcus aureus 04-02981; Staphylococcus lugdunensis; Staphylococcus lugdunensis HKU09; Vibrio cholerae BX 330286; Vibrio sp. Ex25; Pseudomonas savastanoi pv.; Vibrio cholerae B33; Grimontia hollisae CIP 101886; Vibrio mimicus MB-451, Vibrio coralliilyticus ATCC BAA-450; Vibrio cholerae MJ-1236; Zea mays* cytosolic NADP dependent; *Apium graveolens; Vibrio cholerae B33; Vibrio cholerae TMA 21; Vibrio cholerae bv. albensis VL426; Vibrio orientalis CIP 102891; Vibrio cholerae MJ-1236; Vibrio cholerae CT 5369-93; Vibrio sp. RC586; Vibrio furnissii CIP 102972; Vibrio metschnikovii CIP 69.14;* | ADC37857; ADC87332; YP_003471459; ZP_04395517; YP_003287699; ZP_07004478, EFI00105; ZP_04399616 ZP_06052988, EEY71738; ZP_06041160; ZP_05886203; YP_002876243; NP_001105589; AAF08296; EEO17521; EEO13209; EEO01829; ZP_05943395; ACQ62447; ZP_06049761; ZP_06079970; ZP_05878983; ZP 05883187; |
| NADPH-dependent Glyceraldehyde-3 -phosphate dehydrogenase | | |
| **35:** | *Edwardsiella tarda FL6-60; Flavobacteriaceae bacterium 3519-10; Staphylococcus aureus 04-02981; Pseudomonas savastanoi pv. savastanoi NCPPB 3335;* | ADM41489; YP_003095198; ADC36961; ZP_07003925; |
| NAD-dependent Glyceraldehyde-3 -phosphate dehydrogenase | | |
| | *Vibrio cholerae MJ-1236; Streptococcus pyogenes NZ131; Helicobacter pylori 908; Streptococcus pyogenes NZ131; Staphylococcus lugdunensis HKU09; Vibrio sp. Ex25; Stenotrophomonas chelatiphaga; Pseudoxanthomonas dokdonensis; Stenotrophomonas maltophilia; Vibrio cholerae B33; Photobacterium damselae subsp. damselae CIP 102761; Vibrio sp. RC586; Grimontia hollisae CIP 101886; Vibrio furnissii CIP 102972; Acidithiobacillus caldus ATCC 51756; Nostoc sp. PCC 7120; Vibrio cholerae BX 330286; Vibrio cholerae TMA 21; Nostoc sp. PCC 7120; Pinus sylvestris; Cheilanthes yavapensis; Cheilanthes wootonii; Astrolepis laevis;* | ACQ61431, YP_002878104; YP_002285269; ADN80469; ACI60574; ADC88142; ACY51070; ADK67090; ADK67075; ADK67085, ACH90636; ZP_04401333; ZP_06155532; ZP_06080908; ZP_06052393; EEX42220; ZP_05292346; CAC41000; EEO22474; EEO13042; CAC41000; CAA04942; ACO58643, ACO58642; ACO58624, ACO58623; CBH41484, CBH41483; |
| **36:** | *Hydrogenobacter thermophilus TK-6;* | YP_003433013, ADO45737, |
| (R)-Citramalate synthase (EC 2.3.1.182) | *Geobacter bemidjiensis Bem; Geobacter sulfurreducens KN400; Methanobrevibacter ruminantium M1; Leptospira biflexa serovar Patoc strain 'Patoc 1 (Paris)'; Leptospira biflexa serovar Monteralerio; Leptospira interrogans serovar Australis; Leptospira interrogans serovar Pomona; Leptospira interrogans serovar Autumnalis; Leptospira interrogans serovar Pyrogenes; Leptospira interrogans serovar Canicola; Leptospira interrogans serovar Lai; Acetohalobium arabaticum DSM5501; Leadbetterella byssophila DSM 17132; Bacteroides xylanisolvens XB1A; Mucilaginibacter paludis DSM 18603; Prevotella ruminicola 23; Flavobacterium johnsoniae UW101; Victivallis vadensis ATCC BAA-548; Prevotella* c*opri DSM 18205; Alistipes shahii WAL 8301; Methylobacter tundripaludum SV96; Methanosarcina mazei Go1;* | BAI69812; ACH38284; ADI84633; CP001719 ; ABK13757; ABK13756; ABK13755; ABK13753; ABK13754; ABK13752; ABK13751; ABK13750; ABK13749; ADL11763, YP_003998693; CBK66631; EFQ72644; ADE82919; ABQ04337; ZP_06244204, EFA99692; EFB36404, ZP_06251228 ; CBK64953; ZP_07654184; NP 632695; |
| **37:** | *Eubacterium eligens ATCC 27750 Methanocaldococcus jannaschii; Sebaldella termitidis ATCC 33386; Eubacterium eligens ATCC 27750;* | YP_002930810, YP_002930809; P81291; ACZ06998; ACR72362, ACR72361, ACR72363, YP_002930808; |
| (R)-2-Methylmalate dehydratase (large and small subunits) (EC 4.2.1.35) | | |
| **38:** | *Thermotoga petrophila RKU-1; Cyanothece sp. PCC 7822; Syntrophothermus lipocalidus DSM 12680; Caldicellulosiruptor saccharolyticus DSM 8903; Pelotomaculum thermopropionicum SI*, *Caldicellulosiruptor bescii DSM 6725; Caldicellulosiruptor saccharolyticus DSM 8903; E. coli ; Spirochaeta thermophila DSM 6192; Pelotomaculum thermopropionicum SI; Hydrogenobacter thermophilus TK-6; Deferribacter desulfuricans SSM1; Anoxybacillus flavithermus WK1; Thermosynechococcus elongatus BP-1; Geobacillus kaustophilus HTA426; Synechocystis sp. PCC 6803; Chlamydomonas reinhardtii;* | ABQ46641, ABQ46640; YP_003886427, YP_003889452; ADI02900, ADI02899, YP_003703465, ADI01294; ABP66933, ABP66934; |
| 3-Isopropylmalate dehydratase (large + small subunits) (EC 4.2.1.33) | | |
| | | YP_001211082, YP_001211083; YP_002573950, YP_002573949; YP_001180124, YP_001180125; leuC, ECK0074, JW0071; leuD, ECK0073, JW0070; YP_003875294, YP_003873373; YP_001213069, YP_001213068; YP_003433547, YP_003432351; YP_003495505, YP_003495504; ACJ32977, ACJ32978; BAC08461, BAC08786; BAD76941, BAD76940; BAA18738, BAA18298; XP_001702135, XP_001696402; |
| **39:** | *Thermotoga petrophila RKU-1; Cyanothece sp. PCC 7822; Thermosynechococcus elongatus BP-1; Syntrophothermus lipocalidus DSM; Caldicellulosiruptor bescii DSM 6725; Paludibacter propionicigenes WB4; Leadbetterella byssophila DSM 17132; Caldicellulosiruptor saccharolyticus DSM 8903; Thermus thermophilus;* | ABQ46392, YP_001243968; YP_003888480, ADN15205; BAC09152, NP_682390; ADI02898, YP_003703463; ADQ78220; YP_002573948; YP_003998692; ABP66935; AAA16706, YP_001180126; |
| 3-Isopropylmalate dehydrogenase (EC 1.1.1.85) | | |
| | *Pelotomaculum thermopropionicum SI; Geobacillus kaustophilus HTA426; Hydrogenobacter thermophilus TK-6; Spirochaeta thermophila DSM 6192; Deferribacter desulfuricans SSM1; Anoxybacillus flavithermus WK1; Volvox carteri f. nagariensis; Chlamydomonas reinhardtii; Ostreococcus tauri;* | YP_001211084; YP_148510, BAD76942; YP_003433176; YP_003873639; YP_003495917; YP_002314961; XP_002955062, EFJ43816; XP_001701074, XP_001701073; XP_003083133; |
| **40:** | *Thermotoga petrophila RKU-1; Cyanothece sp. PCC 7822; Cyanothece sp. PCC 8802; Nostoc punctiforme PCC 73102; Pelotomaculum thermopropionicum SI; Hydrogenobacter thermophilus TK-6; E. coli ; Caldicellulosiruptor saccharolyticus DSM 8903; Syntrophothermus lipocalidus DSM 12680; Geobacillus kaustophilus HTA426; Caldicellulosiruptor bescii DSM 6725; Anoxybacillus flavithermus WK1; Deferribacter desulfuricans SSM1; Thermosynechococcus elongatus BP-1; Spirochaeta thermophila DSM 6192; Thermotoga lettingae TMO; Volvox carteri f. nagariensis; Micromonas sp. RCC299; Micromonas pusilla CCMP1545; Chlamydomonas reinhardtii;* | ABQ46395, YP_001243971; YP_003890122, ADN16847; ACU99797; ACC82459; YP_001211081; YP_003432474, BAI69273; NP_414616, AAC73185; ABP66753, YP_001179944; YP_003703466, ADI02901; YP_148511, BAD76943; YP_002572404; YP_002314960, ACJ32975; YP_003496874, BAI81118; NP_682187, BAC08949; ADN03009, YP_003875282; YP_001469896, ABV32832; XP_002945733,EFJ52728; ACO69978, XP_002508720; XP_003063010, EEH52949; XP_001696603, EDP08580 ; |
| 2-Isopropylmalate synthase (EC 2.3.3.13) | | |
| **41:** | *Geobacillus kaustophilus HTA426; Anabaena variabilis ATCC 29413; Synechocystis sp. PCC 6803; Anoxybacillus flavithermus WK1; Thermosynechococcus elongatus BP-1; Spirochaeta thermophila DSM 6192; Salmonella enterica subsp. enterica serovar Typhimurium str. D23580; Staphylococcus aureus A5937; Francisella philomiragia subsp. philomiragia ATCC 25015; Neisseria lactamica; Francisella novicida U112; Staphylococcus aureus A5937; Staphylococcus aureus subsp. aureus 68*-*397; Fusobacterium sp. 2_1_31; Francisella novicida GA99-3549; marine bacterium HP15; Bacillus licheniformis ATCC 14580; Rhodobacter sphaeroides 2.4.1; Bordetella petrii DSM 12804; Agrobacterium vitis S4;* | YP_148509, YP_148508; YP_324467, YP_324466; NP_442926, NP_441618; YP_002314962, YP_002314963; NP_682024, NP_681699 ; YP_003873372; CBG23133, CBG23132 ; |
| isopropylmalate isomerase large/small subunits (EC 4.2.1.33) | | |
| | | ZP_05702396; EET20545; AAA53236; ABK88972; EEV86047; ZP_05607839; EE038992; EDN35429; ADP98363, ADP98362; YP_092517, YP_092516; YP_353947, YP_353945; YP_001631647, YP_001631646; YP 002551071, YP_002551071; |
| **42:** | *Lactococcus lactis; Lactococcus lactis subsp. lactis KF147; Lactococcus lactis subsp. Lactis; Kluyveromyces marxianus; Kluyveromyces lactis; Mycobacterium avium 104; Mycobacterium ulcerans Agy99; Mycobacterium bovis; Mycobacterium leprae; Proteus mirabilis HI4320;* | AAS49166; ADA65057, YP_003353820; |
| 2-keto acid decarboxylase (EC 4.1.1.72, etc) | | |
| | | CAG34226; AAA35267; CAA59953; A0QBE6; A0PL16; Q7U140; Q9CBD6; |
| | *Staphylococcus aureus 04-02981; Acetobacter pasteurianus; Saccharomyces cerevisiae; Zymomonas mobilis subsp. mobilis CP4; Mycobacterium tuberculosis; Mycobacterium smegmatis str. MC2 155; Mycobacterium bovis BCG str. Pasteur 1173P2;* | YP_002150004; ADC36400; AAM21208; CAA39398; AAA27696; 053865; A0R480; A1KGY5; |
| **43:** | *Thermoplasma volcanium GSS1; Gluconacetobacter hansenii; Saccharomyces cerevisiae; Aeropyrum pernix K1; Rhodobacterales bacterium HTCC2083; Bradyrhizobium japonicum USDA 110; Syntrophothermus lipocalidus; Fervidobacterium nodosum Rt17-B1; Desulfotalea psychrophila LSv54; Acetobacter pasteurianus IFO 3283-03; Gluconobacter hoxydans 621H; Aeromonas hydrophila; Acetobacter pasteurianus IFO 3283-01; Streptomyces hygroscopicus; Zymomonas mobilis; Ralstonia eutropha H16; Staphylococcus aureus; Staphylococcus epidermidis RP62A; Thalassiosira pseudonana CCMP1335; Rhodobacter sphaeroides; Alcanivorax borkumensis; Geobacillus stearothermophilus; Saccharomyces cerevisiae; Geobacillus sp. JF8; Pseudomonas alcaligenes OT 69; Pseudomonas aeruginosa; Pseudomonas sp. HMP271; Erythrobacter sp. JL475; Komagataeibacter rhaeticus AF1; Hyphomonas atlantica; Mastigocoleus testarum; Blastomonas sp. CACIA14H2;* | BAB59540 ZP_06834544; CAA89136; NP_148480; ZP_05073895; NP_769420; ADI01021; YP_001411173; YP_065604; BAI03878; YP_192500; ABK38651; BAI00830; EFL29096; AFN57379; Q0KDL6; Q5HI63, Q2YSX0; Q5HRD6; EED91217, XP_002291110 ; WP_011337799; WP_011587359; P42328; NP_013800, EDN64471; AGT31264; EQM66009; KHE33423; KGK82208; KEO86527; KDU94382; KCZ64352; WP_027839825; ESZ87639; |
| Alcohol dehydrogenase (NAD dependent) (EC 1.1.1.1); | | |
| **44:** | *Pelotomaculum thermopropionicum SI; Fusobacterium sp. 7*_*1; Pichia pastoris GS115; Pichia pastoris GS115; Escherichia coli str. K-12 substr. MG1655; Clostridium hathewayi DSM 13479; Clostridium butyricum 5521; Clostridium beijerinckii NCIMB 8052; Fusobacterium ulcerans ATCC 49185; Fusobacterium sp. D11; Desulfovibrio desulfuricans subsp. desulfuricans str. G20; Clostridium novyi NT; Clostridium tetani E88; Aureobasidium pullulans; Scheffersomyces stipitis CBS 6054, Thermotoga lettingae TMO; Thermotoga petrophila RKU-1; Coprinopsis cinerea okayama7#130; Saccharomyces cerevisiae EC111B;* | YP_001211038, BAF58669; ZP_04573952, EEO43462; XP_002494014, XP_002490014; CAY71835, XP_002492217, CAY67733; yqhD, NP_417484, AAC76047; EFC99049; ZP_02948287; AAT38119; ZP_05632371; ZP_05440863; YP_389756; YP_878957; NP_782735; ADG56699; ABN66271, XP_001384300; YP_001471424; YP_001244106; XP_001834460; CAY82157; |
| Alcohol dehydrogenase (NADPH dependent) (EC 1.1.1.2); | | |
| | *Saccharomyces cerevisiae JAY291; Klebsiella oxytoca E718; Clostridium intestinale URNW; butyrate-producing bacterium SS3*/*4; Synechocystis sp. PCC 6803; Synechocystis sp. PCC 6714; Calothrix sp. PCC 7507; Aphanizomenon flos-aquae; Planktothrix mougeotii; Synechococcus sp. NKBG042902; Synechococcus sp. NKBG15041c; Synechocystis sp. PCC 7509; Dolichospermum circinale; Neosynechococcus sphagnicola sy1; filamentous cyanobacterium ESFC-1; Fischerella sp. PCC 9605; Arthrospira platensis; Spirulina subsalsa; Thermosynechococcus sp. NK55a; Thermus sp. CCB_US3_UF1; Pelotomaculum thermopropionicum SI; Bacillus subtilis; Gracilibacillus halophilus YIM-C55.5; Hydrogenobacter thermophilus TK-6; Geobacillus sp. JF8; Hyphomicrobium nitrativorans NL23; Gloeobacter violaceus PCC 7421; Trichodesmium erythraeum IMS101; Scytonema hofmanni UTEXB 1581; Nostoc sp. PCC 7120; Fischerella sp.; Chlorogloeopsis fritschii;* | EEU07174; YP_006496277, AFN30383; ERK32339; YP_007825178; WP_010874320, NP_443028; AIE73666; YP_007063887;; KHG39231; WP_026796109; WP_030008223; WP_028954057; WP_024545825; WP_028091627; KGF72916; WP_026222390; WP_026731746; KDR57756; WP_026079920; AHB89154; AEV16814, AEV17127 ; BAF58669; KFC30246; ENH96132; YP_003433496, BAI70295; AGT31348; AHB47697; NP_923681; YP_722487; WP_029632527 NP_489374; WP_026735116, WP_026723997; WP 026087612 |
| **45:** | *Thermaerobacter subterraneus DSM 13965; Cyanothece sp. PCC 7822; Thermus sp.; Rhodothermus marinus; Thermosynechococcus elongatus BP-1; Leadbetterella byssophila DSM 17132; Riemerella anatipestifer DSM 15868; Mucilaginibacter paludis DSM 18603; Truepera radiovictrix DSM 17093; Ferrimonas balearica DSM 9799; Meiothermus silvanus DSM 9946; Nocardiopsis dassonvillei subsp. dassonvillei DSM 43111; E. coli, Meiothermus ruber DSM 1279; Olsenella uli DSM 7084; Ktedonobacter racemifer DSM 44963; Rhodopirellula baltica SH 1; Oceanithermus profundus DSM 14977; marine bacterium HP15; Marivirga tractuosa DSM 4126; Mucilaginibacter paludis DSM 18603; Streptomyces coelicolorA3(2); Delftia acidovorans SPH-1; Actinobacillus pleuropneumoniae serovar 13 str. N273; Prochlorococcus marinus str. MIT 9301; Prochlorococcus marinus str. NATL1A Prochlorococcus marinus str. MIT 9515; Clostridium cellulovorans 743B;* | EFR61439; YP_003887888; BAA07723; CAA67760; NP_682702, BAC09464; YP_003998059, ADQ17706 ; ADQ81501, YP_004045007; EFQ77722; YP_003706036; YP_003911597, ADN74523; YP_003685046; YP_003681843; ZP_07594313, ZP_07565817; ADD27759; YP_003801346, ADK68466; ZP_06967036, EFH90147; NP_866412, CAD78193; ADR36285; ADP96559; ADR23252; ZP_07746438; NP_627344; ABX34873; ZP_07544559; ABO18389; ABM76577; ABM72969; YP_003842669, ADL50905; |
| Phosphoenolpyruvate carboxylase (EC 4.1.1.31) | | |
| | *Neisseria meningitidis Z2491; Deinococcus geothermalis DSM 11300; Micromonospora sp. L5; Chlorobium phaeobacteroides DSM 266; Arthrobacter sp. FB24; Rhodomicrobium vannielii ATCC 17100; Gordonia bronchialis DSM 43247; Thermus aquaticus Y51MC23; Burkholderia ambifaria IOP40-10;* | CAM07667; ABF44963; ZP_06399624; ABL64615; YP_830113; YP_004010507; YP_003273502; ZP_03496338; ZP 02894226; |
| **46:** | *Thermotoga lettingae TMO; Synechococcus elongatus PCC 6301; Synechococcus elongatus PCC 7942; Thermosipho melanesiensis BI429; Thermotoga petrophila RKU-1; Thermus thermophilus; Anoxybacillus flavithermus WK1; Bacillus sp.; E. coli, Pelotomaculum thermopropionicum SI; Phormidium lapideum; Fervidobacterium nodosum Rt17-B1; Geobacillus kaustophilus HTA426; Thermosynechococcus elongatus BP-1; Anoxybacillus flavithermus WK1; Geobacillus kaustophilus HTA426; Spirochaeta thermophila DSM 6192; Caldicellulosiruptor bescii DSM 6725; Caldicellulosiruptor saccharolyticus DSM 8903; Arabidopsis thaliana; Glycine max; Lupinus angustifolius; Chlamydomonas reinhardtii; Micromonas pusilla CCMPI545;* | YP_001470126; YP_172275; YP_401562; YP_001306480; YP_001244588; BAA07487; YP_002315494; AAA22250; aspC: BAB34434; YP_001211971; BAB86290; YP_001410686, YP_001409589; YP_148025, YP_147632, YP_146225; NP_683147; ACJ34747; BAD77213, BAD76064; YP_003874653; YP_002572445; YP_001179582; |
| Aspartate aminotransferase (EC 2.6.1.1) | | |
| | | AAA79371; AAA33942; CAA42430; XP_001696609; XP_003060871; |
| **47:** | *Thermotoga lettingae TMO; Cyanothece sp. PCC 8802; Thermotoga petrophila RKU-1 Hydrogenobacter thermophilus TK-6; Anoxybacillus flavithermus WK1; Bacillus sp.; Spirochaeta thermophila DSM 6192; Anoxybacillus flavithermus WK1; Geobacillus kaustophilus HTA426; Syntrophothermus lipocalidus DSM 12680; E. coli; Thermosynechococcus elongatus BP-1; Fervidobacterium nodosum Rt17-B1; Spirochaeta thermophila DSM 6192; Pelotomaculum thermopropionicum SI; Caldicellulosiruptor saccharolyticus; Caldicellulosiruptor bescii DSM 6725; Thermosipho melanesiensis BI429; Thermotoga lettingae TMO; Arabidopsis thaliana; Chlamydomonas reinhardtii;* | YP_001470361, ABV33297; YP_003136939; YP_001244864, YP_001243977; YP_003432105, BAI68904; ACJ35001; AAA22251; YP_003873788, ADN01515; ACJ34043, YP_002316986; BAD77480, YP_149048 ; ADI02230, YP_003702795; ZP_07594328, ZP_07565832; NP_682623, BAC09385; ABS59942, YP_001410786; YP_003873302, ADN01029; YP_001212149, YP_001211837; ABP66605; YP_002573821; YP_001307097, ABR31712; YP_001470985, ABV33921; CAA67376; XP_001698576, EDP08069, XP_001695256; |
| Aspartokinase (EC=2.7.2.4) | | |
| **48:** | *Thermotoga lettingae TMO; Trichodesmium erythraeum IMS101; Prochlorococcus marinus str. MIT; Thermotoga petrophila RKU-1; Caldicellulosiruptor saccharolyticus* | YP_001470981, ABV33917; ABG50031; ABM76828; ABQ47283, YP_001244859; ABP67176, YP_001180367; |
| Aspartate-semialdehyde dehydrogenase | | |
| | *DSM 8903; Syntrophothermus lipocalidus DSM 12680; E. coli; Fervidobacterium nodosum Rt17-B1 Caldicellulosiruptor bescii DSM 6725; Thermosipho melanesiensis BI429; Spirochaeta thermophila DSM 6192; Pelotomaculum thermopropionicum SI; Hydrogenobacter thermophilus TK-6; Anoxybacillus flavithermus WK1; Geobacillus kaustophilus HTA426; Deferribacter desulfuricans SSM1; Thermosynechococcus elongatus BP-1; Carboxydothermus hydrogenoformans; Chlamydomonas reinhardtii; Polytomella parva; Glycine max; Zea mays; Oryza sativa Indica Group;* | ADI01804, YP_003702369; YP_001460230, YP_001464895; YP_001409594, ABS59937; YP_002573009; YP_001307092, ABR31707; YP_003875128, ADN02855; YP_001211836, BAF59467; YP_003432252, BAI69051; YP_002316029, ACJ34044; YP_147128, BAD75560; YP_003496635, BAI80879; NP_680860, BAC07622; AAG23574, AAG23573; XP_001695059, EDP02211; ABH11018; ACU30050; ACG41594; ABR26065; |
| **49:** | *Syntrophothermus lipocalidus DSM 12680; Cyanothece sp. PCC 7822; Caldicellulosiruptor bescii DSM 6725; Caldicellulosiruptor saccharolyticus DSM 8903; E. coli; Spirochaeta thermophila DSM 6192; Pelotomaculum thermopropionicum SI; Hydrogenobacter thermophilus TK-6; Anoxybacillus flavithermus WK1; Geobacillus kaustophilus HTA426; Deferribacter desulfuricans SSM1; Thermosynechococcus elongatus BP-1; Glycine max; Chlamydomonas reinhardtii; Micromonas sp. RCC299;* | ADI02231, YP_003702796; YP_003887242; YP_002573819; ABP66607, YP_001179798; EFJ98002; YP_003873441, ADN01168; YP_001212151, BAF59782; YP_003431981, BAI68780; YP_002316756, ACJ34771; YP_148817, BAD77249; YP_003496401, BAI80645 ; NP_681068, BAC07830; ABG78600, AAZ98830; XP_001699712, EDP07408; ACO69662, XP 002508404; |
| Homoserine dehydrogenase | | |
| **50:** | *Thermotoga petrophila RKU-1; Cyanothece sp. PCC 7822; Caldicellulosiruptor bescii DSM 6725; Caldicellulosiruptor saccharolyticus DSM 8903; E. coli; Anoxybacillus flavithermus WK1; Geobacillus kaustophilus HTA426; Thermosynechococcus elongatus BP-1; Pelotomaculum thermopropionicum SI; Hydrogenobacter thermophilus TK-6; Chlamydomonas reinhardtii; Prototheca wickerhamii; Arabidopsis thaliana; Glycine max; Zea mays;* | YP_001243979, ABQ46403; YP_003886645; YP_002573820; ABP66606, YP_001179797; AP_000667, BAB96580; YP_002316754, ACJ34769; YP_148815, BAD77247; NP_682555, BAC09317; YP_001212150, BAF59781; YP_003433124, BAI69923 ; XP_001701899, EDP06874; ABC24954; NP_179318, AAD33097; ACU26535; ACG46592; |
| Homoserine kinase (EC 2.7.1.39) | | |
| **51:** | *Thermotoga petrophila RKU-1; Cyanothece sp. PCC 7425; Thermosipho melanesiensis BI429; Syntrophothermus lipocalidus DSM 12680; E. coli; Pelotomaculum thermopropionicum SI; Anoxybacillus flavithermus WK1; Caldicellulosiruptor bescii DSM 6725; Caldicellulosiruptor saccharolyticus DSM 8903; Hydrogenobacter thermophilus TK-6; Geobacillus kaustophilus HTA426;* | YP_001243978, ABQ46402; YP_002485009; YP_001306558, ABR31173; ADI02519, YP_003703084; AP_000668, NP_414545; YP_001213220; YP_002316755, ACJ34770; YP_002572552; YP_001180015, ABP66824; YP_003433070, YP_003433019, BAI69869, BAI69818; YP_148816, YP_147614; |
| Threonine synthase (EC 4.2.99.2) | | |
| | *Thermosynechococcus elongatus BP-1; Spirochaeta thermophila DSM 6192; Deferribacter desulfuricans SSM1; Geobacillus kaustophilus HTA426;* | NP_682017, NP_681772, BAC08534, BAC08779; YP_003873303, ADN01030; YP_003495358, BAI79602; |
| **52:** | *Geobacillus kaustophilus HTA426; Prochlorococcus marinus str. MIT 9202; Synechococcus sp. PCC 7335; Thermotoga petrophila RKU-1; Pelotomaculum thermopropionicum SI; Anoxybacillus flavithermus WK1; Deferribacter desulfuricans SSM1; E. coli; Neisseria lactamica ATCC 23970; Citrobacter youngae ATCC 29220; Neisseria polysaccharea ATCC 43768; Providencia rettgeri DSM 1131; Neisseria subflava NJ9703; Mannheimia haemolytica PHL213; Achromobacter piechaudii ATCC 43553; Neisseria meningitidis ATCC 13091; Synechococcus sp. CC9902; Synechococcus sp. PCC 7002; Synechococcus sp. WH 8109; Cyanobium sp. PCC 7001; Anabaena variabilis ATCC 29413; Microcoleus chthonoplastes PCC 7420; Chlamydomonas reinhardtii;* | BAD76058, BAD75876, YP_147626, YP_147444; ZP_05137562; ZP_05035047; ABQ46585, YP_001244161; YP_001210652, BAF58283; YP_002315804, YP_002315746; YP_003497384, BAI81628; YP_001746093, ZP_07690697; EEZ76650, ZP_05986317; EFE07783, ZP_06571237; EFH23894, ZP_06863451; EFE52186, ZP_06127162; EFC51529, ZP_05985502; ZP_04978734; ZP_06687730, ZP_06684811; ZP_07369980, EFM04207; ABB26032; ACA99606; ZP_05790446, EEX07646; EDY39077, ZP_05045768; ABA20300; ZP_05029756; XP_001701816, EDP06791; |
| Threonine ammonia-lyase (EC 4.3.1.19) | | |
| **53:** | *Caldicellulosiruptor saccharolyticus* | ABP66750, ABP66751, |
| Acetolactate synthase (EC 2.2.1.6) | *DSM 8903;* | YP_001179942, ABP66455, YP_001179941, YP_001179646; |
| | *Thermotoga petrophila RKU-1;* | YP_001243976, YP_003345845, ADA66432, ADA66431, ABQ46399, YP_001243975, ABQ46400, YP_003345846; |
| | *Thermosynechococcus elongatus BP-1;* | NP_682614, BAC09376, NP_681670, BAC08432, NP_682086 ; |
| | *Syntrophothermus lipocalidus DSM 12680;* | ADI02904, YP_003703469, ADI02903, YP_003703468; |
| | *Pelotomaculum thermopropionicum SI;* | BAF58709, BAF58917, YP_001211286, YP_001211078; |
| | *Geobacillus kaustophilus HTA426;* | BAD76946, YP_148514, BAD76945, YP_148513; |
| | *Caldicellulosiruptor bescii DSM 6725;* | ACM59790, ACM59628, ACM59629, YP_002572563, YP_002572401, YP_002572402; YP_003432299, YP_003432300, |
| | *Hydrogenobacter thermophilus TK-6;* | BAI69099, BAI69098; YP_003874926, YP_003874927, |
| | *Spirochaeta thermophila DSM 6192;* | ADN02654, ADN02653, ACJ33615, YP_002314957, |
| | *Anoxybacillus flavithermus WK1;* | ACJ32972, ACJ32973, YP_002314958; YP_003496879, BAI81123, |
| | *Deferribacter desulfuricans SSM1;* | YP_003496878, BAI81122; AP_004121, BAE77622, |
| | *Escherichia coli str. K-12 substr. W3110;* | AP_004122, BAE77623, BAE77528, AP_004027, BAB96646, AP_000741; BAA12700; |
| | *Saccharomyces cerevisiae,* | EDN64495,CAA89744, EDV09697; |
| | *Thermus aquaticus; Synechococcus sp. PCC 7002; Cyanothece sp. PCC 7424; Anabaena variabilis ATCC 29413; Nostoc sp. PCC 7120; Microcystis aeruginosa NIES-843; Synechocystis sp. PCC 6803;* | YP_001735999, ACB00744; YP_002376012; YP_324035; NP_487595, BAB75254; YP_001655615; NP_441297, BAA17984, CAA66718, NP_441304, NP_442206, BAA10276 ; |
| | *Synechococcus sp. JA-2-3B'a(2-13); Synechococcus sp. JA-3-3Ab;* | YP_478353; YP_475372, ABD00213, ABD00270, YP_475476, YP_475533; |
| | *Chlamydomonas reinhardtii;* | AAC03784, AAB88292, XP_001700185, EDO98300, XP_001695168, EDP01876; |
| | *Volvox carteri; Bacillus subtilis subsp. subtilis str. 168; Bacillus licheniformis ATCC 14580;* | AAC04854, AAB88296; CAB07802 (AlsS); AAU42663 (AlsS); |
| **54:** | *Syntrophothermus lipocalidus DSM 12680; Caldicellulosiruptor saccharolyticus DSM 8903; E. coli; Thermotoga petrophila RKU-1; Calditerrivibrio nitroreducens DSM; Spirochaeta thermophila DSM 6192; Pelotomaculum thermopropionicum SI; Cyanothece sp. PCC 7822; Hydrogenobacter thermophilus TK-6; Anoxybacillus flavithermus WK1; Caldicellulosiruptor bescii DSM 6725; Geobacillus kaustophilus HTA426; Deferribacter desulfuricans SSM1; Thermosynechococcus elongatus BP-1; Cyanothece sp. PCC 7425; Nostoc punctiforme PCC 73102; Trichodesmium erythraeum IMS101; Synechococcus sp. PCC 7335; Microcoleus chthonoplastes PCC 7420; Prochlorococcus marinus str. MIT 9301; Cyanobium sp. PCC 7001; Arthrospira sp. PCC 8005; Arabidopsis thaliana; Pisum sativum (pea); Zea mays; Chlamydomonas reinhardtii; Polytomella parva; Nostoc sp. PCC 7120 (Anabaena sp. PCC 7120);* | ADI02902, YP_003703467; |
| Ketol-acid reductoisomerase (EC 1.1.1.86; ilvC) | | ABP66752, YP_001179943; AAA67577, YP_001460567; ABQ46398, YP_001243974; YP_004050904; YP_003874858, ADN02585; YP_001211079, BAF58710; YP_003885458; YP_003433279, BAI70078; YP_002314959, ACJ32974; YP_002572403; YP_148512, BAD76944; YP_003496877, BAI81121; NP_683044, BAC09806; YP_002482078; ACC82013; ABG53327; ZP_05036558; ZP_05026584; ABO18124; EDY39000; ZP_07166132; CAA48253, NP_001078309; CAA76854; ACG35752; XP_001702649, EDP06428; ABH11013; BAB74014, NP_486355, WP_010996471; |
| **55:** | *Thermotoga petrophila RKU-1; Cyanothece sp. PCC 7822; Marivirga tractuosa DSM 4126; Geobacillus kaustophilus HTA426; Syntrophothermus lipocalidus DSM 12680; Spirochaeta thermophila DSM 6192; Anoxybacillus flavithermus WK1; Caldicellulosiruptor bescii DSM 6725; Caldicellulosiruptor saccharolyticus DSM 8903; E. coli;* | YP_001243973, ABQ46397; |
| Dihydroxy-acid dehydratase (EC 4.2.1.9; ilvD) | | YP_003887466; YP_004053736; YP_147899, BAD76331, YP_147822, BAD76254; ADI02905, YP_003703470; YP_003874669, ADN02396; YP_002315593; YP_002572562; YP_001179645, ABP66454; ADR29155, YP_001460564; |
| | *Deferribacter desulfuricans SSM1; Thermosynechococcus elongatus BP-1; Hydrogenobacter thermophilus TK-6; Nostoc punctiforme PCC 73102; 'Nostoc azollae' 0708; Arthrospira maxima CS-328; Prochlorococcus marinus str. MIT 9301; Cyanobium sp. PCC 7001; Synechococcus sp. PCC 7335; Arthrospira platensis str. Paraca; Microcystis aeruginosa NIES-843; Chlamydomonas reinhardtii; Arabidopsis thaliana; Oryza sativa Indica Group; Glycine max; Nostoc sp. PCC 7120 (Anabaena sp. PCC 7120);* | YP_003496880, BAI81124; NP_681848, BAC08610; YP_003431766, BAI68565; ACC82168, ADN14191; ADI62939; EDZ97146; ABO17457; ZP_05044537, EDY37846; ZP_05037932; ZP_06383646; BAG02689; XP_001693179, EDP03205; BAB03011; ABR25557; ACU26534; NP_486811, WP_010996924; |
| **56:** | *Schizosaccharomyces japonicus yFS275;* | XP_002173231, EEB06938; |
| 2-Methylbutyraldehyde reductase (EC 1.1.1.265) | *Pichia pastoris GS115;* | XP_002490018, CAY67737, XM_002489973; |
| | *Saccharomyces cerevisiae S288c;* | DAA12209, NP_010656, NM_001180676 ; |
| | *Aspergillus fumigatus Af293; Debaryomyces hansenii CBS767; Debaryomyces hansenii Kluyveromyces lactis; Lachancea thermotolerans CBS 6340; Lachancea thermotolerans; Saccharomyces cerevisiae EC111B; Saccharomyces cerevisiae JAY291;* | XP_752003; XP_002770138; CAR65507; CAH02579; XP_002554884; CAR24447, CAR23718; CAY78868; EEU08013; |
| **57:** | *Saccharomyces cerevisiae S288c;* | DAA10635, NM_001183405, NP_014490; |
| 3-Methylbutanal reductase (EC 1.1.1.265) | *Saccharomyces cerevisiae EC111B; Saccharomyces cerevisiae JAY291;* | CAY86141; EEU07090; |
| **07':** 3-Ketothiolase (reversible) | *Geobacillus kaustophilus HTA426; Azohydromonas lata;* | YP_147173, BAD75605; |
| | *Rhodoferax ferrireducens T118; Allochromatium vinosum; Dechloromonas aromatica RCB; Rhodobacter sphaeroides ATCC 17029; Rhodobacter sphaeroides ATCC 17025; Bacillus sp. 256; Silicibacter lacuscaerulensis ITI-1157; Aspergillus fumigatus Af293; Rhizobium etli; Citreicella sp. SE45; Silicibacter sp. TrichCH4B; Azohydromonas lata; Chromobacterium violaceum; Dinoroseobacter shibae DFL 12; Alcaligenes sp. SH-69; Candida dubliniensis CD36; Pseudomonas sp. 14-3; Aspergillus flavus NRRL3357; Aedes aegypti;* | YP_523526; CAA01849, CAA01846; YP_286222; YP_001041914; YP_001166229; ABX11181; ZP_05785678; XP_752635; AAK21958; ZP_05784120, ZP_05781517; ZP_05742998; AAC83659, AAD10275; AAC69616; ABV95064; AAP41838; CAX43351, XP_002418052; CAK18903; XP_002375989; EAT37298, EAT37297, XP_001654752, XP_001654751; |
| | *Scheffersomyces stipitis CBS 6054; Cyanothece sp. PCC 7424; Cyanothece sp. PCC 7822;* | ABN68380, XP_001386409; YP_002375827, ACK68959; YP_003886602, ADN13327; |
| | *Microcystis aeruginosa NIES-843;* | BAG04828; |
| **08':** | *Syntrophothermus lipocalidus DSM 12680; Oceanithermus profundus DSM 14977; Anoxybacillus flavithermus WK1; Pelotomaculum thermopropionicum SI; Geobacillus kaustophilus HTA426; Deferribacter desulfuricans SSM1; Glomerella graminicola ML001; Legionella pneumophila str. Corby; Aspergillus fumigatus Af293; Coprinopsis cinerea okayama7#130; Botryotinia fuckeliana B05.10; Coccidioides posadasii; E. coli; Chelativorans sp. BNC1; Nostoc punctiforme PCC 73102; Oscillatoria sp. PCC 6506;* | YP_003702743, ADI02178, ADI01287, ADI01071; ADR36325; YP_002317076, YP_002315864; YP_001210823, BAF58454; YP_149248, YP_147889; YP_003497047, BAI81291; EFQ32520, EFQ35765; YP_001250712, ABQ55366; XP_748706, XP_748351; EAU80763; XP_001559519; ABH10642; YP_001462756; YP_675197; ACC81853, YP_001866796; ZP_07114022, CBN59220; |
| 3-Hydroxyacyl-CoA dehydrogenase | | |
| **09':** | *Bordetella petrii; Bordetella petrii DSM 12804; Anoxybacillus flavithermus WK1; Geobacillus kaustophilus HTA426; Geobacillus kaustophilus; Syntrophothermus lipocalidus DSM 12680; Acinetobacter sp. SE19; Scheffersomyces stipitis CBS 6054; Laccaria bicolor S238N-H82; Alternaria alternate; Ajellomyces dermatitidis ER-3; Aspergillus fumigatus Af293; Cryptococcus neoformans var. neoformans JEC21; E. Coli; Aspergillus flavus NRRL3357; Laccaria bicolor S238N-H82; Neosartorya fischeri NRRL 181; Nostoc sp. 'Peltigera membranacea cyanobiont';* | CAP41574; YP_001629844; YP_002315700, YP_002314932; YP_148541, YP_147845, BAD76199; BAD18341; ADI02939, ADI02740, ADI02007, ADI01364; AAG10018; ABN64617, XP_001382646; EDR09131, XP_001888157; BAH83503, EEQ91989; EAL93360, XP_755398; XP_572730; ADN73405, YP_001458194; XP_002377859; EDR01115; EAW18645; ADA69246; |
| Enoyl-CoA dehydratase | | |
| **10':** | *Xanthomonas campestris pv.* | CAP53709; |
| 2-Enoyl-CoA reductase | *Campestris; Xanthomonas campestris pv. campestris str. B100; Xanthomonas campestris pv. musacearum* | YP_001905744; ZP_06489037; |
| | *NCPPB4381; Xanthomonas campestris pv. vasculorum NCPPB702;* | ZP_06487845; |
| | *Aeromicrobium marinum DSM 15272; Rhodobacterales bacterium HTCC2083; Lysinibacillus fusiformis ZC1; Mycobacterium smegmatis str. MC2; Lysinibacillus sphaericus C3-41; Coprinopsis cinerea okayama7#130; Arthroderma gypseum CBS 118893; Paracoccidioides brasiliensis Pb01; Paracoccidioides brasiliensis Pb18; Ajellomyces capsulatus G186AR; Ostreococcus tauri; Jatropha curcas;* | ZP_07718056, EFQ82338; ZP_05074461, EDZ42121; ZP_07049092, EFI69525; YP_886510, ABK76225; YP_001699417, ACA41287; XP_002910885, EFI27391; EFR05506; XP_002796528, EEH39074; EEH43955; EEH03439; XP_003083795, CAL57762; ACS32302; |
| **11':** | *Clostridium cellulovorans 743B; Thermosphaera aggregans DSM 11486; Delftia acidovorans SPH-1; Comamonas testosteroni KF*-*1; Bifidobacterium longum subsp. infantis* | YP_003845606, ADL53842; YP_003649571, ADG90619; YP_001565543, ABX37158; ZP_03543536; YP_002321654, ACJ51276; |
| Acyl-CoA reductase (EC 1.2.1.50) | | |
| | *ATCC 15697;* | |
| | *Clostridium papyrosolvens DSM 2782; Acidovorax avenae subsp. avenae ATCC 19860;* | ZP_05497968, EEU57047; ZP_06211782, EFA39209; |
| | *Comamonas testosteroni KF-1; Aminomonas paucivorans DSM 12260; Herpetosiphon aurantiacus ATCC 23779;* | EED67822; ZP_07740542, EFQ24431 ; ABX07240, YP_001547368; |
| | *Clostridium beijerinckii NCIMB 8052; Geobacillus sp. G11MC16; Clostridium lentocellum DSM 5427; Leadbetterella byssophila DSM 17132; Actinosynnema mirum DSM 43827; Haliangium ochraceum DSM 14365; Photobacterium phosphoreum; Simmondsia chinensis; Hevea brasiliensis; Arabidopsis thaliana;* | ABR34265, YP_001309221; ZP_03148237, EDY05596; ZP_06885967, EFG96716; YP_003997212, ADQ16859; YP_003101455, ACU37609 ; ACY16972, YP_003268865; AAT00788; AAD38039; AAR88762; ABE65991; |
| **12':** | *Mycobacterium chubuense NBB4;* | ACZ56328; |
| Hexanol dehydrogenase | | |
| **12":** | *Drosophila subobscura;* | ABO61862, ABO65263, |
| Octanol dehydrogenase EC 1.1.1.73 | | CAD43362, CAD43361, CAD54410, CAD43360, CAD43359, CAD43358 CAD43357, CAD43356; |
| **43':** | *Pyrococcus furiosus DSM 3638; Burkholderia vietnamiensis G4; Geobacillus thermoleovorans; Geobacillus kaustophilus HTA426; Anoxybacillus flavithermus WK1; Helicobacter pylori PeCan4; Mycobacterium chubuense NBB4; Mycobacterium avium subsp. avium ATCC 25291; Aspergillus oryzae; cyanobacterium UCYN-A; Anabaena circinalis AWQC131C; Cylindrospermopsis raciborskii T3; Helicobacter pylori Sat464; Helicobacter pylori Cuz20; Mycobacterium intracellulare ATCC 13950; Mycobacterium avium subsp. avium ATCC 25291; Gluconacetobacter hansenii ATCC 23769; Helicobacter pylori Shi470; Mycobacterium avium 104; Citrus sinensis; Gossypium hirsutum; Arabidopsis halleri; Paracoccidioides brasiliensis Pb01; Pyrenophora tritici-repentis Pt-1 C-BFP; Ajellomyces capsulatus H143; Scheffersomyces stipitis CBS 6054;* | AAC25556; ABO56626; BAA94092; YP_146837, BAD75269; YP_002314715, ACJ32730; YP_003927327, ADO07277; ACZ56328; ZP_05215778; BAE71320; YP_003421738, ADB95357; ABI75134; ABI75108; ADO05766; ADO04259; ZP_05228059,ZP_05228058; ZP_05215779; |
| Short chain alcohol dehydrogenase | | |
| | | ZP_06834730, EFG83978; YP_001910563, ACD48533; YP_880627, ABK67217; ADH82118; ABD65462; ABZ02361, ABZ02360; XP_002792148, EEH34889; XP_001940779, EDU43498; EER38733; XP_001382930, ABN64901; |
| **70:** | *Ralstonia eutropha H16;* | NP_942643 (hoxK), NP_942644 |
| Membrane-bound hydrogenase (MBH) | | (hoxG), YP_015633 (hoxZ); AAP85757 (hoxK), AAP85758 |
| | *Ralstonia eutropha H16;* | (hoxG), AAA16463 (hoxZ); ABF08183 (hoxK), YP_583451 |
| | *Cupriavidus metallidurans CH34; Thiocapsa roseopersicina, Thermococcus onnurineus NA1;* | (hoxG), ABF08182 (hoxG); ADK12981, ADK12980; ACJ15972; |
| | *Thermococcus sp. 4557; Thermococcus sp. 4557; Thermococcus sp. 4557; Pyrococcus furiosus DSM 3638; Pyrococcus furiosus DSM 3638; Pyrococcus yayanosii CH1; Pyrococcus yayanosii CH1; Pyrococcus yayanosii CH1; Pyrococcus horikoshii OT3; Hydrogenovibrio marinus; Alcaligenes sp.; Rubrivivax sp.; Hydrogenobacter thermophilus TK-6; Thermococcus gammatolerans EJ3; Methanoplanus petrolearius DSM 11571; Thermococcus gammatolerans EJ3; Oligotropha carboxidovorans OM5;Aquifex aeolicus VF5; Centipeda periodontii DSM 2778; Selenomonas noxia ATCC 43541; Allochromatium vinosum DSM 180; Thiomonas intermedia K12; Aquifex aeolicus VF5;* | YP_004763067,YP_004763083; YP_004763081; AEK73406, AEK73404; NP_579163; NP_579162; YP_004624085; YP_004624086; YP_004624087; NP_142896; BAK19334; CAA63615; CAA63616; BAF73677; ACS32538; ADN36337; YP_002958402; YP_004638463 (hoxZ); AEI08136 (hoxZ); NP_213456 (hoxZ); ZP_08500995 (hoxZ); ZP_06602778 (hoxZ); ADC63224 (hoxZ); ADG32404 (hoxZ); AAC06857 (hoxZ); |
| **71:** | *Ralstonia eutropha H16; Ralstonia eutropha H16; Ralstonia eutropha H16; Ralstonia eutropha H16; Ralstonia eutropha H16; Ralstonia eutropha H16; Ralstonia eutropha H16; Ralstonia eutropha H16; Ralstonia eutropha H16; Rhodobacter capsulatus; Azotobacter vinelandii DJ; Microcystis aeruginosa NIES-843; Acaryochloris marina MBIC11017; Synechococcus sp. PCC 7002; Synechococcus elongatus PCC 6301; Synechococcus elongatus PCC 6301; Allochromatium vinosum; Microcystis aeruginosa PCC 7806; Azotobacter vinelandii DJ; Synechococcus elongatus PCC 6301; Allochromatium vinosum; Arthrospira platensis FACHB341; Microcystis aeruginosa PCC 7806; Lyngbya majuscula CCAP 1446*/*4; Synechococcus elongatus PCC 6301; Cyanothece sp. ATCC 51142; Synechococcus elongatus PCC 6301; Allochromatium vinosum; Arthrospira platensis FACHB341; Synechococcus sp. PCC 7002; Anaerolinea thermophila UNI-1; Caloramator australicus RC3;* | AAP85843 (hoxY), AAP85844 (HoxH); NP_942730 (hoxH), NP_942729(hoxY); NP_942727(hoxF), NP_942728 (hoxU); AAP85841 (hoxF), AAP85842 (hoxU); AAC06140 (hoxF), AAC06141 (hoxU), AAC06142 (hoxY), AAC06143 (hoxH); AAD38065 (hoxH); YP_002797671 (hoxH); BAG01243 (hoxH); ABW32682 (hoxH); AAN03569 (hoxH); CAA66383 (hoxH); CAA66382 (hoxY); AAX89151 (hoxY); CA088137 (hoxY); YP_002797670 (hoxY); CAA66381 (hoxU); AAX89150 (hoxU); ABC26909 (hoxU); CAO88140 (hoxU); AAY57574 (hoxU); YP_172263 (hoxU); YP_001803733 (hoxU); CAA73873 (hoxF); AAX89149 (hoxF); ABC26907 (hoxF); YP_001733465 (hoxF); BAJ63286 (hoxH); CCC57856 (hoxF); |
| Soluble hydrogenase (SH) (NAD(P)-reducing) | | |
| **72:** | *Ralstonia eutropha H16; Ralstonia eutropha H16; Cupriavidus metallidurans CH34; Cupriavidus metallidurans CH34;* | NP_942649 (hoxO), AAP85763 (hoxO), AAA16467 (hoxO); ABF08176 (hoxO); YP_583445 (hoxO); |
| Hydrogenase accessary proteins | | |
| | *Ralstonia eutropha H16;* | NP_942650 (hoxQ), AAP85764 (hoxQ), AAA16468 (hoxQ); |
| | *Cupriavidus metallidurans CH34;* | ABF08175 (hoxQ), YP_583444 (hoxQ); |
| | *Azotobacter vinelandii; Salmonella enterica subsp.;* | AAA19504 (hoxQ); EHC91928 (hoxQ/hoxR), EFX49216 (hoxQ/hoxR), |
| | *Escherichia coli B354; Methyloversatilis universalis FAM5; Shigella flexneri CDC 796-83; Ralstonia eutropha H16;* | ZP_06652932 (hoxQ); ZP_08506135 (hoxQ); EFW61888 (hoxQ); AAA16469 (hoxR), NP_942651 (hoxR) ; |
| | *Azotobacter vinelandii; Ralstonia eutropha H16;* | AAA19505 (hoxR); NP_942652 (hoxT), AAP85766 (hoxT), AAA16470 (hoxT); |
| | *Cupriavidus metallidurans CH34; Azotobacter vinelandii DJ;* | ABF08173 (hoxT); YP_002802114 (hoxT), AC081139 (hoxT); |
| | *Ralstonia eutropha H16;* | NP_942648 (hoxL), AAP85762 (hoxL), AAA16466 (hoxL); |
| | *Azotobacter vinelandii; Oligotropha carboxidovorans OM5; Cupriavidus metallidurans CH34; Salmonella enterica subsp. enterica serovar Weltevreden str. 2007-60-3289-1; Oligotropha carboxidovorans OM5; Oligotropha carboxidovorans OM4; Azotobacter vinelandii DJ;* | AAA19502 (hoxL); YP_015634 (hoxL); ABF08177 (hoxL),YP_583446 (hoxL); CBY95754 (hoxL); YP_004638464 (hoxL); AEI04509 (hoxL); YP_002802118 (hoxL), AC081143 (hoxL); |
| | *Methyloversatilis universalis FAM5;* | ZP_08506137 (hoxL), EGK70316 (hoxL); |
| | *Ralstonia eutropha H16;* | NP_942653 (hoxV), AAP85767 (hoxV), AAA16471 (hoxV); |
| | *Azotobacter vinelandii; Oligotropha carboxidovorans OM5; Cupriavidus metallidurans CH34; Azotobacter vinelandii DJ; Cupriavidus metallidurans CH34; Methyloversatilis universalis FAM5; Methyloversatilis universalis FAM5; Ralstonia eutropha H16 Oligotropha carboxidovorans OM5, Oligotropha carboxidovorans OM4; Azotobacter vinelandii; Azotobacter vinelandii DJ; Cupriavidus metallidurans CH34; Hydrogenobacter thermophilus TK-6; Hydrogenobacter thermophilus TK-6; Thermoproteus tenax Kra 1; Acidithiobacillus sp. GGI-221; Methyloversatilis universalis FAM5; Burkholderiales bacterium 1_1_47; Thiomonas intermedia K12; Thermococcus gammatolerans EJ3;* | AAA19507 (hoxV); YP_015636 (HoxV); ABF08172 (hoxV); YP_002802113 (hoxV); YP_583441 (hoxV); ZP_08506132 (hoxV); EGK70311 (hoxV); NP_942647 (hoxM); YP_004638462 (hoxM); AEI04507 (hoxM); AAA19501 (hoxM); YP_002802119 (hoxM); YP_583447 (hoxM); BAF73673 (hoxM); YP_003432119 (hoxM); CCC80713 (hoxM); EGQ60729 (hoxM); ZP_08506138 (hoxM); ZP_07342912 (hoxM); YP_003644737 (hoxM); YP_002958602 (hybD/hycI/hoxM); |
| | *Ralstonia eutropha H16; Azorhizobium caulinodans ORS 571; Bradyrhizobium japonicum; Hyphomicrobium sp. MC1; Azoarcus sp. BH72;* | NP_942661 (hoxA), AAP85775; AAS91037 (hoxA); CAA78991 (hoxA); YP_004674255 (hoxA); YP_935307 (hoxA); |
| | *Methyloversatilis universalis FAM5; Grimontia hollisae CIP 101886; Oxalobacteraceae bacterium; Ralstonia eutropha H16; Azoarcus sp. BH72;* | ZP_08506123 (hoxA); ZP_06053565 (hoxA); ZP_08276168 (hoxA); NP_942662 (hoxB), AAP85776; YP_935309 (hoxB); |
| | *Oligotropha carboxidovorans OM5; Ralstonia eutropha H16; Azoarcus sp. BH72;* | YP_004638467 (hoxB); AAP85777 (hoxC), NP_942663; YP_935310 (hoxC); |
| | *Oligotropha carboxidovorans OM4; Oligotropha carboxidovorans OM5; Oxalobacteraceae bacterium IMCC9480;* | AEI04502 (hoxC); YP_004638457 (hoxC); ZP_08276171 (hoxJ), EGF30361 (hoxJ); |
| | *Alcaligenes hydrogenophilus; Synechocystis sp. PCC 6803; Ralstonia eutropha H16; Ralstonia eutropha H16; Ralstonia eutropha H16; Cupriavidus metallidurans CH34; Ralstonia eutropha H16; Ralstonia eutropha H16; Butyrivibrio proteoclasticus B316; Oligotropha carboxidovorans OM5; Oligotropha carboxidovorans OM4; Desulfitobacterium metallireducens DSM 15288;* | AAB49362 (hoxJ); BAA18357 (hypA); NP_942654 (hypA1); NP_942733 (hypA2); NP_942716 (hypA3); YP_583440 (hypA); NP_942655 (hypBl); AAP85769 (hypBl); YP_003830670 (hypBl); YP_004638455 (hypB); AEI04500 (hypB); ZP_08976390 (hypB), EHC20145 (hypB); |
| | *Synechocystis sp. PCC 6803; Cyanothece sp. CCY0110; Cupriavidus metallidurans CH34, Ralstonia eutropha H16; Ralstonia eutropha H16; Ralstonia eutropha H16; Cupriavidus metallidurans CH34; Cupriavidus metallidurans CH34; Escherichia coli BL21 (DE3); Synechocystis sp. PCC 6803; Ralstonia eutropha H16; Ralstonia eutropha H16; Ralstonia eutropha H16; Rhizobium leguminosarum; Azotobacter vinelandii; Aeropyrum pernix K1; Sulfolobus solfataricus P2; Hydrogenobacter thermophilus TK-6; Pelotomaculum thermopropionicum SI; Syntrophothermus lipocalidus DSM 12680;* | BAA18180 (hypC); EAZ91066 (hypC); ABF08421(hypC); NP_942657 (hypC1); AAP85826 (hypC2); CAA49734 (hypD); YP_583436 (hypD); ABF08422 (hypD); ACT44398 (hypD); BAA17478 (hypE); CAA49735 (hypE); NP_942659 (hypE1); AAP85829 (hypE2); CAA37164 (hypE); AAA19513 (hypE); NP_148343 (hypE); NP_341628 (hypE); YP_003432665 (hypE); YP_001212249 (hypE); ADI01176 (hypE), YP_003701741 (hypE); |
| | *Hydrogenobacter thermophilus TK-6; Pelotomaculum thermopropionicum SI; Syntrophothermus lipocalidus DSM 12680; Caldicellulosiruptor bescii DSM 6725;* | YP_003432667 (hypF); YP_001212246 (hypF); ADI01173 (hypF), YP_003701738 (hypF); YP_002572964 (hypF); |
| | *Ralstonia eutropha H16; Ralstonia eutropha H16; Ralstonia eutropha H16; Hydrogenobacter thermophilus TK-6; Rhizobium leguminosarum; Methyloversatilis universalis FAM5; Cupriavidus metallidurans CH34; Ralstonia eutropha H16;* | CAA49731 (hypF); NP_942660 (hypX); AAP85774 (hypX) YP_003433460 (hypX); CAA37165 (hypX); ZP_08506124 (hoxX); ABF08424 (hoxX); CAA52735 (hoxX); |
| **73**: | *Desulfobulbus propionicus DSM 2032;* | ADY56959, YP_004195043; |
| NAD(P)-dependent hydrogenase | *Acetohalobium arabaticum DSM5501; Ilyobacter polyt; ropus DSM 2926; beta proteobacterium KB13 Acetohalobium arabaticum DSM5501;* | YP_003826884; ADO82414; EDZ65062, ZP_05082375; ADL11819 |
| **74:** | *Moorella thermoacetica ATCC 39073; Moorella thermoacetica ATCC 39073; Moorella thermoacetica; Methanosaeta harundinacea 6Ac; Methanoculleus marisnigri JR1 ; Methanocorpusculum labreanum Z; Helicobacter bilis ATCC 43879; Helicobacter bilis ATCC 43879; Pelotomaculum thermopropionicum SI; Hydrogenobacter thermophilus TK-6; Hydrogenobacter thermophilus TK-6; Klebsiella variicola At-22; Azospirillum sp. B510; Thermococcus gammatolerans EJ3; Yersinia pestis Antiqua; Thermofilum pendens Hrk 5; Ferrimonas balearica DSM 9799; Thermodesulfatator indicus DSM 15286; Shewanella baltica BA175; Methanocella paludicola SANAE; Methanosaeta harundinacea 6Ac;* | YP_429324, ABC18781; YP_431142, ABC20599; AAB18330 (α), AAB18329 (β); AET63712, AET63711, YP_001047290; YP_001029904, YP_001029903; ZP_04582064 (NADPH); EEO23341 (NADPH); YP_001213196; YP_003432807; YP_003433330 (NDA dependent); ADC58081, YP_003439113; YP_003451652, YP_003450092; YP_002958615; ABG15899; YP_919603; YP_003913071; AEH46025; AEG12633; YP_003357462, YP_003357461; AET64643, AET64987, AET65705; |
| Formate dehydrogenase using NAD(P)H | | |
| **75:** | *Moorella thermoacetica ATCC 39073; Methanocorpusculum labreanum Z; Sphingomonas paucimobilis; Desulfatibacillum alkenivorans AK-01; Corynebacterium aurimucosum; Clostridium acidurici; Sphingobium sp. SYK-6; Listeria monocytogenes serotype 4b str. CLIP 80459; Vibrio fischeri MJ11; Anoxybacillus flavithermus WK1; Thermotoga lettingae TMO; Fervidobacterium nodosum Rt17-B1; Thermosipho melanesiensis BI429; Thermotoga petrophila RKU-1 Pelotomaculum thermopropionicum SI;* | YP_428991; YP_001030445; BAD61061; ACL05327; YP_002834788; AAA53187; YP_004834408; YP_002758587; YP_002156619; YP_002315932; YP_001471133; YP_001410584; YP_001305561; YP_001244647 YP_001210750; |
| 10-Formyl-H₄ folate synthetase (ADP forming, 10-Formyltetrahydrofolate Synthetase ) | | |
| **76:** | *Moorella thermoacetica ATCC 39073; Thermotoga lettingae TMO; Caldicellulosiruptor bescii DSM 6725; Thermotoga petrophila RKU-1; Anoxybacillus flavithermus WK1; Geobacillus kaustophilus HTA426; Geobacillus kaustophilus HTA426; Synechococcus sp. JA-2-3B'a(2-13); Synechococcus sp. JA-3-3Ab; Exiguobacterium sp. AT1b; Thermotoga lettingae TMO;* | YP_430368, ABC19825; ABV34070; YP_002572856; ABQ47072; YP_002315305; BAD76681; YP_148249; YP_476354; YP_475381; YP_002884899; YP_001471134; |
| 5,10-Methenyl-H₄ folate cyclohydrolase (Methenyltetrahydrofolate cyclohydrolase) | | |
| **77:** | *Moorella thermoacetica ATCC 39073; Geobacillus kaustophilus HTA426; Syntrophothermus lipocalidus; Caldicellulosiruptor kronotskyensis; Caldicellulosiruptor kristjanssonii; Caldicellulosiruptor hydrothermalis; Caldicellulosiruptor owensensis OL; Caldicellulosiruptor hydrothermalis;* | ABC19825, YP_430368; BAD76681; ADI01214; ADQ46551; ADQ40482; ADQ07463; ADQ04336; YP_003992832; |
| 5,10-Methylene-H₄ folate dehydrogenase | | |
| | *Kosmotoga olearia TBF 19.5.1; Exiguobacterium sp. AT1b; Komagataella pastoris CBS 7435; Homo sapiens; Taeniopygia guttata; Syntrophobotulus glycolicus DSM 8271; Olsenella uli DSM 7084;* | ACR80790; ACQ69454; CCA37557; AAH09806; XP_002200380; ADY56189; ADK67906; |
| **78:** | *Moorella thermoacetica ATCC 39073; Syntrophothermus lipocalidus; Fervidobacterium nodosum Rt17-B1; Thermotoga petrophila RKU-1; Fervidobacterium nodosum Rt17-B1; Thermotoga lettingae TMO; Thermosipho melanesiensis BI429; Synechococcus sp. JA-2-3B'a(2-13); Hippea maritima DSM 10411; Spirochaeta thermophila DSM 6192; Deferribacter desulfuricans SSM1; Hydrogenobacter thermophilus TK-6; Pelotomaculum thermopropionicum SI;* | YP_430048, ABC19505; ADI02156; ABS61421; ABQ46674; ABS61126; ABV33918; YP_001305980; YP_477166; YP_004340445; YP_003875363; YP_003496368; YP_003432279; BAF59187, YP_001211556; |
| 5,10-Methylene-H₄ folate reductase (Methylenetetrahydrofolate reductase) | | |
| **79:** | *Moorella thermoacetica ATCC 39073; Pelotomaculum thermopropionicum SI; Clostridium carboxidivorans P7; Desulfitobacterium hafniense DCB-2; Dinoroseobacter shibae DFL 12; Ammonifex degensii KC4; Desulfotomaculum acetoxidans; Rhodobacter sphaeroides KD131; Carboxydothermus hydrogenoformans; Rhodobacter sphaeroides 2.4.1; Heliobacterium modesticaldum Ice1; Sinorhizobium meliloti 1021; Acetonema longum DSM 6540* | YP_430950, YP_430174; YP_001211554; ADO12092; YP_002461301; YP_001533020; YP_00323 8352; YP_003190781; YP_002525435; YP_360065; YP_352826; YP_001680302; NP_386092; ZP_08625620; |
| Methyl-H₄ folate: corrinoid iron-sulfur protein Methyltransferase (Methyltetrahydrofolate: corri noid/iron-sulfur protein Methyltransferase) | | |
| **80:** | *Moorella thermoacetica; Carboxydothermus hydrogenoformans Clostridium ragsdalei; Clostridium autoethanogenum; Clostridium sticklandii DSM 519; Clostridium sticklandii;* | AAA23255; 2H9A_A, 2H9A_B; AEI90763, AEI90762; AEI90746, AEI90745; YP_003936194; CBH21289; |
| Corrinoid iron-sulfur protein (CFeSP) | | |
| **81:** | *Moorella thermoacetica ATCC 39073; Moorella thermoacetica ATCC 39073; Moorella thermoacetica; Caldicellulosiruptor kristjanssonii; Caldicellulosiruptor saccharolyticus; Clostridium ragsdalei; Clostridium autoethanogenum; Desulfosporosinus orientis DSM 765; Methanococcus aeolicus Nankai-3; Desulfobacca acetoxidans DSM 11109; Thermodesulfatator indicus; Acetohalobium arabaticum DSM5501; Desulfarculus baarsii DSM 2075; Archaeoglobus veneficus SNP6; Methanosalsum zhilinae DSM 4017; Thermosediminibacter oceani; Desulfotomaculum kuznetsovii; Methanosalsum zhilinae DSM 4017;* | ABC19516, YP_430059; YP_430813 (CODH); AAA23229, AAA23228; ADQ39747; YP_001179230; AEI90761; AEI90744; AET68776; ABR56750; YP_004370981; AEH46031; ADL12817; YP_003806211; YP_004341848; AEH60991; ADL07576; YP_004517493, YP_004516875; AEH60989, AEH60993; |
| CO dehydrogenase/acetyl-CoA synthase (Fd²⁻) | | |
| **82:** | *Thermodesulfobium narugense; Desulfobacca acetoxidans; Archaeoglobus veneficus SNP6;* | YP_004437266; YP_004370392; YP_004341929; |
| Pyruvate synthase (Fd²⁻) | | |
| | *Hippea maritima DSM 10411; Desulfurobacterium thermolithotrophum; Archaeoglobus veneficus; Thermodesulfobium narugense; Archaeoglobus veneficus SNP6; Thermobacillus composti KWC4; Desulfobacca acetoxidans; Methanolinea tarda NOBI-1; Methanobacterium sp. AL-21; Methanocella paludicola SANAE;* | YP_004339618; YP_004281767, YP_004281766, ADY73708 ; AEA47214; AEE14134; YP_004341930; ZP_08918406; AEB09210; EHF09898; YP_004289712, ADZ08740; YP_003356312, YP_003356313; |
| **83:** | *Methanothermobacter marburgensis str. Marburg;* | ADL58895, ADL58894, |
| Formylmethanofuran dehydrogenase (Fmd) (Fd²⁻) | | ADL58283, ADL58893, ADL57751, ADL57749, ADL57750, ADL57748; |
| | *Methanothermobacter thermautotrophicus; Methanothermobacter thermautotrophicus;* | CAA66401, CAA61212, CAA66400, CAA66402; CAA61213, CAA61214, CAA61210, CAA61211, CAA61209; |
| | *Agrobacterium sp. H13-3; Agrobacterium vitis S4; Methylomonas methanica MC09; Desulfobacca acetoxidans DSM 11109; Methylovorus glucosetrophus SIP3-4; Methylotenera mobilis JLW8; Methylotenera versatilis 301; Methanoculleus marisnigri JR1 ;* | YP_004444030; YP_002547540 YP_004511613; YP_004370144, AEB08963; YP_003051278; YP_003048298; ADI29297; YP_001046285 YP_001046287, YP_001046533; |
| | *Methanosaeta harundinacea 6Ac;* | AET63761, AET64650, AET65189, AET64652; |
| | *Methanosphaera stadtrnanae;* | ABC56660, ABC56659, YP_447302, ABC56661, ABC56658, ABC56657 ; |
| **84:** | *Methanothermobacter marburgensis str. Marburg;* | ADL59225, YP_003850538; AET65566; CAA62582; NP_614099; YP_001305762; YP_004369335; YP_002421530; YP_004917963; NP_613403; YP_001046543; YP_001029658, YP_001029834; AAM02029, AAM01333; YP_003356088, BAI61105; |
| Formyl transferase | | |
| | *Methanosaeta harundinacea 6Ac; Methanosarcina barkeri; Methanopyrus kandleri AV19; Thermosipho melanesiensis BI429; Desulfobacca acetoxidans DSM 11109; Methylobacterium chloromethanicum; Methylomicrobium alcaliphilum; Methanopyrus kandleri AV19; Methanoculleus marisnigri JR1 ; Methanocorpusculum labreanum Z; Methanopyrus kandleri AV19; Methanocella paludicola SANAE;* | |
| **85:** | *Methanosphaera stadtmanae; Methanothermus fervidus DSM 2088; Methanosalsum zhilinae DSM 4017; Methanohalophilus mahii DSM 5219; Methanoplanus petrolearius; Archaeoglobus veneficus SNP6; Planctomyces brasiliensis DSM 5305; Methylobacillus flagellates; Xanthobacter autotrophicus; Methylosinus trichosporium OB3b; Methylobacterium organophilum; Methylococcus capsulatus;* | ABC57615, YP_448258; YP_004003819; AEH61193; ADE36644; ADN34846; YP_004342719; YP_004269775; AAD55893; AAD55896; AAD56174; AAD55900; AAD55899; |
| 5,10-Methenyl-tetrahydromethanopterin (H4 methanopterin) cyclohydrolase | | |
| | *Methylomicrobium kenyense; Methylomonas sp. LW13; Methylosinus sp. LW2; Methylomicrobium kenyense; Methanohalophilus mahii DSM 5219; Methanolinea tarda NOBI-1; Methanothermococcus okinawensis 1H1; Methanobacterium sp. SWAN-1; Methylomonas methanica MC09;* | AAS88982; AAS88987; AAS88975; AAS86344; YP_003542289; EHF09908; YP_004577331; YP_004519292; YP_004513168; |
| **86:** | *Methanothermobacter marburgensis; Methanosphaera stadtmanae; Methanococcus maripaludis X1; Methanothermobacter thermautotrophicus; Methanopyrus kandleri; Methylobacterium extorquens AM1; Methylobacillus flagellatus KT; Xanthobacter autotrophicus; Methyloversatilis universalis FAM5; Methylobacterium chloromethanicum; Methylobacterium populi BJ001; Methylobacterium extorquens PA1; Burkholderia sp. CCGE1001; Methylovorus sp. MP688; Methanocaldococcus fervens AG86; Methanocaldococcus jannaschii; Methanobrevibacter smithii;* | ADL57660, YP_003848973; YP_447224; AEK19019; CAA63376; CAA43127; AAC27020; ABE49928; AAD55895; ZP_08504846; ACK83011; YP_001924478; YP_001639299; YP_004230417; YP_004039958; YP_003128308; NP_247770; YP_001273145; |
| 5,10-Methylene-H₄-methanopterin dehydrogenase (F₄₂₀H₂) | | |
| **87:** | *Methanoplanus petrolearius; Methanocaldococcus sp. FS406-22; Methanocaldococcus infernus ME; Methanocaldococcus fervens AG86; anococcus maripaludis C6; Stenotrophomonas sp. SKA14; Amycolatopsis mediterranei S699; Corynebacterium glutamicum; Acinetobacter sp. DR1; Acinetobacter baumannii ABNIH4; Acinetobactersp. DR1; Paenibacillus terrae HPL*-*003; Acinetobacter baumannii ABNIH3; Cupriavidus necator N1; Herbaspirillum seropedicae SmR1; Burkholderia cenocepacia HI2424; Methanobrevibacter ruminantium MI; Methanococcus voltae A3; Methanococcus aeolicus Nankai-3; Methanocaldococcus vulcanius M7;* | ADN36752; YP_003458803; ADG13507; ACV24808; ABX01642; EED39154, ZP_05135093; AEK43785; EHE83474; ADI90167; EGU03459; YP_003731540; AET61191; EGT94264; AEI79563; YP_003777169; YP_840196; YP_003423269, ADC46377; ADI37005; ABR56603; ACX71899; |
| 5,10-Methylene-H₄-methanopterin reductase (F₄₂₀H₂) | | |
| **88:** | *Methanothermobacter marburgensis;* | MTBMA_c02920; |
| Methyl-H4-methanopterin: corrinoid iron-sulfur protein methyltransferase | *Methanothermobacter marburgensis str. Marburg;* | ADL57900; |
| **89:** | *Methanothermobacter marburgensis;* | MTBMA_c02910; |
| Corrinoid iron-sulfur protein (MTBMA c02910) | *Methanothermobacter marburgensis str. Marburg;* | ADL57899; |
| **90:** | *Methanothermobacter marburgensis;* | αMTBMA_c02870/14220/14210/ 14200; |
| CO dehydrogenase /acetyl-CoA synthase (Fd²⁻_{red}) | | |
| | | ε MTBMA_c14190/02880; βMTBMA_c02890; |
| | *Methanothermobacter marburgensis str.* | ADL57895; |
| | *Marburg;* | ADL59006; ADL57897; |
| **91:** | *Methanosphaera stadtmanae; Methanosphaera stadtrnanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae; Methanothermobacter marburgensis; Methanobacterium sp. SWAN-1; Methanobrevibacter ruminantium M1;* | ABC57827 (ehbA); ABC57826 (ehbB); ABC57825 (ehbC); ABC57824 (ehbD); ABC57823 (ehbE); ABC57822 (ehbF); ABC57821 (ehbG); ABC57820 (ehbH); ABC57819 (ehbI); ABC57818 (ehbJ); ABC57817 (ehbK); ABC57816 (ehbL); ABC57815 (ehbM); ABC57814 (ehbN); ABC57813 (ehbO); ABC57812(ehbP); ABC57807 (ehbO); ADL59203, YP_003850516; YP_004520980; YP_003424741, ADC47849; |
| Energy converting hydrogenase (Ech) | | |
| **92:** | *Methanosphaera stadtmanae; Methanosphaera stadtmanae; Methanosphaera stadtmanae; Methanosphaera stadtmanae; Methanosaeta harundinacea 6Ac; Methanopyrus kandleri AV19; Methanoculleus marisnigri JR1 ; Methanoculleus marisnigri JR1 Methanopyrus kandleri AV19; Methanosphaera stadtmanae; Methanosphaera stadtmanae; Archaeoglobus fulgidus DSM 4304; Methanopyrus kandleri AV19; Methanocella paludicola SANAE; Methanosaeta harundinacea 6Ac; Methanoculleus marisnigri JR1 ; Methanocella paludicola SANAE; Methanocella paludicola SANAE; Methanocella paludicola SANAE; Methanocella paludicola SANAE; Methanocella paludicola SANAE; Methanocella paludicola SANAE; Methanocella paludicola SANAE; Methanocella paludicola SANAE;* | ABC56714 (mtrA); ABC56713 (mtrB); YP_447355 (mrtC); YP_447354 (mtrD); AET65445 (mtrE); AAM01871 (mtrE); YP_001046527 (mtrE); YP_001046522 (mtrF); NP_614768 (mtrF); YP_447359 (mtrG); YP_447360 (mtrH); NP_068850 (mtrH); AAM01874 (mtrB); BAI60614 (mtrB); AET65448 (mtrB); YP_001046524 (mtrB); YP_003355598 (mtrA); YP_003355597 (mtrB); YP_003355596 (mtrC); YP_003355595 (mtrD); YP_003355594 (mtrE); BAI60616 (mtrF); YP_003355600 (mtrG); YP_003355601 (mtrH); |
| Methyl-H4MPT: coenzyme M methyltransferase (MtrA-H) | | |
| **93:** | *Methanobacterium aarhusense; Methanobacterium sp. MB4; Methanosphaera stadtmanae; Methanosphaera stadtmanae; Methanosphaera stadtmanae; Methanosphaera stadtmanae; Methanosphaera stadtmanae; Methanosphaera stadtmanae; Methanosphaera stadtmanae;* | AAR27839 (mcrA); ABG78755 (mcrA); CAE48306 (mcrA) CAE48303 (mcrB) ABC56709 (mcrC); CAE48305 (McrG) ABC56731, ABC56728; YP_447371, ABC56730 (mrtG); ABC56794; |
| Methyl-coenzyme M reductase (Mcr) | | |
| **94:** | *Methanocella paludicola SANAE; Methanocella paludicola SANAE; Methanocella paludicola SANAE; Methanosaeta harundinacea 6Ac; Methanosaeta harundinacea 6Ac;* | YP_003357823 (hdrA); YP_003357824 (hdrB); YP_003357825 (hdrC) AET63985 (hdrA); AET63982 (hdrB); |
| Heterodisulfide reductases (HdrABC, HdrDE) | | |
| | *Methanosaeta harundinacea 6Ac; Methanosaeta harundinacea 6Ac; Methanosaeta harundinacea 6Ac; Methanopyrus kandleri AV19; Methanopyrus kandleri AV19; Methanopyrus kandleri AV19;* | AET63983 (C); AET64166 (D); AET64165 (E); NP_613552 (hdrA); NP_613857 (hdrB); NP_613858 (hdrC); |
| **95:** [NiFe]-hydrogenase MvhADG (non-F420 reducing hydrogenase; methyl viologen-reducing hydrogenase) | *Methanosphaera stadtmanae; Cyanobium sp. PCC 7001; Methanothermobacter marburgensis; Methanobrevibacter ruminantium M1; Desulfobacterium autotrophicum HRM2 Desulfatibacillum alkenivorans AK-01; Methanothermobacter marburgensis; Desulfatibacillum alkenivorans AK-01; Methanobrevibacter smithii DSM 2374; Methanothermobacter marburgensis; Methanothermobacter marburgensis; Methanobrevibacter smithii; Methanobrevibacter smithii; Methanothermobacter thermautotrophicus;* | ABC56726 (mvhA); EDY38497 (mvhA); ADL59096 (mvhA) YP_003424648 (mvhA); YP_002602450 (mvhA) ACL06634 (mvhA); ADL59095 (mvhB); ACL06636 (mvhB); ZP_05975561 (mvhB); ADL59098 (mvhD); YP_003850411 (mvhD); YP_001273574 (mvhD); ABQ87206 (mvhD); AAB02349 (mvhD); |
| | *Methanothermobacter marburgensis; Desulfatibacillum alkenivorans AK-01; Cyanobium sp. PCC 7001; Methanosphaera stadtrnanae; Methanobrevibacter smithii DSM 2374; Desulfatibacillum alkenivorans AK-01; Desulfatibacillum alkenivorans AK-01; Methanoculleus marisnigri JR1 ;* | ADL59097 (mvhG); ACL06635 (mvhG); EDY38425 (mvhG); ABC56725 (mvhG); EFC93226 (mvhG); ACL06638; ACL03322; YP_001046332 (hypF); |
| **96:** Coenzyme F420-reducing hydrogenase (Frh) | *Methanocella paludicola SANAE; Methanocella paludicola SANAE; Methanocella paludicola SANAE; Synechococcus elongatus PCC 7942; Synechocystis sp. PCC 6803; Synechococcus sp. WH 7803; Synechococcus sp. RCC307; Cyanothece sp. PCC 8802; Cyanobium sp. PCC 7001; Synechococcus sp. RS9916; Synechococcus sp. JA-2-3B'a(2-13); Pelotomaculum thermopropionicum SI; Methanothermus fervidus DSM 2088; Methanococcus maripaludis S2; Methanococcus maripaludis S2; Methanococcus maripaludis S2; Methanococcus maripaludis S2;* | YP_003357229 (frhB-1); YP_003357467 (frhB-2); YP_003357509 (frhB-3); ABB57389 (frhB); BAA18574, YP_001735870; YP_001225273; YP_001227030; ACV00312 (frhB); EDY39891 (fehB); EAU74116 (frhB); YP_477499; YP_001212042, YP_001211959; YP_004004590; CAF30376 (A), NP_988502 (A); NP_988505 (B); NP_988503 (D); NP_988504 (G); |
| **97:** A₁Aₒ-ATP synthase (AhaA-IK) | *Methanobrevibacter ruminantium M1; Methanobrevibacter ruminantium M1; Methanobrevibacter ruminantium M1; Methanobrevibacter ruminantium M1 Methanobrevibacter ruminantium M1; Methanobrevibacter ruminantium M1; Methanobrevibacter ruminantium M1; Methanobrevibacter ruminantium M1; Methanobrevibacter ruminantium M1; Ferroplasma acidarmanus fer1; Thermococcus sibiricus MM 739; Thermoproteus tenax Kra 1; Thermoproteus tenax Kra 1; Methanosarcina mazei Go1;* | YP_003423444 (ahaA); YP_003423445 (ahaB); YP_003423442 (ahaC); ADC46554 (ahaD); ADC46549 (ahaE); YP_003423443 (ahaF); YP_003423438 (ahaH) ADC46547 (ahaI); YP_003423440 (ahaK); ZP_05570724; YP_002995194; CCC82573; CCC82176; AAC06375 (ahaA); |
| | *Methanosarcina mazei Go1; Methanosarcina mazei Go1; Methanosarcina mazei Go1; Methanosarcina mazei Go1; Methanosarcina mazei Go1; Methanosarcina mazei Go1;* | AAC06376 (ahaB); AAC06373 (ahaC); AAC06377 (ahaD) AAC06372 (ahaE); AAC06374 (ahaF); AAC06378 (ahaG); |
| **98:** | *Methanosarcina mazei Go1; Methanosarcina acetivorans C2A; Archaeoglobus fulgidus DSM 4304; Ferroglobus placidus DSM 10642; Methanosarcina acetivorans C2A; Archaeoglobus fulgidus DSM 4304; Methanosarcina mazei Go1; Methanocella paludicola SANAE; Methanosarcina acetivorans C2A; Methanosarcina mazei Go1; Methanosarcina mazei Go1; Methanosarcina acetivorans C2A; Methanosarcina mazei Go1; Methanocella paludicola SANAE; Methanosarcina acetivorans C2A; Methanosarcina acetivorans C2A; Methanosarcina mazei Go1; Methanocella paludicola SANAE; Methanosarcina acetivorans C2A; Archaeoglobus fulgidus DSM 4304;* | CAA58177 (mhtA); NP_616088 (mhtA); NP_070209 (mhtA); ADC65001 (mhtA); NP_616088 (mhtB); NP_070209 (mhtB); CAA58178 (mhtB); YP_003357991 (mhtC); NP_616084(mhtC); CAA58178 (nhtC); NP_634195 (mhtC); AAM04564 (mhtC); CAA62962 (nhtD); YP_003355429 (mhtD); NP_616085 (mhtD); NP_616087 (mhtG); CAA58176 9 (mhtG); YP_003357989 (mhtG); AAM04562 (mhtG); AAB89863 (mhtG); |
| Membrane bound cytochrome-containing F420-nonreducing hydrogenase (VhtGAC, VhtD) | | |
| **99a:** | *Methanobrevibacter ruminantium M1; Methanobrevibacter ruminantium M1; Methanothermococcus okinawensis IH1; Methanotorris igneus Kol 5; Methanolinea tarda NOBI-1; Methanobacterium sp. SWAN-1; Methanobacterium sp. AL-21; Methanolinea tarda NOBI-1;* | YP_003423415 (cofA); ADC46523 (cofA); YP_004576675; YP_004484309; EHF10591; YP_004520759; YP_004289639; ZP_09042363; |
| CofA: Lactaldehyde dehydrogenase (for F₄₂₀ synthesis) | | |
| **99b:** | *Methanothermobacter marburgensis; Methanothermobacter thermautotrophicus* | cofB; cofB; |
| CofB: L-Lactate kinase (for F₄₂₀ synthesis) | | |
| **99c:** CofC: 2-phospho-L-lactate guanylyltransferase (for F₄₂₀ synthesis) | *Methanothermobacter marburgensis; Haloquadratum walsbyi C23; Methanobrevibacter ruminantium M1; Archaeoglobus veneficus SNP6; Natronobacterium gregoryi SP2; Methanosalsum zhilinae DSM 4017; Methanoplanus petrolearius; Methanolinea tarda NOBI-1;* | ADL58588; CCC41432; YP_003423696; YP_004342334; ZP_08967286; AEH61444; ADN35493; EHF10295; |
| **99d:** | *Methanococcus maripaludis S2; Archaeoglobus veneficus SNP6; Methanospirillum hungatei JF-1; Methanococcus maripaludis XI; Methanocella paludicola SANAE; Methanosphaera stadtmanae; Methanopyrus kandleri AV19; Methanoculleus marisnigri JR1 ; Methanosaeta harundinacea 6Ac; Methanocorpusculum labreanum Z; Methanococcus maripaludis S2;* | NP_987524; YP_004341066; YP_503864; YP_004742044; YP_003356970; YP_448417; NP_614772; YP_001048050; AET64321; YP_001029596; CAF29960; |
| CofD: LPPG:Fo 2-phospho-L-lactate transferase (for F₄₂₀ synthesis) | | |
| **99e:** | *Methanothermobacter thermautotrophicus; Methanocorpusculum labreanum Z;* | NP_276154; |
| CofE: F₄₂₀-0: gamma-glutamyl ligase | | YP_001030766; |
| (for F₄₂₀ synthesis) | *Methanothermus fervidus DSM 2088; Methanohalophilus mahii DSM 5219; Mycobacterium sp. Spyr1; Halogeometricum borinquense; Methanococcus maripaludis C5; Methanosarcina barkeri str. Fusaro; Methanocorpusculum labreanum Z; Methanococcoides burtonii DSM 6242; Methanoculleus marisnigri JR1 ; Methanosaeta thermophila PT; Acidothermus cellulolyticus 11B* | YP_004003885; ADE37403; YP_004078486; YP_004035572; ABO35054; YP_305815; YP_001030766; YP_566482; ABN57125; ABK13958; ABK53734; |
| **99f:** | *Methanobrevibacter ruminantium M1; Methanococcus maripaludis S2; Methanosphaera stadtmanae; Methanocella paludicola SANAE; Methanopyrus kandleri AV19; Synechococcus sp. PCC 7002; Cyanothece sp. PCC 7425; Synechococcus elongatus PCC 7942; Synechocystis sp. PCC 6803 Synechococcus elongatus PCC 7942; Synechocystis sp. PCC 6803; Thermosynechococcus elongatus BP-1; Cyanothece sp. ATCC 51472; Methanosphaera stadtmanae; Methanococcus maripaludis S2; Methanobrevibacter ruminantium M1, Methanosarcina mazei Go1; Methanocella paludicola SANAE;* | YP_003424716 (cofG); CAF30432 (cofG); YP_447349 (cofG) YP_003357513 (cofG); NP_614181 (cofG); YP_001734664 (cofG); YP_002481576 (cofG); ABB56922 (cofG); NP_440537 (cofG) YP_399705 (cofH); NP_440146 (cofH); NP_682387 (cofH); EHC24992 (cofH); ABC56793 (cofH); NP_987177 (cofH); YP_003424008 (cofH); NP_634520 (cofH); YP_003357511 (cofH); |
| CofGH: Fo synthase (for F₄₂₀ synthesis) | | |
| **100:** | *Methanocella paludicola SANAE; Methanobrevibacter ruminantium M1; Thermococcus gammatolerans EJ3; Halobacterium salinarum R1; Methanothermobacter marburgensis; Thermococcus gammatolerans EJ3; Haloferax volcanii DS2;* | YP_003355454; YP_003424638; YP_002960503; YP_001688512; ADL59079; ACS34639; YP_003534871; |
| Pyridoxal phosphate-dependent L-tyrosine decarboxylase (mfnA for methanofuran synthesis) | | |
| **101a:** | *Methanosphaera stadtmanae; Methanobrevibacter ruminantium M1; Methanococcus maripaludis S2; Pyrococcus horikoshii OT3; Thermococcus gammatolerans EJ3; Methanosarcina mazei Go1; Methanospirillum hungatei JF-1; Thermococcus kodakarensis KOD1; Methanopyrus kandleri AV19; Methanosarcina acetivorans C2A; Methanocaldococcus fervens AG86; Methanoregula boonei 6A8; Methanothermobacter thermautotrophicus;* | YP_447347; YP_003424704; NP_987154; NP_143623; YP_002959796; NP_633246; YP_503757; YP_183206; NP_613770; NP_619377; YP_003128348; YP_001403641; NP_276324; |
| MptA: GTP cyclohydrolase (for Methanopterin synthesis) | | |
| | *Methanosarcina barkeri str. Fusaro; Methanocaldococcus_jannaschii;* | YP_304731; NP_247760; |
| **101b:** | *Methanococcus maripaludis C5; Roseobacter denitrificans OCh 114; Arabidopsis thaliana; Zea mays; Medicago truncatula;* | ABO35741; YP_683148; AEE84108; NP_001151923; XP_003629873; |
| MptB: Cyclic phosphodiesterase (for Methanopterin synthesis) | | |
| **101c:** | *Methanothermus fervidus DSM 2088; Methanocella paludicola SANAE; Methanoplanus petrolearius;* | YP_004003771; YP_003356610; ADN37264; |
| RFAP: | | |
| Ribofuranosylaminobenzene 5'-phosphate synthase (for Methanopterin synthesis) | *Methanobrevibacter ruminantium M1; Archaeoglobus veneficus SNP6; Thermococcus sp. AM4; Methanococcus maripaludis S2; Methanothermus fervidus DSM 2088; Methanocella paludicola SANAE;* | YP_003424432; YP_004342012; YP_002582695; NP_987399; ADP77009; BAI61627; |
| **102a:** | *Methanothermobacter marburgensis; Methanococcus maripaludis S2; Methanosphaera stadtmanae; Methanothermus fervidus DSM 2088; Methanothermococcus okinawensis IH1; Methanobacterium sp. SWAN-1; Methanocaldococcus fervens AG86; Methanococcus voltae A3; Methanococcus maripaludis C6; Methanobacterium sp. AL-21; Methanococcus aeolicus Nankai-3; Methanotorris igneus Kol 5; Methanobacterium sp. AL-21 Methanococcus maripaludis X1; Methanocaldococcus infernus ME; Methanocaldococcus sp. FS406-22;* | ADL57861; NP_987393; ABC57647; YP_004004617; YP_004575938; YP_004519242; YP_003127444; ADI36986; YP_001548728; YP_004291430; YP_001324357; AEF96400; ADZ10458; AEK19167; ADG13665; YP_003457919; |
| ComA: Phosphosulfolactate synthase (for Coenzyme M synthesis) | | |
| **102b:** | *Methanococcus maripaludis S2; Methanopyrus kandleri AV19; Methanothermobacter marburgensis; Methanococcus maripaludis S2; Methanocella paludicola SANAE; Methanothermus fervidus DSM 2088; Methanothermus fervidus DSM 2088; Methanobacterium sp. AL-21; Methanobrevibacter ruminantium M1; Synechocystis sp. PCC 6803; Synechococcus sp. JA-2-3B'a(2-13); Synechococcus sp. PCC 7002; Synechococcus sp. WH 7803; Cyanothece sp. ATCC 51472; Synechococcus sp. WH 8016;* | NP_987281; AAM01355; YP_003850451; CAF29717; YP_003357619 YP_004004784; ADP78022; YP_004289567; YP_003424691; BAK50080; YP_476548; YP_001735079; YP_001224757; EHC21417; ZP_08955317; |
| ComB: 2-Phosphosulfolactate phosphatase (for Coenzyme M synthesis) | | |
| **102c:** | *Methanothermobacter marburgensis; Methanosphaera stadtmanae; Methanothermobacter marburgensis; Methanothermus fervidus DSM 2088; Roseobacter litoralis Och 149; Methanococcus maripaludis C5; Methanothermus fervidus DSM 2088;* | ADL59162; ABC56689; YP_003850475; YP_004003953; YP_004689622; ABO34766; ADP77191; |
| ComC: Sulfolactate dehydrogenase (for Coenzyme M synthesis) | | |
| **102d:** | *Methanosarcina acetivorans C2A; Methanocella paludicola SANAE; Methanocorpusculum labreanum Z; Methanoculleus marisnigri JR1 ; Methanosarcina barkeri str. Fusaro; Methanocella paludicola SANAE; Methanosphaera stadtmanae; Methanococcus maripaludis S2;* | NP_618188; YP_003357048; YP_001029945; ABN56047; YP_306991; BAI62065; ABC56687; NP_988809; |
| ComDE: Sulfopyruvate decarboxylase (for Coenzyme M synthesis) | | |
| **102e:** | *Methanothermobacter marburgensis; Methanothermobacter thermautotrophicus* | comF; comF; |
| ComF: Sulfoacetaldehyde dehydrogenase (for Coenzyme M synthesis) | | |
| **103a:** | *Methanopyrus kandleri AV19; Methanothermobacter thermautotrophicus;* | AAM01606; AAB85956; |
| LeuA homolog: Isopropylmalate synthase | | |
| (for Coenzyme B synthesis) | *Thermoproteus tenax; Thermoplasma volcanium GSS1; Methanobrevibacter smithii; Methanosphaera stadtmanae; Methanobrevibacter ruminantium M1; Methanococcus maripaludis S2; Synechocystis sp. PCC 6803 Synechococcus elongatus PCC 7942; Cyanothece sp. ATCC 51472; Synechococcus sp. WH 8016; Synechococcus sp. JA-2-3B'a(2-13) Thermosynechococcus elongatus BP-1;* | CAF18516; NP_111428; ABQ87451; YP_447259; YP_003424897; NP_988183; NP_442009; ABB56460; EHC25498; ZP_08954784; YP_477672; NP_682187; |
| **103b:** | *Methanopyrus kandleri AV19; Methanothermobacter marburgensis; Methanothermus fervidus DSM 2088; Methanocella paludicola SANAE; Methanosphaera stadtrnanae; Methanocella paludicola SANAE; Methanococcus maripaludis S2; Synechocystis sp. PCC 6803; Synechococcus elongatus PCC 7942; Cyanothece sp. ATCC 51472; Synechococcus sp. JA-2-3B'a(2-13; Thermosynechococcus elongatus BP-1;* | NP_614498; ADL58232; YP_004004146; YP_003358048; YP_447715; BAI63065; CAF30095; NP_441348; ABB57535; EHC23198; YP_477855; NP_682390; |
| LeuB homolog: Isopropylmalate dehydrogenase (for Coenzyme B synthesis) | | |
| **103c:** | *Marinobacter adhaerens HP15; Halorhabdus tiamatea SARL4B; Haloarcula marismortui ATCC 43049; Halomicrobium mukohataei; Haladaptatus paucihalophilus DX253; Escherichia coli 0103:H2 str. 12009; Synechocystis sp. PCC 6803; Cyanothece sp. PCC 8801; Nostoc sp. PCC 7120; Synechococcus sp. JA-2-3B'a(2-13); Thermosynechococcus elongatus BP-1;* | ADP98363, ADP98362; ZP_08559069; YP_135090; YP_003178469; ZP_08045715; YP_003220086, YP_003220085; NP_442926, NP_441618; YP_002370476, YP_002373868; NP_485460, NP_485459; YP_478232, YP_476588; NP_681699, NP_682024; |
| LeuCD homolog: Isopropylmalate isomerase (for Coenzyme B synthesis) | | |
| **104:** | *Thauera butanivorans; Zymomonas mobilis; Pectobacterium wasabiae WPP163; Marinomonas mediterranea MMB-1; Glaciecola sp. 4H-3-7+YE-5; Rahnella sp. Y9602; Pseudoalteromonas sp. ND6B; Colwellia psychrerythraea; Escherichia coli KO11FL; Pectobacterium carotovorum; Dickeya dadantii Ech703; Enterobacter cloacae str. Hanford; Shewanella baltica; Sphingobium chlorophenolicum L-1; Hirschia baltica ATCC 49814; Tolumonas auensis DSM 9187; Desulfomicrobium baculatum; Enterobacter asburiae LF7a; Serratia sp.; Serratia plymuthica AS9; Brenneria sp. EniD312; Desulfovibrio africanus str. Walvis Bay; Desulfovibrio alaskensis G20; Ralstonia eutropha; Thermodesulfatator indicus;* | AY093933; AEI37129, AEH62108; ACX88367; ADZ89640; AEE22284; ADW74353; KGJ99007; KGJ96343, KGJ93164 ; ADX51149; ACT13368; ACS85518; EPR39571; AEH13482, ADT93851; AEG49721; ACT60594; ACQ91718; ACU89329; AEN65074; AEG28401, AEF50694; AEF45743; EHD21179; EGJ49660; ABB40021; AAZ64425, CAJ97131; AEH43902; |
| σ⁵⁴-transcriptional activator (BmoR) | | |
| | *Syntrophothermus lipocalidus; Rhodopirellula baltica SWK14; Thauera butanivorans;* | ADI01014, ADI01359; ELP35849, ELP32292; ABU68842; |
| **105:** | *Cloning vector pKaKa2;* | ADO63859; |
| λ-Red single-stranded DNA (ssDNA) binding protein β | *Cloning vector pKaKa1; Broad host range Red recombinase expression vector pRKcIRed;* | ADO63841; AAV68245, ACJ06678; ACJ06694, ACJ06689; |
| **106:** | *Geobacillus sp.;* | JC8061; |
| Lipase | *Pseudomonas fluorescens; Rhodococcus erythropolis; Brachybacterium tyrofermentans; Enterobacter aerogenes (K. mobilis); Enterobacter aerogenes; Pseudomonas sp. XD; Pseudomonas syringae; Proteus sp. K107; Rhizomucor miehei; Rhizopus oryzae; Paenibacilluspolymyxa CR1; Fluviicola taffensis DSM 16823; Streptomyces coelicolorA3(2); Candida Antarctica, Rhizomucor miehei; Pseudomonas sp. 7323; Pseudomonas fluorescens Pseudomonas fluorescens Thermomyces lanuginosus; Synechocystis sp. PCC 6714; Arthrospira platensis; Cyanobium sp. CACIAM 14; Nostoc sp. PCC 7107; Trichodesmium erythraeum; Thermosynechococcus elongatus BP-1; Prochlorococcus marinus; Arthrospira maxima; Auxenochlorella protothecoides; Tetraselmis sp. GSL018; Pseudomonas (Burkholderia) cepacia;* | AAU10321; ACD89059; ACD89058; KHM31672, KHM31091; WP_032716299, WP_032712977; ACD89057; ACD89056, EFW83541; ACC76759; B34959; BAG16821, ACW84344; AIW42352, YP_009097503; YP_004343248; NP_625552; 3W9B_C, CAA83122 (Nov435); 4TGL_A; CAJ76166; BAC98499, BAC98498; AAA25882 (can use butanol); ABV69592, ABV69591; AIE72887; KDR54344, WP_006623978; KEF43153; WP_006670868; WP_011611331; NP_682772; WP_032526888; AFY45335; KFM28616; JAC66493; WP_027791175,M58494, WP_034204948 (use ethanol); |
| **107:** | *Chlamydomonas reinhardtii; Chlamydomonas reinhardtii; Phaeodactylum tricornutum; Chlorella variabilis; Auxenochlorella protothecoides; Coccomyxa subellipsoidea C-169; Bryopsis plumose; Micromonas sp. RCC299; Microcoleus vaginatus FGP-2; Trichodesmium erythraeum IMS101; Prochlorococcus marinus; Synechococcus sp. WH 7805; Synechocystis sp. PCC 6803; Synechococcus elongatus PCC 7942; Arthrospira platensis;* | XP_001700395, XP_001698887; EDP03875, XP_001692397; EEC44787, XP_002183604; XP_005851371; KFM28220,EFN59119; XP_005652245; BAI43481; ACO60738; EGK87709, EGK89095; YP_721603, YP_720732; WP_011863058, WP_011376568; EAR19866, BAA17432, P73456, NP_442714; ABB58124, ABB56302; WP_006623132, WP_014273948, WP_014275283, EKD10810; YP_005067833; WP_011058259, WP_011056416; NP_681159, BAC07773; EDO99169, AAW67003; ELP31779; |
| Cell surface protein | | |
| | *Arthrospira platensis NIES-39; Thermosynechococcus elongatus; Thermosynechococcus elongatus BP-1; Chlamydomonas reinhardtii; Rhodopirellula baltica SWK14;* | |
| | *Phaeodactylum tricornutum; Phaeodactylum tricornutum; Ostreococcus tauri; Auxenochlorella protothecoides; Bathycoccus prasinos; Methanosarcina mazei Tuc01; Methanosarcina barkeri; Mycoplasma bovis; Streptococcus salivarius; Planktothrix agardhii NIVA-CYA 126*/*8; Arthrospira sp. PCC 8005; Synechococcus sp. JA-2-3B'a(2-13); Spirulina subsalsa; Aphanizomenon flos-aquae; Synechococcus sp. JA-3-3Ab; Coleofasciculus chthonoplastes; Lyngbya sp. PCC 8106; Aphanizomenon flos-aquae; Methanosarcina barkeri str. Fusaro;* | EEC44786, EEC44540; ACI65532, XP_002186062; XP_003083807, CEF97038; KFM28329, KFM23052; XP_007508599, CCO20216; YP_007491104; WP_011307725, AAZ71684; ADR25157 (polyamine ABC) AEJ52906 (polyamine); KEI65453 (polyamine ABC); CDM95113 (polyamine ABC; ABD03026 (polyamine ABC); WP_033374023 (polyamine ABC) KHG40085 (polyamine ABC); ABD00934 (polyamine); EDX75495 (polyamine ABC); EAW39210 (polyamine); KHG39958 (polyamine ABC); YP_306783, YP_306264; |
| **108:** | *Rhodnius prolixus; Trichomonas vaginalis G3; Trichomonas vaginalis G3; Tobacco mosaic virus;* | AAQ20830 (polylysine); XP_001301096 (polylysine); XP_001291750 (polylysine); NP_597749; |
| Positively charged polypeptide | | |
| **108:** | *Saccharomyces cerevisiae; Scheffersomyces stipites; Kluyveromyces lactis, Acetobacter pasteurianus; Brettanomyces bruxellensis AWRI1499; Lachancea kluyveri; Rhodosporidium toruloides NP11; Wickerhamomyces anomalus; Pseudozyma brasiliensis GHG001; Kluyveromyces marxianus; Ogataea parapolymorpha DL-1; Pseudozyma hubeiensis SY62; Histoplasma capsulatum G186AR; Cyberlindnera jadinii; Hanseniaspora uvarum; Zymomonas mobilis; Cyanobacterium aponinum PCC 10605; Microcystis aeruginosa; Synechococcus sp. WH 8016; Crocosphaera watsonii WH 0401; Crocosphaera watsonii; Auxenochlorella protothecoides; Coccomyxa subellipsoidea C-169;* | CAA39398, CAA54522; EAZ63682, EAZ63546; CAA59953; AAM21208; EIF49850; AAP75899, AAP75898; EMS25670; CAH56494; EST04586; BAO41366; ESW98764; GAC99677; EEH10009, EEH04743; BAI23188; AAA85103; AFN57569; AFZ53994; GAL91844, WP_002787689; EHA63558; CCQ62787, WP_021836129; WP_007303683, EAM53387; KFM24239; XP_005643654, EIE19110; |
| Pyruvate Decarboxylase | | |
| **109:** | *Candida boidinii; Komagataella pastoris; Methylophaga thiooxydans; Methylophaga nitratireducenticrescens; Mycobacterium vaccae; Hyphomicrobium nitrativorans NL23; Escherichia coli; Vibrio tubiashii ATCC 19109; Cedecea neteri; Klebsiella pneumonia; Pluralibacter gergoviae; Enterobacter sp. R4-368; Citrobacter braakii;* | CAA09466, O13437; BAH57505; KGM07232, KGM07233; AFI83744, AFI83745; BAB69476; AHB48368; AAA23754; AIW15822; AIR05429, KHE39131; AIW99319, KHE25480; AIR00162; YP_008107767; KHE12739, KHE08171; |
| Formate dehydrogenase | | |
| | *Citrobacter freundii; Enterobacter cloacae; Prochlorococcus sp. scB245a_518D8; Mastigocoleus testarum; Cyanothece sp. PCC 8802; Nostoc punctiforme; Trichodesmium erythraeum; Thermodesulfatator indicus; Bacillus subtilis; Sulfobacillus acidophilus DSM 10332; Syntrophothermus lipocalidus; Hydrogenobacter thermophilus TK-6;* | KGY88354; KGY63138; WP_025936369; WP_027846123; WP_015783314; WP_012407412; WP_011610267; AEH45674, AEH46025; KFC29810; AEW04854; ADI02197; BAI69388, YP_003432590; |
| **110**: | *Sulfobacillus acidophilus DSM 10332; Geobacillus thermoglucosidans; Geobacillus sp. GHH01; Rhodopirellula baltica SH 1; Arthrobacter chlorophenolicus A6; Burkholderia multivorans; Granulicella mallensis MP5ACTX8; Arthrobacter sp. PAMC25486; Pseudomonas putida; Rhodospirillum rubrum ATCC 11170; Bacillus sp. BSC154; Bacillus subtilis BEST7613; Hyphomicrobium zavarzinii; Halopiger xanaduensis SH-6; Thalassotalea sp. ND16A; Brevibacillus brevis; Pseudomonas putida; Halalkalicoccus jeotgali B3; Pseudomonas sp. UW4; Candidatus Halobonum tyrrellensis; Pseudomonas plecoglossicida NB2011; Halococcus thailandensis JCM 13552; Halococcus salifodinae DSM 8989; Halococcus saccharolyticus DSM 5350; Haloarcula japonica DSM 6131; Haloferax denitrificans ATCC 35960; Haloferax sulfurifontis ATCC BAA-897; Haloferax alexandrinus JCM 10717; Natrialba aegyptia DSM 13077; Burkholderia sp. SJ98; Burkholderia terrae BS001; Pseudomonas fluorescens A506; Haloquadratum walsbyi C23; Natrialba taiwanensis; Escherichia coli; Synechocystis sp. PCC 6803; Synechococcus elongatus PCC 7942; Arthrospira platensis; Amycolatopsis methanolica 239; Mycobacterium marinum MB2;* | AEW04442, YP_005256114; EID43710; AGE20787; NP_864907; YP_002489735, YP_002487096; YP_001583515; YP_005060061; AIY02235; AHZ74859, YP_004701746; YP_428486; KFI04000; BAM50883; CAC85637; YP_004595370, AEH35491; KGK00333; AEM59539; BAA04743, YP_007227278; YP_003736860, ADJ15068; AFY22196; ESP87727; EPB95789; EMA53947; EMA52447; EMA44468; EMA27812; EMA06490; ELZ96420; ELZ95747; ELZ03549; EKS67251; EIM95920; YP_006326331; YP_005841059; WP_006824465; BAA22412; NP_440484; ABB56491; YP_005071621; AIJ21411; EPQ77120; |
| Formaldehyde dehydrogenase | | |
| **111:** | *Escherichia coli; Cloning vector pRK7813; Cloning vector pKS800; Klebsiella pneumonia; Corynebacterium glutamicum; Thermococcus gammatolerans EJ3; Streptomyces coelicolorA3(2); Streptococcus pneumoniae Taiwan;* | P02981; AGF38340; BAJ06605; YP_008997867, AHF45941; NP_052571, AAD25063; YP_002958733, YP_002959779; CAC14348, NP_625085; ACO23503; |
| Tetracycline resistance protein (tetA) | | |
| | *Vibrio cholerae O1 biovar El Tor; Burkholderia glumae PG1; Rhizobium etli bv. phaseoli str. IE4803;* | BAG66128; AJK48844; AJC80365; |
| **112:** | *Campylobacter jejuni; Cloning vector pIMK; yfp marker plasmid pWM1011; cfp marker plasmid pWM1009; synthetic construct; cfp marker plasmid pWM1012; Cloning vector pNIGEL19;* | CAD35325; CAP74563; AAG34047; AAG34043; CAA05684; AAG34051; ACO48265; |
| Kanamycin resistance protein (Kan^{r}) | | |
| **113:** | *Bacillus cereus; Cloning vector pCR2.1-gentR; Cloning vector pTH1522; Cloning vector pJC8; Plasmid R; Enterococcus gallinarum; Transposon delivery vector pZXL5; Bacillus thuringiensis Bt407; Baculovirus expression vector pFastBacl-HM;* | WP_016513111, EOP95255; ABS71237; ABC47322; AGA63621; AAA19915; AAB49832; AFD97621; YP_006926052; AAT09789; |
| Gentamicin resistance protein | | |
| **114:** | *Streptomyces netropsis Streptomyces spectabilis; Staphylococcus aureus; Cloning vector pCDF-J23100-TetO-lacI-LVA; synthetic vector pCDF*-*MCS; Delivery vector pDGIEF; Delivery vector pIEF16S; Listeria innocua; Micromonospora sp. ATCC 39149; Binary vector pOSCAR; Suicide vector pMRKO1; Transposon delivery vector pAW068; Shuttle vector pMTL83353; Cloning vector pFW GFP;* | AAB66654; AAF63341; KII21062; AIO11020; AIS22788; ABC88414; ABC88419; YP_008119852, AGN12845; EEP72249; ADR73032; ADV78249; ABV70028; ACR43899; AAC53685; |
| Spectinomycin resistance protein (Spcr) | | |
| **114:** | *Streptococcus agalactiae; Klebsiella pneumonia; Integration vector pZR606; Escherichia coli Cloning vector pBAMD1-4;; Streptococcus dysgalactiae; Salmonella enterica; Staphylococcus rostri; Agrobacterium sp. H13-3; Expression vector pBS437V; Cloning vector pBS152v;* | AIK76614, AIK74539; YP_007349701, AIK72509; AHZ10952; AAO49597, ADH82152; AIX94015; BAM60369; BAK19730; CBA13544; ADY64157; AAP45701; AAG09291; |
| Streptomycin resistance protein (aadA) | | |
| **115:** | *Escherichia coli; Pseudoalteromonas haloplanktis; Bacillus weihenstephanensis; Bacillus subtilis MB73*/*2; Cloning vector pUG7; Expression vector pTip-CH2; PCR template vector pUG6; Cloning vector pCAT-Mgen-recA; Recombinase expression vector pSH47; Baculovirus expression vector pRADM; Shuttle vector pNR-46121; synthetic vector pET-T7p(-12T)-SpacerA-GFP-LYA;* | YP_006952162, YP_006952158; EGI75151; AIW88365; EME04863; AAG34548; BAD11229; AAG34544; AFM47375; AAG34516; AEY64193; AIL56504 AIS22810; |
| Ampicillin resistance protein (*amp^{r}*) | | |
| **116:** | *Corynebacterium glutamicum; Cloning vector pDEST414CYC1t7 ;* | NP_044444, AAB46601; AGL80176; |
| Chloramphenicol resistance | | |
| protein (CmR) | *Cloning vector pIPKTA30N; Cloning vector GWluc-basic; Pantoea ananatis LMG 20103; Yeast expression vector pJG518; Binary vector pEAQ-HT-DEST3; Binary vector pIPKb009; expression vector pUCDMIG Plant transient expression vector;* | ABQ65200; ABO76903; ADD75805; AAX94717; ACV49954; ABW70109; AEY64192; AFX83551, |
| **117:** | *Klebsiella pneumonia; Cloning vector pMQ351 Cloning vector pNIGEL17; Salmonella enterica; Cloning vector pMQ300; BiFC vector pCHGC155;* | YP_007349547, CCN79980; ADQ43406; ACO48271; AIT72690; ADK63421; AGH62558; |
| Hygromycin resistance protein (hph) | | |
| **118:** | *Agrobacterium sp. CP4; synthetic construct; Escherichia coli K-12; Amaranthus tuberculatus; Amaranthus palmeri; Gluconobacter frateurii NBRC 103465; Pseudomonas sp. PG2982; Cloning vector pAM3G; Vector mini-Tn7-gat; Cloning vector pwFRT-GSr; Cloning vector pFlp-AB7;* | Q9R4E4 (CP4 EPSP synthase); AII80555, AEM75108 (CP4); P0A6D3 (epsps); ACV67278, ACV67277 (epsps); ACV53022, ACV53021 (epsps); GAD08994; P18896; AD063834; ACX31712; ACJ70055; ACJ70061; |
| Glyphosate resistance protein (epsps; or, glyphosate acetyl transferase (Gat)) | | |
| **119:** | *Thermosynechococcus elongatus BP-1; Arthrospira platensis; Spirulina subsalsa; Arthrospira maxima CS-328; Synechocystis sp. PCC 6803; Synechococcus elongatus PCC 7942; Synechococcus sp. PCC 7002; Nostoc sp. PCC 7107; Cyanobacterium aponinum; Anabaena cylindrica PCC 7122; Synechococcus sp. WH 8016; Prochlorococcus marinus; Chlamydomonas reinhardtii Chlorella variabilis Auxenochlorella protothecoides; Bathycoccus prasinos; Shewanella algae; Emiliania huxleyi CCMP1516 Thalassiosira pseudonana CCMP1335 Ralstonia eutropha H16; Ralstonia eutropha JMP134; Zymomonas mobilis Syntrophothermus lipocalidus; Hippea maritima DSM 10411; Sulfobacillus acidophilus DSM 10332; Thermovirga lienii DSM 17291; Thermodesulfatator indicus; Geobacillus thermoleovorans; Caldicellulosiruptor kristjanssonii; Thermus sp. CCB_US3_UF1; Caldicellulosiruptor hydrothermalis; Melioribacter roseus P3M-2; Caldicellulosiruptor saccharolyticus; Bacillus methanolicus MGA3; Spirochaeta thermophila;* | NP_681156, BAC07918; YP_005067487, BAI88949; WP_017306995; EDZ94142; NP_440604, WP_010871913; ABB58505; ACB00465; YP_007051699; AFZ52419; AFZ58080; EHA59113; WP_032525717, WP_032523803; CAA34615, CAA09001; EFN52305, XP_005844407; KFM28402; XP_007510956; WP_025011590; EOD30692, XP_005783121; EED90549; CAJ94001, Q0K7M0; AAZ63178, AAZ60077 ; WP_023593629, WP_015740233; ADI02913; AEA33832; AEW04357; AER66275; AEH45167; AEV20403; ADQ40738; AEV17054; ADQ07194; AFN74157; ABP67398; AIE61023; ADN02278; |
| Argininosuccinate lyase (arg7) | | |
| | *Hydrogenobacter thermophilus TK-6; Thermotoga petrophila RKU-1; Geobacillus sp. JF8; Bacillus subtilis; Bacillus coagulans 2-6; Anoxybacillus sp. BCO1; Thermus sp. CCB_US3_UF1; Thermoanaerobacter sp. YS13; Methanothermobacter marburgensis; Methanothermobacter thermautotrophicus; Methanocella conradii HZ254; Methanoregula formicica SMSP; Methanococcus maripaludis S2; Methanococcoides methylutens; Methanococcus maripaludis OS7; Methanococcus maripaludis KA1; Methanococcus maripaludis S2; Bacillus methanolicus MGA3; Candida maltosa Xu316; Clostridium pasteurianum NRRL B-598; Nostoc sp. PCC 7120 (Anabaena sp. PCC 7120);* | YP_003433478, BAI70277 ; YP_001244661; AGT33097; KFC31226; AEH53530; KHF26873; AEV16454; KHO61828; WP_013295538, ADL58314 ; BAM69481, AAB84775; YP_005379810, AFC99291; AGB03410; NP_987133; KGK97821; BAP62099; BAP60130; CAF29569; EIJ84201, EIJ79262; EMG47575; ETD70657; NP_487927, BAB75586, WP 010998028; |
| **120:** | *Thermosynechococcus elongatus BP-1; Synechocystis sp. PCC 6803; Synechococcus elongatus PCC 7942; Arthrospira platensis; Arthrospira platensis str. Paraca; Arthrospira sp. PCC 8005; Cyanothece sp. ATCC 51142; Cyanothece sp. PCC 8801; Stanieria cyanosphaera PCC 7437; Synechococcus sp. CC9311; Synechococcus sp. JA-2-3B'a(2-13); Synechococcus sp. PCC 7002; Nostoc sp. PCC 7120; Cyanobium sp. CACIAM 14; Dolichospermum circinale; Aphanizomenon flos-aquae; Planktothrix prolifica; Pseudanabaena sp. PCC 6802; Anabaena sp. 90; Cyanothece sp. ATCC 51142; Prochlorococcus marinus; Prochlorococcus sp. scB241_528J8; Chlamydomonas reinhardtii; Shewanella algae; Chlorella vulgaris; Pseudochlorella pringsheimii; Dunaliella salina; Volvox carteri; Dunaliella viridis; Coccomyxa subellipsoidea C-169; Gracilaria tenuistipitata; Heterosigma akashiwo; Nannochloropsis sp. W2J3B; Bathycoccus prasinos; Phaeodactylum tricornutum; Cyanidioschyzon merolae strain 10D; Thalassiosira oceanica;* | NP_682145, BAC08907; AGF51177, BAA17488; CAA52675; YP_005067893, BAI89355; KDR58325; CDM94135, CCE18678; ACB50564; AAO25121; AFZ35100; ABI46326, ABI46326; ABD01041; ACA99311; NP_484656, BAB72570; KEF41492; WP_028090041; WP_027400668; WP_026796600; WP_026102963; AFW96426; ACB50564; WP_032520378; WP_025926746; EDP00805, XP_001696697; WP_025008973; ACF22999, ABP97095; AAP32278; AGC97428, AAP75705; AAA11144; AAT72293, ABJ91208; XP_005646409, EIE21865; ACX31653, ACX31652; ACS44801; AET85052; XP_007514576; AAV66996; XP_005535839; EJK46860; |
| Nitrate reductase | | |
| | *Ralstonia eutropha H16; Burkholderia sp. K24; Bacillus thuringiensis serovar; Methylomonas denitrificans; Marinithermus hydrothermalis; Bacillus subtilis; Geobacillus sp. JF8; Thermodesulfatator indicus; Caldicellulosiruptor hydrothermalis; Thermovirga lienii DSM 17291; Caldicellulosiruptor kronotskyensis; Desulfovibrio vulgaris str. 'Miyazaki F'; Desulfovibrio sp. FW1012B; Thiorhodococcus drewsii AZ1; Thiocapsa marina 5811; Hyphomicrobium nitrativorans NL23; Ogataea angusta; Rhodotorula glutinis ATCC 204091; Wickerhamomyces anomalus; Blastobotrys adeninivorans; Ogataea angusta;* | CAA50508; KFX64654; EXL37784; KHD30476; AEB12539; KFC29867; AGT32146, AGT31099; AEH45676; ADQ07227; AER66908; ADQ46341; ACL08432; EHJ47733; EGV31372; EGV16886; AHB47344; CAA11232; EGU11696; AAF28059; CAQ77148; CAA88925; |

### Designer Calvin-Cycle-Channeled 1-Butanol Producing Pathways

According to one of the various embodiments, a designer Calvin-cycle-channeled pathway is created that takes the Calvin-cycle intermediate product, 3-phosphoglycerate, and converts it into 1-butanol by using, for example, a set of enzymes consisting of (as shown with the numerical labels **34, 35, 03-05, 36-43** in Figure 4): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** citramalate synthase **36,** 2-methylmalate dehydratase **37,** 3-isopropylmalate dehydratase **38,** 3-isopropylmalate dehydrogenase **39,** 2-isopropylmalate synthase **40,** isopropylmalate isomerase **41,** 2-keto acid decarboxylase **42,** and alcohol dehydrogenase (NAD dependent) **43.** In this pathway design, as mentioned above, the NADPH-dependent glyceraldehyde-3-phosphate dehydrogenase **34** and NAD-dependent glyceraldehyde-3-phosphate dehydrogenase **35** serve as a NADPH/NADH conversion mechanism that can covert certain amount of photosynthetically generated NADPH to NADH which can be used by the NADH-requiring alcohol dehydrogenase **43** (examples of its encoding gene with the following GenBank accession numbers: BAB59540, CAA89136, NP_148480) for production of 1-butanol by reduction of butyraldehyde.

According to one of the various embodiments, it is a preferred practice to also use an NADPH-dependent alcohol dehydrogenase **44** that can use NADPH as the source of reductant so that it can help alleviate the requirement of NADH supply for enhanced photobiological production of butanol and other alcohols. As listed in Table 1, examples of NADPH-dependent alcohol dehydrogenase **44** include (but not limited to) the enzyme with any of the following GenBank accession numbers: YP_001211038, ZP_04573952, XP_002494014, CAY71835, NP_417484, EFC99049, and ZP_02948287.

Note, the 2-keto acid decarboxylase **42** (e.g., AAS49166, ADA65057, CAG34226, AAA35267, CAA59953, A0QBE6, A0PL16) and alcohol dehydrogenase **43** (and/or **44)** have quite broad substrate specificity. Consequently, their use can result in production of not only 1-butanol but also other alcohols such as propanol depending on the genetic and metabolic background of the host photosynthetic organisms. This is because all 2-keto acids can be converted to alcohols by the 2-keto acid decarboxylase **42** and alcohol dehydrogenase **43** (and/or **44)** owning to their broad substrate specificity. Therefore, according to another embodiment, it is a preferred practice to use a substrate-specific enzyme such as butanol dehydrogenase **12** when/if production of 1-butanol is desirable. As listed in Table 1, examples of butanol dehydrogenase **12** are NADH-dependent butanol dehydrogenase (e.g., GenBank: YP_148778, NP_561774, AAG23613, ZP_05082669, ADO12118) and/or NAD(P)H-dependent butanol dehydrogenase (e.g., NP_562172, AAA83520, EFB77036, EFF67629, ZP_06597730, EFE12215, EFC98086, ZP_05979561).

In one of the various embodiments, another designer Calvin-cycle-channeled 1-butanol production pathway is created that takes the Calvin-cycle intermediate product, 3-phosphoglycerate, and converts it into 1-butanol by using, for example, a set of enzymes consisting of (as shown with the numerical labels **34, 35, 03, 04, 45-52** and **40-43 (44/12)** in Figure 4): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** phosphoenolpyruvate carboxylase **45,** aspartate aminotransferase **46,** aspartokinase **47,** aspartate-semialdehyde dehydrogenase **48,** homoserine dehydrogenase **49,** homoserine kinase **50,** threonine synthase **51,** threonine ammonia-lyase **52,** 2-isopropylmalate synthase **40,** isopropylmalate isomerase **41,** 3-isopropylmalate dehydrogenase **39,** 2-keto acid decarboxylase **42,** and NAD-dependent alcohol dehydrogenase **43** (and/or NADPH-dependent alcohol dehydrogenase **44,** or butanol dehydrogenase **12).**

According to another embodiment, the amino-acids-metabolism-related 1-butanol production pathways [numerical labels **03-05, 36-43;** and/or **03, 04, 45-52** and **39-43 (44/12)**] can operate in combination and/or in parallel with other photobiological butanol production pathways. For example, as shown also in Figure 4, the Frctose-6-photophate-branched 1-butanol production pathway (numerical labels **13-32** and **44/12)** can operate with the parts of amino-acids-metabolism-related pathways [numerical labels **36-42,** and/or **45-52** and **40-42)** with pyruvate and/or phosphoenolpyruvate as their joining points.

Examples of designer Calvin-cycle-channeled 1-butanol production pathway genes (DNA constructs) are shown in the DNA sequence listings. SEQ ID NOS: 58-70 represent a set of designer genes for a designer nirA-promoter-controlled Calvin-cycle-channeled 1-butanol production pathway (as shown with numerical labels **34, 35, 03-05,** and **36-43** in Figure 4) in a host oxyphotobacterium such as *Thermosynechococcus elongatus* BP1. Briefly, SEQ ID NO: 58 presents example 58 of a designer nirA-promoter-controlled NADPH-dependent Glyceraldehyde-3-Phosphate Dehydrogenase **(34)** DNA construct (1417 bp) that comprises: a PCR FD primer (sequence 1-20), a 231-bp nirA promoter from *Thermosynechococcus elongatus* BP1 (21-251), an enzyme-encoding sequence (252-1277) selected/modified from the sequences of a *Staphylococcus aureus 04-02981* NADPH-dependent glyceraldehyde-3-phosphate dehydrogenase (GenBank: ADC37857), a 120-bp *rbcS* terminator from BP1 (1278-1397), and a PCR RE primer (1398-1417) at the 3' end.

SEQ ID NO: 59 presents example 59 of a designer nirA-promoter-controlled NAD-dependent glyceraldehyde-3-phosphate dehydrogenase **(35)** DNA construct (1387 bp) that comprises: a PCR FD primer (sequence 1-20), a 231-bp nirA promoter from *Thermosynechococcus elongatus* BP1 (21-251), an enzyme-encoding sequence (252-1247) selected/modified from the sequences of an *Edwardsiella tarda FL6-60* NAD-dependent glyceraldehyde-3-phosphate dehydrogenase (GenBank: ADM41489), a 120-bp *rbcS* terminator from BP1 (1248-1367), and a PCR RE primer (1368-1387) at the 3' end.

SEQ ID NO: 60 presents example 60 of a designer nirA-promoter-controlled Phosphoglycerate Mutase **(03)** DNA construct (1627 bp) that includes a PCR FD primer (sequence 1-20), a 231-bp nirA promoter from *Thermosynechococcus elongatus* BP1 (21-251), an enzyme-encoding sequence (252-1487) selected/modified from the sequences of a *Oceanithermus profundus DSM 14977* phosphoglycerate mutase (GenBank: ADR35708), a 120-bp *rbcS* terminator from BP1 (1488-1607), and a PCR RE primer (1608-1627).

SEQ ID NO: 61 presents example 61 of a designer nirA-promoter-controlled Enolase (04) DNA construct (1678 bp) that includes a PCR FD primer (sequence 1-20), a 231-bp nirA promoter from *Thermosynechococcus elongatus* BP1 (21-251), an enzyme-encoding sequence (252-1538) selected from the sequences of a *Syntrophothermus* Enolase (GenBank: ADI02602), a 120-bp *rbcS* terminator from BP1 (1539-1658), and a PCR RE primer (1659-1678).

SEQ ID NO: 62 presents example 62 of a designer nirA-promoter-controlled Pyruvate Kinase **(05)** DNA construct (2137 bp) that includes a PCR FD primer (sequence 1-20), a 231-bp nirA promoter from *Thermosynechococcus elongatus* BP1 (21-251), an enzyme-encoding sequence (252-1997) selected from the sequences of a *Syntrophothermus lipocalidus* pyruvate kinase (GenBank: ADI02459), a 120-bp *rbcS* terminator from BP1 (1998-2117), and a PCR RE primer (2118-2137).

SEQ ID NO: 63 presents example 63 of a designer *nirA*-promoter-controlled Citramalate Synthase **(36)** DNA construct (2163 bp) that includes a PCR FD primer (sequence 1-20), a 305-bp nirA promoter (21-325), an enzyme-encoding sequence (326-1909) selected and modified from *Hydrogenobacter thermophilus TK-6* citramalate synthase (YP_003433013), a 234-bp *rbcS* terminator from BP1 (1910-2143), and a PCR RE primer (2144-2163).

SEQ ID NO: 64 presents example 64 of a designer nirA-promoter-controlled 3-Isopropylmalate/(R)-2-Methylmalate Dehydratase **(37)** DNA construct (2878 bp) consisting of a PCR FD primer (sequence 1-20), a 231-bp nirA promoter from *Thermosynechococcus elongatus* BP1 (21-251), a 3-isopropylmalate/(R)-2-methylmalate dehydratase large subunit-encoding sequence (252-2012) selected/modified from the sequences of an *Eubacterium 3-*isopropylmalate / (R)-2-methylmalate dehydratase large subunit (YP_002930810), a 231-bp nirA promoter from *Thermosynechococcus* (2013-2243), a 3-isopropylmalate/(R)-2-methylmalate dehydratase small subunit-encoding sequence (2244-2738) selected/modified from the sequences of an *Eubacterium* 3-isopropylmalate/(R)-2-methylmalate dehydratase small subunit (YP_002930809), a 120-bp rbcS terminator from BP1 (2739-2858), and a PCR RE primer (2859-2878).

SEQ ID NO: 65 presents example 65 of a designer nirA-promoter-controlled 3-Isopropylmalate Dehydratase **(38)** DNA construct (2380 bp) comprises: a PCR FD primer (sequence 1-20), a 231-bp nirA promoter from *Thermosynechococcus elongatus* BP1 (21-251), a 3-isopropylmalate dehydratase large subunit-encoding sequence (252-1508) selected/modified from the sequences of a *Thermotoga petrophila* 3-isopropylmalate dehydratase large subunit (ABQ46641), a 231-bp nirA promoter from *Thermosynechococcus elongatus* BP1 (1509-1739), a 3-isopropylmalate dehydratase small subunit-encoding sequence (1740-2240) selected/modified from the sequences of a *Thermotoga* 3-isopropylmalate dehydratase small subunit (ABQ46640), a 120-bp *rbcS* terminator from BP1 (2241-2360), and a PCR RE primer (2361-2380).

SEQ ID NO: 66 presents example 66 of a designer nirA-promoter-controlled 3-Isopropylmalate Dehydrogenase **(39)** DNA construct (1456 bp) consisting of: a PCR FD primer (1-20), a 231-bp nirA promoter from *Thermosynechococcus elongatus* BP1 (21-251), a 3-isopropylmalate dehydrogenase -encoding sequence (252-1316) selected from the sequences of a *Thermotoga* 3-isopropylmalate dehydrogenase (GenBank: CP000702 Region 349983..351047), a 120-bp *rbcS* terminator from BP1 (1317-1436), and a PCR RE primer (1437-1456).

SEQ ID NO: 67 presents example 67 of a designer nirA-promoter-controlled 2-Isopropylmalate Synthase **(40,** EC 4.1.3.12) DNA construct (1933 bp) consisting of: a PCR FD primer (sequence 1-20), a 231-bp nirA promoter from *Thermosynechococcus elongatus (21-*251), an enzyme-encoding sequence (252-1793) selected/modified from the sequences of a *Thermotoga petrophila* 3-isopropylmalate dehydrogenase (CP000702 Region: 352811..354352), a 120-bp *rbcS* terminator from BP1 (1794-1913), and a PCR RE primer (1914-1933).

SEQ ID NO: 68 presents example 68 of a designer nirA-promoter-controlled Isopropylmalate Isomerase **(41)** DNA construct (2632 bp) comprises: a PCR FD primer (sequence 1-20), a 231-bp *nirA* promoter from *Thermosynechococcus elongatus* BP1 (21-251), a isopropylmalate isomerase large subunit-encoding sequence (252-1667) selected/modified from the sequences of a *Geobacillus kaustophilus* 3- isopropylmalate isomerase large subunit (YP_148509), a 231-bp nirA promoter from *Thermosynechococcus* (1668-1898), a isopropylmalate isomerase small subunit-encoding sequence (1899-2492) selected from the sequences of a *Geobacillus kaustophilus* isopropylmalate isomerase small subunit (YP_148508), a 120-bp *rbcS* terminator from BP1 (2493-2612), and a PCR RE primer (2613-2632).

SEQ ID NO: 69 presents example 69 of a designer nirA-promoter-controlled 2-Keto Acid Decarboxylase **(42)** DNA construct (2035 bp) consisting of: a PCR FD primer (sequence 1-20), a 231-bp *nirA* promoter from *Thermosynechococcus elongatus* BP1 (21-251), a 2-keto acid decarboxylase-encoding sequence (252-1895) selected/modified from the sequences of a *Lactococcus lactis* branched-chain alpha-ketoacid decarboxylase (AAS49166), a 120-bp *rbcS* terminator from BP1 (1896-2015), and a PCR RE primer (2016-2035) at the 3' end.

SEQ ID NO: 70 presents example 70 of a designer nirA-promoter-controlled NAD-dependent Alcohol Dehydrogenase **(43)** DNA construct (1426 bp) consisting of: a PCR FD primer (sequence 1-20), a 231-bp *nirA* promoter from *Thermosynechococcus elongatus* BP1 (21-251), an enzyme-encoding sequence (252-1286) selected/modified from the sequences of an *Aeropyrum pernix K1* NAD-dependent alcohol dehydrogenase (NP_148480), a 120-bp *rbcS* terminator from BP1 (1287-1406), and a PCR RE primer (1407-1426).

As mentioned before, use of an NADPH-dependent alcohol dehydrogenase **44** that can use NADPH as the source of reductant can help alleviate the requirement of NADH supply for enhanced photobiological production of butanol and other alcohols. SEQ ID NO: 71 presents example 71 of a designer nirA-promoter-controlled NADPH-dependent Alcohol Dehydrogenase **(44)** DNA construct (1468 bp) that comprises: a PCR FD primer (sequence 1-20), a 231-bp nirA promoter from *Thermosynechococcus elongatus* BP1 (21-251), an enzyme-encoding sequence (252-1328) selected from the sequences of a *Pichia pastoris* NADPH-dependent medium chain alcohol dehydrogenase with broad substrate specificity (XP_002494014), a 120-bp *rbcS* terminator from BP1 (1329-1458), and a PCR RE primer (1459-1468) at the 3' end. In one of the examples, this type of NADPH-dependent alcohol dehydrogenase gene (SEQ ID NO: 71) is also used in construction of Calvin-cycle-channeled butanol production pathway.

However, because of the broad substrate specificity of the 2-keto acid decarboxylase (42, SEQ ID NO: 69) and the alcohol dehydrogenase **(43,** SEQ ID NO: 70; or **44,** SEQ ID NO: 71), the pathway expressed with designer genes of SEQ ID NO: 69 and SEQ ID NO: 71 (and/or SEQ ID NO: 70) can result in the production of alcohol mixtures rather than single alcohols since all 2-keto acids can be converted to alcohols by the two broad substrate specificity enzymes. Therefore, to improve the specificity for 1-butanol production, it is a preferred practice to use a more substrate-specific butanol dehydrogenase **12.** SEQ ID NO: 72 presents example 72 of a designer nirA-promoter-controlled NADH-dependent Butanol Dehydrogenase (**12**a) DNA construct (1555 bp) that includes a PCR FD primer (sequence 1-20), a 231-bp nirA promoter from *Thermosynechococcus elongatus* BP1 (21-251), an enzyme-encoding sequence (252-1415) selected/modified from the sequences of a *Geobacillus kaustophilus* NADH-dependent butanol dehydrogenase (YP_148778), a 120-bp *rbcS* terminator from BP1 (1416-1535), and a PCR RE primer (1536-1555) at the 3' end.

SEQ ID NO: 73 presents example 73 of a designer nirA-promoter-controlled NADPH-dependent Butanol Dehydrogenase (**12**b) DNA construct (1558 bp) consisting of a PCR FD primer (sequence 1-20), a 231-bp nirA promoter from *Thermosynechococcus elongatus* BP1 (21-251), a NADPH-dependent butanol dehydrogenase -encoding sequence (252-1418) selected/modified from the sequences of a *Clostridium perfringens* NADPH-dependent butanol dehydrogenase (NP_562172), a 120-bp *rbcS* terminator from BP1 (1419-1528), and a PCR RE primer (1529-1558) at the 3' end.

Use of SEQ ID NOS: 72 and/or 73 (12a and/or 12b) along with SEQ ID NOS: 58-69 represents a specific Calvin-cycle-channeled 1-butanol production pathway numerically labeled as **34, 35, 03-05, 36-42** and **12** in Figure 4.

SEQ ID NOS: 74-81 represent an alternative (amino acids metabolism-related) pathway (**45-52** in Figure 4) that branches from the point of phosphoenolpyruvate and merges at the point of 2-ketobutyrate in the Calvin-cycle-channeled 1-butanol production pathway. Briefly, SEQ ID NO: 74 presents example 74 of a designer nirA-promoter-controlled Phosphoenolpyruvate Carboxylase **(45)** DNA construct (3646 bp) consisting of: a PCR FD primer (sequence 1-20), a 231-bp nirA promoter from *Thermosynechococcus elongatus* BP1 (21-251), an enzyme-encoding sequence (252-3506) selected/modified from the sequences of a *Thermaerobacter subterraneus DSM 13965* Phosphoenolpyruvate carboxylase (EFR61439), a 120-bp *rbcS* terminator from BP1 (3507-3626), and a PCR RE primer (3627-3646) at the 3' end.

SEQ ID NO: 75 presents example 75 of a designer nirA-promoter-controlled Aspartate Aminotransferase **(46)** DNA construct (1591 bp) that includes a PCR FD primer (sequence 1-20), a 231-bp nirA promoter from *Thermosynechococcus elongatus* BP1 (21-251), an enzyme-encoding sequence (252-1451) selected/modified from the sequences of a *Thermotoga lettingae* aspartate aminotransferase (YP_001470126), a 120-bp *rbcS* terminator from BP1 (1452-1471), and a PCR RE primer (1472-1591).

SEQ ID NO: 76 presents example 76 of a designer nirA-promoter-controlled Aspartate Kinase (47) DNA construct (1588 bp) that includes a PCR FD primer (sequence 1-20), a 231-bp nirA promoter from *Thermosynechococcus elongatus* BP1 (21-251), an enzyme-encoding sequence (252-1448) selected/modified from the sequences of a *Thermotoga lettingae* TMO aspartate kinase (YP_001470361), a 120-bp rbcS terminator from BP1 (1449-1568), and a PCR RE primer (1569-1588).

SEQ ID NO: 77 presents example 77 of a designer nirA-promoter-controlled Aspartate-Semialdehyde Dehydrogenase **(48)** DNA construct (1411 bp) that includes a PCR FD primer (sequence 1-20), a 231-bp nirA promoter from *Thermosynechococcus elongatus* BP1 (21-251), an enzyme-encoding sequence (252-1271) selected/modified from the sequences of a *Thermotoga lettingae TMO* aspartate-semialdehyde dehydrogenase (YP_001470981), a 120-bp *rbcS* terminator from BP1 (1272-1391), and a PCR RE primer (1392-1411) at the 3' end.

SEQ ID NO: 78 presents example 78 of a designer nirA-promoter-controlled Homoserine Dehydrogenase **(49)** DNA construct (1684 bp) that includes a PCR FD primer (sequence 1-20), a 231-bp nirA promoter from *Thermosynechococcus elongatus* BP1 (21-251), an enzyme-encoding sequence (252-1544) selected/modified from the sequences of a *Syntrophothermus lipocalidus* DSM 12680 homoserine dehydrogenase (ADI02231), a 120-bp *rbcS* terminator from BP1 (1545-1664), and a PCR RE primer (1665-1684) at the 3' end.

SEQ ID NO: 79 presents example 79 of a designer nirA-promoter-controlled Homoserine Kinase **(50)** DNA construct (1237 bp) that includes a PCR FD primer (sequence 1-20), a 231-bp nirA promoter from *Thermosynechococcus elongatus* BP1 (21-251), an enzyme-encoding sequence (252-1097) selected/modified from the sequences of a *Thermotoga petrophila RKU-1* Homoserine Kinase (YP_001243979), a 120-bp *rbcS* terminator from BP1 (1098-1217), and a PCR RE primer (1218-1237) at the 3' end.

SEQ ID NO: 80 presents example 80 of a designer nirA-promoter-controlled Threonine Synthase **(51)** DNA construct (1438 bp) that includes a PCR FD primer (sequence 1-20), a 231-bp nirA promoter from *Thermosynechococcus* (21-251), an enzyme-encoding sequence (252-1298) selected from the sequences of a *Thermotoga* Threonine Synthase (YP_001243978), a 120-bp *rbcS* terminator from BP1 (1299-1418), and a PCR RE primer (1419-1438).

SEQ ID NO: 81 presents example 81 of a designer nirA-promoter-controlled Threonine Ammonia-Lyase **(52)** DNA construct (1600 bp) consisting of a PCR FD primer (sequence 1-20), a 231-bp nirA promoter from *Thermosynechococcus elongatus* BP1 (21-251), an enzyme-encoding sequence (252-1460) selected/modified from the sequences of a *Geobacillus kaustophilus* threonine ammonia-lyase (BAD75876), a 120-bp *rbcS* terminator from BP1 (1461-1580), and a PCR RE primer (1581-1600) at the 3' end.

Note, SEQ ID NOS: 58-61, 74-81, 66-69, and 72 (and/or 73) represent a set of sample designer genes that can express a Calvin-cycle 3-phophoglycerate-branched photosynthetic NADPH-enhanced 1-butanol production pathway **of 34, 35, 03, 04, 45-52 40, 41, 39, 42,** and **12** while SEQ ID NOS: 58-69 and 72 (and/or 73) represent another set of sample designer genes that can express another Calvin-cycle 3-phophoglycerate-branched photosynthetic NADPH-enhanced 1-butanol production pathway as numerically labeled as **34, 35, 03-05, 36-42,** and **12** in Figure 4. The net results of the designer photosynthetic NADPH-enhanced pathways in working with the Calvin cycle are photobiological production of 1-butanol (CH₃CH₂CH₂CH₂OH) from carbon dioxide (CO₂) and water (H₂O) using photosynthetically generated ATP (Adenosine triphosphate) and NADPH (reduced nicotinamide adenine dinucleotide phosphate) according to the following process reaction:

4CO₂ + 5H₂O → CH₃CH₂CH₂CH₂OH + 6O₂ [5]

### Designer Calvin-Cycle-Channeled 2-Methyl-1-Butanol Producing Pathways

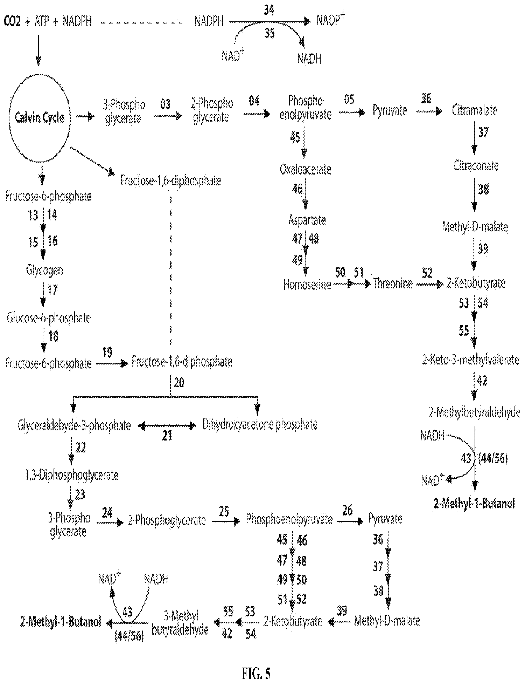

According to one of the various embodiments, a designer Calvin-cycle-channeled 2-Methyl-1-Butanol production pathway is created that takes the Calvin-cycle intermediate product, 3-phosphoglycerate, and converts it into 2-methyl-1-butanol by using, for example, a set of enzymes consisting of (as shown with the numerical labels **34, 35, 03-05, 36-39, 53-55, 42, 43** or **44/56** in Figure 5): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** citramalate synthase **36,** 2-methylmalate dehydratase **37,** 3-isopropylmalate dehydratase **38,** 3-isopropylmalate dehydrogenase **39,** acetolactate synthase **53,** ketol-acid reductoisomerase **54,** dihydroxy-acid dehydratase **55,** 2-keto acid decarboxylase **42,** and NAD-dependent alcohol dehydrogenase **43** (or NADPH-dependent alcohol dehydrogenase **44;** more preferably, 2-methylbutyraldehyde reductase **56).**

In another embodiment, a designer Calvin-cycle-channeled 2-methyl-1-butanol production pathway is created that takes the intermediate product, 3-phosphoglycerate, and converts it into 2-methyl-1-butanol by using, for example, a set of enzymes consisting of (as shown with the numerical labels **34, 35, 03, 04, 45-55, 42, 43** or **44/56** in Figure 5): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** phosphoenolpyruvate carboxylase **45,** aspartate aminotransferase **46,** aspartokinase **47,** aspartate-semialdehyde dehydrogenase **48,** homoserine dehydrogenase **49,** homoserine kinase **50,** threonine synthase **51,** threonine ammonia-lyase **52,** acetolactate synthase **53,** ketol-acid reductoisomerase **54,** dihydroxy-acid dehydratase **55,** 2-keto acid decarboxylase **42,** and NAD dependent alcohol dehydrogenase **43** (or NADPH dependent alcohol dehydrogenase **44;** more preferably, 2-methylbutyraldehyde reductase **56).**

These pathways (Fig. 5) are quite similar to those of Fig. 4, except that acetolactate synthase **53,** ketol-acid reductoisomerase **54,** dihydroxy-acid dehydratase **55,** and 2-methylbutyraldehyde reductase **56** are used to produce 2-Methyl-1-Butanol.

SEQ ID NO: 82 presents example 82 of a designer nirA-promoter-controlled Acetolactate Synthase **(53)** DNA construct (2107 bp) that includes a PCR FD primer (sequence 1-20), a 231-bp *nirA* promoter from *Thermosynechococcus elongatus* BP1 (21-251), an acetolactate synthase-encoding sequence (252-1967) selected/modified from the sequences of a *Bacillus subtilis subsp. subtilis str. 168* acetolactate synthase (CAB07802), a 120-bp *rbcS* terminator from BP1 (1968-2087), and a PCR RE primer (2088-2107) at the 3' end.

SEQ ID NO: 83 presents example 83 of a designer nirA-promoter-controlled Ketol-Acid Reductoisomerase **(54)** DNA construct (1405 bp) that includes a PCR FD primer (sequence 1-20), a 231-bp nirA promoter from *Thermosynechococcus elongatus* BP1 (21-251), a ketol-acid reductoisomerase-encoding sequence (252-1265) selected/modified from the sequences of a *Syntrophothermus lipocalidus* DSM 12680 ketol-acid reductoisomerase (ADI02902), a 120-bp rbcS terminator from BP1 (1266-1385), and a PCR RE primer (1386-1405) at the 3' end.

SEQ ID NO: 84 presents example 84 of a designer nirA-promoter-controlled Dihydroxy-Acid Dehydratase **(55)** DNA construct (2056 bp) that includes a PCR FD primer (1-20), a 231-bp nirA promoter from *Thermosynechococcus elongatus* BP1 (21-251), an enzyme-encoding sequence (252-1916) selected from the sequences of a *Thermotoga* dihydroxy-acid dehydratase (YP_001243973), a 120-bp *rbcS* terminator from BP1 (1917-2036), and a PCR RE primer (2037-2056).

SEQ ID NO: 85 presents example 85 of a designer nirA-promoter-controlled 2-Methylbutyraldehyde Reductase **(56)** DNA construct (1360 bp) that includes a PCR FD primer (sequence 1-20), a 231-bp nirA promoter from *Thermosynechococcus elongatus* BP1 (21-251), an enzyme-encoding sequence (252-1220) selected/modified from the sequences of a *Schizosaccharomyces japonicus* 2-methylbutyraldehyde reductase (XP_002173231), a 120-bp *rbcS* terminator from BP1 (1221-1340), and a PCR RE primer (1341-1360) at the 3' end.

Note, SEQ ID NOS: 58-66, 82-84, 69 and 85 represent another set of sample designer genes that can express a Calvin-cycle 3-phophoglycerate-branched photosynthetic NADPH-enhanced 2-methyl-1-butanol production pathway numerically labeled as **34, 35, 03-05, 36-39, 53-55, 42** and **56;** while SEQ ID NOS: 58-61, 74-84, 69 and 85 represent a set of sample designer genes that can express another Calvin-cycle 3-phophoglycerate-branched photosynthetic NADPH-enhanced 2-methyl-1-butanol production pathway of **34, 35, 03, 04, 45-55, 42** and **56** in Figure 5. These designer genes can be used in combination with other pathway gene(s) to express certain other pathways such as a Calvin-cycle Fructose-6-phosphate branched 2-methyl-1-butanol production pathway numerically labeled as **13-26, 36-39, 53-55, 42** and **56** (and/or, as **13-25, 45-55, 42** and **56)** in Figure 5 as well. The net results of the designer photosynthetic NADPH-enhanced pathways in working with the Calvin cycle are production of 2-methyl-1-butanol [CH₃CH₂CH(CH₃)CH₂OH] from carbon dioxide (CO₂) and water (H₂O) using photosynthetically generated ATP and NADPH according to the following process reaction:

10CO₂ + 12H₂O → 2CH₃CH₂CH(CH₃)CH₂OH + 15O₂ [6]

### Calvin-Cycle-Channeled Pathways for Production of Isobutanol and 3-Methyl-1-Butanol

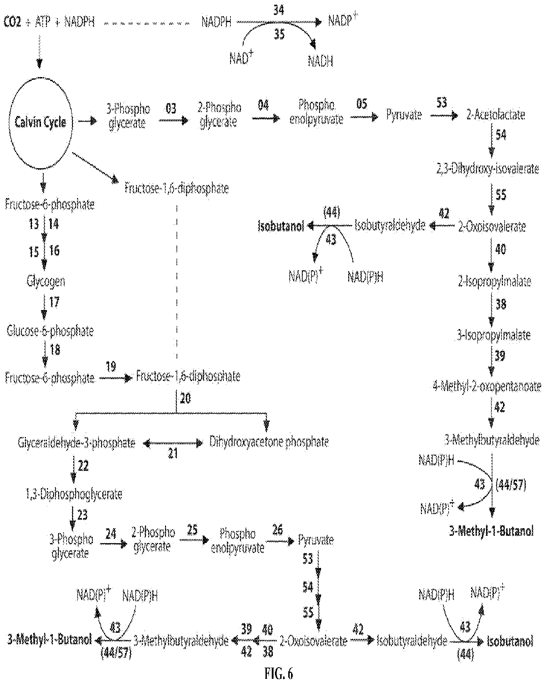

According to one of the various embodiments, a designer Calvin-cycle-channeled pathway is created that takes the Calvin-cycle intermediate product, 3-phosphoglycerate, and converts it into isobutanol by using, for example, a set of enzymes consisting of (as shown with numerical labels **34, 35, 03-05, 53-55, 42, 43 (or 44)** in Figure 6): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** acetolactate synthase **53,** ketol-acid reductoisomerase **54,** dihydroxy-acid dehydratase **55,** 2-keto acid decarboxylase **42,** and NAD-dependent alcohol dehydrogenase **43** (or NADPH-dependent alcohol dehydrogenase **44).** The net result of this pathway in working with the Calvin cycle is photobiological production of isobutanol ((CH₃)₂CHCH₂OH) from carbon dioxide (CO₂) and water (H₂O) using photosynthetically generated ATP and NADPH according to the following process reaction:

4CO₂ + 5H₂O → (CH₃)₂CHCH₂OH + 6O₂ [7]

According to another embodiment, a designer Calvin-cycle-channeled pathway is created that takes the intermediate product, 3-phosphoglycerate, and converts it into 3-methyl-1-butanol by using, for example, a set of enzymes consisting of (as shown with the numerical labels **34, 35, 03-05, 53-55, 40, 38, 39, 42, 43 (or 44/57)** in Figure 6): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** acetolactate synthase **53,** ketol-acid reductoisomerase **54,** dihydroxy-acid dehydratase **55,** 2-isopropylmalate synthase **40,** 3-isopropylmalate dehydratase **38,** 3-isopropylmalate dehydrogenase **39,** 2-keto acid decarboxylase **42,** and NAD-dependent alcohol dehydrogenase **43** (or NADPH-dependent alcohol dehydrogenase **44;** or more preferably, 3-methylbutanal reductase **57).** The net result of this pathway in working with the Calvin cycle is photobiological production of 3-methyl-1-butanol (CH₃CH(CH₃)CH₂CH₂OH) from carbon dioxide (CO₂) and water (H₂O) using photosynthetically generated ATP and NADPH according to the following process reaction:

10CO₂ + 12H₂O → 4CH₃CH(CH₃)CH₂CH₂OH + 15O₂ [8]

These designer pathways (Figure 6) share a number of designer pathway enzymes with those of Figures 4 and 5, except that a 3-methylbutanal reductase **57** is preferably used for production of 3-methyl-1-butanol; they all have a common feature of using an NADPH-dependent glyceraldehyde-3-phosphate dehydrogenase **34** and an NAD-dependent glyceraldehyde-3-phosphate dehydrogenase **35** as an NADPH/NADH conversion mechanism to covert certain amount of photosynthetically generated NADPH to NADH which can be used by NADH-requiring pathway enzymes such as an NADH-requiring alcohol dehydrogenase **43.**

SEQ ID NO: 86 presents example 86 of a designer nirA-promoter-controlled 3-Methylbutanal Reductase **(57)** DNA construct (1420 bp) that includes a PCR FD primer (sequence 1-20), a 231-bp nirA promoter from *Thermosynechococcus elongatus* BP1 (21-251), an enzyme-encoding sequence (252-1280) selected/modified from the sequences of a *Saccharomyces cerevisiae S288c* 3-Methylbutanal reductase (DAA10635), a 120-bp *rbcS* terminator from BP1 (1281-1400), and a PCR RE primer (1401-1420) at the 3' end.

SEQ ID NOS: 58-62, 82-84, 69, 70 (or 71) represent a set of sample designer genes that can express a Calvin-cycle 3-phosphoglycerate-branched photosynthetic NADPH-enhanced isobutanol production pathway **(34, 35, 03-05, 53-55, 42, 43** or **44);** while SEQ ID NOS: 58-62, 82-84, 65-67, 69 and 86 represent another set of sample designer genes that can express a Calvin-cycle 3-phosphoglycerate-branched photosynthetic NADPH-enhanced 3-methyl-1-butanol production pathway **(34, 35, 03-05, 53-55, 40, 38, 39, 42,** and **57** in Figure 6).

These designer genes can be used with certain other designer genes to express certain other pathways such as a Calvin-cycle Fructose-6-phosphate-branched 3-methyl-1-butanol production pathway shown as **13-26, 53-54, 39-40, 42** and **57** (or **43/44)** in Figure 6 as well. The net results of the designer photosynthetic NADPH-enhanced pathways in working with the Calvin cycle are also production of isobutanol ((CH₃)₂CHCH₂OH) and/or 3-methyl-1-butanol (CH₃CH(CH₃)CH₂CH₂OH) from carbon dioxide (CO₂) and water (H₂O) using photosynthetically generated ATP and NADPH.

### Designer Calvin-Cycle-Channeled Pathways for Production of 1-Hexanol and 1-Octanol

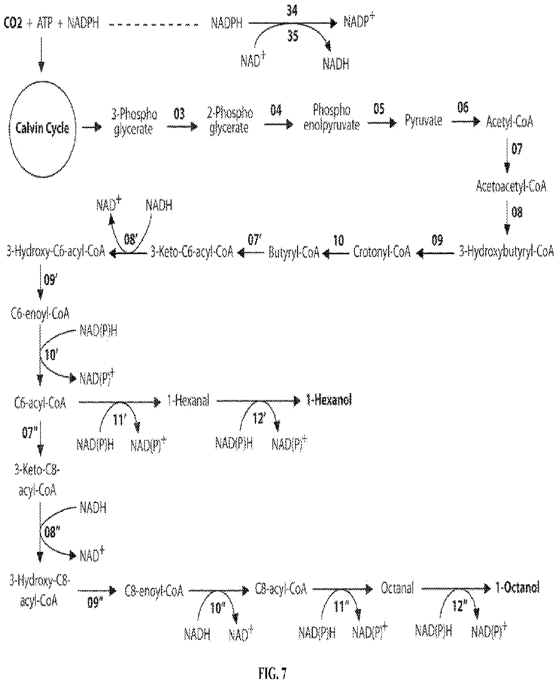

According to one of the various embodiments, a designer Calvin-cycle-channeled pathway is created that takes the Calvin-cycle intermediate product, 3-phosphoglycerate, and converts it into 1-hexanol by using, for example, a set of enzymes consisting of (as shown with the numerical labels **34, 35, 03-10, 07'-12'** in Figure 7): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** pyruvate-ferredoxin oxidoreductase **06,** thiolase **07,** 3-hydroxybutyryl-CoA dehydrogenase **08,** crotonase **09,** butyryl-CoA dehydrogenase **10,** designer 3-ketothiolase **07',** designer 3-hydroxyacyl-CoA dehydrogenase **08',** designer enoyl-CoA dehydratase **09',** designer 2-enoyl-CoA reductase **10',** designer acyl-CoA reductase **11',** and hexanol dehydrogenase **12'.** The net result of this designer pathway in working with the Calvin cycle is photobiological production of 1-hexanol (CH₃CH₂CH₂CH₂CH₂CH₂OH) from carbon dioxide (CO₂) and water (H₂O) using photosynthetically generated ATP and NADPH according to the following process reaction:

6CO₂ + 7H₂O → CH₃CH₂CH₂CH₂CH₂CH₂OH + 9O₂ [9]

According to another embodiment, a designer Calvin-cycle-channeled pathway is created that takes the intermediate product, 3-phosphoglycerate, and converts it into 1-octanol by using, for example, a set of enzymes consisting of (as shown with the numerical labels **34, 35, 03-10, 07'-10',** and **07"-12"** in Figure 7): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** pyruvate-ferredoxin oxidoreductase **06,** thiolase **07,** 3-hydroxybutyryl-CoA dehydrogenase **08,** crotonase **09,** butyryl-CoA dehydrogenase **10,** designer 3-ketothiolase **07',** designer 3-hydroxyacyl-CoA dehydrogenase **08',** designer enoyl-CoA dehydratase **09',** designer 2-enoyl-CoA reductase **10',** designer 3-ketothiolase **07",** designer 3-hydroxyacyl-CoA dehydrogenase **08",** designer enoyl-CoA dehydratase **09",** designer 2-enoyl-CoA reductase **10",** designer acyl-CoA reductase **11**", and octanol dehydrogenase **12".**

These pathways represent a significant upgrade in the pathway designs with part of a previously disclosed 1-butanol production pathway **(03-10).** The key feature is the utilization of an NADPH-dependent glyceraldehyde-3-phosphate dehydrogenase **34** and an NAD-dependent glyceraldehyde-3-phosphate dehydrogenase **35** as a mechanism for NADPH/NADH conversion to drive an NADH-requiring designer hydrocarbon chain elongation pathway (**07**'-**10'**) for 1-hexanol production **(07'-12'** as shown in Figure 7).

SEQ ID NOS: 87-92 represent a set of designer genes that can express the designer hydrocarbon chain elongation pathway for 1-hexanol production (**07**'-**12**' as shown in Figure 7). Briefly, SEQ ID NO: 87 presents example 87 of a designer nirA-promoter-controlled 3-Ketothiolase **(07')** DNA construct (1540 bp) that includes a PCR FD primer (sequence 1-20), a 231-bp nirA promoter from *Thermosynechococcus elongatus* BP1 (21-251), an enzyme-encoding sequence (252-1400) selected/modified from the sequences of a *Geobacillus kaustophilus* 3-Ketothiolase (YP_147173), a 120-bp *rbcS* terminator from BP1 (1401-1520), and a PCR RE primer (1521-1540).

SEQ ID NO: 88 presents example 88 of a designer nirA-promoter-controlled 3-Hydroxyacyl-CoA Dehydrogenase **(08')** DNA construct (1231 bp) that includes a PCR FD primer (sequence 1-20), a 231-bp nirA promoter from *Thermosynechococcus elongatus* BP1 (21-251), an enzyme-encoding sequence (252-1091) selected/modified from the sequences of a *Syntrophothermus lipocalidus* 3-Hydroxyacyl-CoA dehydrogenase (YP_003702743), a 120-bp *rbcS* terminator from BP1 (1092-1211), and a PCR RE primer (1212-1231).

SEQ ID NO: 89 presents example 89 of a designer nirA-promoter-controlled Enoyl-CoA Dehydratase **(09')** DNA construct (1162 bp) that includes a PCR FD primer (sequence 1-20), a 231-bp nirA promoter from *Thermosynechococcus elongatus* BP1 (21-251), an enzyme-encoding sequence (252-1022) selected/modified from the sequences of a *Bordetella petrii* Enoyl-CoA dehydratase (CAP41574), a 120-bp *rbcS* terminator from BP1 (1023-1442), and a PCR RE primer (1443-1162) at the 3' end.

SEQ ID NO: 90 presents example 90 of a designer nirA-promoter-controlled 2-Enoyl-CoA Reductase **(10')** DNA construct (1561 bp) that includes a PCR FD primer (sequence 1-20), a 231-bp nirA promoter from *Thermosynechococcus elongatus* BP1 (21-251), an enzyme-encoding sequence (252-1421) selected/modified from the sequences of a *Xanthomonas campestris* 2-Enoyl-CoA Reductase (CAP53709), a 120-bp *rbcS* terminator from BP1 (1422-1541), and a PCR RE primer (1542-1561).

SEQ ID NO: 91 presents example 91 of a designer nirA-promoter-controlled Acyl-CoA Reductase **(11')** DNA construct (1747 bp) that includes a PCR FD primer (sequence 1-20), a 231-bp nirA promoter from *Thermosynechococcus elongatus* BP1 (21-251), an enzyme-encoding sequence (252-1607) selected/modified from the sequences of a *Clostridium cellulovorans* Acyl-CoA reductase (YP_003845606), a 120-bp *rbcS* terminator from BP1 (1608-1727), and a PCR RE primer (1728-1747).

SEQ ID NO: 92 presents example 92 of a designer nirA-promoter-controlled Hexanol Dehydrogenase **(12')** DNA construct (1450 bp) that includes a PCR FD primer (sequence 1-20), a 231-bp nirA promoter from *Thermosynechococcus elongatus* BP1 (21-251), an enzyme-encoding sequence (252-1310) selected/modified from the sequences of a *Mycobacterium chubuense* hexanol dehydrogenase (ACZ56328), a 120-bp *rbcS* terminator from BP1 (1311-1430), and a PCR RE primer (1431-1450).

SEQ ID NO: 93 presents example 93 of a designer nirA-promoter-controlled Octanol Dehydrogenase **(12")** DNA construct (1074 bp) that includes a PCR FD primer (sequence 1-20), a 231-bp nirA promoter from *Thermosynechococcus elongatus* BP1 (21-251), an enzyme-encoding sequence (252-934) selected/modified from the sequences of a *Drosophila subobscura* octanol dehydrogenase (ABO65263), a 120-bp *rbcS* terminator from BP1 (935-1054), and a PCR RE primer (1055-1074) at the 3' end.

Note, the designer enzymes of SEQ ID NOS: 87-91 have certain broad substrate specificity. Consequently, they can also be used as designer 3-ketothiolase **07",** designer 3-hydroxyacyl-CoA dehydrogenase **08",** designer enoyl-CoA dehydratase **09",** designer 2-enoyl-CoA reductase **10",** and designer acyl-CoA reductase **11".** Therefore, SEQ ID NOS: 87-91 and 93 represent a set of designer genes that can express another designer hydrocarbon chain elongation pathway for 1-octanol production (**07**'-**10**' and **07"-12"** as shown in Figure 7). SEQ ID NO: 93 (encoding for octanol dehydrogenase **12")** is one of the key designer genes that enable production of 1-octanol production in this pathway. The net result of this pathway in working with the Calvin cycle are photobiological production of 1-octanol (CH₃CH₂CH₂CH₂CH₂CH₂CH₂CH₂OH) from carbon dioxide (CO₂) and water (H₂O) using photosynthetically generated ATP and NADPH according to the following process reaction:

8CO₂ + 9H₂O → CH₃CH₂CH₂CH₂CH₂CH₂CH₂CH₂OH + 12O₂ [10]

### Calvin-Cycle-Channeled Pathways for Production of 1-Pentanol, 1-Hexanol and 1-Heptanol

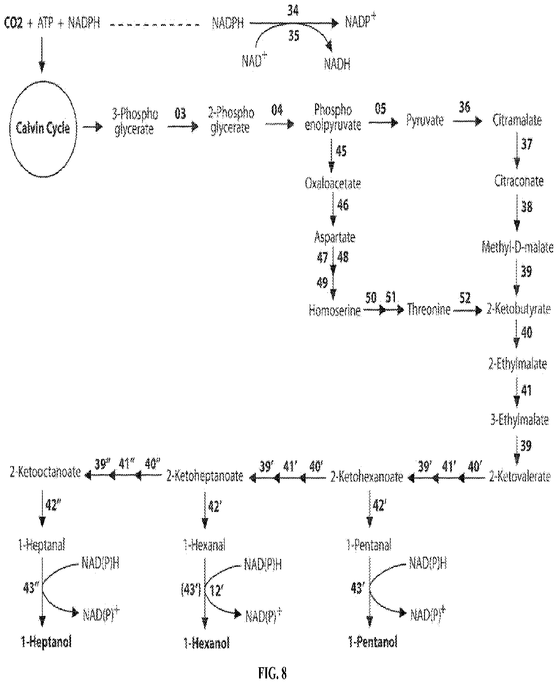

According to one of the various embodiments, a designer Calvin-cycle-channeled pathway is created that takes the Calvin-cycle intermediate product, 3-phosphoglycerate, and converts it into 1-pentanol, 1-hexanol, and/or 1-heptanol by using, for example, a set of enzymes consisting of (as shown with the numerical labels **34, 35, 03-05, 36-41, 39, 39'-43', 39'-43', 12',** and **39"-43"** in Figure 8): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** citramalate synthase **36,** 2-methylmalate dehydratase **37,** 3-isopropylmalate dehydratase **38,** 3-isopropylmalate dehydrogenase **39,** 2-isopropylmalate synthase **40,** isopropylmalate isomerase **41,** 3-isopropylmalate dehydrogenase **39,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** hexanol dehydrogenase **12',** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".** This designer pathway works with the Calvin cycle using photosynthetically generated ATP and NADPH for photobiological production of 1-pentanol (CH₃CH₂CH₂CH₂CH₂OH), 1-hexanol (CH₃CH₂CH₂CH₂CH₂CH₂OH), and/or 1-heptanol (CH₃CH₂CH₂CH₂CH₂CH₂CH₂OH) from carbon dioxide (CO₂) and water (H₂O) according to the following process reactions:

10CO₂ + 12H₂O → 2CH₃CH₂CH₂CH₂CH₂OH + 15O₂ [11]

6CO₂ + 7H₂O → CH₃CH₂CH₂CH₂CH₂CH₂OH + 9O₂ [12]

14CO₂ + 16H₂O → 2CH₃CH₂CH₂CH₂CH₂CH₂CH₂OH + 21O₂ [13]

According to another embodiment, a designer Calvin-cycle-channeled pathway is created that takes the intermediate product, 3-phosphoglycerate, and converts it into 1-pentanol, 1-hexanol, and/or 1-heptanol by using, for example, a set of enzymes consisting of (as shown with the numerical labels **34, 35, 03, 04, 45-52, 40, 41, 39, 39'-43', 39'-43', 12',** and **39"-43"** in Figure 8): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** phosphoenolpyruvate carboxylase **45,** aspartate aminotransferase **46,** aspartokinase **47,** aspartate-semialdehyde dehydrogenase **48,** homoserine dehydrogenase **49,** homoserine kinase **50,** threonine synthase **51,** threonine ammonia-lyase **52,** 2-isopropylmalate synthase **40,** isopropylmalate isomerase **41,** 3-isopropylmalate dehydrogenase **39,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** hexanol dehydrogenase **12',** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

These pathways (Figure 8) share a common feature of using an NADPH-dependent glyceraldehyde-3-phosphate dehydrogenase **34** and an NAD-dependent glyceraldehyde-3-phosphate dehydrogenase **35** as a mechanism for NADPH/NADH conversion to drive production of 1-pentanol, 1-hexanol, and/or 1-heptanol through a designer Calvin-cycle-channeled pathway in combination with a designer hydrocarbon chain elongation pathway **(40', 41', 39').** This embodiment also takes the advantage of the broad substrate specificity (promiscuity) of 2-isopropylmalate synthase **40,** isopropylmalate isomerase **41,** 3-isopropylmalate dehydrogenase **39,** 2-keto acid decarboxylase **42,** and short-chain alcohol dehydrogenase **43** so that they can be used also as: designer isopropylmalate synthase **40',** designer isopropylmalate isomerase **41',** designer 3-isopropylmalate dehydrogenase **39',** designer 2-keto acid decarboxylase **42',** and short-chain alcohol dehydrogenase **43';** isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

In this case, proper selection of a short-chain alcohol dehydrogenase with certain promiscuity is also essential. SEQ ID NO: 94 presents example 94 of a designer nirA-promoter-controlled Short Chain Alcohol Dehydrogenase DNA construct (1096 bp) that includes a PCR FD primer (sequence 1-20), a 231-bp nirA promoter from *Thermosynechococcus elongatus* BP1 (21-251), an enzyme-encoding sequence (252-956) selected/modified from the sequences of a *Pyrococcus furiosus DSM 3638* Short chain alcohol dehydrogenase (AAC25556), a 120-bp *rbcS* terminator from BP1 (957-1076), and a PCR RE primer (1077-1096) at the 3' end.

Therefore, SEQ ID NOS: 58-69 and 94 represent a set of designer genes that can express a designer Calvin-cycle 3-phosphoglycerate-braned photosynthetic NADPH-enhanced pathway for production of 1-pentanol, 1-hexanol, and/or 1-heptanol as shown with numerical labels **34, 35, 03-05, 36-41, 39, 39'-43', 39'-43', 39"-43"** in Figure 8. Similarly, SEQ ID NOS: 58-61,74-81, 66-69, and 94 represent another set of sample designer genes that can express another Calvin-cycle 3-phophoglycerate-branched NADPH-enhanced pathway for production of 1-pentanol, 1-hexanol, and/or 1-heptanol as numerically labeled as **34, 35, 03, 04, 45-52, 40, 41, 39, 39'-43', 39'-43', 39"-43"** in Figure 8. Note, both of these two pathways produce alcohol mixtures with different chain lengths rather than single alcohols since all 2-keto acids (such as 2-ketohexanoate, 2-ketaheptanoate, and 2-ketooctanoate) can be converted to alcohol because of the use of the promiscuity of designer 2-keto acid decarboxylase **42'** and designer short-chain alcohol dehydrogenase **43'.**

To improve product specificity, it is a preferred practice to use substrate specific designer enzymes. For example, use of substrate specific designer 1-hexanol dehydrogenase **12'** (SEQ ID NO: 92) instead of short-chain alcohol dehydrogenase with promiscuity **(43')** can improve product specificity more toward 1-hexanol. Consequently, SEQ ID NOS: 58-69 and 92 represent a set of designer genes that can express a designer Calvin-cycle 3-phosphoglycerate-braned photosynthetic NADPH-enhanced pathway for production of 1-hexanol as shown with numerical labels **34, 35, 03-05, 36-41, 39, 39'-40', 39'-42'** and **12'** in Figure 8.

### Designer Calvin-Cycle-Channeled Pathways for Production of 3-Methyl-1-Pentanol, 4-Methyl-1-Hexanol, and 5-Methyl-1-Heptanol

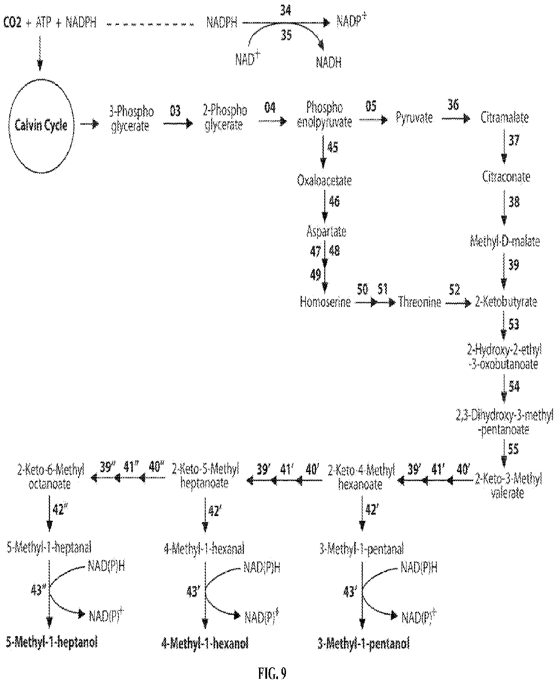

According to one of the various embodiments, a designer Calvin-cycle-channeled pathway is created that takes the Calvin-cycle intermediate product, 3-phosphoglycerate, and converts it into 3-methyl-1-pentanol, 4-methyl-1-hexanol, and/or 5-methyl-1-heptanol by using, for example, a set of enzymes consisting of (as shown with the numerical labels **34, 35, 03-05, 36-39, 53-55, 39'-43', 39'-43',** and **39"-43"** in Figure 9): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** citramalate synthase **36,** 2-methylmalate dehydratase **37,** 3-isopropylmalate dehydratase **38,** 3-isopropylmalate dehydrogenase **39,** acetolactate synthase **53,** ketol-acid reductoisomerase **54,** dihydroxy-acid dehydratase **55,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

According to another embodiment, a designer Calvin-cycle-channeled pathway is created that takes the intermediate product, 3-phosphoglycerate, and converts it into 3-methyl-1-pentanol, 4-methyl-1-hexanol, and/or 5-methyl-1-heptanol by using, for example, a set of enzymes consisting of (as shown with the numerical labels **34, 35, 03, 04, 45-55, 39'-43', 39'-43',** and **39"-43"** in Figure 9): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** phosphoenolpyruvate carboxylase **45,** aspartate aminotransferase **46,** aspartokinase **47,** aspartate-semialdehyde dehydrogenase **48,** homoserine dehydrogenase **49,** homoserine kinase **50,** threonine synthase **51,** threonine ammonia-lyase **52,** acetolactate synthase **53,** ketol-acid reductoisomerase **54,** dihydroxy-acid dehydratase **55,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

These pathways (Figure 9) are similar to those of Figure 8, except they use acetolactate synthase **53,** ketol-acid reductoisomerase **54,** dihydroxy-acid dehydratase **55** as part of the pathways for production of 3-methyl-1-pentanol, 4-methyl-1-hexanol, and/or 5-methyl-1-heptanol. They all share a common feature of using an NADPH-dependent glyceraldehyde-3-phosphate dehydrogenase **34** and an NAD-dependent glyceraldehyde-3-phosphate dehydrogenase **35** as a mechanism for NADPH/NADH conversion to drive production of 3-methyl-1-pentanol, 4-methyl-1-hexanol, and/or 5-methyl-1-heptanol through a designer Calvin-cycle-channeled pathway in combination with a hydrocarbon chain elongation pathway **(40', 41', 39').** This embodiment also takes the advantage of the broad substrate specificity (promiscuity) of 2-isopropylmalate synthase **40,** isopropylmalate isomerase **41,** 3-isopropylmalate dehydrogenase **39,** 2-keto acid decarboxylase **42,** and short-chain alcohol dehydrogenase **43** so that they can also serve as: designer isopropylmalate synthase **40',** designer isopropylmalate isomerase **41',** designer 3-isopropylmalate dehydrogenase **39',** designer 2-keto acid decarboxylase **42',** and short-chain alcohol dehydrogenase **43';** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

Therefore, SEQ ID NOS: 58-69, 82-84, and 94 represent a set of designer genes that can express a designer Calvin-cycle 3-phosphoglycerate-braned photosynthetic NADPH-enhanced pathway for production of 3-methyl-1-pentanol, 4-methyl-1-hexanol, and 5-methyl-1-heptanol as shown with numerical labels **34, 35, 03-05, 36-39, 53-55, 39'-43', 39'-43',** and **39"-43"** in Figure 9. Similarly, SEQ ID NOS: 58-61,74-81, 82-84, 66-69, and 94 represent another set of sample designer genes that can express another Calvin-cycle 3-phophoglycerate-branched NADPH-enhanced pathway for production of 3-methyl-1-pentanol, 4-methyl-1-hexanol, and/or 5-methyl-1-heptanol as numerically labeled as **34, 35, 03, 04, 45-55, 39'-43', 39'-43', 39"-43"** in Figure 9. The net results of the designer photosynthetic NADPH-enhanced pathways in working with the Calvin cycle are production of 3-methyl-1-pentanol (CH₃CH₂CH(CH₃)CH₂CH₂OH), 4-methyl-1-hexanol (CH₃CH₂CH(CH₃)CH₂CH₂CH₂OH), and 5-methyl-1-heptanol (CH₃CH₂CH(CH₃)CH₂CH₂CH₂CH₂OH) from carbon dioxide (CO₂) and water (H₂O) using photosynthetically generated ATP and NADPH according to the following process reactions:

6CO₂ + 7H₂O → CH₃CH₂CH(CH₃)CH₂CH₂OH + 9O₂ [14]

14CO₂ + 16H₂O → 2CH₃CH₂CH(CH₃)CH₂CH₂CH₂OH + 21O₂ [15]

8CO₂ + 9H₂O → CH₃CH₂CH(CH₃)CH₂CH₂CH₂CH₂OH + 12O₂ [16]

### Designer Calvin-Cycle-Channeled Pathways for Production of 4-Methyl-1-Pentanol, 5-Methyl-1-Hexanol, and 6-Methyl-1-Heptanol

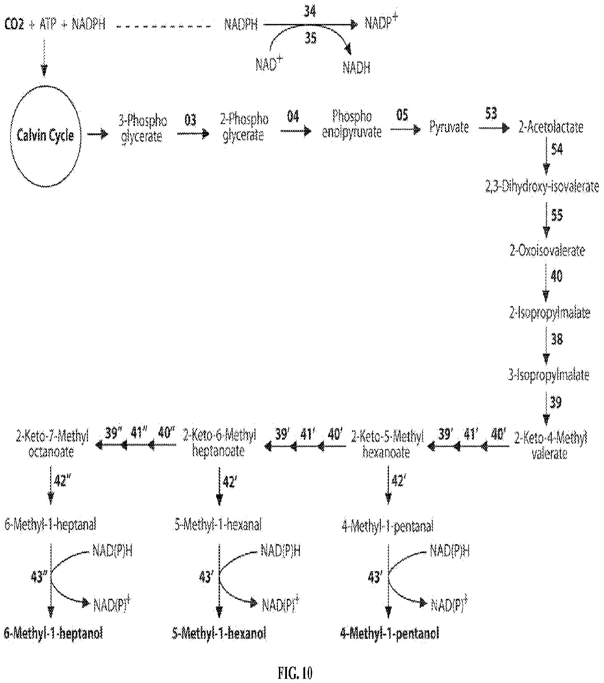

According to one of the various embodiments, a designer Calvin-cycle-channeled pathway is created that takes the Calvin-cycle intermediate product, 3-phosphoglycerate, and converts it into 4-methyl-1-pentanol, 5-methyl-1-hexanol, and 6-methyl-1-heptanol by using, for example, a set of enzymes consisting of (as shown with the numerical labels **34, 35, 03-05, 53-55, 40, 38, 39, 39'-43', 39'-43',** and **39"-43"** in Figure 10): NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** acetolactate synthase **53,** ketol-acid reductoisomerase **54,** dihydroxy-acid dehydratase **55,** isopropylmalate synthase **40,** dehydratase **38,** 3-isopropylmalate dehydrogenase **39,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

This pathway (Figure 10) is similar to those of Figure 8, except that it does not use citramalate synthase **36** and 2-methylmalate dehydratase **37,** but uses acetolactate synthase **53,** ketol-acid reductoisomerase **54,** dihydroxy-acid dehydratase **55** as part of the pathways for production of 4-methyl-1-pentanol, 5-methyl-1-hexanol, and/or 6-methyl-1-heptanol. They all share a common feature of using an NADPH-dependent glyceraldehyde-3-phosphate dehydrogenase **34** and an NAD-dependent glyceraldehyde-3-phosphate dehydrogenase **35** as a mechanism for NADPH/NADH conversion to drive production of 3-methyl-1-butanol, 4-methyl-1-butanol, and 5-methyl-1-butanol through a Calvin-cycle-channeled pathway in combination with a designer hydrocarbon chain elongation pathway **(40', 41', 39').** This embodiment also takes the advantage of the broad substrate specificity (promiscuity) of 2-isopropylmalate synthase **40,** isopropylmalate isomerase **41,** 3-isopropylmalate dehydrogenase **39,** 2-keto acid decarboxylase **42,** and short-chain alcohol dehydrogenase **43** so that they may also serve as: designer isopropylmalate synthase **40',** designer isopropylmalate isomerase **41',** designer 3-isopropylmalate dehydrogenase **39',** designer 2-keto acid decarboxylase **42',** and short-chain alcohol dehydrogenase **43',** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

Therefore, SEQ ID NOS: 58-62, 82-84, 65-69 and 94 represent a set of sample designer genes that can be used to express a designer Calvin-cycle 3-phosphoglycerate-braned photosynthetic NADPH-enhanced pathway for production of 4-methyl-1-pentanol, 5-methyl-1-hexanol, and/or 6-methyl-1-heptanol as shown with numerical labels **34, 35, 03-05, 53-55, 40, 38, 39, 39'-43', 39'-43',** and **39"-43"** in Figure 10. The net results of the designer photosynthetic NADPH-enhanced pathway in working with the Calvin cycle are production of 4-methyl-1-pentanol (CH₃CH(CH₃)CH₂CH₂CH₂OH), 5-methyl-1-hexanol (CH₃CH(CH₃)CH₂CH₂CH₂CH₂OH), and 6-methyl-1-heptanol (CH₃CH(CH₃)CH₂CH₂CH₂CH₂CH₂OH) from carbon dioxide (CO₂) and water (H₂O) using photosynthetically generated ATP and NADPH according to the following process reactions:

6CO₂ + 7H₂O → CH₃CH(CH₃)CH₂CH₂CH₂OH + 9O₂ [17]

14CO₂ + 16H₂O → 2CH₃CH(CH₃)CH₂CH₂CH₂CH₂OH + 21O₂ [18]

8CO₂ + 9H₂O → CH₃CH(CH₃)CH₂CH₂CH₂CH₂CH₂OH + 12O₂ [19]

### Designer Oxyphotobacteria with Calvin-Cycle-Channeled Pathways for Production of Butanol and Related Higher Alcohols

According to one of the various embodiments, use of designer DNA constructs in genetic transform of certain oxyphotobacteria hosts can create various designer transgenic oxyphotobacteria with Calvin-cycle-channeled pathways for photobiological production of butanol and related higher alcohols from carbon dioxide and water. To ensure biosafety for use of the designer transgenic photosynthetic organism-based biofuels production technology, it is a preferred practice to incorporate biosafety-guarded features into the designer transgenic photosynthetic organisms as well. Therefore, in accordance with the present invention, various designer photosynthetic organisms including designer transgenic oxyphotobacteria are created with a biosafety-guarded photobiological biofuel-production technology based on cell-division-controllable designer transgenic photosynthetic organisms. The cell-division-controllable designer photosynthetic organisms contain two key functions: a designer biosafety mechanism(s) and a designer biofuel-production pathway(s). The designer biosafety feature(s) is conferred by a number of mechanisms including: a) the inducible insertion of designer proton-channels into cytoplasm membrane to permanently disable any cell division and/or mating capability, b) the selective application of designer cell-division-cycle regulatory protein or interference RNA (iRNA) to permanently inhibit the cell division cycle and preferably keep the cell at the G₁ phase or Go state, and c) the innovative use of a high-CO₂-requiring host photosynthetic organism for expression of the designer biofuel-production pathway(s). The designer cell-division-control technology can help ensure biosafety in using the designer organisms for biofuel production.

Oxyphotobacteria (including cyanobacteria and oxychlorobacteria) that can be selected for use as host organisms to create designer transgenic oxyphotobacteria for photobiological production of butanol and related higher alcohols include (but not limited to): *Thermosynechococcus elongatus* BP-1, *Nostoc sp.* PCC 7120, *Synechococcus elongatus* PCC 6301, *Syncechococcus* sp. strain PCC 7942, *Syncechococcus* sp. strain PCC 7002, *Syncechocystis* sp. strain PCC 6803, *Prochlorococcus marinus* MED4, *Prochlorococcus marinus* MIT 9313, *Prochlorococcus marinus* NATL1A, *Prochlorococcus* SS120, *Spirulina platensis (Arthrospira platensis), Spirulina pacifica, Lyngbya majuscule, Anabaena sp., Synechocystis sp., Synechococcus elongates, Synechococcus* (MC-A), *Trichodesmium sp., Richelia intracellularis, Synechococcus* WH7803, *Synechococcus* WH8102, *Nostoc punctiforme, Syncechococcus* sp. strain PCC 7943, Synechocyitis PCC 6714 phycocyanin-deficient mutant PD-1, *Cyanothece* strain 51142, *Cyanothece sp.* CCY0110, *Oscillatoria limosa, Lyngbya majuscula, Symploca muscorum, Gloeobacter violaceus, Prochloron didemni, Prochlorothrix hollandica, Prochlorococcus marines, Prochlorococcus* SS120, *Synechococcus* WH8102, *Lyngbya majuscula, Symploca muscorum, Synechococcus bigranulatus,* cryophilic *Oscillatoria sp., Phormidium sp., Nostoc sp.-1, Calothrix parietina,* thermophilic *Synechococcus bigranulatus, Synechococcus lividus,* thermophilic *Mastigocladus laminosus, Chlorogloeopsis fritschii* PCC 6912, *Synechococcus vulcanus, Synechococcus sp.* strain MA4, *Synechococcus sp.* strain MA19, and *Thermosynechococcus elongatus.*

According to one of the examples, use of designer DNA constructs such as SEQ ID NOS: 58-94 in genetic transform of certain oxyphotobacteria hosts such as *Thermosynechococcus elongatus* BP1 can create a series of designer transgenic oxyphotobacteria with Calvin-cycle-channeled pathways for production of butanol and related higher alcohols. Consequently, SEQ ID NOS: 58-61, 74-81, 66-69, and 72 (and/or 73) represent a designer transgenic oxyphotobacterium such as a designer transgenic *Thermosynechococcus* that comprises the designer genes of a Calvin-cycle 3-phophoglycerate-branched photosynthetic NADPH-enhanced pathway (numerically labeled as **34, 35, 03, 04, 45-52, 39-42,** and **12** in Figure 4) for photobiological production of1-butanol from carbon dioxide and water. SEQ ID NOS: 58-69 and 72 (and/or 73) represent another designer transgenic oxyphotobacterium such as designer transgenic *Thermosynechococcus* that comprises the designer genes of a Calvin-cycle 3-phophoglycerate-branched photosynthetic NADPH-enhanced pathway (numerically labeled as **34, 35, 03-05, 36-42,** and **12** in Figure 4) for photobiological production of1-butanol from carbon dioxide and water as well.

Similarly, SEQ ID NOS: 58-66, 82-84, 69 and 85 represent another designer transgenic oxyphotobacterium such as designer transgenic *Thermosynechococcus* with a Calvin-cycle 3-phophoglycerate-branched photosynthetic NADPH-enhanced pathway (numerically labeled as **34, 35, 03-05, 36-39, 53-55, 42** and **56** in Figure 5) for photobiological production of 2-methyl-1-butanol production from carbon dioxide and water; while SEQ ID NOS: 58-61, 74-84, 69 and 85 represent another designer transgenic *Thermosynechococcus* with a Calvin-cycle 3-phophoglycerate-branched photosynthetic NADPH-enhanced 2-methyl-1-butanol production pathway **(34, 35, 03, 04, 45-55, 42** and **56** in Figure 5) for photobiological production of 2-methyl-1-butanol production from carbon dioxide and water.

SEQ ID NOS: 58-63, 82-84, 69, 70 (or 71) represent another designer transgenic oxyphotobacterium such as designer transgenic *Thermosynechococcus* with a Calvin-cycle 3-phosphoglycerate-branched photosynthetic NADPH-enhanced isobutanol production pathway (34, **35, 03-05, 53-5, 42, 43 or 44);** while SEQ ID NOS: 58-62, 81-83, 65-67, 69 and 86 represent another designer transgenic *Thermosynechococcus* with a Calvin-cycle 3-phosphoglycerate-branched photosynthetic NADPH-enhanced 3-methyl-1-butanol production pathway (numerical labels **34, 35, 03-05, 53-55, 40, 38, 39, 42,** and **57** in Figure 6).

SEQ ID NOS: 87-92 represent another designer transgenic *Thermosynechococcus* with a designer hydrocarbon chain elongation pathway **(07'-12'** as shown in Figure 7) for photobiological production of 1-hexanol. SEQ ID NOS: 87-91 and 93 represent another designer transgenic *Thermosynechococcus* with a designer hydrocarbon chain elongation pathway **(07'-10'** and **07"-12"** as shown in Figure 7) for photobiological production of 1-octanol.

SEQ ID NOS: 58-69 and 92 represent another designer transgenic *Thermosynechococcus* with a designer Calvin-cycle 3-phosphoglycerate-braned photosynthetic NADPH-enhanced pathway **(34, 35, 03-05, 36-41, 39, 39'-40', 39'-42'** and **12'** in Figure 8) for photobiological production of 1-hexanol from carbon dioxide and water.

SEQ ID NOS: 58-69, 82-84, and 94 represent a designer transgenic *Thermosynechococcus* with a designer Calvin-cycle 3-phosphoglycerate-braned photosynthetic NADPH-enhanced pathway **(34, 35, 03-05, 36-39, 53-55, 39'-43', 39'-43', 39"-43"** in Figure 9 ) for production of 3-methyl-1-pentanol, 4-methyl-1-hexanol, and 5-methyl-1-heptanol from carbon dioxide and water. Similarly, SEQ ID NOS: 58-61,74-81, 82-84, 66-69, and 94 represent another designer transgenic *Thermosynechococcus* with a Calvin-cycle 3-phophoglycerate-branched NADPH-enhanced pathway **(34, 35, 03, 04, 45-55, 39'-43', 39'-43', 39"-43"** in Figure 9) for photobiological production of 3-methyl-1-pentanol, 4-methyl-1-hexanol, and 5-methyl-1-heptanol from carbon dioxide and water as well.

SEQ ID NOS: 58-62, 82-84, 65-69 and 94 represent a designer transgenic *Thermosynechococcus* with a designer Calvin-cycle 3-phosphoglycerate-braned photosynthetic NADPH-enhanced pathway labels **(34, 35, 03-05, 53-55, 40, 38, 39, 39'-43', 39'-43',** and **39"-43"** in Figure 10) for photobiological production of 4-methyl-1-pentanol, 5-methyl-1-hexanol, and/or 6-methyl-1-heptanol from carbon dioxide and water.

Use of other host oxyphotobacteria such as *Synechococcus sp.* strain PCC 7942, *Synechocystis sp.* strain PCC 6803, *Prochlorococcus marinus, Cyanothece sp. ATCC 51142,* for genetic transformation with proper designer DNA constructs (genes) can create other designer oxyphotobacteria for photobiological production of butanol and higher alcohols as well. For example, use of *Synechococcus sp.* strain PCC 7942 as a host organism in genetic transformation with SEQ ID NOS: 95-98 (and/or 99) can create a designer transgenic *Synechococcus* for photobiological production of 1-butanol. Briefly, SEQ ID NO: 95 presents example 95 of a detailed DNA construct (1438 base pairs (bp)) of a designer NADPH-dependent Glyceraldehyde-3-Phosphate-Dehydrogenase **(34)** gene that includes a PCR FD primer (sequence bp 1-20), a 88-bp *nirA* promoter (21-108) selected from the *Synechococcus sp.* strain PCC 7942 (freshwater cyanobacterium) nitrite-reductase-gene promoter sequence, an enzyme-encoding sequence (109-1110) selected and modified from a *Staphylococcus* NADPH-dependent glyceraldehyde-3-phosphate-dehydrogenase sequence (GenBank accession number: YP_003471459), a 308-bp *Synechococcus rbcS* terminator (1111-1418), and a PCR RE primer (1419-1438).

SEQ ID NO: 96 presents example 96 of a detailed DNA construct (1447 bp) of a designer NAD-dependent Glyceraldehyde-3-Phosphate-Dehydrogenase **(35)** gene that includes a PCR FD primer (sequence bp 1-20), a 88-bp *nirA* promoter (21-108) selected from the *Synechococcus* nitrite-reductase-gene promoter sequence, an enzyme-encoding sequence (109-1119) selected from a *Staphylococcus aureus* NAD-dependent glyceraldehyde-3-phosphate-dehydrogenase sequence (GenBank accession number: ADC36961), a 308-bp *Synechococcus rbcS* terminator (1120-1427), and a PCR RE primer (1428-1447).

SEQ ID NO: 97 presents example 97 of a detailed DNA construct (2080 bp) of a designer 2-Keto Acid Decarboxylase **(42)** gene that includes a PCR FD primer (sequence bp 1-20), a 88-bp *nirA* promoter (21-108) selected from the *Synechococcus* nitrite-reductase-gene promoter sequence, an enzyme-encoding sequence (109-1752) selected from a *Lactococcus lactis* branched-chain alpha-ketoacid decarboxylase (GenBank accession number: AAS49166), a 308-bp *Synechococcus rbcS* terminator (1753-2060), and a PCR RE primer (2061-2080).

SEQ ID NO: 98 presents a detailed DNA construct (1603 bp) of a designer NADH-dependent butanol dehydrogenase (**12**a) gene that include a PCR FD primer (sequence bp 1-20), a 88-bp *nirA* promoter (21-108) selected from the *Synechococcus* nitrite-reductase-gene promoter sequence, an enzyme-encoding sequence (109-1275) selected from a *Clostridium* NADH-dependent butanol dehydrogenase (GenBank accession number: ADO12118), a 308-bp *Synechococcus rbcS* terminator (1276-1583), and a PCR RE primer (1584-1603).

SEQ ID NO: 99 presents example 99 of a detailed DNA construct (1654 bp) of a designer NADPH-dependent Butanol Dehydrogenase (**12**b) gene including: a PCR FD primer (sequence bp 1-20), a 88-bp *nirA* promoter (21-108) selected from the *Synechococcus* nitrite-reductase-gene promoter sequence, an enzyme-encoding sequence (109-1326) selected from a *Butyrivibrio* NADPH-dependent butanol dehydrogenase (GenBank: EFF67629), a 308-bp *Synechococcus rbcS* terminator (1327-1634), and a PCR RE primer (1635-1654).

Note, in the designer transgenic *Synechococcus* that is represented by SEQ ID NOS: 95-98 (and/or 99), *Synechococcus's* native enzymes of **03-05, 36-41** and **45-52** are used in combination with the designer *nirA*-promoter-controlled enzymes of **34, 35, 42** and **12** [encoded by SEQ ID NOS: 95-98 (and/or 99)] to confer the Calvin-cycle 3-phophoglycerate-branched photosynthetic NADPH-enhanced pathways for photobiological production of1-butanol from carbon dioxide and water (Figure 4).

Similarly, use of *Synechocystis sp.* strain PCC 6803 as a host organism in genetic transformation with SEQ ID NOS: 100-102 (and/or 103) creates a designer transgenic *Synechocystis* for photobiological production of 1-butanol. Briefly, SEQ ID NO: 100 presents example 100 of a designer nirA-promoter-controlled NAD-dependent Glyceraldehyde-3-Phosphate Dehydrogenase **(35)** DNA construct (1440 bp) that includes a PCR FD primer (sequence 1-20), a 89-bp *Synechocystis sp.* strain PCC 6803 nitrite-reductase *nirA* promoter (21-109), an enzyme-encoding sequence (110-1011) selected from a *Streptococcus pyogenes* NAD-dependent Glyceraldehyde-3-phosphate dehydrogenase (GenBank: YP_002285269), a 409-bp *Synechocystis sp.* PCC 6803 rbcS terminator (1012-1420), and a PCR RE primer (1421-1440).

SEQ ID NO: 101 presents example 101 of a designer nirA-promoter-controlled 2-Keto Acid Decarboxylase **(42)** DNA construct (2182 bp) that includes a PCR FD primer (sequence 1-20), a 89-bp *Synechocystis sp.* strain PCC 6803 nitrite-reductase *nirA* promoter (21-109), an enzyme-encoding sequence (110-1753) selected from a *Lactococcus lactis* branched-chain alpha-ketoacid decarboxylase (GenBank: AAS49166), a 409-bp *Synechocystis sp.* PCC 6803 rbcS terminator (1754-2162), and a PCR RE primer (2163-2182).

SEQ ID NO: 102 presents example 102 of a designer nirA-promoter-controlled NADH-dependent Butanol Dehydrogenase (**12**a) DNA construct (1705 bp) that includes a PCR FD primer (sequence 1-20), a 89-bp *Synechocystis* sp. strain PCC 6803 nitrite- reductase nirA promoter (21-109), an enzyme-encoding sequence (110-1276) selected from a *Clostridium carboxidivorans P7* NADH-dependent butanol dehydrogenase (GenBank: ADO12118), a 409-bp *Synechocystis sp.* PCC 6803 rbcS terminator (1277-1685), and a PCR RE primer (1686-1705).

SEQ ID NO: 103 presents example 103 of a designer nirA-promoter-controlled NADPH-dependent butanol dehydrogenase (**12**b) DNA construct (1756 bp) that includes a PCR FD primer (sequence 1-20), a 89-bp *Synechocystis sp.* strain PCC 6803 nitrite- reductase *nirA* promoter (21-109), an enzyme-encoding sequence (110-1327) selected from a *Butyrivibrio crossotus* NADPH-dependent butanol dehydrogenase (GenBank: EFF67629), a 409-bp *Synechocystis* sp. PCC 6803 rbcS terminator (1328-1736), and a PCR RE primer (1737-1756).

Note, in the designer transgenic *Synechocystis* that contains the designer genes of SEQ ID NOS: 100-102 (and/or 103), *Synechocystis's* native enzymes **of 34, 03-05, 36-41** and **45-52** are used in conjunction with the designer *nirA*-promoter-controlled enzymes **of 35, 42** and **12** [encoded by SEQ ID NOS: 100-102 (and/or 103)] to confer the Calvin-cycle 3-phophoglycerate-branched photosynthetic NADPH-enhanced pathways for photobiological production ofl-butanol from carbon dioxide and water (Figure 4).

Use of *Nostoc sp.* strain PCC 7120 as a host organism in genetic transformation with SEQ ID NOS: 104-109can create a designer transgenic *Nostoc* for photobiological production of 2-methyl-1-butanol (Figure 5). Briefly, SEQ ID NO: 104 presents example 104 of a designer *hox*-promoter-controlled NAD-dependent Glyceraldehyde-3-Phosphate Dehydrogenase **(35)** DNA construct (1655 bp) that includes a PCR FD primer (sequence 1-20), a 172-bp *Nostoc sp.* strain PCC 7120 (*Anabaena* PCC 7120) *hox* promoter (21-192), an enzyme-encoding sequence (193-1203) selected/modified from the sequence of a *Streptococcus pyogenes NZ131* NAD-dependent glyceraldehyde-3-phosphate dehydrogenase (GenBank: YP_002285269), a 432-bp *Nostoc sp.* strain PCC 7120 *gor* terminator (1204-1635), and a PCR RE primer (1636-1655).

SEQ ID NO: 105 presents example 105 of a designer *hox*-promoter-controlled Acetolactate Synthase **(53)** DNA construct (2303 bp) that includes a PCR FD primer (sequence 1-20), a 172-bp *Nostoc sp.* strain PCC 7120 (*Anabaena* PCC 7120) *hox* promoter (21-192), an enzyme-encoding sequence (193-1851) selected/modified from the sequence of a *Thermosynechococcus elongatus BP-1* acetolactate synthase (GenBank: NP_682614), a 432-bp *Nostoc sp.* strain PCC 7120 *gor* terminator (1852-2283), and a PCR RE primer (2284-2303).

SEQ ID NO: 106 presents example 106 of a designer *hox*-promoter-controlled Ketol-Acid Reductoisomerase **(54)** DNA construct (1661 bp) that includes a PCR FD primer (sequence 1-20), a 172-bp *Nostoc sp.* strain PCC 7120 (*Anabaena* PCC 7120) *hox* promoter (21-192), an enzyme-encoding sequence (193-1209) selected/modified from the sequence of a *Calditerrivibrio nitroreducens* ketol-acid reductoisomerase (GenBank: YP_004050904), a 432-bp *Nostoc sp. gor* terminator (1210-1641), and a PCR RE primer (1642-1661).

SEQ ID NO: 107 presents example 107 of a designer *hox*-promoter-controlled Dihydroxy-Acid Dehydratase **(55)** DNA construct (2324 bp) that includes a PCR FD primer (sequence 1-20), a 172-bp *Nostoc sp.* strain PCC 7120 (*Anabaena* PCC 7120) *hox* promoter (21-192), an enzyme-encoding sequence (193-1872) selected/modified from the sequence of a *Marivirga tractuosa DSM 4126* dihydroxy-acid dehydratase (GenBank: YP_004053736), a 432-bp *Nostoc sp. gor* terminator (1873-2304), and a PCR RE primer (2305-2324).

SEQ ID NO: 108 presents example 108 of a designer *hox*-promoter-controlled branched-chain alpha-Ketoacid Decarboxylase **(42)** DNA construct (2288 bp) that includes a PCR FD primer (sequence 1-20), a 172-bp *Nostoc sp.* (*Anabaena* PCC 7120) *hox* promoter (21-192), an enzyme-encoding sequence (193-1836) selected/modified from the sequence of a *Lactococcus lactis* branched-chain alpha-ketoacid decarboxylase (GenBank: AAS49166), a 432-bp *Nostoc sp. gor* terminator (1837-2268), and a PCR RE primer (2269-2288).

SEQ ID NO: 109 presents example 109 of a designer *hox*-promoter-controlled 2-Methylbutyraldehyde Reductase **(56)** DNA construct (1613 bp) that includes a PCR FD primer (sequence 1-20), a 172-bp *Nostoc sp.* (*Anabaena* PCC 7120) *hox* promoter (21-192), an enzyme-encoding sequence (193-1461) selected/modified from the sequence of a *Schizosaccharomyces japonicus y* 2-methylbutyraldehyde reductase (GenBank: XP_002173231), a 432-bp *Nostoc sp.* strain PCC 7120 *gor* terminator (1462-1893), and a PCR RE primer (1894-1613).

Note, in the designer transgenic *Nostoc* that contains designer *hox*-promoter-controlled genes of SEQ ID NOS: 104-109, *Nostoc's* native enzymes (genes) **of 34, 03-05, 36-39** and **45-52** are used in combination with the designer *hox*-promoter-controlled enzymes of **35, 53-55, 42** and **56** (encoded by DNA constructs of SEQ ID NOS: 104-109) to confer the Calvin-cycle 3-phophoglycerate-branched photosynthetic NADPH-enhanced pathways for photobiological production of 2-methyl-1-butanol from carbon dioxide and water (Figure 5).

Use of *Prochlorococcus marinus* MIT 9313 as a host organism in genetic transformation with SEQ ID NOS: 110-122 can create a designer transgenic *Prochlorococcus marinus* for photobiological production of isobutanol and/or 3-methyl-1-butanol (Figure 6). Briefly, SEQ ID NO:110 presents example 110 for a designer *groE*-promoter-controlled NAD-dependent Glyceraldehyde-3-Phosphate Dehydrogenase **(35)** DNA construct (1300 bp) that includes a PCR FD primer (sequence 1-20), a 137-bp *Prochlorococcus marinus* MIT 9313 heat- and light-responsive *groE* promoter (21-157), an enzyme-encoding sequence (158-1159) selected from a *Vibrio cholerae MJ-1236* NAD-dependent Glyceraldehyde-3-phosphate dehydrogenase (GenBank: ACQ61431), a 121-bp *Prochlorococcus marinus* MIT9313 *rbcS* terminator (1160-1280), and a PCR RE primer (1281-1300).

SEQ ID NO:111 presents example 111 for a designer *groE*-promoter-controlled Phosphoglycerate Mutase **(03)** DNA construct (1498 bp) that includes a PCR FD primer (sequence 1-20), a 137-bp *Prochlorococcus marinus* MIT9313 heat- and light-responsive *groE* promoter (21-157), an enzyme-encoding sequence (158-1357) selected from a *Pelotomaculum thermopropionicum SI* phosphoglycerate mutase (GenBank: YP_001212148), a 121-bp *Prochlorococcus marinus rbcS* terminator (1358-1478), and a PCR RE primer (1479-1498).

SEQ ID NO: 112 presents example 112 for a designer *groE*-promoter-controlled Enolase **(04)** DNA construct (1588 bp) that includes a PCR FD primer (sequence 1-20), a 137-bp *Prochlorococcus* heat- and light-responsive *groE* promoter (21-157), an enzyme-encoding sequence (158-1447) selected from a *Thermotoga* enolase (GenBank: ABQ46079), a 121-bp *Prochlorococcus marinus rbcS* terminator (1448-1568), and a PCR RE primer (1569-1588).

SEQ ID NO: 113 presents example 113 for a designer *groE*-promoter-controlled Pyruvate Kinase **(05)** DNA construct (1717 bp) that includes a PCR FD primer (sequence 1-20), a 137-bp *Prochlorococcus marinus* MIT9313 heat- and light-responsive *groE* promoter (21-157), an enzyme-encoding sequence (158-1576) selected from a *Thermotoga lettingae TMO* pyruvate kinase (GenBank: YP_001471580), a 121-bp *Prochlorococcus marinus* MIT9313 *rbcS* terminator (1577-1697), and a PCR RE primer (1698-1717).

SEQ ID NO: 114 presents example 114 for a designer *groE*-promoter-controlled Acetolactate Synthase **(53)** DNA construct (2017 bp) that includes a PCR FD primer (sequence 1-20), a 137-bp *Prochlorococcus marinus* MIT 9313 heat- and light-responsive *groE* promoter (21-157), an enzyme-encoding sequence (158-1876) selected from a *Bacillus licheniformis ATCC 14580* acetolactate synthase (GenBank: AAU42663), a 121-bp *Prochlorococcus marinus* MIT 9313 *rbcS* terminator (1877-1997), and a PCR RE primer (1998-2017).

SEQ ID NO: 115 presents example 115 for a designer *groE*-promoter-controlled Ketol-Acid Reductoisomerase **(54)** DNA construct (1588 bp) that includes a PCR FD primer (sequence 1-20), a 137-bp *Prochlorococcus marinus* MIT9313 heat- and light-responsive *groE* promoter (21-157), an enzyme-encoding sequence (158-1168) selected from a *Thermotoga petrophila RKU-1* ketol-acid reductoisomerase (GenBank: ABQ46398), a 400-bp *Prochlorococcus marinus* MIT9313 *rbcS* terminator (1169-1568), and a PCR RE primer (1569-1588).

SEQ ID NO:116 presents example 116 for a designer *groE*-promoter-controlled Dihydroxy-Acid Dehydratase **(55)** DNA construct (1960 bp) that includes a PCR FD primer (sequence 1-20), a 137-bp *Prochlorococcus marinus* heat- and light-responsive *groE* promoter (21-157), an enzyme-encoding sequence (158-1819) selected from a *Syntrophothermus lipocalidus DSM 12680* dihydroxy-acid dehydratase (GenBank: ADI02905), a 121-bp *Prochlorococcus marinus rbcS* terminator (1820-1940), and a PCR RE primer (1941-1960).

SEQ ID NO: 117 presents example 117 for a designer *groE*-promoter-controlled 2-Keto Acid Decarboxylase **(42)** DNA construct (1945 bp) that includes a PCR FD primer (sequence 1-20), a 137-bp *Prochlorococcus* heat- and light-responsive *groE* promoter (21-157), an enzyme-encoding sequence (158-1804) selected from a *Lactococcus lactis* Alpha-ketoisovalerate decarboxylase (GenBank: ADA65057), a 121-bp *Prochlorococcus rbcS* terminator (1805-1925), and a PCR RE primer (1926-1945).

SEQ ID NO:118 presents example 118 for a designer nirA-promoter-controlled Alcohol Dehydrogenase **(43/44)** DNA construct (1138 bp) that includes a PCR FD primer (sequence 1-20), a 251-bp *Prochlorococcus nirA* promoter (21-271), an enzyme-encoding sequence (272-997) selected from a *Geobacillus* short chain alcohol dehydrogenase (GenBank: YP_146837), a 121-bp Prochlorococcus rbcS terminator (998-1118), and a PCR RE primer (1119-1138).

Note, in the designer transgenic *Prochlorococcus* that contains the designer genes of SEQ ID NOS: 110-118, *Prochlorococcus's* native gene (enzyme) of **34** is used in combination with the designer *groE* and *nirA*-promoters-controlled genes (enzymes) of **35, 03-05, 53-55, 42** and **43/44** (encoded by DNA constructs of SEQ ID NOS: 110-118) to confer the Calvin-cycle 3-phophoglycerate-branched photosynthetic NADPH-enhanced pathways for photobiological production of isobutanol from carbon dioxide and water (Figure 6). Addition of the following four designer *groE* promoter-controlled genes (SEQ ID NO: 119-122) results in another designer transgenic *Prochlorococcus* that can produce both isobutanol and 3-methyl-1-butanol from carbon dioxide and water **(35, 03-05, 53-55, 42, 43/44,** plus **38-40** and **57** as shown in Figure 6).

Briefly, SEQ ID NO: 119 presents example 119 for a designer *groE*-promoter-controlled 2-Isopropylmalate Synthase **(40)** DNA construct (1816 bp) that includes a PCR FD primer (sequence 1-20), a 137-bp *Prochlorococcus marinus* MIT9313 heat- and light-responsive *groE* promoter (21-157), an enzyme-encoding sequence (158-1675) selected from a *Pelotomaculum thermopropionicum SI* 2-isopropylmalate synthase (GenBank: YP_001211081), a 121-bp *Prochlorococcus marinus rbcS* terminator (1676-1796), and a PCR RE primer (1797-1816).

SEQ ID NO: 120 presents example 120 for a designer *groE*-promoter-controlled 3-Isopropylmalate Dehydratase **(38)** DNA construct (2199 bp) that includes a PCR FD primer (sequence 1-20), a 137-bp *Prochlorococcus marinus* MIT9313 heat- and light-responsive *groE* promoter (21-157), a 3-isopropylmalate dehydratase large subunit-encoding sequence (158-1420) selected from a *Pelotomaculum thermopropionicum SI* 3-isopropylmalate dehydratase large subunit (GenBank: YP_001211082), a 137-bp *Prochlorococcus marinus* MIT9313 heat-and light-responsive *groE* promoter (1421-1557), a 3-isopropylmalate dehydratase small subunit-encoding sequence (1558-2058) selected from a *Pelotomaculum thermopropionicum SI* 3-isopropylmalate dehydratase small subunit (GenBank: YP_001211083), a 121-bp *Prochlorococcus marinus rbcS* terminator (2059-2179), and a PCR RE primer (2180-2199).

SEQ ID NO:121 presents example 121 for a designer *groE*-promoter-controlled 3-Isopropylmalate Dehydrogenase **(39)** DNA construct (1378 bp) that includes a PCR FD primer (sequence 1-20), a 137-bp *Prochlorococcus marinus* MIT9313 heat- and light-responsive *groE* promoter (21-157), an enzyme-encoding sequence (158-1237) selected from a *Syntrophothermus lipocalidus DSM 12680* 3-isopropylmalate dehydrogenase (GenBank: ADI02898), a 121-bp *Prochlorococcus marinus rbcS* terminator (1238-1358), and a PCR RE primer (1359-1378).

SEQ ID NO: 122 presents example 122 for a designer *groE*-promoter-controlled 3-Methylbutanal Reductase **(57)** DNA construct (1327 bp) that includes a PCR FD primer (sequence 1-20), a 137-bp *Prochlorococcus marinus* MIT9313 heat- and light-responsive *groE* promoter (21-157), an enzyme-encoding sequence (158-1186) selected from a *Saccharomyces cerevisiae S288c* 3-Methylbutanal reductase (GenBank: DAA10635), a 121-bp *Prochlorococcus marinus* MIT9313 *rbcS* terminator (1187-1307), and a PCR RE primer (1308-1327).

Note, the use of SEQ ID NOS: 110-117 and 119-122 in genetic transformation of *Prochlorococcus marinus* MIT 9313 creates another designer transgenic *Prochlorococcus marinus* with a *groE* promoter-controlled designer Calvin-cycle-channeled pathway (identified as **34** (native), **35, 03-05, 53-55, 38-40, 42** and **57** in Figure 6) for photobiological production of 3-methyl-1-butanol from carbon dioxide and water.

Use of *Cyanothece sp. ATCC 51142* as a host organism in genetic transformation with SEQ ID NOS: 123-128 can create a designer transgenic *Cyanothece* for photobiological production of 1-pentanol, 1-hexanol, and/or 1-heptanol (Figure 8). Briefly, SEQ ID NO: 123 presents example 123 for a designer *nirA*-promoter-controlled 2-Isopropylmalate Synthase **(40)** DNA construct (2004 bp) that includes a PCR FD primer (sequence 1-20), a 203-bp *Cyanothece sp. nirA* promoter (21-223), an enzyme-encoding sequence (224-1783) selected from a *Hydrogenobacter thermophilus* 2-isopropylmalate synthase sequence (GenBank: BAI69273), a 201-bp *Cyanothece sp.* rbcS terminator (1784-1984), and a PCR RE primer (1985-2004).

SEQ ID NO:124 presents example 124 for a designer *nirA*-promoter-controlled Isopropylmalate Isomerase **(41)** large/small subunits DNA construct (2648 bp) that includes a PCR FD primer (sequence 1-20), a 203-bp *Cyanothece sp. ATCC 51142 nirA* promoter (21-223), an enzyme-large-subunit-encoding sequence (224-1639) selected from a *Anoxybacillus flavithermus WK1* isopropylmalate isomerase large subunit sequence (GenBank: YP_002314962), a 203-bp *Cyanothece sp. ATCC 51142 nirA* promoter (1640-1842), an enzyme-small-subunit-encoding sequence (1843-2427) selected from a *Anoxybacillus flavithermus WK1* isopropylmalate isomerase small subunit sequence (GenBank: YP_002314963), a 201-bp *Cyanothece sp. ATCC 51142* rbcS terminator (2428-1628), and a PCR RE primer (2629-2648).

SEQ ID NO:125 presents example 125 for a designer g *nirA*-promoter-controlled 3-Isopropylmalate Dehydrogenase **(39)** DNA construct (1530 bp) that includes a PCR FD primer (sequence 1-20), a 203-bp *Cyanothece sp. ATCC 51142 nirA* promoter (21-223), an enzyme-encoding sequence (224-1309) selected from a *Thermosynechococcus elongatus BP-1 3-*isopropylmalate dehydrogenase sequence (GenBank: BAC09152), a 201-bp *Cyanothece sp. ATCC 51142* rbcS terminator (1310-1310), and a PCR RE primer (1311-1530).

SEQ ID NO:126 presents example 126 for a designer *nirA*-promoter-controlled 2-Keto Acid Decarboxylase **(42')** DNA construct (2088 bp) that includes a PCR FD primer (sequence 1-20), a 203-bp *Cyanothece nirA* promoter (21-223), an enzyme-encoding sequence (224-1867) selected from a *Lactococcus lactis* 2-keto acid decarboxylase (GenBank: AAS49166), a 201-bp *Cyanothece* rbcS terminator (1868-2068), and a PCR RE primer (2069-2088).

SEQ ID NO:127 presents example 127 for a designer *nirA*-promoter-controlled Hexanol Dehydrogenase **(12')** DNA construct (1503 bp) that includes a PCR FD primer (sequence 1-20), a 203-bp *Cyanothece nirA* promoter (21-223), an enzyme-encoding sequence (224-1282) selected from a *Mycobacterium chubuense* hexanol dehydrogenase (GenBank: ACZ56328), a 201-bp *Cyanothece* rbcS terminator (1283-1483), and a PCR RE primer (1484-1503).

SEQ ID NO:128 presents example 128 for a designer *nirA*-promoter-controlled short-chain Alcohol Dehydrogenase **(43', 43")** DNA construct (1149 bp) that includes a PCR FD primer (sequence 1-20), a 203-bp *Cyanothece sp. ATCC 51142 nirA* promoter (21-223), an enzyme-encoding sequence (224-928) selected from a *Pyrococcus furiosus DSM 3638* Short chain alcohol dehydrogenase (GenBank: AAC25556), a 201-bp *Cyanothece sp. ATCC 51142* rbcS terminator (929-1129), and a PCR RE primer (1130-1149).

Note, in the designer transgenic *Cyanothece* that contains designer *nirA* promoter-controlled genes of SEQ ID NOS: 123-127, *Cyanothece's* native enzymes of **34, 03-05, 36-38,** and **45-52** are used in combination with the designer *nirA*-promoters-controlled enzymes **of 35, 39-41 (39'-41', 39'-41'), 42'** and **12'** (encoded by DNA constructs of SEQ ID NOS: 123-127) to confer the Calvin-cycle 3-phophoglycerate-branched photosynthetic NADPH-enhanced pathways for photobiological production of 1-hexanol from carbon dioxide and water (Figure 8). Addition of a designer *nirA*-promoters-controlled gene (SEQ ID NO: 128) of a short chain alcohol dehydrogenase **43' (43")** with promiscuity results in another designer transgenic *Cyanothece* containing a Calvin-cycle-channeled pathway **(35, 39-41, 39'-43', 39'-43',** and **39"-43"** as shown in Figure 8) that can produce 1-pentanol, 1-hexanol, and 1-hexanol from carbon dioxide and water.

### Designer Advanced Photosynthetic Organisms with Calvin-Cycle-Channeled Pathways for Production of Butanol and Related Higher Alcohols

According to one of the various embodiments, use of certain designer DNA constructs in genetic transformation of eukaryotic photosynthetic organisms such as plant cells, eukaryotic aquatic plants (including, for example, eukaryotic algae, submersed aquatic herbs, duckweeds, water cabbage, water lily, water hyacinth, *Bolbitis heudelotii, Cabomba* sp., and seagrasses) can create designer transgenic eukaryotic photosynthetic organisms for production of butanol and related higher alcohols from carbon dioxide and water. Eukaryotic algae that can be selected for use as host organisms to create designer algae for photobiological production of butanol and related higher alcohols include (but not limited to): *Dunaliella salina, Dunaliella viridis, Dunaliella bardowil, Crypthecodinium cohnii, Schizochytrium sp., Chlamydomonas reinhardtii, Platymonas subcordiformis, Chlorella fusca, Chlorella sorokiniana, Chlorella vulgaris, 'Chlorella' ellipsoidea, Chlorella spp., Haematococcus pluvialis; Parachlorella kessleri, Betaphycus gelatinum, Chondrus crispus, Cyanidioschyzon merolae, Cyanidium caldarium, Galdieria sulphuraria, Gelidiella acerosa, Gracilaria changii, Kappaphycus alvarezii, Porphyra miniata, Ostreococcus tauri, Porphyra yezoensis, Porphyridium sp., Palmaria palmata, Gracilaria spp., Isochrysis galbana, Kappaphycus spp., Laminaria japonica, Laminaria spp., Monostroma spp., Nannochloropsis oculata, Porphyra spp., Porphyridium spp., Undaria pinnatifida, Ulva lactuca, Ulva spp., Undaria spp., Phaeodactylum Tricornutum, Navicula saprophila, Cylindrotheca fusiformis, Cyclotella cryptica, Euglena gracilis, Amphidinium sp., Symbiodinium microadriaticum, Macrocystis pyrifera, Ankistrodesmus braunii, Scenedesmus obliquus, Stichococcus sp., Platymonas sp., Dunalielki sauna, and Stephanoptera gracilis.*

According to another embodiment, the transgenic photosynthetic organism comprises a designer transgenic plant or plant cells selected from the group consisting of aquatic plants, plant cells, green algae, red algae, brown algae, blue-green algae (oxyphotobacteria including cyanobacteria and oxychlorobacteria), diatoms, marine algae, freshwater algae, salt-tolerant algal strains, cold-tolerant algal strains, heat-tolerant algal strains, antenna-pigment-deficient mutants, butanol-tolerant algal strains, higher-alcohols-tolerant algal strains, butanol-tolerant oxyphotobacteria, higher-alcohols-tolerant oxyphotobacteria, and combinations thereof.

According to another embodiment, said transgenic photosynthetic organism comprises a biosafety-guarded feature selected from the group consisting of: a designer proton-channel gene inducible under pre-determined inducing conditions, a designer cell-division-cycle iRNA gene inducible under pre-determined inducing conditions, a high-CO₂-requiring mutant as a host organism for transformation with designer biofuel-production-pathway genes in creating designer cell-division-controllable photosynthetic organisms, and combinations thereof.

The greater complexity and compartmentalization of eukaryotic plant cells allow for creation of a wider range of photobiologically active designer organisms and novel metabolic pathways compartmentally segregated for production of butanol and/or higher alcohols from water and carbon dioxide. In a eukaryotic algal cell, for example, the translation of designer nuclear genes occurs in cytosol whereas the photosynthesis/Calvin cycle is located inside an algal chloroplast. This clear separation of algal chloroplast photosynthesis from other subcellular functions such as the functions of cytoplasm membrane, cytosol and mitochondria can be used as an advantage in creation of a biosafety-guarded designer algae through an inducible insertion of designer proton-channels into cytoplasm membrane to permanently disable any cell division and/or mating capability while keeping the algal chloroplast functional work with the designer biofuel production , pathways to produce butanol and related higher alcohols. However, it is essential to genetically deliver designer enzyme(s) into the chloroplast to tame the Calvin cycle and funnel metabolism toward butanol directly from CO₂ and H₂O. This requires more complicated gene design to achieve desirable results.

According to one of various embodiments, designer Calvin-cycle-channeled pathway enzymes encoded with designer unclear genes are targetedly expressed into algal chloroplast through use of a transit signal peptide sequence. The said signal peptide is selected from the group consisting of the hydrogenase transit-peptide sequences (HydA1 and HydA2), ferredoxin transit-peptide sequence (Frx1), thioredoxin-m transit-peptide sequence (Trx2), glutamine synthase transit-peptide sequence (Gs2), LhcII transit-peptide sequences, PSII-T transit-peptide sequence (PsbT), PSII-S transit-peptide sequence (PsbS), PSII-W transit-peptide sequence (PsbW), CF₀CF₁ subunit-γ transit-peptide sequence (AtpC), CF₀CF₁ subunit-δ transit-peptide sequence (AtpD), CFoCF₁ subunit-II transit-peptide sequence (AtpG), photosystem I (PSI) transit-peptide sequences, Rubisco SSU transit-peptide sequences, and combinations thereof. Preferred transit peptide sequences include the Hydl transit peptide, the Frx1 transit peptide, and the Rubisco SSU transit peptides (such as RbcS2).

SEQ ID NOS. 129 -165 present examples for designer DNA constructs of designer chloroplast-targeted enzymes for creation of designer eukaryotic photosynthetic organisms such as designer algae with Calvin-cycle-channeled photosynthetic NADPH-enhanced pathways for photobiological production of butanol and related higher alcohols. Briefly, SEQ ID NO. 129 presents example 129 for a designer *Nial*-promoter-controlled chloroplast-targeted Phosphoglycerate Mutase **(03)** DNA construct (1910 bp) that includes a PCR FD primer (sequence 1-20), a 2 x 84-bp *Chlamydomonas* Nial (nitrate reductase) promoter (21-188), a 135-bp *Chlamydomonas RbcS2* transit peptide (189-323), a Phosphoglycerate Mutase-encoding sequence (324-1667) selected from *Nostoc azollae* Phosphoglycerate Mutase (ADI65627), a 223-bp *Chlamydomonas RbcS2* terminator (1668-1890), and a PCR RE primer (1891-1910).

SEQ ID NO. 130 presents example 130 for a designer Nial-promoter-controlled chloroplast-targeted Enolase **(04)** DNA construct (1856 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), an Enolase-encoding sequence (324-1613) selected/modified from *Nostoc azollae* Enolase (ADI63801), a 223-bp *Chlamydomonas RbcS2* terminator (1614-1836), and a PCR RE primer (18837-1856).

SEQ ID NO. 131 presents example 131 for a designer Nial-promoter-controlled chloroplast-targeted Pyruvate-Kinase **(05)** DNA construct (1985 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), an enzyme-encoding sequence (324-1742) selected/modified from *Cyanothece sp. PCC 8802* pyruvate-kinase (YP_003138017), a 223-bp *Chlamydomonas RbcS2* terminator (1743-1965), and a PCR RE primer (1966-1985).

SEQ ID NO. 132 presents example 132 for a designer Nial-promoter-controlled chloroplast-targeted NADPH-dependent Glyceraldehyde-3-Phosphate Dehydrogenase **(34)** DNA construct (1568 bp) that includes a PCR FD primer (sequence 1-20), a 2 x 84-bp *Chlamydomonas reinhardtii* Nial promoter (21-188), a 135-bp *Chlamydomonas RbcS2* transit peptide (189-323), a NADPH-dependent Glyceraldehyde-3-phosphate dehydrogenase-encoding sequence (324-1325) selected/modified from *Staphylococcus lugdunensis* NADPH-dependent glyceraldehyde-3-phosphate dehydrogenase (ADC87332), a 223-bp *Chlamydomonas RbcS2* terminator (1326-1548), and a PCR RE primer (1549-1568).

SEQ ID NO. 133 presents example 133 for a designer Nial-promoter-controlled chloroplast-targeted NAD-dependent Glyceraldehyde-3-phosphate dehydrogenase (35) DNA construct (1571 bp) that includes a PCR FD primer (sequence 1-20), a 2 x 84-bp *Chlamydomonas* Nial (nitrate reductase) promoter (21-188), a 135-bp *Chlamydomonas RbcS2* transit peptide (189-323), a NAD-dependent Glyceraldehyde-3-phosphate dehydrogenase-encoding sequence (324-1328) selected/modified from *Flavobacteriaceae bacterium* NAD-dependent Glyceraldehyde-3-phosphate dehydrogenase (YP_003095198), a 223-bp *Chlamydomonas RbcS2* terminator (1329-1551), and a PCR RE primer (1552-1571).

SEQ ID NO. 134 presents example 134 for a designer Nial-promoter-controlled chloroplast-targeted Citramalate Synthase **(36)** DNA construct (2150 bp) that includes a PCR FD primer (sequence 1-20), a 2 x 84-bp *Chlamydomonas* Nial (nitrate reductase) promoter (21-188), a 135-bp *Chlamydomonas RbcS2* transit peptide (189-323), a Citramalate Synthase-encoding sequence (324-1907) selected from *Hydrogenobacter* Citramalate Synthase (ADO45737), a 223-bp *Chlamydomonas RbcS2* terminator (1908-2130), and a PCR RE primer (2131-2150).

SEQ ID NO. 135 presents example 135 for a designer Nial-promoter-controlled chloroplast-targeted 3-Isopropylmalate/(R)-2-Methylmalate Dehydratase **(37)** large/small subunits DNA construct (3125 bp) that includes a PCR FD primer (sequence 1-20), a 2 x 84-bp *Chlamydomonas reinhardtii* Nial promoter (21-188), a 135-bp *Chlamydomonas RbcS2* transit peptide (189-323), a 3-isopropylmalate/(R)-2-methylmalate dehydratase large subunit-encoding sequence (324-2084) selected/modified from *Eubacterium eligens* 3-isopropylmalate/(R)-2-methylmalate dehydratase large subunit (YP_002930810), a 2 x 84-bp *Chlamydomonas* Nial promoter (2085-2252), a 135-bp *Chlamydomonas RbcS2* transit peptide (2253-2387), a 3-isopropylmalate/(R)-2-methylmalate dehydratase small subunit-encoding sequence (2388-2882) selected/modified from *Eubacterium eligens* 3-isopropylmalate/(R)-2-methylmalate dehydratase small subunit (YP_002930809), a 223-bp *Chlamydomonas RbcS2* terminator (2883-3105), and a PCR RE primer (3106-3125).

SEQ ID NO. 136 presents example 136 for a designer Nial-promoter-controlled chloroplast-targeted 3-Isopropylmalate Dehydratase **(38)** large/small subunits DNA construct (2879 bp) that includes a PCR FD primer (sequence 1-20), a 2 x 84-bp *Chlamydomonas* Nial promoter (21-188), a 135-bp *Chlamydomonas RbcS2* transit peptide (189-323), a 3-isopropylmalate dehydratase large subunit-encoding sequence (324-1727) selected/modified from *Cyanothece* 3-isopropylmalate dehydratase large subunit (YP_003886427), a 2 x 84-bp *Chlamydomonas* Nial promoter (1727-1894), a 135-bp *Chlamydomonas RbcS2* transit peptide (1895-2029), a 3-isopropylmalate dehydratase small subunit-encoding sequence (2030-2636) selected from *Cyanothece* 3-isopropylmalate dehydratase small subunit (YP_003889452), a 223-bp *Chlamydomonas r RbcS2* terminator (2637-2859), and a PCR RE primer (2860-2879).

SEQ ID NO. 137 presents example 137 for a designer Nial-promoter-controlled chloroplast-targeted 3-Isopropylmalate Dehydrogenase **(39)** DNA construct (1661 bp) that includes a PCR FD primer (sequence 1-20), a 2 x 84-bp *Chlamydomonas* Nial (nitrate reductase) promoter (21-188), a 135-bp *Chlamydomonas RbcS2* transit peptide (189-323), a 3-isopropylmalate dehydrogenase-encoding sequence (324-1418) selected/modified from *Cyanothece* 3-isopropylmalate dehydrogenase (YP_003888480), a 223-bp *Chlamydomonas RbcS2* terminator (1419-1641), and a PCR RE primer (1642-1661).

SEQ ID NO. 138 presents example 138 for a designer Nial-promoter-controlled chloroplast-targeted 2-Isopropylmalate Synthase **(40)** DNA construct (2174 bp) that includes a PCR FD primer (sequence 1-20), a 2 x 84-bp *Chlamydomonas* Nial promoter (21-188), a 135-bp *Chlamydomonas RbcS2* transit peptide (189-323), a 2-isopropylmalate synthase -encoding sequence (324-1931) selected/modified from *Cyanothece* 2-isopropylmalate synthase (YP_003890122), a 223-bp *Chlamydomonas RbcS2* terminator (1932-2154), and a PCR RE primer (2155-2174).

SEQ ID NO. 139 presents example 139 for a designer Nial-promoter-controlled chloroplast-targeted Isopropylmalate Isomerase **(41)** large/small subunit DNA construct (2882 bp) that includes a PCR FD primer (sequence 1-20), a 2 x 84-bp *Chlamydomonas* Nial promoter (21-188), a 135-bp *Chlamydomonas RbcS2* transit peptide (189-323), an isopropylmalate isomerase large subunit-encoding sequence (324-1727) selected/modified from *Anabaena variabilis* isopropylmalate isomerase large subunit (YP_324467), a 2 x 84-bp *Chlamydomonas reinhardtii* Nial promoter (1728-1895), a 135-bp *Chlamydomonas RbcS2* transit peptide (1896-2030), an isopropylmalate isomerase small subunit-encoding sequence (2031-2639) selected/modified from *Anabaena* isopropylmalate isomerase small subunit (YP_324466), a 223-bp *Chlamydomonas RbcS2* terminator (2640-2862), and a PCR RE primer (2863-2882).

SEQ ID NO. 140 presents example 140 for a designer Nial-promoter-controlled chloroplast-targeted 2-Keto Acid Decarboxylase **(42)** DNA construct (2210 bp) that includes a PCR FD primer (1-20), a 2 x 84-bp *Chlamydomonas* Nial promoter (21-188), a 135-bp *Chlamydomonas RbcS2* transit peptide (189-323), a 2-keto acid decarboxylase-encoding sequence (324-1967) selected from *Lactococcus* 2-keto acid decarboxylase (AAS49166), a 223-bp *Chlamydomonas RbcS2* terminator (1968-2190), and a PCR RE primer (2191-2210).

SEQ ID NO. 141 presents example 141 for a designer Nial-promoter-controlled chloroplast-targeted NADH-dependent Alcohol Dehydrogenase **(43)** DNA construct (1724 bp) that includes a PCR FD primer (sequence 1-20), a 2 x 84-bp *Chlamydomonas* Nial promoter (21-188), a 135-bp *Chlamydomonas RbcS2* transit peptide (189-323), a NADH-dependent alcohol dehydrogenase-encoding sequence (324-1481) selected/modified from *Gluconacetobacter hansenii* NADH-dependent alcohol dehydrogenase (ZP_06834544), a 223-bp *Chlamydomonas RbcS2* terminator (1482-1704), and a PCR RE primer (1705-1724).

SEQ ID NO. 142 presents example 142 for a designer Nial-promoter-controlled chloroplast-targeted NADPH-dependent Alcohol Dehydrogenase **(44)** DNA construct (1676 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), a NADPH-dependent alcohol dehydrogenase-encoding sequence (324-1433) selected/modified from *Fusobacterium* NADPH-dependent alcohol dehydrogenase (ZP_04573952), a 223-bp *Chlamydomonas reinhardtii RbcS2* terminator (1434-1656), and a PCR RE primer (1657-1676).

Note, use of SEQ ID NOS. 129-141 (and/or 142) in genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced pathway **(03-05, 34-43/44** in Figure 4) for photobiological production of 1-butanol from carbon dioxide and water.

SEQ ID NO. 143 presents example 143 for a designer Nial-promoter-controlled chloroplast-targeted Phosphoenolpyruvate Carboxylase **(45)** DNA construct (3629 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), a Phosphoenolpyruvate Carboxylase-encoding sequence (324-3386) selected/modified from *Cyanothece sp. PCC* 7822 Phosphoenolpyruvate Carboxylase (YP_003887888), a 223-bp *Chlamydomonas reinhardtii RbcS2* terminator (3387-3609), and a PCR RE primer (3610-3629).

SEQ ID NO. 144 presents example 144 for a designer Nial-promoter-controlled chloroplast-targeted Aspartate Aminotransferase **(46)** DNA construct (1745 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), a Aspartate Aminotransferase-encoding sequence (324-1502) selected/modified from *Synechococcus elongatus PCC 6301* Aspartate Aminotransferase (YP_172275), a 223-bp *Chlamydomonas reinhardtii RbcS2* terminator (1503-1525), and a PCR RE primer (1526-1745).

SEQ ID NO. 145 presents example 145 for a designer Nial-promoter-controlled chloroplast-targeted Aspartokinase **(47)** DNA construct (2366 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), an Aspartokinase-encoding sequence (324-2123) selected/modified from *Cyanothece* Aspartokinase (YP_003136939), a 223-bp *Chlamydomonas RbcS2* terminator (2124-2346), and a PCR RE primer (2347-2366).

SEQ ID NO. 146 presents example 146 for a designer Nial-promoter-controlled chloroplast-targeted Aspartate-Semialdehyde Dehydrogenase **(48)** DNA construct (1604 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), an Aspartate-semialdehyde dehydrogenase-encoding sequence (324-1361) selected/modified from *Trichodesmium erythraeum IMS101* Aspartate-semialdehyde dehydrogenase (ABG50031), a 223-bp *Chlamydomonas RbcS2* terminator (1362-1584), and a PCR RE primer (1585-1604).

SEQ ID NO. 147 presents example 147 for a designer Nial-promoter-controlled chloroplast-targeted Homoserine Dehydrogenase **(49)** DNA construct (1868 bp) that includes a PCR FD primer (sequence 1-20), a 2 x 84-bp *Chlamydomonas* Nial promoter (21-188), a 135-bp *Chlamydomonas RbcS2* transit peptide (189-323), a homoserine dehydrogenase-encoding sequence (324-1625) selected from *Cyanothece* homoserine dehydrogenase (YP_003887242), a 223-bp *Chlamydomonas RbcS2* terminator (1626-1848), and a PCR RE primer (1849-1868).

SEQ ID NO. 148 presents example 148 for a designer Nial-promoter-controlled chloroplast-targeted Homoserine Kinase **(50)** DNA construct (1472 bp) that includes a PCR FD primer (sequence 1-20), a 2 x 84-bp *Chlamydomonas* Nial promoter (21-188), a 135-bp *Chlamydomonas RbcS2* transit peptide (189-323), a Homoserine kinase-encoding sequence (324-1229) selected/modified from *Cyanothece* Homoserine kinase (YP_003886645), a 223-bp *Chlamydomonas RbcS2* terminator (1230-1452), and a PCR RE primer (1453-1472).

SEQ ID NO. 149 presents example 149 for a designer Nial-promoter-controlled chloroplast-targeted Threonine Synthase **(51)** DNA construct (1655 bp) that includes a PCR FD primer (sequence 1-20), a 2 x 84-bp *Chlamydomonas* Nial promoter (21-188), a 135-bp *Chlamydomonas RbcS2* transit peptide (189-323), a Threonine synthase -encoding sequence (324-1412) selected/modified from *Cyanothece* Threonine synthase (YP_002485009), a 223-bp *Chlamydomonas RbcS2* terminator (1413-1635), and a PCR RE primer (1636-1655).

SEQ ID NO. 150 presents example 150 for a designer Nial-promoter-controlled chloroplast-targeted Threonine Ammonia-Lyase **(52)** DNA construct (2078 bp) that includes a PCR FD primer (sequence 1-20), a 2 x 84-bp *Chlamydomonas* Nial promoter (21-188), a 135-bp *Chlamydomonas RbcS2* transit peptide (189-323), a threonine ammonia-lyase-encoding sequence (324-1835) selected/modified from *Synechococcus* threonine ammonia-lyase (ZP_05035047), a 223-bp *Chlamydomonas RbcS2* terminator (1836-2058), and a PCR RE primer (2059-2078).

Note, use of SEQ ID NOS. 129, 130, 132, 133, 143-150, 137-141 (and/or 141) through genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced pathway **(03, 04, 34, 35, 45-52, 39-43/44** in Figure 4) for photobiological production of 1-butanol from carbon dioxide and water.

SEQ ID NO. 151 presents example 151 for a designer Nial-promoter-controlled chloroplast-targeted Acetolactate Synthase (53) DNA construct (2282 bp) that includes a PCR FD primer (sequence 1-20), a 2 x 84-bp *Chlamydomonas reinhardtii* Nial promoter (21-188), a 135-bp *Chlamydomonas RbcS2* transit peptide (189-323), an acetolactate synthase-encoding sequence (324-2039) selected from *Bacillus subtilis* acetolactate synthase (CAB07802), a 223-bp *Chlamydomonas RbcS2* terminator (2040-2262), and a PCR RE primer (2263-2282).

SEQ ID NO. 152 presents example 152 for a designer Nial-promoter-controlled chloroplast-targeted Ketol-Acid Reductoisomerase **(54)** DNA construct (1562 bp) that includes a PCR FD primer (sequence 1-20), a 2 x 84-bp *Chlamydomonas* Nial promoter (21-188), a 135-bp *Chlamydomonas RbcS2* transit peptide (189-323), an enzyme-encoding sequence (324-1319) selected/modified from *Cyanothece* ketol-acid reductoisomerase (YP_003885458), a 223-bp *Chlamydomonas RbcS2* terminator (1320-1542), and a PCR RE primer (1543-1562).

SEQ ID NO. 153 presents example 153 for a designer Nial-promoter-controlled chloroplast-targeted Dihydroxy-Acid Dehydratase **(55)** DNA construct (2252 bp) that includes a PCR FD primer (sequence 1-20), a 2 x 84-bp *Chlamydomonas* Nial promoter (21-188), a 135-bp *Chlamydomonas RbcS2* transit peptide (189-323), a dihydroxy-acid dehydratase-encoding sequence (324-2009) selected from *Cyanothece* dihydroxy-acid dehydratase (YP_003887466), a 223-bp *Chlamydomonas RbcS2* terminator (2010-2232), and a PCR RE primer (2233-2252).

SEQ ID NO. 154 presents example 154 for a designer Nial-promoter-controlled chloroplast-targeted 2-Methylbutyraldehyde Reductase **(56)** DNA construct (1496 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), an enzyme-encoding sequence (324-1253) selected/modified from *Pichia pastoris GS115 2-*methylbutyraldehyde reductase (XP_002490018), a 223-bp *Chlamydomonas reinhardtii RbcS2* terminator (1254-1476), and a PCR RE primer (1477-1496).

Note, use of SEQ ID NOS. 129-137, 140, and 151-154 in genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced pathway **(03-05, 34-39, 53-55, 42,** and **56** in Figure 5) for photobiological production of 2-methyl-1-butanol from carbon dioxide and water.

SEQ ID NO. 155 presents example 155 for a designer Nial-promoter-controlled chloroplast-targeted 3-Methylbutanal Reductase **(57)** DNA construct (1595 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), a 3-methylbutanal reductase-encoding sequence (324-1352) selected/modified from *Saccharomyces cerevisiae S288c* 3-methylbutanal reductase (DAA10635), a 223-bp *Chlamydomonas reinhardtii RbcS2* terminator (1353-1575), and a PCR RE primer (1576-1595).

Note, use of SEQ ID NOS. 129-133, 151-153, 140 and 141 (or 142) in genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced pathway **(03-05, 34, 35, 53-55, 42,** and **43 (44)** in Figure 6) for photobiological production of isobutanol from carbon dioxide and water. Whereas, SEQ ID NOS. 129-133, 151-153, 136-138, 140 and 155 represent a designer eukaryotic photosynthetic organism such as designer *Chlamydomonas* with a Calvin-cycle 3-phosphogylcerate-branched NADPH-enhanced pathway **(03-05, 34, 35, 53-55, 40, 38, 39, 42,** and 57 in Figure 6) that can photobiologically produce 3-methyl-1-butanol from carbon dioxide and water.

SEQ ID NO. 156 presents example 156 for a designer Nial-promoter-controlled chloroplast-targeted NADH-dependent Butanol Dehydrogenase (**12**a) DNA construct (1739 bp) that includes a PCR FD primer (sequence 1-20), a 2 x 84-bp *Chlamydomonas reinhardtii* Nial (nitrate reductase) promoter (21-188), a 135-bp *Chlamydomonas reinhardtii RbcS2* transit peptide (189-323), an enzyme-encoding sequence (324-1496) selected/modified from *Clostridium perfringens* NADH-dependent butanol dehydrogenase (NP_561774), a 223-bp *Chlamydomonas RbcS2* terminator (1497-1719), and a PCR RE primer (1720-1739).

SEQ ID NO. 157 presents example 157 for a designer Nial-promoter-controlled chloroplast-targeted NADPH-dependent Butanol Dehydrogenase (**12**b) DNA construct (1733 bp) that includes a PCR FD primer (sequence 1-20), a 2 x 84-bp *Chlamydomonas reinhardtii* Nial promoter (21-188), a 135-bp *Chlamydomonas reinhardtii RbcS2* transit peptide (189-323), an enzyme-encoding sequence (324-1490) selected/modified from *Clostridium saccharobutylicum* NADPH-dependent butanol dehydrogenase (AAA83520), a 223-bp *Chlamydomonas reinhardtii RbcS2* terminator (1491-1713), and a PCR RE primer (1714-1733).

Note, use of SEQ ID NOS. 129-140 and 156 (and/or 157) in genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced butanol production pathway **(03-05, 34-42** and **12** in Figure 4) for more specific photobiological production of 1-butanol from carbon dioxide and water. Similarly, SEQ ID NOS. 129, 130, 132, 133, 143-150, 137-140, and 156 (and/or 157) represent another designer eukaryotic photosynthetic organism such as designer *Chlamydomonas* with a Calvin-cycle 3-phosphogylcerate-branched NADPH-enhanced butanol-production pathway **(03, 04, 34, 35, 45-52, 39-42** and **12** in Figure 4) for photobiological production of 1-butanol from carbon dioxide and water.

SEQ ID NO. 158 presents example 158 for a designer Nial-promoter-controlled chloroplast-targeted 3-Ketothiolase **(07')** DNA construct (1745 bp) that includes a PCR FD primer (sequence 1-20), a 2 x 84-bp *Chlamydomonas* Nial (nitrate reductase) promoter (21-188), a 135-bp *Chlamydomonas RbcS2* transit peptide (189-323), a 3-Ketothiolase-encoding sequence (324-1502) selected/modified from *Azohydromonas lata* 3-Ketothiolase (AAD10275), a 223-bp *Chlamydomonas RbcS2* terminator (1503-1725), and a PCR RE primer (1726-1745).

SEQ ID NO. 159 presents a designer Nial-promoter-controlled chloroplast-targeted 3-Hydroxyacyl-CoA dehydrogenase **(08')** DNA construct (1439 bp) that includes a PCR FD primer (sequence 1-20), a 2 x 84-bp *Chlamydomonas* Nial promoter (21-188), a 135-bp *Chlamydomonas RbcS2* transit peptide (189-323), an enzyme-encoding sequence (324-1196) selected/modified from *Oceanithermus* 3-Hydroxyacyl-CoA dehydrogenase (ADR36325), a 223-bp *Chlamydomonas RbcS2* terminator (1197-1419), and a PCR RE primer (1420-1439).

SEQ ID NO. 160 presents example 160 for a designer Nial-promoter-controlled chloroplast-targeted Enoyl-CoA dehydratase **(09')** DNA construct (1337 bp) that includes a PCR FD primer (sequence 1-20), a 2 x 84-bp *Chlamydomonas* Nial promoter (21-188), a 135-bp *Chlamydomonas RbcS2* transit peptide (189-323), an enzyme-encoding sequence (324-1094) selected/modified from *Bordetella petrii* Enoyl-CoA dehydratase (YP_001629844), a 223-bp *Chlamydomonas RbcS2* terminator (1095-1317), and a PCR RE primer (1318-1337).

SEQ ID NO. 161 presents example 161 for a designer Nial-promoter-controlled 2-Enoyl-CoA reductase **(10')** DNA construct (1736 bp) that includes a PCR FD primer (sequence 1-20), a 2 x 84-bp *Chlamydomonas* Nial promoter (21-188), a 135-bp *Chlamydomonas RbcS2* transit peptide (189-323), an enzyme-encoding sequence (324-1493) selected/modified from *Xanthomonas campestris* 2-Enoyl-CoA reductase (YP_001905744), a 223-bp *Chlamydomonas RbcS2* terminator (1494-1716), and a PCR RE primer (1717-1736).

SEQ ID NO. 162 presents example 162 for a designer Nial-promoter-controlled chloroplast-targeted Acyl-CoA reductase **(11')** DNA construct (2036 bp) that includes a PCR FD primer (sequence 1-20), a 2 x 84-bp *Chlamydomonas reinhardtii* Nial promoter (21-188), a 135-bp *Chlamydomonas RbcS2* transit peptide (189-323), an enzyme-encoding sequence (324-1793) selected/modified from *Thermosphaera aggregans* Acyl-CoA reductase (YP_003649571), a 223-bp *Chlamydomonas RbcS2* terminator (1794-2016), and a PCR RE primer (2017-2036).

SEQ ID NO. 163 presents example 163 for a designer Nial-promoter-controlled chloroplast-targeted Hexanol Dehydrogenase **(12')** DNA construct (1625 bp) that includes a PCR FD primer (sequence 1-20), a 2 x 84-bp *Chlamydomonas* Nial promoter (21-188), a 135-bp *Chlamydomonas RbcS2* transit peptide (189-323), an enzyme-encoding sequence (324-1382) selected/modified from *Mycobacterium chubuense* hexanol dehydrogenase (ACZ56328), a 223-bp *Chlamydomonas RbcS2* terminator (1383-1605), and a PCR RE primer (1606-1625).

Note, use of SEQ ID NOS. 158-163 with other proper DNA constructs such as SEQ ID NOS. 132 and 133 in genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced hexanol production pathway **(34, 35, 03-10,** and **07'-12'** in Figure 7) for photobiological production of 1-hexanol from carbon dioxide and water.

SEQ ID NO. 164 presents example 164 for a designer Nial-promoter-controlled chloroplast-targeted Octanol Dehydrogenase **(12")** DNA construct (1249 bp) that includes a PCR FD primer (sequence 1-20), a 2 x 84-bp *Chlamydomonas* Nial promoter (21-188), a 135-bp *Chlamydomonas RbcS2* transit peptide (189-323), an enzyme-encoding sequence (324-1006) selected/modified from *Drosophila subobscura* Octanol dehydrogenase (ABO65263), a 223-bp *Chlamydomonas RbcS2* terminator (1007-1229), and a PCR RE primer (1230-1249).

Note, SEQ ID NOS. 132, 133, and 158-163 represent a designer eukaryotic photosynthetic organism such as a designer *Chlamydomonas* with a designer hydrocarbon chain elongation pathway **(34, 35, 07'-12'** as shown in Figure 7) for photobiological production of 1-hexanol. SEQ ID NOS: 132, 133, 158-162 and 164 represent another designer eukaryotic photosynthetic organism such as a designer *Chlamydomonas* with a designer hydrocarbon chain elongation pathway **(34, 35, 07'-10'** and **07"-12"** as shown in Figure 7) for photobiological production of 1-octanol.

SEQ ID NO. 165: a designer Nial-promoter-controlled chloroplast-targeted Short Chain Alcohol Dehydrogenase **(43')** DNA construct (1769 bp) that includes a PCR FD primer (sequence 1-20), a 2 x 84-bp *Chlamydomonas* Nial promoter (21-188), a 135-bp *Chlamydomonas RbcS2* transit peptide (189-323), an enzyme-encoding sequence (324-1526) selected/modified from *Burkholderia* Short chain alcohol dehydrogenase (ABO56626), a 223-bp *Chlamydomonas RbcS2* terminator (1527-1749), and a PCR RE primer (1750-1769).

Note, use of SEQ ID NOS. 129-140 and 165 in genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced pathway **(03-05, 34-41, 39'-43', 39'-43'** and **39"-43"** in Figure 8) for photobiological production of 1-pentanol, 1-hexanol, and 1-heptanol from carbon dioxide and water. Similarly, SEQ ID NOS. 129-140 and 163 represent another designer eukaryotic photosynthetic organism such as designer *Chlamydomonas* with a Calvin-cycle 3-phosphogylcerate-branched NADPH-enhanced pathway **(03-05, 34-41, 39'-41', 39'-42'** and **12'** in Figure 8) for photobiological production of 1-hexanol from carbon dioxide and water.

Likewise, use of SEQ ID NOS. 129-137, 151-153, 138-140 and 165 through genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced pathway **(03-05, 34-39, 53-55, 39'-43', 39'-43',** and 39"-43" in Figure 9) for photobiological production of 3-methyl-1-pentanol, 4-methyl-1-hexanol, and 5-methyl-1-heptanol from carbon dioxide and water; The expression of SEQ ID NOS. 129, 130, 132,133, 143-150,151-153, 137-140 and 165 in an eukaryotic photosynthetic organism such as a host *Chlamydomonas* represent another designer eukaryotic photosynthetic organism with a Calvin-cycle 3-phosphogylcerate-branched NADPH-enhanced pathway **(03, 05, 34, 35, 42-55, 39'-43', 39'-43',** and **39"-43"** in Figure 9) for photobiological production of 3-methyl-1-pentanol, 4-methyl-1-hexanol, and 5-methyl-1-heptanol from carbon dioxide and water; The expression of SEQ ID NOS. 129-133,151-153, 136-140 and 165 in a host eukaryotic photosynthetic organism such as *Chlamydomonas* represent yet another designer eukaryotic photosynthetic organism with a Calvin-cycle 3-phosphogylcerate-branched NADPH-enhanced pathway **(03-05, 34, 35, 53-55, 40, 38, 39, 39'-43', 39'-43',** and **39"-43"** in Figure 10) for photobiological production of 4-methyl-1-pentanol, 5-methyl-1-hexanol, and 6-methyl-1-heptanol from carbon dioxide and water.

### Use Of Designer Photosynthetic Organisms With Photobioreactor For Production and Harvesting of Butanol and Related Higher Alcohols

The designer photosynthetic organisms with designer Calvin-cycle channeled photosynthetic NADPH-enhanced pathways (Figures 1, and 4-10) can be used with photobioreactors for production and harvesting of butanol and/or related higher alcohols. The said butanol and/or related higher alcohols are selected from the group consisting of: 1-butanol, 2-methyl-1-butanol, isobutanol, 3-methyl-1-butanol, 1-hexanol, 1-octanol, 1-pentanol, 1-heptanol, 3-methyl-1-pentanol, 4-methyl-1-hexanol, 5-methyl-1-heptanol, 4-methyl-1-pentanol, 5-methyl-1-hexanol, 6-methyl-1-heptanol, and combinations thereof.

The said designer photosynthetic organisms such as designer transgenic oxyphotobacteria and algae comprise designer Calvin-cycle-channeled and photosynthetic NADPH-enhanced pathway gene(s) and biosafety-guarding technology for enhanced photobiological production of butanol and related higher alcohols from carbon dioxide and water. According to one of the various embodiments, it is a preferred practice to grow designer photosynthetic organisms photoautotrophically using carbon dioxide (CO₂) and water (H₂O) as the sources of carbon and electrons with a culture medium containing inorganic nutrients. The nutrient elements that are commonly required for oxygenic photosynthetic organism growth are: N, P, and K at the concentrations of about 1-10 mM, and Mg, Ca, S, and Cl at the concentrations of about 0.5 to 1.0 mM, plus some trace elements Mn, Fe, Cu, Zn, B, Co, Mo among others at µM concentration levels. All of the mineral nutrients can be supplied in an aqueous minimal medium that can be made with well-established recipes of oxygenic photosynthetic organism (such as algal) culture media using water (freshwater for the designer freshwater algae; seawater for the salt-tolerant designer marine algae) and relatively small of inexpensive fertilizers and mineral salts such as ammonium bicarbonate (NH₄HCO₃) (or ammonium nitrate, urea, ammonium chloride), potassium phosphates (K₂HPO₄ and KH₂PO₄), magnesium sulfate heptahydrate (MgSO₄.7H₂O), calcium chloride (CaCl₂), zinc sulfate heptahydrate (ZnSO₄.7H₂O), iron (II) sulfate heptahydrate (FeSO₄.7 H₂O), and boric acid (H₃BO₃), among others. That is, large amounts of designer algae (or oxyphotobacteria) cells can be inexpensively grown in a short period of time because, under aerobic conditions such as in an open pond, the designer algae can photoautotrophically grow by themselves using air CO₂ as rapidly as their wild-type parental strains. This is a significant feature (benefit) of the invention that could provide a cost-effective solution in generation of photoactive biocatalysts (the designer photosynthetic biofuel-producing organisms such as designer algae or oxyphotobacteria) for renewable solar energy production.

According to one of the various embodiments, when designer photosynthetic organism culture is grown and ready for photobiological production of butanol and/or related higher alcohols, the designer photosynthetic organism cells are then induced to express the designer Calvin-cycle channeled photosynthetic NADPH-enhanced pathway(s) to photobiologically produce butanol and/or related higher alcohols from carbon dioxide and water. The method of induction is designer pathway gene(s) specific. For example, if/when a nirA promoter is used to control the designer Calvin-cycle channeled pathway gene(s) such as those of SEQ ID NOS: 58-69 and 72 (and/or 73) which represent a designer transgenic *Thermosynechococcus* that comprises the designer genes of a Calvin-cycle 3-phophoglycerate-branched photosynthetic NADPH-enhanced pathway (numerically labeled as **34, 35, 03-05, 36-42,** and **12** in Figure 4) for photobiological production of1-butanol from carbon dioxide and water, the designer transgenic *Thermosynechococcus* is grown in a minimal liquid culture medium containing ammonium (but no nitrate) and other inorganic nutrients. When the designer transgenic *Thermosynechococcus* culture is grown and ready for photobiological production of biofuel 1-butanol, nitrate fertilizer will then be added into the culture medium to induce the expression of the designer nirA-controlled Calvin-cycle-channeled pathway to photobiologically produce 1-butanol from carbon dioxide and water in this example.

For the designer photosynthetic organism(s) with anaerobic promoter-controlled pathway(s) such as the designer transgenic *Nostoc* that contains designer *hox*-promoter-controlled Calvin-cycle 3-phophoglycerate-branched pathway genes of SEQ ID NOS. 104-109, anaerobic conditions can be used to induce the expression of the designer pathway gene(s) for photobiological production of 2-methyl-1-butanol from carbon dioxide and water (Figure 5). That is, when the designer transgenic *Nostoc* culture is grown and ready for photobiological biofuel production, its cells will then be placed (or sealed) into certain anaerobic conditions to induce the expression of the designer *hox*-controlled pathway gene(s) to photobiologically produce 2-methyl-1-butanol from carbon dioxide and water.

For those designer photosynthetic organism(s) that contains a heat- and light-responsive promoter-controlled and nirA-promoter-controlled pathway(s) such as the designer transgenic *Prochlorococcus* that contains a set of designer *groE*-promoter-controlled and nirA-promoter-controlled Calvin-cycle 3-phophoglycerate-branched pathway genes of SEQ ID NOS. 110-118, light and heat are used in conjunction of nitrate addition to induce the expression of the designer pathway genes for photobiological production of isobutanol from carbon dioxide and water (Figure 6).

According to another embodiment, use of designer marine algae or marine oxyphotobacteria enables the use of seawater and/or groundwater for photobiological production of biofuels without requiring freshwater or agricultural soil. For example, designer *Prochlorococcus marinus* that contains the designer genes of SEQ ID NOS: 110-117 and 119-122 can use seawater and/or certain groundwater for photoautotrophic growth and synthesis of 3-methyl-1-butanol from carbon dioxide and water with its *groE* promoter-controlled designer Calvin-cycle-channeled pathway (identified as **34** (native), **35, 03-05, 53-55, 38-40, 42** and **57** in Figure 6). The designer photosynthetic organisms can be used also in a sealed photobioreactor that is operated on a desert for production of isobutanol with highly efficient use of water since there will be little or no water loss by evaporation and/or transpiration that a common crop system would suffer. That is, this embodiment may represent a new generation of renewable energy (butanol and related higher alcohols) production technology without requiring arable land or freshwater resources.

According to another embodiment, use of nitrogen-fixing designer oxyphotobacteria enables photobiological production of biofuels without requiring nitrogen fertilizer. For example, the designer transgenic *Nostoc* that contains designer *hox*-promoter-controlled genes of SEQ ID NOS.104-109 is capable of both fixing nitrogen (N₂) and photobiologically producing 2-methyl-1-butanol from carbon dioxide and water (Figure 6). Therefore, use of the designer transgenic *Nostoc* enables photoautotrophic growth and 2-methyl-1-butanol synthesis from carbon dioxide and water.

Certain designer oxyphotobacteria are designed to perform multiple functions. For example, the designer transgenic *Cyanothece* that contains designer *nirA* promoter-controlled genes of SEQ ID NOS. 123-127 is capable of (1) using seawater, (2) N₂ fixing nitrogen, and photobiological producing 1-hexanol from carbon dioxide and water (Figure 8). Use of this type of designer oxyphotobacteria enables photobiological production of advanced biofuels such as 1-hexanol using seawater without requiring nitrogen fertilizer

According to one of various embodiments, a method for photobiological production and harvesting of butanol and related higher alcohols comprises: a) introducing a transgenic photosynthetic organism into a photobiological reactor system, the transgenic photosynthetic organism comprising transgenes coding for a set of enzymes configured to act on an intermediate product of a Calvin cycle and to convert the intermediate product into butanol and related higher alcohols; b) using reducing power and energy associated with the transgenic photosynthetic organism acquired from photosynthetic water splitting and proton gradient coupled electron transport process in the photobioreactor to synthesize butanol and related higher alcohols from carbon dioxide and water; and c) using a product separation process to harvest the synthesized butanol and/or related higher alcohols from the photobioreactor.

In summary, there are a number of embodiments on how the designer organisms may be used for photobiological butanol (and/or related higher alcohols) production. One of the preferred embodiments is to use the designer organisms for direct photosynthetic butanol production from CO₂ and H₂O with a photobiological reactor and butanol-harvesting (filtration and distillation/evaporation) system, which includes a specific operational process described as a series of the following steps: a) Growing a designer transgenic organism photoautotrophically in minimal culture medium using air CO₂ as the carbon source under aerobic (normal) conditions before inducing the expression of the designer butanol-production-pathway genes; b) When the designer organism culture is grown and ready for butanol production, sealing or placing the culture into a specific condition to induce the expression of designer Calvin-cycle-channeled pathway genes; c) When the designer pathway enzymes are expressed, supplying visible light energy such as sunlight for the designer-genes-expressed cells to work as the catalysts for photosynthetic production of butanol and/or related higher alcohols from CO₂ and H₂O; d) Harvesting the product butanol and/or related higher alcohols by any method known to those skilled in the art. For example, harvesting the butanol and/or related higher alcohols from the photobiological reactor can be achieved by a combination of membrane filtration and distillation/evaporation butanol-harvesting techniques.

The above process to use the designer organisms for photosynthetic production and harvesting of butanol and related higher alcohols can be repeated for a plurality of operational cycles to achieve more desirable results. Any of the steps a) through d) of this process described above can also be adjusted in accordance of the invention to suit for certain specific conditions. In practice, any of the steps a) through d) of the process can be applied in full or in part, and/or in any adjusted combination as well for enhanced photobiological production of butanol and higher alcohol in accordance of this invention.

In addition to butanol and/or related higher alcohols production, it is also possible to use a designer organism or part of its designer butanol-production pathway(s) to produce certain intermediate products of the designer Calvin-cycle-channeled pathways (Figs. 1 and 4-10) including (but not limited to): butyraldehyde, butyryl-CoA, crotonyl-CoA, 3-hydroxybutyryl-CoA, acetoacetyl-CoA, acetyl-CoA, pyruvate, phosphoenolpyruvate, 2-phosphoglycerate, 1,3-diphosphoglycerate, glyceraldehye-3-phosphate, dihydroxyacetone phosphate, fructose-1,6-diphosphate, fructose-6-phosphate, glucose-6-phosphate, glucose, glucose-1-phosphate, citramalate, citraconate, methyl-D-malate, 2-ketobutyrate, 2-ketovalerate, oxaloacetate, aspartate, homoserine, threonine, 2-keto-3-methylvalerate, 2-methylbutyraldehyde, 3-methylbutyraldehyde, 4-methyl-2-oxopentanoate, 3-isopropylmalate, 2-isopropylmalate, 2-oxoisovalerate, 2,3-dihydroxy-isovalerate, 2-acetolactate, isobutyraldehyde, 3-keto-C6-acyl-CoA, 3-hydroxy-C6-acyl-CoA, C6-enoyl-CoA, C6-acyl-CoA, 3-keto-C8-acyl-CoA, 3-hydroxy-C8-acyl-CoA, C8-enoyl-CoA, C8-acyl-CoA, octanal, 1-pentanol, 1-hexanal, 1-heptanal, 2-ketohexanoate, 2-ketoheptanoate, 2-ketooctanoate, 2-ethylmalate, 3-ethylmalate, 3-methyl-1-pentanal, 4-methyl-1-hexanal, 5-methyl-1-heptanal, 2-hydroxy-2-ethyl-3-oxobutanoate, 2,3-dihydroxy-3-methyl-pentanoate, 2-keto-4-methyl-hexanoate, 2-keto-5-methyl-heptnoate, 2-keto-6-methyl-octanoate, 4-methyl-1-pentanal, 5-methyl-1-hexanal, 6-methyl-1-heptanal, 2-keto-7-methyl-octanoate, 2-keto-6-methyl-heptanoate, and 2-keto-5-methyl-hexanoate. According to one of various embodiments, therefore, a further embodiment comprises an additional step of harvesting the intermediate products that can be produced also from an induced transgenic designer organism. The production of an intermediate product can be selectively enhanced by switching off a designer-enzyme activity that catalyzes its consumption in the designer pathways. The production of a said intermediate product can be enhanced also by using a designer organism with one or some of designer enzymes omitted from the designer butanol-production pathways. For example, a designer organism with the butanol dehydrogenase or butyraldehyde dehydrogenase omitted from the designer pathway(s) of Figure 1 may be used to produce butyraldehyde or butyryl-CoA, respectively.

### Designer Calvin-Cycle-Channeled Aerobic Hydrogenotrophic Biofuel Pathways

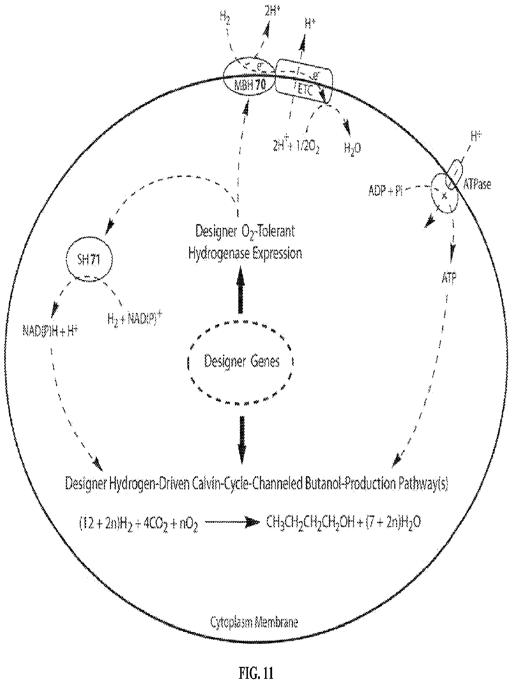

According to one of the various embodiments, a designer hydrogenotrophic Calvin-cycle-channeled pathway technology (Fig. 11) is created that takes hydrogen (H₂), oxygen (O₂) and carbon dioxide (CO₂) to produce advanced biofuels including butanol and related higher alcohols through the designer Calvin-cycle-channeled pathways (Fig. 1 and 4-10). As illustrated in Fig. 11, one of the various embodiments here is the expression of designer oxygen (O₂)-tolerant hydrogenases in a designer microbial cell such as cyanobacteria to generate NAD(P)H and ATP from consumption of hydrogen. The expression of a membrane bound hydrogenase (MBH, **70** and its accessory proteins **72** as listed in Table 1) enables oxidation of H₂ through the respiratory electron transport chain (ETC) system to pump protons (H⁺) across the cytoplasm membrane to create transmembrane electrochemical potential for ATP synthesis; whereas the use of a soluble hydrogenase (SH, **71** and its accessory proteins **72**) enables generation of NAD(P)H through SH-mediated reduction of NAD(P)⁺ by H₂. Use of ATP and NAD(P)H drives the designer Calvin-cycle-channeled pathways (Fig. 1 and 4-10) for CO₂ fixation and biofuel butanol and related higher alcohol production. Therefore, this represents an innovative application of the designer Calvin-cycle-channeled biofuel-production pathways.

For example, the expression of a membrane bound hydrogenase (MBH, **70** and its accessory proteins **72**) and a soluble hydrogenase (SH, **71** and its accessory proteins **72**) in a designer transgenic cyanobacterium that already contains the designer butanol-production-pathway genes of SEQ ID NOS: 58-69 and 72 (and/or 73) can create a hydrogenotrophic Calvin-cycle 3-phophoglycerate-branched 1-butanol production pathway as numerically labeled as **34, 35, 03-05, 36-42,** and **12** in Figure 4. The net result of the designer hydrogenotrophic pathway is the production of 1-butanol (CH₃CH₂CH₂CH₂OH) from hydrogen (H₂), carbon dioxide (CO₂) and oxygen (O₂) according to the following process reaction:

(12 + 2*n*) H₂ + 4CO₂ + *n* O₂ → CH₃CH₂CH₂CH₂OH + (7 + *n*) H₂O [20]

The number (*n*) of oxygen (O₂) molecules used to oxidize hydrogen (H₂) by the respiratory electron-transport-coupled phosphorylation to support the synthesis of a 1-butuanol was estimated to be about 5 in this example.

Note, before the designer genes are turned on, the transgenic cyanobacteria (Fig 11) can grow photoautotrophically using CO₂, H₂O and sunlight just like their wild-type parental strains. When they are grown and ready for use, they can then be placed into a bioreactor supplied with H₂ (about 85%) and CO₂ (about 10%) with limiting amount of O₂ (about 5%) for hydrogenotrophic synthesis of higher alcohols such as 1-butanol, for example, through the Calvin-cycle-channeled butanol-production pathway of Fig. 1 without requiring any photosynthesis or sunlight. Since hydrogen (H₂) can be made from a number of sources including the electrolysis of water, the designer hydrogenotrophic Calvin-cycle-channeled pathway technology (Fig. 11) enables utilization of inexpensive industrial CO₂ and electricity from solar photovoltaic, wind and nuclear power stations to produce "drop-in-ready" liquid transportation fuel such as butanol without requiring any arable lands or photosynthesis.

### Designer Anaerobic Hydrogenotrophic Reductive-Acetyl-CoA Biofuel-Production Pathways

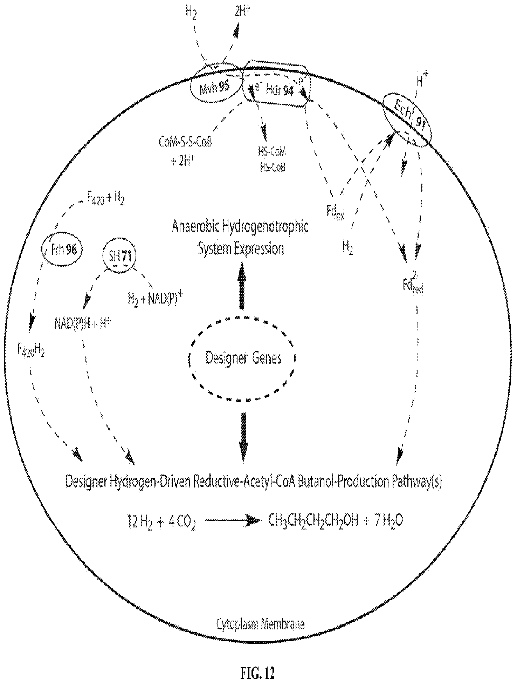

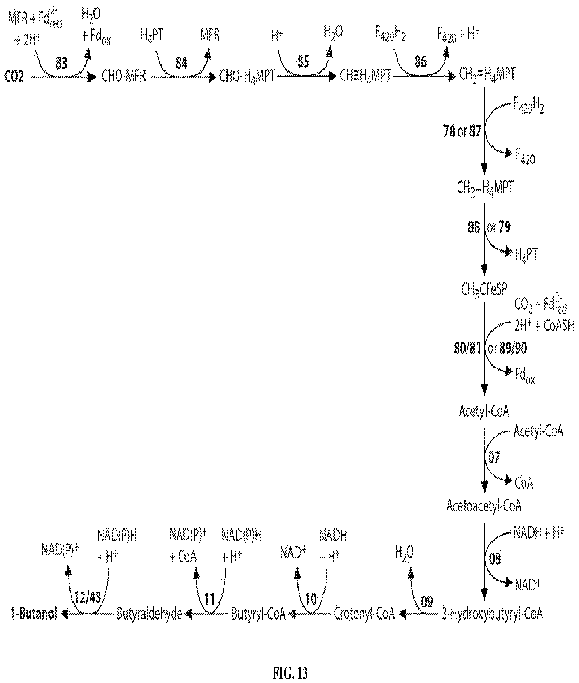

According to one of the various embodiments, a designer hydrogenotrophic reductive-acetyl-CoA biofuel-production pathway technology (Fig. 12) is created that takes hydrogen (H₂) and carbon dioxide (CO₂) to produce advanced biofuels such as butanol and related higher alcohols under anaerobic conditions. As illustrated in Fig. 12, one of the various embodiments here is the expression of a set of designer genes that confer a designer anaerobic hydrogenotrophic system and a reductive-acetyl-CoA butanol-producing pathway (Fig. 13) in a microbial host cell such as a cyanobacterium. Designer anaerobic hydrogenotrophic system includes, for example, energy converting hydrogenase (Ech, **91** in Table 1), [NiFe]-hydrogenase Mvh **(95),** Coenzyme F₄₂₀-reducing hydrogenase (Frh, **96**), native (or heterologous) soluble hydrogenase (SH, **71**), NAD(P)H, reduced ferredoxin (Fd_{red}²⁻), HS-CoM, HS-CoB, and heterodissulfide reductase (Hdr; **94**); while designer reductive-acetyl-CoA butanol-producing pathway (as shown with the numerical labels **83-90** and **07-12/43** in Fig. 13) comprises formylmethanofuran dehydrogenase **83,** formyl transferase **84,** 10-methenyl-tetrahydromethanopterin cyclohydrolase **85,** 10-methylene-H₄ methanopterin dehydrogenase **86,** 10-methylene-H₄-methanopterin reductase **87,** methyl-H₄-methanopterin: corrinoid iron-sulfur protein methyltransferase **88,** corrinoid iron-sulfur protein **89,** CO dehydrogenase/acetyl-CoA synthase **90,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12,** and/or alcohol dehydrogenase **43.** In this example, the net result of the designer anaerobic hydrogenotrophic reductive-acetyl-CoA butanol-production pathway technology (Figs. 12 and 13) is the production of 1-butanol (CH₃CH₂CH₂CH₂OH) from hydrogen (H₂) and carbon dioxide (CO₂) according to the following process reaction:

12 H₂ + 4 CO₂ → CH₃CH₂CH₂CH₂OH + 7 H₂O [21]

The standard free energy change (ΔᵣG°) for this overall reaction is -244.7 kJ/mol 1-butanol, which demonstrates that this hydrogen-driven butanol-production technology is not in violation of thermodynamic laws. This equation shows that the use of 12 molecules (24 electrons) of hydrogen (H₂) can produce one molecule of 1-butanol from 4 molecules of carbon dioxide (CO₂). To produce 12 molecules of H₂ by electrolysis of water, it uses 24 electrons from electricity. Therefore, if electrolysis of water is used as a hydrogen source, then 24 electrons (from electricity) are sufficient to generate one molecule of 1-butanol from 4 molecules of CO₂ through the designer anaerobic hydrogenotrophic reductive-acetyl-CoA butanol-production pathway technology (Figs. 12 and13).

Therefore, in one of the various embodiments, a designer autotrophic organism comprises a set of designer genes (e.g., designer DNA constructs) that express a set of enzymes conferring the designer anaerobic hydrogenotrophic butanol-production-pathway system (as shown in Figs. 12 and 13) that comprises: energy converting hydrogenase (Ech) **91,** [NiFe]-hydrogenase (Mvh) **95,** Coenzyme F₄₂₀-reducing hydrogenase (Frh) **96,** native (or heterologous) soluble hydrogenase (SH) **71,** heterodissulfide reductase (Hdr) **94,** formylmethanofuran dehydrogenase **83,** formyl transferase **84,** 10-methenyl-tetrahydromethanopterin cyclohydrolase **85,** 10-methylene-H₄ methanopterin dehydrogenase **86,** 10-methylene-H₄-methanopterin reductase **87,** methyl-H₄-methanopterin: corrinoid iron-sulfur protein methyltransferase **88,** corrinoid iron-sulfur protein **89,** CO dehydrogenase/acetyl-CoA synthase **90,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12** and/or alcohol dehydrogenase **43.**

Before the designer genes are turned on, the designer transgenic cyanobacteria (Fig 12) can grow photoautotrophically using CO₂, H₂O and sunlight just like their wild-type parental strains. When they are grown and ready for use, they can then be placed into a bioreactor for butanol production from H₂ and CO₂ under anaerobic conditions without requiring any photosynthesis or any respiratory oxidation of H₂ by molecular oxygen (O₂). A unique feature of this designer reductive-acetyl-CoA butanol-production pathway (Fig. 13) is that it does not require any ATP; this pathway uses reduced ferredoxin (Fd_{red}²⁻), F₄₂₀H₂ and NAD(P)H that the designer anaerobic hydrogenotrophic system (Fig. 12) can supply from H₂ employing certain electro-proton-coupled bioenergetics bifurcating mechanism. In accordance with one of the various embodiments, this designer pathway (Fig 13) represents one of the most energy-efficient butanol-production processes identified so for. The standard free energy change (ΔG°) of this specific anaerobic hydrogenotrophic butanol-production process [Eq. 21] is -20.4 kJ/mol per H₂ used. Its maximum hydrogen (H₂)-to-butanol energy conversion efficiency was estimated to be about 91.4%.

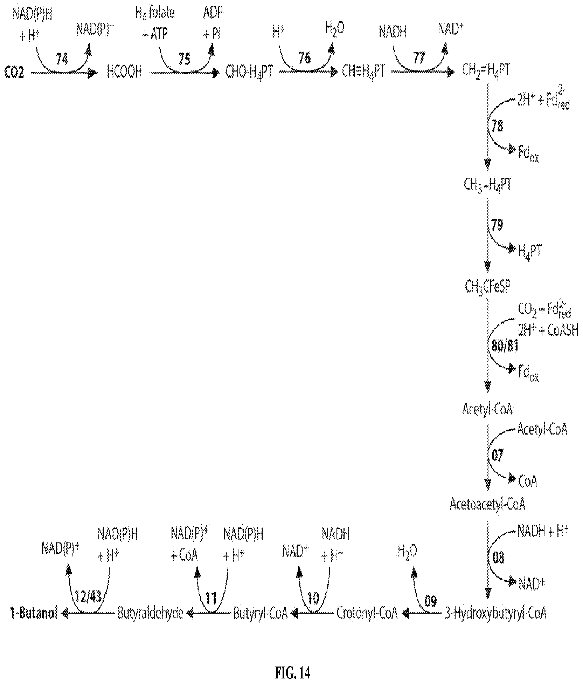

According to one of the various embodiments, another designer anaerobic reductive-acetyl-CoA butanol-production pathway (as shown with the numerical labels **74-81** and **07-12/43** in Figure 14) is created that can produce 1-butanol from H₂ and CO₂ through use of a set of enzymes comprising: formate dehydrogenase **74,** 10-formyl-H₄ folate synthetase **75,** methenyltetrahydrofolate cyclohydrolase **76,** 10-methylene-H₄ folate dehydrogenase 77, 10-methylene-H₄ folate reductase **78,** methyl-H₄ folate: corrinoid iron-sulfur protein methyltransferase **79,** corrinoid iron-sulfur protein **80,** CO dehydrogenase/acetyl-CoA synthase **81,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12,** and/or alcohol dehydrogenase **43.**

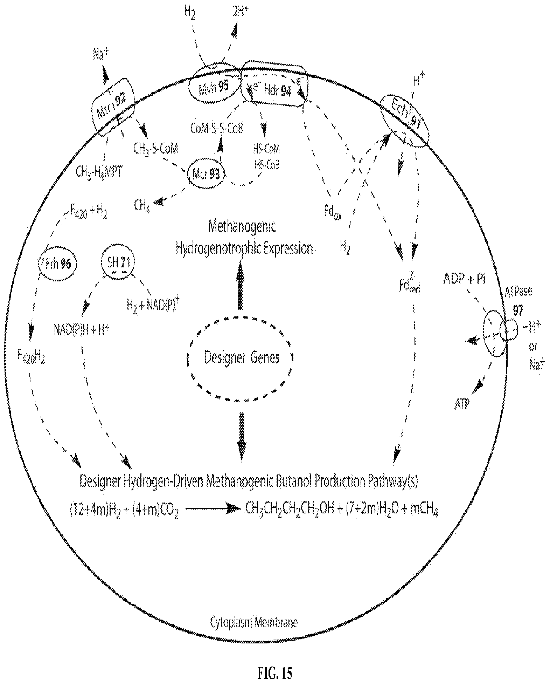

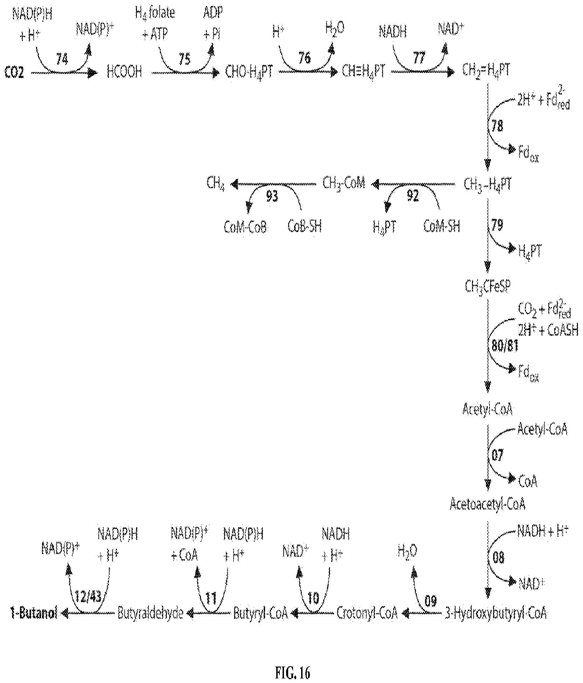

This designer pathway is similar to that of Fig. 13, except that it requires consumption of ATP at the step of 10-formyl-H₄ folate synthetase **75** (Fig. 14). Therefore, it requires ATP supply from other cellular processes in order to operate. According to one of the various embodiments, this pathway (Fig. 14) can be supported by a designer methanogenic hydrogenotrophic cell system (Fig. 15) that produces ATP, Fd_{red}²⁻, F₄₂₀H₂, and NAD(P)H. This designer autotrophic organism comprises a set of designer genes (e.g., designer DNA constructs) that express the designer methanogenic hydrogenotrophic butanol-production-pathway system (as shown in Figs. 14 and 16) comprising: methyl-H4MPT: coenzyme-M methyltransferase Mtr **92,** native (or heterologous) A₁Aₒ-ATP synthase **97,** methyl-coenzyme M reductase Mcr **93,** energy converting hydrogenase (Ech) **91,** [NiFe]-hydrogenase (Mvh) **95,** Coenzyme F₄₂₀-reducing hydrogenase (Frh) **96,** native (or heterologous) soluble hydrogenase (SH) **71,** heterodissulfide reductase (Hdr) **94,** formylmethanofuran dehydrogenase **83,** formyl transferase **84,** 10-methenyl-tetrahydromethanopterin cyclohydrolase **85,** 10-methylene-H₄ methanopterin dehydrogenase **86,** 10-methylene-H₄-methanopterin reductase **87,** methyl-H₄-methanopterin: corrinoid iron-sulfur protein methyltransferase **88,** corrinoid iron-sulfur protein **89,** CO dehydrogenase/acetyl-CoA synthase **90,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12** and/or alcohol dehydrogenase **43.**

For example, the designer methanogenic hydrogenotrophic system (Fig. 15) comprises methyl-H4MPT: coenzyme-M methyltransferase Mtr **92,** A₁Aₒ-ATP synthase **97,** energy converting hydrogenase (Ech; **91** in Table 1), [NiFe]-hydrogenase Mvh **(95),** Coenzyme F₄₂₀-reducing hydrogenase (Frh, **96**), native (or heterologous) soluble hydrogenase (SH, 71), NAD(P)H, reduced ferredoxin (Fd_{red}²⁻), HS-CoM, HS-CoM, methyl-coenzyme M reductase Mcr **93,** and heterodissulfide reductase (Hdr, **94**). The Mtr **92** in this system can take a fraction of the CH₃-H₄MPT intermediate to produce methane and generate a transmembrane electrochemical potential for synthesis of ATP, which can support the ATP-requiring anaerobic reductive-acetyl-CoA butanol-production pathway of Fig.14. Therefore, the combination of the methanogenic hydrogenotrophic system (Fig. 15) and the ATP-requiring anaerobic reductive-acetyl-CoA butanol-production pathway (Fig. 14) results in a combined pathway (Fig. 16) for production of both butanol and methane. The net result is the production of both butanol and methane (CH₄) from hydrogen (H₂) and carbon dioxide (CO₂) according to the following process reaction where m is the number of CH₄ molecules co-generated per 1-butanol produced:

(12 + 4*m*)H₂ + (4 + *m*)CO₂ → CH₃CH₂CH₂CH₂OH + (7+*m*)H₂O + *m*CH₄ [22]

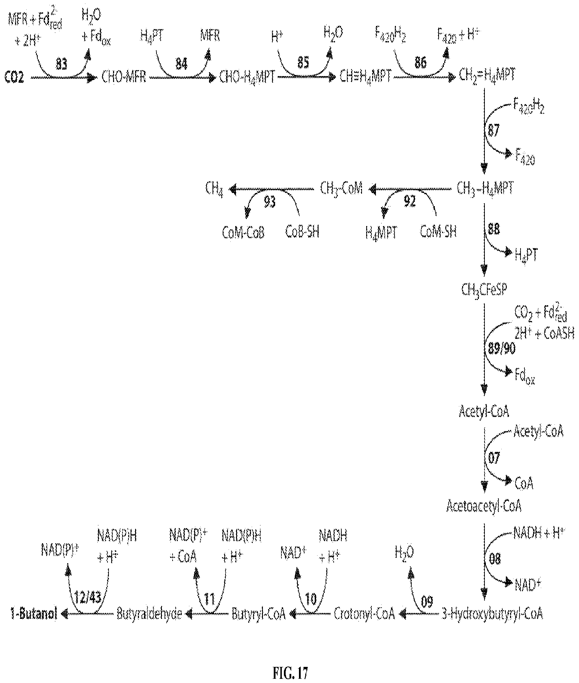

The non-ATP-requiring anaerobic reductive-acetyl-CoA butanol-production pathway (Fig. 13) can, of course, operate with this designer methanogenic hydrogenotrophic system (Fig. 15) as well, resulting in another combined pathway for production of both butanol and methane (Fig. 17). Therefore, in one of the various embodiments, a designer autotrophic organism comprises a set of designer genes (e.g., designer DNA constructs) that express a designer methanogenic hydrogenotrophic butanol-production-pathway system (as shown in Figs. 15, 13, and 17) comprising: methyl-H4MPT: coenzyme-M methyltransferase Mtr **92,** native (or heterologous) A₁Aₒ-ATP synthase **97,** methyl-coenzyme M reductase Mcr **93,** energy converting hydrogenase (Ech) **91,** [NiFe]-hydrogenase (Mvh) **95,** Coenzyme F₄₂₀-reducing hydrogenase (Frh) **96,** native (or heterologous) soluble hydrogenase (SH) **71,** heterodissulfide reductase (Hdr) **94,** formate dehydrogenase **74,** 10-formyl-H₄ folate synthetase **75,** methenyltetrahydrofolate cyclohydrolase **76,** 10-methylene-H₄ folate dehydrogenase 77, 10-methylene-H₄ folate reductase **78,** methyl-H₄ folate: corrinoid iron-sulfur protein methyltransferase **79,** corrinoid iron-sulfur protein **80,** CO dehydrogenase/acetyl-CoA synthase **81,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12,** and/or alcohol dehydrogenase **43.**

Some of these enzymes may naturally exist in some of the host organisms depending on their genetic background; some of these native enzymes may be used in constructing part of the designer pathways (Figs. 12-17) along with designer genes. Therefore, according to one of the various embodiments, a designer autotrophic organism for production of biofuels such as butanol through anaerobic hydrogenotrophic reductive-acetyl-CoA biofuel-production-pathway(s) comprises designer genes that can express at least one of the enzymes selected from the group consisting of: energy converting hydrogenase (Ech) **91,** methyl-H4MPT: coenzyme-M methyltransferase Mtr **92,** methyl-coenzyme M reductase Mcr **93,** heterodissulfide reductase (Hdr) **94,** [NiFe]-hydrogenase (Mvh) **95,** Coenzyme F₄₂₀-reducing hydrogenase (Frh) **96,** soluble hydrogenase (SH) **71,** A₁Aₒ-ATP synthase **97,** formate dehydrogenase **74,** 10-formyl-H₄ folate synthetase **75,** methenyltetrahydrofolate cyclohydrolase **76,** 10-methylene-H₄ folate dehydrogenase **77,** 10-methylene-H₄ folate reductase **78,** methyl-H₄ folate: corrinoid iron-sulfur protein methyltransferase **79,** corrinoid iron-sulfur protein **80,** CO dehydrogenase/acetyl-CoA synthase **81,** formylmethanofuran dehydrogenase **83,** formyl transferase **84,** 10-methenyl-tetrahydromethanopterin cyclohydrolase **85,** 10-methylene-H₄ methanopterin dehydrogenase **86,** 10-methylene-H₄-methanopterin reductase **87,** methyl-H₄-methanopterin: corrinoid iron-sulfur protein methyltransferase **88,** corrinoid iron-sulfur protein **89,** CO dehydrogenase/acetyl-CoA synthase **90,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12** and/or alcohol dehydrogenase **43.**

SEQ ID NOS. 166-198 present examples for designer DNA constructs of designer enzymes for creation of designer hydrogenotrophic biofuel-producing organisms such as designer cyanobacteria with reductive-acetyl-CoA biofuel-production pathways. Briefly, SEQ ID NO: 166 presents example 166 of a designer *hox*-promoter-controlled Formylmethanofuran dehydrogenase (Fmd; **83**) DNA construct (6110 bp) that includes a PCR FD primer (sequence 1-20), a 172-bp *Nostoc* (*Anabaena* PCC 7120) *hox* promoter (21-192), an enzyme-encoding sequence (193-5659) selected/modified from the sequence of formylmethanofuran dehydrogenase subunits B, C, E (GenBank: ADL58895, ADL58894, ADL58893) of *Methanothermobacter marburgensis* and formylmethanofuran dehydrogenase subunits A, D, and G (GenBank: ABC56660, ABC56658, ABC56657 ) of *Methanosphaera stadtmanae,* a 432-bp *Nostoc sp.* strain PCC 7120 *gor* terminator (5659-6090), and a PCR RE primer (6091-6110) at the 3' end.

SEQ ID NO: 167 presents example 167 of a designer *hox*-promoter-controlled Formyl transferase (**84**) DNA construct (1538 bp) that includes a PCR FD primer (sequence 1-20), a 172-bp *Nostoc* (*Anabaena* PCC 7120) *hox* promoter (21-192), an enzyme-encoding sequence (193-1086) selected/modified from the sequence of a formylmethanofurantetrahydromethanopterin formyltransferase (GenBank: ADL59225) of *Methanothermobacter marburgensis,* a 432-bp *Nostoc gor* terminator (1087-1518), and a PCR RE primer (1519-1538).

SEQ ID NO: 168 presents example 168 of a designer *hox*-promoter-controlled 5,10-Methenyl-tetrahydromethanopterin (H₄ methanopterin) cyclohydrolase **(85)** DNA construct (1631 bp) that includes a PCR FD primer (sequence 1-20), a 172-bp *Anabaena* PCC 7120 *hox* promoter (21-192), an enzyme-encoding sequence (193-1179) selected from the sequence of a N(5),N(10)-methenyltetrahydromethanopterin cyclohydrolase (GenBank: ABC57615) of *Methanosphaera stadtmanae,* a 432-bp *Nostoc gor* terminator (1180-1161), and a PCR RE primer (1162-1631).

SEQ ID NO: 169 presents example 169 of a designer *hox*-promoter-controlled 5,10-Methylene-H₄-methanopterin dehydrogenase (**86**) DNA construct (1475 bp) that includes a PCR FD primer (sequence 1-20), a 172-bp *Anabaena* PCC 7120 *hox* promoter (21-192), an enzyme-encoding sequence (193-1023) selected from the sequence of a F₄₂₀-dependent methylene-5,6,7,8-tetrahydromethanopterin dehydrogenase (GenBank: ADL57660) of *Methanothermobacter marburgensis,* a 432-bp *Nostoc gor* terminator (1023-1455), and a PCR RE primer (1456-1475).

SEQ ID NO: 170 presents example 170 of a designer *hox*-promoter-controlled Methylenetetrahydrofolate reductase and/or Methylene-H₄-methanopterin reductase (**78, 87**) DNA construct (2594 bp) that includes a PCR FD primer (sequence 1-20), a 172-bp *Nostoc sp.* strain PCC 7120 (*Anabaena* PCC 7120) *hox* promoter (21-192), an enzyme-encoding sequence (193-2142) selected/modified from the sequence of a methylenetetrahydrofolate reductase (GenBank: YP_430048) of *Moorella thermoacetica* and a coenzyme F₄₂₀-dependent N(5),N(10)-methenyltetrahydromethanopterin reductase (GenBank: ADN36752) of *Methanoplanus petrolearius,* a 432-bp *Nostoc gor* terminator (2143-2574), and a PCR RE primer (2575-2594).

SEQ ID NO: 171 presents example 171 of a designer *hox*-promoter-controlled Methyltetrahydrofolate:corrinoid/iron-sulfur protein methyltransferase (**79, 88**) DNA construct (2819 bp) that includes a PCR FD primer (sequence 1-20), a 172-bp *Nostoc* (*Anabaena* PCC 7120) *hox* promoter (21-192), an enzyme-encoding sequence (193-2467) selected/modified from the sequence of a methyltetrahydrofolate:corrinoid/iron-sulfur protein methyltransferase (GenBank: YP_430950) of *Moorella thermoacetica,* and acetyl-CoA decarbonylase/synthase, subunit gamma (GenBank: ADL57900) of *Methanothermobacter marburgensis,* a 432-bp *Nostoc sp*. strain PCC 7120 *gor* terminator (2468-2899), and a PCR RE primer (2900-2819).

SEQ ID NO: 172 presents example 172 of a designer *hox*-promoter-controlled Corrinoid iron-sulfur protein (**80, 89**) DNA construct (2771 bp) that includes a PCR FD primer (sequence 1-20), a 172-bp *Nostoc* (*Anabaena* PCC 7120) *hox* promoter (21-192), an enzyme-encoding sequence (193-2319) selected/modified from the sequence of a small subunit corrinoid iron-sulfur protein (GenBank: AAA23255) of *Moorella thermoacetica,* and acetyl-CoA decarbonylase/synthase subunit delta (GenBank: ADL57899) of *Methanothermobacter marburgensis,* a 432-bp *Nostoc gor* terminator (2319-2751), and a PCR RE primer (2752-2771).

SEQ ID NO: 173 presents example 173 of a designer *hox*-promoter-controlled CO dehydrogenase /acetyl-CoA synthase (**81, 90**) DNA construct (7061 bp) that includes a PCR FD primer (sequence 1-20), a 172-bp *Nostoc* (*Anabaena* PCC 7120) *hox* promoter (21-192), an enzyme-encoding sequence (193-6609) selected/modified from the sequence of acetyl-CoA decarbonylase/synthase beta subunit / acetyl-CoA decarbonylase / synthase alpha subunit (GenBank: ABC 19516) of *Moorella thermoacetica,* and acetyl-CoA decarbonylase/synthase subunits alpha, beta, epsilon (GenBank: ADL57895, ADL59006, ADL57897) of *Methanothermobacter marburgensis,* a 432-bp *Nostoc sp.* strain PCC 7120 *gor* terminator (6610-7041), and a PCR RE primer (7042-7061).

SEQ ID NO: 174 presents example 174 of a designer *hox*-promoter-controlled Thiolase **(07)** DNA construct (1847 bp) that includes a PCR FD primer (sequence 1-20), a 172-bp *Nostoc* (*Anabaena* PCC 7120) *hox* promoter (21-192), an enzyme-encoding sequence (193-1395) selected/modified from the sequence of thiolase (GenBank: AB190764) of *Butyrivibrio fibrisolvens,* a 432-bp *Nostoc gor* terminator (1396-1827), and a PCR RE primer (1828-1847).

SEQ ID NO: 175 presents example 175 of a designer *hox*-promoter-controlled 3-Hydroxybutyryl-CoA dehydrogenase (**08**) DNA construct (1514 bp) that includes a PCR FD primer (sequence 1-20), a 172-bp *Nostoc* (*Anabaena* PCC 7120) *hox* promoter (21-192), an enzyme-encoding sequence (193-1062) selected/modified from the sequence of 3-hydroxybutyryl coenzyme A dehydrogenase (GenBank: Z92974) of *Thermoanaerobacterium,* a 432-bp *Nostoc gor* terminator (1063-1494), and a PCR RE primer (1495-1514).

SEQ ID NO: 176 presents example 176 of a designer *hox*-promoter-controlled Crotonase (**09**) DNA construct (1430 bp) that includes a PCR FD primer (sequence 1-20), a 172-bp *Nostoc* (*Anabaena* PCC 7120) *hox* promoter (21-192), an enzyme-encoding sequence (193-978) selected from the sequence of crotonase (GenBank: AF494018) of *Clostridium beijerinckii,* a 432-bp *Nostoc gor* terminator (979-1410), and a PCR RE primer (1411-1430).

SEQ ID NO: 177 presents example 177 of a designer *hox*-promoter-controlled Butyryl-CoA dehydrogenase **(10)** DNA construct (1784 bp) that includes a PCR FD primer (sequence 1-20), a 172-bp *Nostoc* (*Anabaena* PCC 7120) *hox* promoter (21-192), an enzyme-encoding sequence (193-1332) selected/modified from the sequence of butyryl-CoA dehydrogenase (GenBank: AF494018) of *Clostridium beijerinckii,* a 432-bp *Nostoc gor* terminator (1333-1764), and a PCR RE primer (1765-1784).

SEQ ID NO: 178 presents example 178 of a designer *hox*-promoter-controlled Butyraldehyde dehydrogenase **(11)** DNA construct (2051 bp) that includes a PCR FD primer (sequence 1-20), a 172-bp *Nostoc* (*Anabaena* PCC 7120) *hox* promoter (21-192), an enzyme-encoding sequence (193-1599) selected/modified from the sequence of butyraldehyde dehydrogenase (GenBank: AY251646) of *Clostridium saccharoperbutylacetonicum,* a 432-bp *Nostoc gor* terminator (1600-2031), and a PCR RE primer (2032-2051).

SEQ ID NO: 179 presents example 179 of a designer hox-promoter-controlled NADH-dependent Butanol dehydrogenase (**12**) DNA construct (1808 bp) that includes a PCR FD primer (sequence 1-20), a 172-bp *Nostoc* (*Anabaena* PCC 7120) *hox* promoter (21-192), an enzyme-encoding sequence (193-1356) selected/modified from the sequence of NADH-dependent butanol dehydrogenase (GenBank: YP_148778) of *Geobacillus kaustophilus,* a 432-bp *Nostoc sp.* strain PCC 7120 *gor* terminator (1367-1788), and a PCR RE primer (1789-1808) at the 3' end.

Note, use of SEQ ID NOS. 166-179 in genetic transformation of a microbial host cell including (but not limited to) bacterial cells such as a cyanobacterium *Anabaena* PCC 7120 can create a designer cyanobacterium such as designer *Anabaena* with a designer reductive-acetyl-CoA biofuel-production pathway (numerically labeled as **83-90** and **07-12** in Figure 13) for production of 1-butanol from hydrogen and carbon dioxide without requiring photosynthesis or sunlight. That is, the expression of SEQ ID NOS. 166-179 in a bacterium such as Anabaena PCC 7120 represents a designer organism with the designer hydrogenotrophic reductive-acetyl-CoA biofuel-production pathway (**83-90** and **07-12** in Figure 13) that can operate for anaerobic chemolithoautotrophic production of butanol from hydrogen and carbon dioxide even if it is in complete darkness.

SEQ ID NO: 180 presents example 180 of a designer *hox*-promoter-controlled Energy converting hydrogenase (Ech) (**91**) DNA construct (10538 bp) that includes a PCR FD primer (sequence 1-20), a 172-bp *Nostoc* (*Anabaena* PCC 7120) *hox* promoter (21-192), an enzyme-encoding sequence (193-10086) selected/modified from the sequence of Energy converting hydrogenase subunits (EchA, B, C, D, E. F, G, H, I, J, K, L, M, N, O, P, Q) (GenBank: ABC57807, and ABC57812- ABC57827) of *Methanosphaera stadtmanae DSM 3091,* a 432-bp *Nostoc gor* terminator (10087-10518), and a PCR RE primer (10519-10538).

SEQ ID NO: 181 presents example 181 of a designer *hox*-promoter-controlled [NiFe]-hydrogenase MvhADG (**95**) DNA construct (3416 bp) that includes a PCR FD primer (sequence 1-20), a 172-bp *Nostoc* (*Anabaena* PCC 7120) *hox* promoter (21-192), an enzyme-encoding sequence (193-2964) selected/modified from the sequence of [NiFe]-hydrogenase MvhADG (GenBank: ADL59096, ADL59098, ADL59097) of *Methanothermobacter marburgensis,* a 432-bp *Nostoc sp.* strain PCC 7120 *gor* terminator (2965-3396), and a PCR RE primer (3397-3416).

SEQ ID NO: 182 presents example 182 of a designer *hox*-promoter-controlled Heterodisulfide reductases (HdrABC, HdrDE) (**94**) DNA construct (6695 bp) that includes a PCR FD primer (sequence 1-20), a 172-bp *Anabaena* PCC 7120 *hox* promoter (21-192), an enzyme-encoding sequence (193-6243) selected/modified from the sequence of Heterodisulfide reductases (HdrABC, HdrDE) (GenBank: AET63985, AET63982, AET63983, AET64166, AET64165) of *Methanosaeta harundinacea,* a 432-bp *Nostoc gor* terminator (6244-6675), and a PCR RE primer (6676-6695).

SEQ ID NO: 183 presents example 183 of a designer *hox*-promoter-controlled Coenzyme F₄₂₀-reducing hydrogenase (Frh) (**96**) DNA construct (3407 bp) that includes a PCR FD primer (sequence 1-20), a 172-bp *Nostoc sp.* strain PCC 7120 (*Anabaena* PCC 7120) *hox* promoter (21-192), an enzyme-encoding sequence (193-2955) selected/modified from the sequence of Coenzyme F₄₂₀-reducing hydrogenase (FrhB1-3) (GenBank: YP_003357229, YP_003357467, YP_003357509) of *Methanocella paludicola SANAE,* a 432-bp *Nostoc sp.* strain PCC 7120 gor terminator (2956-3387), and a PCR RE primer (3388-3407) at the 3' end.

Note, use of SEQ ID NOS. 180-183 in genetic transformation of a microbial host cell including (but not limited to) bacterial cells such as a cyanobacterium *Anabaena* PCC 7120 can confer an anaerobic chemolithoautotrophic hydrogen (H₂) utilization system [which, as shown in Figure 12, comprises Energy converting hydrogenase (Ech) (**91**), [NiFe]-hydrogenase MvhADG (**95**), Coenzyme F₄₂₀-reducing hydrogenase (Frh) (**96**), and Coenzyme F₄₂₀-reducing hydrogenase (Frh) (**96**)] that can produce reducing power (Fd_{red}²⁻ and F₄₂₀H₂) from H₂ in support of the designer reductive-acetyl-CoA butanol-production pathway (**83-90** and **07-12** in Figure 13). Therefore, the expression of SEQ ID NOS. 180-183 along with SEQ ID NOS. 166-179 in a bacterium such as Anabaena PCC 7120 represents a designer organism (such as designer Anabaena) with a full designer reductive-acetyl-CoA biofuel-production pathway system (Figures 12 and 13) that can operate for anaerobic chemolithoautotrophic production of butanol from hydrogen and carbon dioxide without requiring photosynthesis or aerobic respiration. The net result in this example is the anaerobic chemolithoautotrophic production of butanol from hydrogen and carbon dioxide as shown in the process equation [21].

Also note, these designer genes (SEQ ID NOS. 166-183) are controlled by a designer *hox* anaerobic promoter. Therefore, under aerobic conditions such as in an open pond mass culture, the designer Anabaena in this example can quickly grow photoautotrophically using air carbon dioxide and water as the sources of carbon and electrons just like the wild-type parental strain. When the designer Anabaena cells cultures are grown and ready for use (as catalysts in this application), they can then be placed into an anaerobic reactor supplied with industrial CO₂ and H₂ gas for induction of the designer genes expression for anaerobic chemolithoautotrophic production of butanol (as shown in Figures 12 and 13) in dark.

SEQ ID NO: 184 presents example 184 of a designer *hox*-promoter-controlled Methyl-H4MPT: coenzyme M methyltransferase (MtrA-H) **(92)** DNA construct (5417 bp) that includes a PCR FD primer (sequence 1-20), a 172-bp *Anabaena* PCC 7120 *hox* promoter (21-192), an enzyme-encoding sequence (193-4965) selected/modified from the sequence of Methyl-H4MPT: coenzyme M methyltransferase (MtrA-H) (GenBank: ABC56714, ABC56713, YP_447360, YP_447354, YP_447359, YP_447355) of *Methanosphaera stadtmanae,* and mtrEF (AET65445, NC_009051) of *Methanosaeta harundinacea* and *Methanoculleus marisnigri*, a 432-bp *Nostoc sp.* strain PCC 7120 *gor* terminator (4966-5397), and a PCR RE primer (5398-5417).

SEQ ID NO: 185 presents example 185 of a designer *hox*-promoter-controlled Methyl-coenzyme M reductase (Mcr) (**93**) DNA construct (5042 bp) that includes a PCR FD primer (sequence 1-20), a 172-bp *Nostoc sp.* strain PCC 7120 (*Anabaena* PCC 7120) *hox* promoter (21-192), an enzyme-encoding sequence (193-4590) selected/modified from the sequence of methylcoenzyme M reductase subunits A, B. C, G (GenBank: CAE48306, CAE48303, ABC56709, CAE48305) of *Methanosphaera stadtmanae,* a 432-bp *Nostoc sp.* strain PCC 7120 gor terminator (4591-5022), and a PCR RE primer (5023-5042).

Note, use of SEQ ID NOS. 184 and 185 along with SEQ ID NOS. 180-183 in genetic transformation of a microbial host cell including bacterial cells such as a cyanobacterium *Anabaena* PCC 7120 can confer a methanogenic hydrogenotrophic system which, as shown in Figure 15, comprises Methyl-H4MPT: coenzyme M methyltransferase (MtrA-H) (**92**), Methyl-coenzyme M reductase (Mcr) (**93**), Energy converting hydrogenase (Ech) (**91**), [NiFe]-hydrogenase MvhADG (**95**), Coenzyme F₄₂₀-reducing hydrogenase (Frh) (**96**), Coenzyme F₄₂₀-reducing hydrogenase (Frh) (**96**). These enzymes along with a native ATPase **97** can produce ATP and reducing power (Fd_{red}²⁻ and F₄₂₀H₂) from H₂ in support of the designer reductive-acetyl-CoA methanogenic butanol-production pathways (Figures 16 and 17). Therefore, the expression of SEQ ID NOS. 180-185 along with SEQ ID NOS. 166-179 in a bacterium such as Anabaena PCC 7120 represents a designer organism (such as designer Anabaena) with a designer hydrogenotrophic reductive-acetyl-CoA methanogenic biofuel-production pathway system (Figures 15 and 17) that can operate for anaerobic production of both butanol and methane from hydrogen and carbon dioxide without requiring any photosynthesis. The net result in this example is the anaerobic chemolithoautotrophic production of butanol and methane from hydrogen and carbon dioxide as shown in the process equation [22].

SEQ ID NO: 186 presents example 186 of a designer *hox*-promoter-controlled Formate dehydrogenase **(74)** DNA construct (5450 bp) that includes a PCR FD primer (sequence 1-20), a 172-bp *Nostoc sp*. strain PCC 7120 (*Anabaena* PCC 7120) *hox* promoter (21-192), an enzyme-encoding sequence (193-4998) selected/modified from the sequence of formate dehydrogenase alpha and beta subunits (GenBank: AAB18330, AAB18329) of *Moorella thermoacetica,* a 432-bp *Nostoc sp.* strain PCC 7120 *gor* terminator (4999-5430), and a PCR RE primer (5431-5450).

SEQ ID NO: 187 presents example 187 of a designer *hox*-promoter-controlled 10-Formyl-H₄ folate synthetase **(75)** DNA construct (2324 bp) that includes a PCR FD primer (sequence 1-20), a 172-bp *Nostoc* (*Anabaena* PCC 7120) *hox* promoter (21-192), an enzyme-encoding sequence (193-1872) selected/modified from the sequence of 10-formyltetrahydrofolate synthetase (GenBank: YP_428991) of *Moorella thermoacetica*, a 432-bp *Nostoc sp.* strain PCC 7120 *gor* terminator (1873-2304), and a PCR RE primer (2305-2324).

SEQ ID NO: 188 presents example 188 of a designer *hox*-promoter-controlled 10-Methenyl-H₄ folate cyclohydrolase **(76)** DNA construct (1487 bp) that includes a PCR FD primer (sequence 1-20), a 172-bp *Nostoc* (*Anabaena* PCC 7120) *hox* promoter (21-192), an enzyme-encoding sequence (193-1035) selected/modified from the sequence of methenyltetrahydrofolate cyclohydrolase (GenBank: YP_430368) of *Moorella thermoacetica ATCC 39073,* a 432-bp *Nostoc gor* terminator (1036-1467), and a PCR RE primer (1468-1487).

SEQ ID NO: 189 presents example 189 of a designer *hox*-promoter-controlled 10-Methylene-H₄ folate dehydrogenase (77) DNA construct (1487 bp) that includes a PCR FD primer (sequence 1-20), a 172-bp *Nostoc* (*Anabaena* PCC 7120) *hox* promoter (21-192), an enzyme-encoding sequence (193-1035) selected/modified from the sequence of methenyltetrahydrofolate cyclohydrolase /5,10-methylenetetrahydrofolate dehydrogenase (GenBank: ABC 19825) of *Moorella thermoacetica*, a 432-bp *Nostoc sp.* strain PCC 7120 *gor* terminator (1036-1467), and a PCR RE primer (1468-1487).

SEQ ID NO: 190 presents example 190 of a designer *hox*-promoter-controlled 10-Methylene-H₄ folate reductase **(78)** DNA construct (1565 bp) that includes a PCR FD primer (sequence 1-20), a 172-bp *Nostoc* (*Anabaena* PCC 7120) *hox* promoter (21-192), an enzyme-encoding sequence (193-1113) selected/modified from the sequence of methylenetetrahydrofolate reductase (GenBank: ABC19505) of *Moorella thermoacetica*, a 432-bp *Nostoc gor* terminator (1114-1545), and a PCR RE primer (1546-1565).

SEQ ID NO: 191 presents example 191 of a designer *hox*-promoter-controlled Methyl-H₄ folate: corrinoid iron-sulfur protein Methyltransferase **(79)** DNA construct (1442 bp) that includes a PCR FD primer (sequence 1-20), a 172-bp *Anabaena* PCC 7120 *hox* promoter (21-192), an enzyme-encoding sequence (193-690) selected/modified from the sequence of methyltetrahydrofolate:corrinoid/iron-sulfur protein methyltransferase (GenBank: YP_430174) of *Moorella thermoacetica*, a 432-bp *Nostoc gor* terminator (691-1122), and a PCR RE primer (1123-1442).

SEQ ID NO: 192 presents example 192 of a designer hox-promoter-controlled Corrinoid iron-sulfur protein (**80**) DNA construct (2942 bp) that includes a PCR FD primer (sequence 1-20), a 172-bp *Anabaena* hox promoter (21-192), an enzyme-encoding sequence (193-2490) selected/modified from the sequence of corrinoid iron-sulfur protein large and small subunits (GenBank: AEI90745, AEI90746) of *Clostridium autoethanogenum*, a 432-bp Nostoc sp. strain PCC 7120 gor terminator (2491-2922), and a PCR RE primer (2923-2942).

SEQ ID NO: 193 presents example 193 of a designer *hox*-promoter-controlled CO dehydrogenase/acetyl-CoA synthase (**81**) DNA construct (4859 bp) that includes a PCR FD primer (sequence 1-20), a 172-bp *Anabaena* PCC 7120 *hox* promoter (21-192), an enzyme-encoding sequence (193-4407) selected/modified from the sequence of carbon monoxide dehydrogenase alpha subunit alpha and beta subunits (GenBank: AAA23229, AAA23228) of *Moorella thermoacetica*, a 432-bp *Nostoc gor* terminator (4408-4839), and a PCR RE primer (4840-4859).

Note, use of SEQ ID NOS. 186-193 along with SEQ ID NOS. 174-179 in genetic transformation of a microbial host cell such as a cyanobacterium *Anabaena* PCC 7120 confers an ATP-requiring reductive-acetyl-CoA butanol-production pathway (**74-81** and **07-12/42** in Figure 14). Similarly, the expression of SEQ ID NOS. 186-193 and SEQ ID NOS. 180-185 along with SEQ ID NOS. 174-179 in a bacterium such as Anabaena PCC 7120 represents a designer organism (such as designer Anabaena) with a designer ATP-requiring reductive-acetyl-CoA methanogenic biofuel-production pathway and a hydrogenotrophic methanogenesis-coupled ATP-generating system (Figures 15 and 16) that can operate for production of both butanol and methane from hydrogen and carbon dioxide. The net result in this example is the anaerobic chemolithotrophic production of both butanol and methane from hydrogen and carbon dioxide as shown in the process equation [22].

SEQ ID NO: 194 presents example 194 of a designer *hox*-promoter-controlled F₄₂₀ synthesis enzymes (**99**) DNA construct (6428 bp) that includes a PCR FD primer (sequence 1-20), a 172-bp *Anabaena* PCC 7120 *hox* promoter (21-192), enzymes-encoding sequence (193-4976) selected/modified from the sequence of lactaldehyde dehydrogenase CofA (GenBank: ADC46523) of *Methanobrevibacter ruminantium,* 2-phospho-1-lactate guanylyltransferase (GenBank: ADL58588) of *Methanothermobacter Marburgensis*, 2-phospho-L-lactate transferase (GenBank: NP_987524) of *Methanococcus maripaludis*, coenzyme F420-0 gamma-glutamyl ligase (YP_001030766) of *Methanocorpusculum labreanum,* FO synthase subunits 1 and 2 (YP_003357513, YP_003357511) of *Methanocella paludicolam,* a 432-bp *Nostoc sp.* strain PCC 7120 *gor* terminator (4977-6408), and a PCR RE primer (6409-6428).

SEQ ID NO: 195 presents example 195 of a designer *hox*-promoter-controlled Pyridoxal phosphate-dependent L-tyrosine decarboxylase(mfnA for methanofuran synthesis) **(100)** DNA construct (1778 bp) that includes a PCR FD primer (sequence 1-20), a 172-bp *Anabaena* PCC 7120 *hox* promoter (21-192), an enzyme-encoding sequence (193-1326) selected/modified from the sequence of L-tyrosine decarboxylase (GenBank: YP_003355454) of *Methanocella paludicola,* a 432-bp *Nostoc gor* terminator (1327-1758), and a PCR RE primer (1759-1778).

SEQ ID NO: 196 presents example 196 of a designer *hox*-promoter-controlled Methanopterin synthesis enzymes **(101)** DNA construct (3215 bp) that includes a PCR FD primer (sequence 1-20), a 172-bp *Anabaena* PCC 7120 *hox* promoter (21-192), an enzymes-encoding sequence (193-2763) selected/modified from the sequence of GTP cyclohydrolase (GenBank: YP_447347) of *Methanosphaera stadtmanae DSM 3091,* cyclic phosphodiesterase MptB (ABO35741) of *Methanococcus maripaludis C5,* beta-ribofuranosylaminobenzene 5'-phosphate synthase (YP_003356610) of *Methanocella paludicola SANAE*, a 432-bp *Nostoc sp.* strain PCC 7120 gor terminator (2764-3195), and a PCR RE primer (3195-3215).

SEQ ID NO: 197 presents example 197 of a designer *hox*-promoter-controlled Coenzyme M synthesis enzymes **(102)** DNA construct (4226 bp) that includes a PCR FD primer (sequence 1-20), a 172-bp *Nostoc sp.* strain PCC 7120 (*Anabaena* PCC 7120) *hox* promoter (21-192), an enzymes-encoding sequence (193-3774) selected/modified from the sequence of phosphosulfolactate synthase, 2-phosphosulfolactate phosphatase and sulfolactate dehydrogenase (GenBank: ADL57861, YP_003850451, ADL59162) of *Methanothermobacter marburgensis,* and sulfopyruvate decarboxylase (YP_003357048) of *Methanocella paludicola SANAE*, a 432-bp *Nostoc sp.* strain PCC 7120 *gor* terminator (3775-4026), and a PCR RE primer (4027-4226).

SEQ ID NO: 198 presents example 198 of a designer *hox*-promoter-controlled Coenzyme B synthesis enzymes **(103)** DNA construct (5198 bp) that includes a PCR FD primer (sequence 1-20), a 172-bp *Anabaena* PCC 7120 *hox* promoter (21-192), an enzymes-encoding sequence (193-4746) selected/modified from the sequence of isopropylmalate synthase, isopropylmalate dehydrogenase (GenBank: AAM01606, NP_614498) of *Methanopyrus kandleri*, isopropylmalate isomerase large and small subunits (ADP98363, ADP98362 ) of *Marinobacter adhaerens*, a 432-bp *Nostoc gor* terminator (4747-5178), and a PCR RE primer (5179-5198).

Note, the expression of SEQ ID NOS. 194-198 in a microbial host cell such as cyanobacterium *Anabaena* PCC 7120 provides the ability of synthesizing some of the cofactors such as F₄₂₀, methanofuran, methanopterin, Coenzyme M, and Coenzyme B that are needed for the designer hydrogenotrophic reductive-acetyl-CoA biofuel-production pathways (of Figures 13, 14, 16 and 17) to properly operate. Depending on the genetic backgrounds of various host cells such as cyanobacteria, many of them may or may not possess some of these enzymes to synthesize this type of special cofactors. Therefore, in one of the various embodiments, it is a preferred practice to express this type of designer cofactor-synthesis enzymes (e.g., SEQ ID NOS. 194-198) along with the hydrogenotrophic designer reductive-acetyl-CoA biofuel-production pathway genes (e.g., SEQ ID NOS. 166-193) as shown in these examples.

Note, many of the hydrogenotrophic bacteria and methanogens such as *Methanocella paludicola SANAE* naturally possess certain hydrogenotrophic and/or reductive acetyl-CoA pathway(s) and the ability of synthesizing the associated cofactors including F420, methanofuran, methanopterin, Coenzyme M, and Coenzyme B. Therefore, in one of the various embodiments, it is also a preferred practice to express certain designer genes of biofuel-production-pathways (Figs. 1, 4, 5, 6, 7, 8, 10, 13, and 14) such as SEQ ID NOS. 174-179 in a hydrogenotrophic and/or methanogenic host cell for chemolithotrophic production of advanced biofuels such as 1-buatanol from hydrogen (H₂) and carbon dioxide (CO₂). According to one of the various embodiments, a hydrogenotrophic and/or fermentative or methanogenic host organism for this specific application is selected from the group consisting of: *Methanocella paludicola SANAE, Acinetobacter baumannii ABNIH3, Acinetobacter baumannii ABNIH4, Acinetobacter sp. DR1, Agrobacterium sp. H13-3; Agrobacterium vitis S4, Alcaligenes sp., Allochromatium vinosum DSM 180, Amycolatopsis mediterranei S699, Anoxybacillus flavithermus WK1, Aquifex aeolicus VF5, Archaeoglobus fulgidus DSM 4304, Archaeoglobus veneficus SNP6, Azospirillum sp. B510, Burkholderia cenocepacia HI2424, Caldicellulosiruptor bescii DSM 6725, Carboxydothermus hydrogenoformans, Centipeda periodontii DSM 2778, Clostridium autoethanogenum, Clostridium ragsdalei, Clostridium sticklandii DSM 519, Clostridium sticklandii, Corynebacterium glutamicum, Cupriavidus metallidurans CH34, Cupriavidus necator N-1, Desulfobacca acetoxidans DSM 11109, Exiguobacterium sp. AT1b, Ferrimonas balearica DSM 9799, Ferroglobus placidus DSM 10642, Geobacillus kaustophilus HTA426, Helicobacter bilis ATCC 43879, Herbaspirillum seropedicae SmR1, Hydrogenobacter thermophilus TK-6, Hydrogenovibrio marinus, Klebsiella variicola At-22, Methanobacterium sp. SWAN-1, Methanobrevibacter ruminantium M1, Methanocaldococcus fervens AG86, Methanocaldococcus infernos ME, Methanocaldococcus jannaschii, Methanocaldococcus sp. FS406-22, Methanocaldococcus vulcanius M7,Methanococcus aeolicus Nankai-3, Methanococcus maripaludis C6, Methanococcus maripaludis S2, Methanococcus voltae A3,Methanocorpusculum labreanum Z, Methanoculleus marisnigri JR1,Methanohalophilus mahii DSM 5219, Methanolinea tarda NOBI-1, Methanoplanus petrolearius DSM 11571,Methanoplanus petrolearius, Methanopyrus kandleri AV19, Methanoregula boonei 6A8, Methanosaeta harundinacea 6Ac, Methanosalsum zhilinae DSM 4017, Methanosarcina acetivorans C2A, Methanosarcina barkeri str. Fusaro, Methanosarcina mazei Go1, Methanosphaera stadtmanae, Methanospirillum hungatei JF-1, Methanothermobacter marburgensis str. Marburg, Methanothermobacter marburgensis, Methanothermobacter thermautotrophicus, Methanothermococcus okinawensis IH1, Methanothermus fervidus DSM 2088, Methylobacillus flagellates, Methylobacterium organophilum, Methylococcus capsulatus, Methylomicrobium kenyense, Methylomonas methanica MC09, Methylomonas sp. LW13, Methylosinus sp. LW2, Methylosinus trichosporium OB3b, Methylotenera mobilis JLW8, Methylotenera versatilis 301, Methylovorus glucosetrophus SIP3-4, Moorella thermoacetica ATCC 39073, Moorella thermoacetica, Oligotropha carboxidovorans OM5, Paenibacillus terrace HPL-003, Pelotomaculum thermopropionicum SI, Planctomyces brasiliensis DSM 5305, Pyrococcus furiosus DSM 3638, Pyrococcus horikoshii OT3, Pyrococcus yayanosii CH1, Ralstonia eutropha H16, Rubrivivax sp., Selenomonas noxia ATCC 43541, Shewanella baltica BA175, Stenotrophomonas sp. SKA14, Synechococcus sp. JA-2-3B'a(2-13), Synechococcus sp. JA-3-3Ab, Thermococcus gammatolerans EJ3, Thermococcus kodakarensis KOD1, Thermococcus onnurineus NA1, Thermococcus sp. 4557, Thermodesulfatator indicus DSM 15286, Thermofilum pendens Hrk 5, Thermotoga lettingae TMO, Thermotoga petrophila RKU-1, Thiocapsa roseopersicina, Thiomonas intermedia K12, Xanthobacter autotrophicus, Yersinia pestis Antiqua, Phaeodactylum tricornutum, Methanosarcina barkeri,* and *Microcoleus vaginatu,* and combinations thereof.

### Designer Methanol-Production Pathways

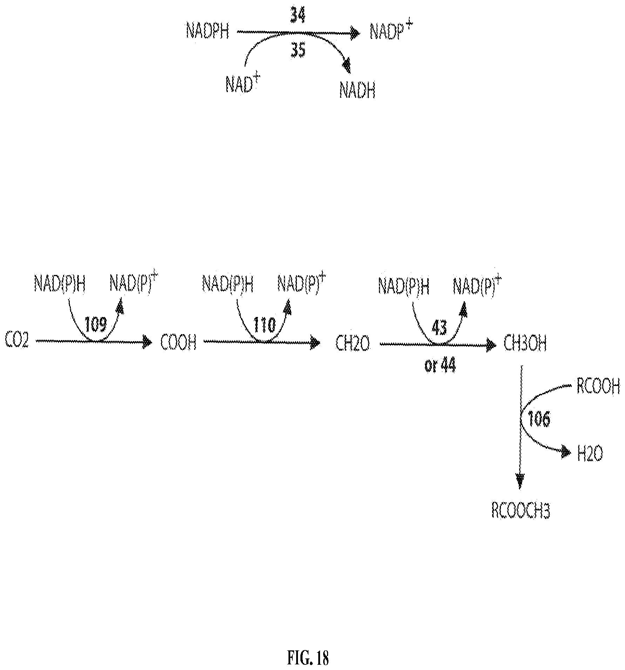

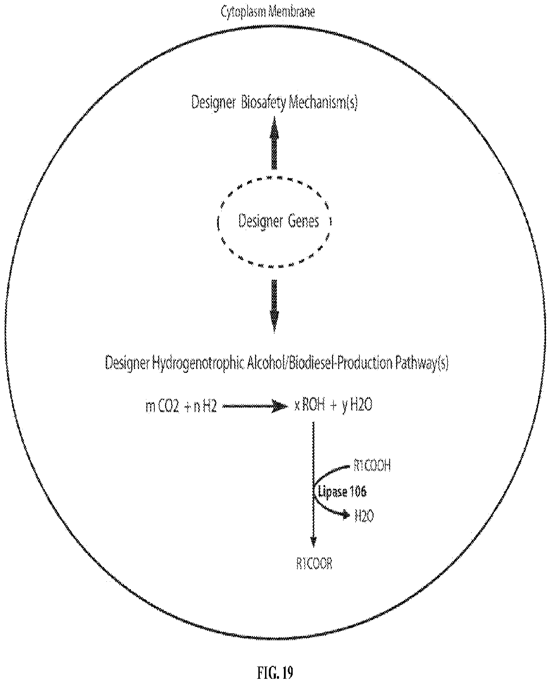

According to one of the various embodiments, a designer methanol-production pathway is created in a transgenic organism to convert carbon dioxide into methanol (Fig. 18). This designer methanol-production pathway comprises three different dehydrogenases: a formate dehydrogenase (FₐₜₑDH) **109,** formaldehyde dehydrogenase (F_{ald}DH) **110,** and an alcohol dehydrogenase (ADH) **43** (or **44**) as numerically labeled in Fig. 18. The methanol-production pathway process consists of three steps: 1) the reduction of CO₂ to formate catalyzed by formate dehydrogenase (FₐₜₑDH), 2) the reduction of formate to formaldehyde by formaldehyde dehydrogenase (F_{ald}DH), and 3) the reduction of formaldehyde to methanol by alcohol dehydrogenase (ADH). Reduced nicotinamide adenine dinucleotide (NADH), acts as a terminal electron donor for each of these dehydrogenase-catalyzed reductions. Note, in a hydrogenotrophic process (Fig. 19), reduced nicotinamide adenine dinucleotide (NADH) can be regenerated through reduction of NAD⁺ by use of molecular hydrogen (H₂) under the catalysis of a hydrogenase. Therefore, use of the designer methanol-production pathway in an anaerobic hydrogenotrophic host organism can produce methanol from CO₂ and H₂ through the following hydrogenotrophic process reaction:

CO₂ + 3H₂ → CH₃OH + H₂O [23]

According to one of the various embodiments, as shown in Fig. 18, the supply of reduced nicotinamide adenine dinucleotide (NADH) for the designer methanol-production pathway in a transgenic photosynthetic organism is accomplished through the use of an NADPH/NADH conversion process that, as disclosed in equations 3 and 4 above, is achieved by a two-step mechanism: 1) Use of an NADPH-dependent glyceraldehyde-3-phosphate dehydrogenase **34,** which uses NADPH in reducing1,3-diphosphoglycerate to glyceraldehydes-3-phosphate; and 2) use of an NAD⁺-dependent glyceraldehyde-3-phosphate dehydrogenase **35,** which produces NADH in oxidizing glyceraldehyde-3-phosphate tol,3-diphosphoglycerate. The net result of this two-step mechanism is the conversion of photosynthetically generated NADPH to NADH, which can support the designer methanol-production pathway for synthesis of methanol from carbon dioxide and water according to the following process reaction:

2CO₂ + 4H₂O → 2CH₃OH + 3O₂ [24]

SEQ ID NO: 199 presents example 199 of a designer nirA-promoter-controlled NAD-dependent formate dehydrogenase DNA construct (1636 bp) that includes a PCR FD primer (sequence 1-20), a 89-bp *Synechocystis sp.* strain PCC 6803 nitrite-reductase nirA promoter (21-109), an enzyme-encoding sequence (110-1207) selected from a *Komagataella pastoris* NAD-dependent formate dehydrogenase (GenBank: AB472090), a 409-bp *Synechocystis sp.* PCC 6803 rbcS terminator (1208-1616), and a PCR RE primer (1617-1636).

SEQ ID NO: 200 presents example 200 of a designer nirA-promoter-controlled Formaldehyde dehydrogenase DNA construct (1567 bp) that includes a PCR FD primer (sequence 1-20), a 89-bp *Synechocystis sp.* strain PCC 6803 nitrite-reductase nirA promoter (21-109), an enzyme-encoding sequence (110-1138) selected from a *Mycobacterium marinum* Formaldehyde dehydrogenase (GenBank: EPQ77120), a 409-bp Synechocystis sp. PCC 6803 rbcS terminator (1139-1547), and a PCR RE primer (1548-1567).

SEQ ID NO: 201 presents example 201 of a designer nirA-promoter-controlled NAD(P)H-dependent Alcohol dehydrogenase DNA construct (1549 bp) that includes a PCR FD primer (sequence 1-20), a 89-bp *Synechocystis sp.* strain PCC 6803 nitrite-reductase nirA promoter (21-109), an enzyme-encoding sequence (110-1120) selected from a *Methylophaga thiooxydans* Alcohol dehydrogenase (GenBank: KGM07167) for methanol, a 409-bp Synechocystis sp. PCC 6803 rbcS terminator (1121-1529), and a PCR RE primer (1530-1549).

Note, use of SEQ ID NOS. 199-201 in genetic transformation of a microbial host cell such as a cyanobacterium *Synechocystis sp.* strain PCC 6803 confers a designer methanol-production pathway (**109, 110, 43** or **44** in Figure 18). As described previously, the use of a soluble hydrogenase (SH, **71** and its accessory proteins **72**) enables generation of NAD(P)H through SH-mediated reduction of NAD(P)⁺ by H₂. Therefore, the expression of SEQ ID NOS. 199-201 in combination with the soluble hydrogenase (SH, **71** and its accessory proteins **72**) can produce methanol from CO₂ and H₂ according to the hydrogenotrophic process reaction [23].

SEQ ID NO: 202 presents example 202 of a designer nirA-promoter-controlled Formate dehydrogenase DNA construct (1180bp) that includes a PCR FD primer (sequence 1-20), a 231-bp nirA promoter from *Thermosynechococcus elongatus* BP1 (21-251), an enzyme-encoding sequence (252-1040) selected/modified from the sequences of a *Bacillus subtilis* Formate dehydrogenase (KFC29810), a 120-bp *rbcS* terminator from BP1 (1041-1160), and a PCR RE primer (1161-1180) at the 3' end.

SEQ ID NO: 203 presents example 203 of a designer nirA-promoter-controlled formaldehyde dehydrogenase DNA construct (1432bp) that includes a PCR FD primer (sequence 1-20), a 231-bp nirA promoter from *Thermosynechococcus elongatus* BP1 (21-251), an enzyme-encoding sequence (252-1292) selected/modified from the sequences of a *Geobacillus thermoglucosidans* formaldehyde dehydrogenase (EID43710), a 120-bp *rbcS* terminator from BP1 (1293-1412), and a PCR RE primer (1413-1432) at the 3' end.

SEQ ID NO: 204 presents example 204 of a designer nirA-promoter-controlled thermo tolerant NADPH-dependent Alcohol Dehydrogenase DNA construct (1579 bp) that includes a PCR FD primer (sequence 1-20), a 231-bp nirA promoter from *Thermosynechococcus elongatus* BP1 (21-251), an enzyme-encoding sequence (252-1439) selected/modified from the sequences of a *Pelotomaculum thermopropionicum* NADPH-dependent Alcohol Dehydrogenase (BAF58669) which can be used for methanol production, a 120-bp *rbcS* terminator from BP1 (1440-1559), and a PCR RE primer (1560-1579) at the 3' end.

Note, use of SEQ ID NOS. 202-204, 58 and 59 in genetic transformation of a microbial host cell including (but not limited to) bacterial cells such as a cyanobacterium *Thermosynechococcus elongatus* can create a designer cyanobacterium such as designer *Thermosynechococcus* with a designer methanol-production pathway (numerically labeled as **109, 110, 44, 34** and **35** in Figure 18) for production of methanol from water and carbon dioxide. The use of an NADPH-dependent glyceraldehyde-3-phosphate dehydrogenase **34** and an NAD⁺-dependent glyceraldehyde-3-phosphate dehydrogenase **35** (encoded by SEQ ID NOS. 58 and 59) provides the two-step mechanism for the conversion of photosynthetically generated NADPH to NADH, which can support the designer methanol-production pathway for production of methanol from carbon dioxide and water according to the photoautotrophic process reaction [24].

### Designer Alcohol-Biodiesel-Production Pathways

According to one of the various embodiments, a designer lipase **106** gene is expressed in combination with an alcohol (ROH)-production-pathway (Figs. 18, 19, and 20), which results in production of biodiesel (R₁COOR) through the following lipase-catalyzed reaction:

R₁COOH + ROH → R₁COOR+H₂O [25]

Wherein fatty acid (R₁COOH) is from the cell's natural fatty-acid synthesis pathway while alcohol (ROH) is from the designer alcohol-production pathway that is expressed in conjunction with the expression of designer lipase gene. Here, the "R₁" group represents the hydrocarbon chain of a fatty acid. The "R" group represents the hydrocarbon part of an alcohol such as methanol, ethanol, propanol, 1-butanol, isobutanol or pentanol molecule.

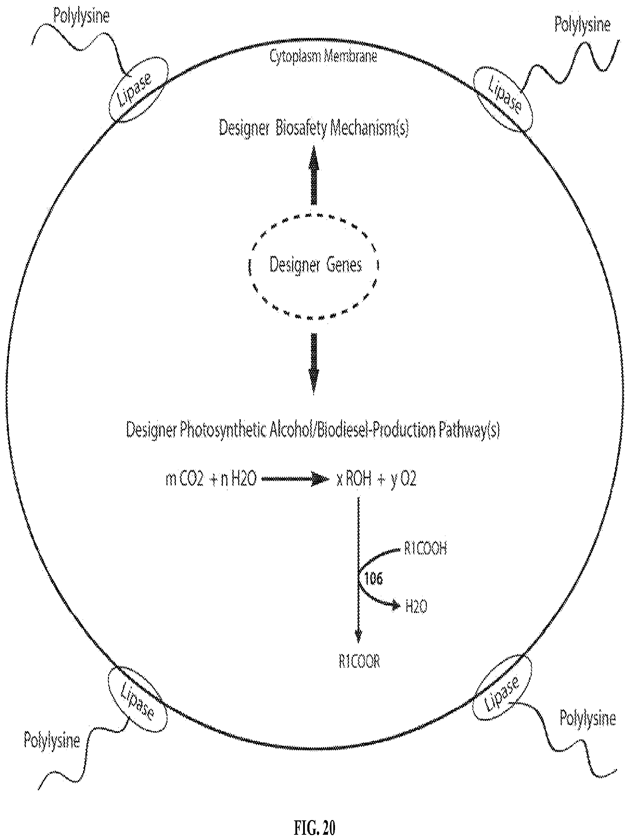

According to one of the various embodiments, host cells typically have native lipid synthesis pathway(s) that can produce triglyceride (R₁OCOCH₂-CH(OCOR₂)-CH₂OCOR₃) in which "R₁", "R₂" and "R₃" represent the three hydrocarbon chains of the acyl groups Therefore, the expression of lipase **106** in combination with alcohol-production pathway(s) in host cells (Figs. 19 and 20) can results in production of biodiesel molecules (R₁COOR, R₂COOR, and R₃COOR) through the following lipase-catalyzed triglyceride transesterification reactions. First, a triglyceride is transesterified with an alcohol (ROH) to a biodiesel (R₁COOR) and a diglyceride (HOCH₂-CH(OCOR₂)-CH₂OCOR₃):

R₁OCOCH₂-CH(OCOR₂)-CH₂OCOR₃ + ROH → R₁COOR + HOCH₂-CH(OCOR₂)-CH₂OCOR₃

Then, the diglyceride (HOCH₂-CH(OCOR₂)-CH₂OCOR₃) from the process above is further transesterified to another biodiesel molecule(R₂COOR) and a monoglyceride (HOCH₂-CH(OH)-CH₂OCOR₃) :

HOCH₂-CH(OCOR₂)-CH₂OCOR₃ + ROH → R₂COOR + HOCH₂-CH(OH)-CH₂OCOR₃

Finally, the monoglyceride (HOCH₂-CH(OH)-CH₂OCOR₃) is transesterified to produce yet another biodiesel molecule (R3COOR) with glycerol (HOCH₂-CH(OH)-CH₂OH) as a byproduct:

HOCH₂-CH(OH)-CH₂OCOR₃ + ROH → R₃COOR + HOCH₂-CH(OH)-CH₂OH

Therefore, the innovative use of a lipase and an alcohol in vivo and/or in vitro enables the conversion of lipids including fatty acids and triglycerides into biodiesel. According to one of the various embodiments, the alcohol (ROH) that is utilized in these lipase-catalyzed biodiesel-production reactions is selected from the group consisting of methanol, ethanol, propanol, 1-butanol, isobutanol, 2-methyl-1-butanol, isobutanol, 3-methyl-1-butanol, 1-hexanol, 1-octanol, 1-pentanol, 1-heptanol, 3-methyl-1-pentanol, 4-methyl-1-hexanol, 5-methyl-1-heptanol, 4-methyl-1-pentanol, 5-methyl-1-hexanol, 6-methyl-1-heptanol, and/or combination thereof.

According to one of the various embodiments, as illustrated in Fig. 18, a designer photoautotrophic methanol-biodiesel-production pathway in a transgenic photosynthetic organism comprises: NADPH-dependent glyceraldehyde-3-phosphate dehydrogenase **34,** NAD⁺-dependent glyceraldehyde-3-phosphate dehydrogenase **35,** formate dehydrogenase (FₐₜₑDH) **109,** formaldehyde dehydrogenase (F_{ald}DH) **110**, alcohol dehydrogenase (ADH) **43** (or **44**), and lipase **106**.

According to another embodiment, a designer hydrogenotrophic methanol-biodiesel production pathway in a transgenic organism comprises: NAD-reducing soluble hydrogenase **71** (Fig 12), formate dehydrogenase (FₐₜₑDH) **109,** formaldehyde dehydrogenase (F_{ald}DH) **110,** alcohol dehydrogenase (ADH) **43** (or **44**), and lipase **106** (Fig. 18).

According to one of the various embodiments, as illustrated in Figs. 3C and 20, a designer ethanol-biodiesel-production pathway in a transgenic photosynthetic organism comprises: NADPH-dependent glyceraldehyde-3-phosphate dehydrogenase **34,** NAD⁺-dependent glyceraldehyde-3-phosphate dehydrogenase **35,** phosphoglycerate mutase **03,** enolase **04,** pyruvate kinase **05,** pyruvate decarboxylase **108,** alcohol dehydrogenase **44,** and lipase **106.**

According to one of the various embodiments, as illustrated in Figs. 1 and 20, a designer butanol-biodiesel-production pathway in a transgenic photosynthetic organism comprises: NAD⁺-dependent glyceraldehyde-3-phosphate dehydrogenase **35,** phosphoglycerate mutase **03,** enolase **04,** pyruvate kinase **05,** pyruvate-ferredoxin oxidoreductase **06,** acetyl-CoA acetyltransferase (or, thiolase **07**), 3-hydroxybutyryl-CoA dehydrogenase **08,** crotonase **09,** trans-enoyl-CoA reductase (or butyryl-CoA dehydrogenase **10**), butyraldehyde dehydrogenase (or aldehyde/alcohol dehydrogenase (AdhE2)) **11,** butanol dehydrogenase **12,** and lipase **106.**

According to one of the various embodiments, as illustrated in Figs. 4 and 20, a designer butanol-biodiesel-production pathway in a transgenic photosynthetic organism comprises: NADPH-dependent glyceraldehyde-3-phosphate dehydrogenase **34,** NAD-dependent glyceraldehyde-3-phosphate dehydrogenase **35,** phosphoglycerate mutase **03,** enolase **04,** phosphoenolpyruvate carboxylase **45,** aspartate aminotransferase **46,** aspartokinase **47,** aspartate-semialdehyde dehydrogenase **48,** homoserine dehydrogenase **49,** homoserine kinase **50,** threonine synthase **51,** threonine ammonia-lyase **52,** 2-isopropylmalate synthase **40,** isopropylmalate isomerase **41,** 3-isopropylmalate dehydrogenase **39,** 2-keto acid decarboxylase **42,** NAD-dependent alcohol dehydrogenase **43** (and/or NADPH-dependent alcohol dehydrogenase **44,** or butanol dehydrogenase **12**), and lipase **106.**

According to one of the various embodiments, as illustrated in Figs. 6 and 20, a designer isobutanol-biodiesel-production pathway in a transgenic photosynthetic organism comprises: NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** acetolactate synthase **53,** ketol-acid reductoisomerase **54,** dihydroxy-acid dehydratase **55,** 2-keto acid decarboxylase **42,** NAD-dependent alcohol dehydrogenase **43** (or NADPH-dependent alcohol dehydrogenase **44**), and lipase **106.**

According to another embodiment, as illustrated in Figs. 6 and 20, a designer 3-methyl-1-butanol-biodiesel-production pathway in a transgenic photosynthetic organism comprises: NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** acetolactate synthase **53,** ketol-acid reductoisomerase 54, dihydroxy-acid dehydratase **55,** 2-isopropylmalate synthase **40,** 3-isopropylmalate dehydratase **38,** 3-isopropylmalate dehydrogenase **39,** 2-keto acid decarboxylase **42,** NAD-dependent alcohol dehydrogenase **43** (or NADPH-dependent alcohol dehydrogenase **44;** or more preferably, 3-methylbutanal reductase **57**), and lipase **106.**

According to another embodiment, as illustrated in Figs. 7 and 20, a designer hexanol - biodiesel-production pathway in a transgenic photosynthetic organism comprises: NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** pyruvate-ferredoxin oxidoreductase **06,** thiolase **07,** 3-hydroxybutyryl-CoA dehydrogenase **08,** crotonase **09,** butyryl-CoA dehydrogenase **10,** designer 3-ketothiolase **07',** designer 3-hydroxyacyl-CoA dehydrogenase **08'**, designer enoyl-CoA dehydratase **09',** designer 2-enoyl-CoA reductase **10',** designer acyl-CoA reductase **11',** hexanol dehydrogenase **12'** and lipase

### 106.

According to another embodiment, as illustrated in Figs. 8, 18 and 20, a designer transgenic alcohol (methanol, 1-pentanol, 1-hexanol, and/or 1-heptanol)-biodiesel-producing organism comprises a set of enzymes comprising at least one of the enzymes selected from the group consisting of lipase **106,** formate dehydrogenase (FₐₜₑDH) **109,** formaldehyde dehydrogenase (FₐₗₐDH) **110,** alcohol dehydrogenase (ADH) **43** (or **44**), NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** citramalate synthase **36,** 2-methylmalate dehydratase **37,** 3-isopropylmalate dehydratase **38,** 3-isopropylmalate dehydrogenase **39,** 2-isopropylmalate synthase **40,** isopropylmalate isomerase **41,** 3-isopropylmalate dehydrogenase **39,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** hexanol dehydrogenase **12',** designer isopropylmalate synthase **40",** designer isopropylmalate isomerase **41",** designer 3-isopropylmalate dehydrogenase **39",** designer 2-keto acid decarboxylase **42",** and designer short-chain alcohol dehydrogenase **43".**

According to another embodiment, as illustrated in Figs. 9 and 20, a designer branched higher alcohols (3-methyl-1-pentanol, 4-methyl-1-hexanol, and/or 5-methyl-1-heptanol) - biodiesel-production pathway in a transgenic photosynthetic organism comprises: NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** citramalate synthase **36,** 2-methylmalate dehydratase **37,** 3-isopropylmalate dehydratase **38,** 3-isopropylmalate dehydrogenase **39,** acetolactate synthase **53,** ketol-acid reductoisomerase **54,** dihydroxy-acid dehydratase **55,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** designer isopropylmalate synthase **40",** designer isopropylmalate isomerase **41",** designer 3-isopropylmalate dehydrogenase **39",** designer 2-keto acid decarboxylase **42",** designer short-chain alcohol dehydrogenase **43",** and lipase **106.**

According to another embodiment, as illustrated in Figs. 10 and 20, a designer branched-chain higher alcohols (4-methyl-1-pentanol, 5-methyl-1-hexanol, and 6-methyl-1-heptanol) -biodiesel-production pathway in a transgenic photosynthetic organism comprises: NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** acetolactate synthase **53,** ketol-acid reductoisomerase **54,** dihydroxy-acid dehydratase **55,** isopropylmalate synthase **40,** dehydratase **38,** 3-isopropylmalate dehydrogenase **39,** designer isopropylmalate synthase **40',** designer isopropylmalate isomerase **41',** designer 3-isopropylmalate dehydrogenase **39',** designer 2-keto acid decarboxylase **42',** short-chain alcohol dehydrogenase **43',** designer isopropylmalate synthase **40",** designer isopropylmalate isomerase **41",** designer 3-isopropylmalate dehydrogenase **39",** designer 2-keto acid decarboxylase **42",** designer short-chain alcohol dehydrogenase **43",** and lipase **106.**

According to one of the various embodiments, as illustrated in Figs. 12, 13 and 19, a designer anaerobic hydrogenotrophic 1-butanol -biodiesel-production pathway in a transgenic organism comprises: energy converting hydrogenase (Ech) **91,** [NiFe]-hydrogenase (Mvh) **95,** Coenzyme F₄₂₀-reducing hydrogenase (Frh) **96,** native (or heterologous) soluble hydrogenase (SH) **71,** heterodissulfide reductase (Hdr) **94,** formylmethanofuran dehydrogenase **83,** formyl transferase **84,** 10-methenyl-tetrahydromethanopterin cyclohydrolase **85,** 10-methylene-H₄ methanopterin dehydrogenase **86,** 10-methylene-H₄-methanopterin reductase **87,** methyl-H₄-methanopterin: corrinoid iron-sulfur protein methyltransferase **88,** corrinoid iron-sulfur protein **89,** CO dehydrogenase/acetyl-CoA synthase **90,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12** and/or alcohol dehydrogenase **43,** and lipase **106.**

According to one of the various embodiments, as illustrated in Figs. 14 and 19, another designer anaerobic hydrogenotrophic 1-butanol-biodiesel-production pathway in a transgenic organism comprises: formate dehydrogenase **74,** 10-formyl-H₄ folate synthetase **75,** methenyltetrahydrofolate cyclohydrolase **76,** 10-methylene-H₄ folate dehydrogenase **77,** 10-methylene-H₄ folate reductase **78,** methyl-H₄ folate: corrinoid iron-sulfur protein methyltransferase **79,** corrinoid iron-sulfur protein **80,** CO dehydrogenase/acetyl-CoA synthase **81,** thiolase **07,** 3-hydroxybutyryl-CoA dehydrogenase **08,** crotonase **09,** butyryl-CoA dehydrogenase **10,** butyaldehyde dehydrogenase **11,** butanol dehydrogenase **12,** and/or alcohol dehydrogenase **43,** and lipase **106.**

SEQ ID NO: 205 presents example 205 of a designer nirA-promoter-controlled lipase DNA construct (1822 bp) that includes a PCR FD primer (sequence 1-20), a 231-bp nirA promoter from *Thermosynechococcus elongatus* BP1 (21-251), an enzyme-encoding sequence (252-1682) selected/modified from the sequences of a *Pseudomonas fluorescens* lipase (AAA25882) which can use butanol, a 120-bp *rbcS* terminator from BP1 (1683-1802), and a PCR RE primer (1803-1822) at the 3' end.

SEQ ID NO: 206 presents example 206 of a designer nirA-promoter-controlled lipase DNA construct (1486 bp) that includes a PCR FD primer (sequence 1-20), a 231-bp nirA promoter from *Thermosynechococcus elongatus* BP1 (21-251), an enzyme-encoding sequence (252-1346) selected/modified from the sequences of a *Burkholderia cepacia* lipase (M58494) which can use ethanol, a 120-bp *rbcS* terminator from BP1 (1347-1466), and a PCR RE primer (1467-1486) at the 3' end.

SEQ ID NO: 207 presents example 207 of a designer nirA-promoter-controlled Pyruvate Decarboxylase DNA construct (2098 bp) that includes a PCR FD primer (sequence 1-20), a 231-bp nirA promoter from *Thermosynechococcus elongatus* BP1 (21-251), an enzyme-encoding sequence (252-1958) selected/modified from the sequences of a *Zymomonas mobilis* Pyruvate Decarboxylase (AFN57569), a 120-bp *rbcS* terminator from BP1 (1959-2078), and a PCR RE primer (2079-2098) at the 3' end.

Note, use of SEQ ID NOS. 205 and 58-70 in genetic transformation of a microbial host cell including (but not limited to) bacterial cells such as a cyanobacterium *Thermosynechococcus elongatus* creates a designer cyanobacterium such as designer *Thermosynechococcus* which comprises lipase and designer nirA-promoter-controlled Calvin-cycle-channeled1-butanol production pathway (as shown with numerical labels **34, 35, 03-05,** and **36-43** in Figure 4) for production of biodiesel from water and carbon dioxide (Fig. 20).

Similarly, the use of SEQ ID NOS. 206, 207, 204, and 58-62 in a microbial host cell including (but not limited to) bacterial cells such as a cyanobacterium *Thermosynechococcus elongatus* represents a designer cyanobacterium such as designer *Thermosynechococcus* comprising designer lipase and Calvin-cycle-channeled ethanol-production pathways for production of biodiesel from water and carbon dioxide (Fig. 20). The photoautotrophic ethanol-biodiesel-production pathway comprises lipase **106,** NADPH-dependent glyceraldehyde-3-phosphate dehydrogenase **34,** NAD-dependent glyceraldehyde-3-phosphate dehydrogenase **35,** phosphoglycerate mutase **03,** enolase **04,** pyruvate kinase **05,** pyruvate decarboxylase **108,** and alcohol dehydrogenase **44.**

SEQ ID NO: 208 presents example 208 of a designer nirA-promoter-controlled Lipase DNA construct (1567 bp) that includes a PCR FD primer (sequence 1-20), a 89-bp *Synechocystis sp.* strain PCC 6803 nitrite-reductase nirA promoter (21-109), an enzyme-encoding sequence (110-1138) selected from a *Candida antarctica* Lipase (GenBank: CAA83122) which can use methanol, a 409-bp Synechocystis sp. PCC 6803 rbcS terminator (1139-1547), and a PCR RE primer (1548-1567).

Note, use of SEQ ID NOS. 208 and 199-201 in genetic transformation of a microbial host cell such as a cyanobacterium *Synechocystis sp.* strain PCC 6803 confers a designer methanol-biodiesel-production pathway (**109, 110, 43/44,** and **106** as numerically labeled in Figure 18). This also represents an example of a designer *Synechocystis* for photoautotrophic production of alcohol (methanol) and biodiesel from CO₂ and H₂O and as illustrated in Figs. 18 and 20.

SEQ ID NO: 209 presents example 209 of a designer *hox*-promoter-controlled Lipase DNA construct (2075 bp) that includes a PCR FD primer (sequence 1-20), a 172-bp *Nostoc sp.* strain PCC 7120 *(Anabaena* PCC 7120) *hox* promoter (21-192), an enzyme-encoding sequence (193-1623) selected/modified from the sequence of Lipase (GenBank: AAA25882) of *Pseudomonas fluorescens,* a 432-bp *Nostoc sp.* strain PCC 7120 *gor* terminator (1624-2055), and a PCR RE primer (2056-2075) at the 3' end.

Note, use of SEQ ID NOS. 209 and 166-179 in genetic transformation of a microbial host cell including (but not limited to) bacterial cells such as a cyanobacterium *Anabaena* PCC 7120 can create a designer cyanobacterium such as designer *Anabaena* with lipase and a designer reductive-acetyl-CoA 1-butanol-production pathway (numerically labeled as 83-90 and 07-12 in Figure 13) for production of biodiesel from molecular hydrogen (H₂) and carbon dioxide (CO₂) without requiring photosynthesis or sunlight. This also represents an example of a designer hydrogenotrophic cell for hydrogenotrophic production of alcohol (butanol) and biodiesel from H₂ and CO₂ as illustrated in Fig. 19.

SEQ ID NO: 210 presents example 210 of a designer *Synechococcus sp.* strain PCC 7942 nirA-promoter-controlled Lipase gene DNA construct (2290 base pairs (bp)) that includes a PCR FD primer (sequence bp 1-20), a 88-bp *nirA* promoter (21-108) selected from the *Synechococcus sp.* strain PCC 7942 (freshwater cyanobacterium) nitrite-reductase-gene promoter sequence, an enzyme-encoding sequence (109-1962) selected and modified from a *Pseudomonas fluorescens* Lipase (GenBank accession number: BAC98499), a 308-bp *Synechococcus sp.* strain PCC 7942 *rbcS* terminator (1963-2270), and a PCR RE primer (2271-2290) at the 3' end.

Note, in the designer transgenic *Synechococcus* that is represented by SEQ ID NOS: 210 and 95-98 (and/or 99), *Synechococcus'* native enzymes of **03-05, 36-41** and **45-52** are used in combination with the designer *nirA*-promoter-controlled enzymes of **34, 35, 42** and **12** [encoded by SEQ ID NOS: 95-98 (and/or 99)] to confer a designer lipase and the Calvin-cycle 3-phophoglycerate-branched photosynthetic NADPH-enhanced pathways for photobiological production of 1-butanol from carbon dioxide and water (Figure 4). The 1-butanol produced from carbon dioxide and water (Figure 4) is used by the lipase (encoded by SEQ ID NO: 210) in the transesterification of triglyceride and/or fatty acid for production of biodiesel (Fig. 20).

SEQ ID NO:211 presents example 211 for a designer *groE*-promoter-controlled Lipase DNA construct (1231 bp) that includes a PCR FD primer (sequence 1-20), a 137-bp *Prochlorococcus marinus* MIT9313 heat- and light-responsive *groE* promoter (21-157), an enzyme-encoding sequence (158-1090) selected from a *Brachybacterium tyrofermentans* Lipase (GenBank: ACD89058), a 121-bp *Prochlorococcus marinus* MIT9313 *rbcS* terminator (1091-1213), and a PCR RE primer (1214-1231).

Use of *Prochlorococcus marinus* MIT 9313 as a host organism in genetic transformation with SEQ ID NOS: 211 and 110-122 can create a designer transgenic *Prochlorococcus marinus* for photobiological production of isobutanol and/or 3-methyl-1-butanol (Figure 6), wherein the produced alcohol(s) is simultaneously and/or subsequently used by the lipase (encoded by SEQ ID NO: 211) in the transesterification of triglyceride and/or fatty acids for production of biodiesel (Fig. 20). Therefore, this also represents an example of a designer *Prochlorococcus* for photoautotrophic production of alcohols (isobutanol and/or 3-methyl-1-butanol) and biodiesel from CO₂ and H₂O and as illustrated in Fig. 20.

SEQ ID NO:212 presents example 212 for a designer *nirA*-promoter-controlled Lipase DNA construct (1386 bp) that includes a PCR FD primer (sequence 1-20), a 203-bp *Cyanothece sp. ATCC 51142 nirA* promoter (21-223), an enzyme-encoding sequence (224-1165) selected from a *Rhodococcus erythropolis* Lipase sequence (GenBank: ACD89059), a 201-bp *Cyanothece sp. ATCC 51142* rbcS terminator (1166-1376), and a PCR RE primer (1377-1386).

Use of *Cyanothece sp. ATCC 51142* as a host organism in genetic transformation with SEQ ID NOS: 212 and 123-128 can create a designer transgenic *Cyanothece* for photobiological production of 1-pentanol, 1-hexanol, and/or 1-heptanol (Figure 8), wherein the produced alcohol(s) is simultaneously and/or subsequently used by the lipase (encoded by SEQ ID NO: 212) in the transesterification of triglyceride and/or fatty acids for production of biodiesel (Fig. 20).

SEQ ID NO. 213 presents example 213 of a designer Nial-promoter-controlled chloroplast-targeted Lipase DNA construct (2420 bp) that includes a PCR FD primer (sequence 1-20), a 2 x 84-bp *Chlamydomonas reinhardtii* Nial (nitrate reductase) promoter (21-188), a 135-bp *Chlamydomonas reinhardtii RbcS2* transit peptide (189-323), a Lipase-encoding sequence (324-2177) selected/modified from *Pseudomonas sp. 7323* Lipase (CAJ76166), a 223-bp *Chlamydomonas reinhardtii RbcS2* terminator (2178-2400), and a PCR RE primer (2401-2420).

Note, use of SEQ ID NOS. 213, 129-133, 151-153, 140 and 141 (or 142) in genetic transformation of an eukaryotic photosynthetic organism such as *Chlamydomonas* can create a designer eukaryotic photosynthetic organism such as designer *Chlamydomonas* with a Calvin-cycle 3-phosphogylcerate-branched NADPH-enhanced pathway **(03-05, 34, 35, 53-55, 42,** and **43 (44)** in Figure 6) for photobiological production of isobutanol from carbon dioxide and water, wherein the produced isobutanol is simultaneously and/or subsequently used by the lipase (encoded by SEQ ID NO: 213) in the transesterification of triglyceride and/or fatty acids for production of biodiesel (Fig. 20). Similarly, the use of SEQ ID NO: 213 and 1-12 in genetic transformation of an eukaryotic photosynthetic organism such as *Chlamydomonas* can create a designer eukaryotic photosynthetic organism such as designer *Chlamydomonas* with a glyceraldehydes-3-phosphate-branched butanol-production pathway **(01-12** in Figure 1), wherein the produced butanol is simultaneously and/or subsequently used by the lipase (encoded by SEQ ID NO: 213) in the transesterification of triglyceride and/or fatty acids for production of biodiesel (Fig. 20).

### Enhanced Evolution and Selection of Biofuel Alcohol-Producing Strains

According to one of the various embodiments, use of a biofuel alcohol-sensing responsive promoter regulatory system in combination of a selectable marker can enhance the screening for the transgenic cells with increased production of the target biofuels such as butanol and related higher alcohols. For example, the σ⁵⁴-transcriptional activator (BmoR) and a σ⁵⁴-dependent alcohol-regulated promoter (P_{BMO}) previously identified from *Thauera butanivorans* can be used as an example for such an alcohol-sensing responsive promoter regulatory system. It has been experimentally demonstrated that this BmoR- P_{BMO} genetic device can sense a wide range of the butanol and related higher alcohols concentrations and can thus be used as genetic switch to control the expression of a selectable marker (or designer gene) according to the concentration levels of the biofuel alcohols in bacteria such as *E. coli.* Therefore, in one of the various embodiments, this BmoR-P_{BMO} genetic device is used as an example to control the expression of a selectable marker gene selected from the group consisting of tetracycline resistance marker gene *tet*A, kanamycin resistance marker gene (kan^{r}), gentamicin resistance marker gene, spectinomycin resistance marker gene *Spcr,* streptomycin resistance *aadA* gene, ampicillin resistance marker gene *amp^{r}*, chloramphenicol resistance *CmR* gene, hygromycin resistance *(hph)* gene, glyphosate resistance genes *epsps,* argininosuccinate lyase (*arg7*) gene, nitrate reductase genes (*narG* and *napA*), and combinations thereof,

With the use of BmoR-P_{BMO} genetic switch in controlling the expression levels of a selectable marker such as tetracycline resistance marker gene *tet*A in responding to the concentrations of the product biofuel alcohols, only the most productive cells that produce sufficient amount of the biofuel alcohols which can turn on the BmoR-P_{BMO} genetic switch to express the selectable marker gene *tet*A will be able to survive in the presence of the antibiotic tetracycline in the culture medium. Therefore, this biofuel product-guided selection process through the use of an alcohol-sensing transcription regulator-coupled selection marker system can positively select better transgenic cells that possess more effective biofuel-production pathways. The selectable marker in this case does not have to be an antibiotic or herbicide resistance gene. Certain nutrient-related genes such as argininosuccinate lyase (*arg7*) gene or nitrate reductase genes (*narG* and *napA*) can also be used as a selectable marker for this biofuel alcohol-guided selection process, when certain auxotroph that lacks of the cognate argininosuccinate lyase (*arg7*) gene or nitrate reductase genes (*narG* and *napA*) is used as a host organism. A significant advantage of the biofuel-guided selection process is that it can eliminate the cheater cells that survive without producing the target biofuel molecule such as butanol and positively select the true biofuel (butanol) producer cells in an effective manner.

According to one of the various embodiments, this biofuel (alcohol) product-guided selection process is used in combination with a mutagenesis process including oligonucleotide-directed genome engineering to accelerate the molecular genetic evolution process in creating optimized biofuel-producing strains. It has been experimentally demonstrated that introduction of certain short (with a length range from about 80 to 100 bp) single-stranded DNA (ssDNA) or oligonucleotides (oligos) into certain host cells such as bacterial cells by electroporation can effectively create many mutations at many targeted locations in the host cell genomic DNA. In this approach, the oligos are directed to the lagging strand of the replication fork during DNA replication. Typically, these oligonucleotides are specially made so that the two end regions (30-40 bp) of each oligo are homologous to certain spots of the host chromosome, enabling them to bind with the lagging strand of the DNA replication fork at the targeted spots of the chromosome DNA for allelic replacements. The middle region of each oligo contains a designed mutation selected from the group consisting of mismatch, insertion and/or deletion. It is a preferred practice for each designer-made oligonucleotide to contain two phosphorothioated bases at its 3' and 5' terminals to increase the stability of the transforming oligonucleotide for higher allelic replacement efficiency. It is also a preferred practice to employ the bacteriophage λ-Red single-stranded DNA (ssDNA) binding protein β, which binds to ssDNA and promotes strand annealing, to mediate the recombineering process in the host cell for enhanced allelic replacement efficiency.

According to one of the various embodiments, use of the designer-made oligonucleotides can simultaneously target many different allelic DNA regions including the transcription regulatory regions, Shine-Dalgarno sequences and the proteins-encoding sequences in the host genome to generate large numbers (billions) of genetic variants for selection by biofuel product-guided selection process to obtain transgenic cells with optimized biofuel productivity. For example, the alcohol productivity may be dramatically optimized by simultaneously targeting the translational regulatory region such as the Shine-Dalgarno sequences of the biofuel-production-pathway genes for enhanced pathway activity while targeting the other competing pathway genes and/or their Shine-Dalgarno sequences for their reduced activity. Typically, in order for mRNA to be translated accurately, its sequence of codons must be brought into proper register with the translational apparatus which is the ribosomes. For example, in certain bacteria such as *E. coli,* the correct registration of mRNA on ribosome requires alignment of a pyrimidine-rich sequence on 3'-end of 16S RNA with a purine-rich part of 5'-end of mRNA. The purine-rich segment of mRNA is the ribosome-binding site also known as the Shine-Dalgarno sequence that is located typically within the first 18-bp upstream immediately from the initiation codon. In many cases where the ribosome-binding on the Shine-Dalgarno sequence positively regulate the translation efficiency, the designer mutation on the Shine-Dalgarno sequence should designed towards the canonical complementation with the16S RNA's 3'-end pyrimidine-rich sequence to enhance translation efficiency for a given mRNA. It is now also known that in certain host cells such as cyanobacteria, the ribosome-binding on the Shine-Dalgarno sequence negatively regulate the translation efficiency. In that case, it is a preferred practice to mutate the Shine-Dalgarno sequence away from the canonical complementation with the16S RNA's 3'-end pyrimidine-rich sequence to enhance translation efficiency for a given mRNA. Other genetic control devices such as riboswitches may also be employed with designer RNA aptamers for regulating designer transgene expression.

SEQ ID NO: 214 presents example 214 of a designer *Synechocystis sp.* PCC 6803 transcription factor-arginine-based alcohol-selection DNA construct (6498 bp) that includes a PCR FD primer (sequence 1-20), an alcohol-inducible σ⁵⁴-transcriptional promoter regulatory sequence (20-4683) selected/modified from a *Thauera butanivorans* transcription factor BmoR and its associated promoter regulatory sequence (AY093933), a selectable marker-encoding sequence (4684-6069) selected from a *Synechocystis sp. PCC 6803* argininosuccinate lyase (GenBank: NP_440604), a 409-bp *Synechocystis* sp. PCC 6803 rbcS terminator (6070-6478), and a PCR RE primer (6479-6498). Use of this DNA construct (SEQ ID NO: 214) enables enhanced evolution and selection for biofuel alcohol-producing designer *Synechocystis* cells based on arginine-nutrient complementation without requiring the use of any antibiotic resistance gene.

SEQ ID NO: 215 presents example 215 of a designer *Thermosynechococcus elongatus* transcription factor and nitrate reductase-based alcohol-selection DNA construct (7034 bp) that includes a PCR FD primer (sequence 1-20), an alcohol-inducible σ⁵⁴-transcriptional promoter regulatory sequence (20-4683) selected/modified from a *Thauera butanivorans* transcription factor BmoR and its associated promoter regulatory sequence (AY093933), a selectable marker-encoding sequence (4684-6884) selected from *Thermosynechococcus elongatus* BP-1 nitrate reductase (NP_682145), a 120-bp *rbcS* terminator from BP1 (6885-7014), and a PCR RE primer (7015-7034) at the 3' end. Use of this DNA construct (SEQ ID NO: 215) enables enhanced evolution and selection for biofuel alcohol-producing designer *Thermosynechococcus* cells through the use of nitrate- nutrient complementation without requiring the use of any antibiotic resistance gene.

SEQ ID NO: 216 presents example 216 of a designer *Synechococcus sp.* strain PCC 7942 transcription factor and Tetracycline resistance-based alcohol-selection and Lipase DNA construct (7006 base pairs (bp)) that includes a PCR FD primer (sequence bp 1-20), an alcohol-inducible σ⁵⁴-transcriptional promoter regulatory sequence (20-4047) selected/modified from a *Thauera butanivorans* transcription factor BmoR and its associated promoter regulatory sequence (AY093933), a selection marker encoding sequence (4048-5247) selected from tetracycline resistance protein sequence (AGF38340), a lipase enzyme-encoding sequence (5248-6678) selected/modified from the sequences of a *Pseudomonas fluorescens* lipase (AAU10321) which can use butanol and ethanol, a 308-bp *Synechococcus sp.* strain PCC 7942 *rbcS* terminator (6679-6986), and a PCR RE primer (6987-7006) at the 3' end. Use of this DNA construct (SEQ ID NO: 216) enables enhanced evolution and selection for alcohol-biodiesel producing designer *Synechococcus* cells with tetracycline resistance protein.

SEQ ID NO:217 presents example 217 for a designer *Prochlorococcus marinus MIT9313* transcription factor and glyphosate tolerant protein-based alcohol-selection DNA construct (5556 bp) that includes a PCR FD primer (sequence 1-20), an alcohol-inducible σ⁵⁴-transcriptional promoter regulatory sequence (20-4047) selected/modified from a *Thauera butanivorans* transcription factor BmoR and its associated promoter regulatory sequence (AY093933), a selectable marker-encoding sequence (4048-5415) selected from a Glyphosate tolerant 5-enolpyruvylshikimate-3-phosphate synthase (GenBank: AEM75108), a 121-bp *Prochlorococcus marinus* MIT9313 *rbcS* terminator (5416-5536), and a PCR RE primer (5537-5556). Use of this DNA construct (SEQ ID NO: 217) enables enhanced evolution and selection for alcohol-producing designer *Prochlorococcus* cells with a Glyphosate tolerant 5-enolpyruvylshikimate-3-phosphate synthase.

SEQ ID NO:218 presents example 218 for a designer *Cyanothece sp. ATCC 51142* transcription factor and nitrate reductase-based alcohol-selection DNA construct (6500 bp) that includes a PCR FD primer (sequence 1-20), an alcohol-inducible σ⁵⁴-transcriptional promoter regulatory sequence (20-4047) selected/modified from a *Thauera butanivorans* transcription factor BmoR and its associated promoter regulatory sequence (AY093933), a selectable marker-encoding sequence (4048-6279) selected from a *Cyanothece sp. ATCC 51142* nitrate reductase sequence (GenBank: ACB50564), a 201-bp *Cyanothece sp. ATCC 51142* rbcS terminator (6278-6480), and a PCR RE primer (6481-6500). Use of this DNA construct (SEQ ID NO: 218) enables enhanced evolution and selection for alcohol-producing designer *Cyanothece* cells based on nutrient (nitrate) complementation without requiring the use of any antibiotic resistance gene.

SEQ ID NO: 219 presents example 219 of a designer *Anabaena* PCC 7120 transcription factor and ketol-acid reductoisomerase/dihydroxy-acid dehydratase-based alcohol-selection DNA construct (7187 bp) that includes a PCR FD primer (sequence 1-20), an alcohol-inducible σ⁵⁴-transcriptional promoter regulatory sequence (20-4047) selected/modified from a *Thauera butanivorans* transcription factor BmoR and its associated promoter regulatory sequence (AY093933), a selectable marker-encoding sequence (4048-6735) selected/modified from an *Anabaena* PCC 7120 Ketol-acid reductoisomerase (GenBank: BAB74014) and an *Anabaena* PCC 7120 dihydroxy-acid dehydratase (NP_486811), a 432-bp *Nostoc sp.* strain PCC 7120 *gor* terminator (6736-7167), and a PCR RE primer (7168-7187) at the 3' end. Use of this DNA construct (SEQ ID NO: 219) enables enhanced evolution and selection for alcohol-producing designer *Anabaena* cells based on nutrients (valine, leucine and isoleucine) complementation without requiring the use of any antibiotic resistance gene.

SEQ ID NO: 220 presents example 220 of a designer *Anabaena* PCC 7120 transcription factor and argininosuccinate lyase-based alcohol-selection lipase DNA construct (5885 bp) that includes a PCR FD primer (sequence 1-20), an alcohol-inducible σ⁵⁴-transcriptional promoter regulatory sequence (20-4047) selected/modified from a *Thauera butanivorans* transcription factor BmoR and its associated promoter regulatory sequence (AY093933), a selectable marker-encoding sequence (4048-5433) selected from an *Anabaena* PCC 7120 argininosuccinate lyase (NP_487927), a 432-bp *Nostoc sp.* strain PCC 7120 *gor* terminator (5434-5865), and a PCR RE primer (5866-5885) at the 3' end. Use of this DNA construct (SEQ ID NO: 220) enables enhanced evolution and selection for alcohol-producing designer *Anabaena* cells based on arginine nutrient complementation without requiring any antibiotic resistance gene.

### Creation of Authoxtrophs for Generating Transformants without Antibiotic Selection Marker

According to one of the various embodiments, special nutrient-based authoxtrophs are created by deletion of an essential "nutrient-gene" such as an essential arginine synthesis gene (argininosuccinate lyase (*arg7*)) which is required for making arginine, or ketol-acid reductoisomerase **(54)** and dihydroxy-acid dehydratase **(55)** which are required for making the precursors to synthesize valine, leucine and isoleucine, or a nitrate reductase gene which is essential to utilize the nitrate nutrient in host organisms. The deletion of an essential nutrient-gene can be achieved by putting an antibiotic selectable marker in the place of the essential nutrient-gene or by cutting it out. For example, the authoxtroph created by deletion of ketol-acid reductoisomerase **(54)** and dihydroxy-acid dehydratase **(55)** can grow only in a culture medium that is supplemented with valine, leucine and isoleucine. Similarly, the arginine-authoxtroph can grow only in a culture medium that is supplemented with arginine while the nitrate-authoxtroph which cannot reduce nitrate to make the needed ammonium can grow in an ammonium-supplemented minimal medium but cannot grow in a minimal medium with nitrate as the only nitrogen source. Therefore, the cognate argininosuccinate lyase (*arg7*) gene, nitrate reductase genes (*narG* and *napA*) or ketol-acid reductoisomerase (54) and dihydroxy-acid dehydratase (55) genes can be used as a selection marker based on nutrient-complementation using their corresponding authoxtrophs as host organisms in genetic transformation.

According to one of the various embodiments, when the DNA construct of the designer genes for the biofuel-production pathways are fully optimized and readily for commercial applications, the DNA construct is then fused with the cognate argininosuccinate lyase (arg7) gene or nitrate reductase genes (*narG* and *napA*) as the selection marker. The entire antibiotic selection marker in the authoxtroph host can then be removed through homologous recombination with the designer DNA construct fused with the cognate argininosuccinate lyase (*arg7*) gene or nitrate reductase genes (*narG* and *napA*) as the selectable marker. In this example, when the antibiotic selection marker is removed, the cognate argininosuccinate lyase (arg7) gene or nitrate reductase genes (*narG* and *napA*) is restored by the incorporation of the designer DNA construct into the host authoxtroph. This process results in a new transgenic strain that contains the desirable designer DNA construct of the fully optimized biofuel-production pathways genes without any antibiotic resistance selection marker for improved biosafety.

### Designer Expression of Positively Charged Polypeptides on Cellar Surfaces Enabling Self-flocculation for Enhanced Harvesting of Microbial Cells

Harvesting of microalgae cells from a liquid culture is a significant technical challenge in achieving cost-effect algal biomass harvesting for production of biodiesel and valuable algal products. The technical difficulty largely stems from the fact that microbials such as algae and cyanobacteria are in micrometer sizes and their cell membranes typically contain negatively charges such as the negatively-charged head groups of phospholipids at the two sides of the cytoplasm membrane. The negative surface charges of microbial cells often keep them apart from each other, which present a serious problem in trying to harvest them from a liquid culture. According to one of the various embodiments, as shown in Fig. 20, this technical challenge is overcome by switchably expressing certain designer positively-charged polypeptides such as polylysine on the outside surfaces of the microbial cells to neutralize the cellular surface charges so that the cells can then aggregate together resulting in self-flocculation that can dramatically enhance the harvesting of microbial cells such as algae from liquid culture. The designer positively-charged polypeptides for expression on microbial cell surfaces are selected from the group consisting of polypeptides rich in lysine residuals, polypeptides rich in arginine residues, polypeptides rich in histidine residues, polypeptides rich in lysine and arginine residues, polypeptides rich in lysine and histidine residues, polypeptides rich in lysine and arginine and histidine residues, lipase-fused polylysine, polyamine-lipase-fused polylysine, lipase-fused positively-charged polypeptides, fluorescent protein-lipase-fused polylysine, fluorescent protein-lipase-fused positively-charged polypeptides, and/or combinations thereof.

According to one of the various embodiments, the designer DNA sequence of a positively-charged polypeptide is fused with the gene of a natural cell surface protein with synthetic biology. The wild-type microalgae cells with negatively charged cell surfaces repel each other forming stable suspension. The designer expression of positively charged polypeptides on cellar surfaces is used to destabilize suspension via charge neutralization. The electrostatically destabilized algal cells in the liquid culture medium come together due to Van der Waals forces of attraction. In cases of polyamine-type polypeptides such as polylysine, the long chain molecules facilitate aggregation by the mechanism called bridging, through which the polylysine chains across link with the neighboring cells by electrostatic charge attraction and neutralization

According to one of the various embodiments, the designer positively-charged polypeptide is fused with a designer lipase and/or a green-fluorescent protein for inducible expression on microbial cell surface. For example, when the microbial cell culture such as microalgae liquid culture is grown and ready for biodiesel production, the designer gene encoding for a positively-charged polypeptide fused with lipase and/or green-fluorescent protein is then expressed on the host cell surface. The green-fluorescent protein serves as a reporter that can be readily visualized under a light microscope. The induced expression of designer positively-charged polypeptide on cell surface leads to self-flocculation for enhanced algal biomass harvesting. The lipase expressed on cell surface is perfect for use as a biocatalyst for transesterification of algal lipids and/or other vegetable oils, animal fats, waste cooking oils to produce biodiesel.

SEQ ID NO. 221 presents example 221 of a designer Nial-promoter-controlled cell surface ZYS1-like protein-polyamine-polylysine DNA construct (1479 bp) that includes a PCR FD primer (sequence 1-20), a 2 x 84-bp *Chlamydomonas reinhardtii* Nial (nitrate reductase) promoter (21-188), a 418-bp (189-627) *Chlamydomonas reinhardtii* cell surface ZYS1-like protein (XP_001700395), a 481-bp polyamine-encoding sequence (628-1153) selected/modified from *Streptococcus salivarius* polyamine-type protein (CAJ76166), a 90-bp synthetic polylysine-encoding sequence plus a stop codon (1154-1246), a 223-bp *Chlamydomonas reinhardtii RbcS2* terminator (1247-1459), and a PCR RE primer (1460-1479).

SEQ ID NO. 222 presents example 222 of a designer Nial-promoter-controlled cell surface ZYS1-like protein-polyamine-linked Lipase-polylysine DNA construct (2717 bp) that includes a PCR FD primer (sequence 1-20), a 2 x 84-bp *Chlamydomonas reinhardtii* Nial (nitrate reductase) promoter (21-188), a 598-bp (189-839) *Chlamydomonas reinhardtii* cell surface fasciclin-like protein (XP_001698887), a 478-bp polyamine-encoding sequence (840-1355) selected/modified from *Streptococcus salivarius* polyamine-type protein (CAJ76166), a Lipase sequence (1356-2381) selected/modified from *Candida antarctica* Lipase (CAA83122), a 90-bp synthetic polylysine-encoding sequence plus a stop codon (2382-2474), a 223-bp *Chlamydomonas reinhardtii RbcS2* terminator (2475-2697), and a PCR RE primer (2698-2717).

SEQ ID NO. 223 presents example 223 of a designer Nial-promoter-controlled cell surface ZYS1-like protein-polyamine-lipase-polyamine-polylysine DNA construct (3617 bp) that includes a PCR FD primer (sequence 1-20), a 2 x 84-bp *Chlamydomonas reinhardtii* Nial (nitrate reductase) promoter (21-188), a 598-bp (189-839) *Chlamydomonas reinhardtii* cell surface fasciclin-like protein (XP_001698887), a 478-bp polyamine-encoding sequence (840-1355) selected/modified from *Streptococcus salivarius* polyamine-type protein (CAJ76166), a Lipase sequence (1356-2447) selected/modified from *Burkholderia cepacia* Lipase (M58494), a polyamine sequence (2448-3281) selected/modified from *Mycoplasma bovis* polyamine ABC protein (ADR25157), a 90-bp synthetic polylysine-encoding sequence plus a stop codon (3282-3374), a 223-bp *Chlamydomonas reinhardtii RbcS2* terminator (3375-3597), and a PCR RE primer (3598-3617).

SEQ ID NO. 224 presents example 224 of a designer Nial-promoter-controlled cell surface ZYS1-like protein-lipase-polyamine-polylysine DNA construct (2687 bp) that includes a PCR FD primer (sequence 1-20), a 2 x 84-bp *Chlamydomonas reinhardtii* Nial (nitrate reductase) promoter (21-188), a 601-bp (189-839) *Chlamydomonas reinhardtii* cell surface fasciclin-like protein (XP_001698887), a Lipase sequence (840-1931) selected/modified from *Burkholderia cepacia* Lipase (M58494), a polylysine-type sequence (1932-2351) selected/modified from *Rhodnius prolixus* polylysine protein (AAQ20830), a 90-bp synthetic polylysine-encoding sequence plus a stop codon (2352-2454), a 223-bp *Chlamydomonas reinhardtii RbcS2* terminator (2455-2667), and a PCR RE primer (2668-2687).

Note, the use of SEQ ID NO. 221in in genetic transformation of an eukaryotic photosynthetic organism such as *Chlamydomonas* can create a designer eukaryotic alga such as designer *Chlamydomonas,* wherein the algal cells in their liquid culture can inducibly self-flocculate for enhanced harvesting of algal biomass upon induction of the designer Nial-promoter-controlled cell surface ZYS1-like protein-polyamine-polylysine (encoded by SEQ ID NO: 221). For example, in an ammonium-based minimal medium, this designer *Chlamydomonas* can grow photoautotrophically just like a wild-type organism using air CO₂ as the sole carbon source. When the designer *Chlamydomonas* culture is grown and ready for algal biomass harvesting, the expression of the designer Nial-promoter-controlled cell surface ZYS1-like protein-polyamine-polylysine (encoded by SEQ ID NO: 221) is induced by addition of nitrate into the liquid culture medium (owning to the use of Nial promoter in controlling the designer gene expression). The induced expression of the designer Nial-promoter-controlled cell surface ZYS1-like protein-polyamine-polylysine (encoded by SEQ ID NO: 221) soon leads to self-flocculation that greatly enhance the harvesting of algal biomass from liquid culture.

Similarly, using any of the SEQ ID NOS. 222, 223 or 224 in genetic transformation of an eukaryotic alga such as *Chlamydomonas* can create a designer eukaryotic alga such as designer *Chlamydomonas,* wherein the algal cells in their mass liquid culture can inducibly self-flocculate for enhanced harvesting of algal biomass upon the express of the designer cell surface-linked Lipase-polylysine (encoded by SEQ ID NO: 222, 223 or 224). In this example, a lipase is fused with a cell surface protein and a polylysine for expression on the cell surface together with the fused long polylysine tail as shown in Fig. 20. The lipase enzyme expressed in this manner on the surfaces of algal cells is perfect for use as a catalyst for transesterification of not only algal lipids, but also other vegetable oils, waste cooking oils, and/or animal fats to produce biodiesel as disclosed above. Therefore, the co-expression of lipase-polylysine on algal cell surfaces produces great value for enhanced algal biomass harvesting and catalytic biodiesel production.

Consequently, the use of SEQ ID NOS. 221, 129-133, 151-153, 140 and 141 (or 142) in an eukaryotic alga host organism such as *Chlamydomonas* constitutes a designer transgenic eukaryotic alga such as designer *Chlamydomonas* with a Calvin-cycle 3-phosphogylcerate-branched NADPH-enhanced pathway **(03-05, 34, 35, 53-55, 42,** and **43 (44)** in Figure 6) for photobiological production of isobutanol from carbon dioxide and water, wherein the algal cells in their mass liquid culture can inducibly self-flocculate for enhanced harvesting of algal biomass upon induction of the designer Nial-promoter-controlled cell surface ZYS1-like protein-polyamine (encoded by SEQ ID NO: 221). Similarly, the use of SEQ ID NO: 222 (and/or 223 or 204), 213 and 1-12 in an eukaryotic photosynthetic host organism such as *Chlamydomonas* constitutes another designer eukaryotic alga such as designer *Chlamydomonas* with a glyceraldehydes-3-phosphate-branched butanol-production pathway **(01-12** in Figure 1), wherein the produced butanol is simultaneously and/or subsequently used by the lipase (encoded by SEQ ID NO: 213) in transesterification of triglyceride and/or fatty acids for production of biodiesel. Furthermore, the algal cells in their mass liquid culture can inducibly self-flocculate for enhanced harvesting of algal biomass upon induction of the designer Nial-promoter-controlled cell surface ZYS1-like protein-polyamine-linked Lipase-polylysine (encoded by SEQ ID NO: 222 (and/or 223 or 204)) as illustrated in Fig. 20. The lipase enzyme expressed in this manner on the surfaces of algal cells here is also perfect for use as a catalyst for transesterification of algal lipids, other vegetable oils, waste cooking oils, and/or animal fats to produce biodiesel as disclosed above.

Similarly, the expression of designer Nial-promoter-controlled cell surface-linked polyamine-linked Lipase-polylysine (encoded by SEQ ID NO: 222 (and/or 223 or 204)) in other eukaryotic algae such as *Botryococcus braunii,* or *Scenedesmus obliquus* represents a designer eukaryotic alga such as designer *Botryococcus,* or designer *Scenedesmus,* wherein the algal cells in their mass liquid culture can inducibly self-flocculate for enhanced harvesting of algal biomass upon the express of the designer cell surface-linked Lipase-polylysine (encoded by SEQ ID NO: 222, 223 or 224). Furthermore, the expression of designer Nial-promoter-controlled cell surface-linked polyamine-linked Lipase-polylysine (encoded by SEQ ID NO: 222 (and/or 223 or 204)) in combination of a designer alcohol-biodiesel-production pathway such as the designer methanol-biodiesel-production pathway **(109, 110, 43/44,** and **106** as numerically labeled in Figure 18) in a host eukaryotic alga such as *Botryococcus braunii,* or *Scenedesmus obliquus* represents designer alcohol-biodiesel-production alga with the capability of self-flocculation. According to one of the various embodiments, the eukaryotic algae for use as host organisms in genetic transformation with designer DNA constructs to enable self-flocculation and biodiesel production are selected from the group consisting of: *Chlamydomonas reinhardtii, Platymonas subcordiformis, Chlorella fusca, Chlorella sorokiniana, Chlorella vulgaris, 'Chlorella' ellipsoidea, Chlorella spp., Dunaliella salina, Dunaliella viridis, Dunaliella bardowil, Haematococcus pluvialis; Parachlorella kessleri, Betaphycus gelatinum, Chondrus crispus, Cyanidioschyzon merolae, Cyanidium caldarium, Galdieria sulphuraria, Gelidiella acerosa, Gracilaria changii, Kappaphycus alvarezii, Porphyra miniata, Ostreococcus tauri, Porphyra yezoensis, Porphyridium sp., Palmaria palmata, Gracilaria spp., Isochrysis galbana, Kappaphycus spp., Laminaria japonica, Laminaria spp., Monostroma spp., Nannochloris bacillaris,* Nannochloris sp., *Nannochloropsis oculata, Porphyra spp., Porphyridium spp., Undaria pinnatifida, Ulva lactuca, Ulva spp., Undaria spp., Phaeodactylum Tricornutum, Navicula saprophila, Crypthecodinium cohnii, Cylindrotheca fusiformis, Cyclotella cryptica, Euglena gracilis, Amphidinium sp., Symbiodinium microadriaticum, Macrocystis pyrifera, Ankistrodesmus braunii, Ankistrodesmus convolutus, Ankistrodesmus falcatus, Ankistrodesmus stipitatus, Botryococcus braunii, Pavlova salina, Pavlova lutheri, Scenedesmus vacuolatus, Scenedesmus acutus, Scenedesmus rotundus, Scenedesmus dimorphus, Scenedesmus sp. Ki4, Scenedesmus sp. LU4, Scenedesmus quadricaudus,* and *Scenedesmus obliquus.*

SEQ ID NO: 225 presents example 225 of a designer nirA-promoter-controlled cell-surface-linked Green fluorescent protein-Lipase-polylysine DNA construct (3205 bp) that includes a PCR FD primer (sequence 1-20), a 89-bp *Synechocystis sp.* strain PCC 6803 nitrite-reductase nirA promoter (21-109), a cell surface protein sequence (110-508) selected from a *Synechocystis sp.* PCC 6803 secreted protein MPB70 sequence (BAA17432), a 714-bp (509-1222) Green fluorescent protein sequence (ACY56286); an enzyme-encoding sequence (1223-2398) selected from a *Rhizopus oryzae* Lipase (GenBank: ACW84344) that can use methanol, a polylysine-type sequence (2399-2683) selected from *Trichomonas vaginalis G3* polylysine protein sequence (XP_001291750), a 90-bp synthetic polylysine-encoding sequence plus a stop codon (2684-2776), a 409-bp *Synechocystis* sp. PCC 6803 rbcS terminator (2778-3185), and a PCR RE primer (3186-3205).

Note, the use of SEQ ID NO. 225 in in genetic transformation of a photosynthetic organism such as *Synechocystis sp.* strain PCC 6803 can create a designer cyanobacterium such as designer *Synechocystis,* wherein the cyanobacterial cells in their mass liquid culture can inducibly self-flocculate for enhanced harvesting of their biomass upon induction of the designer nirA-promoter-controlled cell-surface-linked Green fluorescent protein-Lipase-polylysine (encoded by SEQ ID NO: 225). The green fluorescent protein enables convenient visualization of the fused protein expressed on the cell surface under a light microscope. The lipase enzyme expressed in this manner on the surfaces of cyanobacterial cells here is perfect for use as a catalyst for transesterification of not only cyanobacteria lipids, but also for transesterification of other algae oils, vegetable oils, waste cooking oils, and/or animal fats to produce biodiesel as disclosed above. Consequently, the use of SEQ ID NOS. 225, 208 and 199-201 in a microbial host cell such as a cyanobacterium *Synechocystis sp.* strain PCC 6803 constitutes a designer *Synechocystis* with a methanol-biodiesel-production pathway **(109, 110, 43/44** and **106** in Figure 18), wherein the cyanobacterial cells in liquid culture can inducibly self-flocculate for enhanced harvesting of their biomass upon induction of the designer nirA-promoter-controlled cell-surface-linked Green fluorescent protein-Lipase-polylysine (encoded by SEQ ID NO: 225).

SEQ ID NO: 226 presents example 226 of a designer nirA-promoter-controlled cell surface-linked lipase-polylysine DNA construct (2062 bp) that includes a PCR FD primer (sequence 1-20), a 231-bp nirA promoter from *Thermosynechococcus elongatus* BP1 (21-251), a cell surface protein sequence (252-551) selected from the first 300-bp of *Thermosynechococcus elongatus* BP-1 glycosyltransferase (NP_681159), an enzyme-encoding sequence (552-1424) selected/modified from the sequences of a *Thermomyces lanuginosus* lipase (ABV69592) which can use methanol and ethanol, a polylysine-type sequence (1425-1709) selected from *Trichomonas vaginalis G3* polylysine protein sequence (XP_001291750), a positively charged peptide sequence (1710-1829) selected from *Tobacco mosaic virus* Charged protein (NP_597749), a 90-bp synthetic polylysine-encoding sequence plus a stop codon (1830-1922), a 120-bp *rbcS* terminator from BP1 (1923-2042), and a PCR RE primer (2043-2062) at the 3' end.

Note, the use of SEQ ID NO. 226 in in genetic transformation of a photosynthetic organism such *Thermosynechococcus elongatus* can create a designer cyanobacterium such as designer *Thermosynechococcus,* wherein the thermophilic cyanobacterial cells in their liquid culture can inducibly self-flocculate for enhanced harvesting of their biomass upon the expression of the designer nirA-promoter-controlled cell surface-linked lipase-polylysine (encoded by SEQ ID NO: 226). The thermotolerant lipase enzyme expressed in this manner on the surfaces of cyanobacterial cells here is perfect for use as a catalyst for transesterification of cyanobacteria lipids, other algae oils, vegetable oils, waste cooking oils, and/or animal fats to produce biodiesel as disclosed above. Consequently, the use of SEQ ID NOS. 226, 206, 207, 204, and 58-62 in a microbial host cell including (but not limited to) bacterial cells such as a cyanobacterium *Thermosynechococcus elongatus* represents a designer cyanobacterium such as designer *Thermosynechococcus* with a photoautotrophic ethanol-biodiesel-production pathway (Fig. 20), wherein the thermophilic cyanobacterial cells in their mass liquid culture can inducibly self-flocculate for enhanced harvesting of their biomass upon the expression of the designer nirA-promoter-controlled cell surface-linked lipase-polylysine (encoded by SEQ ID NO: 226).

SEQ ID NO: 227 presents example 227 of a designer *Arthrospira platensis* cell surface-linked Lipase-polylysine DNA construct (2053 bp) that includes a PCR FD primer (sequence 1-20), a cell surface protein sequence (21-662) selected from *Arthrospira platensis NIES-39* fasciclin domain protein (YP_005067833), an enzyme-encoding sequence (663-1545) selected/modified from the sequences of a *Thermomyces lanuginosus* lipase (ABV69592) which can use methanol and ethanol, a polylysine-type sequence (1546-1820) selected from *Trichomonas vaginalis G3* polylysine protein sequence (XP_001291750), a positively charged peptide sequence (1821-1940) selected from *Tobacco mosaic virus* Charged protein (NP_597749), a 90-bp synthetic polylysine-encoding sequence plus a stop codon (1941-2033), and a PCR RE primer (2034-2053) at the 3' end.

SEQ ID NO: 228 presents example 228 of a designer *Arthrospira platensis* cell surface-linked Lipase-GFP-polylysine DNA construct (2971 bp) that includes a PCR FD primer (sequence 1-20), a cell surface protein sequence (21-419) selected from *Arthrospira platensis* beta-Ig-H3/fasciclin (EKD10810), an enzyme-encoding sequence (420-1319) selected/modified from the sequences of an *Enterobacter aerogenes* lipase (KHM31672) which can use methanol, a green-fluorescent protein sequence (1320-2033) selected from GFP sequence (AGT40311), a polylysine-type sequence (2034-2453) selected from *Rhodnius prolixus* polylysine protein sequence (AAQ20830), a polylysine-type sequence (2454-2738) selected from *Trichomonas vaginalis G3* polylysine protein sequence (XP_001291750), a positively charged peptide sequence (2739-2858) selected from *Tobacco mosaic virus* Charged protein (NP_597749), a 90-bp synthetic polylysine-encoding sequence plus a stop codon (2859-2951), and a PCR RE primer (2952-2971) at the 3' end.

Note, the use of SEQ ID NO. 227 in genetic transformation of a photosynthetic organism such *Arthrospira platensis* can create a designer cyanobacterium such as designer *Arthrospira platensis,* wherein the cyanobacterial cells in their mass liquid culture can inducibly self-flocculate for enhanced harvesting of their biomass upon the expression of the designer *Arthrospira platensis* cell surface-linked Lipase-polylysine (encoded by SEQ ID NO: 227). The lipase enzyme expressed in this manner on the surfaces of cyanobacterial cells here is perfect for use as a catalyst for transesterification of not only cyanobacteria lipids, but also other algae oils, vegetable oils, waste cooking oils, and/or animal fats to produce biodiesel as disclosed above. Similarly, the use of SEQ ID NO. 228 in a microbial host cell including (but not limited to) bacterial cells such as a cyanobacterium *Arthrospira platensis* represents a designer cyanobacterium such as designer *Arthrospira,* wherein the cyanobacterial cells in their mass liquid culture can inducibly self-flocculate for enhanced harvesting of their biomass upon the expression of the of a designer *Arthrospira platensis* cell surface-linked Lipase-GFP-polylysine (encoded by SEQ ID NO: 228). SEQ ID NO: 229 presents example 229 of a designer *Phaeodactylum tricornutum* cell surface-linked Lipase-polylysine DNA construct (2524 bp) that includes a PCR FD primer (sequence 1-20), a cell surface protein sequence (21-806) selected from *Phaeodactylum tricornutum* cell surface protein (EEC44540), an enzyme-encoding sequence (807-1706) selected/modified from the sequences of an *Enterobacter aerogenes* lipase (KHM31672) which can use methanol, a polylysine-type sequence (1708-2126) selected from *Rhodnius prolixus* polylysine protein sequence (AAQ20830), a polylysine-type sequence (2127-2411) selected from *Trichomonas vaginalis G3* polylysine protein sequence (XP_001291750), a 90-bp synthetic polylysine-encoding sequence plus a stop codon (2412-2504), and a PCR RE primer (2505-2524) at the 3' end.

Note, the use of SEQ ID NO. 229 in a photosynthetic host organism such as diatom *Phaeodactylum tricornutum* represents a designer diatom such as designer *Phaeodactylum,* wherein the cyanobacterial cells in their mass liquid culture can inducibly self-flocculate for enhanced harvesting of their biomass upon the expression of the designer *Phaeodactylum tricornutum* cell surface-linked Lipase-polylysine (encoded by SEQ ID NO: 229). The lipase enzyme expressed in this manner on the surfaces of diatom cells here is perfect for use as a catalyst for transesterification of not only diatom lipids, but also other algae oils, vegetable oils, waste cooking oils, and/or animal fats to produce biodiesel as disclosed above.

SEQ ID NO: 230 presents example 230 of a designer desert cyanobacterium *Microcoleus vaginatu* cell surface-linked Lipase-polylysine DNA construct (2317 bp) that includes a PCR FD primer (sequence 1-20), a cell surface protein sequence (21-599) selected from a desert cyanobacterium *Microcoleus vaginatu* beta-Ig-H3/fasciclin (EGK87709), an enzyme-encoding sequence (600-1499) selected/modified from the sequences of a *Enterobacter aerogenes* lipase (KHM31672) which can use methanol, a polylysine-type sequence (1500-1919) selected from *Rhodnius prolixus* polylysine protein sequence (AAQ20830), a polylysine-type sequence (1920-2204) selected from *Trichomonas vaginalis G3* polylysine protein sequence (XP_001291750), a 90-bp synthetic polylysine-encoding sequence plus a stop codon (2205-2297), and a PCR RE primer (2298-2317) at the 3' end.

Note, the use of SEQ ID NO. 230 in a photosynthetic host organism such as *Microcoleus vaginatu* represents a designer cyanobacterium such as designer *Microcoleus,* wherein the desert cyanobacterial cells in their mass liquid culture can inducibly self-flocculate for enhanced harvesting of their biomass upon the expression of the designer *Microcoleus vaginatu* cell surface-linked Lipase-polylysine (encoded by SEQ ID NO: 230). The lipase enzyme expressed in this manner on the cell surfaces is perfect for use as a catalyst for transesterification of not only blue-green algae lipids, but also other algae oils, vegetable oils, waste cooking oils, and/or animal fats to produce biodiesel as disclosed above.

SEQ ID NO: 231 presents example 231 of a designer *Methanosarcina barkeri* cell surface-linked Lipase-polylysine DNA construct (4552 bp) that includes a PCR FD primer (sequence 1-20), a cell surface protein sequence (21-2834) selected from *Methanosarcina barkeri* cell surface protein (YP_306783), an enzyme-encoding sequence (2835-3734) selected/modified from the sequences of a *Enterobacter aerogenes* lipase (KHM31672) which can use methanol, a polylysine-type sequence (3735-4154) selected from *Rhodnius prolixus* polylysine protein sequence (AAQ20830), a polylysine-type sequence (4152-4439) selected from *Trichomonas vaginalis G3* polylysine protein sequence (XP_001291750), a 90-bp synthetic polylysine-encoding sequence plus a stop codon (4440-4532), and a PCR RE primer (4533-4552) at the 3' end.

Note, the use of SEQ ID NO. 231 in a methanogen host organism such as *Methanosarcina barkeri* which can grow hydrogenotrophically using H₂ and CO₂ represents a designer hydrogenotrophic bacterium such as designer *Methanosarcina,* wherein the hydrogenotrophic bacterial cells in their mass liquid culture can inducibly self-flocculate for enhanced harvesting of their biomass upon the expression of the designer *Methanosarcina barkeri* cell surface-linked Lipase-polylysine (encoded by SEQ ID NO: 231). Since *Methanosarcina* can grow hydrogenotrophically using H₂ and CO₂ as the energy and carbon sources, the use of SEQ ID NO. 231 (lipase) in combination of formate dehydrogenase (FₐₜₑDH) **109,** formaldehyde dehydrogenase (F_{ald}DH) **110,** and alcohol dehydrogenase (ADH) **43** (or **44)** (encoded by designer genes such as SEQ ID NOS. 199-201 or 202-204) constitutes a designer hydrogenotrophic organism for production of biodiesel from H₂ and CO₂ through the designer methanol-biodiesel-production pathway (Figs. 18 and 19). Furthermore, the lipase enzyme expressed in this manner on the cell surfaces is perfect for use as a catalyst for transesterification of not only hydrogenotrophic bacterial lipids, but also algal oils, vegetable oils, waste cooking oils, and/or animal fats to produce biodiesel as disclosed above.

SEQ ID NO: 232 presents example 232 of a designer *Synechococcus sp.* strain PCC 7942 nirA-promoter-controlled cell surface-linked Lipase-polylysine DNA construct (2533 base pairs (bp)) that includes a PCR FD primer (sequence bp 1-20), a 88-bp *nirA* promoter (21-108) selected from the *Synechococcus sp.* strain PCC 7942 (freshwater cyanobacterium) nitrite-reductase-gene promoter sequence, a cell surface protein sequence (109-507) selected from *Synechococcus elongatus* PCC 7942 Beta-Ig-H3/fasciclin (ABB58124), a lipase enzyme-encoding sequence (508-1407) selected/modified from the sequences of a *Enterobacter aerogenes* lipase (KHM31672) which can use methanol, a polylysine-type sequence (1408-1827) selected from *Rhodnius prolixus* polylysine protein sequence (AAQ20830), a polylysine-type sequence (1828-2112) selected from *Trichomonas vaginalis G3* polylysine protein sequence (XP_001291750), a 90-bp synthetic polylysine-encoding sequence plus a stop codon (2113-2205), a 308-bp *Synechococcus sp.* strain PCC 7942 *rbcS* terminator (2206-2513), and a PCR RE primer (2514-2533) at the 3' end.

Note, the use of SEQ ID NO. 232 in a photosynthetic host organism such as *Synechococcus sp.* strain PCC 7942 represents a designer cyanobacterium such as designer *Synechococcus,* wherein the cyanobacterial cells in their mass liquid culture can inducibly self-flocculate for enhanced harvesting of their biomass upon the expression of the designer *Synechococcus sp.* strain PCC 7942 nirA-promoter-controlled cell surface-linked Lipase-polylysine (encoded by SEQ ID NO: 232). The lipase enzyme expressed in this manner on the cell surfaces is perfect for use as a catalyst for transesterification of not only blue-green algae lipids, but also other algae oils, vegetable oils, waste cooking oils, and/or animal fats to produce biodiesel as disclosed above. Consequently, the use of SEQ ID NO. 232 in combination of SEQ ID NOS: 210 and 95-98 (and/or 99) in a photosynthetic host organism such as *Synechococcus sp.* strain PCC 7942 represents another designer cyanobacterium such as designer *Synechococcus* with the Calvin-cycle 3-phophoglycerate-branched photosynthetic NADPH-enhanced butanol-biodiesel-production pathways as illustrated in Figures 4 and 20, wherein the cyanobacterial cells in their mass liquid culture can inducibly self-flocculate for enhanced harvesting of their biomass upon the expression of the designer *Synechococcus sp.* strain PCC 7942 nirA-promoter-controlled cell surface-linked Lipase-polylysine (encoded by SEQ ID NO: 232).

While the present invention has been illustrated by description of several embodiments and while the illustrative embodiments have been described in considerable detail, it is not the intention of the applicant to restrict or in any way limit the scope of the appended claims to such detail. Additional advantages and modifications will readily appear to those skilled in the art. The invention in its broader aspects is therefore not limited to the specific details, representative apparatus and methods, and illustrative examples shown and described. Accordingly, departures may be made from such details without departing from the spirit or scope of applicant's general inventive concept.

## Claims

1. A method for autotrophic production of alcohols and biodiesel comprising:
introducing a transgenic autotrophic organism into a reactor system, the transgenic autotrophic organism comprising transgenes coding for a set of enzymes to confer a photoautotrophic or a hydrogenotrophic pathway for production of alcohol and biodiesel;
using a photosynthetic or hydrogenotrophic process in the biological reactor to synthesize the alcohol and biodiesel from carbon dioxide and water;
using a product separation process to harvest the synthesized alcohol and biodiesel from the bioreactor; and
harvesting biomass from liquid culture in the bioreactor with self-flocculation wherein the designer transgenic cells in their mass liquid culture inducibly self-flocculate for enhanced harvesting of their biomass upon the expression of the designer cell surface-linked positively charged polypeptides.

2. The method of claim 1, wherein the transgenic autotrophic organism comprises at least one of a transgenic designer plant, plant cell, alga, blue-green alga, cyanobacterium, or bacterial cell selected from the group consisting of blue-green algae (oxyphotobacteria including cyanobacteria and oxychlorobacteria), hydrogenotrophic bacteria, fermentative bacteria, methanogens, aquatic plants, plant cells, green algae, red algae, brown algae, diatoms, marine algae, freshwater algae, salt-tolerant algal strains, cold-tolerant algal strains, heat-tolerant algal strains, antenna-pigment-deficient mutants, butanol-tolerant algal strains, higher-alcohols-tolerant algal strains, butanol-tolerant oxyphotobacteria, butanol-tolerant hydrogenotrophic bacteria and methanogens, higher-alcohols-tolerant oxyphotobacteria, alcohol-tolerant hydrogenotrophic bacteria, alcohol-tolerant fermentative bacteria, biodiesel-tolerant algae, biodiesel-tolerant cyanobacteria, biodiesel-tolerant fermentative bacteria, and biodiesel-tolerant hydrogenotrophic bacteria, alcohol-tolerant and biodiesel-tolerant algae, alcohol-tolerant and biodiesel-tolerant cyanobacteria, alcohol-tolerant and biodiesel-tolerant fermentative bacteria, alcohol-tolerant and biodiesel-tolerant hydrogenotrophic bacteria, and alcohol-tolerant and biodiesel-tolerant methanogens.

3. The method of claim 1 or claim 2, wherein the transgenic autotrophic organism comprises eukaryotic algae, blue-green algae (oxyphotobacteria including cyanobacteria and oxychlorobacteria) and bacteria selected from the group consisting of *Chlamydomonas reinhardtii, Platymonas subcordiformis, Chlorella fusca, Chlorella sorokiniana, Chlorella vulgaris, 'Chlorella'ellipsoidea, Chlorella spp., Dunaliella salina, Dunaliella viridis, Dunaliella bardowil, Haematococcus pluvialis; Parachlorella kessleri, Betaphycus gelatinum, Chondrus crispus, Cyanidioschyzon merolae, Cyanidium caldarium, Galdieria sulphuraria, Gelidiella acerosa, Gracilaria changii, Kappaphycus alvarezii, Porphyra miniata, Ostreococcus tauri, Porphyra yezoensis, Porphyridium sp., Palmaria palmata, Gracilaria spp., Isochrysis galbana, Kappaphycus spp., Laminaria japonica, Laminaria spp., Monostroma spp., Nannochloris bacillaris,* Nannochloris sp., *Nannochloropsis oculata, Porphyra spp., Porphyridium spp., Undaria pinnatifida, Ulva lactuca, Ulva spp., Undaria spp., Phaeodactylum Tricornutum, Navicula saprophila, Crypthecodinium cohnii, Cylindrotheca fusiformis, Cyclotella cryptica, Euglena gracilis, Amphidinium sp., Symbiodinium microadriaticum, Macrocystis pyrifera, Ankistrodesmus braunii, Ankistrodesmus convolutus, Ankistrodesmus falcatus, Ankistrodesmus stipitatus, Pavlova salina, Pavlova lutheri, Botryococcus braunii, Scenedesmus vacuolatus, Scenedesmus acutus, Scenedesmus rotundus, Scenedesmus dimorphus, Scenedesmus sp. Ki4, Scenedesmus sp. LU4, Scenedesmus quadricaudus, Scenedesmus obliquus, Thermosynechococcus elongatus* BP-1, *Nostoc* sp. PCC 7120, *Synechococcus elongatus* PCC 6301, *Syncechococcus* sp. strain PCC 7942, *Syncechococcus* sp. strain PCC 7002, *Syncechocystis* sp. strain PCC 6803, *Prochlorococcus marinus* MED4, *Prochlorococcus marinus* MIT 9313, *Prochlorococcus marinus* NATL1A, *Prochlorococcus* SS120, *Spirulina platensis (Arthrospira platensis), Spirulina pacifica, Lyngbya majuscule, Anabaena* sp., *Synechocystis* sp., *Synechococcus elongates, Synechococcus* (MC-A), *Trichodesmium* sp., *Richelia intracellularis, Synechococcus* WH7803, *Synechococcus* WH8102, *Nostoc punctiforme, Syncechococcus* sp. strain PCC 7943, *Synechocyitis* PCC 6714 phycocyanin-deficient mutant PD-1, *Cyanothece* strain 51142, *Cyanothece* sp. CCY0110, *Oscillatoria limosa, Lyngbya majuscula, Symploca muscorum, Gloeobacter violaceus, Prochloron didemni, Prochlorothrix hollandica, Synechococcus* (MC-A), *Trichodesmium* sp., *Richelia intracellularis, Prochlorococcus marinus, Prochlorococcus* SS120, *Synechococcus* WH8102, *Lyngbya majuscula, Symploca muscorum, Synechococcus bigranulatus,* cryophilic *Oscillatoria* sp., *Phormidium* sp., *Nostoc* sp.-1, *Calothrix parietina,* thermophilic *Synechococcus bigranulatus, Synechococcus lividus,* thermophilic *Mastigocladus laminosus, Chlorogloeopsis fritschii* PCC 6912, *Synechococcus vulcanus, Synechococcus* sp. strain MA4, *Synechococcus* sp. strain MA19, *Methanocella paludicola SANAE, Acinetobacter baumannii ABNIH3, Acinetobacter baumannii ABNIH4, Acinetobacter sp. DR1, Agrobacterium sp. H13-3; Agrobacterium vitis S4, Alcaligenes sp., Allochromatium vinosum DSM 180, Amycolatopsis mediterranei S699, Anoxybacillus flavithermus WK1, Aquifex aeolicus VF5, Archaeoglobus fulgidus DSM 4304, Archaeoglobus veneficus SNP6, Azospirillum sp. B510, Burkholderia cenocepacia HI2424, Caldicellulosiruptor bescii DSM 6725, Carboxydothermus hydrogenoformans, Centipeda periodontii DSM* 2778, *Clostridium autoethanogenum, Clostridium ragsdalei, Clostridium sticklandii DSM 519, Clostridium sticklandii, Corynebacterium glutamicum, Cupriavidus metallidurans CH34, Cupriavidus necator N-1, Desulfobacca acetoxidans DSM 11109, Exiguobacterium sp. AT1b, Ferrimonas balearica DSM 9799, Ferroglobus placidus DSM 10642, Geobacillus kaustophilus HTA426, Helicobacter bilis ATCC 43879, Herbaspirillum seropedicae SmR1, Hydrogenobacter thermophilus TK-6, Hydrogenovibrio marinus, Klebsiella variicola At-22, Methanobacterium sp. SWAN-1, Methanobrevibacter ruminantium M1, Methanocaldococcus fervens AG86, Methanocaldococcus infernus ME, Methanocaldococcus jannaschii, Methanocaldococcus sp. FS406-22, Methanocaldococcus vulcanius M7,Methanococcus aeolicus Nankai-3, Methanococcus maripaludis C6, Methanococcus maripaludis S2, Methanococcus voltae A3,Methanocorpusculum labreanum Z, Methanoculleus marisnigri JR1,Methanohalophilus mahii DSM 5219,Methanolinea tarda NOBI-1, Methanoplanus petrolearius DSM 11571,Methanoplanus petrolearius, Methanopyrus kandleri AV19, Methanoregula boonei 6A8, Methanosaeta harundinacea 6Ac,Methanosalsum zhilinae DSM 4017, Methanosarcina acetivorans C2A,Methanosarcina barkeri str. Fusaro, Methanosarcina mazei Go1, Methanosphaera stadtmanae, Methanospirillum hungatei JF-1, Methanothermobacter marburgensis str. Marburg, Methanothermobacter marburgensis, Methanothermobacter thermautotrophicus, Methanothermococcus okinawensis IH1, Methanothermus fervidus DSM 2088, Methylobacillus flagellates, Methylobacterium organophilum, Methylococcus capsulatus, Methylomicrobium kenyense, Methylomonas methanica MC09, Methylomonas sp. LW13, Methylosinus sp. LW2, Methylosinus trichosporium OB3b, Methylotenera mobilis JLW8, Methylotenera versatilis 301, Methylovorus glucosetrophus SIP3-4, Moorella thermoacetica ATCC 39073, Moorella thermoacetica, Oligotropha carboxidovorans OM5, Paenibacillus terrae HPL-003, Pelotomaculum thermopropionicum SI, Planctomyces brasiliensis DSM 5305, Pyrococcus furiosus DSM 3638, Pyrococcus horikoshii OT3, Pyrococcus yayanosii CH1, Ralstonia eutropha H16, Rubrivivax sp., Selenomonas noxia ATCC 43541, Shewanella baltica BA175, Stenotrophomonas sp. SKA14, Synechococcus sp. JA-2-3B'a(2-13), Synechococcus sp. JA-3-3Ab, Thermococcus gammatolerans EJ3, Thermococcus kodakarensis KOD1, Thermococcus onnurineus NA1, Thermococcus sp. 4557, Thermodesulfatator indicus DSM 15286, Thermofilum pendens Hrk 5, Thermotoga lettingae TMO, Thermotoga petrophila RKU-1, Thiocapsa roseopersicina, Thiomonas intermedia K12, Xanthobacter autotrophicus, Yersinia pestis Antiqua, Thermosynechococcus elongatus, Phaeodactylum tricornutum, Methanosarcina barkeri,* and *Microcoleus vaginatu.*

4. The method of any of claims 1 to 3, wherein said transgenic autotrophic organism comprises a set of designer genes that express one or more designer photoautotrophic methanol-biodiesel production pathways comprising:
• NADPH-dependent glyceraldehyde-3-phosphate dehydrogenase, NAD⁺-dependent glyceraldehyde-3-phosphate dehydrogenase, formate dehydrogenase, formaldehyde dehydrogenase, alcohol dehydrogenase, and lipase; and/or
• NAD-reducing soluble hydrogenase, formate dehydrogenase, formaldehyde dehydrogenase, alcohol dehydrogenase, and lipase.

5. The method of any of claims 1 to 4, wherein said transgenic autotrophic organism comprises a set of designer genes that express a designer photoautotrophic ethanol-biodiesel-production pathway comprising:
• NADPH-dependent glyceraldehyde-3-phosphate dehydrogenase, NAD⁺-dependent glyceraldehyde-3-phosphate dehydrogenase, phosphoglycerate mutase, enolase, pyruvate kinase, pyruvate decarboxylase, alcohol dehydrogenase, and lipase.

6. The method of any of claims 1 to 5, wherein said transgenic autotrophic organism comprises a set of designer genes that express one or more designer photoautotrophic butanol-biodiesel-production pathways comprising:
• NAD⁺-dependent glyceraldehyde-3-phosphate dehydrogenase, phosphoglycerate mutase, enolase, pyruvate kinase, pyruvate-ferredoxin oxidoreductase, acetyl-CoA acetyltransferase, thiolase, 3-hydroxybutyryl-CoA dehydrogenase, crotonase, trans-enoyl-CoA reductase, butyryl-CoA dehydrogenase, butyraldehyde dehydrogenase, aldehyde/alcohol dehydrogenase (AdhE2), butanol dehydrogenase, and lipase; and/or
• NADPH-dependent glyceraldehyde-3-phosphate dehydrogenase, NAD-dependent glyceraldehyde-3-phosphate dehydrogenase, phosphoglycerate mutase, enolase, phosphoenolpyruvate carboxylase, aspartate aminotransferase, aspartokinase, aspartate-semialdehyde dehydrogenase, homoserine dehydrogenase, homoserine kinase, threonine synthase, threonine ammonia-lyase, 2-isopropylmalate synthase, isopropylmalate isomerase, 3-isopropylmalate dehydrogenase, 2-keto acid decarboxylase, NAD-dependent alcohol dehydrogenase, NADPH-dependent alcohol dehydrogenase, butanol dehydrogenase, and lipase.

7. The method of any of claims 1 to 6, wherein said transgenic autotrophic organism comprises a set of designer genes that express a designer photoautotrophic isobutanol-biodiesel-production pathway comprising:
• NADPH-dependent glyceraldehyde-3-phosphate dehydrogenase, NAD-dependent glyceraldehyde-3-phosphate dehydrogenase, phosphoglycerate mutase, enolase, pyruvate kinase, acetolactate synthase, ketol-acid reductoisomerase, dihydroxy-acid dehydratase, 2-keto acid decarboxylase, NAD-dependent alcohol dehydrogenase, NADPH-dependent alcohol dehydrogenase, and lipase.

8. The method of any of claims 1 to 7, wherein said transgenic autotrophic organism comprises a set of designer genes that express a designer photoautotrophic 3-methyl-1-butanol - biodiesel-production pathway comprising:
• NADPH-dependent glyceraldehyde-3-phosphate dehydrogenase, NAD-dependent glyceraldehyde-3-phosphate dehydrogenase, phosphoglycerate mutase, enolase, pyruvate kinase, acetolactate synthase, ketol-acid reductoisomerase, dihydroxy-acid dehydratase, 2-isopropylmalate synthase, 3-isopropylmalate dehydratase, 3-isopropylmalate dehydrogenase, 2-keto acid decarboxylase, NAD-dependent alcohol dehydrogenase, NADPH-dependent alcohol dehydrogenase, 3-methylbutanal reductase, and lipase.

9. The method of claim 1, wherein said transgenic autotrophic organism comprises a set of designer genes that express one or more designer anaerobic hydrogenotrophic 1-butanol-biodiesel-production pathway comprising:
• energy converting hydrogenase, [NiFe]-hydrogenase, Coenzyme F₄₂₀-reducing hydrogenase, soluble hydrogenase, heterodissulfide reductase, formylmethanofuran dehydrogenase, formyl transferase, 10-methenyl-tetrahydromethanopterin cyclohydrolase, 10-methylene-H₄ methanopterin dehydrogenase, 10-methylene-H₄-methanopterin reductase, methyl-H₄-methanopterin: corrinoid iron-sulfur protein methyltransferase, corrinoid iron-sulfur protein, CO dehydrogenase/acetyl-CoA synthase, thiolase, 3-hydroxybutyryl-CoA dehydrogenase, crotonase, butyryl-CoA dehydrogenase, butyaldehyde dehydrogenase, butanol dehydrogenase, alcohol dehydrogenase, and lipase; and/or
• formate dehydrogenase, 10-formyl-H₄ folate synthetase, methenyltetrahydrofolate cyclohydrolase, 10-methylene-H₄ folate dehydrogenase, 10-methylene-H₄ folate reductase, methyl-H₄ folate: corrinoid iron-sulfur protein methyltransferase, corrinoid iron-sulfur protein, CO dehydrogenase/acetyl-CoA synthase, thiolase, 3-hydroxybutyryl-CoA dehydrogenase, crotonase, butyryl-CoA dehydrogenase, butyaldehyde dehydrogenase, butanol dehydrogenase, alcohol dehydrogenase, and lipase.

10. The method of any of claims 1 to 9, wherein said transgenic autotrophic organism comprises a set of designer genes that express a designer autotrophic methanol-production pathway comprising formate dehydrogenase, formaldehyde dehydrogenase, and alcohol dehydrogenase (ADH).

11. The method of any of claims 1 to 10, wherein a biofuel alcohol-sensing responsive transcription regulatory system is used in combination with a selectable marker to enhance the screening for the transgenic cells with increased production of a target biofuel selected from the group consisting of butanol and related higher alcohols.

12. The method of any of claims 1 to 11, wherein:
the transgenic autotrophic organism comprises at least one of a transgenic photosynthetic plant, a transgenic photosynthetic cell, a transgenic alga, a transgenic blue-green alga, a transgenic cyanobacterium, and a transgenic bacterium comprising at least one of a designer photosynthetic pathway and a hydrogenotrophic pathway for autotrophic production of the alcohol; and the alcohol is selected from the group consisting of methanol, ethanol, propanol, 1-butanol, 2-methyl-1-butanol, isobutanol, 3-methyl-1-butanol, 1-hexanol, 1-octanol, 1-pentanol, 1-heptanol, 3-methyl-1-pentanol, 4-methyl-1-hexanol, 5-methyl-1-heptanol, 4-methyl-1-pentanol, 5-methyl-1-hexanol, 6-methyl-1-heptanol and combinations thereof.

13. The method of any of claims 1 to 12, wherein said alcohol is simultaneously and/or subsequently utilized by a lipase in transesterification of triglyceride and fatty acids for production of biodiesel.

14. The method of any of claims 1 to 13, wherein the set of enzymes comprises at least one of the enzymes selected from the group consisting of lipase, formate dehydrogenase (FₐₜₑDH), formaldehyde dehydrogenase (F_{ald}DH), alcohol dehydrogenase (ADH), NADPH-dependent glyceraldehyde-3-phosphate dehydrogenase, NAD-dependent glyceraldehyde-3-phosphate dehydrogenase, phosphoglycerate mutase, enolase, pyruvate kinase, citramalate synthase, 2-methylmalate dehydratase, 3-isopropylmalate dehydratase, 3-isopropylmalate dehydrogenase, 2-isopropylmalate synthase, isopropylmalate isomerase, 3-isopropylmalate dehydrogenase, designer isopropylmalate synthase, designer isopropylmalate isomerase, designer 3-isopropylmalate dehydrogenase, designer 2-keto acid decarboxylase, short-chain alcohol dehydrogenase, hexanol dehydrogenase, designer isopropylmalate synthase, designer isopropylmalate isomerase, designer 3-isopropylmalate dehydrogenase, designer 2-keto acid decarboxylase, and designer short-chain alcohol dehydrogenase.

15. The method of any of claims 1 to 14, wherein said designer transgenic autotrophic organism is made without the use of any antibiotic resistance genes by using nutrient-complementation selection by deletion of an essential nutrient-gene selected from the group consisting of argininosuccinate lyase (*arg7*), nitrate reductase, ketol-acid reductoisomerase and dihydroxy-acid dehydratase.

16. The method of any of claims 1 to 15, wherein the said designer positively-charged polypeptides expressed on transgenic microbial cell surfaces are selected from the group consisting of polypeptides rich in lysine residuals, polypeptides rich in arginine residues, polypeptides rich in histidine residues, polypeptides rich in lysine and arginine residues, polypeptides rich in lysine and histidine residues, polypeptides rich in lysine and arginine and histidine residues, lipase-fused polylysine, polyamine-lipase-fused polylysine, lipase-fused positively-charged polypeptides, fluorescent protein-lipase-fused polylysine, and fluorescent protein-lipase-fused positively-charged polypeptides.

17. The method of any of claims 1 to 16, wherein the transgenic autotrophic organism comprises a biosafety-guarded feature selected from the group consisting of a designer proton-channel gene inducible under pre-determined inducing conditions, a designer cell-division-cycle iRNA gene inducible under pre-determined inducing conditions, a high-CO₂-requiring mutant, and highly thermophilic organism as a host organism for transformation with designer biofuel-production-pathway genes in creating designer cell-division-controllable autotrophic organisms, and combinations thereof; and wherein said transgenic autotrophic organism comprises a set of designer genes exemplified with exemplary designer DNA constructs of SEQ ID NOS. 1-232 shown in the sequence listings for expressing at least one of the proteins selected from the group consisting of: lipase, formate dehydrogenase (FₐₜₑDH), formaldehyde dehydrogenase (F_{ald}DH), alcohol dehydrogenase (ADH), NADPH-dependent glyceraldehyde-3-phosphate dehydrogenase, NAD⁺-dependent glyceraldehyde-3-phosphate dehydrogenase, phosphoglycerate mutase, enolase, pyruvate kinase, pyruvate decarboxylase, alcohol dehydrogenase, enolase, pyruvate kinase, pyruvate-ferredoxin oxidoreductase, acetyl-CoA acetyltransferase, thiolase, 3-hydroxybutyryl-CoA dehydrogenase, crotonase, trans-enoyl-CoA reductase, butyryl-CoA dehydrogenase, butyraldehyde dehydrogenase, aldehyde/alcohol dehydrogenase, butanol dehydrogenase, phosphoenolpyruvate carboxylase, aspartate aminotransferase, aspartokinase, aspartate-semialdehyde dehydrogenase, homoserine dehydrogenase, homoserine kinase, threonine synthase, threonine ammonia-lyase, 2-isopropylmalate synthase, isopropylmalate isomerase, 3-isopropylmalate dehydrogenase, 2-keto acid decarboxylase, NADPH-dependent alcohol dehydrogenase, NADPH-dependent alcohol dehydrogenase, butanol dehydrogenase, acetolactate synthase, ketol-acid reductoisomerase, dihydroxy-acid dehydratase, 2-keto acid decarboxylase, NAD-dependent alcohol dehydrogenase, NADPH-dependent alcohol dehydrogenase, acetolactate synthase, ketol-acid reductoisomerase, dihydroxy-acid dehydratase, 2-isopropylmalate synthase, 3-isopropylmalate dehydratase, 3-isopropylmalate dehydrogenase, 2-keto acid decarboxylase, NAD-dependent alcohol dehydrogenase, NADPH-dependent alcohol dehydrogenase, 3-methylbutanal reductase, oxygen-tolerant soluble hydrogenase (SH), oxygen-tolerant membrane bound hydrogenase (MBH), energy converting hydrogenase (Ech), methyl-H4MPT: coenzyme-M methyltransferase (Mtr), methyl-coenzyme M reductase (Mcr), heterodissulfide reductase (Hdr), [NiFe]-hydrogenase (Mvh), Coenzyme F₄₂₀-reducing hydrogenase (Frh), AₗAₒ-ATP synthase, formate dehydrogenase, 10-formyl-H₄ folate synthetase, methenyltetrahydrofolate cyclohydrolase, 10-methylene-H₄ folate dehydrogenase, 10-methylene-H₄ folate reductase, methyl-H₄ folate: corrinoid iron-sulfur protein methyltransferase, corrinoid iron-sulfur protein, CO dehydrogenase/acetyl-CoA synthase, formylmethanofuran dehydrogenase, formyl transferase, 10-methenyl-tetrahydromethanopterin cyclohydrolase, 10-methylene-H₄ methanopterin dehydrogenase, 10-methylene-H₄-methanopterin reductase, methyl-H₄-methanopterin: corrinoid iron-sulfur protein methyltransferase, corrinoid iron-sulfur protein, CO dehydrogenase/acetyl-CoA synthase, thiolase, 3-hydroxybutyryl-CoA dehydrogenase, crotonase, butyryl-CoA dehydrogenase, butyaldehyde dehydrogenase, butanol dehydrogenase, 2-keto acid decarboxylase, alcohol dehydrogenase, 2-methylbutyraldehyde reductase, 3-methylbutanal reductase, hexanol dehydrogenase, octanol dehydrogenase, short-chain alcohol dehydrogenase, and designer positively-charged polypeptides expressed on transgenic microbial cell surfaces selected from the group consisting of polypeptides rich in lysine residuals, polypeptides rich in arginine residues, polypeptides rich in histidine residues, polypeptides rich in lysine and arginine residues, polypeptides rich in lysine and histidine residues, polypeptides rich in lysine and arginine and histidine residues, lipase-fused polylysine, polyamine-lipase-fused polylysine, lipase-fused positively-charged polypeptides, fluorescent protein-lipase-fused polylysine, and fluorescent protein-lipase-fused positively-charged polypeptides.


SEQUENCE LISTING

<110>  Lee, James Weifu

<120>  DESIGNER PHOTOAUTOTROPHIC AND HYDROGENOTROPHIC PRODUCTION OF
       ALCOHOLS AND BIODIESEL

<130>  JWL-004-PCT-US3

<150>  US 13/997,242
<151>  2011-12-20

<150>  PCT/US11/66090
<151>  2011-12-20

<150>  US 13/075,153
<151>  2011-03-29

<150>  US 12/918,784
<151>  2010-08-20

<150>  PCT/US09/034801
<151>  2009-02-21

<150>  US 61/066,845
<151>  2008-02-23

<150>  US 61/066,835
<151>  2008-02-23

<150>  US 61/426,147
<151>  2010-12-22

<160>  232

<170>  PatentIn version 3.5

<210>  1
<211>  1809
<212>  DNA
<213>  Artificial Sequence

<220>
<223>  Synthetic Construct- Sequence No. 1, Example 1: designer
       Butanol-Dehydrogenase DNA construct (1809 bp)

<400>  1
agaaaatctg gcaccacacc tatatggtag ggtgcgagtg accccgcgcg acttggagct      60

cgatggcccc gggttgtttg gggcgtccgc ctctcgcgct attctgagct ggagaccgag     120

gcgcatgaaa atgcattcgc ttccatagga cgctgcattg tggcttgaag gttcaaggga     180

agggttcaaa cgaccccgcc gtacgaactt ttgtcggggg gcgctcccgg ccccgggctc     240

ttgtgcgcgc attagggctt cgggtcgcaa gcaagacgat acatggccgc cgtcattgcc     300

aagtcctccg tctccgcggc cgtggctcgc ccggcccgct ccagcgtgcg ccccatggcc     360

gcgctgaagc ccgccgtcaa ggctgccccc gtggctgccc cggctcaggc caaccagatg     420

gagaatttta gatttaatgc atatacagag atgctttttg gaaagggaca aatagagaag     480

cttccagagg ttttaaaaag atatggtaaa aatatattac ttgcatatgg tggtggaagt 540

ataaaaaaga atggactcta tgatactatc caaaagctat tgaaagattt taatattgtt 600

gaattaagtg gtattgaacc aaatccaaga attgaaactg taagacgtgg agttgaactt 660

tgcagaaaaa ataaagtaga tgttatttta gctgttggtg gagggagtac aatagactgc 720

tcaaaggtta taggggcagg ttattattat gctggagatg catgggacct tgtaaaaaat 780

ccagctaaaa taggtgaggt tttaccaata gtgacagttt taacaatggc agctactggt 840

tctgaaatga atagaaatgc tgttatttca aagatggata caaatgaaaa gcttggaaca 900

ggatcaccta agatgatccc tcaaacttct attttagatc cagaatattt gtatacattg 960

ccagcaattc aaacagctgc aggttgtgct gatattatgt cacacatatt tgaacaatat 1020

tttaataaaa ctacagatgc ttttgtacaa gataaatttg cggaaggttt gttgcaaact 1080

tgtataaaat attgccctgt tgctttaaag gaaccaaaga attatgaagc tagagcaaat 1140

ataatgtggg ctagttcaat ggctcttaac ggacttttag gaagtgggaa agctggagct 1200

tggacttgtc atccaataga acatgaatta agtgcatttt atgatataac tcatggagta 1260

ggtcttgcaa ttttaactcc aagttggatg agatatatct taagtgatgt aacagttgat 1320

aagtttgtta acgtatggca tttagaacaa aaagaagata aatttgctct tgcaaatgaa 1380

gcaatagatg caacagaaaa attctttaaa gcttgtggta ttccaatgac tttaactgaa 1440

cttggaatag ataaagcaaa ctttgaaaag atggcaaaag ctgcagtaga acatggtgct 1500

ttagaatatg catatgtttc attaaatgcc gaggatgtat ataaaatttt agaaatgtcc 1560

ctttaataaa tggaggcgct cgttgatctg agccttgccc cctgacgaac ggcggtggat 1620

ggaagatact gctctcaagt gctgaagcgg tagcttagct ccccgtttcg tgctgatcag 1680

tctttttcaa cacgtaaaaa gcggaggagt tttgcaattt tgttggttgt aacgatcctc 1740

cgttgatttt ggcctctttc tccatgggcg ggctgggcgt atttgaagcg gttctctctt 1800

ctgccgtta 1809

<210> 2
<211> 2067
<212> DNA
<213> Artificial Sequence

<220>
<223> Synthetic Construct- Sequence No. 2, Example 2: designer
 Butyraldehyde-Dehydrogenase DNA construct (2067 bp)

<400> 2
agaaaatctg gcaccacacc tatatggtag ggtgcgagtg accccgcgcg acttggagct 60

cgatggcccc gggttgtttg gggcgtccgc ctctcgcgct attctgagct ggagaccgag 120

gcgcatgaaa atgcattcgc ttccatagga cgctgcattg tggcttgaag gttcaaggga 180

agggttcaaa cgaccccgcc gtacgaactt ttgtcggggg gcgctcccgg ccccgggctc 240
ttgtgcgcgc attagggctt cgggtcgcaa gcaagacgat acatggccgc cgtcattgcc 300
aagtcctccg tctccgcggc cgtggctcgc ccggcccgct ccagcgtgcg ccccatggcc 360
gcgctgaagc ccgccgtcaa ggctgccccc gtggctgccc cggctcaggc caaccagatg 420
attaaagaca cgctagtttc tataacaaaa gatttaaaat taaaaacaaa tgttgaaaat 480
gccaatctaa agaactacaa ggatgattct tcatgtttcg gagttttcga aaatgttgaa 540
aatgctataa gcaatgccgt acacgcacaa aagatattat cccttcatta tacaaaagaa 600
caaagagaaa aaatcataac tgagataaga aaggccgcat tagaaaataa agagattcta 660
gctacaatga ttcttgaaga aacacatatg ggaagatatg aagataaaat attaaagcat 720
gaattagtag ctaaatacac tcctgggaca gaagatttaa ctactactgc ttggtcagga 780
gataacgggc ttacagttgt agaaatgtct ccatatggcg ttataggtgc aataactcct 840
tctacgaatc caactgaaac tgtaatatgt aatagtatag gcatgatagc tgctggaaat 900
actgtggtat ttaacggaca tccaggcgct aaaaaatgtg ttgcttttgc tgtcgaaatg 960
ataaataaag ctattatttc atgtggtggt cctgagaatt tagtaacaac tataaaaaat 1020
ccaactatgg actctctaga tgcaattatt aagcaccctt caataaaact actttgcgga 1080
actggagggc caggaatggt aaaaaccctc ttaaattctg gtaagaaagc tataggtgct 1140
ggtgctggaa atccaccagt tattgtagat gatactgctg atatagaaaa ggctggtaag 1200
agtatcattg aaggctgttc ttttgataat aatttacctt gtattgcaga aaaagaagta 1260
tttgtttttg agaacgttgc agatgattta atatctaaca tgctaaaaaa taatgctgta 1320
attataaatg aagatcaagt atcaaagtta atagatttag tattacaaaa aaataatgaa 1380
actcaagaat actctataaa taagaaatgg gtcggaaaag atgcaaaatt attcttagat 1440
gaaatagatg ttgagtctcc ttcaagtgtt aaatgcataa tctgcgaagt aagtgcaagg 1500
catccatttg ttatgacaga actcatgatg ccaatattac caattgtaag agttaaagat 1560
atagatgaag ctattgaata tgcaaaaata gcagaacaaa atagaaaaca tagtgcctat 1620
atttattcaa aaaatataga caacctaaat aggtttgaaa gagaaatcga tactactatc 1680
tttgtaaaga atgctaaatc ttttgccggt gttggttatg aagcagaagg ctttacaact 1740
ttcactattg ctggatccac tggtgaagga ataacttctg caagaaattt tacaagacaa 1800
agaagatgtg tactcgccgg ttaataaatg gaggcgctcg ttgatctgag ccttgccccc 1860
tgacgaacgg cggtggatgg aagatactgc tctcaagtgc tgaagcggta gcttagctcc 1920
ccgtttcgtg ctgatcagtc tttttcaaca cgtaaaaagc ggaggagttt tgcaattttg 1980
ttggttgtaa cgatcctccg ttgattttgg cctctttctc catgggcggg ctgggcgtat 2040
ttgaagcggt tctctcttct gccgtta 2067

<210>  3
<211>  1815
<212>  DNA
<213>  Artificial Sequence

<220>
<223>  Synthetic Construct- Sequence No. 3, Example 3: designer
       Butyryl-CoA-Dehydrogenase DNA construct (1815 bp)

<400>  3
agaaaatctg gcaccacacc tatatggtag ggtgcgagtg accccgcgcg acttggagct      60

cgatggcccc gggttgtttg gggcgtccgc ctctcgcgct attctgagct ggagaccgag     120

gcgcatgaaa atgcattcgc ttccatagga cgctgcattg tggcttgaag gttcaaggga     180

agggttcaaa cgaccccgcc gtacgaactt ttgtcggggg gcgctcccgg ccccgggctc     240

ttgtgcgcgc attagggctt cgggtcgcaa gcaagacgat acctcgagca tatggccgcc     300

gtcattgcca agtcctccgt ctccgcggcc gtggctcgcc cggcccgctc cagcgtgcgc     360

cccatggccg cgctgaagcc cgccgtcaag gctgcccccg tggctgcccc ggctcaggcc     420

aaccagatga atttccaatt aactagagaa caacaattag tacaacaaat ggttagagaa     480

ttcgcagtaa atgaagttaa gccaatagct gctgaaatcg acgaaacaga aagattccct     540

atggaaaacg ttgaaaaaat ggctaagctt aaaatgatgg gtatcccatt ttctaaagaa     600

tttggtggag caggcggaga tgttctttca tatataatag ctgtggaaga attatcaaaa     660

gtttgtggta ctacaggagt tattctttca gcgcatacat cattatgtgc atcagtaatt     720

aatgaaaatg gaactaacga acaaagagca aaatatttac ctgatctttg cagcggtaaa     780

aagatcggtg ctttcggatt aactgaacca ggtgctggta cagatgctgc aggacaacaa     840

acaactgctg tattagaagg ggatcattat gtattaaatg gttcaaaaat cttcataaca     900

aatggtggag ttgctgaaac tttcataata tttgctatga cagataagag tcaaggaaca     960

aaaggaattt ctgcattcat agtagaaaag tcattcccag gattctcaat aggaaaatta    1020

gaaaataaga tggggatcag agcatcttca actactgagt tagttatgga aaactgcata    1080

gtaccaaaag aaaacctact tagcaaagaa ggtaagggat ttggtatagc aatgaaaact    1140

cttgatggag gaagaattgg tatagctgct caagctttag gtattgcaga aggagctttt    1200

gaagaagctg ttaactatat gaaagaaaga aaacaatttg gtaaaccatt atcagcattc    1260

caaggattac aatggtatat agctgaaatg gatgttaaaa tccaagctgc taaatactta    1320

gtatacctag ctgcaacaaa gaagcaagct ggtgagcctt actcagtaga tgctgcaaga    1380

gctaaattat ttgctgcaga tgttgcaatg gaagttacaa ctaaagcagt tcaaatcttt    1440

ggtggatatg gttacactaa agaataccca gtagaaagaa tgatgagaga tgctaaaata    1500

tgcgaaatct acgaaggaac ttcagaagtt caaaagatgg ttatcgcagg aagcatttta    1560

agataatcta gataaatgga ggcgctcgtt gatctgagcc ttgccccctg acgaacggcg 1620

gtggatggaa gatactgctc tcaagtgctg aagcggtagc ttagctcccc gtttcgtgct 1680

gatcagtctt tttcaacacg taaaaagcgg aggagttttg caattttgtt ggttgtaacg 1740

atcctccgtt gattttggcc tctttctcca tgggcgggct gggcgtattt gaagcggttc 1800

tctcttctgc cgtta 1815

<210> 4
<211> 1482
<212> DNA
<213> Artificial Sequence

<220>
<223> Synthetic Construct- Sequence No. 4, Example 4: designer
      Crotonase DNA construct (1482 bp)

<400> 4
agaaaatctg gcaccacacc tatatggtag ggtgcgagtg accccgcgcg acttggagct 60

cgatggcccc gggttgtttg gggcgtccgc ctctcgcgct attctgagct ggagaccgag 120

gcgcatgaaa atgcattcgc ttccatagga cgctgcattg tggcttgaag gttcaaggga 180

agggttcaaa cgaccccgcc gtacgaactt ttgtcggggg gcgctcccgg ccccgggctc 240

ttgtgcgcgc attagggctt cgggtcgcaa gcaagacgat acctcgagca tatggccgcc 300

gtcattgcca agtcctccgt ctccgcggcc gtggctcgcc cggcccgctc cagcgtgcgc 360

cccatggccg cgctgaagcc cgccgtcaag gctgcccccg tggctgcccc ggctcaggcc 420

aaccagatgg aattaaaaaa tgttattctt gaaaaagaag ggcatttagc tattgttaca 480

atcaatagac caaaggcatt aaatgcattg aattcagaaa cactaaaaga tttaaatgtt 540

gttttagatg atttagaagc agacaacaat gtgtatgcag ttatagttac tggtgctggt 600

gagaaatctt ttgttgctgg agcagatatt tcagaaatga aagatcttaa tgaagaacaa 660

ggtaaagaat ttggtatttt aggaaataat gtcttcagaa gattagaaaa attggataag 720

ccagttatcg cagctatatc aggatttgct cttggtggtg gatgtgaact tgctatgtca 780

tgtgacataa gaatagcttc agttaaagct aaatttggtc aaccagaagc aggacttgga 840

ataactccag gatttggtgg aactcaaaga ttagcaagaa tagttggacc aggaaaagct 900

aaagaattaa tttatacttg tgaccttata aatgcagaag aagcttatag aataggctta 960

gttaataaag tagttgaatt agaaaaattg atggaagaag caaaagcaat ggctaacaag 1020

attgcagcta atgctccaaa agcagttgca tattgtaaag atgctataga cagaggaatg 1080

caagttgata tagatgcagc tatattaata gaagcagaag actttgggaa gtgctttgca 1140

acagaagatc aaacagaagg aatgactgcg ttcttagaaa gaagagcaga aaagaatttt 1200

caaaataaag gctgctgccc cggctgctgc taatctagat aaatggaggc gctcgttgat 1260

ctgagccttg ccccctgacg aacggcggtg gatggaagat actgctctca agtgctgaag 1320

cggtagctta gctccccgtt tcgtgctgat cagtcttttt caacacgtaa aaagcggagg 1380

agttttgcaa ttttgttggt tgtaacgatc ctccgttgat tttggcctct ttctccatgg 1440

gcgggctggg cgtatttgaa gcggttctct cttctgccgt ta 1482

<210> 5
<211> 1367
<212> DNA
<213> Artificial Sequence

<220>
<223> Synthetic Construct- Sequence No. 5, Example 5: designer
      3-Hydroxybutyryl-CoA-Dehydrogenase DNA construct (1367 bp)

<400> 5
agaaaatctg gcaccacacc atggtagggt gcgagtgacc ccgcgcgact tggaagggtt 60

caaacgaccc cgccgtacga acttttgtcg gggggcgctc ccggctcgag catatggccg 120

ccgtcattgc caagtcctcc gtctccgcgg ccgtggctcg cccggcccgc tccagcgtgc 180

gccccatggc cgcgctgaag cccgccgtca aggctgcccc cgtggctgcc ccggctcagg 240

ccaaccagat gaaaaagatt tttgtacttg gagcaggaac tatgggtgct ggtatcgttc 300

aagcattcgc tcaaaaaggt tgtgaggtaa ttgtaagaga cataaaggaa gaatttgttg 360

acagaggaat agctggaatc actaaaggat tagaaaagca agttgctaaa ggaaaaatgt 420

ctgaagaaga taaagaagct atactttcaa gaatttcagg aacaactgat atgaagttag 480

ctgctgactg tgatttagta gttgaagctg caatcgaaaa catgaaaatt aagaaggaaa 540

tctttgctga gttagatgga atttgtaagc cagaagcgat tttagcttca aacacttcat 600

ctttatcaat tactgaagtt gcttcagcta caaagagacc tgataaagtt atcggaatgc 660

atttctttaa tccagctcca gtaatgaagc ttgttgaaat tattaaagga atagctactt 720

ctcaagaaac ttttgatgct gttaaggaat tatcagttgc tattggaaaa gaaccagtag 780

aagttgcaga agctccagga ttcgttgtaa acggaatctt aatcccaatg attaacgaag 840

cttcattcat ccttcaagaa ggaatagctt cagttgaaga tattgataca gctatgaaat 900

atggtgctaa ccatccaatg ggacctttag ctttaggaga tcttattgga ttagatgttt 960

gcttagctat catggatgtt ttattcactg aaacaggtga taacaagtac agagctagca 1020

gcatattaag aaaatatgtt agagctggat ggcttggaag aaaatcagga aaaggattct 1080

atgattattc taaaggctgc tgccccggct gctgctaatc tagataaatg gaggcgctcg 1140

ttgatctgag ccttgccccc tgacgaacgg cggtggatgg aagatactgc tctcaagtgc 1200

tgaagcggta gcttagctcc ccgtttcgtg ctgatcagtc tttttcaaca cgtaaaaagc 1260

ggaggagttt tgcaattttg ttggttgtaa cgatcctccg ttgattttgg cctctttctc 1320

catgggcggg ctgggcgtat ttgaagcggt tctctcttct gccgtta 1367

<210> 6
<211> 1721
<212> DNA
<213> Artificial Sequence

<220>
<223> Synthetic Construct- Sequence No. 6, Example 6: designer Thiolase
DNA construct (1721 bp)

<400> 6
agaaaatctg gcaccacacc atggtagggt gcgagtgacc ccgcgcgact tggaagggtt 60

caaacgaccc cgccgtacga acttttgtcg gggggcgctc ccggctcgag catatggccg 120

ccgtcattgc caagtcctcc gtctccgcgg ccgtggctcg cccggcccgc tccagcgtgc 180

gccccatggc cgcgctgaag cccgccgtca aggctgcccc cgtggctgcc ccggctcagg 240

ccaaccagat gggcaaagaa agtagtttta gctgtgcatg tcgtacagcc atcggaacaa 300

tgggtggatc tcttagcaca attcctgcag tagatttagg tgctatcgtt atcaaagagg 360

ctcttaaccg cgcaggtgtt aaacctgaag atgttgatca cgtatacatg ggatgcgtta 420

ttcaggcagg acagggacag aacgttgctc gtcaggcttc tatcaaggct ggtcttcctg 480

tagaagtacc tgcagttaca actaacgttg tatgtggttc aggtcttaac tgtgttaacc 540

aggcagctca gatgatcatg gctggagatg ctgatatcgt tgttgccggt ggtatggaaa 600

acatgtcact tgcaccattt gcacttccta atggccgtta cggatatcgt atgatgtggc 660

caagccagag ccagggtggt cttgtagaca ctatggttaa ggatgctctt tgggatgctt 720

tcaatgatta tcatatgatc cagacagcag acaacatctg cacagagtgg ggtcttacac 780

gtgaagagct cgatgagttt gcagctaaga gccagaacaa ggcttgtgca gcaatcgaag 840

ctggcgcatt caaggatgag atcgttcctg tagagatcaa gaagaagaaa gagacagtta 900

tcttcgatac agatgaaggc ccaagacagg gtgttacacc tgaatctctt tcaaagcttc 960

gtcctatcaa caaggatgga ttcgttacag ctggtaacgc ttcaggtatc aacgacggtg 1020

ctgcagcact cgtagttatg tctgaagaga aggctaagga gctcggcgtt aagcctatgg 1080

ctacattcgt agctggagca cttgctggtg ttcgtcctga agttatgggt atcggtcctg 1140

tagcagctac tcagaaggct atgaagaagg ctggtatcga gaacgtatct gagttcgata 1200

tcatcgaggc taacgaagca ttcgcagctc agtctgtagc agttggtaag gatcttggaa 1260

tcgacgtcca caagcagctc aatcctaacg gtggtgctat cgctcttgga cacccagttg 1320

gagcttcagg tgctcgtatc cttgttacac ttcttcacga gatgcagaag aaagacgcta 1380

agaagggtct tgctacactt tgcatcggtg gcggtatggg atgcgctact atcgttgaga 1440

agtacgaagg ctgctgcccc ggctgctgct aatctagata aatggaggcg ctcgttgatc 1500

tgagccttgc cccctgacga acggcggtgg atggaagata ctgctctcaa gtgctgaagc 1560

ggtagcttag ctccccgttt cgtgctgatc agtctttttc aacacgtaaa aagcggagga 1620

gttttgcaat tttgttggtt gtaacgatcc tccgttgatt ttggcctctt tctccatggg 1680

cgggctgggc gtatttgaag cggttctctc ttctgccgtt a 1721

<210> 7
<211> 4211
<212> DNA
<213> Artificial Sequence

<220>
<223> Synthetic Construct- Sequence No. 7, Example 7: designer
      Pyruvate-Ferredoxin-Oxidoreductase DNA construct (4211 bp)

<400> 7
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggct cgagcatatg gccgccgtca ttgccaagtc ctccgtctcc gcggccgtgg 240

ctcgcccggc ccgctccagc gtgcgcccca tggccgcgct gaagcccgcc gtcaaggctg 300

cccccgtggc tgccccggct caggccaacc agatggcgca gaggtgcaag gagcccgtcg 360

acggaacgac agccacgacg cacgtggcct acttcatgag cgacagcgcg ttcatcttcc 420

ccatcacgcc cagctcggtc atgtccgagg tcgcccacga gtggtccatg aacggccgca 480

agaacgcctt cggccagccc acgatggtcc gccagatgca gagcgaggct gggtctgccg 540

gcgccctgca cggcgcgctc agcgagggag cgctggcgac gacgttcacg agcagccagg 600

gcctgctgct catgatcccc aacatgtaca agatcgccgg cgagctcctg ccctgcgtca 660

tgcacatcgc cgcccgcacc gtcgccaccg aggccctctc tatcttcggc gaccacacgg 720

atgtctacgc ggtgaggtcg acggggttcg cgttcctgtg ctccgcgacc gtccaggagt 780

gcatccacat gtccgccgcc gcgcacgccg ccaccctgtc cagcgaggtc ccgttcgccc 840

acttcttcga cggcttccgc acgtcccacg agatccagaa gatcgacttc ccctcggacg 900

ccgacctgct ggcctgcatg aactttgacg acgtccgcag gttccgtggc cgctcgctgt 960

gctgcgagcg cccgctgctg cgcgggacgg cgcagaaccc cgacgtcttc atgcaggcgt 1020

ccgagtcgaa cctggcgacg ctggccaggg tccccgcggc catcgacgag gcgctggctc 1080

gtgtgaacaa ggtgttcggg accaactaca ggacctacga gtactatggc caccccgagg 1140

ccacggacgt gatcgtggcc atgggaagcg gcaccgaagt ggccatctcg actgccaact 1200

tcctcaactc gcgcgacgcg aactcgaggg tcggcgtcgt gagggtgcgg ctgttccggc 1260

cgtttgtgtc ggcggcgttt gtggctgcgc tgcccaagac cgtcaagagg atctgcgttc 1320

tggaccgcgg gagggacggg caggcggccg cggaccccct gcaccaggac gtcctgtcgg 1380

cgctgggtct ggcagcgccc gggagggttc aggtgtgcgt gggaggcgtg tacggtctgt 1440

cgtccaagga cttcaacccc gaccacgtga tcgccgtgta caggaacctc gcgtcggcga 1500

gccccaagaa caggttcagc gtcggcatcg tcgacgacgt gacgcacaac agcctggaca 1560

tgggagagca cgtggacgcg ctgccgcagg ggacgaagca gtgcctgctg tggggcatcg 1620

gcggagacgg gaccatcggg gcgaacaaga cggccatcaa gctgatcgcg gaccacacgg 1680

agctgcacgc gcaggggtac tttgcgtacg acgccaacaa ggccggcggc ctgacagtct 1740

cgcacctgcg gttcggcccg acgcggttcg aggcgccgta cctggtgaac gacagcaact 1800

acgtggcgtg ccacaacttc tcgtacgtgc acaggttcaa cctgctgtcg tcgctgcgca 1860

ccgggggcac gttcgtgctc aactgcccgt gccggaccgt ggaggagctg gacacggcac 1920

tcccggtgcg cctgaggcgc gagatcgcca ggcggcaggc caagttctat gtgatcgacg 1980

cgaccaagat cgccaaggac aacgggatgg gcccgttcat caacatggtc ctccaggccg 2040

tgttcttcta tctgtcccac gtgctcgatg tgaacgaggc agtggcactc ctgaagaaga 2100

gcatccagaa gatgtacgcg cgcaagggcg aggaggttgt caggaagaac gtggcatcgg 2160

tcgacgcgtc gctggatccc aaggcgttgc tgcacatcga gtaccccgca gacaggtggc 2220

ttgcgctggc cgacgagcac gtgccccgca tgggtctgct cactgtcccc gagcgcctgc 2280

agaagttcaa cgccgagctg tacgagccga ccctcgcgta cgatggggag agcatcccgg 2340

tcagcaggtt ccctcgcggc ggcgagacgc cgacgggcac gactcagctg ggcaagcgtg 2400

gcatcgccga gagcgtgccg cactggaacc acgagaagtg cgtgcagtgc aaccagtgct 2460

cgttcgtgtg cccgcacgcc gtcatccggt cgtaccagat cagcgaggag gagatgaaga 2520

acgcccctgc cggcttcgac actcttaagt cgcgcaagcc cgggtatcgt ttccgcatca 2580

acgtcagcgc cctggactgc actggctgca gcgtgtgcgt ggagcagtgc ccagtcaagt 2640

gcctggagat gaagcctctc gagtccgagt tcgagatgca gaaggacgcc atcaggttcg 2700

tccgcgagat ggtcgcgccc aagcccgagc tgggagaccg caagactccc gtcggcatcg 2760

cgtctcacac gccgctgttc gagttcccgg gagcctgcgc cgggtgcggt gagaccccgc 2820

tggtgcgcct cgtgacgcag atgttcggtg agcgcatggt catcgccgcg gccactgggt 2880

gcaactcgat ctggggagcg tcgttcccga acgtgccgta cacaaccaac gcccgcgggg 2940

agggccccgc gtggcacaac tcgctgttcg aggacgcggc ggagctcggg tatggcatta 3000

cgtgtgcgta tcgccagcgc cgcgagcgcc tcatcggcat cgtgcggagc gtcgtcgacg 3060

atgcgggatc cgtgcagggt ctgtctgctg agctgaaggc tctgctggtc gagtggctcg 3120

cgcacgtcag ggacttcgag aagacccgcg agctccgcga caggatgaac cccctgatcg 3180

acgcaatccc agcgaacgcg gactgcaggg ttctggagct cagggagaag cacaaccgcg 3240

agctgatcgc gcgcacgagt ttctggatcc tcggtggcga cgggtgggcg tacgacatcg 3300

gcttcggtgg actggaccac gtgatcgcca acaacgagga cgtcaacatc cttgttctcg 3360

acacggaggt ctactccaac actggtggcc agcgctccaa gtcgacgccg ctcggcgccc 3420

gcgccaagta cgctgtgctg ggcaaggaca ctgggaagaa ggacctgggg cgcatcgcga 3480

tgacctacga gaccgcgtac gtggccagca tcgcgcaggg agccaaccag cagcagtgca 3540

tggacgcgct gagggaggcc gaggcctacc agggcccctc gatcgtcatt gcgtacactc 3600

cgtgcatgga gcaccagatg gtccgcggga tgaaggagag ccagaagaac cagaagctgg 3660

ctgtggagac gggctactgg ctgctgtacc gcttcaaccc cgacctcatc cacgagggca 3720

agaacccctt caccctcgac tcgaagcctc cctcgaagcc tcccaaggag ttcctggaca 3780

cgcagggccg tttcattact ctgcagcgcg agcaccccga gcaggcccac ctccttcacg 3840

aggcactcac ccgctctctg gccacccgct tcgtgcgcta ccagcgcctc gtgcagctgt 3900

acgagcccgc tgcccctgcc gcagctcctg ccacgcatgg ctgctgcccc ggctgctgct 3960

aatctagata aatggaggcg ctcgttgatc tgagccttgc cccctgacga acggcggtgg 4020

atggaagata ctgctctcaa gtgctgaagc ggtagcttag ctccccgttt cgtgctgatc 4080

agtctttttc aacacgtaaa aagcggagga gttttgcaat tttgttggtt gtaacgatcc 4140

tccgttgatt ttggcctctt tctccatggg cgggctgggc gtatttgaag cggttctctc 4200

ttctgccgtt a 4211

<210> 8
<211> 2021
<212> DNA
<213> Artificial Sequence

<220>
<223> Synthetic Construct- Sequence No. 8, Example 8: designer
      Pyruvate-Kinase DNA construct (2021 bp)

<400> 8
agaaaatctg gcaccacacc atggtagggt gcgagtgacc ccgcgcgact tggaagggtt 60

caaacgaccc cgccgtacga acttttgtcg gggggcgctc ccggctcgag catatggccg 120

ccgtcattgc caagtcctcc gtctccgcgg ccgtggctcg cccggcccgc tccagcgtgc 180

gccccatggc cgcgctgaag cccgccgtca aggctgcccc cgtggctgcc ccggctcagg 240

ccaaccagat gtctagatta gaaagattga cctcattaaa cgttgttgct ggttctgact 300

tgagaagaac ctccatcatt ggtaccatcg gtccaaagac caacaaccca gaaaccttgg 360

ttgctttgag aaaggctggt ttgaacattg tccgtatgaa cttctctcac ggttcttacg 420

aataccacaa gtctgtcatt gacaacgcca gaaagtccga agaattgtac ccaggtagac 480

cattggccat tgctttggac accaagggtc cagaaatcag aactggtacc accaccaacg 540

atgttgacta cccaatccca ccaaaccacg aaatgatctt caccaccgat gacaagtacg 600

ctaaggcttg tgacgacaag atcatgtacg ttgactacaa gaacatcacc aaggtcatct 660

ccgctggtag aatcatctac gttgatgatg gtgttttgtc tttccaagtt ttggaagtcg 720

ttgacgacaa gactttgaag gtcaaggctt tgaacgccgg taagatctgt tcccacaagg 780

gtgtcaactt accaggtacc gatgtcgatt tgccagcttt gtctgaaaag gacaaggaag 840

atttgagatt cggtgtcaag aacggtgtcc acatggtctt cgcttctttc atcagaaccg 900

ccaacgatgt tttgaccatc agagaagtct tgggtgaaca aggtaaggac gtcaagatca 960

ttgtcaagat tgaaaaccaa caaggtgtta acaacttcga cgaaatcttg aaggtcactg 1020

acggtgttat ggttgccaga ggtgacttgg gtattgaaat cccagcccca gaagtcttgg 1080

ctgtccaaaa gaaattgatt gctaagtcta acttggctgg taagccagtt atctgtgcta 1140

cccaaatgtt ggaatccatg acttacaacc caagaccaac cagagctgaa gtttccgatg 1200

tcggtaacgc tatcttggat ggtgctgact gtgttatgtt gtctggtgaa accgccaagg 1260

gtaactaccc aatcaacgcc gttaccacta tggctgaaac cgctgtcatt gctgaacaag 1320

ctatcgctta cttgccaaac tacgatgaca tgagaaactg tactccaaag ccaacctcca 1380

ccaccgaaac cgtcgctgcc tccgctgtcg ctgctgtttt cgaacaaaag gccaaggcta 1440

tcattgtctt gtccacttcc ggtaccaccc caagattggt ttccaagtac agaccaaact 1500

gtccaatcat cttggttacc agatgcccaa gagctgctag attctctcac ttgtacagag 1560

gtgtcttccc attcgttttc gaaaaggaac ctgtctctga ctggactgat gatgttgaag 1620

cccgtatcaa cttcggtatt gaaaaggcta aggaattcgg tatcttgaag aagggtgaca 1680

cttacgtttc catccaaggt ttcaaggccg gtgctggtca ctccaacact ttgcaagtct 1740

ctaccgttgg ctgctgcccc ggctgctgct aatctagata aatggaggcg ctcgttgatc 1800

tgagccttgc cccctgacga acggcggtgg atggaagata ctgctctcaa gtgctgaagc 1860

ggtagcttag ctccccgttt cgtgctgatc agtctttttc aacacgtaaa aagcggagga 1920

gttttgcaat tttgttggtt gtaacgatcc tccgttgatt ttggcctctt tctccatggg 1980

cgggctgggc gtatttgaag cggttctctc ttctgccgtt a 2021

<210> 9
<211> 1815
<212> DNA
<213> Artificial Sequence

<220>
<223> Synthetic Construct- Sequence No. 9, Example 9: designer Enolase
DNA construct (1815 bp)

<400> 9

agaaaatctg gcaccacacc tatatggtag ggtgcgagtg accccgcgcg acttggagct 60

cgatggcccc gggttgtttg gggcgtccgc ctctcgcgct attctgagct ggagaccgag 120

gcgcatgaaa atgcattcgc ttccatagga cgctgcattg tggcttgaag gttcaaggga 180

agggttcaaa cgaccccgcc gtacgaactt ttgtcggggg gcgctcccgg ccccgggctc 240

ttgtgcgcgc attagggctt cgggtcgcaa gcaagacgat acctcgagca tatggccgcc 300

gtcattgcca agtcctccgt ctccgcggcc gtggctcgcc cggcccgctc cagcgtgcgc 360

cccatggccg cgctgaagcc cgccgtcaag gctgcccccg tggctgcccc ggctcaggcc 420

aaccaggtga ccaaggctgt tgagaacatc aacgctatta ttgcccccgc cctgaagggc 480

atggaccccg tcaagcaggc ggagattgac cagaagatga aggacctgga cggcactgac 540

aacaagggca agctgggtgc caacgccatc ctggccgtct ccatggccgt gtgcaaggcc 600

ggtgccgctg agaagggcgt gcccctgtac aagcacattg cggacctggc cggcaacagc 660

aagctgatcc tgcccgtgcc ctcgttcaac atcatcaacg gcggcagcca cgccggcaac 720

gccctggcta tgcaggagtt catgatcctg cccgttggcg cctcgagctt ctctgaggcc 780

atgcgcatgg gctgcgaggt gtaccacgcc ctgaagggcc tgatcaaggc caagtacggc 840

caggacgcct gcaacgtggg tgatgagggt ggcttcgccc ccaacatcgg ctccaacgat 900

gagggcctga acttggtgaa cgaggccatc gagaaggccg gctacaccgg caaggtgaag 960

atcggcatgg acgtggcctc gtcggagttc tacaccgagg acggcatgta cgacctggac 1020

ttcaagaacc agcccaacga tggctcgcag aagaagacca aggagcagat gctggagctg 1080

tacaacgagt tctgcaagaa gtacccggtc atctccatcg aggacccctt cgagcaggac 1140

gactgggagc cctgcgccaa gctgaccacc gagaacatct gccaggtggt cggcgacgac 1200

atcctggtga ccaaccccgt gcgcgtgaag aaggccatcg acgccaaggc cgtcaacgct 1260

ctgctgctca aggtcaacca gatcggtacc attaccgagt ccattgaggc cgtgcgcatg 1320

gccaaggagg ccggctgggg tgtcatgacc agccaccgct cgggtgagac tgaggactct 1380

ttcatcgccg acctggcggt gggcctggcc tccggccaga tcaagaccgg cgccccctgc 1440

cgctcggagc gcaatgccaa gtacaaccag ctgctgcgca tcgaggagga gctgggcgag 1500

aacgctgtgt acgctggcga gagctggcgc cacatcggct ggggctgctg ccccggctgc 1560

tgctaatcta gataaatgga ggcgctcgtt gatctgagcc ttgccccctg acgaacggcg 1620

gtggatggaa gatactgctc tcaagtgctg aagcggtagc ttagctcccc gtttcgtgct 1680

gatcagtctt tttcaacacg taaaaagcgg aggagttttg caattttgtt ggttgtaacg 1740

atcctccgtt gattttggcc tctttctcca tgggcgggct gggcgtattt gaagcggttc 1800

tctcttctgc cgtta 1815

<210> 10
<211> 2349
<212> DNA
<213> Artificial Sequence

<220>
<223> Synthetic Construct- Sequence No. 10, Example 10: designer
      Phosphoglycerate-Mutase DNA construct (2349 bp)

<400> 10
agaaaatctg gcaccacacc tatatggtag ggtgcgagtg accccgcgcg acttggagct      60

cgatggcccc gggttgtttg gggcgtccgc ctctcgcgct attctgagct ggagaccgag     120

gcgcatgaaa atgcattcgc ttccatagga cgctgcattg tggcttgaag gttcaaggga     180

agggttcaaa cgaccccgcc gtacgaactt ttgtcggggg gcgctcccgg ccccgggctc     240

ttgtgcgcgc attagggctt cgggtcgcaa gcaagacgat acctcgagca tatggccgcc     300

gtcattgcca agtcctccgt ctccgcggcc gtggctcgcc cggcccgctc cagcgtgcgc     360

cccatggccg cgctgaagcc cgccgtcaag gctgcccccg tggctgcccc ggctcaggcc     420

aaccagatgg cgcacgacta caagctgaag gcccacccgg cgattcctgc gcccgagggc     480

ccgctgctgg tctgcattct ggacggcttc ggcgagaacg agtacaagga tgagttcaac     540

gccgtgcacg tggctaagac gcccactgtg gacgcgctgc gcgctgtgcc ccatcgcttc     600

cgttccatca aggcgcacgg aaaggctgtg ggcctgccca gcgatgccga catgggcaac     660

agcgaggtgg ggcacaacgc cctgggctcg ggccaggtgg tggaccaagg cgcgcgcctg     720

gtggacctgg cgctggagac cggccgtatg ttctcggacc ccggctggaa gctcatcagc     780

gaggccttcc cctcccacac cgtccacttc atcggcctgc tgtccgacgg cggcgtgcac     840

tcgcgcgccg atcagctgca cggctgcctg cgcggcgccg tggagcgcgg cgccaagcgc     900

gtgcgcgtgc acatcctgac tgacggccgc gacgtgccgg acggcagcag catccggttc     960

gtggaggagc tggaggcggt gctggcggag ctgcgcggca agggctgcga catcgccatc    1020

gcctcgggcg gcggccgcat gcaggtcacc atggaccgct acgaggcgga ctggagcatg    1080

gtgaagcgcg gctgggacgc gcacgtgctg ggcaaggcgc cccactactt caaggacgcc    1140

aagaccgcgg tcaccaccct gcgcggctcc gaggacgcgc cggtgtctga ccagtacgtg    1200

gccccctttg tgattgtgga cgaggcggac aagccggtgg gcaccattga ggacggcgac    1260

gcggtggtgc tgttcaactt ccgcgcggac cgcatggtgg agatcagcaa ggccttcgag    1320

tacgaggacg gcttcaccgc ctttgagcgc gagcgcttcc ccaagggcct gcgcttcgtg    1380

ggcatgatgc agtacgacgg cgacctgaag ctgcccgcca acttcctggt gccgccgccc    1440

ctgattgagc acgtgtcggg cgagtacctg tgcaagaacg ggctgagcac cttcgcctgc    1500

tccgagactc agaagttcgg gcacgtgacg ttcttctgga acggcaaccg ctccggctac    1560

ctggacgcca agcaggagca gtacctggag atcccgtcgg acaagatcga gttcaacaag    1620

gctccggaca tgaaggcgcg cgagatcacc gccgccggca ttgaggcgct caagagcggc 1680

aagtacaagg tggtgcgcat caactacgcc aacccggaca tggtcggcca caccggcgac 1740

atggctgcca ccgtccgcgc ctgcgagacc gtggacgggt gcgtgaagga gctgctggag 1800

gtggtggaca gcctgaacgg ccgctggatc gtcacgtccg accacggcaa cgccgacgac 1860

atggtgcagc gcgacaagaa gggcaagccc ctgctgggcg aggacggcaa gccgctgccc 1920

ctgaccagcc acacgctggc gcccgtgccg ttcttcatcg gcggcaaggg cctgccggac 1980

ggcgtggtgc tgcgcgacga cctgccggac gccgggctgg ccaacgtggc cgccaccacc 2040

ttcaacctgc tgggcttcga ggcgcccggc atctacaagc ccagcatggt caaggcgtaa 2100

tctagataaa tggaggcgct cgttgatctg agccttgccc cctgacgaac ggcggtggat 2160

ggaagatact gctctcaagt gctgaagcgg tagcttagct ccccgtttcg tgctgatcag 2220

tctttttcaa cacgtaaaaa gcggaggagt tttgcaattt tgttggttgt aacgatcctc 2280

cgttgatttt ggcctctttc tccatgggcg ggctgggcgt atttgaagcg gttctctctt 2340

ctgccgtta 2349

<210> 11
<211> 1908
<212> DNA
<213> Artificial Sequence

<220>
<223> Synthetic Construct- Sequence No. 11, Example 11: designer
      Phosphoglycerate-Kinase DNA construct (1908 bp)

<400> 11
agaaaatctg gcaccacacc tatatggtag ggtgcgagtg accccgcgcg acttggagct 60

cgatggcccc gggttgtttg gggcgtccgc ctctcgcgct attctgagct ggagaccgag 120

gcgcatgaaa atgcattcgc ttccatagga cgctgcattg tggcttgaag gttcaaggga 180

agggttcaaa cgaccccgcc gtacgaactt ttgtcggggg gcgctcccgg ccccgggctc 240

ttgtgcgcgc attagggctt cgggtcgcaa gcaagacgat acatggccct ctctatgaag 300

atgcgcgcca acgcgcgcgt gtccggtcgc cgcgtcgccg ctgtggcccc ccgcgtggtg 360

cccttctcgt cggcctccag ctccgtgctg cgctctggct tcgcgctgag gtgtctgtgg 420

acatccgccg cgtgggccgc tctcgcatcc gtcgtcgagg cggtgaagaa gtcggttggc 480

gacctgcaca aggctgacct ggagggcaag cgcgtgttcg tccgcgcgga cctgaacgtg 540

cctcttgaca aggccaccct ggccatcacc gacgacaccc gcattcgcgc ggccgtcccc 600

accctgaagt acctgctgga caacggtgct aaggtcctgc tgacctcgca cctgggtcgc 660

ccgaagggcg gtcccgagga caagtaccgc ctgacccccg tggtggcccg cctgtcggag 720

ctgctgggca agcccgtgac caaggtcgat gactgcatcg gccccgaggt ggagaaggcg 780

gtgggcgcca tgaagaacgg cgagctgctg ctgctggaga actgccgctt ctacaaggag 840

gaggagaaga acgagcccga gttcgccaag aagctggccg ccaacgccga cctgtacgtg 900

aacgacgcgt tcggcactgc ccaccgcgcc cacgcctcca ccgagggtgt gaccaagttc 960

ctgaagccct ccgtggccgg cttcctgctg cagaaggagc tggactacct tgatggcgcc 1020

gtgtccaacc ccaagcgccc cttcgtggcc attgtgggcg gctccaaggt gtcctccaag 1080

atcaccgtca ttgaggcgct gatggagaag tgcgacaaga tcatcatcgg cggtggcatg 1140

atcttcacct tctacaaggc ccgcgcgctg aaggtgggct cctcgctggt tgaggacgac 1200

aagatcgagc tggccaagaa gctggaggag atggccaagg ccaagggtgt gcagctgctg 1260

ctgcccaccg acgtggtggt ggccgacaag ttcgacgcca acgccaacac ccagaccgtg 1320

cccatcaccg ccatccccga tggctggatg ggtctggaca ttggcccgga ctccgtcaag 1380

accttcaacg acgccctggc cgacgccaag accgttgtgt ggaacggccc catgggtgtg 1440

ttcgagtttc cccaagttcg ccaacgcacc gtgtcgatcg ccaacaccct ggccggcctg 1500

acgcccaagg gctgcatcac catcattggt ggcggtgact ccgtggctgc cgtcgagcag 1560

gccggcgttg ccgagaagat gagccacatc tccaccggcg gcggtgcctc cctggagctg 1620

ctggagggca aggtcctgcc cggcgtggcc gccctggacg agaagtaaat ggaggcgctc 1680

gttgatctga gccttgcccc ctgacgaacg gcggtggatg gaagatactg ctctcaagtg 1740

ctgaagcggt agcttagctc cccgtttcgt gctgatcagt ctttttcaac acgtaaaaag 1800

cggaggagtt ttgcaatttt gttggttgta acgatcctcc gttgattttg gcctctttct 1860

ccatgggcgg gctgggcgta tttgaagcgg ttctctcttc tgccgtta 1908

<210> 12
<211> 1677
<212> DNA
<213> Artificial Sequence

<220>
<223> Synthetic Construct- Sequence No. 12, Example 12: designer
NAD-dependent Glyceraldehyde-3-Phosphate-Dehydrogenase DNA
construct (1677 bp)

<400> 12
agaaaatctg gcaccacacc tatatggtag ggtgcgagtg accccgcgcg acttggagct 60

cgatggcccc gggttgtttg gggcgtccgc ctctcgcgct attctgagct ggagaccgag 120

gcgcatgaaa atgcattcgc ttccatagga cgctgcattg tggcttgaag gttcaaggga 180

agggttcaaa cgaccccgcc gtacgaactt ttgtcggggg gcgctcccgg ccccgggctc 240

ttgtgcgcgc attagggctt cgggtcgcaa gcaagacgat acatggccgc cgtcattgcc 300

aagtcctccg tctccgcggc cgtggctcgc ccggcccgct ccagcgtgcg ccccatggcc 360

gcgctgaagc ccgccgtcaa ggctgccccc gtggctgccc cggctcaggc caaccagatg 420

gctcccatca agatcggcat caatggtttt ggtcgtattg gccgcctcgt gtggcgtgcc 480

actcttaacc gtgacgatgt cgaggtcgtc gccatcaatg atccattcat tgatgtgcca 540

tacatggtct acatggccaa gtatgactcg gtccacggca acctgaccca cgacgttcag 600

caaggcgacg gcaagctgat ggtcaatggc aagtcaatca ccatcttcgg caagatggat 660

gccaaggaga tcccatggaa ggaggccggc gcgaccttcg tcgttgagtc gactggtgtg 720

ttcaccaccc tggagggcgc cagctctcac ctggtcggcg gtgctgagac cgtcgtcatc 780

tccgccccat caaacgatgc ccccatgttc gtcatgggtg tcaacgagga gggctacaag 840

ccagacatga aagtggtgtc caacgcgtct tgcaccacca actgcctggg ccccctggcc 900

aaggtcatcc accttaagtt cggcatcctg gagggcctga tgaccaccgt ccacgcgacc 960

accgccaccc agaagaccgt cgacgggccg tccaagaagg actggcgcgg cgggcgcggc 1020

atcctggaca acatcatccc ctcggcgact ggtgccgcca aggccgtcgg caaggtgctg 1080

cctgccctga acggcaagct caccggcatg gccttccgcg tgcccacccc cgatgtctcg 1140

gtcgtcgatc tgaccgtgcg cctggagaag ggtgcgtcgt acgacgccat caaggccgag 1200

atcaagcgcg cgagcgagaa cgagctcaag ggcatcctgg cctacaccga ggatgccgtg 1260

gtctccaccg acttcatcgg caacaagcac agctccatct tcgacgccga ggccggcatc 1320

gccctcaacg acaactttgt caagctggtc tcctggtacg acaacgagtg gggctactcc 1380

aaccgtgtcg tcgacctgat cgcgcacatg gccaaggtca aggccgccag ccactaaatg 1440

gaggcgctcg ttgatctgag ccttgccccc tgacgaacgg cggtggatgg aagatactgc 1500

tctcaagtgc tgaagcggta gcttagctcc ccgtttcgtg ctgatcagtc tttttcaaca 1560

cgtaaaaagc ggaggagttt tgcaattttg ttggttgtaa cgatcctccg ttgattttgg 1620

cctctttctc catgggcggg ctgggcgtat ttgaagcggt tctctcttct gccgtta 1677

<210> 13
<211> 2351
<212> DNA
<213> Artificial Sequence

<220>
<223> Synthetic Construct- Sequence No. 13, Example 13: designer
      HydA1-promoter-linked Phosphoglycerate-Mutase DNA construct (2351
      bp)

<400> 13
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg gcgcacgact acaagctgaa ggcccacccg gcgattcctg 480

cgcccgaggg cccgctgctg gtctgcattc tggacggctt cggcgagaac gagtacaagg 540

atgagttcaa cgccgtgcac gtggctaaga cgcccactgt ggacgcgctg cgcgctgtgc 600

cccatcgctt ccgttccatc aaggcgcacg gaaaggctgt gggcctgccc agcgatgccg 660

acatgggcaa cagcgaggtg gggcacaacg ccctgggctc gggccaggtg gtggaccaag 720

gcgcgcgcct ggtggacctg gcgctggaga ccggccgtat gttctcggac cccggctgga 780

agctcatcag cgaggccttc ccctcccaca ccgtccactt catcggcctg ctgtccgacg 840

gcggcgtgca ctcgcgcgcc gatcagctgc acggctgcct gcgcggcgcc gtggagcgcg 900

gcgccaagcg cgtgcgcgtg cacatcctga ctgacggccg cgacgtgccg gacggcagca 960

gcatccggtt cgtggaggag ctggaggcgg tgctggcgga gctgcgcggc aagggctgcg 1020

acatcgccat cgcctcgggc ggcggccgca tgcaggtcac catggaccgc tacgaggcgg 1080

actggagcat ggtgaagcgc ggctgggacg cgcacgtgct gggcaaggcg ccccactact 1140

tcaaggacgc caagaccgcg gtcaccaccc tgcgcggctc cgaggacgcg ccggtgtctg 1200

accagtacgt ggcccccttt gtgattgtgg acgaggcgga caagccggtg ggcaccattg 1260

aggacggcga cgcggtggtg ctgttcaact tccgcgcgga ccgcatggtg gagatcagca 1320

aggccttcga gtacgaggac ggcttcaccg cctttgagcg cgagcgcttc cccaagggcc 1380

tgcgcttcgt gggcatgatg cagtacgacg gcgacctgaa gctgcccgcc aacttcctgg 1440

tgccgccgcc cctgattgag cacgtgtcgg gcgagtacct gtgcaagaac gggctgagca 1500

ccttcgcctg ctccgagact cagaagttcg ggcacgtgac gttcttctgg aacggcaacc 1560

gctccggcta cctggacgcc aagcaggagc agtacctgga gatcccgtcg gacaagatcg 1620

agttcaacaa ggctccggac atgaaggcgc gcgagatcac cgccgccggc attgaggcgc 1680

tcaagagcgg caagtacaag gtggtgcgca tcaactacgc caacccggac atggtcggcc 1740

acaccggcga catggctgcc accgtccgcg cctgcgagac cgtggacggg tgcgtgaagg 1800

agctgctgga ggtggtggac agcctgaacg gccgctggat cgtcacgtcc gaccacggca 1860

acgccgacga catggtgcag cgcgacaaga agggcaagcc cctgctgggc gaggacggca 1920

agccgctgcc cctgaccagc cacacgctgg cgcccgtgcc gttcttcatc ggcggcaagg 1980

gcctgccgga cggcgtggtg ctgcgcgacg acctgccgga cgccgggctg gccaacgtgg 2040

ccgccaccac cttcaacctg ctgggcttcg aggcgcccgg catctacaag cccagcatgg 2100

tcaaggcgta aatggaggcg ctcgttgatc tgagccttgc cccctgacga acggcggtgg 2160

atggaagata ctgctctcaa gtgctgaagc ggtagcttag ctccccgttt cgtgctgatc 2220

agtctttttc aacacgtaaa aagcggagga gttttgcaat tttgttggtt gtaacgatcc 2280

tccgttgatt ttggcctctt tctccatggg cgggctgggc gtatttgaag cggttctctc 2340

ttctgccgtt a 2351

<210> 14
<211> 1796
<212> DNA
<213> Artificial Sequence

<220>
<223> Synthetic Construct- Sequence No. 14, Example 14: designer
      HydA1-promoter-linked Enolase DNA construct (1796 bp)

<400> 14
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccaggtg accaaggctg ttgagaacat caacgctatt attgcccccg 480

ccctgaaggg catggacccc gtcaagcagg cggagattga ccagaagatg aaggacctgg 540

acggcactga caacaagggc aagctgggtg ccaacgccat cctggccgtc tccatggccg 600

tgtgcaaggc cggtgccgct gagaagggcg tgcccctgta caagcacatt gcggacctgg 660

ccggcaacag caagctgatc ctgcccgtgc cctcgttcaa catcatcaac ggcggcagcc 720

acgccggcaa cgccctggct atgcaggagt tcatgatcct gcccgttggc gcctcgagct 780

tctctgaggc catgcgcatg ggctgcgagg tgtaccacgc cctgaagggc ctgatcaagg 840

ccaagtacgg ccaggacgcc tgcaacgtgg gtgatgaggg tggcttcgcc cccaacatcg 900

gctccaacga tgagggcctg aacttggtga acgaggccat cgagaaggcc ggctacaccg 960

gcaaggtgaa gatcggcatg gacgtggcct cgtcggagtt ctacaccgag gacggcatgt 1020

acgacctgga cttcaagaac cagcccaacg atggctcgca gaagaagacc aaggagcaga 1080

tgctggagct gtacaacgag ttctgcaaga agtacccggt catctccatc gaggacccct 1140

tcgagcagga cgactgggag ccctgcgcca agctgaccac cgagaacatc tgccaggtgg 1200

tcggcgacga catcctggtg accaaccccg tgcgcgtgaa gaaggccatc gacgccaagg 1260

ccgtcaacgc tctgctgctc aaggtcaacc agatcggtac cattaccgag tccattgagg 1320

ccgtgcgcat ggccaaggag gccggctggg gtgtcatgac cagccaccgc tcgggtgaga 1380

ctgaggactc tttcatcgcc gacctggcgg tgggcctggc ctccggccag atcaagaccg 1440

gcgccccctg ccgctcggag cgcaatgcca agtacaacca gctgctgcgc atcgaggagg 1500

agctgggcga gaacgctgtg tacgctggcg agagctggcg ccacatcggc tggtaaatgg 1560

aggcgctcgt tgatctgagc cttgccccct gacgaacggc ggtggatgga agatactgct 1620

ctcaagtgct gaagcggtag cttagctccc cgtttcgtgc tgatcagtct ttttcaacac 1680

gtaaaaagcg gaggagtttt gcaattttgt tggttgtaac gatcctccgt tgattttggc 1740

ctctttctcc atgggcgggc tgggcgtatt tgaagcggtt ctctcttctg ccgtta 1796

<210> 15
<211> 1832
<212> DNA
<213> Artificial Sequence

<220>
<223> Synthetic Construct- Sequence No. 15, Example 15: designer
      HydA1-promoter-linked Pyruvate-Kinase DNA construct (1832 bp)

<400> 15
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg tgcgagatgc tggacgcggg cgtggtgggc tgccgcgtgg 480

acctgacgtg gggcccgctg gagttccacc gcaagtcgct tgccaatctg cagcaggcca 540

tgcgcaagag ccgccgcctg tgttgcacca tggtggacac gctgggccgc gagctcatga 600

tccgccgcca gagaggggca ggctggaccc agcgccagag gggtggggtg atcatcacca 660

cgcgcacgga cgtggacgcc agcagcaacg tgctgcccat cacttacagc aagttcacgg 720

agatggcggt caagggcgac accatctaca tcggccgcta cctggtgtgc ggcgcagaca 780

gcgcctcgct gtacctggag gtcatggacg tgcagggcga cgacgtgtac tgcatcgcca 840

agaacgacgc ggtgctggac ggcctgctga cggtgttcca cgcggagcgc tccgtggagg 900

ggctggccaa cgtgcagaac gacctgccgc tgctgtccga ctacgacaag gagtgcctgc 960

acatcctggc gcaggacttc gagcgcgcgc cctacatctc caagctggag tccatcgcct 1020

cctccgccgt gcgcgccgcc gaccgcgtgg gcgccagcct gattgtggtg tacacgcaca 1080

ccggcaagac ggcgcagctg gtggccaagt accggccgcc catgcccatc ctgacgctgg 1140

tggtgccgca cctggtgtct gaccagctca agtggaagct ggagggcagg tccagcgcgc 1200

gccagtgcct catcagtcgc gcgctgctgc cggtgctggc cgcgccctcg cccagcggcg 1260

accagctgct gcaggaggcg gtggccatgg cgggccgcgt caagctggtc aagccgcacg 1320

accacgtggt gtgcgtgcag cgcatccacg acgacttctg cgtcaagatc atctccgtgg 1380

acgacatggg cgcgggcatc aagcgcgacg acacggtcat gtcgcacagc gtgtttggca 1440

gcagccccat ggccgtgcag ggctcgtccg gctacgactc gccgcgcgtg cacaacaacc 1500

ccatcggcaa caagttcggc cccatgccgc ccgccatcat caccaccggc aatagcttca 1560

ccctgggcgg catgggcgtg ggcgtgctgt aaatggaggc gctcgttgat ctgagccttg 1620

ccccctgacg aacggcggtg gatggaagat actgctctca agtgctgaag cggtagctta 1680

gctccccgtt tcgtgctgat cagtcttttt caacacgtaa aaagcggagg agttttgcaa 1740

ttttgttggt tgtaacgatc ctccgttgat tttggcctct ttctccatgg gcgggctggg 1800

cgtatttgaa gcggttctct cttctgccgt ta 1832

<210> 16
<211> 4376
<212> DNA
<213> Artificial Sequence

<220>
<223> Synthetic Construct- Sequence No. 16, Example 16: designer
HydA1-promoter-linked Pyruvate-Ferredoxin-Oxidoreductase DNA
construct (4376 bp)

<400> 16
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg ggaaagaaaa tgatgacgac tgatggcaat acagcgacag 480

cgcacgtggc gtatgccatg agcgaagtcg ccgccatcta ccccatcacc ccttcctcga 540

ccatgggcga ggaggctgac gactgggcgg cgcaaggacg caagaacatc tttggccaga 600

ccctgaccat acgcgaaatg cagtccgagg ccggcgccgc cggcgcggtg cacggggccc 660

tggcggccgg cgccctgacc acgaccttca cggcgtcgca gggtctgctg ctgatgatcc 720

ccaacatgta caagatctcc ggcgaacttc tgcccggcgt gttccacgtc accgcccgcg 780

ccatcgccgc gcacgccctg tccatcttcg gtgaccacca ggatatctac gccgcgcgcc 840

agacaggctt cgccatgctc gcctccagct cggtgcagga ggcccacgac atggccctgg 900

tggcccactt ggcggccatc gagtccaacg tgccgttcat gcacttcttc gacggattcc 960

gcacctcgca cgaaatccag aagatcgagg tcctggacta cgcggacatg gcctcgctgg 1020

tgaaccagaa ggccctggcg gaattccgcg ccaagtccat gaaccccgag cacccccacg 1080

tgcgcggcac ggcccagaac cccgacatct acttccaggg tcgcgaggca gccaacccct 1140

actacctcaa ggtgcccggc atcgttgccg agtacatgca gaaggtcgcc tccctcacgg 1200

gccgcagcta caagctcttt gactacgtgg gtgctcccga cgccgagcgc gtcatcgtgt 1260

ccatgggctc ctcgtgcgag accatcgagg aggtcatcaa ccacctcgcg gccaagggcg 1320

aaaagatcgg cctgatcaag gtccgcctgt acaggccctt cgtaagcgag gccttcttcg 1380

ctgctctgcc cgcttcggcc aaggtcatca cggtcctcga ccgcaccaag gaacccggcg 1440

cgcccggcga tccgctctac ctcgacgtgt gctcggcctt cgtggagcgc ggcgaagcca 1500

tgcccaagat cctggccggc cgctacggcc tgggttccaa ggaattcagc ccggccatgg 1560

tcaagtccgt gtacgacaac atgtccggcg ctaagaagaa ccacttcacc gtgggcatcg 1620

aagacgacgt gaccggcact tcgctgccgg tggacaacgc cttcgccgac accacgccca 1680

agggcaccat ccagtgccag ttctggggcc tcggcgccga cggcactgtg ggcgccaaca 1740

agcaggccat caagatcatc ggcgacaaca cggacctgtt tgcccagggt tacttctcct 1800

acgactccaa gaaatcgggc ggcatcacca tctcgcacct gcgcttcggc gagaagccca 1860

tccagtccac ctacctggtc aacagggccg actatgtcgc ctgtcacaac ccggcctacg 1920

tgggcatata cgacatcctc gaaggcatca aggatggcgg aaccttcgtg ctcaactcgc 1980

cttggagcag cctcgaggac atggacaagc acctgccctc cggcatcaag cgcaccatcg 2040

cgaacaagaa gctcaagttc tacaacatcg acgcggtgaa aatcgccacc gatgtgggac 2100

tgggcggccg catcaacatg atcatgcaga cggccttctt caagctggcc ggagtgctgc 2160

ccttcgaaaa ggccgtggat ctgctcaaga agtccatcca caaggcctac ggcaaaaagg 2220

gcgagaagat cgtcaagatg aacaccgacg ccgtggacca ggccgtcacc tccctgcagg 2280

aattcaagta tccggattcc tggaaggacg ctcccgctga gaccaaggcc gagcccatga 2340

cgaacgagtt cttcaagaac gtcgtcaagc ccatcctgac ccagcagggc gacaagctgc 2400

cggtgagcgc cttcgaggcc gacggccgtt tccccctcgg caccagccag ttcgagaagc 2460

gcggcgtggc catcaacgtg ccgcagtggg tccccgagaa ctgcatccag tgcaaccagt 2520

gcgccttcgt ctgtccgcac agcgccatcc tgcccgtgct ggccaaggaa gaggagttgg 2580

tcggcgcgcc ggcgaacttc acggccctgg aagccaaggg caaggagctc aagggctaca 2640

agttccgcat ccagatcaac accctggact gcatgggctg cggcaactgc gccgacatct 2700

gtccgcccaa ggaaaaggct ctggtcatgc agcccctgga tacccagcgc gacgcgcagg 2760

tgcccaacct ggagtacgca gcgcgcatcc cggtcaaatc cgaggtgctg ccgcgcgact 2820

cgctcaaggg cagccagttc caggagcctc tcatggaatt ctcgggcgcc tgctcgggct 2880

gcggcgagac gccctacgtg cgcgtcatca cccagctctt cggcgagcgc atgttcattg 2940

ccaacgccac gggttgctcg tccatctggg gcgcgtcggc tccttccatg ccttacaaga 3000

ccaaccgcct cggacaaggc ccggcctggg gtaactccct gttcgaagac gcggccgaat 3060

acggcttcgg catgaacatg tccatgttcg cccgccgcac gcatttggcc gatcttgccg 3120

ccaaggccct ggagagcgat gcctccggcg atgtcaagga agccctgcag ggctggcttg 3180

ccggcaagaa cgatcccatc aagtccaagg aatacggcga caagctcaag aagctgctgg 3240

ctggtcagaa ggatggtctg ctcggacaga tcgccgccat gtccgacctg tacaccaaga 3300

agagcgtgtg gatcttcggt ggcgacggct gggcctacga catcggttac ggcggcctgg 3360

accatgtgct cgcctcgggc gaggacgtga acgtcttcgt catggatacc gaggtctact 3420

ccaacaccgg cggccagtcc tccaaggcaa cgcccacggg cgccgtggcc aagttcgcgg 3480

cggccggcaa gcgtaccggc aagaaggacc tggcgcgcat ggtcatgacc tacggctacg 3540

tctacgtggc tacggtctcc atgggttaca gcaagcagca gttcctcaag gtgctcaagg 3600

aagccgaaag cttccccggc ccctcgctgg tcatcgccta tgctacctgc atcaaccagg 3660

gtctgcgcaa gggcatgggc aagagccagg acgtcatgaa caccgcggtc aagtccggtt 3720

actggccgct gttccgctac gatccgcgct tggccgccca gggcaagaac cccttccagc 3780

tcgactccaa ggctcctgac ggttccgtcg aggagttcct gatggcccag aaccgcttcg 3840

ccgtcctcga tcggtccttc cccgaggacg ccaagagact gcgcgcccag gtcgctcacg 3900

aattggacgt gcgtttcaag gagttggagc acatggccgc cacgaacatc ttcgagtcct 3960

tcgcgccagc gggcggcaag gccgatggtt cggtggattt cggcgaaggt gcggagttct 4020

gcacgcgcga cgatactccc atgatggccc gacctgattc cggtgaggcc tgcgaccaga 4080

accgcgctgg cacgagcgaa cagcagggag acctcagcaa gcggacgaag aagtaaatgg 4140

aggcgctcgt tgatctgagc cttgccccct gacgaacggc ggtggatgga agatactgct 4200

ctcaagtgct gaagcggtag cttagctccc cgtttcgtgc tgatcagtct ttttcaacac 4260

gtaaaaagcg gaggagtttt gcaattttgt tggttgtaac gatcctccgt tgattttggc 4320

ctctttctcc atgggcgggc tgggcgtatt tgaagcggtt ctctcttctg ccgtta 4376

<210> 17
<211> 6092

<212>  DNA
<213>  Artificial Sequence

<220>
<223>  Synthetic Construct- Sequence No. 17, Example 17: designer
       HydA1-promoter-linked Pyruvate-NADP+-Oxidoreductase DNA construct
       (6092 bp)

<400>  17
agaaaatctg gcaccacacc gagctgtcat gcgttgttcc gttatgtgtc gtcaaacgcc      60

ttcgagcgct gcccggaaca atgcgtacta gtataggagc catgaggcaa gtgaacagaa     120

gcgggctgac tggtcaaggc gcacgatagg gctgacgagc gtgctgacgg ggtgtaccgc     180

cgagtgtccg ctgcattccc gccggattgg gaaatcgcga tggtcgcgca taggcaagct     240

cgcaaatgct gtcagcttat cttacatgaa cacacaaaca ctctcgcagg cactagcctc     300

aaatggccgc cgtcattgcc aagtcctccg tctccgcggc cgtggctcgc ccggcccgct     360

ccagcgtgcg ccccatggcc gcgctgaagc ccgccgtcaa ggctgccccc gtggctgccc     420

cggctcaggc caaccagatg aagcagtctg tccgcccaat tatttccaat gtactgcgca     480

aggaggttgc tctgtactca acaatcattg gacaagacaa ggggaaggaa ccaactggtc     540

gaacatacac cagtggccca aaaccggcat ctcacattga agttccccat catgtgactg     600

tgcctgccac tgaccgcacc ccgaatcccg atgctcaatt ctttcagtct gtagatgggt     660

cacaagccac cagtcacgtt gcgtacgctc tgtctgacac agcgttcatt tacccaatta     720

cacccagttc tgtgatgggc gagctggctg atgtttggat ggctcaaggg aggaagaacg     780

cctttggtca ggttgtggat gtccgtgaga tgcaatctga ggctggagcc gcaggcgccc     840

tgcatggggc actggctgct ggagctattg ctacaacctt cactgcctct caagggttgt     900

tgttgatgat tcccaacatg tataagattg caggtgagct gatgccctct gtcatccacg     960

ttgcagcccg agagcttgca ggccacgctc tgtccatttt tggaggacac gctgatgtca    1020

tggctgtccg ccaaacagga tgggctatgc tgtgctccca cacagtgcag cagtctcacg    1080

acatggctct catctcccac gtggccaccc tcaagtccag catccccttc gttcacttct    1140

ttgatggttt ccgcacaagc cacgaagtga acaaaatcaa aatgctgcct tatgcagaac    1200

tgaagaaact ggtgcctcct ggcaccatgg aacagcactg ggctcgttcg ctgaacccca    1260

tgcaccccac catccgagga acaaaccagt ctgcagacat ctacttccag aatatggaaa    1320

gtgcaaacca gtactacact gatctggccg aggtcgttca ggagacaatg gacgaagttg    1380

caccatacat cggtcgccac tacaagatct ttgagtatgt tggtgcacca gatgcagaag    1440

aagtgacagt gctcatgggt tctggtgcaa ccacagtcaa cgaggcagtg gaccttcttg    1500

tgaagcgtgg aaagaaggtt ggtgcagtct tggtgcacct ctaccgacca tggtcaacaa    1560

aggcatttga aaaggtcctg cccaagacag tgaagcgcat tgctgctctg gatcgctgca    1620

aggaggtgac tgcactgggt gagcctctgt atctggatgt gtcggcaact ctgaatttgt 1680

tcccggaacg ccagaatgtg aaagtcattg gaggacgtta cggattgggc tcaaaggatt 1740

tcatcccgga gcatgccctg gcaatttacg ccaacttggc cagcgagaac cccattcaaa 1800

gattcactgt gggtatcaca gatgatgtca ctggcacatc cgttcctttc gtcaacgagc 1860

gtgttgacac gttgcccgag ggcacccgcc agtgtgtctt ctggggaatt ggttcagatg 1920

gaacagtggg agccaatcgc tctgccgtga gaatcattgg agacaacagc gatttgatgg 1980

ttcaggccta cttccaattt gatgctttca agtcaggtgg tgtcacttcc tcgcatctcc 2040

gttttggacc aaagcccatc acagcgcaat accttgttac caatgctgac tacatcgcgt 2100

gccacttcca ggagtatgtc aagcgctttg acatgcttga tgccatccgt gaggggggca 2160

cctttgttct caattctcgg tggaccacgg aggacatgga gaaggagatt ccggctgact 2220

tccggcgcaa gctggcacag aagaaggtcc gcttctacaa tgtggatgct cgaaagatct 2280

gtgacagttt tggtcttggg aagcgcatca atatgctgat gcaggcttgt ttcttcaagc 2340

tgtctggggt gctcccactg gccgaagctc agcggctgct gaacgagtcc attgtgcatg 2400

agtatggaaa gaagggtggc aaggtggtgg agatgaacca agcagtggtg aatgctgtct 2460

ttgctggtga cctgccccag gaagttcaag tccctgccgc ctgggcaaac gcagttgata 2520

catccacccg tacccccacc gggattgagt ttgttgacaa gatcatgcgc ccgctgatgg 2580

atttcaaggg tgaccagctc ccagtcagtg tgatgactcc tggtggaacc ttccctgtcg 2640

ggacaacaca gtatgccaag cgtgcaattg ctgctttcat tccccagtgg attcctgcca 2700

actgcacaca gtgcaactat tgttcgtatg tttgccccca cgccaccatc cgacctttcg 2760

tgctgacaga ccaggaggtg cagctggccc cggagagctt tgtgacacgc aaggcgaagg 2820

gtgattacca ggggatgaat ttccgcatcc aagttgctcc tgaggattgc actggctgcc 2880

aggtgtgcgt ggagacgtgc cccgatgatg ccctggagat gaccgacgct ttcaccgcca 2940

cccctgtgca acgcaccaac tgggagttcg ccatcaaggt gcccaaccgc ggcaccatga 3000

cggaccgcta ctccctgaag ggcagccagt tccagcagcc cctcctggag ttctccgggg 3060

cctgcgaggg ctgcggcgag accccatatg tcaagctgct cacccagctc ttcggcgagc 3120

ggacggtcat cgccaacgcc accggctgca gttccatctg gggtggcact gccggcctgg 3180

cgccgtacac caccaacgcc aagggccagg gcccggcctg gggcaacagc ctgttcgagg 3240

acaacgccga gttcggcttt ggcattgcag tggccaacgc ccagaagagg tcccgcgtga 3300

gggactgcat cctgcaggca gtggagaaga aggtcgccga tgagggtttg accacattgt 3360

tggcgcaatg gctgcaggat tggaacacag gagacaagac cttgaagtac caagaccaga 3420

tcattgcagg gctggcacag cagcgcagca aggatcccct tctggagcag atctatggca 3480

tgaaggacat gctgcctaac atcagccagt ggatcattgg tggtgatggc tgggccaacg 3540

acattggttt cggtgggctg gaccacgtgc tggcctctgg gcagaacctc aacgtcctgg 3600

tgctggacac cgagatgtac agcaacaccg gtgggcaggc ctccaagtcc acccacatgg 3660

cctctgtggc caagtttgcc ctgggaggga agcgcaccaa caagaagaac ttgacggaga 3720

tggcaatgag ctatggcaac gtctatgtgg ccaccgtctc ccatggcaac atggcccagt 3780

gcgtcaaggc gtttgtggag gctgagtctt atgatggacc ttcgctcatt gttggctatg 3840

cgccatgcat cgagcatggt ctgcgtgctg gtatggcaag gatggttcaa gagtctgagg 3900

ctgccatcgc cacgggatac tggcccctgt accgctttga cccccgcctg gcgaccgagg 3960

gcaagaaccc cttccagctg gactccaagc gcatcaaggg caacctgcag gagtacctgg 4020

accgccagaa ccggtatgtc aacctgaaga agaacaaccc gaagggtgcg gatctgctga 4080

agtctcagat ggccgacaac atcaccgccc ggttcaaccg ctaccgacgc atgttggagg 4140

gccccaatac aaaagccgcc gcccccagcg gcaaccatgt gaccatcctg tacggctccg 4200

aaactggcaa cagtgagggt ctggcaaagg agctggccac cgacttcgag cgccgggagt 4260

actccgtcgc agtgcaggct ttggatgaca tcgacgttgc tgacttggag aacatgggct 4320

tcgtggtcat tgcggtgtcc acctgtgggc agggacagtt cccccgcaac agccagctgt 4380

tctggcggga gctgcagcgg gacaagcctg agggctggct gaagaacttg aagtacactg 4440

tcttcgggct gggcgacagc acatactact tctactgcca caccgccaag cagatcgacg 4500

ctcgcctggc cgccttgggc gctcagcggg tggtgcccat tggcttcggc gacgatgggg 4560

atgaggacat gttccacacc ggcttcaaca actggatccc cagtgtgtgg aatgagctca 4620

agaccaagac tccggaggaa gcgctgttca ccccgagcat cgccgtgcag ctcaccccca 4680

acgccacccc gcaggatttc catttcgcca agtccacccc agtgctgtcc atcaccggtg 4740

ccgaacgcat cacgccggca gaccacaccc gcaacttcgt cactatccga tggaagaccg 4800

atttgtcgta ccaggtgggt gactctcttg gtgtcttccc tgagaacacc cggtcagtgg 4860

tggaggagtt cctgcagtat tacggcttga accccaagga cgtcatcacc atcgaaaaca 4920

agggcagccg ggagttgccc cactgcatgg ctgttgggga tctcttcacg aaggtgttgg 4980

acatcttggg caaacccaac aaccggttct acaagaccct ttcttacttt gcagtggaca 5040

aggccgagaa ggagcgcttg ttgaagatcg ccgagatggg gccggagtac agcaacatcc 5100

tgtctgagac gtaccactac gcggacatct tccacatgtt cccgtccgcc cggcccacgc 5160

tgcagtacct catcgagatg atccccaaca tcaagccccg gtactactcc atctcctccg 5220

cccccatcca cacccctggc gaggtccaca gcctggtgct catcgacacc tggatcacgc 5280

tgtccggcaa gcaccgcacg gggctgacct gcaccatgct ggagcacctg caggcgggcc 5340

aggtggtgga tggctgcatc caccccacgg cgatggagtt ccccgaccac gagaagccgg 5400

tggtgatgtg cgccatgggc agtggcctgg caccgttcgt tgctttcctg cgcgacggct 5460

ccacgctgcg gaagcagggc aagaagaccg ggaacatggc attgtacttc ggcaacaggt 5520

atgagaagac ggagttcctg atgaaggagg agctgaaggg tcacatcaac gatggtttgc 5580

tgacacttcg atgcgctttc agccgagatg accccaagaa gaaggtgtat gtgcaggacc 5640

ttatcaagat ggacgaaaag atgatgtacg attacctcgt ggtgcagaag ggttctatgt 5700

attgctgtgg atcccgcagt ttcatcaagc ctgtccagga gtcattgaaa cattgcttca 5760

tgaaagctgg tgggctgact gcagagcaag ctgagaacga ggtcatcgat atgttcacga 5820

ccgggcggta caatatcgag gcatggtaat aaatggaggc gctcgttgat ctgagccttg 5880

ccccctgacg aacggcggtg gatggaagat actgctctca agtgctgaag cggtagctta 5940

gctccccgtt tcgtgctgat cagtcttttt caacacgtaa aaagcggagg agttttgcaa 6000

ttttgttggt tgtaacgatc ctccgttgat tttggcctct ttctccatgg gcgggctggg 6060

cgtatttgaa gcggttctct cttctgccgt ta 6092

<210> 18
<211> 1856
<212> DNA
<213> Artificial Sequence

<220>
<223> Synthetic Construct- Sequence No. 18, Example 18: designer
      HydA1-promoter-linked Thiolase DNA construct (1856 bp)

<400> 18
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg aaagaagtag ttattgcaag tggtgtaagg actgctgtcg 480

ggaaatttgg tggcacgctt ctaaatgtac ctgcagtaga tttaggtgct gtgaataata 540

aaagaagcat aaaaagagcc aatgtgaaac ctgaagatgt tagtgaagtg ataatgggaa 600

atgtattgca ggcaggtctt gggcagaacc ccgcaagaca agctgaaata aaagcgggca 660

taccagtaga agttccggct atgactgtaa acatggtatg tggatcaggt cttagagctg 720

tgacacttgc tgctcaggca gttatgcttg gtgatgctga cattgttgta gccggtggaa 780

tggaaaatat gtcaagagca ccatatatat taaatgatgc tcgctttggg tacaggatga 840

acaatggcca gcttgtagat gaaatggtat atgatggttt aacagatgtt tttaaccaat 900

atcacatggg aatcactgcc gaaaatcttg ctgaaaaata cggcatatca agagaagagc 960

aggatgaatt tgcatataga agccaaaaat tagcgtcaga agcgatatca tcaggaagat 1020

ttgaggatga gatagttcct gtgattgtgc cgcagaaaaa aggtgaaccg atagaattta 1080

aagttgatga acatgtgaga cctaatacga caattgaagc acttgcaaaa ttaaaaccag 1140

cattccaaaa agatggaact gtaactgctg gaaatgcatc aggaattaac gatgcagctg 1200

cagcagtagt tgtgatgtca aaagaaaagg catgtgaact tggaataaag accattgcaa 1260

cgattaaatc atttggttat gcaggtgttg accccagcat cacgggaatt ggtccagtat 1320

atgctacgag aaaggcatta gaaaaagcta atctaactgt agatgattta gatttaattg 1380

aagcaaatga agcatttgca gcacaatcac tggctgttgc aaaagaatta aaatttaata 1440

tggacagagt gaatgtaaat ggtggcgcaa ttgcgatagg tcatccaatc ggcgccagcg 1500

gatgtagaat tctagtgacg cttttatatg agatgcagaa gaggaattcg catactggac 1560

ttgcaacatt gtgcatcggc ggaggaatgg gaatagcaat ggttgtcgaa agataaatgg 1620

aggcgctcgt tgatctgagc cttgccccct gacgaacggc ggtggatgga agatactgct 1680

ctcaagtgct gaagcggtag cttagctccc cgtttcgtgc tgatcagtct ttttcaacac 1740

gtaaaaagcg gaggagtttt gcaattttgt tggttgtaac gatcctccgt tgattttggc 1800

ctctttctcc atgggcgggc tgggcgtatt tgaagcggtt ctctcttctg ccgtta 1856

<210> 19
<211> 1550
<212> DNA
<213> Artificial Sequence

<220>
<223> Synthetic Construct- Sequence No. 19, Example 19: designer
      HydA1-promoter-linked 3-Hydroxybutyryl-CoA-Dehydrogenase DNA
      construct (1550 bp)

<400> 19
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg caaaagattt gtgtaatagg tgctggaaca atgggctcag 480

gcatcgctca agtatttgca caaaatggct ttgaagtaat tttacgcgat attgatatga 540

agttcgtaga aaaaggattt ggcacaattg aaaaaattta caaagaaatg ttgacaaagg 600

gaaaattaca gcagatgaga aaacgaattt taagcagaat cagaggtaca acaaatttgg 660

aagacgcaaa agaagcagat tttgtagttg aagcggctat agaaaatatg gatctcaaga 720

aacaaatatt caaagagcta gatgaaatat gcaaaatgga aacaatcctt gcgtcaaata 780

catcatcact atccataaca gaaatagcaa gtgcgacaaa aagacctgag aaagtcatag 840

gaatgcattt cttcaaccca gttccagtaa tgaaacttgt tgaagtcata aaaggattaa 900

agacatcaga gcaaacattt aatgtcgtca gagaattggc tttaaaagta gacaaaacac 960

ctatagaggt caaagaagca cctggatttg ttgtaaatag gattttaatc ccaatgatta 1020

atgaagcaat tggaatactt gcagtggtgt tggcaactga caagagcata gatgaagcta 1080

tgaaacttgg tgcaaatcat ccaataggac ctttggcatt gtctagtttg ataggcaatg 1140

acgtcgttct tgctataatg aatgtgcttt atgaagagta cggcgattcg aaatacagac 1200

cacatccact tctaaaaaaa gtggtaagag gcggattgct gggtagaaaa actggcaaag 1260

gtttctttga atacaaaatt aatcttttaa ggaggagaat atcatgataa atggaggcgc 1320

tcgttgatct gagccttgcc ccctgacgaa cggcggtgga tggaagatac tgctctcaag 1380

tgctgaagcg gtagcttagc tccccgtttc gtgctgatca gtctttttca acacgtaaaa 1440

agcggaggag ttttgcaatt ttgttggttg taacgatcct ccgttgattt tggcctcttt 1500

ctccatgggc gggctgggcg tatttgaagc ggttctctct tctgccgtta 1550

<210> 20
<211> 1457
<212> DNA
<213> Artificial Sequence

<220>
<223> Synthetic Construct- Sequence No. 20, Example 20: designer
      HydA1-promoter-linked Crotonase DNA construct (1457 bp)

<400> 20
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg gattttaata atgttttatt aaataaggat gatgggatag 480

ctctcatcat tataaatcgt ccaaaggctt taaatgcatt aaactatgag acactaaaag 540

agttagatag tgtgcttgat atagttgaaa atgataaaga gataaaagtt ttaattataa 600

ctggcagcgg tgaaaaaacc ttcgttgcag gtgctgatat agctgagatg agtaatatga 660

caccacttga agcgaagaag ttctctcttt atggacagaa agtatttagg aagatagaaa 720

tgctaagtaa gcctgttata gcagcggtaa atggttttgc acttggtggt ggatgcgagc 780

tttctatggc atgtgacata cgtattgcaa gtaaaaatgc aaaatttggt caacctgaag 840

taggacttgg aataatacct ggcttttcag gaactcaaag attaccacgt cttataggca 900

cttctaaagc taaagagctt attttcacag gtgacatgat aaattctgat gaagcatata 960

aaataggcct tatatctaaa gttgttgaac tatctgatct cattgaagaa gcaaaaaaac 1020

tcgcgaaaaa aatgatgtca aaaagtcaaa tagcaatttc tctagcaaag gaagcaataa 1080

ataagggaat ggaaacagac ttagatacag gcaatactat agaagctgag aaattttcct 1140

tatgttttac aacagatgat caaaaagaag gtatgattgc gttttctgaa aagagggcgc 1200

ctaaatttgg caaataaatg gaggcgctcg ttgatctgag ccttgccccc tgacgaacgg 1260

cggtggatgg aagatactgc tctcaagtgc tgaagcggta gcttagctcc ccgtttcgtg 1320

ctgatcagtc tttttcaaca cgtaaaaagc ggaggagttt tgcaattttg ttggttgtaa 1380

cgatcctccg ttgattttgg cctctttctc catgggcggg ctgggcgtat ttgaagcggt 1440

tctctcttct gccgtta 1457

<210> 21
<211> 1817
<212> DNA
<213> Artificial Sequence

<220>
<223> Synthetic Construct- Sequence No. 21, Example 21: designer
      HydA1-promoter-linked Butyryl-CoA-Dehydrogenase DNA construct
      (1817 bp)

<400> 21
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg gacttttcat taacaaagga gcaagaaatg gtaaggcgtg 480

ttgtgagaga attcgctgaa aaagaagttg ctcctaaagc aaaagaaata gatatcacag 540

aagagtttcc atgggataca gtaagaaaaa tggctcaaaa cgatatgatg ggtattcctt 600

atccagaaga gtatggtgga gcaggtggag attacttgag ttatatcata gctgttgaag 660

agatatcaag agcttgtgct acgactggag taattttatc tgctcatact tcattgggaa 720

gttttccaat atatcaatgg ggaacagaag aacaaaaaag aaaatatcta gtgccacttg 780

caaaaggtga aaaattgggc gcttttggcc ttacagaacc taacgcaggt acagatgcag 840

ctggacagca gacaactgca gtattagatg gtgatcacta cgtattaaac ggctcaatat 900

ttattacaaa cggaggaaaa gctgacatat atataatctt tgcaatgaca gacaaatcaa 960

aaggcacaag aggcattagt gcatttatag ttgagaaaga ttttccgggt tttagcattg 1020

gcaaaattga agaaaaaatg ggtataagag cttcatcaac tgccgaactt gtgtttgaag 1080

attgtattgt accaaaagaa aatttacttg gtaaagaagg agaaggtttt aaaattgcga 1140

tggctacact agatggtgga agaataggaa tagcagcgca acgccttgga atagctcagg 1200

ctgctttaga tgaagagata aaatatgcaa aggaaagaca acagtttgga agaccaattg 1260

gaaaatttca aggcattcaa tggtatatag ctgatatggc aacgagaata aatgcttcaa 1320

gatggcttgt atacaatgcc gcttggagaa agcaggtagg tcttccgtac acaatggaag 1380

cagctatggc aaaattatat gcttccgaaa cagcaatgtt tgtaacgaca aaaacagttc 1440

agatatttgg cggctatggc tttacaaaag attatccagt ggaaagattt atgagagatg 1500

caaaaataac agaaatttat gaaggcacat cggaagtcca gaaaatggtt atttccggta 1560

acctattgaa aatgtaaatg gaggcgctcg ttgatctgag ccttgccccc tgacgaacgg 1620

cggtggatgg aagatactgc tctcaagtgc tgaagcggta gcttagctcc ccgtttcgtg 1680

ctgatcagtc tttttcaaca cgtaaaaagc ggaggagttt tgcaattttg ttggttgtaa 1740

cgatcctccg ttgattttgg cctctttctc catgggcggg ctgggcgtat ttgaagcggt 1800

tctctcttct gccgtta 1817

<210> 22
<211> 2084
<212> DNA
<213> Artificial Sequence

<220>
<223> Synthetic Construct- Sequence No. 22, Example 22: designer
 HydA1-promoter-linked Butyraldehyde-Dehydrogenase DNA construct
 (2084 bp)

<400> 22
agaaaatctg gcaccacacc gagctgtcat gcgttgttcc gttatgtgtc gtcaaacgcc 60

ttcgagcgct gcccggaaca atgcgtacta gtataggagc catgaggcaa gtgaacagaa 120

gcgggctgac tggtcaaggc gcacgatagg gctgacgagc gtgctgacgg ggtgtaccgc 180

cgagtgtccg ctgcattccc gccggattgg gaaatcgcga tggtcgcgca taggcaagct 240

cgcaaatgct gtcagcttat cttacatgaa cacacaaaca ctctcgcagg cactagcctc 300

aaatggccgc cgtcattgcc aagtcctccg tctccgcggc cgtggctcgc ccggcccgct 360

ccagcgtgcg ccccatggcc gcgctgaagc ccgccgtcaa ggctgccccc gtggctgccc 420

cggctcaggc caaccagatg attaaagaca cgctagtttc tataacaaaa gatttaaaat 480

taaaaacaaa tgttgaaaat gccaatctaa agaactacaa ggatgattct tcatgtttcg 540

gagttttcga aaatgttgaa aatgctataa gcaatgccgt acacgcacaa aagatattat 600

cccttcatta tacaaaagaa caaagagaaa aaatcataac tgagataaga aaggccgcat 660

tagaaaataa agagattcta gctacaatga ttcttgaaga aacacatatg ggaagatatg 720

aagataaaat attaaagcat gaattagtag ctaaatacac tcctgggaca gaagatttaa 780

ctactactgc ttggtcagga gataacgggc ttacagttgt agaaatgtct ccatatggcg 840

ttataggtgc aataactcct tctacgaatc caactgaaac tgtaatatgt aatagtatag 900

gcatgatagc tgctggaaat actgtggtat ttaacggaca tccaggcgct aaaaaatgtg 960

ttgcttttgc tgtcgaaatg ataaataaag ctattatttc atgtggtggt cctgagaatt 1020

tagtaacaac tataaaaaat ccaactatgg actctctaga tgcaattatt aagcaccctt 1080

caataaaact actttgcgga actggagggc caggaatggt aaaaaccctc ttaaattctg 1140

gtaagaaagc tataggtgct ggtgctggaa atccaccagt tattgtagat gatactgctg 1200

atatagaaaa ggctggtaag agtatcattg aaggctgttc ttttgataat aatttacctt 1260

gtattgcaga aaaagaagta tttgtttttg agaacgttgc agatgattta atatctaaca 1320

tgctaaaaaa taatgctgta attataaatg aagatcaagt atcaaagtta atagatttag 1380

tattacaaaa aaataatgaa actcaagaat actctataaa taagaaatgg gtcggaaaag 1440

atgcaaaatt attcttagat gaaatagatg ttgagtctcc ttcaagtgtt aaatgcataa 1500

tctgcgaagt aagtgcaagg catccatttg ttatgacaga actcatgatg ccaatattac 1560

caattgtaag agttaaagat atagatgaag ctattgaata tgcaaaaata gcagaacaaa 1620

atagaaaaca tagtgcctat atttattcaa aaaatataga caacctaaat aggtttgaaa 1680

gagaaatcga tactactatc tttgtaaaga atgctaaatc ttttgccggt gttggttatg 1740

aagcagaagg ctttacaact ttcactattg ctggatccac tggtgaagga ataacttctg 1800

caagaaattt tacaagacaa agaagatgtg tactcgccgg ttaaatggag gcgctcgttg 1860

atctgagcct tgccccctga cgaacggcgg tggatggaag atactgctct caagtgctga 1920

agcggtagct tagctccccg tttcgtgctg atcagtcttt ttcaacacgt aaaaagcgga 1980

ggagttttgc aattttgttg gttgtaacga tcctccgttg attttggcct ctttctccat 2040

gggcgggctg ggcgtatttg aagcggttct ctcttctgcc gtta 2084

<210> 23
<211> 1733
<212> DNA
<213> Artificial Sequence

<220>
<223> Synthetic Construct- Sequence No. 23, Example 23: designer
      HydA1-promoter-linked Butanol-Dehydrogenase DNA construct (1733
      bp)

<400> 23
agaaaatctg gcaccacacc gagctgtcat gcgttgttcc gttatgtgtc gtcaaacgcc      60

ttcgagcgct gcccggaaca atgcgtacta gtataggagc catgaggcaa gtgaacagaa     120

gcgggctgac tggtcaaggc gcacgatagg gctgacgagc gtgctgacgg ggtgtaccgc     180

cgagtgtccg ctgcattccc gccggattgg gaaatcgcga tggtcgcgca taggcaagct     240

cgcaaatgct gtcagcttat cttacatgaa cacacaaaca ctctcgcagg cactagcctc     300

aaatggccgc cgtcattgcc aagtcctccg tctccgcggc cgtggctcgc ccggcccgct     360

ccagcgtgcg ccccatggcc gcgctgaagc ccgccgtcaa ggctgccccc gtggctgccc     420

cggctcaggc caaccagatg aaaggttttg caatgctagg tattaataag ttaggatgga     480

tcgaaaaaga aaggccagtt gcgggttcat atgatgctat tgtacgccca ttagcagtat     540

ctccgtgtac atcagatata catactgttt ttgagggagc tcttggagat aggaagaata     600

tgattttagg gcatgaagct gtaggtgaag ttgttgaagt aggaagtgaa gtgaaggatt     660

ttaaacctgg tgacagagtt atagttcctt gtacaactcc agattggaga tctttggaag     720

ttcaagctgg ttttcaacag cactcaaacg gtatgctcgc aggatggaaa ttttcaaatt     780

tcaaggatgg agtttttggt gaatattttc atgtaaatga tgcggatatg aatcttgcga     840

ttctacctaa agacatgcca ttagaaaatg ctgttatgat aacagatatg atgactactg     900

gatttcatgg agcagaactt gcagatattc aaatgggttc aagtgttgtg gtaattggca     960

ttggagctgt tggcttaatg ggaatagcag gtgctaaatt acgtggagca ggtagaataa    1020

ttggagtggg gagcaggccg atttgtgttg aggctgcaaa attttatgga gcaacagata    1080

ttctaaatta taaaaatggt catatagttg atcaagttat gaaattaacg aatggaaaag    1140

gcgttgaccg cgtaattatg gcaggcggtg gttctgaaac attatcccaa gcagtatcta    1200

tggttaaacc aggaggaata atttctaata taaattatca tggaagtgga gatgctttac    1260

taataccacg tgtagaatgg ggatgtggaa tggctcacaa gactataaaa ggaggtcttt    1320

gtcctggggg acgtttgaga gcagaaatgt taagagatat ggtagtatat aatcgtgttg    1380

atctaagtaa attagttaca catgtatatc atggatttga tcacatagaa gaagcactgt    1440

tattaatgaa agacaagcca aaagacttaa ttaaagcagt agttatatta taaatggagg    1500

cgctcgttga tctgagcctt gccccctgac gaacggcggt ggatggaaga tactgctctc    1560

aagtgctgaa gcggtagctt agctccccgt ttcgtgctga tcagtctttt tcaacacgta 1620

aaaagcggag gagttttgca attttgttgg ttgtaacgat cctccgttga ttttggcctc 1680

tttctccatg ggcgggctgg gcgtatttga agcggttctc tcttctgccg tta 1733

<210> 24
<211> 1556
<212> DNA
<213> Artificial Sequence

<220>
<223> Synthetic Construct- Sequence No. 24, Example 24: designer
Fructose-Diphosphate-Aldolase DNA construct (1556 bp)

<400> 24
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccctgatg atgaagtcgt cggccagcct gaaggctgtg tcgctggccg 240

ctctcgccgc gccgtcgttg tgcgcgccgg gcaagtacga tgaggagctg attaagaccg 300

ctggcaccgt tgcctccaag ggccgcggta tcctggccat ggacgagtca aacgccacct 360

gcggcaaacg cctggactcc atcggcgtgg agaacaccga ggagaaccgc cgcgcctacc 420

gcgagctgct ggtgaccgcc cccggcctgg gccagtacat ctccggcgct atcctgttcg 480

aggagaccct gtatcagtcc accgcctccg gcaagaagtt cgtcgatgtg atgaaggagc 540

agaacatcgt gcccggcatc aaggtcgaca agggcctggt gccctgtcca acaccaacga 600

tgagctggtg catgggcctg gacggctgga caagcgctgc tgagtactac aaggccggcg 660

ctcgcttcgc caagtggcgc tcggtcgtct cgatccccca cggcccctcg atcatgctgc 720

cgcgactggc ctacggcctg gcccgctacg ccgccatcgc ccagaacgcc ggtctggtgc 780

ccattgtgga gcccgaggtc ctgctggacg gtgagcacga catcgaccgc tgcctggagg 840

tgcaggaggc catctgggcc gagaccttca agtacatggc cgacaacaag gtcatgttgc 900

agggtatcct gctgaagccc gccatggtca cccccggcgc tgactgcaag aacaaggccg 960

gccccgccaa ggttgccgag tacaccctga agatgctggc cgcgcgtgcc cccccggtcc 1020

ccggcatcat gttcctgtcg ggcggccagt ccgagctgga gtcgaccctg aacctgaacg 1080

ccatgaacca gagccccaac ccgtggcacg tgtcgttctc gtacgcccgc gctctgacga 1140

acaccgttct gaagacctgg caggcaagcc cgagaacggt ccaggcgccc aggctcgctg 1200

ctcaagcgcg caaggccaac tcggacgctc agcagggcaa gtacgacgcc accaccgagg 1260

gcaaggaggc tgcccagggc atgtacgaga agggaaaagg ctacgtctac taataaatgg 1320

aggcgctcgt tgatctgagc cttgccccct gacgaacggc ggtggatgga agatactgct 1380

ctcaagtgct gaagcggtag cttagctccc cgtttcgtgc tgatcagtct ttttcaacac 1440

gtaaaaagcg gaggagtttt gcaattttgt tggttgtaac gatcctccgt tgattttggc 1500

ctctttctcc atgggcgggc tgggcgtatt tgaagcggtt ctctcttctg ccgtta 1556

<210> 25
<211> 1379
<212> DNA
<213> Artificial Sequence

<220>
<223> Synthetic Construct- Sequence No. 25, Example 25: designer
      Triose-Phosphate-Isomerase DNA construct (1379 bp)

<400> 25
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggcagctacc tctctcactg cccctccttc tttctccggt ctccgccgca 240

tttctcccaa gctcgacgct gccgccgtct cctcccacca atccttcttc caccgcgtca 300

attcctctac ccgtctcgtt tcttcctctt cttcttctca tcgctccccc agaggtgttg 360

ttgccatggc tggatccgga aagtttttcg ttggaggaaa ctggaagtgt aacgggacta 420

aggactccat cgccaagctt atctccgatc tcaacagtgc aaccttggaa gcagatgtag 480

atgttgttgt gtcacctcca tttgtctaca tcgaccaggt caaatcctcg ttgacagacc 540

gtattgacat atcaggtcag aactcttggg ttgggaaagg tggagccttc actggtgaaa 600

tcagcgtgga acagctcaaa gaccttggct gcaagtgggt cattcttggg cattccgaac 660

ggagacatgt catcggagaa aaagatgagt ttatcgggaa gaaagctgca tatgcattga 720

gtgagggtct tggagtgata gcttgtattg gggaaaagct agaagagagg gaagcaggca 780

agacgtttga tgtttgcttc gcgcaactga aggcgtttgc tgatgctgtg cctagctggg 840

acaatatagt tgttgcatac gagcctgtat gggcaattgg aactggtaaa gttgcatctc 900

ctcagcaagc acaagaagtc catgtagctg tccgcggttg gctaaagaag aatgtctctg 960

aggaagttgc ttccaaaacg agaatcatat atggaggttc tgtcaatgga ggcaacagtg 1020

cagagcttgc caaagaagaa gacattgatg gatttcttgt tggtggtgcc tccttgaagg 1080

gtcctgagtt tgcaaccatt gtgaactcag tcacgtcgaa gaaagttgct gcttgataaa 1140

tggaggcgct cgttgatctg agccttgccc cctgacgaac ggcggtggat ggaagatact 1200

gctctcaagt gctgaagcgg tagcttagct ccccgtttcg tgctgatcag tctttttcaa 1260

cacgtaaaaa gcggaggagt tttgcaattt tgttggttgt aacgatcctc cgttgatttt 1320

ggcctctttc tccatgggcg ggctgggcgt atttgaagcg gttctctctt ctgccgtta 1379

<210>  26
<211>  2156
<212>  DNA
<213>  Artificial Sequence

<220>
<223>  Synthetic Construct- Sequence No. 26, Example 26: designer
       Phosphofructose-Kinase DNA construct (2156 bp)

<400>  26
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggat ggccgccgtc attgccaagt cctccgtctc cgcggccgtg gctcgcccgg     240

cccgctccag cgtgcgcccc atggccgcgc tgaagcccgc cgtcaaggct gcccccgtgg     300

ctgccccggc tcaggccaac cagatggaag cttcgatttc gtttctgggg tcaacaaaac     360

ccaatatttc cttgtttaac ccttcttcaa acgtccttcc tcgtagagat ttccctcttc     420

ctgctttgaa attgaagaaa gtttcagtgc tgcctcgaat cttgcaccag aaacgactca     480

tcagagctca gtgctctgat ggattcaaac cagaggaaga cgatgggttt gtcctagaag     540

acgttcctca cttgaccaaa tttctccctg atttaccgtc atatccaaat ccattgaaag     600

aaagccaagc atatgccatt gttaagcgaa cttttgtcag ttccgaagat gtggttgcgc     660

aaaatattgt agtccagaag ggaagtaagc gaggagtaca ctttaggcga gcagggcctc     720

gagaaagagt gtacttcaga tcagatgaag taaaagcttg catagtgact tgtgggggct     780

tgtgccctgg aatcaatact gttatacggg aaattgtatg tggattgaac aatatgtatg     840

gtgttaataa cattctcggc attcagggag gatatagagg cttttactcc aaaaacacta     900

tgaacctgac acctaaagta gttaacgata ttcataaacg cggtggcact tttcttcaaa     960

cctcaagagg aggacatgat acagcgaaga ttgttgataa tattcaagat agaggaataa    1020

atcaggtata tattattgga ggtggtggga cgcaaaaggg tgcagagaag atatacgagg    1080

aagttgagag gcgtggtctt caagtggcgg tttctggcat tcctaagaca attgataatg    1140

atattgctgt gattgacaaa tcatttggct ttgatacggc ggttgaggaa gcacaacgag    1200

ctattaatgc tgcacatgta gaggtcgaga gcgtggaaaa tggagttggt atcgttaaac    1260

tcatgggcag atacagtggt tttattgcca tgattgcaac tttagcgaat cgtgatgtgg    1320

attgttgctt gattccagag tctccatttt ttcttgaagg aaagggtggg ctctttgagt    1380

ttattgaaga acgactcaaa gagaataggc acatggttat tgtgatagct gaaggagctg    1440

gacaggatta tgttgctcaa agcatgcgtg catctgaaac taaagacgcc tcaggaaata    1500

gactcttgct tgatgttggt ctatggttga ctcaacagat aaaggatcac tttacaaatg    1560

ttcggaaaat gatgataaat atgaagtaca tagacccaac gtatatgata agagcaatac 1620

cgagtaacgc atcagacaat gtctattgca ctcttcttgc ccaaagtgca gttcatggag 1680

caatggctgg gtactcaggt ttcactgtag gaccagttaa cagtagacat gcttacatcc 1740

caatttctgt gacggaagtg acaaatacgg tgaagttaac tgataggatg tgggctagac 1800

tccttgcatc gacaaatcaa ccgagtttct tgactggtga aggagcattg cagaatgtga 1860

tcgacatgga aactcaagaa aagatcgata acatgaagat ctcttctatc taataaatgg 1920

aggcgctcgt tgatctgagc cttgccccct gacgaacggc ggtggatgga agatactgct 1980

ctcaagtgct gaagcggtag cttagctccc cgtttcgtgc tgatcagtct ttttcaacac 2040

gtaaaaagcg gaggagtttt gcaattttgt tggttgtaac gatcctccgt tgattttggc 2100

ctctttctcc atgggcgggc tgggcgtatt tgaagcggtt ctctcttctg ccgtta 2156

<210> 27
<211> 860
<212> DNA
<213> Artificial Sequence

<220>
<223> Synthetic Construct- Sequence No. 27, Example 27: designer
      Nia1-promoter-linked Starch-Synthase-iRNA DNA construct (860 bp)

<400> 27
agaaaatctg gcaccacacc tatatggtag ggtgcgagtg accccgcgcg acttggagct 60

cgatggcccc gggttgtttg gggcgtccgc ctctcgcgct attctgagct ggagaccgag 120

gcgcatgaaa atgcattcgc ttccatagga cgctgcattg tggcttgaag gttcaaggga 180

agggttcaaa cgaccccgcc gtacgaactt ttgtcggggg gcgctcccgg ccccgggctc 240

ttgtgcgcgc attagggctt cgggtcgcaa gcaagacgat acatgccagc cggctcacca 300

ccgccaccag cggcttgcgg gggtccacct ccaggcccag acccttctgc agaaactcct 360

tgcacagcgc cttcccggcg gggcggtcgg cgtcgaagtt ggctggcagc agcgcgtcag 420

tggccgggtt ccactcctca cagtcaatgc cgttcaggat gccgtggaac ttggagcgca 480

gctcggggcg cgcgaaggtg gatctcgccg tcccagcggt agcccttggg cacctcgatg 540

tcgcattcgt gcttgaggcc ctcaatctgg tccttgggca ggcactcgta gaacggcagc 600

atgaccgtca cgaagtgtaa atggaggcgc tcgttgatct gagccttgcc ccctgacgaa 660

cggcggtgga tggaagatac tgctctcaag tgctgaagcg gtagcttagc tccccgtttc 720

gtgctgatca gtctttttca acacgtaaaa agcggaggag ttttgcaatt ttgttggttg 780

taacgatcct ccgttgattt tggcctcttt ctccatgggc gggctgggcg tatttgaagc 840

ggttctctct tctgccgtta 860

<210>  28
<211>  1328
<212>  DNA
<213>  Artificial Sequence

<220>
<223>  Synthetic Construct- Sequence No. 28, Example 28: designer
       HydA1-promoter-linked Starch-Synthase-iRNA DNA construct (1328
       bp)

<400>  28
agaaaatctg gcaccacacc gagctgtcat gcgttgttcc gttatgtgtc gtcaaacgcc      60

ttcgagcgct gcccggaaca atgcgtacta gtataggagc catgaggcaa gtgaacagaa     120

gcgggctgac tggtcaaggc gcacgatagg gctgacgagc gtgctgacgg ggtgtaccgc     180

cgagtgtccg ctgcattccc gccggattgg gaaatcgcga tggtcgcgca taggcaagct     240

cgcaaatgct gtcagcttat cttacatgaa cacacaaaca ctctcgcagg cactagcctc     300

aaatgaagag cttcatgcgg agggatgcgc tcggcgcggg gctccgcggt gcagccagca     360

caaagcccgt ctcaagggtc gccagcgtga ggcctgcgcc taccgcctac cgcactgcct     420

gccaagttgc gaaggtggat gaaatggtgt cggtggatga ggagcttact cgtctccgca     480

aggagaacga gctcctgcgc gcccaactgg cgctgtacca gcagaaccag cagccgtccg     540

tgggtgccgc tgccgttgcc ccgcctgctg ccgccacgaa ggtgctggag aagccggcgc     600

cgtaagtaac ctaacggtga gcagcatgca atattttagc gtcgatactc ggaaactata     660

ggagcgcatc agccgaccga tgttcgcgtt gctgtcgcag gcccaaccgt gccaccgccg     720

tggtgtgcaa ggcgcagaag gcggccaggc cgccgctgcc gctgctctgg ccataagtaa     780

cctaacggcg ccggcttctc cagcaccttc gtggcggcag caggcggggc aacggcagcg     840

gcacccacgg acggctgctg gttctgctgg tacagcgcca gttgggcgcg caggagctcg     900

ttctccttgc ggagacgagt aagctcctca tccaccgaca ccatttcatc caccttcgca     960

acttggcagg cagtgcggta ggcggtaggc gcaggcctca cgctggcgac ccttgagacg    1020

ggctttgtgc tggctgcacc gcggagcccc gcgccgagcg catccctccg catgaagctc    1080

ttcattaaat ggaggcgctc gttgatctga gccttgcccc ctgacgaacg gcggtggatg    1140

gaagatactg ctctcaagtg ctgaagcggt agcttagctc cccgtttcgt gctgatcagt    1200

ctttttcaac acgtaaaaag cggaggagtt ttgcaatttt gttggttgta acgatcctcc    1260

gttgattttg gcctctttct ccatgggcgg gctgggcgta tttgaagcgg ttctctcttc    1320

tgccgtta                                                              1328

<210>  29
<211>  1889
<212>  DNA
<213>  Artificial Sequence

<220>
<223>  Synthetic Construct- Sequence No. 29, Example 29: designer
      Amylase DNA construct (1889 bp)

<400>  29
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggct cgagcatatg gccgccgtca ttgccaagtc ctccgtctcc gcggccgtgg     240

ctcgcccggc ccgctccagc gtgcgcccca tggccgcgct gaagcccgcc gtcaaggctg     300

cccccgtggc tgccccggct caggccaacc agatggcgaa caaacacatg tccctttctc     360

tcttcatcgt cctccttggc ctctcgtgca gcttggcctc cgggcaagtc ctgtttcagg     420

gttttaactg ggagtcgtgg aagcacaatg gcgggtggta caacttcctg atgggcaagg     480

tggacgacat cgccgccgct ggcgtcacgc acgtgtggct ccccccggcg tcgcagtccg     540

tcgccgagca agggtacatg ccgggccggc tctacgacct ggacgcctcc aagtacggca     600

acaaggcgca gctcaagtcc ctcatcggcg cgctccacgg caagggcgtc aaggccatcg     660

ccgacatcgt catcaaccac cgcacggcgg agcgcaagga cggccggggc atctactgca     720

tcttcgaggg cggcaccccg gacgcgcgcc tcgactgggg cccccacatg atctgccgcg     780

acgaccggcc ctacgccgac ggcaccggca acccggacac cggcgccgac ttcggggccg     840

cgccggacat cgaccacctc aacccgcgcg tccagaagga gctcgtcgag tggctcaact     900

ggctcaggac cgacgtcggc ttcgacggct ggcgcttcga cttcgccaag ggctactccg     960

cggacgtggc caagatctac gtcgaccgct ccgagcccag cttcgccgtc gccgagatat    1020

ggacgtcgct ggcgtacggc ggggacggca agccgaacct caaccaggac ccgcaccggc    1080

aggagctggt gaactgggtg aacaaggtgg gcggctccgg ccccgccacc acgttcgact    1140

tcaccaccaa gggcatcctc aacgtggccg tggagggcga gctgtggcgc ctgcgcggca    1200

ccgacggcaa ggcgccgggc atgatcgggt ggtggccggc caaggcggtg accttcgtcg    1260

acaaccacga caccggctcc acgcagcaca tgtggccctt cccttccgac agggtcatgc    1320

agggatatgc ctacatcctc acgcacccag ggaccccatg catcttctac gatcatttct    1380

tcgactgggg cttgaaggag gagatcgatc gtctggtgtc aatcaggacc cgacagggga    1440

tacacagtga gagcaagctg cagatcatgg aggccgacgc cgacctttac cttgccgaga    1500

tcgacggcaa ggtcatcgtc aagctcgggc caagatacga tgtcggacac ctcattcctg    1560

aaggcttcaa ggtggtcgcg catggcaatg actatgccgt atgggagaaa gtataaggct    1620

gctgccccgg ctgctgctaa tctagataaa tggaggcgct cgttgatctg agccttgccc    1680

cctgacgaac ggcggtggat ggaagatact gctctcaagt gctgaagcgg tagcttagct    1740

ccccgtttcg tgctgatcag tctttttcaa cacgtaaaaa gcggaggagt tttgcaattt 1800

tgttggttgt aacgatcctc cgttgatttt ggcctctttc tccatgggcg ggctgggcgt 1860

atttgaagcg gttctctctt ctgccgtta 1889

<210> 30
<211> 3089
<212> DNA
<213> Artificial Sequence

<220>
<223> Synthetic Construct- Sequence No. 30, Example 30: designer
      Starch-Phosphorylase DNA construct (3089 bp)

<400> 30
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggcgg atgcgaaagc aaacggaaag aatgaggcgg 360

ccaaactggc gaaaattccg gcggctgcga atccattggc taatgaacca tcggcgattg 420

catcaaatat aagttaccac gtgcagtaca gtcctcattt ctcgccgact aagttcgagc 480

cggagcaagc tttctttgcc acggcggagg ttgtccgcga tcgtcttatt caacaatgga 540

atgagacata ccaccatttt aataaagttg atccgaagca aacatactac ctatcaatgg 600

aatttcttca aggaaggact ttgactaatg caattggcag tttggacatt cagaatgcat 660

atgctgatgc tttaaataat ttggggcatg tccttgagga gatagctgaa caggaaaaag 720

atgctgcact aggaaatggt gggctgggca ggctagcttc atgcttctta gactccatgg 780

caacattgaa tttgcctgca tggggttatg gtttgagata ccggtatggg ctgttcaagc 840

agaagatcac caagcagggt caagaagaag ttgctgaaga ttggcttgag aaatttagtc 900

cttgggaagt tgtcaggcat gatgtggtat ttccggtcag atttttgggg agtgttatgg 960

ttaatccaaa tggaacgaga aaatgggttg ggggtgaagt tgtccaagcc gtagcttatg 1020

atataccaat tccagggtac aaaaccaaga acactatcag tcttcgtctc tgggacgcta 1080

aagctagcgc tgaggatttc aatttatttc agtttaatga tggacaatac gaatctgctg 1140

cacagcttca ttctcgagct caacagattt gtgctgtgct ctaccccggg gattctactg 1200

aagaagggaa gcttttaagg ctgaaacaac aattctttct ctgcagtgct tcacttcagg 1260

atatgattct tagattcaag gagaggaaaa gtggaaggca gtggtctgaa tttcccagca 1320

aggtagctgt acaactgaat gatactcatc caacacttgc aattccagag ttgatgcgat 1380

tgctaatgga tgaggaagga cttggatggg atgaagcatg ggatataaca acaaggactg 1440

ttgcttatac caatcacaca gtacttcctg aagcacttga gaagtggtca caagcagtaa 1500

tgtggaagct tcttcctcgc catatggaaa taattgaaga gattgacaag agattcattg 1560

caatggtccg ctccacaagg agtgaccttg agagtaagat tcccagcatg tgcatcttgg 1620

ataataatcc caaaaagccg gttgttagga tggcaaactt atgtgtagta tctgcgcata 1680

cggtaaatgg tgttgctcag ttgcacagtg atatcttaaa ggccgacttg ttcgctgact 1740

atgtttctct atggccaaac aaactccaaa ataaaactaa tggcattact cctcgtcgat 1800

ggctccggtt ttgcaatcct gagctcagca aaattatcac aaaatggtta aaaaccgatc 1860

agtgggttac gaaccttgac ctgcttgtag gtcttcgtca gtttgctgac aacacagaac 1920

tccaagctga atgggaatct gctaagatgg ccagtaagaa acatttggca gactacatat 1980

ggcgagtaac cggtgtaacg attgatccta atagcttatt tgacatacaa gtcaagcgca 2040

ttcatgaata caagagacaa ctgctaaata ttttgggcgc aatctacaga tacaagaagt 2100

tgaaggagat gagccctcag gagcggaaga aaactactcc acgcaccatt atgtttggag 2160

ggaaagcatt tgcaacatat acaaacgcaa aaagaatagt aaagttggtt aatgatgttg 2220

gtgaagtcgt caacaccgat cctgaggtca atagttattt gaaggtggta tttgttccaa 2280

attacaatgt ctctgttgcg gagttgctta ttccaggaag tgagctatct cagcatatta 2340

gcacagcagg catggaggca agtggcacaa gcaacatgaa attttctcta aatggttgcc 2400

tcattatagg aacattggat ggagctaatg tggaaatcag gcaggagata ggagaggaga 2460

atttctttct ctttggtgca ggagcagacc aagtccctaa gctgcggaag gaaagagaag 2520

atggattgtt caaaccagat cctcggtttg aagaggccaa gcaatttata agaagtggag 2580

catttggaag ctatgactac aacccgcttc ttgattccct ggaggggaac actggttatg 2640

gtcgtggtga ttattttcta gttggttatg acttcccaag ttacttagag gctcaggaca 2700

gagttgacca agcttacaag gaccggaaga agtggctgaa gatgtctata ttaagtacag 2760

ctggcagtgg gaaattcagc agtgatcgca caattgcaca gtatgctaag gaaatctgga 2820

acataacaga atgccgtaca tcatgataaa tggaggcgct cgttgatctg agccttgccc 2880

cctgacgaac ggcggtggat ggaagatact gctctcaagt gctgaagcgg tagcttagct 2940

ccccgtttcg tgctgatcag tctttttcaa cacgtaaaaa gcggaggagt tttgcaattt 3000

tgttggttgt aacgatcctc cgttgatttt ggcctctttc tccatgggcg ggctgggcgt 3060

atttgaagcg gttctctctt ctgccgtta 3089

<210> 31
<211> 1949
<212> DNA
<213> Artificial Sequence

<220>
<223>  Synthetic Construct- Sequence No. 31, Example 31: designer
      Hexose-Kinase DNA construct (1949 bp)

<400>  31
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggat ggccgccgtc attgccaagt cctccgtctc cgcggccgtg gctcgcccgg     240

cccgctccag cgtgcgcccc atggccgcgc tgaagcccgc cgtcaaggct gcccccgtgg     300

ctgccccggc tcaggccaac cagatggcta taacaccccg ccgaaaacct tcccggaagg     360

gatcaatggc tgatatgccg aaggatgtgc ttgaccagct caagacgctg gaagagctct     420

tcacagttga ccaggagaag ctgaagcaga tcgttgagca tttcatcaag gagttacaga     480

agggcctcag tgtcgaaggc ggaaacattc ccatgaacgt gacttgggtt ctgggatttc     540

ccactggcca tgagaaaggt acatttctgg ctctggacat ggggggcacc aacctgcgcg     600

tctgcgaaat tgagctctcc gaagagaagg gcgagtttga tgtcacacag tccaagtatc     660

gaatccccga agagctcaag agcggtgaat catcagaact atgggaatat attgccgact     720

gtgtacagca gttcatagaa tactaccatg acggttgcac ggctttgcca gacctgccgc     780

tgggctttac cttttcgtac cctgctactc aagaatatgt tgaccacggt gtcctacaga     840

gatggaccaa gggttttgat attgacggcg tcgagggcaa agacgtcgtc ccaatgttag     900

aagaagcttt ggctaagaag gttaaaaatt cagctctttc cccatttttc tttggctata     960

tggtgctaat tactttacag ggtctcccca ttaaagttgc cgctctagta aacgacacga    1020

ctggcacact tattgcttcc gcctacactg acccagagat gaaaatcggc tgtatcttcg    1080

gcacaggcgt caacgccgcc tacatggaaa atgcgggctc tatccctaaa atagcccact    1140

acaatttacc tcccgacacc ccagtcgcta tcaactgcga atacggcgcc ttcgacaacg    1200

aactcattgt cctcccccga acgcagtatg acgacgtatc ccaactacgt aaaccatact    1260

ccctggactc ctccttccta gccttcatcg aagaagatcc cttcgagaac ctgtcagaaa    1320

cgcgagatct cttcgaacgc accctgggga tctacgcatt gccctcggag ctagaattct    1380

gcagacgcct ggcggaattg atcggcacac gtgccgcacg cctctccgct tgcggtgttg    1440

cggccatctg caagaagaaa aatatcaccc attgccatgt cggagcggac gggtcggtgt    1500

tcgagaagta cccgcatttc aaggccaggg gcgccagagc cctgcgggag atccttgact    1560

ggccagatag tgaaccggat cgggttgtga tgagcggagc ggaggatggg tctggcgttg    1620

gtgcggcgct tattgcggct ttgacgcttg agagggttaa acaagcttct tgggaatgga    1680

agtacatcgg aagcggtctg tcttaataaa tggaggcgct cgttgatctg agccttgccc    1740

cctgacgaac ggcggtggat ggaagatact gctctcaagt gctgaagcgg tagcttagct 1800

ccccgtttcg tgctgatcag tctttttcaa cacgtaaaaa gcggaggagt tttgcaattt 1860

tgttggttgt aacgatcctc cgttgatttt ggcctctttc tccatgggcg ggctgggcgt 1920

atttgaagcg gttctctctt ctgccgtta 1949

<210> 32
<211> 2249
<212> DNA
<213> Artificial Sequence

<220>
<223> Synthetic Construct- Sequence No. 32, Example 32: designer
      Phosphoglucomutase DNA construct (2249 bp)

<400> 32
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtccg atttctccgt ccagaccatt gccaccacgg 360

ccttcacaga ccaaaagcct ggaacctctg gtctcagaaa gaaagttact gtgtttcaac 420

agcctcacta cactgaaaac ttcattcagg ctattctcga tgccattccg gaaggtgccc 480

aaggtgccac tcttgttgta ggaggtgatg gccgtttcta caacgacaag gtcatcaact 540

tgatcgccaa aatcgcctcg gccaacggag tttccaagtt gattttgggt caagacggga 600

ttctttccac tccagcaact tcgcatgtaa tcaggatcag gggtgcaact ggaggaatta 660

ttctcactgc ttcacacaac cccggaggcc ccaaaaacga tttgggtatt aagtacaact 720

tgggaaacgg tgcaccagct ccagaatcgg ttaccaacaa gatctatgat gtctccaagg 780

aattgacttc gtacaagctc attgatttac ccgacattga tttgtccaaa acccagaccg 840

tgcaattggg ccctcttgaa gtggaaatca ttgactccac ctctgattac gtagccatgt 900

tgaaggatat ctttgacttc cccttgatca agtcgttcct cgagactgcc actaaggagc 960

agggattcaa ggttttattt gattcgctca atggtgtcac tggcccctac ggctacaaga 1020

tcttcgttga agaattagga ttgcctctta actcaatcca aaattaccac ccattgcctg 1080

actttggtgg tttacaccca gatccaaact tgacctatgc tcatactttg gtcgagaggg 1140

tcgataagga gaatattgcc tttggtgctg catctgatgg tgacggtgac agaaacatga 1200

tctacggtgc tggtaccttt gtttcgcctg gtgactctgt agccatcatc tcggaatacg 1260

ccgattccat cccttacttc aagaagcaag gtgtctacgg tttggccaga tccatgccta 1320

cctctggagc catcgatttg gtagcaaagg ctaaaggatt gaatgtttac gaagtgccaa 1380

ccggttggaa gttcttctgc aaccttttcg acgctgacaa gttgagtatc tgtggtgaag 1440

agtcgtttgg aacaggctcc aaccacatca gagaaaagga cggcctttgg gctgtagttg 1500

cctggttgaa cgtgctagca gattacaacg tcaagaatcc agaatccaag acatctattt 1560

ctgtagtgca gaactcgttt tggaagaaat acggaagaac tttcttcact agatatgact 1620

acgaaaacgt atcgtctgaa ggtgctgccg agctcatcaa cttgttgtct tctattgttg 1680

actctaagaa accaggaagt agcttagctg atggctacgt cgtcaaggaa gctgctaact 1740

tctcgtacac cgatttggac ggctctgttt cgtccaacca aggtttgttc atcaagtttg 1800

aaagcggctt gagattcata gtaagattgt ctggtactgg atcatccggt gctacagtca 1860

gattatatct cgaaaagcac tctgccgacg aatccaccta tggcttaggc gtagaccagt 1920

acttagttga tgacatcaag tttgtcttgg acttgttgaa gttcaagcag ttcttgggaa 1980

aggatgaacc agatgttcgt acctagtaaa tggaggcgct cgttgatctg agccttgccc 2040

cctgacgaac ggcggtggat ggaagatact gctctcaagt gctgaagcgg tagcttagct 2100

ccccgtttcg tgctgatcag tctttttcaa cacgtaaaaa gcggaggagt tttgcaattt 2160

tgttggttgt aacgatcctc cgttgatttt ggcctctttc tccatgggcg ggctgggcgt 2220

atttgaagcg gttctctctt ctgccgtta 2249

<210> 33
<211> 2231
<212> DNA
<213> Artificial Sequence

<220>
<223> Synthetic Construct- Sequence No. 33, Example 33: designer
      Glucosephosphate-Isomerase DNA construct (2231 bp)

<400> 33
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtcca ataactcatt cactaacttc aaactggcca 360

ctgaattgcc agcctggtct aagttgcaaa aaatttatga atctcaaggt aagactttgt 420

ctgtcaagca agaattccaa aaagatgcca agcgttttga aaaattgaac aagactttca 480

ccaactatga tggttccaaa atcttgttcg actactcaaa gaacttggtc aacgatgaaa 540

tcattgctgc attgattgaa ctggccaagg aggctaacgt caccggtttg agagatgcta 600

tgttcaaagg tgaacacatc aactccactg aagatcgtgc tgtctaccac gtcgcattga 660

gaaacagagc taacaagcca atgtacgttg atggtgtcaa cgttgctcca gaagtcgact 720

ctgtcttgaa gcacatgaag gagttctctg aacaagttcg ttctggtgaa tggaagggtt 780

ataccggtaa gaagatcacc gatgttgtta acatcggtat tggtggttcc gatttgggtc 840

cagtcatggt cactgaggct ttgaagcact acgctggtgt cttggatgtc cacttcgttt 900

ccaacattga cggtactcac attgctgaaa ccttgaaggt tgttgaccca gaaactactt 960

tgtttttgat tgcttccaag actttcacta ccgctgaaac tatcactaac gctaacactg 1020

ccaagaactg gttcttgtcg aagacaggta atgatccatc tcacattgct aagcatttcg 1080

ctgctttgtc cactaacgaa accgaagttg ccaagttcgg tattgacacc aaaaacatgt 1140

ttggtttcga aagttgggtc ggtggtcgtt actctgtctg gtcggctatt ggtttgtctg 1200

ttgccttgta cattggctat gacaactttg aggctttctt gaagggtgct gaagccgtcg 1260

acaaccactt cacccaaacc ccattggaag acaacattcc attgttgggt ggtttgttgt 1320

ctgtctggta caacaacttc tttggtgctc aaacccattt ggttgctcca ttcgaccaat 1380

acttgcacag attcccagcc tacttgcaac aattgtcaat ggaatctaac ggtaagtctg 1440

ttaccagagg taacgtgttt actgactact ctactggttc tatcttgttt ggtgaaccag 1500

ctaccaacgc tcaacactct ttcttccaat tggttcacca aggtaccaag ttgattccat 1560

ctgatttcat cttagctgct caatctcata acccaattga gaacaaatta catcaaaaga 1620

tgttggcttc aaacttcttt gctcaagctg aagctttaat ggttggtaag gatgaagaac 1680

aagttaaggc tgaaggtgcc actggtggtt tggtcccaca caaggtcttc tcaggtaaca 1740

gaccaactac ctctatcttg gctcaaaaga ttactccagc tactttgggt gctttgattg 1800

cctactacga acatgttact ttcactgaag gtgccatttg gaatatcaac tctttcgacc 1860

aatggggtgt tgaattgggt aaagtcttgg ctaaagtcat cggcaaggaa ttggacaact 1920

cctccaccat ttctacccac gatgcttcta ccaacggttt aatcaatcaa ttcaaggaat 1980

ggatgtgata aatggaggcg ctcgttgatc tgagccttgc cccctgacga acggcggtgg 2040

atggaagata ctgctctcaa gtgctgaagc ggtagcttag ctccccgttt cgtgctgatc 2100

agtctttttc aacacgtaaa aagcggagga gttttgcaat tttgttggtt gtaacgatcc 2160

tccgttgatt ttggcctctt tctccatggg cgggctgggc gtatttgaag cggttctctc 2220

ttctgccgtt a 2231

<210> 34
<211> 1709
<212> DNA
<213> Artificial Sequence

<220>
<223>  Synthetic Construct- Sequence No. 34, Example 34: designer
       oxyphotobacterial Butanol Dehydrogenase DNA construct (1709 bp)

<400>  34
agaaaatctg gcaccacacc atccaaactc gccacccgca aaccaatggc atggccgagc      60

gctgcaacgg tcgcattgcc aagattctgc gtgctgagcg ctttgtctcc gctgctgatc     120

tgcaagagac gctcacgcga tacctctggg cgtgcaatca ccgcattccc caacgcgctt     180

tgggccacat gacccccatc gagagactcc gaacgtggca aatggaggga ccagagttgt     240

tcagttcaca ggtagataat gtcgcgggtc ttgatagtta gcaataaata cagtttcaga     300

atatctgtaa tacaaaaact gtatcgagac aagaaaaaag tagcaaaatt tacaaatgtt     360

catgattcat ctggctaaat tggatgttca actgacccat tgaagacaag ggcaacaacc     420

atggagaatt ttagatttaa tgcatataca gagatgcttt ttggaaaggg acaaatagag     480

aagcttccag aggttttaaa aagatatggt aaaaatatat tacttgcata tggtggtgga     540

agtataaaaa agaatggact ctatgatact atccaaaagc tattgaaaga ttttaatatt     600

gttgaattaa gtggtattga accaaatcca agaattgaaa ctgtaagacg tggagttgaa     660

ctttgcagaa aaaataaagt agatgttatt ttagctgttg gtggagggag tacaatagac     720

tgctcaaagg ttataggggc aggttattat tatgctggag atgcatggga ccttgtaaaa     780

aatccagcta aaataggtga ggttttacca atagtgacag ttttaacaat ggcagctact     840

ggttctgaaa tgaatagaaa tgctgttatt tcaaagatgg atacaaatga aaagcttgga     900

acaggatcac ctaagatgat ccctcaaact tctattttag atccagaata tttgtataca     960

ttgccagcaa ttcaaacagc tgcaggttgt gctgatatta tgtcacacat atttgaacaa    1020

tattttaata aaactacaga tgcttttgta caagataaat ttgcggaagg tttgttgcaa    1080

acttgtataa aatattgccc tgttgcttta aaggaaccaa agaattatga agctagagca    1140

aatataatgt gggctagttc aatggctctt aacggacttt taggaagtgg gaaagctgga    1200

gcttggactt gtcatccaat agaacatgaa ttaagtgcat tttatgatat aactcatgga    1260

gtaggtcttg caattttaac tccaagttgg atgagatata tcttaagtga tgtaacagtt    1320

gataagtttg ttaacgtatg gcatttagaa caaaaagaag ataaatttgc tcttgcaaat    1380

gaagcaatag atgcaacaga aaaattcttt aaagcttgtg gtattccaat gactttaact    1440

gaacttggaa tagataaagc aaactttgaa aagatggcaa aagctgcagt agaacatggt    1500

gctttagaat atgcatatgt ttcattaaat gccgaggatg tatataaaat tttagaaatg    1560

tccctttaat aaggctgaga tcttcttcag tgcattgtag ttgaatgaag ggttaggggg    1620

gaaatgcccc cctatttttt gtctagccat cctgccacgt ttgacagggt agcaatttcg    1680

acacgatagg gttctctctt ctgccgtta                                      1709

<210>  35
<211>  1967
<212>  DNA
<213>  Artificial Sequence

<220>
<223>  Synthetic Construct- Sequence No. 35, Example 35: designer
       oxyphotobacterial Butyraldehyde Dehydrogenase DNA construct (1967
       bp)

<400>  35
agaaaatctg gcaccacacc atccaaactc gccacccgca aaccaatggc atggccgagc      60

gctgcaacgg tcgcattgcc aagattctgc gtgctgagcg ctttgtctcc gctgctgatc     120

tgcaagagac gctcacgcga tacctctggg cgtgcaatca ccgcattccc caacgcgctt     180

tgggccacat gacccccatc gagagactcc gaacgtggca aatggaggga ccagagttgt     240

tcagttcaca ggtagataat gtcgcgggtc ttgatagtta gcaataaata cagtttcaga     300

atatctgtaa tacaaaaact gtatcgagac aagaaaaaag tagcaaaatt tacaaatgtt     360

catgattcat ctggctaaat tggatgttca actgacccat tgaagacaag ggcaacaacc     420

atgattaaag acacgctagt ttctataaca aaagatttaa aattaaaaac aaatgttgaa     480

aatgccaatc taaagaacta caaggatgat tcttcatgtt tcggagtttt cgaaaatgtt     540

gaaaatgcta taagcaatgc cgtacacgca caaaagatat tatcccttca ttatacaaaa     600

gaacaaagag aaaaaatcat aactgagata agaaaggccg cattagaaaa taaagagatt     660

ctagctacaa tgattcttga agaaacacat atgggaagat atgaagataa aatattaaag     720

catgaattag tagctaaata cactcctggg acagaagatt taactactac tgcttggtca     780

ggagataacg ggcttacagt tgtagaaatg tctccatatg gcgttatagg tgcaataact     840

ccttctacga atccaactga aactgtaata tgtaatagta taggcatgat agctgctgga     900

aatactgtgg tatttaacgg acatccaggc gctaaaaaat gtgttgcttt tgctgtcgaa     960

atgataaata aagctattat ttcatgtggt ggtcctgaga atttagtaac aactataaaa    1020

aatccaacta tggactctct agatgcaatt attaagcacc cttcaataaa actactttgc    1080

ggaactggag ggccaggaat ggtaaaaacc ctcttaaatt ctggtaagaa agctataggt    1140

gctggtgctg gaaatccacc agttattgta gatgatactg ctgatataga aaaggctggt    1200

aagagtatca ttgaaggctg ttcttttgat aataatttac cttgtattgc agaaaaagaa    1260

gtatttgttt ttgagaacgt tgcagatgat ttaatatcta acatgctaaa aaataatgct    1320

gtaattataa atgaagatca agtatcaaag ttaatagatt tagtattaca aaaaaataat    1380

gaaactcaag aatactctat aaataagaaa tgggtcggaa aagatgcaaa attattctta    1440

gatgaaatag atgttgagtc tccttcaagt gttaaatgca taatctgcga agtaagtgca    1500

aggcatccat ttgttatgac agaactcatg atgccaatat taccaattgt aagagttaaa    1560

gatatagatg aagctattga atatgcaaaa atagcagaac aaaatagaaa acatagtgcc 1620

tatatttatt caaaaaatat agacaaccta aataggtttg aaagagaaat cgatactact 1680

atctttgtaa agaatgctaa atcttttgcc ggtgttggtt atgaagcaga aggctttaca 1740

actttcacta ttgctggatc cactggtgaa ggaataactt ctgcaagaaa ttttacaaga 1800

caaagaagat gtgtactcgc cggttaataa ggctgagatc ttcttcagtg cattgtagtt 1860

gaatgaaggg ttagggggga aatgcccccc tattttttgt ctagccatcc tgccacgttt 1920

gacagggtag caatttcgac acgatagggt tctctcttct gccgtta 1967

<210> 36
<211> 1602
<212> DNA
<213> Artificial Sequence

<220>
<223> Synthetic Construct- Sequence No. 36, Example 36: designer
      oxyphotobacterial Butyryl-CoA Dehydrogenase DNA construct (1602
      bp)

<400> 36
agaaaatctg gcaccacacc tgatctgcaa gagacgctca cgcgatacct ctgggcgtgc 60

aatcaccgca ttccccaacg cgctttgggc cacatgaccc ccatcgagag actccgaacg 120

tggcaaatgg agggaccaga gttgttcagt tcacaggtag ataatgtcgc gggtcttgat 180

agttagcaat aaatacagtt tcagaatatc tgtaatacaa aaactgtatc gagacaagaa 240

aaaagtagca aaatttacaa atgttcatga ttcatctggc taaattggat gttcaactga 300

cccattgaag acaagggcaa caaccatgaa tttccaatta actagagaac aacaattagt 360

acaacaaatg gttagagaat tcgcagtaaa tgaagttaag ccaatagctg ctgaaatcga 420

cgaaacagaa agattcccta tggaaaacgt tgaaaaaatg gctaagctta aaatgatggg 480

tatcccattt tctaaagaat ttggtggagc aggcggagat gttctttcat atataatagc 540

tgtggaagaa ttatcaaaag tttgtggtac tacaggagtt attctttcag cgcatacatc 600

attatgtgca tcagtaatta atgaaaatgg aactaacgaa caaagagcaa aatatttacc 660

tgatctttgc agcggtaaaa agatcggtgc tttcggatta actgaaccag gtgctggtac 720

agatgctgca ggacaacaaa caactgctgt attagaaggg gatcattatg tattaaatgg 780

ttcaaaaatc ttcataacaa atggtggagt tgctgaaact ttcataatat ttgctatgac 840

agataagagt caaggaacaa aaggaatttc tgcattcata gtagaaaagt cattcccagg 900

attctcaata ggaaaattag aaaataagat ggggatcaga gcatcttcaa ctactgagtt 960

agttatggaa aactgcatag taccaaaaga aaacctactt agcaaagaag gtaagggatt 1020

tggtatagca atgaaaactc ttgatggagg aagaattggt atagctgctc aagctttagg 1080

tattgcagaa ggagcttttg aagaagctgt taactatatg aaagaaagaa aacaatttgg 1140

taaaccatta tcagcattcc aaggattaca atggtatata gctgaaatgg atgttaaaat 1200

ccaagctgct aaatacttag tatacctagc tgcaacaaag aagcaagctg gtgagcctta 1260

ctcagtagat gctgcaagag ctaaattatt tgctgcagat gttgcaatgg aagttacaac 1320

taaagcagtt caaatctttg gtggatatgg ttacactaaa gaatacccag tagaaagaat 1380

gatgagagat gctaaaatat gcgaaatcta cgaaggaact tcagaagttc aaaagatggt 1440

tatcgcagga agcattttaa gataaggctg agatcttctt cagtgcattg tagttgaatg 1500

aagggttagg ggggaaatgc ccccctattt tttgtctagc catcctgcca cgtttgacag 1560

ggtagcaatt tcgacacgat agggttctct cttctgccgt ta 1602

<210> 37
<211> 1248
<212> DNA
<213> Artificial Sequence

<220>
<223> Synthetic Construct- Sequence No. 37, Example 37: designer
      oxyphotobacterial Crotonase DNA construct (1248 bp)

<400> 37
agaaaatctg gcaccacacc tgatctgcaa gagacgctca cgcgatacct ctgggcgtgc 60

aatcaccgca ttccccaacg cgctttgggc cacatgaccc ccatcgagag actccgaacg 120

tggcaaatgg agggaccaga gttgttcagt tcacaggtag ataatgtcgc gggtcttgat 180

agttagcaat aaatacagtt tcagaatatc tgtaatacaa aaactgtatc gagacaagaa 240

aaaagtagca aaatttacaa atgttcatga ttcatctggc taaattggat gttcaactga 300

cccattgaag acaagggcaa caaccatgga attaaaaaat gttattcttg aaaaagaagg 360

gcatttagct attgttacaa tcaatagacc aaaggcatta aatgcattga attcagaaac 420

actaaaagat ttaaatgttg ttttagatga tttagaagca gacaacaatg tgtatgcagt 480

tatagttact ggtgctggtg agaaatcttt tgttgctgga gcagatattt cagaaatgaa 540

agatcttaat gaagaacaag gtaaagaatt tggtatttta ggaaataatg tcttcagaag 600

attagaaaaa ttggataagc cagttatcgc agctatatca ggatttgctc ttggtggtgg 660

atgtgaactt gctatgtcat gtgacataag aatagcttca gttaaagcta aatttggtca 720

accagaagca ggacttggaa taactccagg atttggtgga actcaaagat tagcaagaat 780

agttggacca ggaaaagcta aagaattaat ttatacttgt gaccttataa atgcagaaga 840

agcttataga ataggcttag ttaataaagt agttgaatta gaaaaattga tggaagaagc 900

aaaagcaatg gctaacaaga ttgcagctaa tgctccaaaa gcagttgcat attgtaaaga 960

tgctatagac agaggaatgc aagttgatat agatgcagct atattaatag aagcagaaga 1020

ctttgggaag tgctttgcaa cagaagatca aacagaagga atgactgcgt tcttagaaag 1080

aagagcagaa aagaattttc aaaataaata aggctgagat cttcttcagt gcattgtagt 1140

tgaatgaagg gttagggggg aaatgccccc ctattttttg tctagccatc ctgccacgtt 1200

tgacagggta gcaatttcga cacgataggg ttctctcttc tgccgtta 1248

<210> 38
<211> 1311
<212> DNA
<213> Artificial Sequence

<220>
<223> Synthetic Construct- Sequence No. 38, Example 38: designer
      oxyphotobacterial 3-Hydroxybutyryl-CoA Dehydrogenase DNA
      construct (1311 bp)

<400> 38
agaaaatctg gcaccacacc tgatctgcaa gagacgctca cgcgatacct ctgggcgtgc 60

aatcaccgca ttccccaacg cgctttgggc cacatgaccc ccatcgagag actccgaacg 120

tggcaaatgg agggaccaga gttgttcagt tcacaggtag ataatgtcgc gggtcttgat 180

agttagcaat aaatacagtt tcagaatatc tgtaatacaa aaactgtatc gagacaagaa 240

aaaagtagca aaatttacaa atgttcatga ttcatctggc taaattggat gttcaactga 300

cccattgaag acaagggcaa caaccatgaa aaagattttt gtacttggag caggaactat 360

gggtgctggt atcgttcaag cattcgctca aaaaggttgt gaggtaattg taagagacat 420

aaaggaagaa tttgttgaca gaggaatagc tggaatcact aaaggattag aaaagcaagt 480

tgctaaagga aaaatgtctg aagaagataa agaagctata ctttcaagaa tttcaggaac 540

aactgatatg aagttagctg ctgactgtga tttagtagtt gaagctgcaa tcgaaaacat 600

gaaaattaag aaggaaatct ttgctgagtt agatggaatt tgtaagccag aagcgatttt 660

agcttcaaac acttcatctt tatcaattac tgaagttgct tcagctacaa agagacctga 720

taaagttatc ggaatgcatt tctttaatcc agctccagta atgaagcttg ttgaaattat 780

taaaggaata gctacttctc aagaaacttt tgatgctgtt aaggaattat cagttgctat 840

tggaaaagaa ccagtagaag ttgcagaagc tccaggattc gttgtaaacg gaatcttaat 900

cccaatgatt aacgaagctt cattcatcct tcaagaagga atagcttcag ttgaagatat 960

tgatacagct atgaaatatg gtgctaacca tccaatggga cctttagctt taggagatct 1020

tattggatta gatgtttgct tagctatcat ggatgtttta ttcactgaaa caggtgataa 1080

caagtacaga gctagcagca tattaagaaa atatgttaga gctggatggc ttggaagaaa 1140

atcaggaaaa ggattctatg attattctaa ataaggctga gatcttcttc agtgcattgt 1200

agttgaatga agggttaggg gggaaatgcc cccctatttt ttgtctagcc atcctgccac 1260

gtttgacagg gtagcaattt cgacacgata gggttctctc ttctgccgtt a 1311

<210>  39
<211>  1665
<212>  DNA
<213>  Artificial Sequence

<220>
<223>  Synthetic Construct- Sequence No. 39, Example 39: designer
       oxyphotobacterial Thiolase DNA construct (1665 bp)

<400>  39
agaaaatctg gcaccacacc tgatctgcaa gagacgctca cgcgatacct ctgggcgtgc      60

aatcaccgca ttccccaacg cgctttgggc cacatgaccc ccatcgagag actccgaacg     120

tggcaaatgg agggaccaga gttgttcagt tcacaggtag ataatgtcgc gggtcttgat     180

agttagcaat aaatacagtt tcagaatatc tgtaatacaa aaactgtatc gagacaagaa     240

aaaagtagca aaatttacaa atgttcatga ttcatctggc taaattggat gttcaactga     300

cccattgaag acaagggcaa caaccatggg caaagaaagt agttttagct gtgcatgtcg     360

tacagccatc ggaacaatgg gtggatctct tagcacaatt cctgcagtag atttaggtgc     420

tatcgttatc aaagaggctc ttaaccgcgc aggtgttaaa cctgaagatg ttgatcacgt     480

atacatggga tgcgttattc aggcaggaca gggacagaac gttgctcgtc aggcttctat     540

caaggctggt cttcctgtag aagtacctgc agttacaact aacgttgtat gtggttcagg     600

tcttaactgt gttaaccagg cagctcagat gatcatggct ggagatgctg atatcgttgt     660

tgccggtggt atggaaaaca tgtcacttgc accatttgca cttcctaatg gccgttacgg     720

atatcgtatg atgtggccaa gccagagcca gggtggtctt gtagacacta tggttaagga     780

tgctctttgg gatgctttca atgattatca tatgatccag acagcagaca acatctgcac     840

agagtggggt cttacacgtg aagagctcga tgagtttgca gctaagagcc agaacaaggc     900

ttgtgcagca atcgaagctg gcgcattcaa ggatgagatc gttcctgtag agatcaagaa     960

gaagaaagag acagttatct tcgatacaga tgaaggccca agacagggtg ttacacctga    1020

atctctttca aagcttcgtc ctatcaacaa ggatggattc gttacagctg gtaacgcttc    1080

aggtatcaac gacggtgctg cagcactcgt agttatgtct gaagagaagg ctaaggagct    1140

cggcgttaag cctatggcta cattcgtagc tggagcactt gctggtgttc gtcctgaagt    1200

tatgggtatc ggtcctgtag cagctactca gaaggctatg aagaaggctg gtatcgagaa    1260

cgtatctgag ttcgatatca tcgaggctaa cgaagcattc gcagctcagt ctgtagcagt    1320

tggtaaggat cttggaatcg acgtccacaa gcagctcaat cctaacggtg gtgctatcgc    1380

tcttggacac ccagttggag cttcaggtgc tcgtatcctt gttacacttc ttcacgagat    1440

gcagaagaaa gacgctaaga agggtcttgc tacactttgc atcggtggcg gtatgggatg    1500

cgctactatc gttgagaagt acgaataagg ctgagatctt cttcagtgca ttgtagttga    1560

atgaagggtt aggggggaaa tgccccccta ttttttgtct agccatcctg ccacgtttga 1620

cagggtagca atttcgacac gatagggttc tctcttctgc cgtta 1665

<210> 40
<211> 4071
<212> DNA
<213> Artificial Sequence

<220>
<223> Synthetic Construct- Sequence No. 40, Example 40: designer
      oxyphotobacterial Pyruvate-Ferredoxin Oxidoreductase DNA
      construct (4071 bp)

<400> 40
agaaaatctg gcaccacacc tgatctgcaa gagacgctca cgcgatacct ctgggcgtgc 60

aatcaccgca ttccccaacg cgctttgggc cacatgaccc ccatcgagag actccgaacg 120

tggcaaatgg agggaccaga gttgttcagt tcacaggtag ataatgtcgc gggtcttgat 180

agttagcaat aaatacagtt tcagaatatc tgtaatacaa aaactgtatc gagacaagaa 240

aaaagtagca aaatttacaa atgttcatga ttcatctggc taaattggat gttcaactga 300

cccattgaag acaagggcaa caaccatggc gcagaggtgc aaggagcccg tcgacggaac 360

gacagccacg acgcacgtgg cctacttcat gagcgacagc gcgttcatct tccccatcac 420

gcccagctcg gtcatgtccg aggtcgccca cgagtggtcc atgaacggcc gcaagaacgc 480

cttcggccag cccacgatgg tccgccagat gcagagcgag gctgggtctg ccggcgccct 540

gcacggcgcg ctcagcgagg gagcgctggc gacgacgttc acgagcagcc agggcctgct 600

gctcatgatc cccaacatgt acaagatcgc cggcgagctc ctgccctgcg tcatgcacat 660

cgccgcccgc accgtcgcca ccgaggccct ctctatcttc ggcgaccaca cggatgtcta 720

cgcggtgagg tcgacggggt tcgcgttcct gtgctccgcg accgtccagg agtgcatcca 780

catgtccgcc gccgcgcacg ccgccaccct gtccagcgag gtcccgttcg cccacttctt 840

cgacggcttc cgcacgtccc acgagatcca gaagatcgac ttcccctcgg acgccgacct 900

gctggcctgc atgaactttg acgacgtccg caggttccgt ggccgctcgc tgtgctgcga 960

gcgcccgctg ctgcgcggga cggcgcagaa ccccgacgtc ttcatgcagg cgtccgagtc 1020

gaacctggcg acgctggcca gggtccccgc ggccatcgac gaggcgctgg ctcgtgtgaa 1080

caaggtgttc gggaccaact acaggaccta cgagtactat ggccaccccg aggccacgga 1140

cgtgatcgtg gccatgggaa gcggcaccga agtggccatc tcgactgcca acttcctcaa 1200

ctcgcgcgac gcgaactcga gggtcggcgt cgtgagggtg cggctgttcc ggccgtttgt 1260

gtcggcggcg tttgtggctg cgctgcccaa gaccgtcaag aggatctgcg ttctggaccg 1320

cgggagggac gggcaggcgg ccgcggaccc cctgcaccag gacgtcctgt cggcgctggg 1380

tctggcagcg cccgggaggg ttcaggtgtg cgtgggaggc gtgtacggtc tgtcgtccaa 1440

ggacttcaac cccgaccacg tgatcgccgt gtacaggaac ctcgcgtcgg cgagccccaa 1500

gaacaggttc agcgtcggca tcgtcgacga cgtgacgcac aacagcctgg acatgggaga 1560

gcacgtggac gcgctgccgc aggggacgaa gcagtgcctg ctgtggggca tcggcggaga 1620

cgggaccatc ggggcgaaca agacggccat caagctgatc gcggaccaca cggagctgca 1680

cgcgcagggg tactttgcgt acgacgccaa caaggccggc ggcctgacag tctcgcacct 1740

gcggttcggc ccgacgcggt tcgaggcgcc gtacctggtg aacgacagca actacgtggc 1800

gtgccacaac ttctcgtacg tgcacaggtt caacctgctg tcgtcgctgc gcaccggggg 1860

cacgttcgtg ctcaactgcc cgtgccggac cgtggaggag ctggacacgg cactcccggt 1920

gcgcctgagg cgcgagatcg ccaggcggca ggccaagttc tatgtgatcg acgcgaccaa 1980

gatcgccaag gacaacggga tgggcccgtt catcaacatg gtcctccagg ccgtgttctt 2040

ctatctgtcc cacgtgctcg atgtgaacga ggcagtggca ctcctgaaga agagcatcca 2100

gaagatgtac gcgcgcaagg gcgaggaggt tgtcaggaag aacgtggcat cggtcgacgc 2160

gtcgctggat cccaaggcgt tgctgcacat cgagtacccc gcagacaggt ggcttgcgct 2220

ggccgacgag cacgtgcccc gcatgggtct gctcactgtc cccgagcgcc tgcagaagtt 2280

caacgccgag ctgtacgagc cgaccctcgc gtacgatggg gagagcatcc cggtcagcag 2340

gttccctcgc ggcggcgaga cgccgacggg cacgactcag ctgggcaagc gtggcatcgc 2400

cgagagcgtg ccgcactgga accacgagaa gtgcgtgcag tgcaaccagt gctcgttcgt 2460

gtgcccgcac gccgtcatcc ggtcgtacca gatcagcgag gaggagatga agaacgcccc 2520

tgccggcttc gacactctta agtcgcgcaa gcccgggtat cgtttccgca tcaacgtcag 2580

cgccctggac tgcactggct gcagcgtgtg cgtggagcag tgcccagtca agtgcctgga 2640

gatgaagcct ctcgagtccg agttcgagat gcagaaggac gccatcaggt tcgtccgcga 2700

gatggtcgcg cccaagcccg agctgggaga ccgcaagact cccgtcggca tcgcgtctca 2760

cacgccgctg ttcgagttcc cgggagcctg cgccgggtgc ggtgagaccc cgctggtgcg 2820

cctcgtgacg cagatgttcg gtgagcgcat ggtcatcgcc gcggccactg ggtgcaactc 2880

gatctgggga gcgtcgttcc cgaacgtgcc gtacacaacc aacgcccgcg gggagggccc 2940

cgcgtggcac aactcgctgt tcgaggacgc ggcggagctc gggtatggca ttacgtgtgc 3000

gtatcgccag cgccgcgagc gcctcatcgg catcgtgcgg agcgtcgtcg acgatgcggg 3060

atccgtgcag ggtctgtctg ctgagctgaa ggctctgctg gtcgagtggc tcgcgcacgt 3120

cagggacttc gagaagaccc gcgagctccg cgacaggatg aaccccctga tcgacgcaat 3180

cccagcgaac gcggactgca gggttctgga gctcagggag aagcacaacc gcgagctgat 3240

cgcgcgcacg agtttctgga tcctcggtgg cgacgggtgg gcgtacgaca tcggcttcgg 3300

tggactggac cacgtgatcg ccaacaacga ggacgtcaac atccttgttc tcgacacgga 3360

ggtctactcc aacactggtg gccagcgctc caagtcgacg ccgctcggcg cccgcgccaa 3420

gtacgctgtg ctgggcaagg acactgggaa gaaggacctg gggcgcatcg cgatgaccta 3480

cgagaccgcg tacgtggcca gcatcgcgca gggagccaac cagcagcagt gcatggacgc 3540

gctgagggag gccgaggcct accagggccc ctcgatcgtc attgcgtaca ctccgtgcat 3600

ggagcaccag atggtccgcg ggatgaagga gagccagaag aaccagaagc tggctgtgga 3660

gacgggctac tggctgctgt accgcttcaa ccccgacctc atccacgagg gcaagaaccc 3720

cttcaccctc gactcgaagc ctccctcgaa gcctcccaag gagttcctgg acacgcaggg 3780

ccgtttcatt actctgcagc gcgagcaccc cgagcaggcc cacctccttc acgaggcact 3840

cacccgctct ctggccaccc gcttcgtgcg ctaccagcgc ctcgtgcagc tgtacgagcc 3900

cgctgcccct gccgcagctc ctgccacgca ttaaggctga gatcttcttc agtgcattgt 3960

agttgaatga agggttaggg gggaaatgcc cccctatttt ttgtctagcc atcctgccac 4020

gtttgacagg gtagcaattt cgacacgata gggttctctc ttctgccgtt a 4071

<210> 41
<211> 1806
<212> DNA
<213> Artificial Sequence

<220>
<223> Synthetic Construct- Sequence No. 41, Example 41: designer
      oxyphotobacterial Pyruvate Kinase DNA construct (1806 bp)

<400> 41
agaaaatctg gcaccacacc tgatctgcaa gagacgctca cgcgatacct ctgggcgtgc 60

aatcaccgca ttccccaacg cgctttgggc cacatgaccc ccatcgagag actccgaacg 120

tggcaaatgg agggaccaga gttgttcagt tcacaggtag ataatgtcgc gggtcttgat 180

agttagcaat aaatacagtt tcagaatatc tgtaatacaa aaactgtatc gagacaagaa 240

aaaagtagca aaatttacaa atgttcatga ttcatctggc taaattggat gttcaactga 300

cccattgaag acaagggcaa caaccgtgtt cactaaaatt gtagctacat tggggccttc 360

gactgataga ctgccggata taacggccct gttgagcaag gttcacggcg tgcggataaa 420

tatgtctcac gcatcgccat cggaggtaga ggcccgcgtg aacgccgtga ggaagtatga 480

ggagaccagc gggaggtata tagccattat agcggatcta aggggcccca gcgtcaggac 540

cggccttatg cgccctctac agataacggc gggcgcccgc gtctccttta aattagccga 600

gaagggggac ggcttcgtac ctgtgccgcg gcgtgagttc ttcgaagtaa tcgaggaggg 660

agacgaggtt cttatgttag acggaaaact cgtcttgagg ataatcagcg cagcgcagac 720

ctcggccgag gccgagtcgt tatcctccgg cgtcatatcc agcaataagg caatagtggt 780

caaaggcaag gaatatcata tagagcagcc tgtggaggaa gacataaggg cgcttcagac 840

gctctctcgg ttcagagacg acgtagacta cgtggccctc agccttgtga gagacggagc 900

agacgtgagg aaaatgagga gcgtcgtcga ggaggctggg ctcacctccg gcataatggc 960

caaaatagag acgaagagcg cagtagataa aatcgaggag ataatcaatg cggccgacta 1020

catagttata gcgagaggcg atctggcgct gcactacgga ctggagtaca ttcctaaagt 1080

acagaggctc ttggtggaga gatctctctc ggcaggaagg cccgtggcgg tggccacgca 1140

gcttttggac tctatgcaga ccaacacgac gcccactagg gcggaggtca acgacgtgta 1200

cacaacggcg agtctcggag tggactctct gtggctgacc aacgagactg cgagcggaga 1260

gcacccgtta gaggcagtgg attggctgag gaggatagtg tcgcaggtcg agttcgggag 1320

acttaaggct gcgtcgccgg ccgacgcacg cgataggttc gccaaagccg tggtagatat 1380

ggccgaggac atgggagggg aaatcgcagt atactcaatg acgggaactc tggcgaagag 1440

aatagctaaa tttaggccga tgacgacagt ctacgtcgga gtcaacgaga ggaggctcgc 1500

gaggatgttg gagctccgcg aggatgttgg agctcatatg gggcctagag cctgtggtcg 1560

tgccggcgca tacttacgag gagggcctcg agaggctcct ctccagattc tccgacaaag 1620

tcttgatagc cacgtatggg ctcagaggcg gcacacatac tattaataag gctgagatct 1680

tcttcagtgc attgtagttg aatgaagggt taggggggaa atgcccccct attttttgtc 1740

tagccatcct gccacgtttg acagggtagc aatttcgaca cgatagggtt ctctcttctg 1800

ccgtta 1806

<210> 42
<211> 1696
<212> DNA
<213> Artificial Sequence

<220>
<223> Synthetic Construct- Sequence No. 42, Example 42: designer
      oxyphotobacterial Enolase DNA construct (1696 bp)

<400> 42
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac cttatttttt cttcaagtta aagaatgcgt tcattccagg ataaacggca 300

atgctgccaa gctcttcttc aattctcaag agctgattgt attttgctac tctgtctgtt 360

cttgacggtg cacctgtctt tatctgacca gcatttactg caacaacaag gtcagcaatt 420

gttgtatctt cagtctcacc tgatctgtgg gatacaactg cagtgtagcc tgctctattt 480

gccatttcaa tagcttctaa agtttctgta agtgttccta tctgattaag cttaatcaat 540

attgagtttg caacgccaag ttctattccc tttgcaagcc tctttgtgtt tgtaacaaac 600

aaatcatcac ccacaagctg aatcttcttg ccaagtgctt cagttagcat cttccagcct 660

tcccagtcct cttctgcaac accgtcttca attgatacaa ttgggtactt ttcaacaagt 720

tttacccaga attctaccat ttcttctttt gttctaactt taccttctct ttcgaaatga 780

tactttccat cttcttcatt gtagagctca gatgttgcag ggtcaagcgc aattgcaata 840

tccttaccag gagtataacc agctttttca attgcttcga caattacttc caatggctct 900

tcgttagact tcaagtttgg tgcaaatcca ccttcatcac ccactgttgt gttgtatcct 960

cttgccttca atacatttct taattgatgg aatgtctcag cacacatcct gagtgcttcg 1020

ctaaaagatt ttgcaccaac tggcattatc ataaactctt gtaggtcaac agagttgtca 1080

gcatgctttc caccgttcaa aatattcatc attggcacag gtaaatactt tgcattgaca 1140

ccaccaatgt attggtacag tggaagacca agtgcgtttg ccgctgcctt cgcaactgcc 1200

aaagatacac ccaaaattgc atttgcacca agcttgctct tgttctctgt cccatcaagc 1260

tcaatcataa gcctgtcaat ctcaacttgg ttaagagcgt tcattccaat tatttctggc 1320

gcaataacct cgtttacatt ttcgactgct ttgagaaccc cttttcccat atatcttttt 1380

ttatcaccgt ctctgagttc aacagcctcg aacatacctg ttgacgcacc tgatggaaca 1440

gcagctctac ctacaaattc atcatttaca acaacttcta cttcaacagt tgggtttcct 1500

cttgaatcca gaatttctct tgcttttaca gctgtaattg aaagatcaac cttcattaag 1560

gctgagatct tcttcagtgc attgtagttg aatgaagggt taggggggaa atgcccccct 1620

attttttgtc tagccatcct gccacgtttg acagggtagc aatttcgaca cgatagggtt 1680

ctctcttctg ccgtta 1696

<210> 43
<211> 2029
<212> DNA
<213> Artificial Sequence

<220>
<223> Synthetic Construct- Sequence No. 43, Example 43: designer
 oxyphotobacterial Phosphoglycerate-Mutase DNA construct (2029 bp)

<400> 43
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac cctacgccgg cgcctgcttc tcctcctgcc ggcagcactt ctccaaaggg 300

tgcacgttcg cttctcttgt aatcagggtc tggccggtca tctcggccgg tttcgggatg 360

cccagcaggt gcaggatggt gggggccaca tcccgcaggc tgccgtcccg cagcgcaatg 420

ccggcggtat cccgcccgat caggatgaac ggcaccgggc tggtggtgtg ggccgtatga 480

ggctgtccct cttcgtccac catctcatcc gcattgccgt ggtctgccgt tatcaggagc 540

gtgccgtcct tttccaggac ggcccgcgcc acctttccaa ggcagcggtc gattgtttct 600

atggccttta ccgttgcctt catgtcgccg gtatgcccga ccatgtcggg attggcgtaa 660

ttcattatga ttacgtcgta cttgcccgag gccagccgct ccagaaaggt gccggtgacc 720

tcgttggcgc tcatttcggg cttcaggtcg taggtggcca cccgcgggga gggcaccagg 780

atcctgtctt cgccggggta tggcttttct aagccgccgt tgaagaagaa ggtcacatgg 840

gcgtactttt ccgtttcggc caggcggagc tgggtcatgc cgtgcctgct taaaacctcg 900

cccagggtat tgcgcagctc ctgcggctga aacgccaccg gcgccttaat ggtcttgtcg 960

taaagggtca tgcaggtaaa atgcacggca gggtagccct gctttctggc aaacccggtg 1020

aaatcctcgt ccacaaaggc cctggtaatc tggcgggccc ggtccggccg gaagttaaag 1080

aaaataacgg cgtcgccctt cattattttg gcggccggcc cacccgaccc gtttaccacg 1140

acggtgggct ggataaactc gtcggtttca tcccttccgt accccaggtc aaccgcctcc 1200

agcgggcttg ttgcctgaat gccctcgcct aaaaccattg cgttgtacgc ccgctcggtg 1260

cggtcccagc ggcggtctct gtccatggcg taatagcgcc ccattaccgt tgccaccgcc 1320

ccaaagccca gttcgcccag cttcttcctt aactgctcga agtattcttt tgcgttggcc 1380

ggcggcacgt cgcgcccgtc caggaaggca tggacaaaga cgttgcgcat gttctcgcgg 1440

gcggccaggt ccaggagggc gaaaaggtgg ctgatatggc tgtgcactcc gccgtccgat 1500

aaaagcccca tcaggtgaag ggccttatta ttctccctgg cgtatctcac cgcctccagc 1560

aggacttcgt tcttgaaaaa ggtcccgtcc ttgatggcgc ggcttattct ggtaagctcc 1620

tggtacacca ccctgccggc gcctatgttc aagtgtccca cctcggaatt gcccatctgg 1680

ccctcgggaa gccccacgtc ctcgccggaa cagctcaggg cacagtgggg gtaaccggcc 1740

agaaagctct tgaaattcgg tgtgctggcc agggctatgg cattgccccg gacattggaa 1800

ctgaggcccc agccgtccag aaccaccagc accaggggcc tgccgccggc ataccggccg 1860

cagggcgttg cagctacgtc ttccttcaat aaggctgaga tcttcttcag tgcattgtag 1920

ttgaatgaag ggttaggggg gaaatgcccc cctatttttt gtctagccat cctgccacgt 1980

ttgacagggt agcaatttcg acacgatagg gttctctctt ctgccgtta 2029

<210> 44
<211> 1687
<212> DNA

<213>  Artificial Sequence

<220>
<223>  Synthetic Construct- Sequence No. 44, Example 44: designer
       oxyphotobacterial Phosphoglycerate Kinase DNA construct (1687 bp)

<400>  44
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac cttatttatc gagcagcgcc cttactcccg gcagttgctt cccttccaga     300

aactccaggg aagcgccgcc gccggttgag atatgggtca ttttgccggc tacgccggcc     360

ttcttggccg ccgccgccgt gtcaccgccg ccgattacgg tgacggcgtt taattcggcc     420

agcgtccggg ctattgcttc ggtgcccctg gcaaaaggat ccatttcaaa aacgcccatt     480

ggtccgttcc agaccacggt cctggccgcc ctgagggctt cggtgaaaag tctgatggac     540

tcgggcccta tatccagggc catccactcc gccgggattt gatcgaccgg caccgtcctt     600

tgctcctggc cgggcgccgg ccccggcgcc accaccacat ccaccggcag gaggagcttt     660

acttccctgc ttctggcttc tgcaatcagc ttcctggcca ggtcaatctt gtcggcctcc     720

agcagggact taccgacgct gtacccttgt gccttcagaa aggtattggc catcccgccg     780

ccaatgataa ccgtatcgac tttggtcagc aggttgaaaa ttactcccag cttgtcggaa     840

actttcgagc cgcccacgac ggctgcaaaa gggcgctccg ggctggtcag cagcctgccc     900

agtatttcca gctctttttc catcagcagg cctgccacgg ccggcaaaaa cccggcaacg     960

ccctcggtgg aggcgtgggc ccggtgtgcg gttccaaacg catcgtttac aaagacatct    1020

gccagctcag ccagttgccg ggcaaacttc tcgtcgtttt tctcctcctc cgggtggaaa    1080

cggacgtttt ccagcagcac cacgtccccg tcctgcatct gggcaacggc ggacctggcg    1140

gcttctccca cgcagtcgcc ggccttaacc accgttttcc ccagcagttc ggaaaggcgc    1200

ctggcaacgg gatccatttt gtacctctcg tccaccctgc ccttgggccg gcccaggtgc    1260

gaaaccagaa taaccctggc tttttgtccg ataaggtagt ttatggtggg cacggcctcc    1320

tttattttaa cgtcatcggc cacccggccg ttttccatcg gcacgttgaa gtccacccgc    1380

aacaggaccc gcttgccctt tacatctata tcccttaccg tttttttggc cactaaggct    1440

gagatcttct tcagtgcatt gtagttgaat gaagggttag gggggaaatg cccccctatt    1500

ttttgtctag ccatcctgcc acgtttgaca gggtagcaat ttcgacacga tagcgtgctg    1560

tactgttttt tgctcgtcag ggttgggttt tgtcatcgac acccaaggat tggagtcggt    1620

gctcaataat cgccagttgc tgttgggcag ccgccaattg cgcctgaggt tctctcttct    1680

gccgtta                                                              1687

<210>  45
<211>  1514
<212>  DNA
<213>  Artificial Sequence

<220>
<223>  Synthetic Construct- Sequence No. 45, Example: designer
       oxyphotobacterial Glyceraldehyde-3-Phosphate Dehydrogenase DNA
       construct (1514 bp)

<400>  45
agaaaatctg gcaccacacc tgatctgcaa gagacgctca cgcgatacct ctgggcgtgc      60

aatcaccgca ttccccaacg cgctttgggc cacatgaccc ccatcgagag actccgaacg     120

tggcaaatgg agggaccaga gttgttcagt tcacaggtag ataatgtcgc gggtcttgat     180

agttagcaat aaatacagtt tcagaatatc tgtaatacaa aaactgtatc gagacaagaa     240

aaaagtagca aaatttacaa atgttcatga ttcatctggc taaattggat gttcaactga     300

cccattgaag acaagggcaa caaccaatgg atttgggcgg atcggacgtt tagcattcag     360

aagaattcaa gatgtagaag gtcttgaagt agttgcagtt aacgacttaa cagatgacga     420

tatgttagct catttattaa aatacgatac tatgcaaggt cgtttcactg gagaagttga     480

agttatcgaa ggtggattcc gtgttaacgt aaaagaaatt aaatcattcg atgaccagat     540

gctgggtaaa ttaccatggg gcgatttaga tatcgacgta gtattagaat gtactggttt     600

ctatactgat aaagaaaaag cacaagctca catcgatgca ggtgctaaaa aagtattaat     660

ctcagctcca gctaaaggtg atgtaaaaac aatcgtattc aacactaacc atgacgcatt     720

agacggttca gaaacagttg tttcaggtgc ttcttgtact actaactcat tagcaccagt     780

tgcaaaagtt ttaagtgatg aattcggttt agttgaaggt ttcatgacta caattcacgc     840

ttacactggt gaccaaaata cacaagacgc acctcacaga aaaggtgaca aacgtcgtgc     900

acgtgcagca gcagaaaata ttatccctaa ctcaacaggt gctgctaaag ctatcggtaa     960

agttattcca gaaatcgatg gtaaattaga cggtggagca caacgtgttc cagttgctac    1020

tgggtcttta actgaattaa ctgtagtatt agacaaacaa gatgtaactg ttgaccaagt    1080

taacagtgct atgaaacaag cttcagacga atcattcggt tacactgaag acgaaatcgt    1140

atcttctgat atcgttggta tgacttacgg ttcattattc gatgcgactc aaactcgtgt    1200

tatgactgtt ggagatcgtc aattagttaa agttgcagct tggtacgaca aagagtgggg    1260

taaggctgag atcttcttca gtgcattgta gttgaatgaa gggttagggg ggaaatgccc    1320

ccctattttt tgtctagcca tcctgccacg tttgacaggg tagcaatttc gacacgatag    1380

cgtgctgtac tgttttttgc tcgtcagggt tgggttttgt catcgacacc caaggattgg    1440

agtcggtgct caataatcgc cagttgctgt tgggcagccg ccaattgcgc ctgaggttct    1500

ctcttctgcc gtta                                                    1514

<210>  46
<211>  609
<212>  DNA
<213>  Artificial Sequence

<220>
<223>  Synthetic Construct- Sequence No. 46, Example 46: designer
       Nial-promoter-controlled Proton-Channel DNA construct (609 bp)

<400>  46
agaaaatctg gcaccacacc tatatggtag ggtgcgagtg accccgcgcg acttggagct      60

cgatggcccc gggttgtttg gggcgtccgc ctctcgcgct attctgagct ggagaccgag     120

gcgcatgaaa atgcattcgc ttccatagga cgctgcattg tggcttgaag gttcaaggga     180

agggttcaaa cgaccccgcc gtacgaactt ttgtcggggg gcgctcccgg ccccgggctc     240

ttgtgcgcgc attagggctt cgggtcgcaa gcaagacgat acatggccgg catcggcgcc     300

gtgctgaagg tcctgaccac cggcctgccc gccctgatca gctggatcaa gcgcaagcgc     360

cagcagtaaa tggaggcgct cgttgatctg agccttgccc cctgacgaac ggcggtggat     420

ggaagatact gctctcaagt gctgaagcgg tagcttagct ccccgtttcg tgctgatcag     480

tctttttcaa cacgtaaaaa gcggaggagt tttgcaattt tgttggttgt aacgatcctc     540

cgttgatttt ggcctctttc tccatgggcg ggctgggcgt atttgaagcg gttctctctt     600

ctgccgtta                                                             609

<210>  47
<211>  1360
<212>  DNA
<213>  Artificial Sequence

<220>
<223>  Synthetic Construct- Sequence No. 47, Example 47: designer
       nirA-promoter-controlled NAD-dependent
       Glyceraldehyde-3-Phosphate-Dehydrogenase DNA construct (1360 bp)

<400>  47
agaaaatctg gcaccacacc cttcttgcag aacatgcatg atttacaaaa agttgtagtt      60

tctgttacca attgcgaatc gagaactgcc taatctgccg agtatatgaa cggatttggc     120

aggataggac gactggtgtt gcgggcggcg gtggagaagg gcacggtgga ggtggtggcg     180

gtgaacgatc cgttcatctt cccggacgcg gcgtacgctg cgtacatgct gcagtacgac     240

tcgacgcacg gggcgttccc gggtgaggtg ggcagcgacg gggagcactt ggtggtgaac     300

gggaagaagc tggcgtgctt tgcgatccgc gatccggcgg agatcccgtg gggctcggtc     360

ggcgccgact acgtcgtgga gtccaccggc gtgttcaccg tgaccgagaa ggcgtcgttg     420

cacgtcaagg gcggcgcgaa gaaggtggtt atatcggcgc cgtcgaagga tgcgcccatg     480

tttgtgatgg gcgtgaacca tgacgcctac accaaggact tgacggtggt gtcgaatgcg 540

tcttgcacca ccaacttgtt tggcgccgct ggccaagatc atcgacgagg cgttcggcat 600

cgggatgggc ctcatgagca ccatccacgc ggtgacggcc acgcaaaaga cggtggatgg 660

gccgagctcc aaagactggc gcggtgtcgc ggcgcgttcc agtcgattat tcccagcagc 720

accggcgctg cgaaagcggt cggcaaggtg tacccgaagc tgaacggcaa gctgaccggc 780

atggcgttcc gcgtgccggt gcccgacgtg tccgtggtag acttgacagt gaccctgaag 840

aaggagacca actacgagga gatcaaaaag gctgtcaagc aggcgtcgca gagcccgcac 900

tacaagggca tcgtggcgta caccgagcac cccatcgtgt cggccgacct ggtgcacaac 960

ccgtactcgt cggtgttcga tgccgaagcc ggtatcatgc tgtcgcccac gtttgtgaaa 1020

ctggtcagct ggtaatagtg atcccggccg ctactaaagc ctgatttgtc ttgatagctg 1080

ctcctgcctt tgggcagggg cttttttctg tctgccattc ttgaggatgg cggactcttt 1140

cccttttgct ctacgcccat gaatgcgatc gcagtctccc ctgtccagca cgttggagtg 1200

attggtggtg gccagttagc ttggatgctg gcaccagcag cgcaacagtt ggggatgtcg 1260

ctgcacgttc aaacacccaa tgatcacgac ccagcagtag cgatcgcgga tcaaaccgta 1320

ttagcagcag ttgctgacgc ggttctctct tctgccgtta 1360

<210> 48
<211> 1621
<212> DNA
<213> Artificial Sequence

<220>
<223> Syntheitc Construct- Sequence No. 48, Example 48: designer
      nirA-promoter-controlled Phosphoglycerate-Kinase DNA construct
      (1621 bp)

<400> 48
agaaaatctg gcaccacacc cttcttgcag aacatgcatg atttacaaaa agttgtagtt 60

tctgttacca attgcgaatc gagaactgcc taatctgccg agtatatgtc atttgtcttc 120

gagcgcgacg acacccggca gctgttttcc ttccataaac tcgagcgaag cgccgccgcc 180

ggtggagata tgatccattt tgtcggccaa gccgaatttc tcaaccgccg ccgccgaatc 240

cccgccgccg atgaccgaat aggtgtcggg cgcttccgcc agtgcttcgg cgatcgcttt 300

tgtcccatgg gcgaacgctt ccatttcaaa gacgcccatc gggccgttcc agacaacgag 360

cttcgattga cgaatgacat cgcggtacaa ttcgcgcgtt ttcgggccga tgtcaagcgc 420

ctcccaatcg ctcggaatgg cgtcgatggc gacgactttc gtgttggcgt cgttcgcaaa 480

ccggtcggcg acgaccacgt ccaccggcat ataaaaacgg acgccttttt ctttcgcctt 540

ttccataaac gatttggcga gttcgatttt gtcctcctca agcagcgact tgccgacgtc 600

atggccgagc gctttgacga acgtatacgc cagtccgccg ccgatgatca agttgtcgac 660

tttttcaagc aaattgtcga tgacgccgat tttgtctttc actttcgcgc cgccgatgat 720

cgccgtaaac gggcggtccg gattcgagag cgctttgccg agcacttcga gttctttttc 780

catcaaaaac ccggccaccg caggcaagta atgggcgatg ccttccgtcg acgcatgagc 840

gcggtgggcg gcgccgaacg catcgttgac atacagatcc gcgagctccg caaacgcttt 900

ggccagctct ggatcgtttt tctcttcgcc agggtaaaaa cggacgttct caagcaagag 960

cacgtcgcct tcgttcaaac ggtcgaccgc cgctttcacc tcatcgccga ccgcttcatt 1020

cgttttggcg accggccgtt caagcagctc gccgagccgc ttcgcaacgg catccaaacg 1080

caattcttcg accacttttc ctttcggcgg gccgaggtgg ctcgccaaaa tgactttcgc 1140

cccgtgctcg atcaaatagc ggatcgtcgg gagtgcggcg cgaatgcgcg tgtcatcggt 1200

gatggcgcct tgctccatcg gaacgttgaa atcgacgcgg caaaagacgc gctttcccct 1260

cacctcaacg tcgcggatcg tcttcttgtt cattaatagt gatcccggcc gctactaaag 1320

cctgatttgt cttgatagct gctcctgcct ttgggcaggg gcttttttct gtctgccatt 1380

cttgaggatg gcggactctt tcccttttgc tctacgccca tgaatgcgat cgcagtctcc 1440

cctgtccagc acgttggagt gattggtggt ggccagttag cttggatgct ggcaccagca 1500

gcgcaacagt tggggatgtc gctgcacgtt caaacaccca atgatcacga cccagcagta 1560

gcgatcgcgg atcaaaccgt attagcagca gttgctgacg cggttctctc ttctgccgtt 1620

a 1621

<210> 49
<211> 1990
<212> DNA
<213> Artificial Sequence

<220>
<223> Synthetic Construct- Sequence No. 49, Example 49: designer
      nirA-promoter-controlled Phosphoglycerate-Mutase DNA construct
      (1990 bp)

<400> 49
agaaaatctg gcaccacacc cttcttgcag aacatgcatg atttacaaaa agttgtagtt 60

tctgttacca attgcgaatc gagaactgcc taatctgccg agtatatgct cgagcattta 120

tttaataata agcgagcttc ccttcatctc tgaaggtttt tctaacccta agatgtctaa 180

gattgttgga gcaatgtctg ctaagattcc atcatctctt aatttaacat tgccatatcc 240

cacaagatac aaaggcacct tatttgttgt atgagctgta tgaggctcac ctgtctcata 300

atcaatcatc tgttcacagt tgccatggtc agcagtaata ataaccactc cacccttttc 360

taaaaccttg ttaacaactt ttccaataca ctcatctaca gcctcaactg cctttattgc 420

agcctctaaa acgcctgtgt gccctaccat gtcaccattt gcatagttac atattatcac 480

atcatattca tctctttcaa ttctctcaag taaagcttct gttacctcgt atgcactcat 540

ctcaggttta agatcatatg ttgcaacctt tggtgatggt accaataccc tgtcttctcc 600

gacatttggt acttccacac cgccgttgaa gaaaaaggtg acatgagcat acttttctgt 660

ctcagcaatt cgaagttgtt ttaaccctaa cttgctaaaa tactctccca aagtgtttgt 720

caggttctct ggtttgaatg caacatggca attttttatt gtcacatcat actgagtcat 780

gcatacaaag aacacttcga aatatccttt tttcctttca aaaccgtcaa attcaacatc 840

acaaaacgct cttgtaagct gtcttgctct gtcaggtctg aagttaaaga aaataatact 900

gtcatgttca tttattgttg cgacaggttt tccattttca agcacaacag tcggaattac 960

aaactcatca gtgttacctt ttttatacga cttttcaacc gcctctaatc ctgagcttgc 1020

atactcgcct tcaccaaaga ccattgcatt atatgccttt tcaactcttt cccatctttt 1080

gtctctgtcc attgcatagt atctgcccat cactgttgca atcttaccac aaccaatttc 1140

ttttatcttc tgttcaagct cttcaatgta aatttttgcg ctcgaaggtg gaacatctcg 1200

cccatccaaa aagcaatgaa catatacttt ttcaagattg tgcctctttg caagttttaa 1260

aagtgcgtaa agatgtgtgt tgtggctgtg aacaccacca tctgataaaa gtcccatcag 1320

atgaagagaa gagttatatt ttttgcaatt ctctattgcc atcaaaaact cttctttttc 1380

aaaaaaatca ccgtctttaa ttgactttgt tattcttgta aattcttggt aaacaattct 1440

tcctgcaccc aggttcagat gtccaacttc agaattcccc atttgtcctt cgggaagacc 1500

aacatccata ccactgctac caatcagggt atatgggtaa ttcttttcgt aatagtcaag 1560

gttaggggtc ttacccaaag caacagcgtt tccctcttgc tttgggttat aaccccaacc 1620

gtccatgata atcaacacaa caggtttttt cattaatcta gataatagtg atcccggccg 1680

ctactaaagc ctgatttgtc ttgatagctg ctcctgcctt tgggcagggg cttttttctg 1740

tctgccattc ttgaggatgg cggactcttt cccttttgct ctacgcccat gaatgcgatc 1800

gcagtctccc ctgtccagca cgttggagtg attggtggtg gccagttagc ttggatgctg 1860

gcaccagcag cgcaacagtt ggggatgtcg ctgcacgttc aaacacccaa tgatcacgac 1920

ccagcagtag cgatcgcgga tcaaaccgta ttagcagcag ttgctgacgc ggttctctct 1980

tctgccgtta 1990

<210> 50
<211> 1765
<212> DNA
<213> Artificial Sequence

<220>
<223> Synthetic Construct- Sequence No. 50, Example 50: designer
 nirA-promoter-controlled Enolase DNA construct (1765 bp)

<400> 50
agaaaatctg gcaccacacc cttcttgcag aacatgcatg atttacaaaa agttgtagtt 60

tctgttacca attgcgaatc gagaactgcc taatctgccg agtatatgct cgagcatatg 120

ctaaataaac ccgtcgtttc cattgaagaa attaccgcta gagaaatttt agactctcgt 180

ggccgtccta ccattgaagc agaagtctta ctggaaacag gggctttcgg tattgcccag 240

gttcccagtg gcgcgtcaac tggtagcttc gaggcccacg aattacggga tgatgacccc 300

aaccgctacg gtggtaaagg cgttctcaaa gcggttagta acgttataga cgaaattgcc 360

cctaaaatta tcggaatgga tgggttagat caaactgcga tcgatcacac catgattgag 420

ttagacggtt ctactaataa aaaagaatta ggggccaatg ctatccttgc cgtttcctta 480

gccactgcaa aagctgccgc cgatgaatta gcccttcccc tgtaccgtta tttagggggt 540

cccttggcca atgtcttacc cgtccccatg atgaacgtga ttaacggggg ttctcacgcg 600

gataataacg tagacttcca ggagtttatg attatgccag tgggtgcgga ctcttttaaa 660

gaagctttga ggtggggggc cgaagtgttt gcttccctca gtaaagttct aaaagagcgt 720

aaattgctct ctggggtagg agacgagggg ggatacgccc cgaacctggg atcgaaccag 780

gaagccttag atttgctcat agaagccatt gaaaaggcgg ggtataagcc aggggaacag 840

gtggctttag cgatggatgt ggcttcaagt gagttttata aggatggcga atatatttat 900

gatggttctc cccattcccc tcaagaattt atcgattatt taggtaaatt agtggatcaa 960

tatcctatta tttccattga agatggctta caagaagatg actgggatag ctggaaaagt 1020

ttgaccgata cgttaggatc tcgcattcag ttagttgggg acgatctttt tgtcacgaac 1080

cccactcgtc tgcaaaaagg cattgatatg ggtgtgggta atagtattct cattaaactc 1140

aatcaaattg gtagtttaac ggaaacgtta gatacgattg ctttagcgac tcgtcatcaa 1200

tatagttccg ttatttccca tcgttccgga gaaaccgaag acactaccat tgcagactta 1260

gccgtagcta cacgcgctgg acaaatcaaa accggttctc tgtgtcgtag tgaacgggta 1320

gccaaatata accgactatt acgtattgaa gaagaattag gcgatcgcgc agtttatgct 1380

gcaaaagtgg gtttaggccc tcaataaggc tgctgccccg gctgctgcta atctagataa 1440

tagtgatccc ggccgctact aaagcctgat ttgtcttgat agctgctcct gcctttgggc 1500

aggggctttt ttctgtctgc cattcttgag gatggcggac tctttccctt ttgctctacg 1560

cccatgaatg cgatcgcagt ctcccctgtc cagcacgttg gagtgattgg tggtggccag 1620

ttagcttgga tgctggcacc agcagcgcaa cagttgggga tgtcgctgca cgttcaaaca 1680

cccaatgatc acgacccagc agtagcgatc gcggatcaaa ccgtattagc agcagttgct 1740

gacgcggttc tctcttctgc cgtta 1765

<210> 51

<211>  1888
<212>  DNA
<213>  Artificial Sequence

<220>
<223>  Synthetic Construct- Sequence No. 51, Example 51: designer
       nirA-promoter-controlled Pyruvate-Kinase DNA construct (1888 bp)

<400>  51
agaaaatctg gcaccacacc cttcttgcag aacatgcatg atttacaaaa agttgtagtt      60

tctgttacca attgcgaatc gagaactgcc taatctgccg agtatatgct cgagcatatg     120

ttaaaaaaga cgaaaatcgt ttgcacgcag ggtccgtcca cagagaaacc gggcgtaatt     180

gatgcactga ttgccaatgg catgaactgc gcacgcttca atttctccca tggtgaccac     240

gaagaacatc ttggccgtat caatatggtt cgtgaagctg ccaagaaggc tggcaaggtt     300

atctctttaa tcctcgatac caaaggtccg gaaatgcgtc tgggcgagtt caaagatggc     360

aaagttatgc tcgaaaaggg caacaagttc actttgacct atgacgatga accgggtgat     420

gaaactcatg tttccgtaaa ccacaaaggt ctttacacgg aagttaagcc gggcgacacc     480

ctgctcctct ccgatggcct cgtagctctc aaagttgatg aaatcaaggg caaggatatc     540

gttacgacga ttcagaacag cggtaagatg agcacgcgca agcgcgtagc tgctccgggc     600

gtaccccttg gtctgcctcc tatctccgaa caggatgcta aggacatcat ctttggctgc     660

gaacaggata tggatttcgt agctgcttcc ttcatccagc gtccggatga tgttatcgcc     720

atccgcaagc tcatcgaaga gcacaatggc cacatggaaa ttctgccgaa gatcgaaaac     780

ctcgaaggtg ttaagaactt cgatgcaatc ctggaagttt ccgacggcat catggttgcc     840

cgtggtgacc tgggcgtaga agttccggca gaagatgtgc cccttattca gaaggaaatc     900

atccgcaagt gcaacgctgc tggcaagccg gttatcgttg ctacgcagat gctcgactcc     960

atggaacgca acccgcgtcc gacccgtgca gaagtttctg acgttggtaa cgccatcctc    1020

gatggtacgg atgccatcat gctgtccggc gaaacggctt ccggtgacta tccggtagaa    1080

gcagttgcca cgatgaaccg cattgcacag cgcatggaaa gctcccttga atacaaggaa    1140

ctctatgtag aacgtggtct gcagcacatg gaatcccgta cgcgtgctat cgctcatgct    1200

acggttcaga tggcttatga gctcgatgct ccggctatta tcacgccgac cgaatccggt    1260

tacacgacga aggtcgtttc caagtatcgt ccgaaggctg ctatcgtagc ttacacgccg    1320

agcgaaaaag ttctgcgtca gctgaacctg cgttggggcg tatatccggt actcggcacc    1380

cagtggagcg atgtggatga aatgatcagc aatgcaacgg ctgctgctgt taaggaagac    1440

ctcgtacagc gcggcgacct caccatcatc acctccggtg tgaagatgga atcccgtacg    1500

cgtgctatcg ctcatgctac ggacatctaa ggctgctgcc ccggctgctg ctaatctaga    1560

taatagtgat cccggccgct actaaagcct gatttgtctt gatagctgct cctgcctttg    1620

ggcaggggct tttttctgtc tgccattctt gaggatggcg gactctttcc cttttgctct 1680

acgcccatga atgcgatcgc agtctcccct gtccagcacg ttggagtgat tggtggtggc 1740

cagttagctt ggatgctggc accagcagcg caacagttgg ggatgtcgct gcacgttcaa 1800

acacccaatg atcacgaccc agcagtagcg atcgcggatc aaaccgtatt agcagcagtt 1860

gctgacgcgg ttctctcttc tgccgtta 1888

<210> 52
<211> 2188
<212> DNA
<213> Artificial Sequence

<220>
<223> Synthetic Construct- Sequence No. 52, Example 52: designer
      nirA-promoter-controlled Pyruvate-Decarboxylase DNA construct
      (2188 bp)

<400> 52
agaaaatctg gcaccacacc cttcttgcag aacatgcatg atttacaaaa agttgtagtt 60

tctgttacca attgcgaatc gagaactgcc taatctgccg agtatatgct cgagcatatg 120

gtatcaacct acccagaatc agaggttact ctaggaaggt acctctttga gcgactccac 180

caattgaaag tggacaccat tttcggcttg ccgggtgact tcaacctttc cttattggac 240

aaagtgtatg aagttccgga tatgaggtgg gctggaaatg ccaacgaatt gaatgctgcc 300

tatgctgccg atggttactc cagaataaag ggattgtctt gcttggtcac aacttttggt 360

gttggtgaat tgtctgcttt aaacggagtt ggtggtgcct atgctgaaca cgtaggactt 420

ctacatgtcg ttggagttcc atccatatcg tcacaggcta aacagttgtt gctccaccat 480

accttgggta atggtgactt cactgttttt cacagaatgt ccaatagcat ttctcaaact 540

acagcatttc tctcagatat ctctattgca ccaggtcaaa tagatagatg catcagagaa 600

gcatatgttc atcagagacc agtttatgtt ggtttaccgg caaatatggt tgatctcaag 660

gttccttcta gtctcttaga aactccaatt gatttgaaat tgaaacaaaa tgatcctgaa 720

gctcaggaag aagttgttga aacagtcctg aagttggtgt cccaagctac aaaccccatt 780

atcttggtag acgcttgtgc cctcagacac aattgcaaag aggaagtcaa acaattggtt 840

gatgccacta attttcaagt ctttacaact ccaatgggta aatctggtat ctccgaatct 900

catccaagat ttggcggtgt ctatgtcggg acaatgtcga gtcctcaagt caaaaaagcc 960

gttgaaaatg ccgatcttat actatctgtt ggttcgttgt tatcggactt caatacaggt 1020

tcattttcat actcctacaa gacgaagaat gttgttgaat tccactctga ctatatgaaa 1080

atcagacagg ccaccttccc aggagttcaa atgaaagaag ccttgcaaca gttgataaaa 1140

agggtctctt cttacatcaa tccaagctac attcctactc gagttcctaa aaggaaacag 1200

ccattgaaag ctccatcaga agctcctttg acccaagaat atttgtggtc taaagtatcc 1260

ggctggttta gagagggtga tattatcgta accgaaactg gtacatctgc tttcggaatt 1320

attcaatccc attttcccag caacactatc ggtatatccc aagtcttgtg ggctcaattt 1380

ggtttcacag taggtgcaac agttggtgct gccatggcag cccaggaaat cgaccctagc 1440

aggagagtaa ttttgttcgt cggtgatggt tcattgcagt tgacggttca ggaaatctct 1500

acgttgtgta aatgggattg taacaatact tatctttacg tgttgaacaa tgatggttac 1560

actatagaaa ggttgatcca cggcaaaagt gccagctaca acgatataca gccttggaac 1620

catttatcct tgcttcgctt attcaatgct aagaaatacc aaaatgtcag agtatcgact 1680

gctggagaat tggactcttt gttctctgat aagaaatttg cttctccaga taggataaga 1740

atgattgagg tgatgttatc gagattggat gcaccagcaa atcttgttgc tcaagcaaag 1800

ttgtctgaac gggtaaacct tgaaaattga ggctgctgcc ccggctgctg ctaatctaga 1860

taatagtgat cccggccgct actaaagcct gatttgtctt gatagctgct cctgcctttg 1920

ggcaggggct tttttctgtc tgccattctt gaggatggcg gactctttcc cttttgctct 1980

acgcccatga atgcgatcgc agtctcccct gtccagcacg ttggagtgat tggtggtggc 2040

cagttagctt ggatgctggc accagcagcg caacagttgg ggatgtcgct gcacgttcaa 2100

acacccaatg atcacgaccc agcagtagcg atcgcggatc aaaccgtatt agcagcagtt 2160

gctgacgcgg ttctctcttc tgccgtta 2188

<210> 53
<211> 1510
<212> DNA
<213> Artificial Sequence

<220>
<223> Synthetic Construct- Sequence No. 53, Example 53: designer
nirA-promoter-controlled NAD(P)H-dependent Alcohol-Dehydrogenase
DNA construct (1510 bp)

<400> 53
agaaaatctg gcaccacacc cttcttgcag aacatgcatg atttacaaaa agttgtagtt 60

tctgttacca attgcgaatc gagaactgcc taatctgccg agtatatgtt agctacctct 120

gtgccagaaa cccaaaaggg tgttattttc tatgagaatg gtggtaaatt ggaatacaag 180

gacattccag ttccaaagcc aaagccaaat gaaatcttga tcaacgtcaa gtactccggt 240

gtgtgtcata ccgatttgca cgcatggaag ggtgactggc cattgccaac caagttgcca 300

ttggtcggtg gtcacgaagg tgctggtgtc gttgttgcta tgggtgaaaa cgtcaagggc 360

tggaacattg gtgactttgc gggtatcaaa tggttgaacg gttcttgtat gtcctgtgaa 420

tactgtgaat tgtccaatga atccaactgt ccagatgctg acttgtctgg ttacacccac 480

gatggttctt tccaacaata ccgtaccgca gatgctgttc aagctgccag aattccaaag 540

ggtaccgatt tggctgaagt tgctccaacc ctatgtgccg gtgttactgt ttacaaggct 600

ttgaaaagtg ctaacttgaa ggctggtgac tgggttgcca tctctggtgc tgctggtggt 660

ctaggttctc tagctgtcca atacgccaag gccatgggtt acagagtcgt tggtatcgac 720

ggtggtgaag aaaagggtaa gttggtcaag caattgggtg gtgaagcctt tgttgatttc 780

accaaaacca aggacatggt tgctgaaatc caagaaatca ccaacggtgg tccacacggt 840

gtcattaacg tctctgtttc tgaagctgcc atgaacgctt ccactcaatt cgtcagacca 900

actggtactg tcgtattggt cggtttgcca gctggtgccg tcatcaagtc cgaagtcttc 960

tcccacgtcg ttaagtctat taacatcaag ggttcttacg tcggtaacag agctgacacc 1020

agagaagcta tcaacttctt cgctaacggt cacgtccact ctccaatcaa ggttgttggt 1080

ttgtccgaac taccaaaggt ttacgaattg atggaacaag gtaagatttt gggtagatac 1140

gttgttgaca cctccaacta gggctgctgc cccggctgct gctaatagtg atcccggccg 1200

ctactaaagc ctgatttgtc ttgatagctg ctcctgcctt tgggcagggg cttttttctg 1260

tctgccattc ttgaggatgg cggactcttt cccttttgct ctacgcccat gaatgcgatc 1320

gcagtctccc ctgtccagca cgttggagtg attggtggtg gccagttagc ttggatgctg 1380

gcaccagcag cgcaacagtt ggggatgtcg ctgcacgttc aaacacccaa tgatcacgac 1440

ccagcagtag cgatcgcgga tcaaaccgta ttagcagcag ttgctgacgc ggttctctct 1500

tctgccgtta 1510

<210> 54
<211> 56
<212> PRT
<213> Artificial Sequence

<220>
<223> Synthetic Construct- Sequence No. 54, Example 54: designer
selected Hyd1 transit peptide (amino acids sequence)

<400> 54

Met Ser Ala Leu Val Leu Lys Pro Cys Ala Ala Val Ser Ile Arg Gly
1               5                   10                  15

Ser Ser Cys Arg Ala Arg Gln Val Ala Pro Arg Ala Pro Leu Ala Ala
            20                  25                  30

Ser Thr Val Arg Val Ala Leu Ala Thr Leu Glu Ala Pro Ala Arg Arg
        35                  40                  45

Leu Gly Asn Val Ala Cys Ala Ala
    50                  55

<210> 55

<211>  45
<212>  PRT
<213>  Artificial Sequence

<220>
<223>  Synthetic Construct- Sequence No. 55, Example 55: designer
       selected RbcS2 transit peptides (amino acids sequence)

<400>  55

Met Ala Ala Val Ile Ala Lys Ser Ser Val Ser Ala Ala Val Ala Arg
1               5                   10                  15

Pro Ala Arg Ser Ser Val Arg Pro Met Ala Ala Leu Lys Pro Ala Val
            20                  25                  30

Lys Ala Ala Pro Val Ala Ala Pro Ala Gln Ala Asn Gln
        35                  40                  45

<210>  56
<211>  7
<212>  PRT
<213>  Artificial Sequence

<220>
<223>  Synthetic Construct- Sequence No. 56, Example 56: designer
       selected ferredoxin transit peptide (amino acids sequence)

<400>  56

Met Ala Met Ala Met Arg Ser
1               5

<210>  57
<211>  33
<212>  PRT
<213>  Artificial Sequence

<220>
<223>  Synthetic Construct- Sequence No. 57, Example 57: designer
       selected CF0CF1 subunit-a transit peptide (amino acids sequence)

<400>  57

Met Leu Ala Ala Lys Ser Ile Ala Gly Pro Arg Ala Phe Lys Ala Ser
1               5                   10                  15

Ala Val Arg Ala Ala Pro Lys Ala Gly Arg Arg Thr Val Val Val Met
            20                  25                  30

Ala

<210>  58
<211>  1417
<212>  DNA

<213>  Artificial Sequence

<220>
<223>  Synthetic Construct- Sequence No. 58, Exmaple 58:  designer
      nirA-promoter-controlled NADPH-dependent
      Glyceraldehyde-3-Phosphate Dehydrogenase DNA construct (1417 bp)

<400>  58
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgacttgc acttacagtt tcttttgatg tcaaaagtgc tccaatttgc     300

tcagcaacat ctacaactct atttgaataa ccccattcat tatcatacca agcaataact     360

tttactttat tccctgacat gaccattgtt gattttgcat caataatagc tgaatttgga     420

ttagtattaa aatcaacaga cactagtggt tgatgttcga cttctatgat accttctaaa     480

cctgcatttt caaaagcttg gtttacttct tctgcagtta cttctttttc taaatcaaca     540

actaaatcaa cgagcgatac attctttgtt ggtacacgta atgccatgcc gtgtaattta     600

ccttctaatt ctggtaatac ttcttttaaa gctttcgccg caccagtaga agtaggaata     660

atgctttcat tacatgaacg tgcacgtctt aaatctttat gtggattatc aatatttttt     720

tggtcatttg taatagcgtg aacagtagtc attaaaccat taactattcc aaactgatta     780

tttaaaactt ttgcaactgg accaatgcaa ttagtagtac atgaagcatt actaaaaatg     840

tcaaatgctt ctatatctaa ttggttatca tttacgcctt taactaacat ttgaacatgt     900

ccaccttttg aaggaccagt taacaatact tttttggcac ctgctttaat atgtgcgatg     960

gctttatcac catgattaaa tttaccagtt gcatctatag caatatcgat atctaattct    1020

ttccatggca agttttcagg attgcgatca gcaaccaatt taattttatg atcaccaact    1080

tgcaatccat tttcaatcgg ttcaactttt agattatatt ttccatgtgt tgtatcgtaa    1140

ttgattaaat gtgcaattgt ttcgggtgga taactagcat ttatcgctac tacatttaaa    1200

tttttatttt gtaatgcaat acgtaatacc attcttccaa ttctacccat accattaatt    1260

gcaatattcg ttgacattaa ggctgagatc ttcttcagtg cattgtagtt gaatgaaggg    1320

ttagggggga aatgcccccc tattttttgt ctagccatcc tgccacgttt gacagggtag    1380

caatttcgac acgatagggt tctctcttct gccgtta                             1417


<210>  59
<211>  1387
<212>  DNA
<213>  Artificial Sequence

<220>

<223>  Synthetic Construct- Sequence No. 59, Exmaple 59: designer
      nirA-promoter-controlled NAD-dependent Glyceraldehyde-3-Phosphate
      Dehydrogenase DNA construct (1387 bp)

<400>  59
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgcttaga gatgtgtgcg atcagatcca gaactttgtt ggagtagcca     300

gtttcgttgt cataccaaga aaccagtttc acgaagttgt cgttcagaga gataccggcg     360

tcggcatcaa acacggaggt gcatacttcg ccgttgaagt cggtagaaac gacggcttcg     420

tcggtgtagc ccagaacgcc tttcatttcg ccttcagaag cggccttcat cacgtcacag     480

atctgctgat aggtcgccgg cttggccagg cgagcagtca ggtcaacaac ggagacgttc     540

ggggtcggaa cgcggaaagc cataccggtc agtttgccgt tcagctccgg gatgaccttg     600

ccaacggcct tggctgcacc ggtagaggac gggatgatgt tctggctagc gccgcggccg     660

ccgcgccaat ctttcatgga cgggccgtcg acagttttct gggtcgcggt ggtagcgtga     720

acggtggtca tcagcgcttc aacgatgccg aagttgtcgt tcaggacttt agccagcgga     780

gccaggcagt tggtggtgca ggatgcgttg gaaacgatct cctggccagc gtagttcttg     840

tggtttacgc ccataacgaa catcggggta gcatctttag acgggccagt catgacgact     900

ttcttggcac cggcggcgat gtgcttacgc gcggtttcgt cggtcaggaa cagaccggtc     960

gcttcggcaa caacgtcaac gccgatttcg ttccacttca ggttagccgg atctctttca    1020

gcggtaacac ggattttttt accgttaacg atcaggtggc catctttcac ttcaacagtg    1080

ccgttgaaac gaccgtgagt agagtcgtac ttcagcatgt atgccatgta gttggcatcc    1140

agcagatcgt tgatgccaac gatttcgatg tcagaacgtt cctgagcagc acggaaaaca    1200

atacggccga tacggccaaa accgttgata cctactttga tagtcattaa ggctgagatc    1260

ttcttcagtg cattgtagtt gaatgaaggg ttagggggga aatgcccccc tattttttgt     1320

ctagccatcc tgccacgttt gacagggtag caatttcgac acgatagggt tctctcttct    1380

gccgtta                                                              1387

<210>  60
<211>  1627
<212>  DNA
<213>  Artificial Sequence

<220>
<223>  Synthetic Construct- Sequence No. 60, Exmaple 60: designer
      nirA-promoter-controlled phosphoglycerate mutase DNA construct
      (1627 bp)

<400>  60
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgggcccc gaacttttgc agcttgcccg cgtgcgccag caggagcggc     300

agcacgtggg tgccgtgcag gtcgccgagc gcgccccctg ccgcctcgtc ctcggtgaag     360

cggcgggccg cgtcggcgcg caggttgggg ccgtagagca ggaagggcac cgggtgccag     420

gagtgcgcct tcatcacgct gggcgtggag tggtcgccgg tgatggcgat cacctcgggc     480

tccagcgcca gcagctcggg cagcagggcg tcgaaggcct cgatcttgtg caccttcgcg     540

tcgaagtcgc cgtcctcgcc ggtggagtcg gtcttcttga agtgcaggta gaagtagtcg     600

aaggcttccc agtgggcctt cagggccgcg agcttgcctt cgggggcgtc ggggtcctcg     660

cccacctcga cctccagcgc ggtcatcccc accagcgagg ccaccccgcg gtacatcggg     720

tagctggcga ccgccgcggg ggtgagcccg gtgatctcgc ccagggtggg ccagaccggg     780

cgcttggaga cgccgcgaag cagcaccccg ttgatcctgg gctcgtccgc gagcgcctgc     840

cgcgccagcg cgctgaactt gttgagcacg tcggccgtct tggcgctggg ctcgtcgctt     900

tcgtcgtgcg gacgggccgc gagcggcggc accccggtct tctgggggtc ggtgtcgtgg     960

acccggtcgc ccagccccac gccccgcagc accagcacga agcggtgctc cgactcggtg    1020

tggagctcga tcctgacgcc gtcgatctcg cggatgcgct ccctcagctt ggcgagcacc    1080

cgccggtttt cctcggtggg ggggcggccg gcgcggcggt cggcgatggt gccgtcgggg    1140

ttcaaggtgg cgaagttgcc gcgcaccgcg acgtcgtcgg gccccagctc gatgcccagc    1200

cccagcgccg agaggacgcc gcgcccgacc tcgtagcgga aggggtcgta gccgaagagg    1260

ctcaggtggc cgggcccgga gccgggcgcg aagccggggg ccaccagggt gactcgcccc    1320

aggttggcct tttcggccag ggcgtcgagg ttgggggtgc gggccgcggc cagctccgtg    1380

ggcccgcccg gctcgcgggg cagcccgccc accccgtcga gcacgacgaa gaggatcttg    1440

ctgggcgtgg tgcggctcag ttccttgagg tgggggagga ggtccattaa ggctgagatc    1500

ttcttcagtg cattgtagtt gaatgaaggg ttagggggga aatgcccccc tattttttgt    1560

ctagccatcc tgccacgttt gacagggtag caatttcgac acgatagggt tctctcttct    1620

gccgtta                                                              1627

<210>  61
<211>  1678
<212>  DNA
<213>  Artificial Sequence

<220>
<223>  Synthetic Construct- Sequence No. 61, Exmaple 61: designer
       nirA-promoter-controlled Enolase DNA construct (1678 bp)

<400>  61
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgtttttg taaattatag aaaaccccca acccacgata cagcccgata     300

tcgcttaatt cttcttctat ccgcaacagc tggttgtatt tcgctacccg gtcagtcctg     360

gccggagccc cggtctttat ctgccctacg ttggtcgcta ctaccaggtc agcgatgaag     420

gtgtcctccg tctccccgga acggtgggat actacacagg tgtaaccggc tcttttagcc     480

atttcaatcg tatccaaggt ctcagtgacc gtgcctatct gattcagttt gataagtatg     540

ctgttggcca cacccttttt tattcctgtg gtaaaacgct ggggattggt tacgaaaata     600

tcatctccga cgatctgaat cttcttgccc agcttacggg tgagcttttt ccacccttcc     660

cagtcttctt cctgcagccc gtcttccagg gatatgatag gatacttacg cacaaggcca     720

tcgtaaaact cgaccacctc atcggcggtc agctcttggc cggtgctggc aaaaacgtat     780

ttcccgtcct tgaaaatctc attggcagca acatccagtc ccaggtaaat atccttcccc     840

ggcttgtacc ccgccgtttt aatagcctcc aaaattactt caatcgcggc ttcattcgaa     900

ggcaagttgg gagcaaatcc tccttcatcc ccgatggaag tagaaaaccc ttttttactc     960

aataccttct tcagggtatg gtagacctcc acccccatgc gcagggcctc ggcaaaagaa    1020

gtagctccta ccggtaggat caagaattct tgaatgtcca cattattatc cgcgtgcttg    1080

ccgccgttca agatgttcat ttgcgggatc ggcagttctt tggcgtttac gccgcccagg    1140

tactggtaaa gcggcatgga taggtaggaa gcagcagccc gcgccacagc catcgacacg    1200

cctaagatag cgttagctcc cagcttgccc ttgttgtcag tcccatcgag gtcaatcatc    1260

aaccggtcaa tccccacctg gtcgagcgca tccatcccca caacctccgg ggcgatgacc    1320

gtgttgacgt tatccaccgc attcagcacg cctttgccgc cgaaacgctc tgcgtcccca    1380

tcccgcagtt ccaccgcctc aaaagcacca gtggacgcgc cggaaggaac cgcagcccgt    1440

cccatggtcc catcttctaa aaggacctcg acctccaccg tcgggtttcc ccgcgaatcc    1500

agtatttctc tggcgtaaac ctcggtgata atactcacta aggctgagat cttcttcagt    1560

gcattgtagt tgaatgaagg gttagggggg aaatgccccc ctattttttg tctagccatc    1620

ctgccacgtt tgacagggta gcaatttcga cacgataggg ttctctcttc tgccgtta      1678

<210> 62
<211> 2137
<212> DNA
<213> Artificial Sequence

<220>
<223> Synthetic Construct- Sequence No. 62, Exmaple 62: designer
      nirA-promoter-controlled Pyruvate Kinase DNA construct (2137 bp)

<400> 62
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgccgcac ttgggcttgg ccgcggtaga tttgtcccct agccgtatcc     300

atggttatgg tttctccgtc ttggatgaca cgggttgcct cggcggctcc aacgatgaca     360

ggaatatcca agttaatacc tactatggct gcatgagaag tcaggcctcc ctgttccgtg     420

ataatccccc cggccttttc caacaacggc atatagtcct tgtctgttcc gactgccacc     480

actatatctc ccggagcaaa cgagcccggt ttgcccggat ccagtatcac ccgcgcctta     540

ctggtaacgg cccggctacc tatacccgtt cccttaacca gcaccttgcc tactacttga     600

actctcagta gattggtagt cccagggatt ccaggagtac ctgcggttat gactaccagg     660

tcaccggggg agattagccc tcgcctgact gcggcatcca gggctgtttc tatcatctgg     720

tcagttccgt gggtttccgg gaccgtgagc gcgtataccc cccataccaa agttagcttg     780

cgtaccactt cgggtgaagg cgtagtagcc acgatcggag cccggggacg gtacttggcc     840

accattctcg ccgtatggcc cgattttgta gcagtaacaa tggccgaggc ccccaggtcg     900

gtagcggtag cacaggtggc ataactgatg gcatcggtca cggtacgcct gccttcgaat     960

cgtcttgccg ccagcatgtt ctcatagggc aaagccatct ccgtccgccg ggctattcgc    1020

gccatggttt ccaccgcaac taccggatat ttaccggctg cggtctctgc cgacagcata    1080

atggcgtctg ccccctcaaa tatggcatta gccacgtctg acgcttcggc ccgggtgggg    1140

cgcggcacgt ttaccatcga ttcgagcatc tgggtggcaa ttatcaccgg cttaccctga    1200

gcccggcatt tctcgattat aaccttttgc accagaggta cttcttcggt aggtatttca    1260

acccccaggt ctccccgggc gaccatgacg ccatcagcaa cctttattat gtcatccagg    1320

ttgtccagcc cctcctggct ctcaatctta gcgattatat cgatgtcggc tcccttttcc     1380

tctaatatgc gtctgatatc caaaacatca tcggccgtcc ttacgaacga ggcagcaatg    1440

aaatccatat tctgctggat gccgaagtta atgtcttcga tgtctttctg gctcaaaaaa    1500

ggcaggttgg ttctcacacc cggcaggttt atgcctttcc tttcaccgag tactcctccc    1560

gcaaccacct ggcagacgat gtcggtgtcg ttcgcctcta aaacagacag ctggatgacg    1620

ccgtcagcga tcaatatgca gtcacccgct tttacctgcg aaggtaactc atggtaactt 1680

atctggacct cgtcttcgtt accttcaacc ggacggttgg tgagaacgaa cttttgacca 1740

ggacgcagct ctatcttacc ttctttcaag ggcccggtcc tgatctccgg ccctttggta 1800

tcaagcatca accctacttc cgcattcagt tcccgcgcca cctcacgcac catgcggatg 1860

cggcgctcat gctcatcata agtgccgtgt gaaaaattca aacgggccac gttcatgccg 1920

ttggtgatca aagctcttag ccgctcataa tcatcggtgg aaggtcccag ggtacagatg 1980

attttggtct tccgcaataa ggctgagatc ttcttcagtg cattgtagtt gaatgaaggg 2040

ttagggggga aatgcccccc tattttttgt ctagccatcc tgccacgttt gacagggtag 2100

caatttcgac acgatagggt tctctcttct gccgtta 2137

<210> 63
<211> 2163
<212> DNA
<213> Artificial Sequence

<220>
<223> Synthetic Construct- Sequence No. 63, Example 63: designer
      nirA-promoter-controlled citramalate synthase DNA construct (2163
      bp)

<400> 63
agaaaatctg gcaccacacc tgatctgcaa gagacgctca cgcgatacct ctgggcgtgc 60

aatcaccgca ttccccaacg cgctttgggc cacatgaccc ccatcgagag actccgaacg 120

tggcaaatgg agggaccaga gttgttcagt tcacaggtag ataatgtcgc gggtcttgat 180

agttagcaat aaatacagtt tcagaatatc tgtaatacaa aaactgtatc gagacaagaa 240

aaaagtagca aaatttacaa atgttcatga ttcatctggc taaattggat gttcaactga 300

cccattgaag acaagggcaa caaccatgga gcaggttttt atctacgaca ccaccttgag 360

ggatggctcg caggcagaag gtataaactt ttccgtagag gataagatgc gcatacttca 420

aaaactggac gaatttggag tgcattacat agagtgcgga tggcccggtg cgaacccaaa 480

agacactatt ctctttgaaa ggctgagaaa gataaaaact caaaatgcca aaatagtagc 540

ctttggtgca acaagaaaag ctggaaagaa ggcgcacgaa gataagcagg tggaaaacct 600

tttgaaatcg ggtgccaagg tgataaccgt atttggcaag agctgggact ttcatgtaac 660

gcatgccata gggaccacct tagaggaaaa cctggacatg gtttacgaga cggtaagcta 720

tcttaaaaag catgtggagg aggttatctt tgacgcagag cacttctttg acggatacag 780

gcacaacgaa agctatgctt ttaaggtatt ggaggcagct tttcaggcag gtgcggactg 840

gatagtcctc tgcgatacca acggtggcac ccttcccaat gaggtttatg agataaccaa 900

aaaggttgta caaaagtttc cacaggcacg cgtaggcata cacgctcaca acgattcaga 960

tactgctgtg gctaactctc ttatggcggt gcttgcaggt gcaaggcagg ttcacggcac 1020

tataaacggc ttgggggaaa gaacgggcaa tgctaatctg tgttccataa tacctaacct 1080

tcagctcaag ctgggcttta gtgtagtgcc ttcccaaaac ctcaaaaagc tcaccgagct 1140

tgctcacttt gtctccgaaa tctccaacac gccactgccc aaaaacatgc cttatgtagg 1200

ggagagtgct tttacccaca aagcaggcgt acacgcctct gcagttatga aaaggtcaga 1260

aacatacgaa cacatagacc cttctttggt aggaaacaga aggaaggtga cagtgtctga 1320

cctttctgga aggagtaata tactttacaa gctcagggaa atggggcttg aggtggatga 1380

taagtcccct gagcttatca aactccttga aaagataaag gaacttgaga aggaaggcta 1440

ccactttgaa gcagctgaag cttcttttga gcttctttgc aagaggcatt ttgggcttgt 1500

taaaaactat tttgaccttg atgcttacag ggtgctaata gccagaagga gtacagacct 1560

atctcctgtt tcggaagcca ccgtaagact ctatgtggaa gacataaagg agcatacagc 1620

agctcttggt aacggaccag tgagcgccct tgacagagcc ctcagaaaag ccttggaaga 1680

gttttatcca agccttaaag atgttcagct catagactac aaggtgagaa tagttaacga 1740

atcggagggt acatctgcca aagtgagggt gcttatagaa tctaccgatg gtagaagaaa 1800

gtggggaacg gtgggagttt cggaaaacat aatagaagcc tcttggatag ccttaactga 1860

tagcctcgta tataaactct taaaagacga agaagagggt ataatgtgat aaggctgaga 1920

tcttcttcag tgcattgtag ttgaatgaag ggttaggggg gaaatgcccc cctatttttt 1980

gtctagccat cctgccacgt ttgacagggt agcaatttcg acacgatagc gtgctgtact 2040

gttttttgct cgtcagggtt gggttttgtc atcgacaccc aaggattgga gtcggtgctc 2100

aataatcgcc agttgctgtt gggcagccgc caattgcgcc tgaggttctc tcttctgccg 2160

tta 2163

<210> 64
<211> 2878
<212> DNA
<213> Artificial Sequence

<220>
<223> Synthetic Construct- Sequence No. 64, Example 64: designer
nirA-promoter-controlled 3-Isopropylmalate/(R)-2-Methylmalate
Dehydratase DNA construct (2878 bp)

<400> 64
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgtaccat gtctgctgtt gctatctcgc ccttgattgc tgatgctgta 300

acagtagccg ctgaagcaag atatacaaaa gaatctttat gtcctgcacg tcccttgaag 360

tttcgtgtac ctgtactgat aagagtctca ccctcaccga taacaccctg acagcttccc 420

cagcatacag agcagttagg attcataaca attgcacctg cgtccatgaa tatatcaagg 480

agtccctctt tcatagcctg aagatatacc gaacggcttg caggaactac aaggaatctt 540

accttaggag caaccttttt ccctttgatg atcgctgcgc caactcttaa atcctcgatt 600

cgtccattgt tacatgaacc aagaaatgct tcatcaatct ttacaccaag tgattcctta 660

gccggaacta cattgtcaac aaaatgtggc tttgcaacaa ttggctgtat tgttgaaagg 720

tcaatatcat aaacctgctc aaatactgca tcatcatctg atgtaaagca tgcctttggc 780

tctctgccat gctccttaag ataatccatt gcaacatcat caacttccat gagtgcagtc 840

ttagcacctg cctctacaca aaggttacag attgatattc tgtctgccat tgaaaggctg 900

tgtaagcctt ctcctgcaaa ttccattgct ttatagttag caccgttagc gccaatcttt 960

ccaataatag agagtattaa atctcttgca tatactccat cgttaagctt tcccttaagg 1020

ttgaatctta atgttcccgg aaccattacc catgatgttc ctgtaaccat tgcatacaaa 1080

taatctgtac aaccaacacc tgtaccaaat gcacctaacg caccatatgc acaagtatgg 1140

ctgtctgctc caaatataag ctcacccggc actacatgat tttccatcat aacctgatga 1200

cacacaccct cgccctcgta gaacttaata tcattagcct tagcaaagtc tctcatcttc 1260

ttctgtgagg ctgctgtctt aggactgtct gatggaatat tgtggtctac aatccataca 1320

agcttatcct tgtcagcaat atgaggattc tttaacttct catacatacc aatagtaaga 1380

tgtgttgttc catcattact cataagtctg tcaagagtaa cagttgcaat atcaccagcc 1440

ttaacctgtg aaagacctgc tgcccttgcg ataatcttct ctgcaatagt catgccatgc 1500

tttgcctcat ctgcaggtac ggctgtactc tcagactctt ctttctcacc atcaagtgat 1560

gcaataagac caccctgatt aagaatagcc tgcatcttgg ctggaagctt agtacatgta 1620

taagtctttc cattaacagt tataattcca tcttctaatg aaagctcaca ttcatccccc 1680

gcattaactt cgtcatggag ttctttacat acaataacag gaagtcctat attaatagca 1740

ttacgataga atattcttgc aaatgatttg gcaatcactg ccttgacacc taatgcctta 1800

agtacgcttg gtgcctgctc tcttgatgaa ccacatccaa agttgtcatc tgcaacaacg 1860

aaatctcccg gctttacggc agaagcaaaa tcagagtcta atgattcaaa tgtatgactc 1920

ttcatctcat caattgtcgg aaacaaaaga tactgcgatg caataatctg atctgtatca 1980

acatctttat caaacttaaa tatcctaccc attgaccccc atcgagagac tccgaacgtg 2040

gcaaatggag ggaccagagt tgttcagttc acaggtagat aatgtcgcgg gtcttgatag 2100

ttagcaataa atacagtttc agaatatctg taatacaaaa actgtatcga gacaagaaaa 2160

aagtagcaaa atttacaaat gttcatgatt catctggcta aattggatgt tcaactgacc 2220

cattgaagac aagggcaaca accatggtca agaccgttaa gctttctcat tgccttaaca 2280

agtccgcctg cattaagtat atccacaaga ttatcaggaa gtgaagcaat aggatatgct 2340

tttccattgt gtgtaatctt tgcatttact tcaacatcaa tagtatcgcc ttccgtaact 2400

tcgtcgtgaa ggtctgcatt ctctataagg agaagtccgt tattaataga atttctgaag 2460

aatattcttg catatgattt ggcaataaca catttaatac ctaatgcctt aataacctca 2520

ggtgcctgct ctcttgatga accacaacca aagttctttc ctgcaacaat gatgtcgcct 2580

ggcttaatct gacctgcaag ttctggtctt aatggcgaaa atgcatatgg tttcatatct 2640

tctactgtct ttaatgcaag gtactctgta gggataatga tatctgtatc aatgtcatca 2700

ccaagtaccc atactttacc gctaaatttc tcgttcatta aggctgagat cttcttcagt 2760

gcattgtagt tgaatgaagg gttagggggg aaatgccccc ctattttttg tctagccatc 2820

ctgccacgtt tgacagggta gcaatttcga cacgataggg ttctctcttc tgccgtta 2878

<210> 65
<211> 2380
<212> DNA
<213> Artificial Sequence

<220>
<223> Synthetic Construct- Sequence No. 65, Example 65: designer
      nirA-promoter-controlled 3-isopropylmalate dehydratase DNA
      construct (2380 bp)

<400> 65
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgaatgaa tcttcttgga tctgtgatgt atccggttac cgcggtggct 300

gccgccgttg caggagaagc aaggtatatc tccgcattgg gattccccat ccttcccttg 360

aagtttctgt tctgcgtgga aagtaccctc tctccgtctc caagaacacc catgtggatt 420

ccaacacatg ggccgcaacc tggtggtata actgccgctc cgagttcaac aaatttcttg 480

attattccct tttcaagggc gtccatgtag accttccttg aagcggggcc gacgatcagc 540

ctcacatccg ggtgctttcc gtgtttctca agaattttca aagcgatctc aagatcctga 600

agtcttccgt tcgtacaggt tcctatgaac acttgatcta tctttatctt ttccttttca 660

acctcgctca cctttctcac gttgtccaca tagtgaggca aagagacgag tggttcgagt 720

gtgtcggcat ctatctctat ctctgtctcg taaaccgcgt ctggatctgc tttcaactct 780

ctgaagtcct cctctcttcc catctttttc aggaactctc tggtcttctc atcagaaggc 840

atgagacctg ctttcgctcc cacttccacc gccatgttgg aaatggtgag tctgtcctcc 900

acattcatat tttcgataca gcttccatgg aactccaacg ctttgtaagt tgcgccgtcg 960

cttcccagaa ttctcgcgat ctcgagaatg atgtctttcg cgtaaactcc atcctgtaac 1020

ttcccgttca ccacaacctt gatcgtctca ggtactttga accagttctg tccaagcccg 1080

aagatgatcg caacatctgt ggaccccatt cccgttccga aagcaccgag cccaccggca 1140

gtgcaggtgt gcgaatccgc acctgctacc agatcgccgg gtttcacgta tttttccgcg 1200

aggatctggt gggatatccc gtctcccgca tcgaaaacct tgactcccat ctcttttcca 1260

aattctctca tcatcttctg cgaattcgaa agctctttcc tcgggctcgg agaagcgtga 1320

tcgatgaaga ggaaggcctt cgggaccttc acttctttga agccgagttc tctgaattcg 1380

tttatcatca gggggcctgt tccatcctgg gccatggcta tatccactct cgcgagtacg 1440

atttctccgg ctttcacgtc tcttccagta tgttcagaaa agatcttttc tgcgagtgtc 1500

ttacccattg acccccatcg agagactccg aacgtggcaa atggagggac cagagttgtt 1560

cagttcacag gtagataatg tcgcgggtct tgatagttag caataaatac agtttcagaa 1620

tatctgtaat acaaaaactg tatcgagaca agaaaaaagt agcaaaattt acaaatgttc 1680

atgattcatc tggctaaatt ggatgttcaa ctgacccatt gaagacaagg gcaacaacca 1740

tgaactcttg ggaaggaacc gtgctttttc agatagttca ctataccgtc ttctttcagt 1800

atctcgagga gaaacttcgg aatcggagtg aatctgtatt cttttcctgt tgtgaggttc 1860

ttcaaaacac cgttttcgag atctatctca agttcgtctc cctggttgat ctcgtcgact 1920

tccttgagct ctatgactgg aagtcccaca ttgatggcgt ttcggtagaa gatccttgcg 1980

aaagacttcg ccacgataca ggaaacacca gcgatcttta tgatacgcgc agcgtgctct 2040

ctggaagaac caagtccgaa gttcttgcca gccacgatga tgtcaccttt ctgcaccttc 2100

ttcgcgaaat cctccatggc atcttccaag acgtgttttg cgagctcctc aagattgttc 2160

ctcagatgaa aataccttcc aggtgctata tggtcagtcg atatattgtc accgaatttc 2220

cagactcttc cccttatcat taaggctgag atcttcttca gtgcattgta gttgaatgaa 2280

gggttagggg ggaaatgccc ccctattttt tgtctagcca tcctgccacg tttgacaggg 2340

tagcaatttc gacacgatag ggttctctct tctgccgtta 2380

<210> 66
<211> 1456
<212> DNA
<213> Artificial Sequence

<220>
<223> Synthetic Construct- Sequence No. 66, Example 66: designer
 nirA-promoter-controlled 3-Isopropylmalate Dehydrogenase DNA
 construct (1456 bp)

<400>  66
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgccatat ttcttcgagt tttttacata tgagatctcc catctgagag     300

gtcgaaaccg ccttttctgg atcctctgcg atgtctctgg ttctgtatcc ctcttctatc     360

accagctcaa ccgctctttc tatctttctt gcctcttcca ccattccaaa ggaatgctcg     420

agcatcatgg cgagagagag gatctgtgcg atcgggttgg cgatgttctt tccggctata     480

tcaggagcgg aacctcctgc cggctcgtag aggttcttat caccgaaaga cgcggacggc     540

agaagaccaa gagaaccagg aagtgccgca ctctcatccg agagaatgtc tccaaacatg     600

ttcgttgtga ggatcacatc gaactgcgat ggtttcagga tgagctgcat ggcagcgttg     660

tccacataca tgtgcgtcag ctccacatca gggtattctc tcgctacttc gttcacaact     720

ttcctccaca gcatggaact gtagaggacg ttcgctttgt caacggaggt gacctttttt     780

cttctgtttt ttgcgatttc aaaggcagtt ctcgcgatcc gttccacggt ttttctgtcg      840

tagatcatgg tgtcgaatcc cttttcttca tccaatcccc tcggctggcc gtagtaaact     900

ccgtaggaaa gttccctgac ggtcacaaga tcgaccccgg atccaatcac cttttctttc     960

aaaggagaga catgcacaag cgatctgtag acctttatcg gtcttatgtt tgcgtaaagg    1020

ttgagcatct tccttagggc aagaagcccc cctatttccg gcctcttctc cggaggaaga    1080

tcgtcccatt taggtcctcc gacgcttcca aggaagatcg cgtcggcttc cagacatatc    1140

ttttttgtct cttcaggaag gggttcaccg aatttgtcta tggcatcccc tccgatgtgt    1200

ccaaagactt tctcaaaggt tttccccgtt ttcttttcca ccacctcgag cactttaaga    1260

gcttccctta caacctcggg acctatgccg tctccaggca aaaccgctat cttcattaag    1320

gctgagatct tcttcagtgc attgtagttg aatgaagggt taggggggaa atgcccccct    1380

attttttgtc tagccatcct gccacgtttg acagggtagc aatttcgaca cgatagggtt     1440

ctctcttctg ccgtta                                                    1456

<210>  67
<211>  1933
<212>  DNA
<213>  Artificial Sequence

<220>
<223>  Synthetic Construct- Sequence No. 67, Example 67: designer
       nirA-promoter-controlled 2-Isopropylmalate Synthase DNA construct
       (1933 bp)

<400> 67
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgctcagc acccccgttt tttctgagaa ggcctttagc gatgaggtac 300

ttgtttattg cgtttatgta agctatcgca gaggcttcca ctatgtccgt ggagacaccc 360

cttccactgt agagttcacc gtttattctc aacgtgagtt tgacctctcc ctgtgcgttc 420

tttccagttc caaccgcctg aattatgtac tcctcgagct tcggttgaat accgagcgct 480

ttgtctatag ccttgaagat agcatccact ggaccgtttc ctgcctctgc tgcttctttc 540

ttttcatctc caacctgaag cacgaccgcc gcggttggaa gcagcgtgtt tccggtgtgt 600

acatggaagt gaacgagctt gtaaccgttg atgggctccc tcaaaacttc cgagacgatc 660

gagaaaagat catcgtcgta aacctctttc tttctgtcgg cgagttctgt gaacttctcg 720

aacactttct ggaaggtctc ttcgtcgagt ttgatgccgt agctctccag cttctttctg 780

agggcgtgct ttccggagtg tcttccaagc acgagcgtct cggaagacct gccgatatcg 840

gatggtttca tgatctcgta ggtctccctg tgtttcagca caccatcctg gtgtataccc 900

gactcgtgaa ggaacacgtt ctctcccact atgggtttgt ttctggacgg gatgagcccc 960

gttatatgtg tgaggagcct ggaagcgggg tatatgagct ctgtctttat acccgtctcg 1020

tagggaagtt tgtctttcct caccttgagg atcatcacga actcttccag ggcacagttt 1080

cctgccctct ctccgatacc gttcagagtc acttcgacct gggtggctcc gttctgaacg 1140

gcagcgaggg agttcgccac agcgagtcca agatcgttgt gacagtgcac agaaagatcg 1200

acattctcta taccgggcac accctctctc aaggtcttta tgagttctcc aaactcatcg 1260

ggaagggcgt accccaccgt gtccggaaca ttgatcgttg tggctccggc ttcgatcgcc 1320

gtcttgtagg cttctatcaa aaagggaacc tccgttctcg aagcgtcttc cgccgagaac 1380

tccacaaggt cgaaaaactg ttttgcgtag ccgacgtatc ttctgatcct ctcgaggatt 1440

tcctctttct ccattctcag tttgtatttt ctgtgaatcg gagaggtcgc tatgaaaacg 1500

tgtatcatac gtttgtcttt tggtcgatcc ttgagagcct cgtacaccgc gtctatgtcc 1560

ttttcaacac accttgcgag tcccaccact atgggtttct gaacggcgct cgcaactctt 1620

tttacagctt caaactgcac gggagatgaa acgggaaacc cagcctcgat gagatcgaca 1680

ccgagatcct ctaacatgag tgccatttct actttttcct caaccgacat cgaagcccct 1740

ggggattgct ctccatccct caacgttgta tcgaagatct taattctcct cattaaggct 1800

gagatcttct tcagtgcatt gtagttgaat gaagggttag gggggaaatg cccccctatt 1860

ttttgtctag ccatcctgcc acgtttgaca gggtagcaat ttcgacacga tagggttctc 1920

tcttctgccg tta 1933

<210> 68
<211> 2632
<212> DNA
<213> Artificial Sequence

<220>
<223> Synthetic Construct- Sequence No. 68, Example 68: designer
nirA-promoter-controlled Isopropylmalate Isomerase DNA construct
(2632 bp)

<400> 68
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgacagac cggctcggct tcaagctggc gcacatcgac aaaatgtccg 300

taaatcgcag ccgccgcggc catcaccggg ctgacgagat gcgtgcgcgc cccttttcct 360

tgccgccctt caaagttgcg gttcgacgtt gacgcacagt gctcgccttc tgggatgatg 420

tccgggttca tgcccaaaca ggcgctgcag ccggagtcgc gccattcaaa accggcgtcg 480

atgaaaattt gtgccaaccc ttccgcttcg gcttgttttt tcacttgctg cgatccaggc 540

acgacgagcg cccgtacacc aggagccact tttttccctt tcacgatgct cgccgccgcg 600

cgcaaatcgc tgaggcgtga gttggtgcac gaaccgatga agacgtgctg caccggaata 660

tccgtaatcg gcgtgcccgg cttgagcccc atgtactcaa gcgcccggcg caccgcgttt 720

tgctctgttt tgctttcaaa ttgctcggga tgcggcacga cgccatcgac ggaagtgctc 780

atcgcggggt tcgtccccca cgtcaccatc ggagcgatcg tcgacgcatc gatttcaatc 840

gttttatcgt attttgcccc ctcatcgctg gctaacgccc gccaccgttc caccgccttg 900

tcaaactcct cgccttttgg tgcatatttg cggccgcgca aataggcgaa cgtcgtttca 960

tccggactga tgaggccggc tctcgcccca gcttcaatcg acatgttgca aatcgtcatt 1020

cgctcttcca tcgacatgcg tcggatcgct tcgcctgtaa attcgataat ataaccggtg 1080

ccgacgccaa cgccatagcg gccgataatg gccaagatga cgtctttggc ggtcactcct 1140

ttgccgaggc ggccgttgat gcagatttgc agcgttttcg gcttatgctg ccaaagcgtt 1200

tgtgtagcca atacatgctc gacttcgctc gtgccgatgc caaacgccaa ggcgccaaac 1260

gccccgtgcg tcgacgtatg gctgtcgccg caaacgatcg ttttccccgg ctgggtcaac 1320

ccgagctctg ggccgatgac gtgaacgatt ccttgctctt cgctgtgtag gtcggcgagc 1380

ggaatgccga actcgcggca gttgcgctca agcgcagcga tttggttgcg cgccacttcg 1440

tcggtaatca caaatcggtt aacggttggc acgttatggt ccatcgtcgc aaaggtcaaa 1500

tccggccgcc gcaccttccg tcctttttgc cgcaaccctt caaacgcttg cggcgaggtc 1560

acttcatgca ctaagtgcaa atcgatgtac aataaatccg gtttgccctc ctcacggtag 1620

acgacgtggt tttcccaaat tttatcgatg atcgttttcg gcttcattga cccccatcga 1680

gagactccga acgtggcaaa tggagggacc agagttgttc agttcacagg tagataatgt 1740

cgcgggtctt gatagttagc aataaataca gtttcagaat atctgtaata caaaaactgt 1800

atcgagacaa gaaaaaagta gcaaaattta caaatgttca tgattcatct ggctaaattg 1860

gatgttcaac tgacccattg aagacaaggg caacaaccat gcggccgtgg acaatgccgt 1920

cgttcgtagg cggcgatatg cgcttcgtac acaaacgtca aatcaatttc gtcccatcct 1980

tttagcaaca gctgttttcg atacgggtcg atgtcaaacg gacgcgaaaa tccttcatcg 2040

tcaaataccc gctgttcttc aagcgaaacc gtcagttcat agtctgcgcg ctcgctttgg 2100

cgcagcaagt agcggacatc ctctttatcc agccggatcg gcaacagtcc attttttaag 2160

cagttgttgt aaaaaatatc ggcaaacgat ggggcaatga ttacgcggaa tccgtaatct 2220

tgcagcgccc acggcgcatg ttcgcgcgat gagccgcaac cgaagttttc atcggcgact 2280

aaaatcgtcg ccccttcgtt ttccgggcgg ttgagctcaa actccggatt tggcgtgccg 2340

tcgctcaaat accgccaatc gtaaaagaga aactggccaa agccggtgcg ttcaatccgt 2400

ttcaaaaact gctttggaat gatttgatcg gtatcaatat tcgcccgatc gatgccggcg 2460

gtttttccgc gatggatcgt aaacggcttc attaaggctg agatcttctt cagtgcattg 2520

tagttgaatg aagggttagg ggggaaatgc ccccctattt tttgtctagc catcctgcca 2580

cgtttgacag ggtagcaatt tcgacacgat agggttctct cttctgccgt ta 2632

<210> 69
<211> 2035
<212> DNA
<213> Artificial Sequence

<220>
<223> Synthetic Construct- Sequence No. 69, Example 69: designer
      nirA-promoter-controlled 2-Keto Acid Decarboxylase DNA construct
      (2035 bp)

<400> 69
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgtataca gtaggagatt acctgttaga ccgattacac gagttgggaa 300

ttgaagaaat ttttggagtt cctggtgact ataacttaca atttttagat caaattattt 360

cacgcgaaga tatgaaatgg attggaaatg ctaatgaatt aaatgcttct tatatggctg 420

atggttatgc tcgtactaaa aaagctgccg catttctcac cacatttgga gtcggcgaat 480

tgagtgcgat caatggactg gcaggaagtt atgccgaaaa tttaccagta gtagaaattg 540

ttggttcacc aacttcaaaa gtacaaaatg acggaaaatt tgtccatcat acactagcag 600

atggtgattt taaacacttt atgaagatgc atgaacctgt tacagcagcg cggactttac 660

tgacagcaga aaatgccaca tatgaaattg accgagtact ttctcaatta ctaaaagaaa 720

gaaaaccagt ctatattaac ttaccagtcg atgttgctgc agcaaaagca gagaagcctg 780

cattatcttt agaaaaagaa agctctacaa caaatacaac tgaacaagtg attttgagta 840

agattgaaga aagtttgaaa aatgcccaaa aaccagtagt gattgcagga cacgaagtaa 900

ttagttttgg tttagaaaaa acggtaactc agtttgtttc agaaacaaaa ctaccgatta 960

cgacactaaa ttttggtaaa agtgctgttg atgaatcttt gccctcattt ttaggaatat 1020

ataacgggaa actttcagaa atcagtctta aaaattttgt ggagtccgca gactttatcc 1080

taatgcttgg agtgaagctt acggactcct caacaggtgc attcacacat catttagatg 1140

aaaataaaat gatttcacta aacatagatg aaggaataat tttcaataaa gtggtagaag 1200

attttgattt tagagcagtg gtttcttctt tatcagaatt aaaaggaata gaatatgaag 1260

gacaatatat tgataagcaa tatgaagaat ttattccatc aagtgctccc ttatcacaag 1320

accgtctatg gcaggcagtt gaaagtttga ctcaaagcaa tgaaacaatc gttgctgaac 1380

aaggaacctc attttttgga gcttcaacaa ttttcttaaa atcaaatagt cgttttattg 1440

gacaaccttt atggggttct attggatata cttttccagc ggctttagga agccaaattg 1500

cggataaaga gagcagacac cttttattta ttggtgatgg ttcacttcaa cttaccgtac 1560

aagaattagg actatcaatc agagaaaaac tcaatccaat ttgttttatc ataaataatg 1620

atggttatac agttgaaaga gaaatccacg gacctactca aagttataac gacattccaa 1680

tgtggaatta ctcgaaatta ccagaaacat ttggagcaac agaagatcgt gtagtatcaa 1740

aaattgttag aacagagaat gaatttgtgt ctgtcatgaa agaagcccaa gcagatgtca 1800

atagaatgta ttggatagaa ctagttttgg aaaaagaaga tgcgccaaaa ttactgaaaa 1860

aaatgggtaa attatttgct gagcaaaata aatagtaagg ctgagatctt cttcagtgca 1920

ttgtagttga atgaagggtt aggggggaaa tgccccccta ttttttgtct agccatcctg 1980

ccacgtttga cagggtagca atttcgacac gatagggttc tctcttctgc cgtta 2035

<210> 70
<211> 1426
<212> DNA
<213> Artificial Sequence

<220>
<223>  Synthetic Construct- Sequence No. 70, Example 70: designer
      nirA-promoter-controlled NAD-dependent Alcohol Dehydrogenase DNA
      construct (1426 bp)

<400>  70
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catggggtat caggactgcc ctgcctagaa cttcaccttt ctctagcctc     300

tcaagcacat cgtttatttc atctagcttg tggatatcaa cctctaccct gaccttaccc     360

tgaagggcta acgtgactag ctcatggagc tctacatagt ttcctactag gcttccttca     420

aaggatacct cagaggatat caccctgatc gtggggaatc taagctcacc cccatagcct     480

acgatgataa gcctccccat cctccctagc aggtatggtg tataatcgac tgtagcctga     540

gagcctacga agtccattgc aacgttaact cctctccccc tggtaagctc catgacctgc     600

tttacagggt ctcgtctagc gtcaaccacg tgatccgctc caagcctctc ggccagcttt     660

agtttttctt ccttaacgtc cagtgctatc accgtcgcgg gtgtcataac tttaagtagc     720

tgaactgcaa tatgacctaa tcctcccacg cccactatag cgacgtatgc gccgggatag     780

agggttcggg cggccttctt aacagcccta taagccgtta tcccagcgtc cgctagaggg     840

gccatttcaa caagtttctc cctgctaata tccttaggca gctttatcac agacctgtgc     900

gaggtcctca tgaactctgc aaatccacca tcgatattaa gtcctgggaa ctctaggttc     960

tcgcagtgca tatcctcacc agctctacag gctagacagg ttccatctgt gaccgccggg    1020

tgaagaatta ccgggtcccc cttctctaag ccttccactc cttcggcaac ttcttcaata    1080

tacccgacgt tctcatggcc taaagtgtag ggtagcttag gctgcaatag ctcatgccac    1140

attccctgga caaggtggag gtccgtatgg catacgccag cgcctgcaat ccttacaata    1200

acgtcaaatc taccttctag cctcggatag tcgacatcct ctatcctcaa cggcttgtta    1260

tactcgtgga gcctggcagc tttcaataag gctgagatct tcttcagtgc attgtagttg    1320

aatgaagggt taggggggaa atgcccccct attttttgtc tagccatcct gccacgtttg    1380

acagggtagc aatttcgaca cgatagggtt ctctcttctg ccgtta                   1426

<210>  71
<211>  1468
<212>  DNA
<213>  Artificial Sequence

<220>
<223>  Synthetic Construct- Sequence No. 71, Example 71: designer

nirA-promoter-controlled NADPH-dependent Alcohol Dehydrogenase
DNA construct (1468 bp)

<400> 71
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggcgtac ccagacacct ttgaaggatt tgccgtcact gacactgcaa 300

aatggtccac aaccaagaag atagaattca ccccaaaaag gttccaggaa catgatatcg 360

atgtcaagat ccatgcctgt ggtatctgcg ggagtgatgt tcacactgtt tgcgggggat 420

gggcaaaacc agaccttccc gtgatcccag gacatgagat cgttggtgag gttgttagag 480

tgggcccaaa agtgaaggga tttgaaattg ggcaaagagt tggtgttgga gctcaagttt 540

gggcctgtct agagtgcgac acatgcaagg ataacaacga aacgtactgt cctcaatggg 600

tggacactta caatgccact tatcctgatg gtgacaaggc atggggtggt tattcctctc 660

acatcagagt ccacgatcac tttgtattcc ctattcctga tgaacttcca actaatgctg 720

tggccccaat gttgtgcgct ggtatcacca cgtactctcc gttggtaaga aatggagctg 780

gtccaggaaa gaaggtgggt atcatcggaa ttggagggtt gggacatttt gccatcatgt 840

gggctagggc tcttggttgc gaagtgtaca cgttttctag aacacatagc aaggaagctg 900

atgctaagaa attgggaact gaccatttta ttgcgacgtg ggaggacaaa gactgggcca 960

agaagattgg cagaaagctg gactttatca tttcgtgtgg aaattcggcc acgaactttg 1020

atatggatgg ttacctcagt gtgctgaagg ttcatggtaa actcatttcc gtcggccttc 1080

cagaggagcc attcacgctg tctgctggaa gctttatcaa gaacggttgc tacttgggat 1140

cgtcccactt ggggaacaga caggagatgc ttgatatgct gaaacttgct gctgataagg 1200

gcattggttc ttggtatgag gagctcccaa tctctgagga agggctgaag gaaggactgg 1260

agagatgcca caacaatgac gttaagtata ggttcaccct gaccggttac gataaggcat 1320

tcaaatagta aggctgagat cttcttcagt gcattgtagt tgaatgaagg gttagggggg 1380

aaatgccccc ctattttttg tctagccatc ctgccacgtt tgacagggta gcaatttcga 1440

cacgataggg ttctctcttc tgccgtta 1468

<210> 72
<211> 1555
<212> DNA
<213> Artificial Sequence

<220>
<223> Synthetic Construct- Sequence No. 72, Example 72: designer
nirA-promoter-controlled NADH-dependent Butanol Dehydrogenase DNA

construct (1555 bp)

<400>  72
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgcaggga agcgcgcaaa atggcgagca catcatcgcg gttcaatgtt     300

ttgaaacggc cgaactcgcc aaacgccatc gctttatcgg ccatcagctc caagttttcc     360

tcgccgatgc catagtccgc aagccgcgac ggcgccccga ggctcgacca gaactcgcgc     420

agccgctcga tgccttcaag cgccacatcc cgctccgatt tgcccgccgg atcgacatca     480

aagacgcgca ccgcgagctg ggcgaaacgg ctgacatttt catcgagcac atgcttcatc     540

cagttcggga acaaaatcgc caatcccccg gcgtgcggga tgtcatacac ggcggaaacg     600

gcatgctcaa tattgtgcgt cgcccagtcg ccgcggacgc ccatttgcaa gaagccgttt     660

aaggcgatcg tgcccgagta catgatcgtc tcgcgcagct cgtagttttc caaatcgttg     720

atcagtttgg gcgccgtttc gatcacggtt ttcaacaccg cttcacacat ccggtcttgc     780

aatggcgtgt tcggcgtatg gtggaaatat tgctcaaaca catgcgacat catatcgaca     840

atgccgtaaa ccgtatggtc tttcggaacg gtcatcgtat acgtcggatc caaaatcgaa     900

aattgcggga acgtaaacgg gctgccccag ccgtattttt ctttcgtctc ccagtttgtg     960

atcaccgaac cagaattcat ctccgacccg gtcgccgcca gcgtcaagac gacgccaaac    1020

ggcagggcgc cggtgacggg cgcttttttc gtgataaact cccacggatc gccatcgaat    1080

ttcgccccgg cggcgatcgc tttcgtgcag tcgatgacgc tgccgccgcc aaccgccagc    1140

aaaaattcga cgccttcccg cttgcaaatg tccacccctt ttcttacggt cgagacgcgc    1200

gggttcggtt cgacgcctgg cagttcgatg acctcggcgc caatattccc caatatcttc    1260

atgacttcct catacaatcc gtttcgtttg atgctgccgc cgccatacac gagcagcact    1320

ttcttgccgt agcgcggcac ttcttctttg agacgttcga gctgcccttt tccgaaaatc    1380

agtttcgtcg ggttgcggaa aataaactct tgcattaagg ctgagatctt cttcagtgca    1440

ttgtagttga atgaagggtt aggggggaaa tgccccccta ttttttgtct agccatcctg    1500

ccacgtttga cagggtagca atttcgacac gatagggttc tctcttctgc cgtta         1555

<210>  73
<211>  1558
<212>  DNA
<213>  Artificial Sequence

<220>
<223>  Synthetic Construct- Sequence No. 73, Example 73: designer

nirA-promoter-controlled NADPH-dependent butanol dehydrogenase
DNA construct (1558 bp)

<400> 73
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgaaaatc cacatcactg ccgtaatatg cacaagtata taatttttcc 300

atagtttcat cattaatctc ccttggattt gatcctgtgc atggatctaa aacagcatta 360

tgagctataa atttaagatt agctttaaat tcattctcat ctattccata ctctttcatt 420

gatgaaggta tatttaattc cttattaaac ttatttatta aatctattag gtcatctgtt 480

aattctcttt cactttctcc ctttaatcca atatgtcttg caatgttagc atatctattt 540

tcacaagctt ttctattata tttaattaca tatggcaaga agatagcatt agcacatcca 600

tgtggtatat ggaatacagc ccctacctta tgagccatag aatgtactat tcctaaaagg 660

gcattagaga aggccattcc tgctaaacat tgagcctcat gcatttctcc cctagcttcc 720

atatctccct tatatgaatt tactaagtgc atattaacca tttcaattgc ctttaaagct 780

aaaggatctg taaagtttga tcttaaactt gcagtataag cctcaatagc atgagttaag 840

gcatccattc ctgtgtgagc tactaacttc tctggcatag tttctgctaa gctaggatca 900

acaatagcta tatctggagt tatttcaaaa tctgctaaag gatatttaat cttagcctta 960

taatcagtta ttactgagaa ggcagttacc tctgtagcag ttccagaagt tgatggaata 1020

gctacaaact ttgcttttct tctaagctta ggtaatccaa aaggaacaat ggccttttca 1080

aaagtaaaat caggatactc gtagaaaatc cacatagcct ttgctgcatc aataggtgaa 1140

cctcctccta tggaaactat ccagtctgga ttaaattcct ccatttcctt tgcacccttc 1200

ataacagttt ctactgatgg atctggttct actccttcaa aaacctttgt ttccatatta 1260

gcttccttta aataacttaa aaccttatct aaaaagccaa atcttttcat tgatccgcca 1320

ccaataacta taaaggcctt tttaccttct aaactcttta atacttctaa ggaatctttt 1380

ccatgatata tgtcccttgg taaagtaaat cttgccatta aggctgagat cttcttcagt 1440

gcattgtagt tgaatgaagg gttagggggg aaatgccccc ctattttttg tctagccatc 1500

ctgccacgtt tgacagggta gcaatttcga cacgataggg ttctctcttc tgccgtta 1558

<210> 74
<211> 3646
<212> DNA
<213> Artificial Sequence

<220>

<223>  Synthetic Construct- Sequence No. 74, Example 74: designer
      nirA-promoter-controlled Phosphoenolpyruvate Carboxylase DNA
      construct (3646 bp)

<400>  74
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catggccggc gttccgcaag cccgccgcga tgcccaggat ggagagcagc     300

agcgcccgct ccagggccgg gcgctcgggg tcgccttccg gcgaggcccg gtaccgggcc     360

agcagctcca cctgcaggta gctgatgggg tcgacgtacg ggttgcgcaa ggccgtctgc     420

cgcgccagca cggggtggtg ggccagaagc ggcccgccga aggtctcctc caagagcgcc     480

cgggtccgca ccatggcctc ctcgagccgg gggaagaacc ggttcgccag gggcccgggc     540

accagccgca ggtactccct ggccacggcc aggtcggcct tggccagggc cagggccgcg     600

ccgtccagca ccgagcggaa gaagggccag cgggcaaaca tcgcctggcg aaggtcccgc     660

ggcaccgccg caagaccttc ggccagcccg taccagccgg gcagcagaag acgcacctgg     720

gtccaggcca tcacccaggg gatggcccgc agatcctgga cgcgccgggc ccggcccgtg     780

cgggccaccg ggcgggaagc gatcttcagg gcggcgatct cccggatcgg ggtgaagtgc     840

tcgaagaact cgaagaaccc gggctccgcc agcagctggc ggtaggcctc cgccgaccgg     900

gcggcggccc ggtccatggc ctcccgccag gccgccggga cgggggaacc gcccggggcg     960

agcccctggc gcccgccgcc cggcctgctc ggcgcggtcc cggccccgcc gggcgggccg    1020

ggttcctccg gcgaaagcac cgcctcttgg gcctgggcca gcgtgtcccg ggccgccgcc    1080

agaaggaagt ggtagaggag ctgctccagg ttgcgcaggg ccagctcggg gtgagcgtag    1140

cggtccgcca gcgcctctcc ctgctcggtg agccgcaggc ggcggcccac ggtgcccggc    1200

ggcaggctgg cgatggcgcg gccggcggtc cccccgccgc cccgggctgt ggacgtgccg    1260

cgaccgtgga agaaggacac cggcacgccc gccgcccggg ccaccgccgc gatgccctcc    1320

tgggcccggt agagggccca gttggccgcc aggtagcccg cgtccttgct ggagtcggag    1380

tagccgatca tcacctcgca gccgccgcgg ccgcgggcgt ggacgccgaa gacggggttc    1440

tgcagcagct gctccatggc gcggggcgcc gcctccaggt cggccagggt ctcgaacagg    1500

gggaccacgt caaagggcaa ggggtggccg gggcggtaca gccccacctc ccgcgccagg    1560

acgaacacct ccagcacgtc ggcggggccg cggcagccgc tgatgatgta agccccccgg    1620

tcctgccagg cccgcagagc atccagggcc acggccagct cgcggctgcg gggccggtag    1680

cccaccggcg ccagcggccg gggcgaagcc agctcccggg tcagcaccgc ctcccgcccg    1740

gcggcatcca gggccaggta ctcccctgcc gccatgaccc caccggcttc cagcagctcc 1800

gccaccgccc gcccgtgggc ctcggcatgc tcccgcaggt cgagggcgcc catggcctcg 1860

ccaaagacct gggcccgcca gcgcagggga cgcaccaggg tggccgccgc ctcgcccagc 1920

ccggcatccc gcagcccctc ttccacctgg aagagcaggc gatccagggc ggtggaaccg 1980

gcggcctccg gcccctcgcc ggccggcggt tccccggcgg tcccctttgg tggcgcggcc 2040

gcggcgggcc gggaaggccg ttcgccggct gggtccgctg gaacggcggg ccctccaggg 2100

atgccctccg ctgaagcggg cggccgggga ggcattgccg ctccctccgg cggagccgcc 2160

ggccggggag gcggtgccgt tgcctcgact gcagcgtccg gccggtgggg tacagccggt 2220

gcctcggctg gagccgccgc ccgggcgggc gtctcgctcc acacctcttc cggcaccgcc 2280

cttgccgccc agtgccgctg cagccggtag gcgaaccgcc ggtagggctc gccggggaac 2340

cggccggcgg ccgccggcgg caggggcgcc acggcctctg cgggcaagga gggcagggga 2400

ggcagggaag cccgttcttc cgccacggac agatcccgcg tcagggcggc caggcctccg 2460

gcgtactttc gggcgatctc ggcccgcgcg taggcctggg cccaggcggt gacctccggg 2520

gtcacaaagg ggttgccgtc ccggtcacca ccgatccagc tccggaaggc caggcgcggg 2580

ggaagaaccg gccgccgccc gtagcgggcg gccaccgccc cctcgagggc ctccatgagc 2640

cggggcaccg cctcccacag ggtggtgggc aggtagtaga ggccgccgcg gacctcgtcc 2700

tccacccggg gcggggccgg gcgcagctcg cgggtggtcc agagcagggt gacccgggcc 2760

accacctcgt ccaggtcccc ctcgccccgc tccagccggt ccagggcctg gttgagctgc 2820

agcaggtggt ggcgcagggt gcgccgccgc gtctcggtcg gatgggccgt gaaggtcagc 2880

tccagccggg ccgaggcgag gaggcgcacc acgtcgtcga attccatccc ttgggcctga 2940

agctgggtga ccagggccag cagcgactcg ggccggggcc ggtcgggcgt gctggcctgc 3000

tcgcgccggc ggttcacccg cacccggtgc cgctcctccg ccaggttgac cagatggaaa 3060

taggtcgaaa aggcccggat cagcccttcg gccgcggcca ccgaaagccc gccgatctcg 3120

gcccgcagcg cctgccgggc ggcgtcgtcc cccgggtgct gccggaggtg cttggtgtgg 3180

gcgcggatgt cctcttccag ttcgaacagg cgatcccccg agaggcggcg gatggcctcc 3240

cccagggccc gtcccaggag gtcgacctcc cgcttgagaa gggggtacag ctcctcttcc 3300

ggccgcccat ccggcggggg aacggatccc gccggggcag gcaacgcctc gccggacgag 3360

ccggccgccg cagggcggct cccctgcccg ccgccccggt cctggcggtc ctgaccgtcc 3420

ttgcggtccc ggccgtcctt caggtcccgg ctccccttcc ccgccgccga gccggcccct 3480

gccggccggt caccctcgcc gctcactaag gctgagatct tcttcagtgc attgtagttg 3540

aatgaagggt taggggggaa atgcccccct attttttgtc tagccatcct gccacgtttg 3600

acagggtagc aatttcgaca cgatagggtt ctctcttctg ccgtta 3646

<210>  75
<211>  1591
<212>  DNA
<213>  Artificial Sequence

<220>
<223>  Synthetic Construct- Sequence No. 75, Example 75: designer
       nirA-promoter-controlled Aspartate Aminotransferase DNA construct
       (1591 bp)

<400>  75
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgagaatt tcggaaagat caaaaaaggc gccggcgagt cccataagaa     300

aattagtgcc atttgccgag gaagctgtaa aaaaaggtaa aaaaatctat tatttaaaca     360

taggtcaacc tgatatacca acaccttcaa tttattttca gtatgaggaa aaacacagac     420

cccagattgt agcttataca cactcagcag gtcttctgcc actgagggag gcttttacaa     480

aatattacgc cagattcgat atagatgttt tcccggatga aatcatcgtt accaatggtg     540

gaagtgaagc tgtcctgttc gctatgacag tcgtggcgga tccgggagat gaaatacttg     600

ttctggagcc gttctatgcc aattacgctg gatttgctgc tcaacttggt atcaatcttg     660

ttccagttag aactcgcccg gaggatggat atcaaatacc aaagatgggt gattttctgg      720

agaaaatcag tcacaggact aaagcaatca ttttttccaa tccctgcaat cctacggggg     780

ctgtttacga tgaaaaacaa cttgaagtta ttgctgaagt tgctttgaag agagatttgt     840

ttgtgatttc agacgaggtt tatagagaat tcacttttga tggttttagg gccatatcta     900

tgatgagttt ttctaatatt tctgacaaag tcatagttgt tgacagtatt tctaaaagat     960

atagtgcctg cggcgcgcgg attggtacat ttattaccaa gaataaagat atatatcaag    1020

cagctatgaa actggctcag gcgagactct gtcctgctat gacatctcaa tacggtacta    1080

ttggtcttct gacgcttgac gatttgtatt attcacaaat gagaaaagag tatgaaatga    1140

gaagagatgt tgtttacgaa gaacttcagc gcattgatgg agcagtcttc aagaaacctc    1200

acggggcttt ttatatttcg gtgaaacttc caatcgacaa ttctgaagat tttgtgaaat    1260

ttatgttgac ggagtatgag gttgaaggaa aaacgacaat ggtggcacct ctcagtggat    1320

tttatgtaac accatctacg gggatgagtg agatcagaat agcgtatgtt ctggaacgcg    1380

aacaactgag ggatgcagtt gcaattttga cttcaggttt gaaaacttac atagagagaa    1440

gaaataaata ataaggctga gatcttcttc agtgcattgt agttgaatga agggttaggg    1500

gggaaatgcc cccctatttt ttgtctagcc atcctgccac gtttgacagg gtagcaattt 1560

cgacacgata gggttctctc ttctgccgtt a 1591

<210> 76
<211> 1588
<212> DNA
<213> Artificial Sequence

<220>
<223> Synthetic Construct- Sequence No. 76, Example 76: designer
      nirA-promoter-controlled Aspartate Kinase DNA construct (1588 bp)

<400> 76
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgtagtcc gaaaacttga cagagttctt tcaacaaatc ttctgcttta 300

tcttcatgta caaggcatga tatctttatt tcagaagttg ttatcatttc tggctctatg 360

tttttctttc tcaaaacttc aaaaaatttt gctgcaacac ctcgttgata tttcattcct 420

aaacctatta ttgatacctt agaaaacccc cctgtgatct taatttcaca atcaagatct 480

ctcattgcct tctgtacgtc aaccgagtta ctttcaacaa ttgtgaaaga tagattaatg 540

tctcctgagt tactcaccaa cgatatcata tcaacgttaa agcctttttc agcgagttct 600

ctaaatacgt cggcggttgc tttagaattt ttcaagctat aaacgctgac ctttacctga 660

tttttctcta tcgtcgcgcc tgtaacaacg ggtcgctcaa gccattccgg aagttttccc 720

atcacccacg ttccctcctc atttgaaaac gaagaagcgc aataaattgg tacgctgtat 780

ttttttgcta tctcaacact tcgagaatgt agaaccctgg caccaagtgc ggagaattcc 840

aacatttcat cgtatgtaat ataagacagt ttttttgcct ggggaaagat cctgggatct 900

gttgtatata tgccagcgac gtcgctgtat atttcgcaga ttgttccaag ttttgcggca 960

atagcaacag cagaagtatc tgatcctcct cttccaagag ttgtcagttc atcgttttcg 1020

tttattccct gaaaccctgt aacaagtaaa acatcgttat gaaaagccag cgatcttaat 1080

tttcggtcat ctatatcttt tatccttgcc gaattaaaat cgctggttgt cagaatccga 1140

gcctgaaacg cattcaaaga tacagctttc atacctattc tgtcaaggta tatggatagc 1200

aaagcagcgg atatttgttc gccacaggat aacaacatat ccagttctct tggattgggt 1260

ttttcagaca gccttcttgc aagaaaaacc aatttgtctg ttgtttttcc cattgcagag 1320

acaacaacga ttaacttttt cccatttttt actgttttca ctattttctc ggtaattttt 1380

ttgattcttt ctatgctggc aagcgatgag ccaccatatt tttgtacaac aagtttcaga 1440

aaaatcacta aggctgagat cttcttcagt gcattgtagt tgaatgaagg gttagggggg 1500

aaatgccccc ctattttttg tctagccatc ctgccacgtt tgacagggta gcaatttcga 1560

cacgataggg ttctctcttc tgccgtta 1588

<210> 77
<211> 1411
<212> DNA
<213> Artificial Sequence

<220>
<223> Synthetic Construct- Sequence No. 77, Example 77: designer
      nirA-promoter-controlled Aspartate-Semialdehyde Dehydrogenase DNA
      construct (1411 bp)

<400> 77
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgtcccct gaaaatatta taaatctctg cattgagaac tgcacacccg 300

gcagcccctc tgattgtatt atgaacaaga gcaacgaacc caaactgttt gttcgaatac 360

tgtttcaacc tgccaacact gacactcatt ccaccaccaa gatctctgtg aaacttcggc 420

tgcggagaat caactcctgt caaataaaaa acaggttttt caggtgcggt tggaagatac 480

attcctttaa gtggttgaaa atcttcaaaa gctctcacaa tttcacttaa agtggccttt 540

tcctgagttt tgatagttat agatagcata tgaccgtcaa caacggctac cctgttgcac 600

tgtgcgaaaa tatcgagatt cgccgggtat atttttccat ctttcaatct gccaagtatt 660

ttttttgatt cggtcatgat tttttcttcc tcatttttta tgaacggcac aacattgtca 720

attatatcca tagatggtac accgggatat cccgcgccag agatagcctg cattgtgaca 780

acatttgcct cttctatacc gaatctatcc attatgggtt taagaaccat tgtaaaacct 840

atcgttgagc aattagggtt ggtgattatt tttccttttc tcttttgagt ttcggttatt 900

ttcaaatgat ccagattaac ttcagggata attagaggta catcctcgtc cattctatgg 960

cttgcggcat ttgagaaaac aatgtatcct gcgtttgcaa attcctcctc aatttcgcct 1020

gcaacatctg aaggcaaagc agaaaacaca taatcacaat ctatatcagg tgtgcatttt 1080

tttaaaacca tatctcctgc tttttcggct acaggaacat tcaagcgcca ttgaactgct 1140

tctctgtact tttttcccgc agaattatca gaagctgcca gagctgttat ctcaaaaaat 1200

ggatgatttg aaagcaattg aacaaatctc tgtccaacaa gccctgttgc accaagaata 1260

gctaccttca ttaaggctga gatcttcttc agtgcattgt agttgaatga agggttaggg 1320

gggaaatgcc cccctatttt ttgtctagcc atcctgccac gtttgacagg gtagcaattt 1380

cgacacgata gggttctctc ttctgccgtt a 1411

<210> 78
<211> 1684
<212> DNA
<213> Artificial Sequence

<220>
<223> Synthetic Construct- Sequence No. 78, Example 78: designer
      nirA-promoter-controlled Homoserine Dehydrogenase DNA construct
      (1684 bp)

<400> 78
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgaacttt actatcttcg ccttccaccc ggatgacgtt gcaaatctct 300

gccactatac tcatttctcc caacaccatc agggcatctc gcaggtcaga ttcaggaact 360

ctatgggtca ctaagaccag ctgggcgaac tcctcgccgc ttgtcttctg cagcacggta 420

gctatgctca gctgatggct gccaaaaacc ccggcgatgg ccgccaatac cccagggcgg 480

tccttaacca gcatccgaat atagaacttg ctctccactt tgtcgatagg aataatctcc 540

ttggcttcaa aacaagtaca gcccagcatg ccggtggtac catgactgag gttatggcat 600

atctccatta tgtctcccac caccgcgcta gcagtgggca tctgccctgc ccctcttcca 660

aagaacatga cttctcccac cgcgtccccg ttcacgaata cggcgttgta aacccccttt 720

accgcggcca gagggtggtg cataggtatg aaagccgggt ggacccgggc ttgcacccgg 780

ccgtcgattt cctgcgcgat agccaggagc ttcaccacat agcccaattc ataaccgtac 840

ttgatatcta aagggcttaa acgggtaatt ccttcaacgt aaacgttttc gaacgtaacc 900

cggctgttga aggcgatcga agccaggatg gcaattttac gagcagcatc atagccttct 960

acgtctgaag taggatcagc ttcagcatac cccagttcct gcgcctcttt caaagccctc 1020

gaaaactcta ggccttcctc gctcatctta gtaaggatgt agttggttgt cccgttaaca 1080

atccccatta cttctttgat tcgattggca cctagcgaat gcttcaaagg ataaattaag 1140

gggatgcccc caccaacact ggcttcaaaa aagaagtcca ccttgttctc ttcagctgcg 1200

gccagcagtt cctgcccgtg gaccgctatt aaatctttat tggcagtcac cacgttcttg 1260

cctttgcgta acgcctgcaa aataaaggtt cgagccggtt cgatgcctcc tatgagttct 1320

acaacaacac ttatatggtc atcatccagt atgtctttga tatcggcgca aaggacgtct 1380

tcgcttaaac ctagactcaa aaccttctcc gggtcttttt cgaggattcg cttgatcgcg 1440

actccttgtc cggtacggag cgaaataaca tcccggtttg aggctaaaag cttgaccaca 1500

ccggatccaa ccgttccaca acccaagagg ccaatattaa ccactaaggc tgagatcttc 1560

ttcagtgcat tgtagttgaa tgaagggtta ggggggaaat gcccccctat tttttgtcta 1620

gccatcctgc cacgtttgac agggtagcaa tttcgacacg atagggttct ctcttctgcc 1680

gtta 1684

<210> 79
<211> 1237
<212> DNA
<213> Artificial Sequence

<220>
<223> Synthetic Construct- Sequence No. 79, Example 79: designer
      nirA-promoter-controlled Homoserine Kinase DNA construct (1237
      bp)

<400> 79
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgaaggtc ctggtaccgg ccacaacgac caatctcgga gcgggattcg 300

acgtctttgg actcgcgctg gatcttttca acgaagtgga gttttctttc gatacaaaag 360

agacaaccat agaaagtact ggaaaatacg cttcggattt gaaggaccac aatctgtttt 420

tcgaagtctt caggttcttt gagagaaaaa ccgggtacag agttccgcca gtcaggatca 480

agcagacatg caacatccct gtatcgagcg gtcttggatc gagcgccgct gtgatcgtcg 540

cggcactcca cattgcgaac gaaggaacgg gcagaaatct ttcacgggaa gatcttatga 600

aactcgctgt ggagctggaa ggacaccctg acaacgttgt acccgctttc acaggggggc 660

ttgtggtctg ttatcaaaac ggaagtcatc ttgattttga aaagttcgag atcgatcttt 720

ctctcacatt tttcgttcca aacttttcga tgtgcacgaa cgagatgaga aagatccttc 780

cggagaaggt ccctttcgaa gatgcggtct tcaacataaa gaattcatgc cagttccttg 840

caaagatcgc agctggaaag atcaaagagg ctctgaaata cgtgggagat cgacttcacc 900

agaactacag gataaacggc aataagaaga tgaaagagtt tgtggaagcc atcttatcaa 960

aaaatcccga gtactggttt gtgagcggat ccggtccttc tgtttgttcc aatataaatg 1020

actttgaagg gattccctat ctcaaggacg ttctgaagct gagggtgaac aacaggggga 1080

tgatagtttc agaatagtaa ggctgagatc ttcttcagtg cattgtagtt gaatgaaggg 1140

ttagggggga aatgcccccc tattttttgt ctagccatcc tgccacgttt gacagggtag 1200

caatttcgac acgatagggt tctctcttct gccgtta 1237

<210>  80
<211>  1438
<212>  DNA
<213>  Artificial Sequence

<220>
<223>  Synthetic Construct- Sequence No. 80, Example 80: a designer
       nirA-promoter-controlled Threonine Synthase DNA construct (1438
       bp)

<400>  80
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgaaattg ggaatactcg aaaagtacag agaatttctt cccgtaacag     300

acaaaacccc catgctctct ttgaatgaag ggaacactcc tctcataccc ctcgtgaaca     360

tgagcaggga actcggaata aacatctacg taaaatacga aggggccaat ccgacggggt     420

ccttcaaaga cagaggaatg gtcgttgccg tcgcaaaggc actggaagaa ggctcgaaag     480

ccatcatgtg cgcttcaacg gggaacacct ccgcatccgc tgccgcgtac gccgcaaggg     540

caggaataaa ggcgatcgtt ctgataccag aagggaagat cgcactcgga aagctggctc     600

aggcgatgat atacggcgcc gtggtgcttc aggtgagagg gaatttcgac aagtgtctgg     660

aactggtcaa ggagatcaca tccaaatatc ccatcacact cgtgaacagt atcaatccct     720

acagactcga aggtcagaaa acggccgctt ttgagatagt cgacgagctc ggagatgcac     780

cggactacca cttcatcccc gtgggaaacg cgggcaacat ctccgcttac tggatgggat     840

acaaggagta ttatcagcat gggttctcca ccaaactgcc gaagatgatg ggattccagg     900

cggaaggggc cgcccccata gttcgcggtc atcccataga aaacccggag acggtcgcca     960

ctgcaataag gatcggtaac cccgcgaact gggaaaaagc ggtccgggca cgcgatgaat    1020

cgggtggaga catcgacatg gtgagcgacg aagaaatact gcgcgcacag agactcttgg    1080

ctcagaaaga agggatcttc tgtgagcccg catccgctgc atcgatagcg gggcttttga     1140

agaagcacag acagggaatc ttcaggggtg gagagatcgt tgtgtgtacc ctcacaggga    1200

acggtttgaa agatccgaac atcgtcatct cacagcttga acccccaagg atcatagaag    1260

gaagagtaga agagattctg gaggtactcg acatatgata aggctgagat cttcttcagt    1320

gcattgtagt tgaatgaagg gttagggggg aaatgccccc ctattttttg tctagccatc     1380

ctgccacgtt tgacagggta gcaatttcga cacgataggg ttctctcttc tgccgtta      1438

<210>  81

<211>  1600
<212>  DNA
<213>  Artificial Sequence

<220>
<223>  Synthetic Construct- Sequence No. 81, Example 81: designer
       nirA-promoter-controlled Threonine Ammonia-Lyase DNA construct
       (1600 bp)

<400>  81
agaaaatctg gcaccacacc tgacccccat cgagagactc cgaacgtggc aaatggaggg      60

accagagttg ttcagttcac aggtagataa tgtcgcgggt cttgatagtt agcaataaat     120

acagtttcag aatatctgta atacaaaaac tgtatcgaga caagaaaaaa gtagcaaaat     180

ttacaaatgt tcatgattca tctggctaaa ttggatgttc aactgaccca ttgaagacaa     240

gggcaacaac catgttgaca ttagcagaca ttgaacaagc gcgagcgaaa atgaaaggca     300

tcgtccatca aacgccgctt gagcattcgc aaacgttcag ccggctgtct ggcaatgatg     360

tatatatgaa actcgaaaat ttgcaaaaaa cgggctcgtt taaagtaaga ggttcattca     420

ataaaattat gtcgctcacg gaagaagaga gggcgcgcgg cgtcatcgcc gcttcggccg     480

gcaaccacgc ccaaggggtc gcctatgcga gcggcatgct tcatattccg tgcacgatcg     540

tcatgccaaa aggcgcgccg ctcagcaaaa ttgaagcgac gaaaagctac ggggcggaag     600

tcgtgctgta cggcgatgtg tttgacgagt ctttggaata tgcgttagag ttgcagcgtg     660

aacgggggat gacgtttgtt catccgtttg acgacttggc ggtgatggcc ggccaaggga     720

cgatcggctt agagctgatc gagcagcttc ccgacgtcga tgtcgttctt tgtccagtcg     780

gcggcggcgg gttgcttgcg ggggtggcgc ttacgttaaa acagctgaag ccgtcggttg     840

aagtgtacgg cgttgagtca tcggcttgcc ccggcatgac ggcggccata cgccataaac     900

agcccgtctc cattgccgca tcgaatacga tcgccgatgg gattgccgtg aaaaagccgg     960

gcaatattac gtaccaatac attgagcaat acgtcgatgg cgttgtatgc gtggaagagg    1020

cggaaatttc gcggacgatg ctgtatgtgc tcgagcggaa caagctgttg atcgaagggg    1080

cggcagcttg tccgctggcg gcattgttgt atcaaaagct gccgtttcgc ggcaaaaaag    1140

tcgccgccat tttaagcggc ggcaacgtcg atgtgacgct catttcccgc atcatcgagc    1200

gggggctcgt cgaagccggg cgattcgtta cgtttacaac ggtcatctcc gacaagccgg    1260

gccagttgaa caagctgctg cgcattattg cggagcttga ggcaaacgtg atgtcgattc    1320

atcatcagcg catcggcgcc aaagtgctgc caggtcaggc ggaaattcac ttttcgctcg    1380

agacaaaaaa cgaagaccac attcagcaaa tctaccaagt gttgttgaaa gaaggctacg    1440

atgtacagtt ttaccgatga taaggctgag atcttcttca gtgcattgta gttgaatgaa    1500

gggttagggg ggaaatgccc ccctattttt tgtctagcca tcctgccacg tttgacaggg    1560

tagcaatttc gacacgatag ggttctctct tctgccgtta                          1600

<210> 82
<211> 2107
<212> DNA
<213> Artificial Sequence

<220>
<223> Synthetic Construct- Sequence No. 82, Example 82: designer
nirA-promoter-controlled Acetolactate Synthase DNA construct
(2107 bp)

<400> 82
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgttgaca aaagcaacaa aagaacaaaa atcccttgtg aaaaacagag 300

gggcggagct tgttgttgat tgcttagtgg agcaaggtgt cacacatgta tttggcattc 360

caggtgcaaa aattgatgcg gtatttgacg ctttacaaga taaaggacct gaaattatcg 420

ttgcccggca cgaacaaaac gcagcattca tggcccaagc agtcggccgt ttaactggaa 480

aaccgggagt cgtgttagtc acatcaggac cgggtgcctc taacttggca acaggcctgc 540

tgacagcgaa cactgaagga gaccctgtcg ttgcgcttgc tggaaacgtg atccgtgcag 600

atcgtttaaa acggacacat caatctttgg ataatgcggc gctattccag ccgattacaa 660

aatacagtgt agaagttcaa gatgtaaaaa atataccgga agctgttaca aatgcattta 720

ggatagcgtc agcagggcag gctggggccg cttttgtgag ctttccgcaa gatgttgtga 780

atgaagtcac aaatacgaaa aacgtgcgtg ctgttgcagc gccaaaactc ggtcctgcag 840

cagatgatgc aatcagtgcg gccatagcaa aaatccaaac agcaaaactt cctgtcgttt 900

tggtcggcat gaaaggcgga agaccggaag caattaaagc ggttcgcaag cttttgaaaa 960

aggttcagct tccatttgtt gaaacatatc aagctgccgg taccctttct agagatttag 1020

aggatcaata ttttggccgt atcggtttgt tccgcaacca gcctggcgat ttactgctag 1080

agcaggcaga tgttgttctg acgatcggct atgacccgat tgaatatgat ccgaaattct 1140

ggaatatcaa tggagaccgg acaattatcc atttagacga gattatcgct gacattgatc 1200

atgcttacca gcctgatctt gaattgatcg gtgacattcc gtccacgatc aatcatatcg 1260

aacacgatgc tgtgaaagtg gaatttgcag agcgtgagca gaaaatcctt tctgatttaa 1320

aacaatatat gcatgaaggt gagcaggtgc ctgcagattg gaaatcagac agagcgcacc 1380

ctcttgaaat cgttaaagag ttgcgtaatg cagtcgatga tcatgttaca gtaacttgcg 1440

atatcggttc gcacgccatt tggatgtcac gttatttccg cagctacgag ccgttaacat 1500

taatgatcag taacggtatg caaacactcg gcgttgcgct tccttgggca atcggcgctt 1560

cattggtgaa accgggagaa aaagtggttt ctgtctctgg tgacggcggt ttcttattct 1620

cagcaatgga attagagaca gcagttcgac taaaagcacc aattgtacac attgtatgga 1680

acgacagcac atatgacatg gttgcattcc agcaattgaa aaaatataac cgtacatctg 1740

cggtcgattt cggaaatatc gatatcgtga aatatgcgga aagcttcgga gcaactggct 1800

tgcgcgtaga atcaccagac cagctggcag atgttctgcg tcaaggcatg aacgctgaag 1860

gtcctgtcat catcgatgtc ccggttgact acagtgataa cattaattta gcaagtgaca 1920

agcttccgaa agaattcggg gaactcatga aaacgaaagc tctctagtaa ggctgagatc 1980

ttcttcagtg cattgtagtt gaatgaaggg ttagggggga aatgcccccc tattttttgt 2040

ctagccatcc tgccacgttt gacagggtag caatttcgac acgatagggt tctctcttct 2100

gccgtta 2107

<210> 83
<211> 1405
<212> DNA
<213> Artificial Sequence

<220>
<223> Synthetic Construct- Sequence No. 83, Example 83: designer
      nirA-promoter-controlled Ketol-Acid Reductoisomerase DNA
      construct (1405 bp)

<400> 83
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgttttac ctctttaagc caaggcatca tcgccctaag ttccttacct 300

actttttcta tcaggtgctc ctgccctttc ctccgcaagg cactgaagac cggacggccc 360

acttggaatt cgagcaagag ctcccgggca aaagttccat cctggatggc agccaaaacc 420

tttttcatct cagccctggt gttttcgttt attatgcgcg gacctaccgt gaggtcgccg 480

tactcagcgg tatcactgac cgagtaccgc ataaggccga taccgccttc gtatataagg 540

tctactatga gcttaagctc gtgcaggcac tcgaaatagg ctatctccgg ctggtatcct 600

gcctctacca aggtatcaaa accggcttta ataagttcag tgaccccgcc acagagcaca 660

cattgttctc cgaataggtc ggtctcggtt tcttctttaa aagtagtagc gatgacacct 720

gcacgggtac agccgatacc tttagcatag gctaaccccg tttccaaggc tttccccgta 780

tggtcattat gtacggctat gagcccgggg actcccactc cttgcctgta catacgcctg 840

accagatgac caggactctt aggcgctacc atgaagacat cgacggaagg cggaggcaca 900

atttgcccga aatgtatgtt gaacccatga gaaaatccca acgcatcgcc ttcgttaagg 960

taaggttcga tcttttcccg gtaaaccttg gcctggatat catccggcac caaaatctgg 1020

attatctggg cagctcgggc cgcttcatct accggtaaag gcgtaagccc gtcggctaca 1080

acctgattcc actccgcagt ggtaaaatcg tcctccggct tacgcaaccc taccaccact 1140

tcgagaccac tgtcgtgaag gttctgggcc tgagcgtgtc cctggctgcc ataaccgatc 1200

acggcaatgg tcttgccttt aagcaggtca aggtttgcat cagcatcata atacatccta 1260

gccattaagg ctgagatctt cttcagtgca ttgtagttga atgaagggtt aggggggaaa 1320

tgccccccta ttttttgtct agccatcctg ccacgtttga cagggtagca atttcgacac 1380

gatagggttc tctcttctgc cgtta 1405

<210> 84
<211> 2056
<212> DNA
<213> Artificial Sequence

<220>
<223> Synthetic Construct- Sequence No. 84, Example 84: designer
      nirA-promoter-controlled Dihydroxy-Acid Dehydratase DNA construct
      (2056 bp)

<400> 84
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggggctt cctgaagatt gcccccttgc tcgccgactg tacgaagaat 300

gcgtaccttc tcaggtaatc gctgtccact tctttcacca gaggcgtgaa ctctttcatt 360

cttctttcga actcttcgtc tgagatcaag agattcaggg ttctcttttc aaaatctatc 420

tcgatgaggt ccccgtcttt cacgatacct ataggaccgc cttctgccgc ttctggagaa 480

acgtgaccta tcacggcacc gtgcgatcca cccgagaacc taccgtctgt gatgagagcc 540

acgtcctccg caaggcccat ccccacgatg gcggaggtgg gtgagagcat ctctctcatc 600

ccgggaccgc ccttcggccc ttcgtagcgg atcacaacca catctccttt tttgatcttt 660

ccagatagaa tggcttttgt cgcctcttct ccgtcttcaa agacgacggc cgggccaacg 720

tggtgcatca tcttctcggg aacaccggag agtttggcaa ccgctccttc tggagcgagg 780

ttcccgaaga ggataccgag tccgccctct ttgtggtacg gattatcgaa gggcctgatc 840

acatcttcat tcaggatctt agcctctctg acgagatctc caatctttct caaatagatg 900

gtcatggcgt cttccttcaa aagaccattt tcctggagac gtttcatcac agcgtagata 960

ccaccagcat cgtcgagatc ctggatgtgg tacggaccaa cgggagagat gttgcagatg 1020

tgaggaatct tcctgctgag ttcgtcaaag agctttatat cgaaatctat tccaaaactc 1080

tcggctatcg ccttcaaatg cagaactgtg ttcgtggaac ctcccgttgc gaggtccacc 1140

atgacagcgt tcatgaaaga gtccagagtg acgatatccc ttggttttac atctcttttc 1200

acgagttcca caacgagcat ccccgcttct ttcgccattc tcaacctctt cgcgtggacg 1260

gccggtacag tcccattccc cctcggtgca attccgagag cttccgccag agagttcatc 1320

gtgttcgcgg tgaacaatcc agcacacgaa ccggcaccgg gacacgcgag gtcttctatc 1380

gctttgagcg tttcttcatc gacttttccc actttgtatc caccaaccgc ttcgaagacg 1440

gtgatgagat cgatgtctct gccgttgtag cgacctgcga gcatgggacc gccggatatc 1500

agaacggacg ggatgttcaa tcttcccatg gccatcatca tgccgggtgt gatcttgtcg 1560

cagttgggga cgaagaccaa accatcgaag gggaaaccgc ttgcaacgat ctctatggag 1620

tccgctatga gttccctcga gggcaaggaa aacttcatcc ccctgtgatc cattgctatt 1680

ccgtcacaga tcccgatcgt tggaaagacg aagggaactc ccccggccat tctcacaccg 1740

gctttcaccg cttcaacgac cttgtcaagg tggacatggc cgggaatgat ctcgttccac 1800

gaggacacta tgccgatgaa aggccttcgc atttcgtcgt ccgttattcc gagcgctttc 1860

aaaagtgatc tatggggagc cctttcgaga cctttcttta tcacatcact cctcattaag 1920

gctgagatct tcttcagtgc attgtagttg aatgaagggt taggggggaa atgcccccct 1980

attttttgtc tagccatcct gccacgtttg acagggtagc aatttcgaca cgatagggtt 2040

ctctcttctg ccgtta 2056

<210> 85
<211> 1360
<212> DNA
<213> Artificial Sequence

<220>
<223> Synthetic Construct- Sequence No. 85, Example 85: designer
 nirA-promoter-controlled 2-Methylbutyraldehyde Reductase DNA
 construct (1360 bp)

<400> 85
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggcttct gtaaatgact actttgagaa cgccaagacg acgtacttta 300

ctttgagatc gggtgacaag atccccgctg ttggattggg tacttggcaa tcacccacca 360

acgagactaa agaggcagtc aagtacgctt tgcagcacgg ttaccgtcac atcgatgctg 420

ccgccattta tggtaacgaa gacgaggttg gtgacggtat caaggagagt ggaatccctc 480

gtgaccaaat ctgggtcaca tctaagctct ggtgcaatgc tcatgctccc gaggctgtcc 540

ccaaggcttt ggagaagacc ttgcgtgagc tgaaacttga ttaccttgac ctttacctca 600

tccactggcc tatttctttg aagaccggcg atgacttggt tcccaaggac aaggacggca 660

acaccatcac tgtcgaaatt cccctcgagg acacctggaa ggctatggag ggtcttgtga 720

agtccggcaa ggtgaagaac attggtattt ccaatttcaa caacgaagag ttggatcgta 780

ttttgaaggt tgccgagatt cctcctgccg tccaccaaat ggaaactcat ccttacttga 840

agcagacgga gttcattgag aagcacaaga agcttggcat tcacgtcacc gcttactcgc 900

ctttggccaa ccaaaatgct ctttacggca atgccgttcc caagttgatt gagcacaaga 960

ctcttgtcga cattgccaag accaagggtg agggcgtcac tggtgccaac attgctattt 1020

cttgggcagt caagcgcggt acttcggtta ttcctaagtc tgttcatgcc aacagaatta 1080

agagcaactt cctcgttgtt cccttgactg atgacgagat gaaggccatc gataacattg 1140

gtgtcagcaa gcgtttcaat tggagcaaag ttttctgcaa tgagaattgt ttctacggtc 1200

ttgaggatgg tcctcagtaa taaggctgag atcttcttca gtgcattgta gttgaatgaa 1260

gggttagggg ggaaatgccc ccctattttt tgtctagcca tcctgccacg tttgacaggg 1320

tagcaatttc gacacgatag ggttctctct tctgccgtta 1360

<210> 86
<211> 1420
<212> DNA
<213> Artificial Sequence

<220>
<223> Synthetic Construct- Sequence No. 86, Example 86: designer
 nirA-promoter-controlled 3-Methylbutanal Reductase DNA construct
 (1420 bp)

<400> 86
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgtcagtt ttcgtttcag gtgctaacgg gttcattgcc caacacattg 300

tcgatctcct gttgaaggaa gactataagg tcatcggttc tgccagaagt caagaaaagg 360

ccgagaattt aacggaggcc tttggtaaca acccaaaatt ctccatggaa gttgtcccag 420

acatatctaa gctggacgca tttgaccatg ttttccaaaa gcacggcaag gatatcaaga 480

tagttctaca tacggcctct ccattctgct ttgatatcac tgacagtgaa cgcgatttat 540

taattcctgc tgtgaacggt gttaagggaa ttctccactc aattaaaaaa tacgccgctg 600

attctgtaga acgtgtagtt ctcacctctt cttatgcagc tgtgttcgat atggcaaaag 660

aaaacgataa gtctttaaca tttaacgaag aatcctggaa cccagctacc tgggagagtt 720

gccaaagtga cccagttaac gcctactgtg gttctaagaa gtttgctgaa aaagcagctt 780

gggaatttct agaggagaat agagactctg taaaattcga attaactgcc gttaacccag 840

tttacgtttt tggtccgcaa atgtttgaca aagatgtgaa aaaacacttg aacacatctt 900

gcgaactcgt caacagcttg atgcatttat caccagagga caagataccg gaactatttg 960

gtggatacat tgatgttcgt gatgttgcaa aggctcattt agttgccttc caaaagaggg 1020

aaacaattgg tcaaagacta atcgtatcgg aggccagatt tactatgcag gatgttctcg 1080

atatccttaa cgaagacttc cctgttctaa aaggcaatat tccagtgggg aaaccaggtt 1140

ctggtgctac ccataacacc cttggtgcta ctcttgataa taaaaagagt aagaaattgt 1200

taggtttcaa gttcaggaac ttgaaagaga ccattgacga cactgcctcc caaattttaa 1260

aatttgaggg cagaatataa taaggctgag atcttcttca gtgcattgta gttgaatgaa 1320

gggttagggg ggaaatgccc ccctattttt tgtctagcca tcctgccacg tttgacaggg 1380

tagcaatttc gacacgatag ggttctctct tctgccgtta 1420

<210> 87
<211> 1540
<212> DNA
<213> Artificial Sequence

<220>
<223> Synthetic Construct- Sequence No. 87, Example 87: designer
nirA-promoter-controlled 3-Ketothiolase DNA construct (1540 bp)

<400> 87
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgcgtgaa gcggtcattg tcgaagcggt caggacgccg gtcggcaagc 300

ggaacggcgt cttccgggac gttcatccgg tccatttggc cgcggtggtg ctcgatgaag 360

tcgtgcgccg ggccggcatg gacaaagggg cggtggaaga catcgtcatg ggctgcgtga 420

cgccggtcgc cgaacaaggg tacaacatcg gccggctggc ggcgcttgag gccggattcc 480

cgatcgaagt gccggcagtg caaatcaacc gaatgtgcgg ctcggggcag caggcgattc 540

atttcgccgc ccaggaaatc cgctccggcg atatggatgt cacgatcgcc gccggggtcg 600

aaagcatgac gaaagtgccg attttaagcg atggcaacga gcggacgatt ccgccgtcgc 660

tgcatgaaaa atacgaattc atccaccaag gcgtctcggc tgagcggatc gccaaaaaat 720

acggcctaac gcgcgaggag cttgacgcct acgcgtacga aagccatcaa cgcgccttgg 780

cggccttgcg cgaagggaag tttcgcgcgg aaatcgtccc ggtgaaaggg cttgaccgcg 840

atggccgcga aatccttgtc accgatgatg aagggccgcg ggccgacaca tcgccggaag 900

cgctcgccgc gctcaagccg gtgtttcaag aagacggtct catcaccgct ggcaatgcga 960

gccaaatgag cgacggggcg gccgctgtgc ttttgatgga acgggaggcg gcgaggcggt 1020

tcggactgaa gccgaaagcg cgcattgtcg cgcaaacggt cgtcggctcc gacccgacgt 1080

atatgctcga tggcgtcatt ccggcgacga ggcaagtgct gaaaaaagcc ggcctctcga 1140

tcgatgacat cgacctcatt gaaatcaacg aagcgttcgc cccggtcgtg ctcgcctggc 1200

aaaaagaaat cggcgctccg cttgagaagg tgaatgtcaa cggcggcgcc attgcgcttg 1260

gccatccgct cggcgccacc ggtgcgaagc tcatgacgtc gcttgttcat gaacttgaac 1320

ggcgcggcgg ccgctatggg ctattgacga tttgcatcgg ccacgggatg gcgacggcca 1380

cgatcatcga gcgggagtaa taaggctgag atcttcttca gtgcattgta gttgaatgaa 1440

gggttagggg ggaaatgccc ccctattttt tgtctagcca tcctgccacg tttgacaggg 1500

tagcaatttc gacacgatag ggttctctct tctgccgtta 1540

<210> 88
<211> 1231
<212> DNA
<213> Artificial Sequence

<220>
<223> Synthetic Construct- Sequence No. 88, Example 88: designer
 nirA-promoter-controlled 3-Hydroxyacyl-CoA Dehydrogenase DNA
 construct (1231 bp)

<400> 88
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgatactt aaagaaacct tctccggtct tacgccccag cttccctgca 300

cggaccatct taaccaagag cgggcaagga cgatacttgt catcgccaaa ctcgcgatgc 360

aaagtctgca tgatagccaa acatatatct agcccaatca tgtctgctaa agcgagggga 420

cccataggat gccctgctcc gagcttcatg gaggtatcca cgtcctcggg gcttgcaacc 480

ccctccatga cggcatacat accttcgttc agcattggaa taagcaggcg gttgacaaca 540

aagccaggag cttcgttgat ctcaaccggg gtcttgccca gcttaattga aagatccttg 600

atggtattaa aagtttcctg gctagtagaa gcccctttga taatctcgat aagcttcatt 660

gccggtaccg ggttgaagaa atgcatgcct attaccctgt ctgcccgctt ggttgctgct 720

cctatctcgg ttatgctcag agctgatgtg ttagaagcca ggatacattc aggcttgcag 780

atctcgtcca gttccttgaa aatcgctttt ttgatgtcca tattctcgat agcagcttca 840

attaccacat ccacatcttt ggccgcagcc atgtcgaccg taccgctaat cctggccatc 900

accgcgttct tgtcatctgc gctcatcttg cccttttcga ccattttgct gagacccttg 960

tcgatgcctt ttataccatt gtcaacaaac tcttgtttaa tatcacgtac gattacttcg 1020

aacccagctt gagcagcgac ttgaacaatc ccagctccca tagtacctgc gcctaaaacc 1080

attattttca ttaaggctga gatcttcttc agtgcattgt agttgaatga agggttaggg 1140

gggaaatgcc cccctatttt ttgtctagcc atcctgccac gtttgacagg gtagcaattt 1200

cgacacgata gggttctctc ttctgccgtt a 1231

<210> 89
<211> 1162
<212> DNA
<213> Artificial Sequence

<220>
<223> Synthetic Construct- Sequence No. 89, Example 89: designer
      nirA-promoter-controlled Enoyl-CoA Dehydratase DNA construct
      (1162 bp)

<400> 89
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgacggtt cgactggaat acgatggcgg gttcgcgcac ctgacgctca 300

gccgcccgca ggtcctgaat gcgctcagtt tcgagctgct cgccgagttg agccgggcgc 360

ttgccggcgt cgccgaatcc gatgcgcgcg ccctgatcgt cacgggcgag ggcgacaagg 420

cgttctgcgc cggcgcggac attcccgagc tgatgaatcg gccgctcatg caagagctcg 480

aaggggccgc gaaaggccag gcggtgttca gccggatcgc cgagctgaag attccgtctg 540

tcgccgtcat ccagggttat gccttcggcg gcgggctgga gcttgccctg gcatgcacat 600

tccgcgttgc cactgatcgc gcccgcatgg ggctgcccga ggtcaagctc ggcctgatcc 660

cgggttatgg cggaacgcag cgtctgccga ggctgatcgg cgaggggcgc gcactcgacc 720

tgatcatgtc cggccgcacg atagacggcg gggaagccga gcgaatcggc ctggtcaatc 780

gcatagacaa cgaggggacg cccctggaga tcggcaagcg gtttctggag ccttatctca 840

agcacagtct ctgcgccttg tattttgccc gcgaggccgt gcagagggga ggcggtgtcg 900

ccattgcgga tggcctgcgc atcgagcggg atctttccac gctggcttac cggagccagg 960

atgcggccga ggggctgcgc gcttttgtgg aaaaacggcc cgcgtctttc aaggactgct 1020

gataaggctg agatcttctt cagtgcattg tagttgaatg aagggttagg ggggaaatgc 1080

ccccctattt tttgtctagc catcctgcca cgtttgacag ggtagcaatt tcgacacgat 1140

agggttctct cttctgccgt ta 1162

<210> 90
<211> 1561
<212> DNA
<213> Artificial Sequence

<220>
<223> Synthetic Construct- Sequence No. 90, Example 90: designer
      nirA-promoter-controlled 2-Enoyl-CoA Reductase DNA construct
      (1561 bp)

<400> 90
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggccggc gcgcagcagg atcttgccgc tgcgtccggg cttgtcgctg 300

gccgcggcgg ccttggcggc atcgtgcagg tcgaacaccg cttccaccgg cagcgccagg 360

ctgccatcga gcgcggcggt gagcagttcg ccgatcatgc ggcgcttgtc ctcggccttg 420

gtggcctgca tcaccttgct gccccagaag ccacgcacgg tggcctgctt gaagatcaca 480

tcgccgctgg atatctgcag cggctcgccg gtcatcgagc caaaggaaat cagctcgccg 540

ccttcggcca gcaaggccat cagctcaccc gctgcattgc cggccaccga atcgatggcg 600

cgcacgatgg gcgcatcgcc ggccagcgcg cgcaccttgt cctgccagcc tgcttgcgca 660

gtggagattg cgttgccgat gcccagcgct ttcagctcgt ccacgccggc gtcgcggcgc 720

accaggttga tcacgttgat gccgcgtgcg gcggcgagca tcgccaccgt cttgccgacc 780

gcaccgttgg cggtgttctg cacgatccag tcgccctgtt tcacctgcag gaattcgatc 840

agcatcagcg cgctcagcgg catggcgatc aactggcaac cacgctcgtc gtccaggcca 900

tccggcaacg gcaccacgcc ggaggcgtcg gcaaggaagt actcggccca ggcctcatgc 960

acaccggcgg cgaccacgcg ctggccaacc tgcaagccct cgacaccctc acccagcgca 1020

tcgatgacac ccgccgcttc gctgccgccg atggctggca gttccggctt gtagccgtaa 1080

ttgccgcgca cggtccacag gtcatggtta tggatcggcg cgcgccgcat cgcaacgcgc 1140

acctggccct tgcctggctg cggcgtgggg cgctcgccca gttcgagcac cttggccgga 1200

tcgccgaatt gggtatggat ggctgcgcgc atggaggtct cctgccgggc acgctcttgc 1260

tgcgacgcgc ccgatcgttg tgaaaggtgg cgcgatgcta tcggcagggc tgcaaggaag 1320

ggatgaagcg aacggaactg ctgtgtgaag ttgttggcgt gcgcgcgtag tgacgatgct 1380

ctgctgcagc gccggaggac tgcgtgcagg ccgaccctca ttaaggctga gatcttcttc 1440

agtgcattgt agttgaatga agggttaggg gggaaatgcc cccctatttt ttgtctagcc 1500

atcctgccac gtttgacagg gtagcaattt cgacacgata gggttctctc ttctgccgtt 1560

a 1561

<210> 91
<211> 1747
<212> DNA
<213> Artificial Sequence

<220>
<223> Synthetic Construct- Sequence No. 91, Example 91: designer
      nirA-promoter-controlled Acyl-CoA Reductase DNA construct (1747
      bp)

<400> 91
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgtagttg tctactaact acgtaagtca tttcctgcaa attgtgcatt 300

ccatcatgag aagttcctgg ataatgtgct ggcatttccc ctggcttagt gactatgctt 360

acaccaagta acgtcatttt ttcaataaat tcgtggtcgt caccactata ccccatagtt 420

tgaaggaatt gctttaaatt ttcttcaaca taatcatgta cttcatcttt attttcatat 480

gggcaaacaa atataagtct attaaagcat ctatcaatat cctttttttc aggcattctg 540

ttgcttaaaa tcacagtata gtcggcatta cacgatgcaa acacctttgc cggtttctcc 600

tcatcaacac tatattttaa taaacaatac tgtcggtctt gtattgattt catagaactc 660

caaggactca gatatgcctt tgggaaaacc tctgtaagct ctttcaagct ttctgctaca 720

gtttctgcaa gaatattaat gtctattttt ttattggcaa ataccatcct aggagacaaa 780

caggcttttt gctcccaaca tatcacatca ctagcaatac cttttgcaat agtcttaata 840

tcttctactt tatctataac ttcaaaacta attttagcac catgcattat taaatgagaa 900

ttatattttg cacataactc tgccattatc cttcctgaat attctccacc ccaatgtata 960

acacaatcca tttctctcac gacagtctca tatatatcag aacattcact actaaagtat 1020

aaaacagata gtctatcttt tatacttgga tcaagctgta ccaaactttc atagaaagca 1080

tacgcaaaat atggttcatc agcagaaacc tttactaaat tacagttctt tgataataac 1140

cccataccta tacttgtcgg aacaactaca aatgcatttc cagaaatatt atgaaacatc 1200

acacctcttg gctgtctatg cacagctcca taacttgttg gaacccaatt atctagtata 1260

tcaatgttac caagttcttc tttaatgatt atctcaagat tttctcttaa aagcattctc 1320

atactatttt caagttcata tgttacaagt tcttcacttt gattcaatat gttagctaat 1380

ctttctatat gtactttgga gtatcctcta tcaagccaca atcttccaca cctatccaaa 1440

agatcaattg tatcctgcac tgatattgca tgactcttac ttttactttt tctaagtctt 1500

tttatttcct caattacctg atctctactt gagtaagtta attctaattc caggccattt 1560

atattcttta ttaaaacatt actttcacaa acagtttcgc tcttcattaa ggctgagatc 1620

ttcttcagtg cattgtagtt gaatgaaggg ttagggggga aatgcccccc tattttttgt 1680

ctagccatcc tgccacgttt gacagggtag caatttcgac acgatagggt tctctcttct 1740

gccgtta 1747

<210> 92
<211> 1450
<212> DNA
<213> Artificial Sequence

<220>
<223> Synthetic Construct- Sequence No. 92, Example 92: designer
      nirA-promoter-controlled Hexanol dehydronase DNA construct (1450
      bp)

<400> 92
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggaactc gacctcgacg gtcccggggt tggtgaagtg ctgatcaagt 300

acaccgccgc ggggttgtgc cattcggacc tgcacttgac cgacggggac ctaccgccgc 360

gctatccaat cgtcgggggg cacgaggggt caggcatcat cgaggacgtc ggacctgggg 420

tcaccaaggt caaaccaggc gatcacgttg tttgcagctt catcccgaac tgcggaacct 480

gtcggtactg cgccaccgga cgctccaacc tctgcgatat gggcgccacc atcctcgaag 540

ggtgcatgcc cgacggcagt taccggttcc acagtaacgg cctggatttc ggtgcgatgt 600

gcatgctcgg cacattctcc gaacgcgcaa ctatctccca gcattcggtg gtcaagatcg 660

acgactggct gccgctcgag accgcggtgg tcgtcggctg cggcgtgccg actggctggg 720

gcacctccgt ctatgccggc ggggttcgtt gcggtgacac caccgtcatc tatggcgtcg 780

gcggcctggg agtcaacgcc gtccaaggcg cggtgagtgc gggcgcgaag tacatcgtgg 840

tcgtcgatcc ggttgcgttc aaacgcgaca ccgcgctcaa gttcggcgcc acccacgcgt 900

tcgccgacgc cgccaccgcc gcggccaagg tcgacgaact gacctgggga cagggtgccg 960

atcaggcgct gatcctggtc ggcaccgtcg acgaggacgt ggtctcggcg gcgactgcgg 1020

tgatcggtaa gggaggcacc gtcgtgatca ccggactggc ggacccagca aagctcacgg 1080

tgcacgtttc gggaacggac ctgacgctta acgagaagac aatcaagggc acgttgttcg 1140

gctcgtccaa tccgcaatac gacatcgtac ggctgctccg tctctacgac gccggccagc 1200

taaaactcga cgatctgatc accacccgat acacgctcga ccaggtcaac cagggctacc 1260

aggatctgcg agacggcaag aacatccgcg gcgtgatcat ccacgcctga taaggctgag 1320

atcttcttca gtgcattgta gttgaatgaa gggttagggg ggaaatgccc ccctattttt 1380

tgtctagcca tcctgccacg tttgacaggg tagcaatttc gacacgatag ggttctctct 1440

tctgccgtta 1450

<210> 93
<211> 1074
<212> DNA
<213> Artificial Sequence

<220>
<223> Synthetic Construct- Sequence No. 93, Example 93: designer
      nirA-promoter-controlled Octanol Dehydrogenase DNA construct
      (1074 bp)

<400> 93
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgttggga ggccaagaag ccgctggtga ttgaggacat tgaggtggcg 300

ccacctcagg cttggcaggt tcgcatcaag attacagcca ctggcgtttg ccacacggat 360

tctttttcgt tgagcggctc tgatcctgag ggtctctttc ccgtggtcct tggccatgag 420

ggcgccggca tcgtggagag cgttggcgag ggcgtaacca actttaaggc cggcgatcat 480

gtcattgccc tctacatacc ccagtgcaat gagtgcaaat tctgcaagag cggcaagaca 540

aatctctgcc agaagattcg cctcacccag ggcgctggtg tcatgcccaa tggatcctcc 600

cgcttgtcgt gcaagggtca gcagctgttc catttcatgg gcacctcaac tttcgccgag 660

tacgcggtgg tggccgacat atcggtgacc aaaatcaacg agtcggctcc attggagaag 720

gtgtgccttc tgggctgtgg catttccacg ggctatggtg ccgccttgaa cacctttagg 780

tggaacctgg cagcacttgc gccgtctggg gtctgggtgc tgttggactg gcagtgggtc 840

tgggctgcaa gaaggctggc gccgccaagg tctacggcat cgacatcaat ccctccaaat 900

tcgagctggc caggaagttc ggcttcaccg actttaaggc tgagatcttc ttcagtgcat 960

tgtagttgaa tgaagggtta ggggggaaat gcccccctat tttttgtcta gccatcctgc 1020

cacgtttgac agggtagcaa tttcgacacg atagggttct ctcttctgcc gtta 1074

<210> 94
<211> 1096
<212> DNA
<213> Artificial Sequence

<220>
<223> Synthetic Construct- Sequence No. 94, Example 94: designer
      nirA-promoter-controlled Short Chain Alcohol Dehydrogenase DNA
      construct (1096 bp)

<400> 94
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgaaggtt gccgtaatta ctggggcatc ccgtggaatc ggggaagcta 300

tagcaaaggc ccttgctgaa gatggatatt cccttgcctt aggggctaga agtgttgata 360

ggttagagaa gattgccaag gaactcagcg aaaaacatgg ggtggaggta ttttacgact 420

acctcgatgt atcaaaacca gaaagcgttg aagagtttgc aaggaaaacg ctagctcact 480

ttggagatgt ggacgttgtt gtggccaatg cggggcttgg ttactttggt aggcttgaag 540

agcttacaga agagcagttc cacgaaatga ttgaagtaaa ccttttggga gtttggagaa 600

caataaaagc tttcttaaac tccttaaagc ggactggagg agtggctatt gttgttactt 660

cagatgtttc tgcaaggcta cttccatacg gtggaggtta tgtggcaact aaatgggctg 720

caagagcatt ggtaaggacc ttccagattg agaatccaga tgtgaggttc ttcgagctaa 780

gacctggagc agtagataca tattttggag ggagcaaagc tgggaagcca aaggagcaag 840

ggtatttaaa acctgaggaa gttgctgagg cagtaaaata cctcctaaga cttccaaagg 900

atgttagggt tgaggaatta atgttgcgct caatttatca aaaacctgag tattgataag 960

gctgagatct tcttcagtgc attgtagttg aatgaagggt taggggggaa atgcccccct 1020

attttttgtc tagccatcct gccacgtttg acagggtagc aatttcgaca cgatagggtt 1080

ctctcttctg ccgtta 1096

<210> 95
<211> 1438
<212> DNA
<213> Artificial Sequence

<220>

<223>  Synthetic Construct- Sequence No. 95, Example 95: designer
      Synechococcus sp. strain PCC 7942 nirA-promoter-controlled
      NADPH-dependent Glyceraldehyde-3-Phosphate-Dehydrogenase DNA
      construct (1438 bp)

<400>  95
agaaaatctg gcaccacacc cttcttgcag aacatgcatg atttacaaaa agttgtagtt      60

tctgttacca attgcgaatc gagaactgcc taatctgccg agtatatgat gtcaacgaat     120

attgcaatta atggaatggg tagaattgga agaatggtgc taagaatagc actaaagaat     180

gaagcattga atgtagttgc catcaatgct agctatcctc ctgaaacaat tgcacattta     240

attaattatg acacaacaca tgggagatac gataaaagag tagaacctat tgaaagtgga     300

attcgagtgg aaggccatga tattaaatta gtgtctgata gaaacccaga aaatttaccc     360

tggaaagatt tagaaataga tatcgtcatt gaagcgaccg gtaaatttaa ccatggtgat     420

aaagctaagg cacatattca agcaggagct aaaaaagtgt tattgacagg accatcaaaa     480

ggcggaaaag tacagatggt ggttaaaggt gttaacgatc aagacttaga tacagataca     540

tatgacatat ttagtaatgc gtcgtgtact acgaattgta tcggaccagt tgcaaaagtt     600

ttaaatgata gttttggcat tgaaaatggc ttaatgacaa cggtacatgc aattacaaat     660

gatcaaaata atatagataa tccgcataaa gatttgagaa gagcgcgttc ttgtggggaa     720

agtattatac caacatcaac aggtgctgct aaagcattaa aagaagttat gccagaattg     780

aatggcaaac tacatggcat agcacttcgt gtgccaactc aaaatgtatc attagttgat     840

ttagtcattg atttaaaaca aaaagtgaca gtagatgaag ttaatcatgc atttagagat     900

gcaaacttac aaggaattat tgatgttgaa gaggcccctc tagtttctaa ggactataat     960

acaaatcctc attcagcagt tatagatgct aaaaatacaa tggtcatggg agataataag    1020

gttaaagtta tagcctggta tgataacgaa tggggatatt ctaatagagt agttgaggta    1080

gcaaatcaac ttggagaact aattaaataa taatagtgat cccggccgct actaaagcct    1140

gatttgtctt gatagctgct cctgcctttg ggcaggggct tttttctgtc tgccattctt    1200

gaggatggcg gactctttcc cttttgctct acgcccatga atgcgatcgc agtctcccct    1260

gtccagcacg ttggagtgat tggtggtggc cagttagctt ggatgctggc accagcagcg    1320

caacagttgg ggatgtcgct gcacgttcaa acacccaatg atcacgaccc agcagtagcg    1380

atcgcggatc aaaccgtatt agcagcagtt gctgacgcgg ttctctcttc tgccgtta      1438

<210>  96
<211>  1447
<212>  DNA
<213>  Artificial Sequence

<220>
<223>  Synthetic Construct- Sequence No. 96, Example 96: designer
      Synechococcus sp. strain PCC 7942 nirA-promoter-controlled

NAD-dependent Glyceraldehyde-3-Phosphate-Dehydrogenase DNA
construct (1447 bp)

<400> 96
agaaaatctg gcaccacacc cttcttgcag aacatgcatg atttacaaaa agttgtagtt 60

tctgttacca attgcgaatc gagaactgcc taatctgccg agtatatgat ggcagtaaaa 120

gtagcaatta atggttttgg tagaattggt cgtttagcat tcagaagaat tcaagaagta 180

gaaggtcttg aagttgtagc agtaaacgac ttaacagatg acgacatgtt agcgcattta 240

ttaaaatatg acactatgca aggtcgtttc acaggtgaag tagaggtagt tgatggtggt 300

ttccgcgtaa atggtaaaga agttaaatca ttcagtgaac cagatgcaag caaattacct 360

tggaaagact taaatatcga tgtagtgtta gaatgtactg gtttctacac tgataaagat 420

aaagcacaag ctcatattga agcaggcgct aaaaaagtat taatctcagc accagctact 480

ggtgacttaa aaacaatcgt attcaacact aaccaccaag agttagacgg ttctgaaaca 540

gttgtttcag gtgcttcatg tactacaaac tcattagcac cagttgctaa agttttaaac 600

gatgactttg gtttagttga aggtttaatg actacaattc acgcttacac aggtgatcaa 660

aatacacaag acgcacctca cagaaaaggt gacaaacgtc gtgctcgtgc agcggcagaa 720

aacatcatcc ctaactcaac aggtgctgct aaagctatcg gtaaagttat tcctgaaatc 780

gatggtaaat tagatggtgg tgcacaacgt gttcctgtag ctacaggttc attaactgaa 840

ttaacagtag tattagaaaa acaagacgta acagttgaac aagttaacga agctatgaaa 900

aatgcttcaa acgaatcatt cggttacact gaagacgaaa tcgtttcttc agacgttgta 960

ggtatgactt acggttcatt attcgacgct acacaaactc gtgtaatgtc agttggcgac 1020

cgtcaattag ttaaagttgc agcttggtat gataacgaaa tgtcatatac tgcacaatta 1080

gttcgtacat tagcatactt agctgaactt tctaaataat aatagtgatc ccggccgcta 1140

ctaaagcctg atttgtcttg atagctgctc ctgcctttgg gcaggggctt ttttctgtct 1200

gccattcttg aggatggcgg actctttccc ttttgctcta cgcccatgaa tgcgatcgca 1260

gtctcccctg tccagcacgt tggagtgatt ggtggtggcc agttagcttg gatgctggca 1320

ccagcagcgc aacagttggg gatgtcgctg cacgttcaaa cacccaatga tcacgaccca 1380

gcagtagcga tcgcggatca aaccgtatta gcagcagttg ctgacgcggt tctctcttct 1440

gccgtta 1447

<210> 97
<211> 2080
<212> DNA
<213> Artificial Sequence

<220>
<223> Synthetic Construct- Sequence No. 97, Example 97: designer
Synechococcus sp. strain PCC 7942 nirA-promoter-controlled 2-Keto

Acid Decarboxylase DNA construct (2080 bp)

<400> 97
agaaaatctg gcaccacacc cttcttgcag aacatgcatg atttacaaaa agttgtagtt 60

tctgttacca attgcgaatc gagaactgcc taatctgccg agtatatgat gtatacagta 120

ggagattacc tgttagaccg attacacgag ttgggaattg aagaaatttt tggagttcct 180

ggtgactata acttacaatt tttagatcaa attatttcac gcgaagatat gaaatggatt 240

ggaaatgcta atgaattaaa tgcttcttat atggctgatg gttatgctcg tactaaaaaa 300

gctgccgcat ttctcaccac atttggagtc ggcgaattga gtgcgatcaa tggactggca 360

ggaagttatg ccgaaaattt accagtagta gaaattgttg gttcaccaac ttcaaaagta 420

caaaatgacg gaaaatttgt ccatcataca ctagcagatg gtgattttaa acactttatg 480

aagatgcatg aacctgttac agcagcgcgg actttactga cagcagaaaa tgccacatat 540

gaaattgacc gagtactttc tcaattacta aaagaaagaa aaccagtcta tattaactta 600

ccagtcgatg ttgctgcagc aaaagcagag aagcctgcat tatctttaga aaaagaaagc 660

tctacaacaa atacaactga acaagtgatt ttgagtaaga ttgaagaaag tttgaaaaat 720

gcccaaaaac cagtagtgat tgcaggacac gaagtaatta gttttggttt agaaaaaacg 780

gtaactcagt ttgtttcaga aacaaaacta ccgattacga cactaaattt tggtaaaagt 840

gctgttgatg aatctttgcc ctcattttta ggaatatata acgggaaact ttcagaaatc 900

agtcttaaaa attttgtgga gtccgcagac tttatcctaa tgcttggagt gaagcttacg 960

gactcctcaa caggtgcatt cacacatcat ttagatgaaa ataaaatgat ttcactaaac 1020

atagatgaag gaataatttt caataaagtg gtagaagatt ttgattttag agcagtggtt 1080

tcttctttat cagaattaaa aggaatagaa tatgaaggac aatatattga taagcaatat 1140

gaagaattta ttccatcaag tgctccctta tcacaagacc gtctatggca ggcagttgaa 1200

agtttgactc aaagcaatga aacaatcgtt gctgaacaag gaacctcatt ttttggagct 1260

tcaacaattt tcttaaaatc aaatagtcgt tttattggac aacctttatg gggttctatt 1320

ggatatactt ttccagcggc tttaggaagc caaattgcgg ataaagagag cagacacctt 1380

ttatttattg gtgatggttc acttcaactt accgtacaag aattaggact atcaatcaga 1440

gaaaaactca atccaatttg ttttatcata aataatgatg gttatacagt tgaaagagaa 1500

atccacggac ctactcaaag ttataacgac attccaatgt ggaattactc gaaattacca 1560

gaaacatttg gagcaacaga agatcgtgta gtatcaaaaa ttgttagaac agagaatgaa 1620

tttgtgtctg tcatgaaaga agcccaagca gatgtcaata gaatgtattg gatagaacta 1680

gttttggaaa aagaagatgc gccaaaatta ctgaaaaaaa tgggtaaatt atttgctgag 1740

caaaataaat agtaatagtg atcccggccg ctactaaagc ctgatttgtc ttgatagctg 1800

ctcctgcctt tgggcagggg cttttttctg tctgccattc ttgaggatgg cggactcttt 1860

cccttttgct ctacgcccat gaatgcgatc gcagtctccc ctgtccagca cgttggagtg 1920

attggtggtg gccagttagc ttggatgctg gcaccagcag cgcaacagtt ggggatgtcg 1980

ctgcacgttc aaacacccaa tgatcacgac ccagcagtag cgatcgcgga tcaaaccgta 2040

ttagcagcag ttgctgacgc ggttctctct tctgccgtta 2080

<210> 98
<211> 1603
<212> DNA
<213> Artificial Sequence

<220>
<223> Synthetic Construct- Sequence No. 98, Example 98: designer
      Synechococcus sp. strain PCC 7942 nirA-promoter-controlled
      NADH-dependent Butanol Dehydrogenase DNA construct (1603 bp)

<400> 98
agaaaatctg gcaccacacc cttcttgcag aacatgcatg atttacaaaa agttgtagtt 60

tctgttacca attgcgaatc gagaactgcc taatctgccg agtatatgat gtcaagattt 120

acactaccaa gagatattta tttcggagaa aacactttag aaactttaaa aactttaaaa 180

ggtaagaaag ctataattgt tgttggagga ggatcaatga aaaaatttgg tttccttcaa 240

aaagttgaag aatatctaaa agaagcagga atggaaataa aattaataga aggtgttgaa 300

ccagatccat cagttgaaac cgttatgaaa ggtgcagaaa tcatgagaga ttttgagcct 360

gattggatag tatccatagg tggaggatca ccaatagatg ctgctaaagc tatgtggata 420

ttctatgaat acccagaatt tacttttgag caagctgttg ttccttttgg aataccagat 480

ttaagacaaa aagctaaatt tgttgctata ccatctacaa gtggaacagc tacagaagtt 540

actgcttttt cagttataac tgattacaaa gctaagataa aatatccttt agctgatttt 600

aatttaacac cagatgtagc tattatagat ccagctcttg ctcaaacaat gcctgcaaaa 660

ttaacagctc atacaggtat ggatgcttta actcatgcaa tagaagctta tgtagcagga 720

ttaagatcat atttctcaga tcctcttgca atgcaagcta tagttatgac aaaagataat 780

ttaataaaat cctatgaagg agataaagaa gcaagagatg aaatgcatat agctcaatgt 840

ttagcaggaa tggcattctc aaatgcgcta cttggaatta ctcatagtat ggcacataag 900

acaggagcag tattccacat tcctcatggt tgtgcaaatg ctatattcct tccttatgta 960

atagatttta ataagaaaac atgtaaagat agatatgcaa ctatagctaa aactttaggt 1020

ttagcaggaa atactgatga tgaattagta gatgcattaa cttctatgat acaagaaatg 1080

aataagaaaa tggatatacc actaaactta aaagaatatg gagtaacaga agaagatttt 1140

aatgaaaact tagatttcat agcacataat gcagtgttag atgcatgtac tggatcaaat 1200

ccaagaccta taactgaaga agaaatgaaa aaagtattca aatgcacatt tactggagag 1260

aaagttaatt tttaataata gtgatcccgg ccgctactaa agcctgattt gtcttgatag 1320

ctgctcctgc ctttgggcag gggctttttt ctgtctgcca ttcttgagga tggcggactc 1380

tttccctttt gctctacgcc catgaatgcg atcgcagtct cccctgtcca gcacgttgga 1440

gtgattggtg gtggccagtt agcttggatg ctggcaccag cagcgcaaca gttggggatg 1500

tcgctgcacg ttcaaacacc caatgatcac gacccagcag tagcgatcgc ggatcaaacc 1560

gtattagcag cagttgctga cgcggttctc tcttctgccg tta 1603

<210> 99
<211> 1654
<212> DNA
<213> Artificial Sequence

<220>
<223> Synthetic Construct- Sequence No. 99, Example 99: designer
      Synechococcus sp. strain PCC 7942 nirA-promoter-controlled
      NADPH-dependent Butanol Dehydrogenase DNA construct (1654 bp)

<400> 99
agaaaatctg gcaccacacc cttcttgcag aacatgcatg atttacaaaa agttgtagtt 60

tctgttacca attgcgaatc gagaactgcc taatctgccg agtatatgct agaaatcaac 120

ttctgtatcg taataacagc acttaagcag tttttccatt tcttctacgc ttggctgtct 180

tgggttagaa cctgtgcagg catcgcctat ggcattaact gcaatgtcat gtaacctctc 240

caggaataca ttttcaggca caaatccctg tgcaaccgga tagctgtctg caccgtaatt 300

tttaatgcag tgcggtatat taagttcatc attcatctta cggagataac cgattaatga 360

agctaccttt tcatcaaggt ctgctccgcc aagtcccatg aaatcagcaa tttcaccata 420

acgcttctta gcctgttcat cctttgcgtt aaatgcaatt accttaggga gatacattgc 480

attcgcagca ccgtgaatga tgtgtgcgcc gtaatcggca aatgccgcac ctgttttatg 540

cgccattgaa tgtacaatac caagaagtgc attagaaaat gccattcctg cgagacattg 600

tgcattatgc attgaatctc ttttttccat atcaccgtta tatgaaccga caaggtctct 660

ttgaatcatt ttaattgcat ggagtgccaa tgggtctgta aaatcacaat ttgcggtgga 720

tacatatgcc tcgatagcat gtgtcattgc atccatacct gtatgtgcca ccaatttttg 780

tggcatggtc tctgccagtt cagggtctac tattgcaaca tcaggtgtta tttcaaaatc 840

ggctattgga tattttattc ctttttcata atctgtaata attgaaaaag cagttacctc 900

ggtagcggtt cctgaagtag aagatattgc acaaaaatgt gcttttttac gaagtgaagg 960

tatgccaaat actttacaca tatcctcaaa ggtaatatca ggatattcat atttaatcca 1020

cattgcttta gccgcatcaa tcggagaacc tccgcctatt gcaacaatcc agtcaggttc 1080

aaactctgac atcgctttgg cacctttcat aacggtttcc accgaagggt caggttcaat 1140

tccttcaaaa agtctgactt ccataccggc ttccttaaga tactgttctg ccctgtcaag 1200

gaaaccaaaa cgtttcattg aacctccgcc aacacaaatc atggcttttt tgccttgaaa 1260

tgtcttaagt gcctctaatg caccctttcc atgatacaaa tctcttggta acgtaaatct 1320

tgccattaat agtgatcccg gccgctacta aagcctgatt tgtcttgata gctgctcctg 1380

cctttgggca ggggcttttt tctgtctgcc attcttgagg atggcggact ctttcccttt 1440

tgctctacgc ccatgaatgc gatcgcagtc tcccctgtcc agcacgttgg agtgattggt 1500

ggtggccagt tagcttggat gctggcacca gcagcgcaac agttggggat gtcgctgcac 1560

gttcaaacac ccaatgatca cgacccagca gtagcgatcg cggatcaaac cgtattagca 1620

gcagttgctg acgcggttct ctcttctgcc gtta 1654

<210> 100
<211> 1440
<212> DNA
<213> Artificial Sequence

<220>
<223> Synthetic Construct- Sequence No. 100, Example 100: designer
      Synechocystis sp. PCC 6803 nirA-promoter-controlled NAD-dependent
      Glyceraldehyde-3-Phosphate Dehydrogenase DNA construct (1440 bp)

<400> 100
atggtagtta aagttggtat taacggtttc ggtcgtatcg gacgtcttgc attccgccgt 60

attcaaaata tcgaaggtgt tgaagtaact cgtatcaacg accttacaga tccaaatatg 120

cttgcacact tgttgaaata cgatacaact caaggtcgtt ttgatggaac agttgaagtt 180

aaagaaggtg gatttgaagt aaacggaaac ttcattaaag tttctgctga acgtgatcca 240

gaaaacatcg actgggcaac tgatggggtt gaaatcgttc ttgaagcaac tggtttcttt 300

gctaaaaaag aagcagctga aaaacactta catgctaacg gtgctaaaaa agttgttatc 360

acagctcctg gtggaaacga tgttaaaaca gttgttttca acactaacca cgacattctt 420

gacggtactg aaacagttat ctcaggtgct tcatgtacta caaactgttt agctcctatg 480

gctaaagctc ttcacgatgc attcggtatt caaaaaggtc ttatgactac aatccacgct 540

tacactggtg accaaatgat ccttgacgga ccacaccgtg gtggtgacct tcgtcgtgca 600

cgcgctggtg ctgcaaatat cgttcctaac tcaactggtg ctgctaaagc tatcggtctt 660

gttatcccag aacttaacgg taaacttgac ggtgctgcac aacgtgttcc tgttccaact 720

ggatcagtaa ctgagttggt tgtaactctt gacaaaaacg tttctgttga cgaaatcaac 780

gctgctatga aagctgcttc aaacgatagc ttcggttaca ctgaagatcc aatcgtttct 840

tcagatatcg taggcgtatc atacggttca ttgtttgacg caactcaaac taaagtaatg 900

gaagttgacg gatcccaatt ggttaaagtt gtatcatggt atgacaacga aatgtcttac 960

actgctcaac ttgtacgtac tcttgagtac ttcgcaaaaa ttgctaaata atagtaatga 1020

gttacagttt tggcaattac taaaaaactg acttcaattc aatgttagcc cgctcccgcg 1080

ggttttttgt tgctttttca cagtgactat aggtaatcag caacacaata cggccctgtt 1140

ctttggacag tttttgtata atgttgaccg catcctgacc ggatttttta tctaagtggg 1200

gaattgtcaa ttgtcaatta aagctaagtt ctactaatgt tttagaaggc attgtcgatt 1260

gaaaataagg gttgaatgga gaaaattttg agcctttgtc aaagataaaa atttatttca 1320

acagtttttt aactagccga accagagaat gacccagtgg cgctgacttt gctcccgagt 1380

ttttgttaga aattaccctc aagaagtaat ctaataataa ggttctctct tctgccgtta 1440

<210> 101
<211> 2182
<212> DNA
<213> Artificial Sequence

<220>
<223> Synthetic Construct- Sequence No. 101, Example 101: designer
      Synechocystis sp. PCC 6803 nirA-promoter-controlled 2-Keto Acid
      Decarboxylase dehydrogenase DNA construct (2182 bp)

<400> 101
agaaaatctg gcaccacacc ctaaatgcgt aaactgcata tgccttggct gagtgtaatt 60

tacgttacaa attttaacga aacgggaacc ctatattgat ctctacatga tgtatacagt 120

aggagattac ctgttagacc gattacacga gttgggaatt gaagaaattt ttggagttcc 180

tggtgactat aacttacaat ttttagatca aattatttca cgcgaagata tgaaatggat 240

tggaaatgct aatgaattaa atgcttctta tatggctgat ggttatgctc gtactaaaaa 300

agctgccgca tttctcacca catttggagt cggcgaattg agtgcgatca atggactggc 360

aggaagttat gccgaaaatt taccagtagt agaaattgtt ggttcaccaa cttcaaaagt 420

acaaaatgac ggaaaatttg tccatcatac actagcagat ggtgatttta aacactttat 480

gaagatgcat gaacctgtta cagcagcgcg gactttactg acagcagaaa atgccacata 540

tgaaattgac cgagtacttt ctcaattact aaaagaaaga aaaccagtct atattaactt 600

accagtcgat gttgctgcag caaaagcaga gaagcctgca ttatctttag aaaaagaaag 660

ctctacaaca aatacaactg aacaagtgat tttgagtaag attgaagaaa gtttgaaaaa 720

tgcccaaaaa ccagtagtga ttgcaggaca cgaagtaatt agttttggtt tagaaaaaac 780

ggtaactcag tttgtttcag aaacaaaact accgattacg acactaaatt ttggtaaaag 840

tgctgttgat gaatctttgc cctcattttt aggaatatat aacgggaaac tttcagaaat 900

cagtcttaaa aattttgtgg agtccgcaga ctttatccta atgcttggag tgaagcttac 960

ggactcctca acaggtgcat tcacacatca tttagatgaa aataaaatga tttcactaaa 1020

catagatgaa ggaataattt tcaataaagt ggtagaagat tttgatttta gagcagtggt 1080

ttcttcttta tcagaattaa aaggaataga atatgaagga caatatattg ataagcaata 1140

tgaagaattt attccatcaa gtgctccctt atcacaagac cgtctatggc aggcagttga 1200

aagtttgact caaagcaatg aaacaatcgt tgctgaacaa ggaacctcat tttttggagc 1260

ttcaacaatt ttcttaaaat caaatagtcg ttttattgga caacctttat ggggttctat 1320

tggatatact tttccagcgg ctttaggaag ccaaattgcg gataaagaga gcagacacct 1380

tttatttatt ggtgatggtt cacttcaact taccgtacaa gaattaggac tatcaatcag 1440

agaaaaactc aatccaattt gttttatcat aaataatgat ggttatacag ttgaaagaga 1500

aatccacgga cctactcaaa gttataacga cattccaatg tggaattact cgaaattacc 1560

agaaacattt ggagcaacag aagatcgtgt agtatcaaaa attgttagaa cagagaatga 1620

atttgtgtct gtcatgaaag aagcccaagc agatgtcaat agaatgtatt ggatagaact 1680

agttttggaa aaagaagatg cgccaaaatt actgaaaaaa atgggtaaat tatttgctga 1740

gcaaaataaa tagtagtaat gagttacagt tttggcaatt actaaaaaac tgacttcaat 1800

tcaatgttag cccgctcccg cgggtttttt gttgcttttt cacagtgact ataggtaatc 1860

agcaacacaa tacggccctg ttctttggac agtttttgta taatgttgac cgcatcctga 1920

ccggattttt tatctaagtg gggaattgtc aattgtcaat taaagctaag ttctactaat 1980

gttttagaag gcattgtcga ttgaaaataa gggttgaatg gagaaaattt tgagcctttg 2040

tcaaagataa aaatttattt caacagtttt ttaactagcc gaaccagaga atgacccagt 2100

ggcgctgact ttgctcccga gtttttgtta gaaattaccc tcaagaagta atctaataat 2160

aaggttctct cttctgccgt ta 2182

<210> 102
<211> 1705
<212> DNA
<213> Artificial Sequence

<220>
<223> Synthetic Construct- Sequence No. 102, Example 102: designer
      Synechocystis sp. PCC 6803 nirA-promoter-controlled
      NADH-dependent Butanol Dehydrogenase DNA construct (1705 bp)

<400> 102
agaaaatctg gcaccacacc ctaaatgcgt aaactgcata tgccttggct gagtgtaatt 60

tacgttacaa attttaacga aacgggaacc ctatattgat ctctacatga tgtcaagatt 120

tacactacca agagatattt atttcggaga aaacacttta gaaactttaa aaactttaaa 180

aggtaagaaa gctataattg ttgttggagg aggatcaatg aaaaaatttg gtttccttca 240

aaaagttgaa gaatatctaa aagaagcagg aatggaaata aaattaatag aaggtgttga 300

accagatcca tcagttgaaa ccgttatgaa aggtgcagaa atcatgagag attttgagcc 360

tgattggata gtatccatag gtggaggatc accaatagat gctgctaaag ctatgtggat 420

attctatgaa tacccagaat ttacttttga gcaagctgtt gttccttttg gaataccaga 480

tttaagacaa aaagctaaat ttgttgctat accatctaca agtggaacag ctacagaagt 540

tactgctttt tcagttataa ctgattacaa agctaagata aaatatcctt tagctgattt 600

taatttaaca ccagatgtag ctattataga tccagctctt gctcaaacaa tgcctgcaaa 660

attaacagct catacaggta tggatgcttt aactcatgca atagaagctt atgtagcagg 720

attaagatca tatttctcag atcctcttgc aatgcaagct atagttatga caaaagataa 780

tttaataaaa tcctatgaag gagataaaga agcaagagat gaaatgcata tagctcaatg 840

tttagcagga atggcattct caaatgcgct acttggaatt actcatagta tggcacataa 900

gacaggagca gtattccaca ttcctcatgg ttgtgcaaat gctatattcc ttccttatgt 960

aatagatttt aataagaaaa catgtaaaga tagatatgca actatagcta aaactttagg 1020

tttagcagga aatactgatg atgaattagt agatgcatta acttctatga tacaagaaat 1080

gaataagaaa atggatatac cactaaactt aaaagaatat ggagtaacag aagaagattt 1140

taatgaaaac ttagatttca tagcacataa tgcagtgtta gatgcatgta ctggatcaaa 1200

tccaagacct ataactgaag aagaaatgaa aaaagtattc aaatgcacat ttactggaga 1260

gaaagttaat ttttaatagt aatgagttac agttttggca attactaaaa aactgacttc 1320

aattcaatgt tagcccgctc ccgcgggttt tttgttgctt tttcacagtg actataggta 1380

atcagcaaca caatacggcc ctgttctttg gacagttttt gtataatgtt gaccgcatcc 1440

tgaccggatt ttttatctaa gtggggaatt gtcaattgtc aattaaagct aagttctact 1500

aatgttttag aaggcattgt cgattgaaaa taagggttga atggagaaaa ttttgagcct 1560

ttgtcaaaga taaaaattta tttcaacagt tttttaacta gccgaaccag agaatgaccc 1620

agtggcgctg actttgctcc cgagtttttg ttagaaatta ccctcaagaa gtaatctaat 1680

aataaggttc tctcttctgc cgtta 1705

<210> 103
<211> 1756
<212> DNA
<213> Artificial Sequence

<220>
<223> Synthetic Construct- Sequence No. 103, Example 103: designer
Synechocystis sp. PCC 6803 nirA-promoter-controlled
NADPH-dependent Butanol Dehydrogenase DNA construct (1756 bp)

<400> 103
agaaaatctg gcaccacacc ctaaatgcgt aaactgcata tgccttggct gagtgtaatt 60

tacgttacaa attttaacga aacgggaacc ctatattgat ctctacatgc tagaaatcaa 120

cttctgtatc gtaataacag cacttaagca gtttttccat ttcttctacg cttggctgtc 180

ttgggttaga acctgtgcag gcatcgccta tggcattaac tgcaatgtca tgtaacctct 240

ccaggaatac attttcaggc acaaatccct gtgcaaccgg atagctgtct gcaccgtaat 300

ttttaatgca gtgcggtata ttaagttcat cattcatctt acggagataa ccgattaatg 360

aagctacctt ttcatcaagg tctgctccgc caagtcccat gaaatcagca atttcaccat 420

aacgcttctt agcctgttca tcctttgcgt taaatgcaat taccttaggg agatacattg 480

cattcgcagc accgtgaatg atgtgtgcgc cgtaatcggc aaatgccgca cctgttttat 540

gcgccattga atgtacaata ccaagaagtg cattagaaaa tgccattcct gcgagacatt 600

gtgcattatg cattgaatct cttttttcca tatcaccgtt atatgaaccg acaaggtctc 660

tttgaatcat tttaattgca tggagtgcca atgggtctgt aaaatcacaa tttgcggtgg 720

atacatatgc ctcgatagca tgtgtcattg catccatacc tgtatgtgcc accaattttt 780

gtggcatggt ctctgccagt tcagggtcta ctattgcaac atcaggtgtt atttcaaaat 840

cggctattgg atattttatt cctttttcat aatctgtaat aattgaaaaa gcagttacct 900

cggtagcggt tcctgaagta gaagatattg cacaaaaatg tgctttttta cgaagtgaag 960

gtatgccaaa tactttacac atatcctcaa aggtaatatc aggatattca tatttaatcc 1020

acattgcttt agccgcatca atcggagaac ctccgcctat tgcaacaatc cagtcaggtt 1080

caaactctga catcgctttg gcacctttca taacggtttc caccgaaggg tcaggttcaa 1140

ttccttcaaa aagtctgact tccataccgg cttccttaag atactgttct gccctgtcaa 1200

ggaaaccaaa acgtttcatt gaacctccgc caacacaaat catggctttt ttgccttgaa 1260

atgtcttaag tgcctctaat gcaccctttc catgatacaa atctcttggt aacgtaaatc 1320

ttgccattag taatgagtta cagttttggc aattactaaa aaactgactt caattcaatg 1380

ttagcccgct cccgcgggtt ttttgttgct ttttcacagt gactataggt aatcagcaac 1440

acaatacggc cctgttcttt ggacagtttt tgtataatgt tgaccgcatc ctgaccggat 1500

tttttatcta agtggggaat tgtcaattgt caattaaagc taagttctac taatgtttta 1560

gaaggcattg tcgattgaaa ataagggttg aatggagaaa attttgagcc tttgtcaaag 1620

ataaaaattt atttcaacag ttttttaact agccgaacca gagaatgacc cagtggcgct 1680

gactttgctc ccgagttttt gttagaaatt accctcaaga agtaatctaa taataaggtt 1740

ctctcttctg ccgtta 1756

<210> 104
<211> 1655
<212> DNA
<213> Artificial Sequence

<220>
<223> Synthetic Construct- Sequence No. 104, Example 104: designer
Anabaena PCC 7120 hox-promoter-controlled NAD-dependent

Glyceraldehyde-3-Phosphate Dehydrogenase DNA construct (1655 bp)

<400> 104
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggtagt taaagttggt attaacggtt tcggtcgtat cggacgtctt 240

gcattccgcc gtattcaaaa tatcgaaggt gttgaagtaa ctcgtatcaa cgaccttaca 300

gatccaaata tgcttgcaca cttgttgaaa tacgatacaa ctcaaggtcg ttttgatgga 360

acagttgaag ttaaagaagg tggatttgaa gtaaacggaa acttcattaa agtttctgct 420

gaacgtgatc cagaaaacat cgactgggca actgatgggg ttgaaatcgt tcttgaagca 480

actggtttct ttgctaaaaa agaagcagct gaaaaacact tacatgctaa cggtgctaaa 540

aaagttgtta tcacagctcc tggtggaaac gatgttaaaa cagttgtttt caacactaac 600

cacgacattc ttgacggtac tgaaacagtt atctcaggtg cttcatgtac tacaaactgt 660

ttagctccta tggctaaagc tcttcacgat gcattcggta ttcaaaaagg tcttatgact 720

acaatccacg cttacactgg tgaccaaatg atccttgacg gaccacaccg tggtggtgac 780

cttcgtcgtg cacgcgctgg tgctgcaaat atcgttccta actcaactgg tgctgctaaa 840

gctatcggtc ttgttatccc agaacttaac ggtaaacttg acggtgctgc acaacgtgtt 900

cctgttccaa ctggatcagt aactgagttg gttgtaactc ttgacaaaaa cgtttctgtt 960

gacgaaatca acgctgctat gaaagctgct tcaaacgata gcttcggtta cactgaagat 1020

ccaatcgttt cttcagatat cgtaggcgta tcatacggtt cattgtttga cgcaactcaa 1080

actaaagtaa tggaagttga cggatcccaa ttggttaaag ttgtatcatg gtatgacaac 1140

gaaatgtctt acactgctca acttgtacgt actcttgagt acttcgcaaa aattgctaaa 1200

taatgaagta agtaggaagc agggagcagg ggaaagaaaa ttgacaactg tacaagatta 1260

atcgcgtctc tgagcaatga ccaaatacat ctacctccac ggttttcttc cagcccccta 1320

tctgcgaaag cacaagatat tagcaagcgt ttcgcccaaa ttcacataca gctaacaatc 1380

cctgatctca atgctggtga attttctcag ttaacaatca cgcgccaaat tcaacaagtt 1440

gccgcaattt tccctgataa ttctgaacca ataacgctga taggttctag tttaggcggt 1500

ttaactgctg cttatctagg acagcgatat ttacaagtac aacgcttagt tttattagcg 1560

ccagtttggt tttttatccc attggttgcc caaaatgggt gaagaagctg tcacaagttg 1620

gcaacaaacg atataggttc tctcttctgc cgtta 1655

<210> 105
<211> 2303
<212> DNA

<213>  Artificial Sequence

<220>
<223>  Synthetic Construct- Sequence No. 105, Example 105: designer
      Anabaena PCC 7120 hox-promoter-controlled Acetolactate Synthase
      DNA construct (2303 bp)

<400>  105
agaaaatctg gcaccacacc gcagaaatat aggggctagg agttgagggt actctggttc      60

gtcaagcatt tgggatgatt tcccctcaca agttcctcaa attattctcc tataaacaat     120

agatataagg tcaaaacttg agttatgagt gctgagtaaa aaattactct ccacgcctca     180

gaggtagata tgctaagggg tgcagctgag ctccccaaga cgttggttaa agcgcagatt     240

ttcactatag tccacaggga catcaatgag agtgggaaca ttctgtgcca aggcggtttt     300

gagggttggg ataaagtcag tggtctcctc aatgcgatag cctttgagtc ccatgctttc     360

ggctaacttg acaaaatcgg gattactaaa gtggacatag gcagattcac caaagtagcg     420

ctgctgcttc cactcaatga gaccatagcc gccgtcatta aagatgatgg tggtaaagtt     480

tgtccccata cgcagggcag tttccagttc ttggaaattc atcatgaagc caccatcccc     540

cgtgactgcc accacatgcc gctgcggata gactaatttg gcggccattg cccccggtac     600

cgcaatcccc attgccgcaa agccattgga aatcaagcag gtgttggggc gatcgcagtg     660

gtagtgacga gcaatccaca ttttatgggc acccacatca gaaataacaa tgtcctcggg     720

ccccatgact tggcgcaggt catagattag cttttggggc ttaaccggaa aactctcatc     780

ctgggcatat tggtaatagt ccgccacaat ctcctgacgc agttgcacgg catagggggt     840

gggcttatct tggcgatcgg cccgcttaag aatttcatag agggagtcag aaatatcgcc     900

gacaacctca acgacaggga tatagctgct gtcaatttcc gcaggagtgg ccgcaatatg     960

gataatcggc aagcggccct cggggttcca gctttttggg gaatactcaa ttaagtcata    1020

gccaacggca atcactaagt cagcatgatc aaagccgcag ctaatgtaat cccgctgttg    1080

gagccccacg gtccacagag caagggggtg ttgataggga atgacccctt tgcccatgaa    1140

ggtattggcc acggggatat tcagcttttc ggcaaaatgg gtgagggcag cggcagcatg    1200

ggcgcgaatg gcgccattcc ccactaggat cagggggttc tcggcagcat tgatgagttc    1260

agcggcctta agaatactct ggaaagaggc ataggttttt tcgggggagc tgggcttaag    1320

gggagcgccc tcggcttcca tggcggcaat gttttcaggc acatcaatgt gaacggcgcc    1380

cggcttctca ttctgggcaa ttttaaaggc cttgcggaca atttctgggg taatactagg    1440

gcggacaatc tgggcattcc atttggttac ggggctaaac atggccacca agtccaaata    1500

ttggtgggac tcgatgtgca tgcgatccgt ccccacttgc cctgtaatcg ccaccagggg    1560

agcgccgtcg aggttggcat cggcaacacc ggtcattaaa ttcgtggccc cggggccgag    1620

ggtagaaaga cagacccctg ctttgccggt gaggcgacca tagacatcgg ccataaaggc    1680

cgccccctgt tcgtggcggg tggttataaa ttgaatccga gagcgatgga gggcatggag 1740

gacatctaga ttctcttccc ccggcaaacc aaaaatatat tcaacgcctt cattttcaag 1800

gcattttacg agtaattcgg cggtattcat aggatgggcg atcgcaggca ctgaagtaag 1860

taggaagcag ggagcagggg aaagaaaatt gacaactgta caagattaat cgcgtctctg 1920

agcaatgacc aaatacatct acctccacgg ttttcttcca gccccctatc tgcgaaagca 1980

caagatatta gcaagcgttt cgcccaaatt cacatacagc taacaatccc tgatctcaat 2040

gctggtgaat tttctcagtt aacaatcacg cgccaaattc aacaagttgc cgcaattttc 2100

cctgataatt ctgaaccaat aacgctgata ggttctagtt taggcggttt aactgctgct 2160

tatctaggac agcgatattt acaagtacaa cgcttagttt tattagcgcc agtttggttt 2220

tttatcccat tggttgccca aaatgggtga agaagctgtc acaagttggc aacaaacgat 2280

ataggttctc tcttctgccg tta 2303

<210> 106
<211> 1661
<212> DNA
<213> Artificial Sequence

<220>
<223> Synthetic Construct- Sequence No. 106, Example 106: designer
      Anabaena PCC 7120 hox-promoter-controlled Ketol-Acid
      Reductoisomerase DNA construct (1661 bp)

<400> 106
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttaatttt tatccttatc cactatcttt ccttttccaa gccagctcat 240

catacttcta agtttagatc ccacctgctc gatgggatgg ttttcatcag ccctggtgag 300

ggcattaaat actggtctgc cagctttatt ttccagcatc cattcttttg caaattctcc 360

tgattgtatc tgtcttaaaa cttccctcat gttgtcttta acctggggat ctattaccac 420

aggccccctt gtgaggtcac catattgggc tgtgttgctt atggaatatc tcatgttgga 480

gataccacct tcatagagaa gatccactat cagttttacc tcatgcatac attcaaaata 540

agccatttct ggagcgtagc cagcctccac caatgtttca aaaccatatt taataagctg 600

tgtcaaacca ccacataaaa ccgcctgctc accgaaaaga tctgtttctg tttcctcttt 660

gaagtttgtt tcaagcacac ctgctctggc tccaccaatg gcagctgcgt atgacaaagc 720

cacctctctt gaatcaccgg aatagtcttg atgcacagcg atgaggcatg gtactccgcc 780

accctttgta tattctgctc ttacaagatg ccctggccct ttaggtgcaa tcattataac 840

gtttacgttt ttaggaggca ctatctgtcc aaaatggatg ttgaaaccgt gggcaaatgc 900

aagatatgtc ccctctttta tataaggggc aatctgctct ctgtaaagat ccccctgtat 960

ctcatcagga acgagtatca tgatgaggtc tgcccatttt gtggcctctg atatctccat 1020

aactttaagc ccggagcttt ccgctttttt ccaggaatca ccaccctttc ttagggcaac 1080

ggcaacgtca acaccactat ctttgaggtt attagaatgt ccataaccct gacttccgta 1140

accaactatg gccacctttt tctttttaat caattctaaa tttgcatcct tttcgtagta 1200

tactttcatt gaagtaagta ggaagcaggg agcaggggaa agaaaattga caactgtaca 1260

agattaatcg cgtctctgag caatgaccaa atacatctac ctccacggtt ttcttccagc 1320

cccctatctg cgaaagcaca agatattagc aagcgtttcg cccaaattca catacagcta 1380

acaatccctg atctcaatgc tggtgaattt tctcagttaa caatcacgcg ccaaattcaa 1440

caagttgccg caattttccc tgataattct gaaccaataa cgctgatagg ttctagttta 1500

ggcggtttaa ctgctgctta tctaggacag cgatatttac aagtacaacg cttagtttta 1560

ttagcgccag tttggttttt tatcccattg gttgcccaaa atgggtgaag aagctgtcac 1620

aagttggcaa caaacgatat aggttctctc ttctgccgtt a 1661

<210> 107
<211> 2324
<212> DNA
<213> Artificial Sequence

<220>
<223> Synthetic Construct- Sequence No. 107, Example 107: designer
      Anabaena PCC 7120 hox-promoter-controlled Dihydroxy-Acid
      Dehydratase DNA construct (2324 bp)

<400> 107
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttataaat ccgttataca tccaaaagat gctgatgata ccagcttgct 240

atacttcctt aagcttccac tcacatttcg ctcgggcttt ataggagttt gctcctttcg 300

tttttcccat tcctcttcct ccatcaatac actaattgaa ttttccactg catctattct 360

gatgcggtcg ccattttcta cataagctaa tgcgccatta tcaaaagctt cgggtgaaat 420

gtgtcccact acaaaaccgt gagttcctcc tgagaacctt ccatctgtaa tgagggctac 480

tttctttccc aaacctgctc ccattatagc agcagttggc tttaacattt cgggcattcc 540

aggacctcct tttggccctt catatcgaat caccactaca tcaccttctt ttatctcgcc 600

tctggcaata ccatcattgg ccagaaattc accatcataa actctagcag ttccttcgaa 660

tatctctcct tcctttccgg tgattttagc aacagctcct ccaggtgcta aattgccttt 720

caatattcgc aaatgacctg agcttttgat tggatttttc acagagtgaa tgatgtcttg 780

cttttcgcct aaaccctgaa catctttata attttcagct aaagttttac ctgttatggt 840

catgcaatcg ccatgaagaa gtccttcccc caacatcatt ttcatcactg caggaatccc 900

gccattttga tgaagatctt ccatcaagta tttaccagaa ggctttaagt cggctagata 960

aggagtttcg gcactgattc gagcgaaatc ttccaatgtt aaatcgattt caaaagcatt 1020

ggcaacagct aataaatgta aaactgcatt ggtcgatcct cccaaaactg taatcaaccg 1080

tatggcgttt tcaaatgatt ttttagtgac gatatcccgt ggcttcaaat cattttcaat 1140

caatttttta atggctaaac cagcctctgc acattctttc cttttctcag cactttgagc 1200

gggattggaa gcaccataag gcaagcataa tcctaaggct tcaatagcgg atgccatcgt 1260

attggcagtg tacattcctc cacaggctcc aggacctggg catgcatttg ccactactcc 1320

cttaaaatct tcttcagaaa tagtgttggc ttgcttctta cctaaagctt caaaagcaga 1380

aaccacatct aatttttctc cattatggca acctggagca atggttcctc cataaattat 1440

gagtgaaggt cgattcaatc tgcccatagc cagtaaggcg cctggcatat ttttatcgca 1500

tcccgtaata gcaatcatgg catcgtaagc ttgcgcattc actaccgttt ccatggaatc 1560

ggctatgata tcgcgggaag gcaatgaata gcgcattccg ttggtaccca tagaaatacc 1620

atcgcttact ccaatagtat tgaaaatcaa ccctacccac tcataagatt tgatgctttt 1680

cttgacctct acagccaaat cattcaaatg catattacat ggattgccct caaaaccagt 1740

gcttgcaata cctatttgtg gtttttccaa atcttcatca gacaaaccaa tggcatgcaa 1800

catggcttga gcggcaggtt gtgtaggatc ttgtgtaact gctttactat atggatttaa 1860

ttccttcgcc attgaagtaa gtaggaagca gggagcaggg gaaagaaaat tgacaactgt 1920

acaagattaa tcgcgtctct gagcaatgac caaatacatc tacctccacg gttttcttcc 1980

agccccctat ctgcgaaagc acaagatatt agcaagcgtt tcgcccaaat tcacatacag 2040

ctaacaatcc ctgatctcaa tgctggtgaa ttttctcagt taacaatcac gcgccaaatt 2100

caacaagttg ccgcaatttt ccctgataat tctgaaccaa taacgctgat aggttctagt 2160

ttaggcggtt taactgctgc ttatctagga cagcgatatt tacaagtaca acgcttagtt 2220

ttattagcgc cagtttggtt ttttatccca ttggttgccc aaaatgggtg aagaagctgt 2280

cacaagttgg caacaaacga tataggttct ctcttctgcc gtta 2324

<210> 108
<211> 2288
<212> DNA
<213> Artificial Sequence

<220>
<223> Synthetic Construct- Sequence No. 108, Example 108: designer

Anabaena PCC 7120 hox-promoter-controlled branched-chain
alpha-Ketoacid Decarboxylase DNA construct (2288 bp)

<400> 108
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgtatac agtaggagat tacctgttag accgattaca cgagttggga 240

attgaagaaa tttttggagt tcctggtgac tataacttac aatttttaga tcaaattatt 300

tcacgcgaag atatgaaatg gattggaaat gctaatgaat taaatgcttc ttatatggct 360

gatggttatg ctcgtactaa aaaagctgcc gcatttctca ccacatttgg agtcggcgaa 420

ttgagtgcga tcaatggact ggcaggaagt tatgccgaaa atttaccagt agtagaaatt 480

gttggttcac caacttcaaa agtacaaaat gacggaaaat ttgtccatca tacactagca 540

gatggtgatt ttaaacactt tatgaagatg catgaacctg ttacagcagc gcggacttta 600

ctgacagcag aaaatgccac atatgaaatt gaccgagtac tttctcaatt actaaaagaa 660

agaaaaccag tctatattaa cttaccagtc gatgttgctg cagcaaaagc agagaagcct 720

gcattatctt tagaaaaaga aagctctaca acaaatacaa ctgaacaagt gattttgagt 780

aagattgaag aaagtttgaa aaatgcccaa aaaccagtag tgattgcagg acacgaagta 840

attagttttg gtttagaaaa aacggtaact cagtttgttt cagaaacaaa actaccgatt 900

acgacactaa attttggtaa aagtgctgtt gatgaatctt tgccctcatt tttaggaata 960

tataacggga aactttcaga aatcagtctt aaaaattttg tggagtccgc agactttatc 1020

ctaatgcttg gagtgaagct tacggactcc tcaacaggtg cattcacaca tcatttagat 1080

gaaaataaaa tgatttcact aaacatagat gaaggaataa ttttcaataa agtggtagaa 1140

gattttgatt ttagagcagt ggtttcttct ttatcagaat taaaaggaat agaatatgaa 1200

ggacaatata ttgataagca atatgaagaa tttattccat caagtgctcc cttatcacaa 1260

gaccgtctat ggcaggcagt tgaaagtttg actcaaagca atgaaacaat cgttgctgaa 1320

caaggaacct cattttttgg agcttcaaca attttcttaa aatcaaatag tcgttttatt 1380

ggacaacctt tatggggttc tattggatat acttttccag cggctttagg aagccaaatt 1440

gcggataaag agagcagaca ccttttattt attggtgatg gttcacttca acttaccgta 1500

caagaattag gactatcaat cagagaaaaa ctcaatccaa tttgttttat cataaataat 1560

gatggttata cagttgaaag agaaatccac ggacctactc aaagttataa cgacattcca 1620

atgtggaatt actcgaaatt accagaaaca tttggagcaa cagaagatcg tgtagtatca 1680

aaaattgtta gaacagagaa tgaatttgtg tctgtcatga aagaagccca agcagatgtc 1740

aatagaatgt attggataga actagttttg gaaaaagaag atgcgccaaa attactgaaa 1800

aaaatgggta aattatttgc tgagcaaaat aaatagtgaa gtaagtagga agcagggagc 1860

aggggaaaga aaattgacaa ctgtacaaga ttaatcgcgt ctctgagcaa tgaccaaata 1920

catctacctc cacggttttc ttccagcccc ctatctgcga aagcacaaga tattagcaag 1980

cgtttcgccc aaattcacat acagctaaca atccctgatc tcaatgctgg tgaattttct 2040

cagttaacaa tcacgcgcca aattcaacaa gttgccgcaa ttttccctga taattctgaa 2100

ccaataacgc tgataggttc tagtttaggc ggtttaactg ctgcttatct aggacagcga 2160

tatttacaag tacaacgctt agttttatta gcgccagttt ggttttttat cccattggtt 2220

gcccaaaatg ggtgaagaag ctgtcacaag ttggcaacaa acgatatagg ttctctcttc 2280

tgccgtta 2288

<210> 109
<211> 1613
<212> DNA
<213> Artificial Sequence

<220>
<223> Synthetic Construct- Sequence No. 109, Example 109: designer
 Anabaena PCC 7120 hox-promoter-controlled 2-Methylbutyraldehyde
 Reductase DNA construct (1613 bp)

<400> 109
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggcttc tgtaaatgac tactttgaga acgccaagac gacgtacttt 240

actttgagat cgggtgacaa gatccccgct gttggattgg gtacttggca atcacccacc 300

aacgagacta aagaggcagt caagtacgct ttgcagcacg gttaccgtca catcgatgct 360

gccgccattt atggtaacga agacgaggtt ggtgacggta tcaaggagag tggaatccct 420

cgtgaccaaa tctgggtcac atctaagctc tggtgcaatg ctcatgctcc cgaggctgtc 480

cccaaggctt tggagaagac cttgcgtgag ctgaaacttg attaccttga cctttacctc 540

atccactggc ctatttcttt gaagaccggc gatgacttgg ttcccaagga caaggacggc 600

aacaccatca ctgtcgaaat tcccctcgag gacacctgga aggctatgga gggtcttgtg 660

aagtccggca aggtgaagaa cattggtatt tccaatttca acaacgaaga gttggatcgt 720

attttgaagg ttgccgagat tcctcctgcc gtccaccaaa tggaaactca tccttacttg 780

aagcagacgg agttcattga gaagcacaag aagcttggca ttcacgtcac cgcttactcg 840

cctttggcca accaaaatgc tctttacggc aatgccgttc ccaagttgat tgagcacaag 900

actcttgtcg acattgccaa gaccaagggt gagggcgtca ctggtgccaa cattgctatt 960

tcttgggcag tcaagcgcgg tacttcggtt attcctaagt ctgttcatgc caacagaatt 1020

aagagcaact tcctcgttgt tcccttgact gatgacgaga tgaaggccat cgataacatt 1080

ggtgtcagca agcgtttcaa ttggagcaaa gttttctgca atgagaattg tttctacggt 1140

cttgaggatg gtcctcagta atgaagtaag taggaagcag ggagcagggg aaagaaaatt 1200

gacaactgta caagattaat cgcgtctctg agcaatgacc aaatacatct acctccacgg 1260

ttttcttcca gccccctatc tgcgaaagca caagatatta gcaagcgttt cgcccaaatt 1320

cacatacagc taacaatccc tgatctcaat gctggtgaat tttctcagtt aacaatcacg 1380

cgccaaattc aacaagttgc cgcaattttc cctgataatt ctgaaccaat aacgctgata 1440

ggttctagtt taggcggttt aactgctgct tatctaggac agcgatattt acaagtacaa 1500

cgcttagttt tattagcgcc agtttggttt tttatcccat tggttgccca aaatgggtga 1560

agaagctgtc acaagttggc aacaaacgat ataggttctc tcttctgccg tta 1613

<210> 110
<211> 1300
<212> DNA
<213> Artificial Sequence

<220>
<223> Synthetic Construct- Sequence No. 110, Example 110: designer
      Prochlorococcus marinus MIT9313 groE-promoter-controlled
      NAD-dependent Glyceraldehyde-3-Phosphate Dehydrogenase DNA
      construct (1300 bp)

<400> 110
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgtta tgccaagccg actttacgaa 180

ccaattccgc ggtgcgcgtg gcgtaaccca tttcgttgtc ataccaagtg tagattttca 240

ccattcgctt accaaccacc atggtggaga gcgcatccac aattgttgaa cgttgatcgc 300

cttggtaatc aatcgacacc agtggacgct cttcaaaacc cagaatgcct ttaagctcgt 360

tttctgaggc ttgttttaac agctgattga tctcttccac cgtcgtatca cgctgcacat 420

caaaaatgat gtcggttaac gaggcattcg ccaacggtac acgtacggcg tgtccatcaa 480

tcttgccttt cagatccggg aaaatttcga taatcgcttt agcagagccg gtggttgtgg 540

ggatgaggct cataccgcaa gcacgtgcac ggcgtaaatc tttatgcggt gcatccaaaa 600

tggtttgcgt attggttagg ttatggatgg tagtaaaaga ggcttgtgcg atacccagtt 660

tttcatggat tactttcacc actggagcaa tacagttagt ggtacaagaa gcggcagtga 720

caatgcgatg ttgctccgga ttgaagatgt gatcgttcac cccgaccacg atattggcga 780

tcccctcttc tttgacaggc gctgaaacga cgacgcgttt tactccttgt gccaaatact 840

ggttcaagaa ttcgccttta cggtgcttac ccgtagcctc aatcaccaca tcacagcccg 900

accaatccac tgcatcaatc gatttttctt gtgttgtgcg aatgcgtttg ccgttgatca 960

ggatagcatc cgcttcactg cccacagcat gatgccaacg accttgtacc gaatcgaact 1020

ccaaaaggtg cgctaatgtt gcagcatcac ccgccacatc gttgatctgt acaaactcaa 1080

tctcaggcca atcaaacgaa gctcttaaag ccaaacgccc aatacgacca aatccattaa 1140

ttccgacttt aattgccatt gatttagttt cggtgtctat ctcttaatag cctcgattta 1200

ttttcggggc tattaatcaa ctctcagagg cgacaagctt cttcttccct tacgacgttt 1260

ttattggttg gacatggcaa ggttctctct tctgccgtta 1300

<210> 111
<211> 1498
<212> DNA
<213> Artificial Sequence

<220>
<223> Synthetic Construct- Sequence No. 111, Example 111: designer
 Prochlorococcus marinus MIT9313 groE-promoter-controlled
 Phosphoglycerate Mutase DNA construct (1498 bp)

<400> 111
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgaaa tatgtaatct tacttggcga 180

cggcatggcc gacgaaaaaa ttgccgaact ggacggcaaa acgcctcttc aatacgccag 240

cacacccaat atggacaggc tggcggcagg cggggaaacc ggcatggtac ataccgtccc 300

ggatggattt ccgcctggca gcgatgtagc caacctctcg gtaatggggt acaatccgcg 360

ggaatattat accgggcgtt ctccgctgga agcggtaagc atgggggttg aactttcaga 420

tgacgatgta gccttccgct gcaacctggt taccctgtca gaagaagagg tatatgaaaa 480

caagattatg gtggattaca gctcggacga aattaccacc tctgaatcgc atgaactgat 540

cagggaagtc gccaaccgcc tgggcagtaa agagttgcgc ttttacccgg gtttcggatt 600

tagacacctt cttgtatgga aaacagggcc tgttggcggg aagctaacac caccccacga 660

tatctcgggg cgtaccattg ccccatacct ccccaaaggc gagggtagcg acactttaaa 720

gcggttaatg aaagaaagca acaggttcct gccagagcac ccggttaacc aaaaaagggt 780

aagggccggt cttaggcctg ccacttccat ctggttctgg ggacagggaa agaaaccctc 840

gataccgaag ttctacgaca aatacggggt aaccggctct gttatctctg ccgtggacct 900

gattaagggg attggcatct gtgccggctt cgatatagtt aaggtggagg gtgtaacggg 960

caccatccat accaacttcc gggggaaagt gcaggccgcc ctggaagaac tgaaaaaggg 1020

aaaggacctg gtctacattc acgttgaggc tccggacgca gcaagccaca ggggtgaaac 1080

tgttaccaaa gttaaagcta ttgaaatggt ggacaacatg ctgggccagc ttttaaacaa 1140

actggacgaa ttcggcatgt acaaaataat gctcttgccc gatcatccaa ccccgctcag 1200

cactaaaacc cactctaaca gccctgtccc ctttgttatc tatgccaagg ggcggaaaaa 1260

taaaagcgcc gcttcttttg acgaagaaac ggcggcaaaa agcggacttg ttttccgggc 1320

gggccatgag ttaatggatt actttatccg cagctaatga tttagtttcg gtgtctatct 1380

cttaatagcc tcgatttatt ttcggggcta ttaatcaact ctcagaggcg acaagcttct 1440

tcttccctta cgacgttttt attggttgga catggcaagg ttctctcttc tgccgtta 1498

<210> 112
<211> 1588
<212> DNA
<213> Artificial Sequence

<220>
<223> Synthetic Construct- Sequence No. 112, Example 112: designer
      Prochlorococcus marinus MIT9313 groE-promoter-controlled Enolase
      DNA construct (1588 bp)

<400> 112
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatggtg tacgtggaaa tcgtggatgt 180

aagagcaaga gaggtcctgg attcgagagg aaatcccacc gttgaagcgg aagtcgtgct 240

tgaagacgga acaatgggaa gagccatcgt gccctctggt gcctccactg gaaaattcga 300

agccctggaa atcagagaca aagacaagaa gagatacctc gggaagggtg ttctgaaggc 360

cgtagagaac gtgaacgaaa ccatagctcc cgcgctgatt ggaatgaacg cattcgacca 420

gccactcgtt gacaagacac tgatagaact ggatggcaca gagaacaaat ctaaactggg 480

tgccaacgct atactcgccg tttctatggc agttgccaga gcggcggcga attacctcgg 540

attgcccctc tacaaatacc ttggaggagt caacgcaaag gttctgccag tacctttgat 600

gaacgtgatc aacggtggac agcacgcaga caacaatctt gaccttcagg aattcatgat 660

cgttcccgcc ggatttgaca gcttcagaga agctttgagg gcaggagcgg aaatattcca 720

cacgttgaaa aagatactcc acgaagccgg tcacgtgaca gcagtaggag acgagggtgg 780

attcgcaccc aatctgtctt ccaacgaaga agccataaag gttctgattg aagccataga 840

gaaagctggc tacaagcccg gagaagaagt cttcatagct cttgattgcg cagcatcttc 900

cttctacgat gaggaaaagg gagtttacta cgtcgatggt gaagaaaaat ccagcgaagt 960

tctcatggga tactacgaag aactggtggc gaagtacccc atcatatcca tcgaagatcc 1020

gttcgcggag gaagactggg atgcatttgt ggaattcaca aagagagtag gaaacaaggt 1080

tcagatcgtt ggagatgacc tttacgtgac caacgtgaaa agactttcca aaggaataga 1140

actcaaagcg accaactcca tactcatcaa actcaatcag ataggcaccg tcacggaaac 1200

tctcgacgcg gtggagatgg cacagaagaa caacatgaca gccatcattt cccacagatc 1260

tggagagagt gaagacacgt tcattgcgga tctcgctgtg gcaacgaacg ctggtttcat 1320

caagacaggt tccctctcca gaagcgaaag gatagccaag tacaaccagc ttttgagaat 1380

cgaggaagaa ctcggaaaag tggcagaatt cagaggtttg aaatctttct actctataaa 1440

gagataatga tttagtttcg gtgtctatct cttaatagcc tcgatttatt ttcggggcta 1500

ttaatcaact ctcagaggcg acaagcttct tcttccctta cgacgttttt attggttgga 1560

catggcaagg ttctctcttc tgccgtta 1588

<210> 113
<211> 1717
<212> DNA
<213> Artificial Sequence

<220>
<223> Synthetic Construct- Sequence No. 113, Example 113: designer
      Prochlorococcus marinus MIT9313 groE-promoter-controlled Pyruvate
      Kinase DNA construct (1717 bp)

<400> 113
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgatg agaaaaacca agcttatatg 180

ttcgatagga cctaaaaccg agaaaccaga gaaaataaag gaattgctaa aaagaggggt 240

aaatgctttt aggataagcg cagttcatta cacgatggaa aaaatcacag agctggtaga 300

gctaatcaaa gatattagat atgaactcaa aatgcctgtt tccatcattc ttgatttacc 360

aggttgcaaa ctccggacgg gagatcagaa agaagaaatc atcgaactta gacaaggtga 420

aaaagtgact gtaacaagcg aaaaaacttt ttcttctcgg gatactataa gcataaattt 480

ttccggacca tttcagggtg taaaaaccgg cgatttaatc ctcgtagatg atggaaaaat 540

acaactcaga gtggaaagaa tttctccaaa aaaagtagaa tgcgtcgtgg agataggagg 600

aattttgaag aaaaacagtg gcgtcaattt tcccaattct gatctacctg tcgaagtacc 660

aacagaagaa gatattaaga tcatagctga aactgtcaat atgggactgg actattactg 720

tgtatcattt gcaagaaacg caaaagatgt tcaaaaaatc aaaaaacatc ttgaatcttt 780

cgattcaagt gcgaaaattc ttacgaaaat agaaacaaaa aaatccatag aaacgctgga 840

agatatatgt cgcgtgagcg atggaataat cgtagcaaga ggagatttag cggtggagac 900

atctctgata gatttgccga tattgcaaaa aaagataata aataccgcat caaattataa 960

aatacctgtg atcgtcgcaa ccgaaatact caattctatg atcaacagtt catcaccaac 1020

aagggttgaa ataatggatg ttgcaaacat agttttagat ggagcagatg ctatactgct 1080

aacctctgaa acagctgtgg ggaactttcc gatcgaaaca gttgaaaaaa ttaatgagat 1140

tgttgaaaat gttgagaact acctacccga aatcaatgct cattttaaag aacgcaggtt 1200

tgaaaaaatc gaagatccat ctgaagctat tgcaaggagt agttactaca tttctgaaga 1260

aataaatgcc aaagctataa taatatcaac agcttctgga agcactgcaa gaagggtggc 1320

ctatttcaag ccacttcgtc ctatcatagc tacgacccca gatgaaaaca cctttcatca 1380

gttgtctatt gtttggggga tagttccgat gctaattcca gaagtccatt ccacagatat 1440

aatgatccac gtggccgtcg agaaggttaa agctgtcgga tatgttcaaa attccgacat 1500

tgtggttgtt acttctggtg ctccgtgtgg tattgttgga acaactaaca tgctcaaagt 1560

tcacatagtt gagtagtgat ttagtttcgg tgtctatctc ttaatagcct cgatttattt 1620

tcggggctat taatcaactc tcagaggcga caagcttctt cttcccttac gacgttttta 1680

ttggttggac atggcaaggt tctctcttct gccgtta 1717

<210> 114
<211> 2017
<212> DNA
<213> Artificial Sequence

<220>
<223> Synthetic Construct- Sequence No. 114, Example 114: designer
      Prochlorococcus marinus MIT9313 groE-promoter-controlled
      Acetolactate Synthase DNA construct (2017 bp)

<400> 114
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgtca agattgctta gaggcttctt 180

tattaaaatg ttcctcaaat ttttttggaa aacgctgatc ggccaaatga atgttgtcgc 240

tgtaatcgac cggaatatca atgacaacag gcccctctgc atcaagacca gctttaagca 300

cctctgccaa ttcgtcaggt gaattgaccc ttaaaccttt tgcaccaaag ctttcagcat 360

attttacaat atcgattccg ccgaagtcga ctccggacgt ccgcttgtat ttcatctcct 420

gctggaacgc aaccatatcg tatgtgctgt cattccagac aatgtgaacg atcggcgctt 480

ttaatctgac cgctgtctca agctccatcg cggagaacag gaagcccccg tccccggaaa 540

cagacacgac tttctgtccc ggattgacca gcgttgctgc aatcgcccac ggcaaagcca 600

ccccaagcgt ctgcatgccg ttggaaatca gcagtccatg cggacggtag gtgcggaaat 660

atctagacat ccaaatcgca tgggagccga tgtcgcaagt caccgttatg tcatcgctca 720

gcagttcacg caaatcgcga acgatttgca gcggatgaac aagatcagtt tttgtttctt 780

taggaggttc gctttgctcc tccagtgctt tcttcaagta atcaaggaca ggtgcaaagg 840

actcgtcgat ggaaaccggc agagaatcat gttcaatatg gtttaacgtc tctgcgatat 900

cgccgatcaa ctcgatttcg ggctgatagt catgatcgat atcggcttgt atttcgtcaa 960

gatgaatcac gcttcgttcg ccttttccat tccaaaagac cggatcgtat tcaatcggat 1020

catagccgac cgtcaaaacg acatccgctt tttccaatag catgtctccg ggctgattgc 1080

ggaatagtcc gatccggccg aagtactggt cttccaaatc gtgagacagc gtacccgctg 1140

cttggtatgt ttcaacaaac ggcagtttca ctttccttag cagacgccga accgcttcaa 1200

tcgcttcagg tcttccgcct ttcatcccga caagcacgac aggaaggttc gcattgtgaa 1260

ttttggcgat ggccgcgctg atttgttcgt ccgaagccgc gcccagcttc ggcgccggca 1320

tggttttcac cggcttggca gttgccggac cggccgtaac gtcctgcgga aagctgagaa 1380

acgctgcgcc agcctgtcca gaagccgccg ctctgaatgc attggttaca gcctcaggta 1440

tgttgttcgc atcttccact tctgcgctat atttcgtaat cggctgaaac aacgccgcat 1500

tatccatcga ttgatgagtt tttttgagac gatccgctct ttttacagca cccgccaggg 1560

caacaaccgg atctccttct gtattggctg ttacaagacc ggtcgctaaa ttagacgctc 1620

ccggacctga agtcaccagg caaacaccgg gctttccagt caatcgtccg actgccgccg 1680

ccataaatgc tgcattctgc tcgtgacggc aaacgatcaa ttcaggcccc ttgtctttca 1740

atacgtcaaa caccgcatcg attttcgctc ccggaatacc gaaaacatga gtgacacctt 1800

gctgaatgag actatccacc acaagctctg ctcctcttac agtaagagtt tcatttttag 1860

cggctacatt attcaatgat ttagtttcgg tgtctatctc ttaatagcct cgatttattt 1920

tcggggctat taatcaactc tcagaggcga caagcttctt cttcccttac gacgttttta 1980

ttggttggac atggcaaggt tctctcttct gccgtta 2017

<210> 115
<211> 1588
<212> DNA
<213> Artificial Sequence

<220>
<223> Synthetic Construct- Sequence No. 115, Example 115: designer
Prochlorococcus marinus MIT9313 groE-promoter-controlled
Ketol-Acid Reductoisomerase DNA construct (1588 bp)

<400> 115
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgtca ctcctcatcc acgttcctct 180

ccttgagcca aggcatcatt tttctgagtt cttttcctac tttttctatg agatgctctg 240

attctttctt tctcatggtg tagaagtagg gtcttcccgc ctgattttcg agtatccagt 300

ccttggcgaa ctttcccgtc tgaatgtcct tgagcatctg cttcatgttc tccctcactt 360

cttttgtaac gatcttttcc tgactgatgt agtcaccgta ctccgcggtg ttgctgacgg 420

agtatctcat gaaagagaga ccaccctcgt agatgaggtc aacgatgagc ttgagctcat 480

tgagacactc aaagtaagct atttccggtt gataacccgc ctccacaaga gtttcgaaac 540

cagcttttat gagagccgtt actccaccac agaggaccgc ctgctctcca aacaaatccg 600

tttccgtctc ttccttgaag gtcgtctcta tcacacccgc ccttgtcaca ccgatacctt 660

tggcataagc gagcgctata tctttggctt taccggtgta gtcctgatag accgctacga 720

gagccggcac accccttcct tcgacgtatt ctcttctcac gatgtgacca gggctcttcg 780

gagcgatcat cgtcacatcc acgttcttcg gaggtatgat ctggtgatag tggatgttga 840

acccgtgggc gaacatcagc atcttaccct cggtgaggtg tttttctatg tattttttgt 900

agatctccgg ctggttctca tctgggatga gcatcatgat gatgtcggcc tcttttgccg 960

cttcttctat tgctttcacg gtgagaccct gttcctccgc cttcttccag ctcttgcttc 1020

cctctctcaa tccgaccaca acgttgagac cgctgtcttt cagattcaac gcgtgcgcat 1080

gcccctgact tccgtaccct atgatcgcga tctttttgtc cctgatcagt tcgagatccg 1140

cgtctttgtc ataataaatc actgccattg atttagtttc ggtgtctatc tcttaatagc 1200

ctcgatttat tttcggggct attaatcaac tctcagaggc gacaagcttc ttcttccctt 1260

acgacgtttt tattggttgg acatggcaaa acaatccagt cgagagctag cgcttgaacg 1320

ccgtaaggcc ctgagtaatt caggtaagaa atcaaccaca ttaaatggat caagtcctaa 1380

tcgcatccgt actgcctctg atgcacgtct aaccaggact gatcaatctt tcgttaaggc 1440

tgggaaagaa tctgtgcagc taaccgctcc taagagagag caactagata cgtcttttgt 1500

tgcttctaga gaatcatccg gagcttcgcg ccgtcaagtg aaaacgatcc gaaattcaag 1560

cagagaatgg ttctctcttc tgccgtta 1588

<210> 116
<211> 1960
<212> DNA
<213> Artificial Sequence

<220>
<223> Synthetic Construct- Sequence No. 116, Example 116: designer
      Prochlorococcus marinus MIT9313 groE-promoter-controlled
      Dihydroxy-Acid Dehydratase DNA construct (1960 bp)

<400> 116
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgcta atctttcaag atagcgcctg 180

tactggcgga ttgcaccatt tttgaatatc ttttcatata gcctgagttt atcttgggtt 240

ccggaactcg ccactgcgtt ctcctggcag caagttcctc gtcgctaagt cgtacactaa 300

ggctgtggtt cggtatatca atgacgatta tgtctccgtc ctgaataaga gcaatattgc 360

ccccttgtgc cgcctcggga gatacgtgcc ctatagaagc tccccgggta gcaccagaaa 420

aacggccatc ggttataagg gctacgtcct tgtctagccc catccccgct atagcagaag 480

taggcgtgag catttccctc atccctggcc ctccgcgcgg cccttcatac cggattacga 540

cgacttctcc tttttcaatc ttgcctccta gaatggctgc aacagcttct tcttccgagt 600

cgaaaacccg tgctttaccc tcgtgataga gcatgtccgg gtcaactgcc cccttcttca 660

ccaccgcgcc ctcctcggcc aggttaccga acagtatagc caaccccccg gtagtactgt 720

gcggattctc tatactacgg atgacctcgt ggtccttcac agggtagtca ttgataacct 780

ccccaaccgt cttaccagta accgtcaggc actggcggtt taccagccct gccttgtcta 840

actcgttgaa aatggcctga actccacccg cagcgtacaa gtcttctata aaatggttgc 900

cggcaggttc gattttacat aggtgaggag tggtatcgct gatatggttt atcaggttca 960

agtccagctt aacgccggct tcgtgagcga tagccataag atgcaaaacc gtattggtag 1020

aacaacctat tgccatgtcc aggcgtaagg cattgatgaa agcctcttgg gtcatgatat 1080

cccgggcttt tatgtccctt tcccatagct ccatcacttt catgcccgcc tgtttggcta 1140

gacggattcg ttcagagtgt accgccggta tagttccgtt cccgggtaag cccattccta 1200

ccgcttccgt caggcagttc atggaattag cggtaaacat accggcacag ctgccacaac 1260

ccgggcaagc tacatcttca agctccgcta agtccgaaag cgacatctta cctgcgctga 1320

ccgcgcctac cccttcaaag accgtgttga ggctgacctt gcgcccccgg aaattgccag 1380

ccaacatcgg ccctccgctg acaaacatac aaggaagatt gaggcgagcg gccgccatca 1440

gcatcccagg gatgattttg tcgcagttag gtataaaaac cagggcatcg aaaggatgag 1500

ccatagccat tatctcaata gaatcggcaa tcagttcccg gctggccaga gagtatttca 1560

taccgatatg gttcatggca ataccatcgc atacccctat agtagaaaac tctataggag 1620

tgcccccttt catcctcacc ccggctttga ccgcctcggc tatacggtct agatgaatat 1680

gccctggaat aatctcgttg gccgagttga ctataccgac taagggtctc tccaactcct 1740

catcggtcaa tcctaaagct ttaaacagtg aacggtgagg tgctttttct agaccctttt 1800

tcgccaggtc acttctcatt gatttagttt cggtgtctat ctcttaatag cctcgattta 1860

ttttcggggc tattaatcaa ctctcagagg cgacaagctt cttcttccct tacgacgttt 1920

ttattggttg gacatggcaa ggttctctct tctgccgtta 1960

<210> 117
<211> 1945

<212>  DNA
<213>  Artificial Sequence

<220>
<223>  Synthetic Construct- Sequence No. 117, Example 117: designer
       Prochlorococcus marinus MIT9313 groE-promoter-controlled 2-Keto
       Acid Decarboxylase DNA construct (1945 bp)

<400>  117
agaaaatctg gcaccacacc ccctttcaga gcggcgcaac attaccactg catggcgaga      60

tcttctcagg gttcggtgac ccgcacaggt atccactagt cggcacagca tcaacacaca     120

tagggttggc actcaatggc cacgagtgct actcatgtta tgatttattt tgttcagcaa     180

atagtttacc catttttttc agtacttttg gtgcatcttc ttttgccaaa actaactcaa     240

tccagtacat tctatttgga tctgcttgag cttctttcat gacagacaca aattcatttt     300

cagttctaac gattttcgag actactcgtt cttctgttgc tccaaatgat tctggtaatt     360

ttgagtaatt ccacattgga atatcattgt agctttgatt tggtccatga atttctcttt     420

cgactgtata accatcatta ttgataataa agcaaattgg attaattttt tctctgattg     480

ctaatcctaa ttcttgcact gtaagttgaa gtgaaccatc accaataaat aaaaggtgtc     540

tgctttcttt atctgcaatt tggcttccta atgctgctgg gaatgtatat ccaattgatc     600

cccataaggg ttgaccaata aaatgactct ttggttttaa gaaaattgat gaagcgccaa     660

agaatgatgt cccttgttca gcaacgattg tttcattgct ttgagttagg ttttcaactg     720

cttgccatag gcggtcttgt gataaaagcg catttgatgg aacaaagtct tcttgctttt     780

tatcgatata ttttcctttg tattctattc cgcttaggtc taagagagag gagatgaggg     840

attcaaaatc aaaattttgg atgctttcgt taaatatttt tccttcgtct atgttcagtg     900

aaatcatttt attttcattt aaatgatggg taaatgctcc tgttgaagag tctgtgagtt     960

taactccaag catcaggatg aagtcggctg attccacgaa ttctttaaga ttaggctctg    1020

agagtttacc attatagatt cctaaaaatg aagggagagt ttcatcaact gaactttttc    1080

caaagtttaa tgtcgtaata gggagttttg tctttgaaat aaattgagtg actgtatttt    1140

ctaagccaaa gctaattatt tcatgtcctg taatcacgat tggttttttg gcatttttca    1200

agctttcttg aattttattc aaaatctctt ggtcacttgt atttgaagtt ggattttctt    1260

ttttcaaagg gagtgagggt ttctctgctt ttgcagcagc aacatcaact ggtaagttga    1320

tatagacagg ttttctttct tttagtagtg cagaaagtac tcggtcaatt tcaacggttg    1380

cattttctgc tgtcagtaaa gttcgagctg ctgtaacagg ttcgtgcatt ttcataaagt    1440

gtttaaaatc accgtcagcc agcgtatgat gaacaaattt tccttcattt tggacttttg    1500

atgtaggtga tcccactatt tctactactg gtaaattttc ggcgtaactt cctgctaatc    1560

cattaactgc actcaattca cctactccaa aggttgtaag aaatgcggca gcttttttag     1620

tacgagcata gccatcagcc atataagaag catttaattc attagcattt ccgacccatt 1680

tcatatcctt gcgggaaata atttgatcta aaaattgtaa gttatagtct ccagggactc 1740

caaaaatttc ttcaattcct aactcgtgta atcggtctaa taggtaatct cctactgtat 1800

acattgattt agtttcggtg tctatctctt aatagcctcg atttattttc ggggctatta 1860

atcaactctc agaggcgaca agcttcttct tcccttacga cgtttttatt ggttggacat 1920

ggcaaggttc tctcttctgc cgtta 1945

<210> 118
<211> 1138
<212> DNA
<213> Artificial Sequence

<220>
<223> Synthetic Construct- Sequence No. 118, Example 118: designer
      Prochlorococcus marinus MIT9313 nirA-promoter-controlled Alcohol
      Dehydrogenase DNA construct (1138 bp)

<400> 118
agaaaatctg gcaccacacc cctctagttg ccaacatcag ccgggttcag aatgtacaca 60

aagacaccaa ttcttgaatt tcacacaaat gctcagtttt gttcaatctg ataccgccaa 120

taccctcttc catcaggctt aatgactcca ttcgcaactt acccttgctt caataatggc 180

agggcctaaa aaccatggct atcgaatgcg cgaataaaat ttaaacaaca tgatcacttc 240

ttttcaagtt taatttcaac aaaatttcat gttgtcaccg gtggagcgaa tgggatcggc 300

aaggcgatcg ctagagcaat tcgcaaaaca gggagcgaac gtcgtgatca tcgaccgcga 360

tattcaaaac ggtgaagcgt tcgccgcgca attgcaatcg gacgggttcg aggcgatctt 420

tgtggcggcg gatgtgcgga aggtggacga tattgaacgg tttgtacaag aagctgccgg 480

ccgcttcggc cgcattgact atttgatcaa caatgctggc gtctcacgct ggaagtcgcc 540

gtatgagctg acggttgagg agtgggatga cgtgctgtca acgaatttgc gcagcgcttt 600

ttttgcttct cgagaagcag ctaaatatat gcgccgcaat gcaaaaggcg gagcaatcgt 660

caacattgcc tcgacaaggg cgctcatgtc cgagccgaat tccgaggcgt acgctgcatc 720

gaaaggcggc cttgtcgctt tgacccatgc gctggcggtg tcgtttgcgg atgatcgcat 780

tcgcgtcaat tgcatcagcc ccggttggat tgaaacgggc gattatggcc aactgcgaga 840

cattgaccac cggcagcacc cggccggccg cgtcggcaaa ccggatgata tcgcccgcgc 900

ttgtctgtat ttatgcgatg aggaaaacga ttttatcacc ggggtaaatt tggtcatcga 960

cgggggaatg accaggaaaa tgatttatat tgagtagtga tttagtttcg gtgtctatct 1020

cttaatagcc tcgatttatt ttcggggcta ttaatcaact ctcagaggcg acaagcttct 1080

tcttccctta cgacgttttt attggttgga catggcaagg ttctctcttc tgccgtta 1138

<210>  119
<211>  1816
<212>  DNA
<213>  Artificial Sequence

<220>
<223>  Synthetic Construct- Sequence No. 119, Example 119: designer
       Prochlorococcus marinus MIT9313 groE-promoter-controlled
       2-Isopropylmalate Synthase DNA construct (1816 bp)

<400>  119
agaaaatctg gcaccacacc ccctttcaga gcggcgcaac attaccactg catggcgaga      60

tcttctcagg gttcggtgac ccgcacaggt atccactagt cggcacagca tcaacacaca     120

tagggttggc actcaatggc cacgagtgct actcatggtg agccagcgcg tttatatttt     180

tgacaccact ttgagggacg gcgagcagtc gcccggcgta agcctgaacg taggcgagaa     240

ggtgcaaatt gccaggcagt tagccaagct cggggtggac ataattgagg ccggctttcc     300

gattacctcg ccgggggact ttaaagccgt aagcgaaatt gcccggcagg tgaagggcgt     360

tacggtggcc gccctggcca gggccaactt ccaggatatc gaccgggcct gggaggccgt     420

gcgccacgcc gagcagccgc ggattcatac ctttattgcc acttccgaca ttcatttaaa     480

atacaagctg cgcatgagcc gggaggaagt cctggatgcg gcggtggcgg cggtaaagcg     540

cgccagggcc tacaccggcg atgtggagtt ttcggcggag gacgcctccc gctccgacct     600

ggacttcctc tgccgggtgc tggccgcggc cattgaggcg ggggctaccg taataaatat     660

accggatacg gtcggttatg ccgttcctga ggaatggggg aaatttatca atactattta     720

tcataaagtt cccggaattg aaaaggtcat tgtcagcgtg cactgccaca acgacctggg     780

catggccgtg gccaactccc ttgctgccgt aatgaacggc gccaggcagg tggaaggggc     840

catcaacggc attggcgagc gggcgggaaa cgctgccatc gaagagatgg taatggccct     900

ttatacccgt aaagatcagt acaaccttta caccaacatc aaaaccgagg aaatttacag     960

gaccagcaag ctggtgagcg ccctgacggg catgaaggtg cagccgaaca aggccgtggt    1020

gggcaaaaac gcctttgccc acgaggccgg cattcaccag gacggggtgc tgaaggagcg    1080

caccacctac gagataatga acccggccat ggtagggatc agcaagagca acctggtgct    1140

gggcaagcat tccgggcggc atgcattccg ccaccggctg gaggaaatgg gctacaatct    1200

ttcggacgaa gagctgaaca gcgcctttga gcgcttcaaa aagctggccg acaagaagat    1260

ggagattacc gacgaagacc tggaagccat tatagaagaa gaaatgcgcc ttgtgccgca    1320

cacctacacc cttgagtacc tgcatatttc cagcggcacc acggtggtgc ctaccgccac    1380

ggtgggctta aagcgggacg ggcagcttat ggaagaggcg gcctgcggca acggcccggt    1440

ggacgccatc tgcaaggcaa ttgataaaat aacggggctt aactgcacca tgacgagctg    1500

gggaatcaac gccgtcactg cgggcaagga cgcccttggc gacgtcagcc tgaaggtgac    1560

cgccgacggc gagaaggttt acgttgggcg cggaatcagc accgatgtgc tggaggccag 1620

cgccaaagct tacgtcaacg cggtcaacaa actcatctgg gattcgcaga aataatgatt 1680

tagtttcggt gtctatctct taatagcctc gatttatttt cggggctatt aatcaactct 1740

cagaggcgac aagcttcttc ttcccttacg acgtttttat tggttggaca tggcaaggtt 1800

ctctcttctg ccgtta 1816

<210> 120
<211> 2199
<212> DNA
<213> Artificial Sequence

<220>
<223> Synthetic Construct- Sequence No. 120, Example 120: designer
      Prochlorococcus marinus MIT9313 groE-promoter-controlled
      3-Isopropylmalate Dehydratase DNA construct (2199 bp)

<400> 120
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgatg gccatgacca taaccgaaaa 180

aattctggcc gatcacgccg gcaaaaagca ggttgagccc ggcgaactga tcagcgtaaa 240

ggttgatctg gtgctgggca acgacataac ggcgccggtg gcgattaaag agtttgagaa 300

aataggggtg gcggaagtct ttgaccggga gcgggtggcc ctggtcccgg atcactttac 360

ccctaacaag gacattaagt cggcggaaca gtctaaaatt ctaagggagt tttccaaaaa 420

gcacaacctt gccaactatt tcgaggtggg ccgggccggc attgagcact gccttctgcc 480

cgaggaaggg ctggtaggcc ccggcgacct ggttatcggc gccgactcgc acacctgcac 540

ctacggcgcc ctgggggcct tctccacggg cgtgggcagc accgacctgg cggctgccat 600

ggcgctgggg gaaacctggc tgaaagtgcc ggagtcaatc aaattcgaat atgacgggga 660

aatgcagccc tgggtaggcg gcaaggacat gatcctgcac acaatcgggg atatcggggt 720

ggacggggcc ctttacaagg ctatggagtt taccggcccg gccgttgaaa aactttccat 780

ggacgggcgc tttaccatgt gcaacatggc cgtagaggcc gggggtaaga acggcattat 840

tgctccggac gaaacaaccc gggtctatgt cgagggccgc tgcaagcgac cctatcgttt 900

ttatcggagc gacccggacg ccaaatacga aaagatctac cgctacgacg cggcgcagat 960

cgaaccgcag gtggcctttc cccacctgcc cgaaaactcc cggccggtca gcgaggcagg 1020

caacattgaa atcgatcagg ttgttatcgg ctcctgcacc aacggccgga tggaggacct 1080

gcgggaggcc gccagggtgc tgaagggcag aaaagtgcat aaaaacgtcc gccttattat 1140

ttttccggga acgccgaaaa tttacctgca ggccttgcgg gaggggctga tcgaaacttt 1200

tgtcgaagct ggcggagtcg tgagcacgcc cacctgcggg ccctgcctgg gcggccactc 1260

gggcattctg gccaggggag agcgctgcgt tgccaccacc aaccgcaact ttgtaggcag 1320

gatggggcat cctgaaagcg aagtgtacct gtccaacccg gcagttgccg cggcttcggc 1380

cgtgctgggc cggataggcg gtccatggga ggtggattga ccctttcaga gcggcgcaac 1440

attaccactg catggcgaga tcttctcagg gttcggtgac ccgcacaggt atccactagt 1500

cggcacagca tcaacacaca tagggttggc actcaatggc cacgagtgct actcatgatg 1560

gaaattaaag ggaaagtgtg gaagttcggc ccggatatcg atacagacgc cattataccg 1620

gcaaggtacc tcaacacctc cgacccggaa gaactggcca ggcactgcat ggaggatgcc 1680

gacccgtcct ttcccgcccg ggtcaggccc ggcgacgtga ttgtggccgg caagaatttc 1740

gggtgcggca gttcccggga gcacgccccc atagcaatca aggccgccgg ggtgtcgtgc 1800

gtgattgccg cgtcgtttgc gcggatcttc taccgcaacg ccttcaacat agggctgccc 1860

attttcgagt ctcccgaagc cgccgggggc attggccagg gcgacgaggt ggcggtggac 1920

gcggctgccg gcattataac cgacctgacc accggcaaga cctaccgggc ggcgccggtt 1980

ccgcctttca tgcggcagat cattgccgcc ggagggctga tcaattacgt ggccgggaag 2040

gtgagaggca atgcataatg atttagtttc ggtgtctatc tcttaatagc ctcgatttat 2100

tttcggggct attaatcaac tctcagaggc gacaagcttc ttcttccctt acgacgtttt 2160

tattggttgg acatggcaag gttctctctt ctgccgtta 2199

<210> 121
<211> 1378
<212> DNA
<213> Artificial Sequence

<220>
<223> Synthetic Construct- Sequence No. 121, Example 121: designer
Prochlorococcus marinus MIT9313 groE-promoter-controlled
3-Isopropylmalate Dehydrogenase DNA construct (1378 bp)

<400> 121
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgcta cacctcgacc tcctcaactt 180

ttctcgctac taaatcaccc atttccctgg tgttaaccag cttctgatcc ggctccgtaa 240

tgtccggcgt ccggtagcct tcggccagaa cttcgcggac cgcctgctca accgccaaag 300

cctcttgttc caaatcgaac gaatacctca gcatcatggc agccgacagt atcgtagcca 360

acgggtttgc tttcccctgc ccggcgatat cgggagctga cccgtgagaa ggctcataca 420

ttcctacttt cccgccaata gaggcagaag gtagcattcc caaagatccg gtcagcatag 480

aggcttcgtc ggtcaatata tctccaaaca tgttttcagt tacgatcaca tcgaattgac 540

gcggattgcg tatgagctgc atggcacagt tgtcgacgta catgtggctg aattcgacgt 600

caggatactc cagagctact cgattggcca cctcgcgcca taacctagag ctttctagaa 660

cattggcctt gtccaccgat gtcactttct ttctccgttt cctcgccgcc tcgcaggcca 720

aacgaactat gcgttcgatc tcatacgtcg agtactccag aacatcgata gccctttctc 780

cgcccagaag cttctcccgc cgcttctccc cgaagtacaa cccgccggtc agttccctca 840

ctaccaagag atctactccc tcgataatat cgggtttcag ggaggaagca tgaaccagtt 900

ccgggaacag gtaagcgggc cgcaggttag cgtaaagccc aagttcctta cgcagagcca 960

acagcgctgc cgcctcgggc ctgagcgcag ccggcaggtt atcccatttg ggaccaccta 1020

tggctcctag aagaacagcg tcgctatctt tgcacagggc cagggtttct tcaggcaaag 1080

gaacccccac ctcgtcgata gccgctcccc cgaccagggc ttcggtaaaa gcgaattcgt 1140

gtttgaacct cttagcaact gctttcaata ctttttgcgc ttcaggtacg atctcggtcc 1200

cgataccatc cccgggtaac acggctatct taaacactga tttagtttcg gtgtctatct 1260

cttaatagcc tcgatttatt ttcggggcta ttaatcaact ctcagaggcg acaagcttct 1320

tcttccctta cgacgttttt attggttgga catggcaagg ttctctcttc tgccgtta 1378

<210> 122
<211> 1327
<212> DNA
<213> Artificial Sequence

<220>
<223> Synthetic Construct- Sequence No. 122, Example 122: designer
 Prochlorococcus marinus MIT9313 groE-promoter-controlled
 3-Methylbutanal Reductase DNA construct (1327 bp)

<400> 122
agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatgatg tcagttttcg tttcaggtgc 180

taacgggttc attgcccaac acattgtcga tctcctgttg aaggaagact ataaggtcat 240

cggttctgcc agaagtcaag aaaaggccga gaatttaacg gaggcctttg gtaacaaccc 300

aaaattctcc atggaagttg tcccagacat atctaagctg gacgcatttg accatgtttt 360

ccaaaagcac ggcaaggata tcaagatagt tctacatacg gcctctccat tctgctttga 420

tatcactgac agtgaacgcg atttattaat tcctgctgtg aacggtgtta agggaattct 480

ccactcaatt aaaaaatacg ccgctgattc tgtagaacgt gtagttctca cctcttctta 540

tgcagctgtg ttcgatatgg caaaagaaaa cgataagtct ttaacattta acgaagaatc 600

ctggaaccca gctacctggg agagttgcca aagtgaccca gttaacgcct actgtggttc 660

taagaagttt gctgaaaaag cagcttggga atttctagag gagaatagag actctgtaaa 720

attcgaatta actgccgtta acccagttta cgtttttggt ccgcaaatgt ttgacaaaga 780

tgtgaaaaaa cacttgaaca catcttgcga actcgtcaac agcttgatgc atttatcacc 840

agaggacaag ataccggaac tatttggtgg atacattgat gttcgtgatg ttgcaaaggc 900

tcatttagtt gccttccaaa agagggaaac aattggtcaa agactaatcg tatcggaggc 960

cagatttact atgcaggatg ttctcgatat ccttaacgaa gacttccctg ttctaaaagg 1020

caatattcca gtggggaaac caggttctgg tgctacccat aacacccttg gtgctactct 1080

tgataataaa aagagtaaga aattgttagg tttcaagttc aggaacttga aagagaccat 1140

tgacgacact gcctcccaaa ttttaaaatt tgagggcaga atataatgat ttagtttcgg 1200

tgtctatctc ttaatagcct cgatttattt tcggggctat taatcaactc tcagaggcga 1260

caagcttctt cttcccttac gacgttttta ttggttggac atggcaaggt tctctcttct 1320

gccgtta 1327

<210> 123
<211> 2004
<212> DNA
<213> Artificial Sequence

<220>
<223> Synthetic Construct- Sequence No. 123, Example 123: designer
      Cyanothece sp. ATCC 51142 nirA-promoter-controlled
      2-Isopropylmalate Synthase DNA construct (2004 bp)

<400> 123
agaaaatctg gcaccacacc tattaaatct aaaatagctg ttttagctaa aatagtcaat 60

agcaagtctt ataggtaatc aaacgcaact aaaatgcaaa aaatccataa ttaaaatgca 120

aaaaacggat ttttaataca attttgttac attagctaca aaatatctca aatggtagag 180

gttaaatagg tacaactcga ccagatggag ggttttccct gtgatgaact gttccttctt 240

cccttatact ctccttggat agaatgtagt tccttctcat gatggctctg tttaaggcat 300

ctacaaaacc gcttacgctg gcttttatta tgtccgtatc cacgcctcta cctgaggctt 360

ttacattatc cagctctatc acaaggcgcg cctctgcctg cgcatccgtg ttgggggtga 420

gagcttttat agaaaagtca atgagtctgg gctccacctt aagagctttc tgtatggctt 480

ttatcacagc atccacagga ccgtttcccg tagatgtggc agtcctttct tcacctctaa 540

agctcagcac tactgtagcg gtaggaagca ggttgtcccc tgtctgaacc tgatagtgtt 600

ttacctttat aggctcttcc tcctccacct tcataaactc ttcgtatatg agggcttcca 660

aatcctcatc atatacctcc tttttcttat ccgcaagagc cttgaacttt tcaaagatcc 720

tctccaggtc ttcatcgctt agcttaaagc caagttcatt cagtctcctc tttagagcgt 780

gcctccctga gtgtttacca agtattattc tggtggaggg aaaacctaca tcctcggggt 840

tcattatctc gtaggtgaga gggtgagcca gcacaccgtg ctggtgtatg cccgattcat 900

gagcaaaggc attatccccc actatagcct tgttgggttg aacaaaagag ccggttatcc 960

tgcaaaggag cctgctggtt ttgtatatct ctctggtgtt tatgtccgtg tagagccctc 1020

caaagaagtc tttgcgcact ttgagagcca tcactatctc ctcaagggct gcgtttcctg 1080

ctctttcacc tatgccgttg atggtgcact ctacctgtct tgcaccgtgc tttaccgcca 1140

taagggagtt ggcaacagcc atcccaaggt catcatgaca gtgcacgctt ataatggctc 1200

tgtctatgtt gggcacgttg ttccttatgt cctctatgag ccttgcaaac tcttctggca 1260

ccgcatagcc aacggtgtcg ggaatgttta taacggtagc acctgccttt atggctgttt 1320

ctatcaccct gtagaggaac tctctctggc ttctggtggc atcctcgcag gaaaactcca 1380

catcgtcagt aaaccttctg gcaaactcca cagctttttt agccctttcc agaacctcct 1440

ctggggacat cctaagcttg tacttcatgt gtatctcgga agtagctatg aaggtgtgta 1500

tcctctttct tctggctggc tttagagcct cccctgctag ctctatgtcc ttttccaatg 1560

ctctggcaag ggagcatatt atcggacctt ctacctgctg tgctatcaga tggacgctct 1620

caaagtctcc cttagatgct gctgcaaagc ctgcctctat aacatccacc cccagcttgg 1680

caagttggtg agccatctga agtttttcat cagcagtcat agaaaaaccc ggcgcttgct 1740

ctccgtctct cagcgtggtg tcaaatatgt aaaccttctc cattaagctg ttttagagaa 1800

atttgttcgg taaatattag cctacctaca gttgttgtgg gtaggctaat attatgaatt 1860

gagtcctact gaaccaatga ttatcgttac gactaaaagt aataaatgtc atcagcagga 1920

taggggttga taggaaaagt tttttaatcg gatggttttc gagttagagg ttagggtttc 1980

tttaggttct ctcttctgcc gtta 2004

<210> 124
<211> 2648
<212> DNA
<213> Artificial Sequence

<220>
<223> Synthetic Construct- Sequence No. 124, Example 124: designer
 Cyanothece sp. ATCC 51142 nirA-promoter-controlled
 Isopropylmalate Isomerase large/small subunits DNA construct
 (2648 bp)

<400> 124
agaaaatctg gcaccacacc tattaaatct aaaatagctg ttttagctaa aatagtcaat 60

agcaagtctt ataggtaatc aaacgcaact aaaatgcaaa aaatccataa ttaaaatgca 120

aaaaacggat ttttaataca attttgttac attagctaca aaatatctca aatggtagag 180

gttaaatagg tacaactcga ccagatggag ggttttccct gtgatggtgc caaagacgat 240

tattgaaaaa atttgggatg aacacgtggt ttaccgtgaa gatgggaaac ccgatttatt 300

atatattgat ttacatctcg ttcatgaagt gacatcgccg caagcttttg aaggattgcg 360

acaaaaagga agaaaagtgc gtcgcccaga tttaacattt gcgacgatgg accataacgt 420

tccaacgatt aatcggtccg ttgttgaaga tgaagtggcg aaaaatcaaa tgacagcatt 480

ggagcggaac tgtcgtgagt tcggtgttcc gcttgccgat ttaaacagtc cagaacaagg 540

gattgttcat gtcatcggtc cagaactcgg gttgacacag cctgggaaaa ccattgtgtg 600

tggagatagc catacgtcta cacatggcgc ttttggggca ttagcgttcg ggatcggaac 660

gagtgaagtc gaacacgtat tagcgacgca aacgttatgg caacatcgtc caaagacgat 720

gcaagtgaac gttacgggat cgttagcacc gggcgtatca gcaaaagacg ttattttagc 780

gatcattggc aagtttggag ttgattttgg cacaggctac gtgcttgagt ttacaggcga 840

tgttattcgt cgtatgtcaa tggaagagcg gatgacaatt tgcaatatgt cgattgaagc 900

aggggcacga gctgggttaa ttgccccaga tgatgtgacg tttgcatatt taaaagagag 960

aaaatatgca ccgaaaggag aagcgtttga gcaggcagtt gaaaagtgga agcagttatg 1020

cacagatgaa ggagcggtat acgatcgtgt cgttcatatt gatggaagtg aaattgctcc 1080

aacagtgaca tggggcacaa cgccagcaat gagctctccg atcgatggaa ctgttccaga 1140

tccgaacgag tttgcgacag aaacagagag aaaagctgta cagttagcgt tgcaatatat 1200

gggattaaag ccaggaacga aaatgacgga tattgcggtg caacatgtgt ttatcggatc 1260

atgcacaaac tcgcgcataa gcgatttacg ggaagcggcg caaattgtaa aaggaaaaaa 1320

agtcgcaccg ggcgtcagag cgctcgtcgt tccgggctca caacaagtaa aaaagcaggc 1380

agaagaagaa ggaattgctc aaacgtttat tgacgcaggc tttgaatggc gcgattccgg 1440

ctgtagcatg tgtcttggaa tgaatccaga tactgttcca gcaggggaac attgcgcctc 1500

aacgtcaaac cgcaatttcg aagggagaca aggaaaaggg gcgcgcacgc atctcgtgag 1560

tccagcaatg gcagccgcgg ctgcgattta cgggcatttt gtcgatgtgc gtacattgta 1620

taaagaagtg gtaagatagt attaaatcta aaatagctgt tttagctaaa atagtcaata 1680

gcaagtctta taggtaatca aacgcaacta aaatgcaaaa aatccataat taaaatgcaa 1740

aaaacggatt tttaatacaa ttttgttaca ttagctacaa aatatctcaa atggtagagg 1800

ttaaataggt acaactcgac cagatggagg gttttccctg tgatggaacc attcgtcgtt 1860

cataaaggaa aagtggctgg cttagatcga gcaaatatag atacggatca aattattccg 1920

aaacaatttt taaaacgaat tgaacgcacc ggatttggtc aatttctttt ttacgattgg 1980

cgttatttat cggacggaac accaaaccca cattttgagt taaaccgtcc tgaaaacgag 2040

ggcgcgacca ttttagtcgc aaatgaaaat ttcggatgtg gttcatcgcg cgaacacgct 2100

ccttgggcgc ttgcggatta cggatttcgt gccattattg ctccttcatt tgctgatatt 2160

ttttacaaca actgtttgaa aaatagttta cttcctatta aacttccaaa agaagacgtc 2220

gcttatttgt taaaacaagc ggaacgggca gattacgaac taacgatttc gcttgaacaa 2280

caagtcgttt ttgatgatga agggtttaca agctcgttcg acatcgatcc gtatcgaaaa 2340

cagctccttt taaaaggttg ggacgaaatt gatttaacgt tcgtgtatga accatatatt 2400

atcgcctacg aaaaaaaacg ctcttgataa gctgttttag agaaatttgt tcggtaaata 2460

ttagcctacc tacagttgtt gtgggtaggc taatattatg aattgagtcc tactgaacca 2520

atgattatcg ttacgactaa aagtaataaa tgtcatcagc aggatagggg ttgataggaa 2580

aagtttttta atcggatggt tttcgagtta gaggttaggg tttctttagg ttctctcttc 2640

tgccgtta 2648

<210> 125
<211> 1530
<212> DNA
<213> Artificial Sequence

<220>
<223> Synthetic Construct- Sequence No. 125, Example 125: designer
      Cyanothece sp. ATCC 51142 nirA-promoter-controlled
      3-Isopropylmalate Dehydrogenase DNA construct (1530 bp)

<400> 125
agaaaatctg gcaccacacc tattaaatct aaaatagctg ttttagctaa aatagtcaat 60

agcaagtctt ataggtaatc aaacgcaact aaaatgcaaa aaatccataa ttaaaatgca 120

aaaaacggat ttttaataca attttgttac attagctaca aaatatctca aatggtagag 180

gttaaatagg tacaactcga ccagatggag ggttttccct gtgatggcaa ccacgtatcg 240

cattgctgtt ttggcggggg atggtattgg cccagaaatt acggccgttg cccttgatgt 300

attgcgggcg atcgcccctc gctttgggtt ggactttgac tttgttcccg cccttgtggg 360

gggctgcgcc attgatgctg tgggggaacc cttgccagca gcaacgctag ccacctgtcg 420

tcagagtgat gccgtgctcc tagctgccat tggcggaacg cagtgggata gcctaccccg 480

tcatctgcgc ccggaaaccg gattacttgc cctgcggtct ggtctaggtt tatttgccaa 540

cctacgcccc gccaaaatct ttccccagct tctccatgcc tcctccctca agccggaagt 600

gattgccggt gtggatctca tggtggtgcg cgaactgacg ggtggcattt actttggtca 660

accgcgcggt attttcacca ctgaaacggg tgagcagcgg ggggtgaata cgatggccta 720

taccgccacg gaaattgatc gcattggccg tgttgccttt gaaaccgctc gcaaacggca 780

gggcaaactc tgctccgtgg ataaggccaa tgtccttgaa gtctcccaac tgtggcgcga 840

tcgcctgacc gccctcagtg ctgagtaccc ggatgtggaa ctgacgcacc tttatgtgga 900

caatgcagca atgcaactgg tgcgcgcccc gaaacagttt gacacgattg tgaccagtaa 960

cctctttggt gatatcctct ccgatattgc cgccatgctc accggtagta ttggcatgct 1020

tccctccgcc agcctagggg aatcggggcc agctctgttt gaaccggttc atggctctgc 1080

ccccgacatt gccggccaag acaaggccaa ccccctcgcc atggtgctca gtgcggcaat 1140

gatgctgcgt tatggtctga accaaccagc ggcagcgcaa gcgatcgaag aggccattac 1200

tgccgtttta gatcagggct accgcaccgg cgatttaatg tctgagggct gcacgcttgt 1260

gggctgtcgc gaaatgggca acctcctaat caaggaattg tcccgataat aagctgtttt 1320

agagaaattt gttcggtaaa tattagccta cctacagttg ttgtgggtag gctaatatta 1380

tgaattgagt cctactgaac caatgattat cgttacgact aaaagtaata aatgtcatca 1440

gcaggatagg ggttgatagg aaaagttttt taatcggatg gttttcgagt tagaggttag 1500

ggtttcttta ggttctctct tctgccgtta 1530

<210> 126
<211> 2088
<212> DNA
<213> Artificial Sequence

<220>
<223> Synthetic Construct- Sequence No. 126, Example 126: designer
      Cyanothece sp. ATCC 51142 nirA-promoter-controlled 2-Keto Acid
      Decarboxylase DNA construct (2088 bp)

<400> 126
agaaaatctg gcaccacacc tattaaatct aaaatagctg ttttagctaa aatagtcaat 60

agcaagtctt ataggtaatc aaacgcaact aaaatgcaaa aaatccataa ttaaaatgca 120

aaaaacggat ttttaataca attttgttac attagctaca aaatatctca aatggtagag 180

gttaaatagg tacaactcga ccagatggag ggttttccct gtgatgtata cagtaggaga 240

ttacctgtta gaccgattac acgagttggg aattgaagaa atttttggag ttcctggtga 300

ctataactta caatttttag atcaaattat ttcacgcgaa gatatgaaat ggattggaaa 360

tgctaatgaa ttaaatgctt cttatatggc tgatggttat gctcgtacta aaaaagctgc 420

cgcatttctc accacatttg gagtcggcga attgagtgcg atcaatggac tggcaggaag 480

ttatgccgaa aatttaccag tagtagaaat tgttggttca ccaacttcaa aagtacaaaa 540

tgacggaaaa tttgtccatc atacactagc agatggtgat tttaaacact ttatgaagat 600

gcatgaacct gttacagcag cgcggacttt actgacagca gaaaatgcca catatgaaat 660

tgaccgagta ctttctcaat tactaaaaga aagaaaacca gtctatatta acttaccagt 720

cgatgttgct gcagcaaaag cagagaagcc tgcattatct ttagaaaaag aaagctctac 780

aacaaataca actgaacaag tgattttgag taagattgaa gaaagtttga aaaatgccca 840

aaaaccagta gtgattgcag gacacgaagt aattagtttt ggtttagaaa aaacggtaac 900

tcagtttgtt tcagaaacaa aactaccgat tacgacacta aattttggta aaagtgctgt 960

tgatgaatct ttgccctcat ttttaggaat atataacggg aaactttcag aaatcagtct 1020

taaaaatttt gtggagtccg cagactttat cctaatgctt ggagtgaagc ttacggactc 1080

ctcaacaggt gcattcacac atcatttaga tgaaaataaa atgatttcac taaacataga 1140

tgaaggaata attttcaata aagtggtaga agattttgat tttagagcag tggtttcttc 1200

tttatcagaa ttaaaaggaa tagaatatga aggacaatat attgataagc aatatgaaga 1260

atttattcca tcaagtgctc ccttatcaca agaccgtcta tggcaggcag ttgaaagttt 1320

gactcaaagc aatgaaacaa tcgttgctga acaaggaacc tcattttttg gagcttcaac 1380

aattttctta aaatcaaata gtcgttttat tggacaacct ttatggggtt ctattggata 1440

tacttttcca gcggctttag gaagccaaat tgcggataaa gagagcagac accttttatt 1500

tattggtgat ggttcacttc aacttaccgt acaagaatta ggactatcaa tcagagaaaa 1560

actcaatcca atttgtttta tcataaataa tgatggttat acagttgaaa gagaaatcca 1620

cggacctact caaagttata acgacattcc aatgtggaat tactcgaaat taccagaaac 1680

atttggagca acagaagatc gtgtagtatc aaaaattgtt agaacagaga atgaatttgt 1740

gtctgtcatg aaagaagccc aagcagatgt caatagaatg tattggatag aactagtttt 1800

ggaaaaagaa gatgcgccaa aattactgaa aaaaatgggt aaattatttg ctgagcaaaa 1860

taaatagtaa gctgttttag agaaatttgt tcggtaaata ttagcctacc tacagttgtt 1920

gtgggtaggc taatattatg aattgagtcc tactgaacca atgattatcg ttacgactaa 1980

aagtaataaa tgtcatcagc aggatagggg ttgataggaa aagtttttta atcggatggt 2040

tttcgagtta gaggttaggg tttctttagg ttctctcttc tgccgtta 2088

<210> 127
<211> 1503
<212> DNA
<213> Artificial Sequence

<220>
<223> Synthetic Construct- Sequence No. 127, Example 127: designer
      Cyanothece sp. ATCC 51142 nirA-promoter-controlled Hexanol
      Dehydronase DNA construct (1503 bp)

<400> 127
agaaaatctg gcaccacacc tattaaatct aaaatagctg ttttagctaa aatagtcaat 60

agcaagtctt ataggtaatc aaacgcaact aaaatgcaaa aaatccataa ttaaaatgca 120

aaaaacggat ttttaataca attttgttac attagctaca aaatatctca aatggtagag 180

gttaaatagg tacaactcga ccagatggag ggttttccct gtgatggaac tcgacctcga 240

cggtcccggg gttggtgaag tgctgatcaa gtacaccgcc gcggggttgt gccattcgga 300

cctgcacttg accgacgggg acctaccgcc gcgctatcca atcgtcgggg ggcacgaggg 360

gtcaggcatc atcgaggacg tcggacctgg ggtcaccaag gtcaaaccag gcgatcacgt 420

tgtttgcagc ttcatcccga actgcggaac ctgtcggtac tgcgccaccg gacgctccaa 480

cctctgcgat atgggcgcca ccatcctcga agggtgcatg cccgacggca gttaccggtt 540

ccacagtaac ggcctggatt tcggtgcgat gtgcatgctc ggcacattct ccgaacgcgc 600

aactatctcc cagcattcgg tggtcaagat cgacgactgg ctgccgctcg agaccgcggt 660

ggtcgtcggc tgcggcgtgc cgactggctg gggcacctcc gtctatgccg gcggggttcg 720

ttgcggtgac accaccgtca tctatggcgt cggcggcctg ggagtcaacg ccgtccaagg 780

cgcggtgagt gcgggcgcga agtacatcgt ggtcgtcgat ccggttgcgt tcaaacgcga 840

caccgcgctc aagttcggcg ccacccacgc gttcgccgac gccgccaccg ccgcggccaa 900

ggtcgacgaa ctgacctggg gacagggtgc cgatcaggcg ctgatcctgg tcggcaccgt 960

cgacgaggac gtggtctcgg cggcgactgc ggtgatcggt aagggaggca ccgtcgtgat 1020

caccggactg gcggacccag caaagctcac ggtgcacgtt tcgggaacgg acctgacgct 1080

taacgagaag acaatcaagg gcacgttgtt cggctcgtcc aatccgcaat acgacatcgt 1140

acggctgctc cgtctctacg acgccggcca gctaaaactc gacgatctga tcaccacccg 1200

atacacgctc gaccaggtca accagggcta ccaggatctg cgagacggca agaacatccg 1260

cggcgtgatc atccacgcct gataagctgt tttagagaaa tttgttcggt aaatattagc 1320

ctacctacag ttgttgtggg taggctaata ttatgaattg agtcctactg aaccaatgat 1380

tatcgttacg actaaaagta ataaatgtca tcagcaggat aggggttgat aggaaaagtt 1440

ttttaatcgg atggttttcg agttagaggt tagggtttct ttaggttctc tcttctgccg 1500

tta 1503

<210> 128
<211> 1149
<212> DNA
<213> Artificial Sequence

<220>
<223> Synthetic Construct- Sequence No. 128, Example 128: designer
Cyanothece sp. ATCC 51142 nirA-promoter-controlled short-chain
Alcohol Dehydrogenase DNA construct (1149 bp)

<400> 128
agaaaatctg gcaccacacc tattaaatct aaaatagctg ttttagctaa aatagtcaat 60

agcaagtctt ataggtaatc aaacgcaact aaaatgcaaa aaatccataa ttaaaatgca 120

aaaaacggat ttttaataca attttgttac attagctaca aaatatctca aatggtagag 180

gttaaatagg tacaactcga ccagatggag ggttttccct gtgatgaagg ttgccgtaat 240

tactggggca tcccgtggaa tcggggaagc tatagcaaag gcccttgctg aagatggata 300

ttcccttgcc ttaggggcta gaagtgttga taggttagag aagattgcca aggaactcag 360

cgaaaaacat ggggtggagg tattttacga ctacctcgat gtatcaaaac cagaaagcgt 420

tgaagagttt gcaaggaaaa cgctagctca ctttggagat gtggacgttg ttgtggccaa 480

tgcggggctt ggttactttg gtaggcttga agagcttaca gaagagcagt tccacgaaat 540

gattgaagta aaccttttgg gagtttggag aacaataaaa gctttcttaa actccttaaa 600

gcggactgga ggagtggcta ttgttgttac ttcagatgtt tctgcaaggc tacttccata 660

cggtggaggt tatgtggcaa ctaaatgggc tgcaagagca ttggtaagga ccttccagat 720

tgagaatcca gatgtgaggt tcttcgagct aagacctgga gcagtagata catattttgg 780

agggagcaaa gctgggaagc caaaggagca agggtattta aaacctgagg aagttgctga 840

ggcagtaaaa tacctcctaa gacttccaaa ggatgttagg gttgaggaat taatgttgcg 900

ctcaatttat caaaaacctg agtattgata agctgtttta gagaaatttg ttcggtaaat 960

attagcctac ctacagttgt tgtgggtagg ctaatattat gaattgagtc ctactgaacc 1020

aatgattatc gttacgacta aaagtaataa atgtcatcag caggataggg gttgatagga 1080

aaagtttttt aatcggatgg ttttcgagtt agaggttagg gtttctttag gttctctctt 1140

ctgccgtta 1149

<210> 129
<211> 1910
<212> DNA
<213> Artificial Sequence

<220>
<223> Synthetic Construct- Sequence No. 129, Example 129: a designer
      Nial-promoter-controlled chloroplast-targeted Phosphoglycerate
      Mutase DNA construct (1910 bp)

<400> 129
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgactc gtgtcatcat tgtgcgtcat ggtcaaagta 360

catataatgt tgaacgacgt atccaaggac gtactgatgc gtcaacttta acggataaag 420

gtcggagcga tgctggtaaa gtgggtaaag ccctgagtaa tatagcattt acagcaatat 480

atagcagtcc tctcaaccga gcgaagacga cagcggaaat tattcgcagt gagttggttg 540

aacattcgtc cgtgattcag gtttctgaac atctggttga agtagattta cctttgtggg 600

caggaatgtt atctcttgat gtgaaagaga agtttcctga tgactatagt atttggaaaa 660

aacgtcccca cgaattgcat atgattgtca gtgacgcaca cgggacacga gaacttttcc 720

cagttctggc tttatatgaa caagccaagc agttttggca agaaatgtta tctcgtcatc 780

aaggggaaac tattctcatt gttggacata atggtattaa ccgcgctctg attagtacgg 840

ctttgggtat tcctcccagt gtttatcacg gactacaaca gtctaattgc gcgattagcg 900

ttttaaattt tgcgggtggt ttgggtgata cggttcagct agattcaatg aatcagacgc 960

aacatttgga agatacttta cccactttgc gaccaaatca tcaaggattt agattattat 1020

tagtacgtca tggggaaaca gaatggaatc gtcaaggtaa gtttcaaggc caaattgacg 1080

ttcctctgaa tgataatggc agagcgcaag caggaaaaac tggggagttt ctccaagagg 1140

tggcgcttga ttttgctttt agtagcacta tggcgcgtcc aaaagaaaca gcggaaatta 1200

ttcttcagaa gcatgctgat ataaagttgg aattactaga tggtttacgg gaaatcagtc 1260

acggcagttg ggaaggcaag tttgagtcag aaatagaaca agagtttccc ggagtgttgg 1320

aacgctggcg tactgtacct gctgaagtac aaatgccgca aggggaaaat ttacaacagc 1380

tatgggaacg tagtgtggct gcttggcagt caatattaca atcggctgag gtaaatcaat 1440

ggcaaattgg gttggtagtg gctcacgatg ctactaataa aactttactc tgcaatatct 1500

tgggtttatc tccagaaaat ttctggaatt tccgtcaagg taatggggca gttagtgtta 1560

ttgactaccc tttaggcgct agtggtttac cagtactgca agcgatgaac attactagtc 1620

atttgagtgg tggtgtatta gataaaacgg cagcaggagc attgtagtaa atggaggcgc 1680

tcgttgatct gagccttgcc ccctgacgaa cggcggtgga tggaagatac tgctctcaag 1740

tgctgaagcg gtagcttagc tccccgtttc gtgctgatca gtctttttca acacgtaaaa 1800

agcggaggag ttttgcaatt ttgttggttg taacgatcct ccgttgattt tggcctcttt 1860

ctccatgggc gggctgggcg tatttgaagc ggttctctct tctgccgtta 1910

<210> 130
<211> 1856
<212> DNA
<213> Artificial Sequence

<220>
<223> Synthetic Construct- Sequence No. 130, Example 130: designer
      Nial-promoter-controlled chloroplast-targeted Enolase DNA
      construct (1856 bp)

<400> 130
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgctta ggtcctaaac caacagtacc agcataaaca 360

gcgcgatcgc ctaattcatc ttcaattctg agtaaacgat tgtattttgc tacccgttca 420

ctacgacaaa gagaaccagt tttaatttga cctgcacgag tggctacagc caaatcagca 480

atagttgtat cctcagtttc accagaacga tggctaatga ctgagcggaa accgttgcga 540

gtagctaaat caatagtttc caaagtttca gtcagtgaac caatttgatt caacttaatc 600

aaaatcgagt tacctgcttt ttgctcaatc cctttttgta accgagtagc gttagtaaca 660

aataaatcat cacccaccaa ctgtactcgt gaacccaact tctgagtcag taattgccaa 720

ctttcccaat cttcctcatg taaaccatct tcaatggaaa caatcggata ttggtcaacc 780

aactggccta aataatcaat aaactcaact gggctatggg gtttaccatc ataaacatac 840

tgcccatttt tgtaaaactc actcgctgcc acatccaagg ctaaagcaac ttcttcccca 900

ggcttgtaac cagcttgttt aatagcagct agcaataatt ctaaagctac ttggttagag 960

tccaggttag gtgcaaaacc accttcatca ccaacaccag tcagcaaacc cttatcatcc 1020

aaaaccttgc tgagagttgc aaaaacttcc gcaccccaac gcaaagcttc ctggaaggaa 1080

ggcgcactga cgggtacaat cataaactcc tgaaaatcga cattattggc tgcgtgcgct 1140

ccaccattga tcacattcat caaaggtaca ggtagcaaat ttgctaaagg cccacccaca 1200

tagcgataca aaggaattcc caaagactca gcagcagctt tagcagctgc tagtgaaacc 1260

gacaaaattg catttgcgcc caaattagct ttattgggtg aaccatccaa agagatcatg 1320

attttatcta atgattcttg gtctagggca tccaagccta acaattgggg tgctaatacc 1380

tctttcacat tctgcactgc cttgagtact cctttgccac cataacggct tttatcacca 1440

tctcgcagtt catgagcctc aaaagtacct gtggaagcac cgctaggaac ttgcgctagt 1500

cctactgtac cattagccaa atgtactgca gcctcaacag tcggtcttcc ccgtgaatca 1560

agaatttcgc gggcgataat agcttcaata gcggtatcca gaaattttgt cattaaatgg 1620

aggcgctcgt tgatctgagc cttgccccct gacgaacggc ggtggatgga agatactgct 1680

ctcaagtgct gaagcggtag cttagctccc cgtttcgtgc tgatcagtct ttttcaacac 1740

gtaaaaagcg gaggagtttt gcaattttgt tggttgtaac gatcctccgt tgattttggc 1800

ctctttctcc atgggcgggc tgggcgtatt tgaagcggtt ctctcttctg ccgtta 1856

<210> 131
<211> 1985
<212> DNA
<213> Artificial Sequence

<220>
<223> Synthetic Construct- Sequence No. 131, Example 131: designer
      Nial-promoter-controlled chloroplast-targeted Pyruvate-Kinase DNA
      construct (1985 bp)

<400> 131

agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgaaac ccttaaattt tcggactaaa attgttgcta 360

ctatcggtcc tgcgagtaat actcccgaag tattacgtca aatgctctta gccggagtca 420

atgttgcgcg gttgaatttt tcccacggta gctacgaaga tcacgctcag atggttaaac 480

tcttacgttc tttgtccgaa gaattagact tacccctgac cattttacaa gaccttcaag 540

gtccaaaaat tcgggtaggc aaattacccc cagacggact taacctcatc gaaggacaat 600

ctctaacctt ggttcccctt gctgcttgga aaaatcaagc caataccgtt ggcattgatt 660

atccctacgt cgctgaagaa gcgcaacccg gtactcaagt gctgcttgat gacggtttat 720

tggagttaac cgttgaacaa gtcaagggaa atgaggtcat ctgtcaagtg gttgaaggag 780

gcattctcaa aagcaataag ggggttaatt tgccaaccct caatctacgc ttgccttcca 840

tgaccgaaaa agataagaaa gatctcgaat ttggactatc ccaaggcgtt gacatcattt 900

ccctaagctt tgtccgcaaa cccgaagata ttcaagaact caaggaattt attgcccaaa 960

gatcggcaaa agttcccgtt ttagcgaaaa ttgaaaagcc ccaagccgtt gacaatattg 1020

aagccattat cgatgaatgc gatgctatta tggttgcgcg gggagactta ggggtagaaa 1080

tgcgccccga aaaggttcca ggtatccaaa aacgcatcat taagctgtgt aaccaaaaag 1140

gcatccccgt tattaccgcc acccagatgc tcgatagcat gattcgtaac ccccgtccca 1200

cccgtgctga agccagtgac gtagccaatg ctatcattga tggaaccgat gcggttatgt 1260

tatcaggaga atcagcgatc ggagattatc ccgtgcaagc ggtgcaaatg ctggctaata 1320

ttgccaaaga tattgaacca ggactgaatt ttgccaatta tcctcctcga cggcagaata 1380

aagcccacgc catagccgaa gctctcaata ccatcgacaa gattcttgat ttacaatgta 1440

ttgtcacctt tacggaaacc gggtattctg ctaaattagc tgctgctgaa cggccacggg 1500

ttcccatagt ggctttaaca cctgatcacc aagtttatca tcgccttaat ttagtttggg 1560

gagtccgacc cattttattt gactacgatg agtcttccct agacgacttg atggttaaag 1620

tagaagatat gttaaaaacc cgaaactacg cgacatcagg ggataaagtg ttgattatgg 1680

gtggtttacc cctcagaaaa gccagtacca cgagttttct cgatattcat acgattactt 1740

aataaatgga ggcgctcgtt gatctgagcc ttgccccctg acgaacggcg gtggatggaa 1800

gatactgctc tcaagtgctg aagcggtagc ttagctcccc gtttcgtgct gatcagtctt 1860

tttcaacacg taaaaagcgg aggagttttg caattttgtt ggttgtaacg atcctccgtt 1920

gattttggcc tctttctcca tgggcgggct gggcgtattt gaagcggttc tctcttctgc 1980

cgtta 1985

<210> 132
<211> 1568
<212> DNA
<213> Artificial Sequence

<220>
<223> Synthetic Construct- Sequence No. 132, Example 132: designer
      Nial-promoter-controlled NADPH-dependent chloroplast-targeted
      NADPH-dependent Glyceraldehyde-3-phosphate dehydrogenase DNA
      construct (1568 bp)

<400> 132
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtcaa cgaatattgc aattaatgga atgggtagaa 360

ttggaagaat ggtgctaaga atagcactaa agaatgaagc attgaatgta gttgccatca 420

atgctagcta tcctcctgaa acaattgcac atttaattaa ttatgacaca acacatggga 480

gatacgataa aagagtagaa cctattgaaa gtggaattcg agtggaaggc catgatatta 540

aattagtgtc tgatagaaac ccagaaaatt taccctggaa agatttagaa atagatatcg 600

tcattgaagc gaccggtaaa tttaaccatg gtgataaagc taaggcacat attcaagcag 660

gagctaaaaa agtgttattg acaggaccat caaaaggcgg aaaagtacag atggtggtta 720

aaggtgttaa cgatcaagac ttagatacag atacatatga catatttagt aatgcgtcgt 780

gtactacgaa ttgtatcgga ccagttgcaa aagttttaaa tgatagtttt ggcattgaaa 840

atggcttaat gacaacggta catgcaatta caaatgatca aaataatata gataatccgc 900

ataaagattt gagaagagcg cgttcttgtg gggaaagtat tataccaaca tcaacaggtg 960

ctgctaaagc attaaaagaa gttatgccag aattgaatgg caaactacat ggcatagcac 1020

ttcgtgtgcc aactcaaaat gtatcattag ttgatttagt cattgattta aaacaaaaag 1080

tgacagtaga tgaagttaat catgcattta gagatgcaaa cttacaagga attattgatg 1140

ttgaagaggc ccctctagtt tctaaggact ataatacaaa tcctcattca gcagttatag 1200

atgctaaaaa tacaatggtc atgggagata ataaggttaa agttatagcc tggtatgata 1260

acgaatgggg atattctaat agagtagttg aggtagcaaa tcaacttgga gaactaatta 1320

aataataaat ggaggcgctc gttgatctga gccttgcccc ctgacgaacg gcggtggatg 1380

gaagatactg ctctcaagtg ctgaagcggt agcttagctc cccgtttcgt gctgatcagt 1440

ctttttcaac acgtaaaaag cggaggagtt ttgcaatttt gttggttgta acgatcctcc 1500

gttgattttg gcctctttct ccatgggcgg gctgggcgta tttgaagcgg ttctctcttc 1560

tgccgtta 1568

<210> 133
<211> 1571
<212> DNA
<213> Artificial Sequence

<220>
<223> Synthetic Construct- Sequence No. 133, Example 133: designer
      Nial-promoter-controlled chloroplast-targeted NAD-dependent
      Glyceraldehyde-3-Phosphate Dehydrogenase DNA construct (1571 bp)

<400> 133
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtaaa ctggctgaat gtacaagcat atccgcaagt 360

ttgttggagt aacccatttc gttatcgtac catgaaacta ctttcacgaa atttggagat 420

aacataattc ccgcatcctt gtcgaatacc gaagttctct tctcgcccac gaaatcctga 480

gaaaccacag cgtcttcagt ataccctaaa attcctttaa gttcaccttc cgatgccgct 540

ttcattgctg cacagatttc ttcgtatgaa gtagatttct ctaatctgac cgttaaatcc 600

actacagaaa catctgcagt tgggactctg aatgacatac cggttaattt accgttaagg 660

gcaggaatta cttttcctac tgcttttgca gctccggtag aagatgggat gatgttgttc 720

aatgcagaac gcccgcctct ccagtctttc atagaaggcc cgtcaacagt tttctgggtt 780

gcagtagttg cgtgcacggt cgtcattaaa ccttcgatga ttccgaaatt atcgtgaagt 840

acttttgcta atggagcaag acagttagtc gtacagctcg cgttagaaaa aatagtaacg 900

tcatctgtaa gatccttgtg gttaacaccc attacgaaca tcggcgtatc gtcttttgaa 960

ggagcagaaa ggattgcttt ttttgcaccc gcgttgatat gtgcctgtgc cgcctccttg 1020

gtaaggaata aaccggttga ttccacgatg tattctgcgc ctacttcgtt ccatttcagg 1080

ttgttaggat ctttttcggc ggttacacga atctttttgc cattcacgat aaggtcgttt 1140

ccttctacag aaacttcgcc cgcaaatgtg ccgtgtaccg agtcatactt aagcatgtac 1200

gccatatatt tggcatcgat aagatcgtta attcccacca cttcgatgtt ttctctctcg 1260

gccatcgctc tgaaaaccaa gcgtccaatc ctaccgaatc cgttgattcc tactttaatt 1320

gttgacatta aatggaggcg ctcgttgatc tgagccttgc cccctgacga acggcggtgg 1380

atggaagata ctgctctcaa gtgctgaagc ggtagcttag ctccccgttt cgtgctgatc 1440

agtctttttc aacacgtaaa aagcggagga gttttgcaat tttgttggtt gtaacgatcc 1500

tccgttgatt ttggcctctt tctccatggg cgggctgggc gtatttgaag cggttctctc 1560

ttctgccgtt a 1571

<210> 134
<211> 2150
<212> DNA
<213> Artificial Sequence

<220>
<223> Synthetic Construct- Sequence No. 134, Example 134: designer
      Nial-promoter-controlled chloroplast-targeted Citramalate
      Synthase DNA construct (2150 bp)

<400> 134
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggagc aggtttttat ctacgacacc accttgaggg 360

atggctcgca ggcagaaggt ataaactttt ccgtagagga taagatgcgc atacttcaaa 420

aactggacga atttggagtg cattacatag agtgcggatg gcccggtgcg aacccaaaag 480

acactattct ctttgaaagg ctgagaaaga taaaaactca aaatgccaaa atagtagcct 540

ttggtgcaac aagaaaagct ggaaagaagg cgcacgaaga taagcaggtg gaaaaccttt 600

tgaaatcggg tgccaaggtg ataaccgtat ttggcaagag ctgggacttt catgtaacgc 660

atgccatagg gaccacctta gaggaaaacc tggacatggt ttacgagacg gtaagctatc 720

ttaaaaagca tgtggaggag gttatctttg acgcagagca cttctttgac ggatacaggc 780

acaacgaaag ctatgctttt aaggtattgg aggcagcttt tcaggcaggt gcggactgga 840

tagtcctctg cgataccaac ggtggcaccc ttcccaatga ggtttatgag ataaccaaaa 900

aggttgtaca aaagtttcca caggcacgcg taggcataca cgctcacaac gattcagata 960

ctgctgtggc taactctctt atggcggtgc ttgcaggtgc aaggcaggtt cacggcacta 1020

taaacggctt gggggaaaga acgggcaatg ctaatctgtg ttccataata cctaaccttc 1080

agctcaagct gggctttagt gtagtgcctt cccaaaacct caaaaagctc accgagcttg 1140

ctcactttgt ctccgaaatc tccaacacgc cactgcccaa aaacatgcct tatgtagggg 1200

agagtgcttt tacccacaaa gcaggcgtac acgcctctgc agttatgaaa aggtcagaaa 1260

catacgaaca catagaccct tctttggtag gaaacagaag gaaggtgaca gtgtctgacc 1320

tttctggaag gagtaatata ctttacaagc tcagggaaat ggggcttgag gtggatgata 1380

agtcccctga gcttatcaaa ctccttgaaa agataaagga acttgagaag gaaggctacc 1440

actttgaagc agctgaagct tcttttgagc ttctttgcaa gaggcatttt gggcttgtta 1500

aaaactattt tgaccttgat gcttacaggg tgctaatagc cagaaggagt acagacctat 1560

ctcctgtttc ggaagccacc gtaagactct atgtggaaga cataaaggag catacagcag 1620

ctcttggtaa cggaccagtg agcgcccttg acagagccct cagaaaagcc ttggaagagt 1680

tttatccaag ccttaaagat gttcagctca tagactacaa ggtgagaata gttaacgaat 1740

cggagggtac atctgccaaa gtgagggtgc ttatagaatc taccgatggt agaagaaagt 1800

ggggaacggt gggagtttcg gaaaacataa tagaagcctc ttggatagcc ttaactgata 1860

gcctcgtata taaactctta aaagacgaag aagagggtat aatgtgataa atggaggcgc 1920

tcgttgatct gagccttgcc ccctgacgaa cggcggtgga tggaagatac tgctctcaag 1980

tgctgaagcg gtagcttagc tccccgtttc gtgctgatca gtctttttca acacgtaaaa 2040

agcggaggag ttttgcaatt ttgttggttg taacgatcct ccgttgattt tggcctcttt 2100

ctccatgggc gggctgggcg tatttgaagc ggttctctct tctgccgtta 2150

<210> 135
<211> 3125
<212> DNA
<213> Artificial Sequence

<220>
<223> Synthetic Construct- Sequence No. 135, Example 135: designer
      Nial-promoter-controlled chloroplast-targeted
      3-Isopropylmalate/(R)-2-Methylmalate Dehydratase large/small
      subunits DNA construct (3125 bp)

<400> 135
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtacc atgtctgctg ttgctatctc gcccttgatt 360

gctgatgctg taacagtagc cgctgaagca agatatacaa aagaatcttt atgtcctgca 420

cgtcccttga agtttcgtgt acctgtactg ataagagtct caccctcacc gataacaccc 480

tgacagcttc cccagcatac agagcagtta ggattcataa caattgcacc tgcgtccatg 540

aatatatcaa ggagtccctc tttcatagcc tgaagatata ccgaacggct tgcaggaact 600

acaaggaatc ttaccttagg agcaaccttt ttccctttga tgatcgctgc gccaactctt 660

aaatcctcga ttcgtccatt gttacatgaa ccaagaaatg cttcatcaat ctttacacca 720

agtgattcct tagccggaac tacattgtca acaaaatgtg gctttgcaac aattggctgt 780

attgttgaaa ggtcaatatc ataaacctgc tcaaatactg catcatcatc tgatgtaaag 840

catgcctttg gctctctgcc atgctcctta agataatcca ttgcaacatc atcaacttcc 900

atgagtgcag tcttagcacc tgcctctaca caaaggttac agattgatat tctgtctgcc 960

attgaaaggc tgtgtaagcc ttctcctgca aattccattg ctttatagtt agcaccgtta 1020

gcgccaatct ttccaataat agagagtatt aaatctcttg catatactcc atcgttaagc 1080

tttcccttaa ggttgaatct taatgttccc ggaaccatta cccatgatgt tcctgtaacc 1140

attgcataca aataatctgt acaaccaaca cctgtaccaa atgcacctaa cgcaccatat 1200

gcacaagtat ggctgtctgc tccaaatata agctcacccg gcactacatg attttccatc 1260

ataacctgat gacacacacc ctcgccctcg tagaacttaa tatcattagc cttagcaaag 1320

tctctcatct tcttctgtga ggctgctgtc ttaggactgt ctgatggaat attgtggtct 1380

acaatccata caagcttatc cttgtcagca atatgaggat tctttaactt ctcatacata 1440

ccaatagtaa gatgtgttgt tccatcatta ctcataagtc tgtcaagagt aacagttgca 1500

atatcaccag ccttaacctg tgaaagacct gctgcccttg cgataatctt ctctgcaata 1560

gtcatgccat gctttgcctc atctgcaggt acggctgtac tctcagactc ttctttctca 1620

ccatcaagtg atgcaataag accaccctga ttaagaatag cctgcatctt ggctggaagc 1680

ttagtacatg tataagtctt tccattaaca gttataattc catcttctaa tgaaagctca 1740

cattcatccc ccgcattaac ttcgtcatgg agttctttac atacaataac aggaagtcct 1800

atattaatag cattacgata gaatattctt gcaaatgatt tggcaatcac tgccttgaca 1860

cctaatgcct taagtacgct tggtgcctgc tctcttgatg aaccacatcc aaagttgtca 1920

tctgcaacaa cgaaatctcc cggctttacg gcagaagcaa aatcagagtc taatgattca 1980

aatgtatgac tcttcatctc atcaattgtc ggaaacaaaa gatactgcga tgcaataatc 2040

tgatctgtat caacatcttt atcaaactta aatatcctac ccatatggta gggtgcgagt 2100

gaccccgcgc gacttggaag ggttcaaacg accccgccgt acgaactttt gtcggggggc 2160

gctcccggat ggtagggtgc gagtgacccc gcgcgacttg gaagggttca aacgaccccg 2220

ccgtacgaac ttttgtcggg gggcgctccc ggatggccgc cgtcattgcc aagtcctccg 2280

tctccgcggc cgtggctcgc ccggcccgct ccagcgtgcg ccccatggcc gcgctgaagc 2340

ccgccgtcaa ggctgccccc gtggctgccc cggctcaggc caaccagatg gtcaagaccg 2400

ttaagctttc tcattgcctt aacaagtccg cctgcattaa gtatatccac aagattatca 2460

ggaagtgaag caataggata tgcttttcca ttgtgtgtaa tctttgcatt tacttcaaca 2520

tcaatagtat cgccttccgt aacttcgtcg tgaaggtctg cattctctat aaggagaagt 2580

ccgttattaa tagaatttct gaagaatatt cttgcatatg atttggcaat aacacattta 2640

atacctaatg ccttaataac ctcaggtgcc tgctctcttg atgaaccaca accaaagttc 2700

tttcctgcaa caatgatgtc gcctggctta atctgacctg caagttctgg tcttaatggc 2760

gaaaatgcat atggtttcat atcttctact gtctttaatg caaggtactc tgtagggata 2820

atgatatctg tatcaatgtc atcaccaagt acccatactt taccgctaaa tttctcgttc 2880

attaaatgga ggcgctcgtt gatctgagcc ttgccccctg acgaacggcg gtggatggaa 2940

gatactgctc tcaagtgctg aagcggtagc ttagctcccc gtttcgtgct gatcagtctt 3000

tttcaacacg taaaaagcgg aggagttttg caattttgtt ggttgtaacg atcctccgtt 3060

gattttggcc tctttctcca tgggcgggct gggcgtattt gaagcggttc tctcttctgc 3120

cgtta 3125

<210> 136
<211> 2879
<212> DNA
<213> Artificial Sequence

<220>
<223> Synthetic Construct- Sequence No. 136, Example 136: designer
      Nial-promoter-controlled chloroplast-targeted 3-Isopropylmalate
      Dehydratase large/small subunits DNA construct (2879 bp)

<400> 136
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgaatg aactgcctta catcagaaac ttttccatta 360

accgccgccg ccactaccat cgctggactc atcaataaag tgcgaccggt tgatgacccc 420

tgacggcctt taaaattacg gttagaagaa gaagcactaa tttgatcgcc aactaactta 480

tcagggttca tggctaaaca catggaacag ccggcttctc gccattcaaa cccggcttgg 540

gtaaaaattt tatcgagtcc ttcggcttct gcttgttgtt taaccctttc cgatccggga 600

acgacaaatg ctttaacccc tgatgctacc tgtcttcctt gggcaaattt agccgcttct 660

cgtagatcgc tgatgcgtcc attggtacaa ctaccaataa aacagacatc gacaggagtc 720

cccataatag gcgatccggg tttgagttgc atatattcat aagcttcttg agcaataaag 780

cgatcgcctt ctggtaaact ttcaggagta ggaaccactt cagtcacgcc tattccttga 840

ccgggggtaa taccccaagt aacagtcggt tcgatctcac cggcatcaaa caccacgaca 900

tcatcatatt gggcattcgc atcactgcgg atactcttcc accattcgac ggccttatcc 960

cagtcttggc ctttgggaga aaagtctctg cctttgaggt attcaaaagt cacctcatca 1020

gggttaatat agccgcatct agcgccgcct tcgatggcca tattacaaac ggtcattcgt 1080

tcttccatcg acatggcctc aaaggtagtg ccggcatatt cataagcgta acctacgccg 1140

ccttttaccc ctaatttgcg gatgatgtgc aggacgacat ctttggcata gactccgggg 1200

ggtaaggttc cgttaacttc aattttacgg actttgagtt tagagagggc tagggtttgg 1260

gtagcgagga catctcgcac ttgggaggtt cctatgccaa aagcgatggc cccaaaagcg 1320

ccatgagtgg aggtatgaga gtcaccgcaa gcaatggtca ttccgggttg agttagtcct 1380

tgttcggggg cgatcacatg aacaataccc tgatttcctg agccgatatt ataaaaaggg 1440

atattattat ctttagcatt agtttcaata gcccgcatca tttcttcagc gaggtcgtct 1500

acaaagggac ggtgttggtt ctcggtgggt acaatatgat ccactgtggc gacggtgcga 1560

tcggggaata ataccttcag ttttctgtcc cgtagcatag caaaagcttg tggactggta 1620

acttcatgaa tgaggtgaag tcctataaat agttgggttt gtcccgatgg taagatgcgg 1680

acggtatgta agtcccaaac tttgtcaaac agtgttcctg tactcatatg gtagggtgcg 1740

agtgaccccg cgcgacttgg aagggttcaa acgaccccgc cgtacgaact tttgtcgggg 1800

ggcgctcccg gatggtaggg tgcgagtgac cccgcgcgac ttggaagggt tcaaacgacc 1860

ccgccgtacg aacttttgtc ggggggcgct cccggatggc cgccgtcatt gccaagtcct 1920

ccgtctccgc ggccgtggct cgcccggccc gctccagcgt gcgccccatg gccgcgctga 1980

agcccgccgt caaggctgcc cccgtggctg ccccggctca ggccaaccag ttatacctct 2040

acggcatttt tccaatttaa ataaggtaat tttttagcag tttcttgaat ttcagaaagg 2100

ttttgaatga gttgtccgca gctatcccaa gagccttcaa ttaacatttg tctagaccct 2160

tctcccatgc taacatcagc aacaaattcg ccacattgca ctttcatagc ggctaaatct 2220

aaactgaggg acaaggcagg attttcttgc aggagagatt gtattttttc tacggtttca 2280

ggagaagctg tcacacaggg aaccccgtta gcaatacaat tgccaaagaa aatttcagca 2340

aaactttcac cgataattgc tttaattccc caacgaataa tggcttgtgg ggcgtgttct 2400

cttgaagatc cacagccaaa attagcgtta actaccaaga gattagctcc ctgatattgc 2460

ggcagatcaa agggatgttt tccttgcatc tgttggcgat catcaataaa aacttgttct 2520

cctaagcctt caaaggtgac gcaacgtaga aaccgcgcag gaataatacg atcagtatcg 2580

atatcatcgc ccactaaagg gatacctcgt cctgaaattt gggtgacttg actcattaaa 2640

tggaggcgct cgttgatctg agccttgccc cctgacgaac ggcggtggat ggaagatact 2700

gctctcaagt gctgaagcgg tagcttagct ccccgtttcg tgctgatcag tctttttcaa 2760

cacgtaaaaa gcggaggagt tttgcaattt tgttggttgt aacgatcctc cgttgatttt 2820

ggcctctttc tccatgggcg ggctgggcgt atttgaagcg gttctctctt ctgccgtta 2879

<210> 137
<211> 1661
<212> DNA
<213> Artificial Sequence

<220>
<223> Synthetic Construct- Sequence No. 137, Example 137: designer
      Nial-promoter-controlled chloroplast-targeted 3-Isopropylmalate
      Dehydrogenase DNA construct (1661 bp)

<400> 137
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgactc ggcaacaccg cataacccta cttcctggcg 360

atggtatcgg acctgaaatt ttagccgtaa ccgtagatgt cctaaaggtg ataggcaaac 420

aattcgacct aaattttgag tttacagaag ccctcatcgg cggtgctgcc attgatgcaa 480

ccggaaaccc cttacccgaa gaaaccttaa agatttgtcg caacagtgat gcagtgcttt 540

tagccgccat cgggggttat aagtgggata atttgccccg tcatcaacgc ccagaaacgg 600

gattattagg catcagagcc ggcttaggat tatttgctaa cttacgtccg gccaccattt 660

taccgcagtt aatcgacgct tccaccctca aacgagaagt cgtcgaaggc gtggacatta 720

tggtggtgcg agaactcacc ggcggcattt attttggtca accgaaggga atttttgaga 780

cagaaagcgg cgaaaaacgg ggcgtgaata ccatggccta tacagaatca gaaatagacc 840

gcattgctca aatcggcttt gaaacagccc aaaaacgtcg aggaaagctc tgttctgtgg 900

ataaagccaa tgtcttagat gtctcccaat tatggcgcga tcgcgtaact ttaatggccg 960

aaaaataccc agatgtagaa ctgtctcatc tctatgttga caatgcggct atgcagctag 1020

tgcgtaaccc aaaacaattt gataccatcg tcaccggcaa tttatttggc gatatcctct 1080

cggatgcagc cgctatgtta accggtagta ttgggatgtt accctctgct agtttaggtt 1140

cagatggacc cggactattt gaaccggtac atggttcagc ccccgatatt gcaggacttg 1200

ataaagctaa cccgcttgct caggtactca gtgccgccat gatgttgaaa tatggcttaa 1260

atgagccaga agccgccgat caaatcgaac aagcggtttt agccgtatta gaaaaaggct 1320

atcgtacagg agacatcatg tcagaaggaa tgacgttagt gggatgtaag ggcatgggag 1380

aagttttgat taatgtctta gaatctttac aagggtgata aatggaggcg ctcgttgatc 1440

tgagccttgc cccctgacga acggcggtgg atggaagata ctgctctcaa gtgctgaagc 1500

ggtagcttag ctccccgttt cgtgctgatc agtctttttc aacacgtaaa aagcggagga 1560

gttttgcaat tttgttggtt gtaacgatcc tccgttgatt ttggcctctt tctccatggg 1620

cgggctgggc gtatttgaag cggttctctc ttctgccgtt a 1661

<210> 138
<211> 2174
<212> DNA
<213> Artificial Sequence

<220>
<223> Synthetic Construct- Sequence No. 138, Example 138: designer
      Nial-promoter-controlled chloroplast-targeted 2-Isopropylmalate
      Synthase DNA construct (2174 bp)

<400> 138
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtgat gctgtaacag ccgcttgttc tttttgggtt 360

ttttcttgtt ctagggcaac ataaagacgg ttaagagcgc taatataagc ccgtgccgaa 420

gcaacgataa tatcagtatt agcggcatga cccgagaaag ttcggttttc gtatttcaga 480

cgaatggtga cttctcccat tgcatcaatg ccttctgtga ctgacttaac cgaaaattca 540

atcaactggt taggaacatt aaccactcgg ttaatcgctt tataaaccgc atctacgggt 600

ccggttccaa tagccgcatc cattaattct tcgccgttgg gaccttttaa gatcacggtt 660

gctgttggac gggcctgatc gccacatgac acttgtacta attcgagacg gaagatttct 720

ggggcaacat gaatttcatc attgacgatg gcttctaagt cccagtcagt aatttctttg 780

cgcttgtctg ccacttcttt aaagcgtaca aaggctttat ttagctcagt ttccgtcaat 840

tcaaagccta attcttttag acgagtgccg aaagcattac gccctgagag tttcccgagg 900

acaatttgat tattggttaa cccgatagac tcagcgtcca taatttcgta ggttaattta 960

ttttttagca cgccatcctg atgaataccg gattcatgag cgaaagcatt agcgccgaca 1020

attgccttat tcggttgtac cgccatcccg gtgagactgg aaactaaacg ggatgttttg 1080

tagatttctt tggtattgat attggttagg ggttcagtgg agtctgctgg tcgtcctaag 1140

aaaggattgt aataggagcg gcgtacatga agcgccatca ctaattcttc taaggcggcg 1200

ttgccggctc gttctcctat gccgttaata gtacattcta actgtctggc tccattttta 1260

acggcttcaa ggaagttagc caccgctaac cctaaatcat tatgtccatg aaccgagata 1320

atagcattgt ctatgttagg aacattttct ttgatgccgc gaatcaattg accaaattca 1380

gagggagtta aatagcctac ggtatcggga atattaacgg tagttgctcc ggctgctatg 1440

gctctttcta atacttgata caaaaattct gggtcactac ggcctgcatc ttctggggaa 1500

aattctacat catctacaaa agacttcgca taagccacca tttctgggac gatttctagg 1560

acttcttgac gagtcttttt gagtttatag gccaagtgaa tatcggaggt agccaaaaag 1620

gtatgaatgc ggggtttagc ggcgggtttg agggcttctg cggctttagt gatatcttgt 1680

cgagtggctc ttgccaagcc gcaaattgtg gggcctcctt ggactcctac tactttagcg 1740

attttttgta cagcttcaaa atctccggga cttgcgtaag gaaaacccgc ttctatgaca 1800

tctactccca gtcgtgccag tgcgcgagca acggttagct tctcatcaac attcagggtt 1860

gctcccgggg actgttcccc atctcggaga gtggtatcga agatgataac gcgatcgggt 1920

tgtttactca ttaaatggag gcgctcgttg atctgagcct tgccccctga cgaacggcgg 1980

tggatggaag atactgctct caagtgctga agcggtagct tagctccccg tttcgtgctg 2040

atcagtcttt ttcaacacgt aaaaagcgga ggagttttgc aattttgttg gttgtaacga 2100

tcctccgttg attttggcct ctttctccat gggcgggctg ggcgtatttg aagcggttct 2160

ctcttctgcc gtta 2174

<210> 139
<211> 2882
<212> DNA
<213> Artificial Sequence

<220>
<223> Synthetic Construct- Sequence No. 139, Example 139: designer
Nial-promoter-controlled chloroplast-targeted Isopropylmalate
Isomerase large/small subunits DNA construct (2882 bp)

<400> 139
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgcaac aactcccgca catccgcaac ttcaccctga 360

atcgccgcag tagcgaccat cgccggactc attaacaaag tgcgaccgga agctgatcct 420

tgtcgtcctt taaagttgcg gttggaggag gaagcactaa tttgtctacc ttccagcttg 480

tcggggttca tggctagaca catagaacat ccaggttcgc gccattcaaa gcctgctgct 540

tcaaagattt tatctaaacc ttcagcttcg gcagcttttt tcactcgttc ggaaccggga 600

accacaaaag ccttgactcc ttccgctacg tggcgacctt tggcaatttt cgcggcttct 660

tgcaggtcac taagtctacc gttagtgcag ctaccgataa agcaaacgtc aattttcgtt 720

cccttaatcg gttgaccagg atataaatcc atgtaacggt aagcttcttc agctacaaag 780

cggtcttctt ctaggagttc ttctggctgg ggaatcaact gattcacacc aataccttga 840

ccgggggtaa ttccccaggt aacagtgggg ggaatatccg cagcgttgaa tactattaca 900

tcatcgtatt cagcatcagc atcactcttg attgattccc accaagccac ggctttttcc 960

caatcagcgc cttggggggc aaagtctcta ccttggagat aatcataggt aacttgatca 1020

ggattgacat aaccgcatct agcgccaccc tcaatggcca tgttgcagac agtcatccgt 1080

tcttccatat tcatttgctc aaaagtcgta cccgcgtatt cgtaggcgta acctacacca 1140

cctttcactc ccagggtacg gatgatatgc aggatgacat ctttggcata aaccccaggg 1200

ttgagtgtgc cgttaacttc aattttacgg actttgagtt tggataggga taaggtttgg 1260

gaggcgagaa cgtcccgcac ttggctagta ccaataccaa aagcgatcgc cccaaacgcc 1320

ccatgacttg aagtgtggct atcaccacag gcgatcgtca ttcccggctg tgtcagtccc 1380

agttctggcg caatcacatg aactatacct tgattgccgg aaccaatgtt ataaaaagta 1440

atgttatttt cttgacaatt ctgctctagg gcttggatca tttcctcagc caagcgatcg 1500

acaaaaggac gcgcctgatt ctctgtaggc acgatgtgat ccacagtagc cacagtccgc 1560

tcaggaaata gtacctttaa acctcgttcc cgtaacatag caaaggcttg tggactagta 1620

acttcatgga ctaggtgcag gccaataaat agctgtgtta gccctgaagg aagtgtacca 1680

acagtgtgta agtcccaaac tttatcaaac agggtgcctt tgctcatatg gtagggtgcg 1740

agtgaccccg cgcgacttgg aagggttcaa acgaccccgc cgtacgaact tttgtcgggg 1800

ggcgctcccg gatggtaggg tgcgagtgac cccgcgcgac ttggaagggt tcaaacgacc 1860

ccgccgtacg aacttttgtc ggggggcgct cccggatggc cgccgtcatt gccaagtcct 1920

ccgtctccgc ggccgtggct cgcccggccc gctccagcgt gcgccccatg gccgcgctga 1980

agcccgccgt caaggctgcc cccgtggctg ccccggctca ggccaaccag atgaccagca 2040

gccagtttac cccaactcac ataaggtaac ttagcagatg ttacccgcac ttgctcggtg 2100

ttcgctacca actgaccgca agcatcccaa gccccagtaa taaaggtgct tctggttcct 2160

tcaccaatgg agattggcgc ggtgaaatca ccaacttgta cttgcagagt ttctaagttg 2220

atgctgacat tagcttgagg attagcggct actaactctt gcaattgttt aacgatcgcc 2280

tcatcagcag tgacacaagg tacaccgatg gctacgcaat taccgaagaa aatttctgca 2340

aaactttcac caatcacgga ttgaatcccc catttagaaa gggcttgggg tgcgtgttcc 2400

cgtgaagaac cacagccaaa gttgcggtta actatgagga tatttgcgcc ttgatactgc 2460

ggttggtcaa aaggatgctc cccttttagg gctgtgcggt catcaataaa cgcgccttca 2520

cgtaacccat caaaggtaat ggctttgaga taacgagcag gaataatgcg atcggtatca 2580

atatcattac ccactaaggg tatgccacgc cctgtaactt ctttaacttc actgaccatt 2640

aaatggaggc gctcgttgat ctgagccttg ccccctgacg aacggcggtg gatggaagat 2700

actgctctca agtgctgaag cggtagctta gctccccgtt tcgtgctgat cagtcttttt 2760

caacacgtaa aaagcggagg agttttgcaa ttttgttggt tgtaacgatc ctccgttgat 2820

tttggcctct ttctccatgg gcgggctggg cgtatttgaa gcggttctct cttctgccgt 2880

ta 2882

<210> 140
<211> 2210
<212> DNA
<213> Artificial Sequence

<220>
<223> Synthetic Construct- Sequence No. 140, Example 140: designer
      Nial-promoter-controlled chloroplast-targeted 2-Keto Acid
      Decarboxylase DNA construct (2210 bp)

<400> 140
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtata cagtaggaga ttacctgtta gaccgattac 360

acgagttggg aattgaagaa atttttggag ttcctggtga ctataactta caatttttag 420

atcaaattat ttcacgcgaa gatatgaaat ggattggaaa tgctaatgaa ttaaatgctt 480

cttatatggc tgatggttat gctcgtacta aaaaagctgc cgcatttctc accacatttg 540

gagtcggcga attgagtgcg atcaatggac tggcaggaag ttatgccgaa aatttaccag 600

tagtagaaat tgttggttca ccaacttcaa aagtacaaaa tgacggaaaa tttgtccatc 660

atacactagc agatggtgat tttaaacact ttatgaagat gcatgaacct gttacagcag 720

cgcggacttt actgacagca gaaaatgcca catatgaaat tgaccgagta ctttctcaat 780

tactaaaaga aagaaaacca gtctatatta acttaccagt cgatgttgct gcagcaaaag 840

cagagaagcc tgcattatct ttagaaaaag aaagctctac aacaaataca actgaacaag 900

tgattttgag taagattgaa gaaagtttga aaaatgccca aaaaccagta gtgattgcag 960

gacacgaagt aattagtttt ggtttagaaa aaacggtaac tcagtttgtt tcagaaacaa 1020

aactaccgat tacgacacta aattttggta aaagtgctgt tgatgaatct ttgccctcat 1080

ttttaggaat atataacggg aaactttcag aaatcagtct taaaaatttt gtggagtccg 1140

cagactttat cctaatgctt ggagtgaagc ttacggactc ctcaacaggt gcattcacac 1200

atcatttaga tgaaaataaa atgatttcac taaacataga tgaaggaata attttcaata 1260

aagtggtaga agattttgat tttagagcag tggtttcttc tttatcagaa ttaaaaggaa 1320

tagaatatga aggacaatat attgataagc aatatgaaga atttattcca tcaagtgctc 1380

ccttatcaca agaccgtcta tggcaggcag ttgaaagttt gactcaaagc aatgaaacaa 1440

tcgttgctga acaaggaacc tcattttttg gagcttcaac aattttctta aaatcaaata 1500

gtcgttttat tggacaacct ttatggggtt ctattggata tacttttcca gcggctttag 1560

gaagccaaat tgcggataaa gagagcagac accttttatt tattggtgat ggttcacttc 1620

aacttaccgt acaagaatta ggactatcaa tcagagaaaa actcaatcca atttgtttta 1680

tcataaataa tgatggttat acagttgaaa gagaaatcca cggacctact caaagttata 1740

acgacattcc aatgtggaat tactcgaaat taccagaaac atttggagca acagaagatc 1800

gtgtagtatc aaaaattgtt agaacagaga atgaatttgt gtctgtcatg aaagaagccc 1860

aagcagatgt caatagaatg tattggatag aactagtttt ggaaaaagaa gatgcgccaa 1920

aattactgaa aaaaatgggt aaattatttg ctgagcaaaa taaatagtaa atggaggcgc 1980

tcgttgatct gagccttgcc ccctgacgaa cggcggtgga tggaagatac tgctctcaag 2040

tgctgaagcg gtagcttagc tccccgtttc gtgctgatca gtctttttca acacgtaaaa 2100

agcggaggag ttttgcaatt ttgttggttg taacgatcct ccgttgattt tggcctcttt 2160

ctccatgggc gggctgggcg tatttgaagc ggttctctct tctgccgtta 2210

<210> 141
<211> 1724
<212> DNA
<213> Artificial Sequence

<220>
<223> Synthetic Construct- Sequence No. 141, Example 141: designer
      Nial-promoter-controlled chloroplast-targeted NADH-dependent
      Alcohol Dehydrogenase DNA construct (1724 bp)

<400> 141
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgggcg gcggcttcga gtatctgacg gctgacgtca 360

agggtaatat cgccgttttc acccagatgg gtcatgccgt ggtcctccaa ctgctgcacc 420

aggtcggtaa tgccttgcgc accgatgtca taggcggaca ggcgggtcgg cacaccaagg 480

ttttcaaaga attcctccgt cttgcggata accgcattga tccgctcatc ttccgttcca 540

tcggtcaggt tccatacccg tactgcatac tgaagcagtt tggcacgctt ttcctcgcga 600

cgcaccttca gcagggcagg aaataccatg gccagcgtcc gggcgtggtc aatgtggtat 660

tttgccgtga tttcgtggcc gatcatatgc gtgctccagt catgtggcac ccctgcgccg 720

atcaggccat taagcgccaa tgtcgccacc cacatgaggt tggagcgcag gtcataatca 780

tcgggtgccg ccatgatacg tggcccgatt tccacaaggc tggagagcag cccttcggaa 840

aagcgatcct gcgccattcc gtccacagga taggtcatgt actgttccat cacatggacg 900

aaggagtcca caacaccgtt gataacctgc ttcatcggca ggctcatggt gcgtgtcggg 960

tcaagcacgg aaaagcgtgg atagaccagg ggattggaaa acaggagctt atccccagtg 1020

gactgccgtg aaatcacgct catgcagttc atttcagacc ccgtggcagg cagggtgacc 1080

accgtgccca gtggcagggc cttggtcgcg gctgtgcctt tgctggtcag gatgtcccat 1140

gcctcacctt catatgggac agcggcagcg acgaacttgg tcccatccat gacagagccc 1200

ccacccacgg caagcaggaa gtcgagccct tcttcacgca ccatggttac ggccttcatc 1260

agggtttcat aggtgggatt ggcctcgatg ccaccgaatt cccgaaaggt ccggctaccg 1320

agggcggcgc gtacctcggc aagcgtcccg ctgcgctcgg cgcttgaacc gccatacagg 1380

acgaggacac gggcctgggg tgacaactga tcatctagac ggccaatcat gcctttgccg 1440

aacaggacac gtgttgggtt atagaattcg aaattctgca ttaaatggag gcgctcgttg 1500

atctgagcct tgccccctga cgaacggcgg tggatggaag atactgctct caagtgctga 1560

agcggtagct tagctccccg tttcgtgctg atcagtcttt ttcaacacgt aaaaagcgga 1620

ggagttttgc aattttgttg gttgtaacga tcctccgttg attttggcct ctttctccat 1680

gggcgggctg ggcgtatttg aagcggttct ctcttctgcc gtta 1724

<210> 142
<211> 1676
<212> DNA
<213> Artificial Sequence

<220>
<223> Synthetic Construct- Sequence No. 142, Example 142: designer
Nial-promoter-controlled chloroplast-targeted NADPH-dependent
Alcohol Dehydrogenase DNA construct (1676 bp)

<400> 142
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgccta tatacttttt caaataattt ctttaaatct 360

tttaaattta cttctctgac atttccacta gtacaaatat cttcaagagc agatttagac 420

ataatattta tttcattaaa atatttttct tcatctattg ctaaatcttt tacacaacta 480

ggaagtccta agtttttatt taaaacttcc actgccacag ctaaactttc tgctccttct 540

tctgtattat ttgcaggaaa acctaaatct tttgaaattt cataatatct ttgagcagta 600

gttttatttt ctgaattgaa tctaattatg tagggtaata tagttccatt tatttttcca 660

tgagctatat gaaactttcc accaatagcg tgggctatac tatgatttat tcctaaacca 720

gatttttcaa aagcaaaacc tgctatacaa gatgcctttg ccatttcaat cctagcttcc 780

tcatctttta tatctctata cattctcaaa agatttttaa aaataagtct tatagctgaa 840

agagcatata tttgagtata aaaatttgct tctttgcaag tgtatgactc aatagcatga 900

gttagagcat ctatacctga atcagctaca actgattttg gtagtgtttt tgtaagttca 960

ggatctagta ttgcatattc aggtatcatc tcattatctt ttaatggaat ttttacatta 1020

ttctttttat ctgtaagaac tgcataggaa cttacttctg aacctgttcc acttgtagtt 1080

ggtaaggcta ttaaagggat agataatcca gattttttta caaaatattt aattgactta 1140

gcagtatcaa gagaagaacc tcctcctatt gctaccatca catctggaag aaaatcaata 1200

accttatcta aggctttact aactatttca aatgctggat caacttcaac ttcattaaaa 1260

atcctataat ctatattttt ttgcttaaat atattttcaa attttttagt cattcctatt 1320

tttgacataa ctgaatcagt tactataaag gcttttttag ctttaatttt attaataact 1380

tcatcaaatt tgtctcctac ataaacattt gtatttactt caaaaatttt cattaaatgg 1440

aggcgctcgt tgatctgagc cttgccccct gacgaacggc ggtggatgga agatactgct 1500

ctcaagtgct gaagcggtag cttagctccc cgtttcgtgc tgatcagtct ttttcaacac 1560

gtaaaaagcg gaggagtttt gcaattttgt tggttgtaac gatcctccgt tgattttggc 1620

ctctttctcc atgggcgggc tgggcgtatt tgaagcggtt ctctcttctg ccgtta 1676

<210> 143
<211> 3629
<212> DNA
<213> Artificial Sequence

<220>
<223> Synthetic Coonstruct- Sequence No. 143, Example 143: designer

Nial-promoter-controlled chloroplast-targeted Phosphoenolpyruvate
Carboxylase DNA construct (3629 bp)

<400> 143
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgaccg gtatttctca ttcctgccgc aattccatta 360

atggttaata gcgctcctct gagcaactct tccttagaat aacggaaatt aatgactcca 420

gactcagctt gtgcgttgta ctgacgtagg cgtttgatga gagatacttg caaaaatcct 480

agaggaacaa ttgttccatt gcgtaactga acagaacgct gtaaggtggg atcgccatct 540

aggagccgtt tattctcggt aatttctaag actaagcggc aagtgcgatg atattcttgg 600

gctatttgct caaacagccg ctcaaagcgt tctctatctt ccggttgaga caattccttt 660

acataatgat aggcaatttg caaatctacc ttagataagg tcatttccac ttttgagatg 720

accattttaa agaaggccca tttgagataa aaatagcgca acagtttcaa atgttcttca 780

ggttcggtgc taataaactg ttctaaagcg gttcctaccc cataccaagc gggtaacaga 840

aaacgggttt gagtccaact aaagacccaa ggaattgcgc gtagggtact gaggtctttt 900

ttcccacttt tacgtctagc cgggcgagaa ctaatttgga gttgactaat ttcttgaatg 960

ggggtgacag acatgaagaa atccacaaaa tccggttctt cataaatcaa ggcacgatag 1020

gctttacgag aacaactggc gagttcttcc atgatctcat tccaaacctg aatatcatca 1080

aagccgcagc ctaacaaact cgattgaata acggcagtaa caatggtttc taggttataa 1140

agggctaatt cagggagaga atatttagaa gcgaggactt ctccctgttc agtaatttta 1200

attctgccat taaccgtcgc tgtcggttga gcgagaatag cggcataagc gggtccaccg 1260

cctcgtccta ctgaaccccc gcgtccgtga aacaggcgta aatctaagcc atatctttta 1320

gcgacttttt gtaaagcttt ttgggcttta tgaatctccc aattactgct taaaaaccca 1380

gaatctttgt tactgtcgga ataaccgacc ataatttctt gtaggttagt gggttctaag 1440

agggggggtt taagttcgcc cacattttcc ccctgtaaag aggctaaatg atcatatcct 1500

cctgccaaag tggcgcggta aagagtcaat tcaaatagcg cccgcatgat ttcgggtgcc 1560

cgtttaaggt cttctacggt ttcaaatagg ggtacaatgc ggatcgtgct ggaacaagtg 1620

gccgggtcat agagtccggc ttcttgtgcc aagagaagaa cctctaagac atcgctgacc 1680

tcattagtca tactgataat gtaggtatga cagatctcta aaccaaattc ttgttgtagc 1740

tgccgcaaca tcctcaaggt ttctatcact tcgcaggttt tctcagaaaa cggcatttcc 1800

tggggaatga gaggacgacg agtttttaat tcttcgatta accaggcggt tctttcggct 1860

tcagtcagtt ggttgtaggg tttaggcaga atttgtaaat attctgctat ttcgttgatc 1920

gcatctgagt ggttactcga ctcttggcgg aaatctagtt gcgtgaggtt aaacccataa 1980

acttctacct gacaaattaa gctatctaat tcttgacaac ttaagccagt gctttctagg 2040

ttacgccgca ttaatttgag ttcttctata aactcttctt tattttggta attgttggcg 2100

ttggttgtct tgaacactaa aaggcgttct tctgggttag ctaggcggct attgcggtcg 2160

cgggtatttt ccaggcgttt tttaatataa gctaacttaa gacgataggg ttcttgacgg 2220

taacgaatgg ctaactgatt ataaacttgt gggatttgta cgcggtcttt ttctagggag 2280

tctaacaaat ccggcaggac gttacaccaa tggagggaag gactaaggat attcgataac 2340

tcatctaccc tttcgatata tttttcgata acgacattgc gttgataaca ggccgtggcc 2400

caagtcactt ctggtgtaac aaaggggttc ccatctcgat cgcctcccac ccaagagcca 2460

aaataacaga aattattctt cggtgggcgt agtctgggga aggcactttt tagagttcgt 2520

ttgagacgaa gggctaattg aggaattgcc tcaaagagga cttcattaaa atagtgtagg 2580

gagtagtcca cttcatctaa cacggtgggt ttaaactggt gtaactcatc ggtacgccac 2640

cagaggcgaa tttcttcttt aagctgttct ttagcttctt ctgcttccca ggagttggtt 2700

agccccattc ctctaaaggt ttcttctgcc tggtctaact tttgtaaaat atgagcgata 2760

cgccgttgtt tgcgtcgaat ggtgtggcga acaatttcgg tgggatgggc tgtaaaaacc 2820

aggcgcacat ctagttgttc gagtaggcgt tgaatttgtt gaggtggaac atttaattgt 2880

tttaaatagg gaaatagcca gtgaaaagtt cctatttttt gatcattttg tttttcatcg 2940

ttcaaacttc tttctaacca atttgctcca aatgccgagg agaatatact ggtttgctcg 3000

ccgttaatgc catttttggc actggactct ccttcattgt aggtggcccg tcgtgagagt 3060

tgttggtctc gttgttcgta gtgttgttca acaatattaa tgagttggaa atagagagca 3120

aaagcgcgag acgttcttac tgcttcattg aggtcgagtt tttcaatcaa ttgggtaatg 3180

gagtcttcta acgctttttg ggcttgtcct tgttcagaac aaattgcacg cagttttatg 3240

agcagatcca ccaaatcttg cccgcattct gcctttagca cggcttccca taaatcttcg 3300

actaatttta gtctagcttg taaaaataag tctgatgtgg agaagaattg taatggggtt 3360

gtggggactt gaactagcga actcattaaa tggaggcgct cgttgatctg agccttgccc 3420

cctgacgaac ggcggtggat ggaagatact gctctcaagt gctgaagcgg tagcttagct 3480

ccccgtttcg tgctgatcag tctttttcaa cacgtaaaaa gcggaggagt tttgcaattt 3540

tgttggttgt aacgatcctc cgttgatttt ggcctctttc tccatggcgg ggctgggcgt 3600

atttgaagcg gttctctctt ctgccgtta 3629

<210> 144
<211> 1745
<212> DNA
<213> Artificial Sequence

<220>
<223> Synthetic Construct- Sequence No. 144, Example 144: designer
      Nial-promoter-controlled chloroplast-targeted Aspartate
      Aminotransferase DNA construct (1745 bp)

<400> 144
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggat ggccgccgtc attgccaagt cctccgtctc cgcggccgtg gctcgcccgg     240

cccgctccag cgtgcgcccc atggccgcgc tgaagcccgc cgtcaaggct gcccccgtgg     300

ctgccccggc tcaggccaac cagatgaacc gagacaccaa gcaggctgtt ttggaagagc     360

tgtaaccgtt ccagaccctt ttcaatcgtt tggcagtcag tggcgtagga gagccggatg     420

ctgcgatcgt cgccaaaggc aatgccggga atggctgcaa cttgatgttg atccaacagt     480

tgacggcaat aggtcatcga gtcgagacct gttttgctga tgtccacgaa gacgtagaac     540

gccccttccg gtattggaca ggagagcccc gcgatttgat tcagtccgtt caagatcaac     600

tgacgccgct ccgtaaaggc agccagcatt tctgccacac aatcctgtgg accttgcaga     660

gctgcgatcg cgccgtactg ggcaaaggtg cagacgtttg aggtgctgtg gctttgcagc     720

gatgcagcag cagcaattag ctcgctcgga cccgcgaggt agccaacccg ccatcctgtc     780

atcgagtagg ctttggcgaa gccgttgctg atcagcgttc gttcaaaaca ggcggggctc     840

aagctgccaa tgctgtggtg atcggctccg tcgtaaagga tcttttcgta aatttcatca     900

gaaacgaccc aaaagtcatg ggcttcaatg atcggcgcga tcgcttccag ttcttgccgg     960

ctatagacca tccccgtggg attggagggg gaattcagca ctagcagccg tgtccgaggc    1020

gtaatcgccc ctgccaattg ctgcggctgg agtttaaagc cgtcgctggc gaaagtttca    1080

acgatgacgg gcacaccgcc cgccaacttg accatttcgg gatagctcaa ccagtaaggt    1140

gcggggataa tcacctcatc gcccggatcg agcagcacct gcatcaggtt gtagagtgac    1200

tgcttaccgc cattggtgac gagaatgttg gcggcttggt aatcgagtcc gttgtcggcg    1260

cgcaattttt gggcgatcgc ttcgcgcaga tcaggttcac cggctgcagg accgtagcga    1320

gttttgccct ctgctagcgc ttgagctgct gcattgcgaa tgtgcaaagg tgtttcaaag    1380

tcgggctccc cggcgctgaa gctgcagaca tccaagccct cagctttcat cgctttggct    1440

tgggcagcga tcgcgagagt caacgatggt gacactcgcc ccacacgctc ggatagtttc    1500

attaaatgga ggcgctcgtt gatctgagcc ttgccccctg acgaacggcg gtggatggaa     1560

gatactgctc tcaagtgctg aagcggtagc ttagctcccc gtttcgtgct gatcagtctt 1620

tttcaacacg taaaaagcgg aggagttttg caattttgtt ggttgtaacg atcctccgtt 1680

gattttggcc tctttctcca tgggcgggct gggcgtattt gaagcggttc tctcttctgc 1740

cgtta 1745

<210> 145
<211> 2366
<212> DNA
<213> Artificial Sequence

<220>
<223> Synthetic Construct- Sequence No. 145, Example 145: designer
      Nial-promoter-controlled chloroplast-targeted Aspartokinase DNA
      construct (2366 bp)

<400> 145
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggcgt taattgtcca aaaatacggt ggaacctctg 360

tcggttcagt agaacgcatt caaacagttg cccaacggat tcaaaaaaca gcccaaaatg 420

gcaatcaagt cgtggttgtt gtttcggcca tgggaaaaac caccgatact ttagtcaatt 480

tagccaaaga gattacccca aatccctgtc gtcgggaaat ggatatgtta ttgtccacgg 540

gagaacaagt atcgatcgcc ttgatgagta tggccttaca gaaattagga caagcggcca 600

tctccttaac tggggcacaa gtggggatcg tcaccgaagc agaacacagt cgagcccgca 660

tcctttccat taaaccccat cgcattcaac gccatctcga tcgcggtgaa gttgtcgtgg 720

tcgccgggtt tcaaggcatt actaacgcag atgacttaga aattaccacc ctagggcgag 780

ggggctcaga tacctccgcc gtcgccattg cggcagcctt aaaagccagt tgctgcgaaa 840

tctatacaga tgtccccggc attctcacca ccgatccccg catcgtcccc gatgcccaat 900

taatggggga aattacctgc gatgagatgc tagagttggc cagtttaggg gctaaggttc 960

ttcatccgag ggcggtggaa attgcgcgta attatggcat tcctttagtg gtgcgatcga 1020

gttggagtga tgcccccgga acccgcgtga cttctcccat tcctaaaccg cgatcgctag 1080

aaggcttaga actgacaaaa gccgttgatg gggtgcaatt tgaccccgat caagccaaaa 1140

tcgccttgtt acgagtcccc gatcgccccg gagtcgctgc ccgcctattt ggggaaattg 1200

cccaccagca ggtggatgta gacttaatta ttcaatcgat ccacgaaggg aatagtaacg 1260

atatcgcctt tacggtggtt aaaaatgtac tcactaaggc cgaagccgtc gctgaagcga 1320

tcgccccggc tttacggagt cattcagcga atagcgatga agcagaggta ttagtcgaga 1380

cgggagtggc gaaaattgcc atttcagggg caggaatgat cggacggcca ggtattgccg 1440

cgaaaatgtt caaaattctc gcccaagagg ggattaatat cgaaatgatc tccacctcgg 1500

aagtgaaggt cagttgtgtg attcgtcaag aagagggcga tcgcgccatt aaagccctat 1560

gccaagggtt tgaggtggaa ttgtccccga cggggattcc tgagtcagta gtagcggtgt 1620

tacctccagt tcgaggagtc gctttagatg aaaaacaagc acaaatcgcc ctaattcatg 1680

ttcaagatcg gccggggatg gctgctagta tctttggagt cttagcggat cataacatca 1740

gtattgatac gattattcaa tcccaacgct gtcgaattgt tgagggaata cccacccgtg 1800

atatcgcctt taccgttgcc caaattgatg tagaagctgc tcaaaatgcg ttaaaaaccc 1860

tagccagtgc gtttagtgaa atgatcgtcg atagcgatgt tgctaaagtc agtattgtag 1920

gggcgggaat ggcgggacaa cccggggtag cggccaagtt ttttgatgct ttagctagac 1980

atcaaattaa tattaaaatg attgcaactt cagaaataaa aattagttgt gttgttagca 2040

aagatcaagg aattaaagct ttaaaagcag ttcatgaagc ctttcaatta gccggagaag 2100

aacgggtaga agttccagct taataaatgg aggcgctcgt tgatctgagc cttgccccct 2160

gacgaacggc ggtggatgga agatactgct ctcaagtgct gaagcggtag cttagctccc 2220

cgtttcgtgc tgatcagtct ttttcaacac gtaaaaagcg gaggagtttt gcaattttgt 2280

tggttgtaac gatcctccgt tgattttggc ctctttctcc atgggcgggc tgggcgtatt 2340

tgaagcggtt ctctcttctg ccgtta 2366

<210> 146
<211> 1604
<212> DNA
<213> Artificial Sequence

<220>
<223> Synthetic Construct- Sequence No. 146, Example 146: designer
      Nial-promoter-controlled chloroplast-targeted
      Aspartate-Semialdehyde Dehydrogenase DNA construct (1604 bp)

<400> 146
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtcag attcatacag agtagcaata ttaggagcaa 360

ctggtgcagt aggtacggaa ttactagaat tactggaaac tcggaacttt ccagtaggag 420

aattaaaact cttagcttca gaattttcag cgggtaagac cttaaaattt aaggaacaaa 480

gtttacaagt agaagctgta acaaatgatt catttaataa agtagattta gtgctagcat 540

cagcaggtgc atctgtatca aaagtatggg caaagaaagc agtagaggct ggagctgtag 600

ttattgacaa ttctagtgct tttcgtatgg actcccaagt acctcttgta gtcccagaag 660

ttaacccaga agcagcggct ttacataaag gtatagttgc taaccctaac tgcacaacaa 720

tattaatgag tgtagcagtg tggccattgc acaaaatcca gccagtccaa aggttagtag 780

tggccactta tcaatcagca agtggggccg ggtcaagggc tatggcagaa atgaaaattc 840

aggcccaaga aatcttagat ggaaaaactc caacaacaga tatttttccc tacccattag 900

catttaattt gttccctcat aattctcaac tcaatgagca gggatattgt caagaagaaa 960

tgaaaatgct tgatgaaacc agaaaaatat ttggctctaa ggaactgaga attacagcaa 1020

cttgtattcg agtaccagta ttaagagctc attcagaagc aattaatttg gaatttgctg 1080

aaccatttag tgtagttaaa gcacgggaag tattaagtca agcaccagga gtgacactgg 1140

tagaaaattg gcaagaaaat tattttccta tgcctatggt tgcaagtggt aaagatgatg 1200

tattggtggg gagaattcgt caggatattt ctcaagctga ggggttagaa ttatggttaa 1260

gtggagacca ggtaagaaaa ggagctgcct tgaatgcagt acaaatagct gaattattgg 1320

tggcaaaaaa ttggctgaga ataccagtag gaacatttta ataaatggag gcgctcgttg 1380

atctgagcct tgccccctga cgaacggcgg tggatggaag atactgctct caagtgctga 1440

agcggtagct tagctccccg tttcgtgctg atcagtcttt ttcaacacgt aaaaagcgga 1500

ggagttttgc aattttgttg gttgtaacga tcctccgttg attttggcct ctttctccat 1560

gggcgggctg ggcgtatttg aagcggttct ctcttctgcc gtta 1604

<210> 147
<211> 1868
<212> DNA
<213> Artificial Sequence

<220>
<223> Synthetic Construct- Sequence No. 147, Example 147: designer
 Nial-promoter-controlled chloroplast-targeted Homoserine
 Dehydrogenase DNA construct (1868 bp)

<400> 147
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgaagc actcgcagga cgctaggaat gctattaatc 360

gcttgagatt gctcaatttc agctaaggct tgccgaaaat tgccttctcg tacatcatga 420

gtgaccacga cgatttctgc taactgtccc tgaaatccaa tttgaacgac ggattctaga 480

ctaacatggt gctgaccaaa acaagttcct aaatgcccaa tcaccccagg gacatcttca 540

cagagaaagc gggcataaaa gcgagttttt aaatcttcaa ttggggtcag actacaataa 600

tgttgatggg tgacgttcaa taaaggatct aaagattgtg cttgtcctcc actgctttgc 660

agaatgccga cgatattcat aatatctgaa acgactgcac tggcggttgg acctgccccc 720

gcaccgggtc caaaaaacat cacttgtcct aacggttctc ccttgaccaa aatggcgtta 780

taaaccccat taatactggc tagtggatga tctttggcta ttaaagtggg atgtactcgc 840

acttgtaagg tttctgaatc atctccttta gaaccttggg caatggctaa taatttaatc 900

acaaatccga gtttatcagc ataagtaata tcggcggcac tgacttgacg aatgccctca 960

caataaatct cttcgcgttt tacccgtccg gcaaaaccga tggaggccaa aatagcaatt 1020

ttatcggctg catctaatcc gtctacatct gccgtcggat cggcttcagc atagcctaat 1080

ttttgggctt ctgctaatac ctcgccaaaa tcggctccct cagaggtcat ttggctgagg 1140

atataattgg tcgttccgtt aataatgcca ataatattac taatccgatt ggcccctaat 1200

gattgtttga ggggtttaat cactggaatt ccccccccca cagccgcttc taataacaca 1260

taaacgccgg ctgcattggc cgcttcataa atttcatccc cataacgagc gatcactgcc 1320

ttattggccg tgacaatgtg ctttttatgg gcaatggcct tcatgatgag tgacttggct 1380

ggttctagtc ctccgagcag ttctacgaca atatcaatct ctggatcaat gacaatactt 1440

tctagatctg ttgtaatcac ggcgggaggg agttgaactt gacggggttt gtcaagagag 1500

cgcactcctg cccgtttaat ctcgatatct tttaagatag gattacgtcc ccagggatcg 1560

agtagaattt gtgctgtccc cgttcccaca gttcccaagc ctaataaacc tattttaaat 1620

gccactaaat ggaggcgctc gttgatctga gccttgcccc ctgacgaacg gcggtggatg 1680

gaagatactg ctctcaagtg ctgaagcggt agcttagctc cccgtttcgt gctgatcagt 1740

ctttttcaac acgtaaaaag cggaggagtt ttgcaatttt gttggttgta acgatcctcc 1800

gttgattttg gcctctttct ccatgggcgg gctgggcgta tttgaagcgg ttctctcttc 1860

tgccgtta 1868

<210> 148
<211> 1472
<212> DNA
<213> Artificial Sequence

<220>
<223> Synthetic Construct- Sequence No. 148, Example 148: designer
 Nial-promoter-controlled chloroplast-targeted Homoserine Kinase

DNA construct (1472 bp)

<400> 148
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgatta acaattttgg ccccttccat atctaaagag 360

agtgcttgca cttgcgccgc taccccttca ttttcccacg ccaccctcat cgcttcagtc 420

accctatccg cttcaccctg agaacttaaa gcgagtaaag tgggacctgc cccactgatt 480

accataccat aagccccggc tgcgatagcc gctcgtttca ccgcttcgta acctttaatt 540

aaaccttgac gatagggttg atgcagctta tcctccatcg ccatagctaa ccagtctcct 600

cgcccctgtt cgagtccttg taacaataac cctaaacgtg caatattaaa aatagcatca 660

gcgcggctat actgagtggg taaaacgcct ctcgcttctt gagtagataa ttcaaaatcc 720

ggaatagcca caatgggaat cagtttgtca taccagggaa tctcgcaaat ttgccaattc 780

cccatttccc ccacacataa gcgactgctt cccaataaag cgggaaccac attatcggga 840

tgtccttcta aagatatggc taattccatc acttcagatt gagttaaagg gttaccggct 900

agataatttg cccccactaa accccctaca atggctgtgg ctgaacttcc taaccctctg 960

gctaaaggaa cccctaattt gatctcaatt tctacagcag gaaccggttg attgagatgt 1020

tgatagaaaa gcgcaaaaga ttgataaagt aaattagttt tatctcgact aacccgttct 1080

gcttctgcgc cgcttacgag aattttctct tcggtttctg aagtgagagt aaacttaaat 1140

tgattataaa gggttaaagc ggctcctaga caatcaaagc ctggaccaag attagcggta 1200

gtagcaggaa cggttagggt aacggtcatt aaatggaggc gctcgttgat ctgagccttg 1260

ccccctgacg aacggcggtg gatggaagat actgctctca agtgctgaag cggtagctta 1320

gctccccgtt tcgtgctgat cagtcttttt caacacgtaa aaagcggagg agttttgcaa 1380

ttttgttggt tgtaacgatc ctccgttgat tttggcctct ttctccatgg gcgggctggg 1440

cgtatttgaa gcggttctct cttctgccgt ta 1472

<210> 149
<211> 1655
<212> DNA
<213> Artificial Sequence

<220>
<223> Synthetic Construct- Sequence No. 149, Example 149: designer
Nial-promoter-controlled chloroplast-targeted Threonine Synthase
DNA construct (1655 bp)

<400>  149
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggat ggccgccgtc attgccaagt cctccgtctc cgcggccgtg gctcgcccgg     240

cccgctccag cgtgcgcccc atggccgcgc tgaagcccgc cgtcaaggct gcccccgtgg     300

ctgccccggc tcaggccaac caggtgaccc taacttggcc tatttctaaa tctgcgtcct     360

ggtcaggtct gatcaacgcc taccgttcct atttacctgt cacggaagcc actccgattg     420

tcaccctgca tgaagggaac acgcccctga ttccggtgcc cagcattgcc gctgaaattg     480

gccggcaagt tcaggtctat gtcaagtacg acggtttgaa ccccacgggc agctttaaag     540

atcggggtat gaccatggcg atctccaagg ctaaggaagc cggagccaag gcggtgatct     600

gtgccagtac cggcaatacc tctgcggcgg cagcggccta tggacggcgg ggcggcatgc     660

gggtctttgt cctcatcccc gatggttatg tcgctctggg aaaattagcc caagccctgg     720

tctatggcgc agaggtgctg gccattcagg gcaactttga tcaggcattg accctggtgc     780

agcaattagc cgaaacccag cccgtcaccc tggtgaattc cgtcaacccc taccggctgg     840

aaggtcagaa aactgctgcc tttgaagtgg tggatgccct gggtaatgcc cccgactggc     900

tctgtattcc cgtgggcaat ggcggcaata tcaccgctta ctggatggga ttctgtcagt     960

atcgggaaca ggatcgttgc gatcgtctac cccggatgat gggttttcaa gcagccggct    1020

ctgctcccct tgtccatggc caggtggtga cccatcctga aactgtagcg accgccattc    1080

ggattggtaa cccggccaac tggcagcggg cgatggccgt gcgggatgcc agccagggag    1140

aattcaatgc tgtcagcgat gccgaaattc tcgctgccta ccgtctgctg gccagtcagg    1200

aagggatctt ttgtgaaccc gccagtgccg cgtccgtcgc cggtctatta aaggtgaaag    1260

atcaggttcc gacgggggca acggtggtct gtgtcctgac ggggaatgga ttgaaagatc    1320

ctgatagcgc aattaagcag caaagtaacc agttccatca gggcatccca gctcagctcg    1380

aagccgtggc agccgtgatg ggcttccgtt agtaaatgga ggcgctcgtt gatctgagcc    1440

ttgccccctg acgaacggcg gtggatggaa gatactgctc tcaagtgctg aagcggtagc    1500

ttagctcccc gtttcgtgct gatcagtctt tttcaacacg taaaaagcgg aggagttttg    1560

caattttgtt ggttgtaacg atcctccgtt gattttggcc tctttctcca tgggcgggct    1620

gggcgtattt gaagcggttc tctcttctgc cgtta                                1655

<210>  150
<211>  2078
<212>  DNA
<213>  Artificial Sequence

<220>
<223>  Synthetic Construct- Sequence No. 150, Example 150: designer
       Nial-promoter-controlled chloroplast-targeted Threonine
       Ammonia-Lyase DNA construct (2078 bp)

<400>  150
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggat ggccgccgtc attgccaagt cctccgtctc cgcggccgtg gctcgcccgg     240

cccgctccag cgtgcgcccc atggccgcgc tgaagcccgc cgtcaaggct gcccccgtgg     300

ctgccccggc tcaggccaac cagatgtaag aagagtcgat aggctggatt gtcactttcg     360

ttccagtacc gatagcccag ggttttgaga aatgattccc actgagccat ctcttgtgga     420

gggacttgca tcccgacgac gattctgccg tagtctgcgc cgtgattgcg gtagtggaat     480

aggctgatgt tccaatctgg gctcattttc gtgacaaagt tagctaatgc gcccggtcgc     540

tcaggaaatt caaaccgata gagcagttcg ttttgagcta gcggtgactt gccgcctacc     600

atgtgccgca aatgcatctt ggctagctca tcatcagtta gatctagcgt cttaaatcca     660

ttgtctctaa acttgtgggc gatcgcctgc gcatcggctc tattttctat ctgaatgccc     720

acaaaaatat gagcttgatc ggcatctgca atccgatagt tgaattcgct aaggctgtgg     780

ctaccgagga tatcgcaaaa tttgctgaga ctgcctgccc gctctggaat cgtaacggca     840

aagacggctt ctcttccttc tcctagctcg gctcgctcgg ctacaaagcg cagacgatca     900

aagttcatat tggccccaca agcgatcgcc accaacgttt ctccctgaat ttcttctcgc     960

tctacatagg ctttgatacc tgcgatcgcg agcgccccag cgggttccaa aattgagcga    1020

gtatcttcaa atacgtcctt gatcgcggcg caggtatcat ctgtagttac cagcaacaca    1080

tcatcgacat actgttggca cagacgaaac gtttcctccc cgacctggcg caccgctacg    1140

ccatctgcaa acaggcctac ttgatccaac ttgacacgat tcccttttgc taatgattga    1200

cgcatggcat cagcatccac cggctccaca ccaatgatct taacttcagg acgtaagcgt    1260

ttgatataag cggcaatgcc cgaaattaaa ccaccccccc caatcgcaac aaaaatggca    1320

tgaatcggct tctgatgctg tcgcaagatc tccatgccga tcgttccctg cccggcaatg    1380

acgtctggat cgtcaaacgg atgcacaaag gtaagtcctt tttccacttc tagctggcgt    1440

gcgtgtgcat aggcatcgtc gtaggtgtca ccgtgtaaga caacgagtcc gcctctggcc    1500

ttcaccgcat caatcttgac ttgcggcgtc gtgatcggca taacaattac cgctgacgta    1560

cccaattctc tagcgcctag cgccaccccc tgtgcgtggt tgcccgcaga agctgcaatc    1620

accccacgct gcaacagttc tgaaggtagc tgcgccattt tgttataggc accgcgtagt    1680

ttgaaggaga aaacagactg tacatcttcc cgttttagca gcacctgatt acctagccgc 1740

tccgatagcc tcggcgcgat atctaaaggc gtttcttgcg ccacgtcata aacgcgggcc 1800

ttcaaaatgc gttctaaata gtcagaataa accactaaat ggaggcgctc gttgatctga 1860

gccttgcccc ctgacgaacg gcggtggatg gaagatactg ctctcaagtg ctgaagcggt 1920

agcttagctc cccgtttcgt gctgatcagt ctttttcaac acgtaaaaag cggaggagtt 1980

ttgcaatttt gttggttgta acgatcctcc gttgattttg gcctctttct ccatgggcgg 2040

gctgggcgta tttgaagcgg ttctctcttc tgccgtta 2078

<210> 151
<211> 2282
<212> DNA
<213> Artificial Sequence

<220>
<223> Synthetic Construct- Sequence No. 151, Example 151: designer
      Nial-promoter-controlled chloroplast-targeted Acetolactate
      Synthase DNA construct (2282 bp)

<400> 151
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgttga caaaagcaac aaaagaacaa aaatcccttg 360

tgaaaaacag aggggcggag cttgttgttg attgcttagt ggagcaaggt gtcacacatg 420

tatttggcat tccaggtgca aaaattgatg cggtatttga cgctttacaa gataaaggac 480

ctgaaattat cgttgcccgg cacgaacaaa acgcagcatt catggcccaa gcagtcggcc 540

gtttaactgg aaaaccggga gtcgtgttag tcacatcagg accgggtgcc tctaacttgg 600

caacaggcct gctgacagcg aacactgaag gagaccctgt cgttgcgctt gctggaaacg 660

tgatccgtgc agatcgttta aaacggacac atcaatcttt ggataatgcg gcgctattcc 720

agccgattac aaaatacagt gtagaagttc aagatgtaaa aaatataccg gaagctgtta 780

caaatgcatt taggatagcg tcagcagggc aggctggggc cgcttttgtg agctttccgc 840

aagatgttgt gaatgaagtc acaaatacga aaaacgtgcg tgctgttgca gcgccaaaac 900

tcggtcctgc agcagatgat gcaatcagtg cggccatagc aaaaatccaa acagcaaaac 960

ttcctgtcgt tttggtcggc atgaaaggcg gaagaccgga agcaattaaa gcggttcgca 1020

agcttttgaa aaaggttcag cttccatttg ttgaaacata tcaagctgcc ggtacccttt 1080

ctagagattt agaggatcaa tattttggcc gtatcggttt gttccgcaac cagcctggcg 1140

atttactgct agagcaggca gatgttgttc tgacgatcgg ctatgacccg attgaatatg 1200

atccgaaatt ctggaatatc aatggagacc ggacaattat ccatttagac gagattatcg 1260

ctgacattga tcatgcttac cagcctgatc ttgaattgat cggtgacatt ccgtccacga 1320

tcaatcatat cgaacacgat gctgtgaaag tggaatttgc agagcgtgag cagaaaatcc 1380

tttctgattt aaaacaatat atgcatgaag gtgagcaggt gcctgcagat tggaaatcag 1440

acagagcgca ccctcttgaa atcgttaaag agttgcgtaa tgcagtcgat gatcatgtta 1500

cagtaacttg cgatatcggt tcgcacgcca tttggatgtc acgttatttc cgcagctacg 1560

agccgttaac attaatgatc agtaacggta tgcaaacact cggcgttgcg cttccttggg 1620

caatcggcgc ttcattggtg aaaccgggag aaaaagtggt ttctgtctct ggtgacggcg 1680

gtttcttatt ctcagcaatg gaattagaga cagcagttcg actaaaagca ccaattgtac 1740

acattgtatg gaacgacagc acatatgaca tggttgcatt ccagcaattg aaaaaatata 1800

accgtacatc tgcggtcgat ttcggaaata tcgatatcgt gaaatatgcg gaaagcttcg 1860

gagcaactgg cttgcgcgta gaatcaccag accagctggc agatgttctg cgtcaaggca 1920

tgaacgctga aggtcctgtc atcatcgatg tcccggttga ctacagtgat aacattaatt 1980

tagcaagtga caagcttccg aaagaattcg gggaactcat gaaaacgaaa gctctctagt 2040

aaatggaggc gctcgttgat ctgagccttg ccccctgacg aacggcggtg gatggaagat 2100

actgctctca agtgctgaag cggtagctta gctccccgtt tcgtgctgat cagtcttttt 2160

caacacgtaa aaagcggagg agttttgcaa ttttgttggt tgtaacgatc ctccgttgat 2220

tttggcctct ttctccatgg gcgggctggg cgtatttgaa gcggttctct cttctgccgt 2280

ta 2282

<210> 152
<211> 1562
<212> DNA
<213> Artificial Sequence

<220>
<223> Synthetic Construct- Sequence No. 152, Example 152: designer
 Nial-promoter-controlled chloroplast-targeted Ketol-Acid
 Reductoisomerase DNA construct (1562 bp)

<400> 152
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgagcc ttttttagcc aactaaacat agctcgtaaa 360

tctttaccga cttcttcaat aggatgttca gcttcttgac gacgcatggc gataaatccg 420

ggttttcctg actggttttc taagacaaat tcccgagcaa attgaccgga ttgaatttct 480

ttaaggattt tccgcatttc ttgccgggtt tgatccgtaa caatacgagg acctctagta 540

tagtctccat attctgccgt attggaaatg ctatcgcgca tcttagctaa tccgccttct 600

accaccagat caacgattag tttaacttcg tggagacatt caaaataagc caattcaggc 660

tgatatccgg cttcgactaa ggtttcaaat ccggctttaa ttaaagcact tagaccgcca 720

cagaggacta cttgttcacc aaataaatca gtttctgttt cttctcggaa agtcgtttct 780

aaaacaccgg cacgagttcc tccaatccct ttagcataag ccatagcgcg atcgcgggct 840

tgtcctgaag catcttgaaa gactgcaaat aaacagggga ctccttcgcc ttgggtgtag 900

gtacgtctga cgagatgtcc tggacctttt ggtgccacca taaccacatc taccgtagaa 960

ggaggaatta cttgtccaaa atgaatatta aatccatgag caaacaaaag aactttgcct 1020

tcttttaaat ggggttcaat ttcattttta tagacgcttt tttgtacctc atctggcagc 1080

aaaatcataa tccagtcggc ggcggcggcg gcatcggcta cacttttaac cgttaagccg 1140

gcttcagtgg ctttttgggc tgacttactc ccaggataca gccccacaat aacattaact 1200

ccgctatctt taagattaag ggcatgggca tggccttgag aaccatagcc gataatggca 1260

accgttttat tagcaagtaa gtctaaattg gcatcttcat cgtaatacat tcgagccatt 1320

aaatggaggc gctcgttgat ctgagccttg ccccctgacg aacggcggtg gatggaagat 1380

actgctctca agtgctgaag cggtagctta gctccccgtt tcgtgctgat cagtcttttt 1440

caacacgtaa aaagcggagg agttttgcaa ttttgttggt tgtaacgatc ctccgttgat 1500

tttggcctct ttctccatgg gcgggctggg cgtatttgaa gcggttctct cttctgccgt 1560

ta 1562

<210> 153
<211> 2252
<212> DNA
<213> Artificial Sequence

<220>
<223> Synthetic Construct- Sequence No. 153, Example 153: designer
      Nial-promoter-controlled chloroplast-targeted Dihydroxy-Acid
      Dehydratase DNA construct (2252 bp)

<400> 153
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtcag ataattacag aagtcgcatc attacacaag 360

gcagtcaacg tacaccaaac cgggccatgc ttcgggcggt aggttttgga gataatgact 420

ttactaagcc cattgtggga gtggctaacg gatacagcac cattacccct tgtaatatgg 480

gcatcaatga tctcgcgctg cgggccgaag ccggactcaa gcaagcggga gccatgccgc 540

aaattttcgg caccattacc gtgagtgacg gaatttctat gggaacagaa ggaatgaaat 600

actccctcgt ctcgcgcgat gtgatcgctg actctatcga aaccgcttgt aacggtcaaa 660

gtatggatgg tgtgcttgcc attggcggct gtgataaaaa tatgcccggg gccatgatcg 720

ccatcgctcg tatgaatatc cctgctatct tcgtctatgg cggtaccatt aaacccggaa 780

aatacaacgg acaagattta accgttgtca gtgcctttga agccgtagga caacatagtg 840

ccggtaaaat agatgatgct caattattag gaatagaacg gaatgcttgc cccggggcgg 900

gttcctgtgg gggaatgttt accgctaaca ccatgtcctc cgcttttgaa gtaatgggga 960

tgagcttacc ttattcttcc actatggcag cagaagatgc agaaaaagcc gacagtaccg 1020

aaaaatccgc ttttgtgctg gtagatgcca tcagaaagca aattttgccc agtcagattt 1080

taacccgtaa agcctttgag aatgcgattt ccgtgattat ggccgttggg ggatcgacca 1140

acgcggtttt acatttatta gcgatcgctc ataccatagg ggtagaactg agcatcgatg 1200

actttgaagc cattagagct agagttcccg tactttgtga cctcaaaccg agtggacgct 1260

atgtcatcgt tgatttacat caggcggggg gcattcccca agtgatgaaa atgcttctcg 1320

tccatgactt attacacggg gatgctttaa ccatcaccgg tcaaacggtt gcagaagttt 1380

taaaagacgt acccgatgaa ccccctcaag gacaagatgt cattcgtcct tggaataacc 1440

cagtgtataa agaaggacac ctagcgatct taaaaggaaa tttagccacc gagggagcag 1500

tcgctaaaat tagcggggtc aaaaatccta aaattaccgg tccggcgcga gtatttgaat 1560

ccgaggaaag ctgtctagag gcgattcttg caggtaaaat tcaagctggc gatgtgatta 1620

ttgttcgtta tgaagggccc aaaggtggcc ccggtatgag agaaatgtta gccccgactt 1680

cagccattat tggggcagga ttgggagatt ccgtaggatt aattactgat gggcgttttt 1740

ctgggggaac ttacgggcta gtcgtcggtc atgttgcccc agaagcagca gtaggcggaa 1800

atattgccct cgtacaagag ggagatagca ttaccattga tgcgaaagag cgattattac 1860

agcttaatgt agctgaagat gaattaatcc gtcgtcgcgc taactggcaa ccgcccatcc 1920

ctcgttatac caaaggtgta ttagcgaaat atgccaaatt agtctcttct agtagtatag 1980

gagcggttac cgacaaagat ttattctaat aaatggaggc gctcgttgat ctgagccttg 2040

ccccctgacg aacggcggtg gatggaagat actgctctca agtgctgaag cggtagctta 2100

gctccccgtt tcgtgctgat cagtcttttt caacacgtaa aaagcggagg agttttgcaa 2160

ttttgttggt tgtaacgatc ctccgttgat tttggcctct ttctccatgg gcgggctggg 2220

cgtatttgaa gcggttctct cttctgccgt ta 2252

<210> 154
<211> 1496
<212> DNA
<213> Artificial Sequence

<220>
<223> Synthetic Construct- Sequence No. 154, Example 154: designer
      Nial-promoter-controlled chloroplast-targeted
      2-Methylbutyraldehyde Reductase DNA construct (1496 bp)

<400> 154
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggcag ttccatccaa ttctactaaa actttcaagc 360

tgaacaacgg cctgtccatt ccagcagtcg gtctcggaac atggcaatcc accgatgaag 420

aggcttacaa tgccgttatt gctgcattga aagctggata cagacacatc gacacagctt 480

actgttacgg aaatgaagag ccaattggca aagctattaa ggactctgga gttgcaagaa 540

aggacatttt tattactacc aaactttggg gaactgacca caccagaact gaggaaggtt 600

tggataggtc tctgaaattg ttgggtttgg actatgttga tctgttcttg atgcattggc 660

cagttccaat gaaccctaat ggaaaccacg acaaatttcc tacattacca gacggtaaac 720

gtgacattct gtttgactgg aacttcgttg atacatacag agagatgcaa aaattagttg 780

cctctggaaa gaccaaggca atcggtgtgt ccaatttttc tatcactaac ttgaagaaat 840

tgcttgcaga cccagaaatc accatcaagc cagttgtcaa ccaagttgaa attcacgggt 900

atctgccgca gcagagactt ttggagtatg cgaaggaaaa tgatattgtt ttggaggcat 960

attcaccgtt gggatccact ggtgccccat tgctgaaaga tgagctggtg caggacctag 1020

ccaagaagaa tggtatttct gaatctactc tcttaatttc ctgggcagtg tggagaggta 1080

tcgtcgtttt accaaaatct gtaacgcctt ccagaattgc tgataatctt aagatcattg 1140

agttgtgtga ggaggatgga aaaaaactta atgaattggc ctcgattaga ggagaaaaac 1200

gattagttag ccctccttgg gatcctattg tcgtcttcaa cgatgaagac taataaatgg 1260

aggcgctcgt tgatctgagc cttgccccct gacgaacggc ggtggatgga agatactgct 1320

ctcaagtgct gaagcggtag cttagctccc cgtttcgtgc tgatcagtct ttttcaacac 1380

gtaaaaagcg gaggagtttt gcaattttgt tggttgtaac gatcctccgt tgattttggc 1440

ctctttctcc atgggcgggc tgggcgtatt tgaagcggtt ctctcttctg ccgtta 1496

<210> 155
<211> 1595
<212> DNA
<213> Artificial Sequence

<220>
<223> Synthetic Construct- Sequence No. 155, Example 155: designer
      Nial-promoter-controlled chloroplast-targeted 3-Methylbutanal
      Reductase DNA construct (1595 bp)

<400> 155
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgtcag ttttcgtttc aggtgctaac gggttcattg 360

cccaacacat tgtcgatctc ctgttgaagg aagactataa ggtcatcggt tctgccagaa 420

gtcaagaaaa ggccgagaat ttaacggagg cctttggtaa caacccaaaa ttctccatgg 480

aagttgtccc agacatatct aagctggacg catttgacca tgttttccaa aagcacggca 540

aggatatcaa gatagttcta catacggcct ctccattctg ctttgatatc actgacagtg 600

aacgcgattt attaattcct gctgtgaacg gtgttaaggg aattctccac tcaattaaaa 660

aatacgccgc tgattctgta gaacgtgtag ttctcacctc ttcttatgca gctgtgttcg 720

atatggcaaa agaaaacgat aagtctttaa catttaacga agaatcctgg aacccagcta 780

cctgggagag ttgccaaagt gacccagtta acgcctactg tggttctaag aagtttgctg 840

aaaaagcagc ttgggaattt ctagaggaga atagagactc tgtaaaattc gaattaactg 900

ccgttaaccc agtttacgtt tttggtccgc aaatgtttga caaagatgtg aaaaaacact 960

tgaacacatc ttgcgaactc gtcaacagct tgatgcattt atcaccagag gacaagatac 1020

cggaactatt tggtggatac attgatgttc gtgatgttgc aaaggctcat ttagttgcct 1080

tccaaaagag ggaaacaatt ggtcaaagac taatcgtatc ggaggccaga tttactatgc 1140

aggatgttct cgatatcctt aacgaagact tccctgttct aaaaggcaat attccagtgg 1200

ggaaaccagg ttctggtgct acccataaca cccttggtgc tactcttgat aataaaaaga 1260

gtaagaaatt gttaggtttc aagttcagga acttgaaaga gaccattgac gacactgcct 1320

cccaaatttt aaaatttgag ggcagaatat aataaatgga ggcgctcgtt gatctgagcc 1380

ttgccccctg acgaacggcg gtggatggaa gatactgctc tcaagtgctg aagcggtagc 1440

ttagctcccc gtttcgtgct gatcagtctt tttcaacacg taaaaagcgg aggagttttg 1500

caattttgtt ggttgtaacg atcctccgtt gattttggcc tctttctcca tgggcgggct 1560

gggcgtattt gaagcggttc tctcttctgc cgtta 1595

<210> 156
<211> 1739
<212> DNA
<213> Artificial Sequence

<220>
<223> Synthetic Construct- Sequence No. 156, Example 156: designer
      Nial-promoter-controlled chloroplast-targeted NADH-dependent
      Butanol Dehydrogenase DNA construct (1739 bp)

<400> 156
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgaata atttactttt ttataatcct actaaaatta 360

actttggtag aggagttgaa tcctctgttg gagaagagat taaaagctta ggagctagta 420

aagtgctttt tcattatggt ggaggaagta ttagaaaaaa tgggttatat gatagaattt 480

tagattcttt aaataaagca ggattacatg taatagaact tggaggagtt aagcctaatc 540

ctagattaag cttagttaaa aagggaatag aactttgtaa aaatagtaaa gttgatttta 600

tacttgcagt tggaggagga agtgttattg actcagctaa agccatagct cttggtgttc 660

cttatgatgg agatgtatgg gatttcttta ctaaaaatat taaaatagaa aaagctttac 720

cattaggtac agtattaaca ataccagcgg caggaagtga atcaagctca ggaactgtta 780

taactaatga agatggatgg tataagagat caacaggatc accacttcta tatcctaaat 840

tttcaatgtt aaatccagaa ctatgcttta ctttaccaga atatcaaata gcatcaggaa 900

gtgcagatat tttagcacat ttaatggaaa gatatttcac aaacacaaag aatgttgaac 960

ttatagatag attaattgag ggaacaatga aaacagtaat aaataatgtt cctaaggtat 1020

taaaaaataa ggaagactat gatagctttg cagaggttat gtgggctgga acaatagcac 1080

ataataatct tttaagcaca ggaagagaaa cagattgggc atcacataat atagaacatg 1140

aattaagtgg aatatatgat gttactcatg gtgcaggatt agctgttata ttcccagctt 1200

ggatgaagtt tgtttataag catgatttag atagattcaa ccaatttgct actagagtat 1260

ttgatgttca agttgaagat aaaactaagg aagaggttgc cttagaagga attaagaaac 1320

ttgaagaatt cttcaaatca ataaatcttc cagtaacttt aaaagagtta gaaataggtg 1380

aagatagatt agaagaaatg gctaaaaaat gcacagataa tgatgagcat acagtaggtc 1440

attttgtaga attaaacaca gaggacatcc ttgaaatata caaattagct ttatagtaaa 1500

tggaggcgct cgttgatctg agccttgccc cctgacgaac ggcggtggat ggaagatact 1560

gctctcaagt gctgaagcgg tagcttagct ccccgtttcg tgctgatcag tctttttcaa 1620

cacgtaaaaa gcggaggagt tttgcaattt tgttggttgt aacgatcctc cgttgatttt 1680

ggcctctttc tccatgggcg ggctgggcgt atttgaagcg gttctctctt ctgccgtta 1739

<210> 157
<211> 1733
<212> DNA
<213> Artificial Sequence

<220>
<223> Synthetic Construct- Sequence No. 157, Example 157: designer
      Nial-promoter-controlled NADPH-dependent chloroplast-targeted
      NADPH-dependent Butanol Dehydrogenase DNA construct (1733 bp)

<400> 157
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgatga gatttacatt accaagagat atttattatg 360

gaaaaggatc actagaacaa ctaaaaaatc ttaaaggtaa aaaggcaatg ctagtacttg 420

gtggcggttc gatgaaaaga tttggatttg tcgataaggt gttaggatac ttaaaggaag 480

ctggaataga agtaaaatta atagaaggag ttgaaccaga cccatcagtt gaaacagtgt 540

tcaagggcgc tgagttaatg agacaatttg aaccagattg gattatagct atgggtggtg 600

gatcaccaat tgatgctgca aaagcaatgt ggatttttta tgaacaccca gaaaaaactt 660

ttgatgacat taaagatccg tttacagtac cagaattaag aaataaggct aagttcctag 720

cgattccatc aacaagtggt acagcaacag aagtaacagc attttctgta attacagatt 780

ataagactga aataaaatat cctttagctg attttaatat aactccagat gtagctgtag 840

tagattcaga attagctgaa acaatgccac ctaagttaac tgcccataca ggaatggatg 900

cattaactca tgcaattgaa gcttatgtag caacattaca ttcaccattt actgatccac 960

tagctatgca agcgattgaa atgattaatg aacatttatt taaatcatat gaaggcgata 1020

aagaagctag agaacaaatg cattatgctc aatgtttagc tggaatggct ttctctaatg 1080

cactattagg aatatgtcat agtatggcgc ataaaacagg ggctgtattc catatccctc 1140

atggatgtgc gaatgcaatc tatttaccat atgtaattaa gtttaattca aaaacttcat 1200

tagaaagata tgctaaaata gcaaaacaaa tttcattagc aggaaataca aatgaggaat 1260

tagttgattc attaataaac ttagttaaag aattaaataa gaagatgcaa ataccaacaa 1320

cattaaaaga atatggtatt catgaacaag aatttaagaa taaggttgat ttgatttcag 1380

aaagagctat tggagatgct tgtactggat caaatccaag acaattaaat aaagatgaaa 1440

tgaaaaagat ttttgaatgc gtatattatg gtacagaagt tgatttttaa taaatggagg 1500

cgctcgttga tctgagcctt gccccctgac gaacggcggt ggatggaaga tactgctctc 1560

aagtgctgaa gcggtagctt agctccccgt ttcgtgctga tcagtctttt tcaacacgta 1620

aaaagcggag gagttttgca attttgttgg ttgtaacgat cctccgttga ttttggcctc 1680

tttctccatg ggcgggctgg gcgtatttga agcggttctc tcttctgccg tta 1733

<210> 158
<211> 1745
<212> DNA
<213> Artificial Sequence

<220>
<223> Synthetic Construct- Sequence No. 158, Example 158: designer
      Nial-promoter-controlled chloroplast-targeted 3-Ketothiolase DNA
      construct (1745 bp)

<400> 158
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgaccg acatcgtcat cgtcgccgca gcccgcaccg 360

ccgtgggcaa gttcggcggc tcgctggcca agatcccggc gcctgagctg ggcgctgccg 420

tgatcaaggc cctgctggaa aaaaccggcg tgggcgccga ccagatcggt gaagtcatca 480

tgggccaggt gctggccgcc ggcgcgggcc agaacccggc tcgccaggcc atgatgaagg 540

cgggcatcgc caaggaaacg ccggcgctga ccatcaacgc cgtgtgcggc tccggcctga 600

aggccgtgat gctggcggcc caagccatcg cctggggcga cagcgagatc gtcatcgccg 660

gcggccagga aaacatgtcc gccagcccgc acgtgctgca aggcagccgc gacggccagc 720

gcatgggcga ctggaagatg gtcgacacca tgatcaacga cggcctgtgg gacgtgtaca 780

acaagtacca catgggcatc acggccgaga acgtcgccaa ggcgcacgac atcacccgtg 840

agcagcagga cgccctggcc ctggccagcc agcaaaaggc caccgccgcc caggaagccg 900

gcaagttcaa ggacgagatc gttcccgtcg ccattccgca gcgcaagggc gatccggtga 960

tgttcgacac cgacgagttc atcaacaaga agaccaacgc cgaagcgctg gccggcctgc 1020

gtccggcctt cgacaaggcc ggctcggtga ccgcgggcaa cgcctccggc atcaacgacg 1080

gcgccgccgc cgtgatggtc atgtcggccg ccaaggccga gcaactgggc ctgaagccgc 1140

tggcgcgcat cgccagcttc ggcaccagcg gcctggaccc cgccaccatg ggcatgggcc 1200

cggtgccggc cacgcgcaag gcgctggagc gcgccggctg gcaagtcggc gacgtggacc 1260

tgttcgagct gaacgaagcc ttcgccgccc aagcctgcgc ggtgaacaag gagctgggcg 1320

tggacccggc caaggtcaac gtcaacggtg gcgccatcgc catcggccac cccatcggcg 1380

cctccggctg ccgcgtgctg gtgacgctgc tgcacgaaat gcagcgccgc gacgccaaga 1440

agggcgtggc cgcgctgtgc atcggtggcg gcatgggcgt gtcgctggcc gtcgagcgct 1500

gataaatgga ggcgctcgtt gatctgagcc ttgccccctg acgaacggcg gtggatggaa 1560

gatactgctc tcaagtgctg aagcggtagc ttagctcccc gtttcgtgct gatcagtctt 1620

tttcaacacg taaaaagcgg aggagttttg caattttgtt ggttgtaacg atcctccgtt 1680

gattttggcc tctttctcca tgggcgggct gggcgtattt gaagcggttc tctcttctgc 1740

cgtta 1745

<210> 159
<211> 1439
<212> DNA
<213> Artificial Sequence

<220>
<223> Synthetic Construct- Sequence No. 159, Example 159: designer
      Nial-promoter-controlled chloroplast-targeted 3-Hydroxyacyl-CoA
      dehydrogenase DNA construct (1439 bp)

<400> 159
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggaga ttcggaagat cggcgtgatc ggcgcagggc 360

agatggggtc gggcatcgcc caggtggcgg cccagtcggg ttacgaggtc gtgctaatgg 420

acgtcgagga gctgagcctc aagaaggggc tcgaggccat ccgaaggtcg ctcgaccgct 480

tcctgcgcaa ggagaagatc acccaggagg aggccgaaaa ggccctggcc cgcatcaaga 540

cgacgctcaa ccccgccgac ttcgcggact gcgacctggt cgtcgaggcc atcgtggaga 600

acgagtcggt caagggtaag ctcttccaga cgctcgacaa ggtggtgaag cctgaggccg 660

tcttcgcttc caacacctcc tcgattccca tcaccaagct ggccagctac acctcccgcc 720

ccgagcgttt catcggcatg cacttcatga acccggtgcc gctgatgaag ctcgtcgagg 780

tcatccgcgg ctacaagacc tcggacgagg tcacccgggt ggtcatggcg acggccgaga 840

agatgggcaa ggtgccggtc gaggtcaacg actaccccgg cttcgtctcc aaccgcgtgc 900

tcatccccat gctcaacgag gccatccaag cggtcatgga gggcgtggcc acccccgagg 960

ccatcgacac cgtgatgaag ctgggcatga accaccccat gggcccgctg acgctcgccg 1020

acttcatcgg cctcgacacc gtgctggcca tcatggaggt gctgcacgag ggctttggcg 1080

acagcaagta ccgcccctcg ccgctgctca agaagatggt ccaggcgggt ctgctgggcc 1140

gcaagagcgg gcaggggttc tacaagtacg acgagaaggg gaacaagatc ggctagtaaa 1200

tggaggcgct cgttgatctg agccttgccc cctgacgaac ggcggtggat ggaagatact 1260

gctctcaagt gctgaagcgg tagcttagct ccccgtttcg tgctgatcag tctttttcaa 1320

cacgtaaaaa gcggaggagt tttgcaattt tgttggttgt aacgatcctc cgttgatttt 1380

ggcctctttc tccatgggcg ggctgggcgt atttgaagcg gttctctctt ctgccgtta 1439

<210> 160
<211> 1337
<212> DNA
<213> Artificial Sequence

<220>
<223> Synthetic Construct- Sequence No. 160, Example 160: designer
      Nial-promoter-controlled chloroplast-targeted Enoyl-CoA
      Dehydratase DNA construct (1337 bp)

<400> 160
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgacgg ttcgactgga atacgatggc gggttcgcgc 360

acctgacgct cagccgcccg caggtcctga atgcgctcag tttcgagctg ctcgccgagt 420

tgagccgggc gcttgccggc gtcgccgaat ccgatgcgcg cgccctgatc gtcacgggcg 480

agggcgacaa ggcgttctgc gccggcgcgg acattcccga gctgatgaat cggccgctca 540

tgcaagagct cgaaggggcc gcgaaaggcc aggcggtgtt cagccggatc gccgagctga 600

agattccgtc tgtcgccgtc atccagggtt atgccttcgg cggcgggctg gagcttgccc 660

tggcatgcac attccgcgtt gccactgatc gcgcccgcat ggggctgccc gaggtcaagc 720

tcggcctgat cccgggttat ggcggaacgc agcgtctgcc gaggctgatc ggcgaggggc 780

gcgcactcga cctgatcatg tccggccgca cgatagacgg cggggaagcc gagcgaatcg 840

gcctggtcaa tcgcatagac aacgagggga cgcccctgga gatcggcaag cggtttctgg 900

agccttatct caagcacagt ctctgcgcct tgtattttgc ccgcgaggcc gtgcagaggg 960

gaggcggtgt cgccattgcg gatggcctgc gcatcgagcg ggatctttcc acgctggctt 1020

accggagcca ggatgcggcc gaggggctgc gcgcttttgt ggaaaaacgg cccgcgtctt 1080

tcaaggactg ctgataaatg gaggcgctcg ttgatctgag ccttgccccc tgacgaacgg 1140

cggtggatgg aagatactgc tctcaagtgc tgaagcggta gcttagctcc ccgtttcgtg 1200

ctgatcagtc tttttcaaca cgtaaaaagc ggaggagttt tgcaattttg ttggttgtaa 1260

cgatcctccg ttgattttgg cctctttctc catgggcggg ctgggcgtat ttgaagcggt 1320

tctctcttct gccgtta 1337

<210> 161
<211> 1736
<212> DNA
<213> Artificial Sequence

<220>
<223> Synthetic Construct- Sequence No. 161, Example 161: designer
 Nial-promoter-controlled chloroplast-targeted 2-Enoyl-CoA
 Reductase DNA construct (1736 bp)

<400> 161
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggccg gcgcgcagca ggatcttgcc gctgcgtccg 360

ggcttgtcgc tggccgcggc ggccttggcg gcatcgtgca ggtcgaacac cgcttccacc 420

ggcagcgcca ggctgccatc gagcgcggcg gtgagcagtt cgccgatcat gcggcgcttg 480

tcctcggcct tggtggcctg catcaccttg ctgccccaga agccacgcac ggtggcctgc 540

ttgaagatca catcgccgct ggatatctgc agcggctcgc cggtcatcga gccaaaggaa 600

atcagctcgc cgccttcggc cagcaaggcc atcagctcac ccgctgcatt gccggccacc 660

gaatcgatgg cgcgcacgat gggcgcatcg ccggccagcg cgcgcacctt gtcctgccag 720

cctgcttgcg cagtggagat tgcgttgccg atgcccagcg ctttcagctc gtccacgccg 780

gcgtcgcggc gcaccaggtt gatcacgttg atgccgcgtg cggcggcgag catcgccacc 840

gtcttgccga ccgcaccgtt ggcggtgttc tgcacgatcc agtcgccctg tttcacctgc 900

aggaattcga tcagcatcag cgcgctcagc ggcatggcga tcaactggca accacgctcg 960

tcgtccaggc catccggcaa cggcaccacg ccggaggcgt cggcaaggaa gtactcggcc 1020

caggcctcat gcacaccggc ggcgaccacg cgctggccaa cctgcaagcc ctcgacaccc 1080

tcacccagcg catcgatgac acccgccgct tcgctgccgc cgatggctgg cagttccggc 1140

ttgtagccgt aattgccgcg cacggtccac aggtcatggt tatggatcgg cgcgcgccgc 1200

atcgcaacgc gcacctggcc cttgcctggc tgcggcgtgg ggcgctcgcc cagttcgagc 1260

accttggccg gatcgccgaa ttgggtatgg atggctgcgc gcatggaggt ctcctgccgg 1320

gcacgctctt gctgcgacgc gcccgatcgt tgtgaaaggt ggcgcgatgc tatcggcagg 1380

gctgcaagga agggatgaag cgaacggaac tgctgtgtga agttgttggc gtgcgcgcgt 1440

agtgacgatg ctctgctgca gcgccggagg actgcgtgca ggccgaccct cattaaatgg 1500

aggcgctcgt tgatctgagc cttgccccct gacgaacggc ggtggatgga agatactgct 1560

ctcaagtgct gaagcggtag cttagctccc cgtttcgtgc tgatcagtct ttttcaacac 1620

gtaaaaagcg gaggagtttt gcaattttgt tggttgtaac gatcctccgt tgattttggc 1680

ctctttctcc atgggcgggc tgggcgtatt tgaagcggtt ctctcttctg ccgtta 1736

<210> 162
<211> 2036
<212> DNA
<213> Artificial Sequence

<220>
<223> Synthetic Construct- Sequence No. 162, Example 162: designer
      Nial-promoter-controlled chloroplast-targeted Acyl-CoA Reductase
      DNA construct (2036 bp)

<400> 162
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggatg acgataatgt tgtaaaaccc atttactatg 360

gtaaaataga ggttcaaaat aatggcacgg gagtatggga gcctaccgtt gagtggttga 420

atgagttttt aaaccggagt atggaactat cacagagtct tttacaacta ggctttagta 480

gaagggttag agtgttgagc gagatgggga agatttggag ggagaagctt tcgttagtcg 540

aggagaaact agctcctaat atttctaaga atacaggcta cagcgtggag aatgtgaaaa 600

tggacttaag actggttgaa gaagtgttta atgagaccaa tattgttgag ctcttcgata 660

aaggcttaat cgggggatgg cgtagtcttg acaagcccgt tgaaatcatt gatggggagt 720

tcgtgtggaa tagaccgcta ggcgtatccc ttataatatc ctccggaaac accgtcatac 780

cagctattct cccagcagtg gtttctttag catcggggaa cgtaactata ctaaggccgt 840

ctttcagcaa ctaccaagca gtagtcgaaa tttttaaaac acttttcgat ctagcagaca 900

gctccgtaga aggtgctaga gaaatggctt cggctcttct ggtcgcatac tttaaacatg 960

agagcaaggt atttgaacac ctattagcat cagcacctct cggcatcgtc aattactggg 1020

gcggggagcc aggtagaagc gtgatcgcta gtagggtttt gaagaatccg tttcatccta 1080

agttaatcgt caatggacct ctaacggggt tagcgataat agatgaagag tcagcgtcgg 1140

aaaaagtagc ctacggatta gcgagggatg tggtactgta tgatcaacag ttatgtagct 1200

ctcccactta cgccatattc ataggttcga aagatagcgc gttgaagttt gcacagagac 1260

taggggaagc cctgaataat gtggggagaa ggttcccccg tgatttgaag gaaggagaac 1320

tgtacaattt aatactgctt aggaaaaacc ttgagatcca aggtgtgaga gttttctact 1380

cggaaaaccc cggaaatgct tggacgattg cggtgaaaac actagagtca gtcactaatt 1440

ttgcatatag tttaaaatat ccacatacaa tccctaggag acggttcatt gaaataatag 1500

tgttgaaaga cgccaaagaa ctcaaggaga cgatcttaca cctaattgaa gacttgagga 1560

gaaacggggt tgataagttc cagacagcat cgataaaggt ttctgaaaga aaccttaacc 1620

acttattgaa ggttctctat attcttggga tttacagggt tgtcccaata ggggaatcct 1680

tttttagaac gccgttagaa ccgtacgatg gtgaattctt acctaaatac ttcacttaca 1740

cgatgtatct tagatttatc gagaagtcgg atgcgctaaa acaccctgaa tgataaatgg 1800

aggcgctcgt tgatctgagc cttgccccct gacgaacggc ggtggatgga agatactgct 1860

ctcaagtgct gaagcggtag cttagctccc cgtttcgtgc tgatcagtct ttttcaacac 1920

gtaaaaagcg gaggagtttt gcaattttgt tggttgtaac gatcctccgt tgattttggc 1980

ctctttctcc atgggcgggc tgggcgtatt tgaagcggtt ctctcttctg ccgtta 2036

<210> 163
<211> 1625
<212> DNA
<213> Artificial Sequence

<220>
<223> Synthetic Construct- Sequence No. 163, Example 163: designer
 Nial-promoter-controlled chloroplast-targeted Hexanol Dehydronase
 DNA construct (1625 bp)

<400> 163
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatggaac tcgacctcga cggtcccggg gttggtgaag 360

tgctgatcaa gtacaccgcc gcggggttgt gccattcgga cctgcacttg accgacgggg 420

acctaccgcc gcgctatcca atcgtcgggg ggcacgaggg gtcaggcatc atcgaggacg 480

tcggacctgg ggtcaccaag gtcaaaccag gcgatcacgt tgtttgcagc ttcatcccga 540

actgcggaac ctgtcggtac tgcgccaccg gacgctccaa cctctgcgat atgggcgcca 600

ccatcctcga agggtgcatg cccgacggca gttaccggtt ccacagtaac ggcctggatt 660

tcggtgcgat gtgcatgctc ggcacattct ccgaacgcgc aactatctcc cagcattcgg 720

tggtcaagat cgacgactgg ctgccgctcg agaccgcggt ggtcgtcggc tgcggcgtgc 780

cgactggctg gggcacctcc gtctatgccg gcggggttcg ttgcggtgac accaccgtca 840

tctatggcgt cggcggcctg ggagtcaacg ccgtccaagg cgcggtgagt gcgggcgcga 900

agtacatcgt ggtcgtcgat ccggttgcgt tcaaacgcga caccgcgctc aagttcggcg 960

ccacccacgc gttcgccgac gccgccaccg ccgcggccaa ggtcgacgaa ctgacctggg 1020

gacagggtgc cgatcaggcg ctgatcctgg tcggcaccgt cgacgaggac gtggtctcgg 1080

cggcgactgc ggtgatcggt aagggaggca ccgtcgtgat caccggactg gcggacccag 1140

caaagctcac ggtgcacgtt tcgggaacgg acctgacgct taacgagaag acaatcaagg 1200

gcacgttgtt cggctcgtcc aatccgcaat acgacatcgt acggctgctc cgtctctacg 1260

acgccggcca gctaaaactc gacgatctga tcaccacccg atacacgctc gaccaggtca 1320

accagggcta ccaggatctg cgagacggca agaacatccg cggcgtgatc atccacgcct 1380

gataaatgga ggcgctcgtt gatctgagcc ttgccccctg acgaacggcg gtggatggaa 1440

gatactgctc tcaagtgctg aagcggtagc ttagctcccc gtttcgtgct gatcagtctt 1500

tttcaacacg taaaaagcgg aggagttttg caattttgtt ggttgtaacg atcctccgtt 1560

gattttggcc tctttctcca tgggcgggct gggcgtattt gaagcggttc tctcttctgc 1620

cgtta 1625

<210> 164
<211> 1249
<212> DNA
<213> Artificial Sequence

<220>
<223> Synthetic Construct- Sequence No. 164, Example 164: designer
      Nial-promoter-controlled chloroplast-targeted Octanol
      Dehydrogenase DNA construct (1249 bp)

<400>  164
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggat ggccgccgtc attgccaagt cctccgtctc cgcggccgtg gctcgcccgg     240

cccgctccag cgtgcgcccc atggccgcgc tgaagcccgc cgtcaaggct gcccccgtgg     300

ctgccccggc tcaggccaac cagatgttgg gaggccaaga agccgctggt gattgaggac     360

attgaggtgg cgccacctca ggcttggcag gttcgcatca agattacagc cactggcgtt     420

tgccacacgg attctttttc gttgagcggc tctgatcctg agggtctctt tcccgtggtc     480

cttggccatg agggcgccgg catcgtggag agcgttggcg agggcgtaac caactttaag     540

gccggcgatc atgtcattgc cctctacata ccccagtgca atgagtgcaa attctgcaag     600

agcggcaaga caaatctctg ccagaagatt cgcctcaccc agggcgctgg tgtcatgccc     660

aatggatcct cccgcttgtc gtgcaagggt cagcagctgt tccatttcat gggcacctca     720

actttcgccg agtacgcggt ggtggccgac atatcggtga ccaaaatcaa cgagtcggct     780

ccattggaga aggtgtgcct tctgggctgt ggcatttcca cgggctatgg tgccgccttg     840

aacaccttta ggtggaacct ggcagcactt gcgccgtctg gggtctgggt gctgttggac      900

tggcagtggg tctgggctgc aagaaggctg gcgccgccaa ggtctacggc atcgacatca     960

atccctccaa attcgagctg gccaggaagt tcggcttcac cgactttaaa tggaggcgct    1020

cgttgatctg agccttgccc cctgacgaac ggcggtggat ggaagatact gctctcaagt    1080

gctgaagcgg tagcttagct ccccgtttcg tgctgatcag tctttttcaa cacgtaaaaa    1140

gcggaggagt tttgcaattt tgttggttgt aacgatcctc cgttgatttt ggcctctttc    1200

tccatgggcg ggctgggcgt atttgaagcg gttctctctt ctgccgtta                1249

<210>  165
<211>  1769
<212>  DNA
<213>  Artificial Sequence

<220>
<223>  Synthetic Construct- Sequence No. 165, Example 165: designer
       Nial-promoter-controlled chloroplast-targeted Short Chain Alcohol
       Dehydrogenase DNA construct (1769 bp)

<400>  165
agaaaatctg gcaccacacc atggtagggt gcgagtgacc ccgcgcgact tggaagggtt      60

caaacgaccc cgccgtacga acttttgtcg gggggcgctc ccggatggta gggtgcgagt     120

gaccccgcgc gacttggaag ggttcaaacg accccgccgt acgaactttt gtcggggggc     180

gctcccggat ggccgccgtc attgccaagt cctccgtctc cgcggccgtg gctcgcccgg     240

cccgctccag cgtgcgcccc atggccgcgc tgaagcccgc cgtcaaggct gcccccgtgg 300

ctgccccggc tcaggccaac cagatgatca tcaaaccgcg cgtgcgtggc ttcatctgcg 360

tgaccactca tccggtcggc tgcgaggcca acgtcaagga acagatcgac tacgtgactt 420

cgcacggccc gatcgccaac ggcccgaaga aggtgctcgt gatcggcgcg tcgaccggct 480

acggcctcgc ggcccggatc tcggccgcct tcggctcggg cgcggacacg cttggcgtgt 540

tcttcgagcg cgccggcagc gacaccaagc cgggcaccgc cggctggtac aacagcgccg 600

cgttcgagaa attcgccgcc gaaaaggggc tctatgcgcg cagcatcaac ggcgacgcgt 660

tctccgacaa ggtcaagcag atcacgatcg acacgatcaa gcaggacctc ggcaaggtcg 720

atctggtcgt ctacagcctg gccgcgccgc gccgcacgca tccgaagacg ggcgagacga 780

tcagctcgac gctgaagccg gtcggcaagt cggtgacgtt ccgcggcctc gacaccgaca 840

aggaaacgat ccgcgaggtg acgctcgagc cggcgacgca ggaagagatc gacggcaccg 900

tcgccgtgat gggcggcgag gactggcaga tgtggatcga cgcgctcgcc gacgccggcg 960

tgctggccga cggcgcgaag accaccgcgt tcacgtatct cggcgagcag atcacgcacg 1020

acatctactg gaacggctcg atcggtgagg cgaagaagga tctcgacaag aaggtcgtgt 1080

cgatccgcga gaagctcgcc gtgcatggcg gcgacgcgcg cgtgtcggtg ctgaaggccg 1140

tcgtcacgca ggcgagctcg gcgatcccga tgatgccgct gtacctgtcg ctgctgttca 1200

aggtgatgaa ggaaaagggc acgcacgaag gctgcatcga gcaggtgtac gggctgctga 1260

aggacagcat gtacggcgcg acgccgcaca tcgacgaaga aggccggctg cgcgcggact 1320

acaaggaact cgatccgcag gtgcaggcgc aggtcgtcgc gatgtgggac aaggtcacga 1380

acgacaacct gtacgagatg accgacttcg ccggttacaa gaccgagttc ctgcgtctgt 1440

tcggcttcga gatcgccggc gtcgactacg acgcggacgt gaacccggac gtgaagatcc 1500

ccggcatcat cgacacgacg gcctgataaa tggaggcgct cgttgatctg agccttgccc 1560

cctgacgaac ggcggtggat ggaagatact gctctcaagt gctgaagcgg tagcttagct 1620

ccccgtttcg tgctgatcag tctttttcaa cacgtaaaaa gcggaggagt tttgcaattt 1680

tgttggttgt aacgatcctc cgttgatttt ggcctctttc tccatgggcg ggctgggcgt 1740

atttgaagcg gttctctctt ctgccgtta 1769

<210> 166
<211> 6110
<212> DNA
<213> Artificial Sequence

<220>
<223> Synthetic Construct- SEQ ID NO: 166, example 166: a designer
hox-promoter-controlled Formylmethanofuran dehydrogenase (Fmd)
DNA construct (6110 bp)

<400> 166
agaaaatctg gcaccacacc gcagaaatat aggggctagg agttgagggt actctggttc 60
gtcaagcatt tgggatgatt tcccctcaca agttcctcaa attattctcc tataaacaat 120
agatataagg tcaaaacttg agttatgagt gctgagtaaa aaattactct ccacgcctca 180
gaggtagata tgatgagcgt ctataaaaat gtgacatgtc ccgtctgcgg agggtcttgt 240
gatgacatag aggttctcta tgatgggaaa acaataaaaa caaggaatgc ttgcaggatg 300
gggaatgcaa agtttcagga gatggtcagc tcccacagaa tacttagacc ccagattaaa 360
actgaaagtg gtttcagatc cgcagaatgg gatgaagccc ttgacgctgc agcagagata 420
cttacagagt caaaaagacc aaccctattc atgggtagtg agatgtcaac tgaagccatg 480
gctgcaggtc ttgaacttgg tgagtatcta aatgccatgg tggattcaaa tgcaaccata 540
tgtcatggcc ccacattaat gggaatccag gaagctggac agagcgctgc tacggcaggt 600
gagataaaga atagagctga cgtcatcatt tattggggta caaatgttat ggactccatg 660
cctcgccaca tgtcacgtta cagcatattc atgcgcggat ttttcagaga acgtggtaaa 720
aaagatagaa ctgtcatctc cgtggatccc cgtgaaacag caacaactaa agcctccgat 780
atccatctgc agctgaaacc aaactcagat tatgaacttt tttcagcact gcttacagtt 840
ataaggggca atgaaccgca caggagtatt gagagtataa cagggatatc agttgaaacc 900
ataaaggaag tcgcagacat aatgctgaat gcgcagtttg gagccatcta tggaggtctt 960
ggacttgcat catcagaagg taagcataga aacgttgaga tggttctcaa actggtagct 1020
gcacttaatg aacacaccaa atttacaata ggagccataa gaggtcattg taatgttgca 1080
ggatttaatc aggtggcttc atgggagtat ggtttcccct ttggtgttga ttttacaagg 1140
ggttatccac gctacaaccc cggagaaaca actatagtcg atcttctcca gagaaaagaa 1200
tccgatgcgg tcctcgttat atgttcagat cttggagctc acctccccaa agaatgcgtt 1260
gagtatatga aagagattcc tgtaatatgc atagatattg ccccatgtcc aacaacactt 1320
gtatctgatg ttgtgatccc tggagttatt gatgctatgg agtccagcgg caccttctac 1380
cgctttgaca atgttcccat ccaccataag gcattcacaa catcaccatt ccctgaaaca 1440
gagagcaacg aacatactct tagacagatc cttgagaggg ttaaatcaat taaaggcgaa 1500
taaatgggat ttgtgttggt accaaaatca gatttccaga tacctctgga agcagatacc 1560
ataagacccg atctgtttga agggctggat cttgatgaaa ttcgttccct tcaagtatac 1620
gaagggaaca tcaaaagacc cctaggcgaa ttttttgaga tagcagagac atcccatgaa 1680
gatcagttaa tacggatcga tggggatgtc agcagggtga agtatatcgg ttcagggatg 1740
aaatccggaa aaatcatcat aaacggcgat gtcggactcc agcttggctg tgaaatgaaa 1800
ggcggtgaaa tagaggtaaa tggaaatgtt tcttcatgga tcggcatgga aatgcatggt 1860

ggcaccataa aaattaatgg aaacgcgggg gattatgttg gctgcgcata taggggagag 1920

tggcgtggca tgaaaggagg taaaatcatt atacagggta atgcagggaa caatattggg 1980

ggtggtatga tggccggaga aatttatata ggtggagatg caggtaattt ctgcggcatc 2040

cgaatgaatg ggggtgaaat aacagtacgc ggggatgctg gaagggcccc cggagcagag 2100

atggtatcag gtatcatcaa aattcatgga cggatctcct ctctcctccc gggattcaag 2160

gagatctcaa cattcaaaga agatggatcc cttatgatcc tattcaaagg agatctatcc 2220

gaaaaaaatc cggagggtaa tctatacatc aattataata agaaccttca cattctagag 2280

aatgagaccg atgaagggag agtcatcaca aaaaagggaa ttaaagtaat ctacaacagt 2340

ggaagtacga tccgtgaagg gcagataata aagggaggca ataaactcac agatgattat 2400

atagatgaat gtgcacgctg ctgtattagt cctgaagact ataagctcct aggtgaaccc 2460

gaaaatgttg tcgtatcatc acatggaaat gaagttgttc tgagagctgt tgaggaccct 2520

ggaatccaga tgggaacaat attcataccc agaggtatat gggccaatgt gctaacgcca 2580

ccgtataccg agtcaaccgg ttccccaatg tataaaggag tgccggttta tcttagaaag 2640

gcttcacaag gtgaaaggat cctgagtgcc gaggaacttg tagaggaata cggggtggga 2700

aaatgagtgg ggcaggtcag agaatacctt gatttaataa aaaatttcag tgggttcgca 2760

agcccgggca gtgtcatagg agctcatatg ttactaatag ccaggaaggt ccttgatttt 2820

gaagtcgatg aagaaattta tgttacatgt gaaacgacaa actgtttacc cgacgcattc 2880

caggccatat gcaaatcaac aataggaaat gggagactga atatcctgga cactggaaaa 2940

atggctgtca tcatcaacag aaagggtatg cctggagaaa cagtccaggc tcttagaata 3000

atactggatc ctgaaaagac cgtcaattac ccgatcatac atgaatggta catgaacacc 3060

cgcaaggtat ccgcagaaga agtgaatcca gaactcataa gagccggtga aaatctatat 3120

tcctggtatt ttgttgatgt gatcgttcca gaaaaagaaa agaagataat tgaaatctgt 3180

aatctttgca acgaaccttt cataaaaaga aatgagctgg atctctgtcc agcttgtctg 3240

aaaagataaa tggaatatat tttaaaaaat ggtattgtat ttgatccagc aaataatatt 3300

gatggagaaa aaaaggacat actaataaaa gatggataca tagttagtga agtatcaaag 3360

gatgcaaaga ttatagatgt tacaaataaa ttagtaatgc ctggaggagt agatttacac 3420

tcacacatag caggaccaaa actatcagtg ggaagattat atcgtccaga agatacaaga 3480

cgtggaataa aacctgcacc atgtggtgct attgagggat ttgaagcagg attttccata 3540

ccaacatgtc caacaactgg gtacaggtat actcgtatgg gatatacaac agtaacagaa 3600

gcagcagtac cacccttaga ggcaaaacat gcacatgaag aaataaacag tattccaaac 3660

cttgatatac caacattaac actctttggt aataactggt tcatgcttaa atatgttaag 3720

gaaaataact tagaaaaact agctttattc atatcaaaat ggttgaaact agcaaagggt 3780

tatggaatta aaatagtaaa tccatgtgga agtgaagcat ggggttgggg aaaaaatgta 3840

gatggacttg atgatccaga accacactgg ggtgtaacag gacgtcaagt aatacaggct 3900

ctaactaaag taaatgaaaa attaggttta ccccattctg tgcatgttca tacaaatgac 3960

cttggacatc ctggaaacta tgaaacaaca cttgaaacat ttgattgtgt aaaggatatt 4020

aagaaaaata agaatgtaac aagagatcaa gtgttacatg caacacacgt acagttccat 4080

gcatataagg gaacatcatg gagggatgtt acatcaggtg caccagaatt gattgattat 4140

ataaataagt caaaacatat gacttgtgat attggacaga taaccttcga tgaaacaaca 4200

acaatgactg cagatgcacc aatggagtat gatttataca gactaacagg acttaaatgg 4260

gcaaataagg atatagaggt tgaaacttca tctggactta ttccatcaat atattctaaa 4320

cgagcaccag taagtgtact tcaatgggct attgggcttg aattcttcct tggaataaaa 4380

gatccatgga aattggcatt aacaactgat tcaccaaatg gtggtccatt tatcagatat 4440

ccacgtgtaa tttcatggat aatgagtaat gaaaaactaa atgatatgct tgataatcaa 4500

gtacatagtt gggcaacaag aagaacatca cttggaagtt atgataggga atattcctgg 4560

aatgaaatag caacaattac aagggcaagt ccagcaaaaa tacttggatt aacagataga 4620

ggacatcttg gtgtaggtgc aaaggctgat gtttcagtat atgatataga tgtctatgac 4680

tttgatacaa cacttcttaa aaattcacaa acacttgaaa acaagttatt aaacagttta 4740

tacactataa aagatggtat gatcatggtt gatcatggaa aaattgttaa attagtagat 4800

agtaaacata tatggactaa tgtttgtggc ttagaaaaag aagaggctaa tcttgtagat 4860

gaaataacac cagaatttaa taagtattat actataaaat atgaaaatta tggtgttcca 4920

gatcattata taaaacctga tacttgtgta gatatagaat atgaggatta gatggatgaa 4980

atggatatta tcataaatac aggtacgagt attatccaag cattttatga aaagaaaggt 5040

tctacattaa aggatgaata cagacaatct acagctgtag cttttatgga tccaagggat 5100

atgaaaaaat tatctctaaa accacgtgat aaaataaatg taacatctaa atggggaagt 5160

gtaacaatat atgcagataa atcccatgat gcaccacatg aaggtatgat attcatacca 5220

aggggaccat gggccaatat agtaataagt ccagaaacat attgctgtaa tattccaaca 5280

tataagggag taaaagcagt aattaaaaaa acagacgatg aagtattact cgtagcaaat 5340

ctaatgcata aaacatacaa taaatataaa tataacacaa aaactcttgg acaaaagcca 5400

gtatataaaa aaatagggga gtaaatgtta tgtgttaatc aagatcattg tttagggtgt 5460

ggagcatgtg ttatagtatg tcctgttaat caagaaatct atccagaggt tattggaggt 5520

aatgggccag atacaacaga tgtggtaatg ttagttgaaa atggagtaat aaaactattc 5580

catccagaaa aatgtgtaac atgtatgaaa tgtaatgata catgtccaac aaaggcaata 5640

tatcataagg gagactaatg aagtaagtag gaagcaggga gcaggggaaa gaaaattgac 5700

aactgtacaa gattaatcgc gtctctgagc aatgaccaaa tacatctacc tccacggttt 5760

tcttccagcc ccctatctgc gaaagcacaa gatattagca agcgtttcgc ccaaattcac 5820

atacagctaa caatccctga tctcaatgct ggtgaatttt ctcagttaac aatcacgcgc 5880

caaattcaac aagttgccgc aattttccct gataattctg aaccaataac gctgataggt 5940

tctagtttag gcggtttaac tgctgcttat ctaggacagc gatatttaca agtacaacgc 6000

ttagttttat tagcgccagt ttggtttttt atcccattgg ttgcccaaaa tgggtgaaga 6060

agctgtcaca agttggcaac aaacgatata ggttctctct tctgccgtta 6110

<210> 167
<211> 1538
<212> DNA
<213> Artificial Sequence

<220>
<223> Synthetic Construct- SEQ ID NO: 167, example 167: a designer
      hox-promoter-controlled Formyl transferase DNA construct (1538
      bp)

<400> 167
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttagaaga ggtcatggag tttgatctgg tatgctccaa ggttgccgcc 240

gtagttccct gcacttatct taattatgcc tggaacggta catgcggcct ttataccttc 300

cttcattgcc tcccttacaa cctcttcgtt gacaccgtct ataactatct catatacccc 360

gttgacgttt tcaggtatct cgctgccctc aacctcatcc ttgagtgtca cgcacatctt 420

ctcattggtt gaggcgttga ggaacttgta cttgtttgat ccaacctttg aacctgaagc 480

caccacacca ccggggaatg gtgttaccgt gccttcaact gcggctattg catccactgc 540

agcctgggcc gcaaggaggg ctgatgcctg actgtcaccc atgatgaaga agtttccgcc 600

tgcaaccccg tcctttattc caaattcgct ttcaatgagg aagtcgcctg acattatcgg 660

gattgagtgg attttccttc catcaatttc aagttccttc tcgtagccat ccccgaagaa 720

cttgagtttg aatcccacat tgagcttctc gtcctcgtca tcgagagcat cgaatactgc 780

ggttgttggt gctgtaagta tacccatccc tatcctttca aggagttcat ggtccaggtt 840

cttctttgat gggttgcaga tcattattat gtatccgggc cttccatcgg gtgtctcctc 900

tggtggaacg tagcagtcta tacctgcctc tgcagggcac cctataacag atgtaccgta 960

acctgttgcc tcggttgcag ctatctttgc aagttttttg gttgctgcag ttacaaggac 1020

ccttgaaacc ttgataccga aggcctccgc aaatgtatcc tctatttcaa caccatttat 1080

ctccattgaa gtaagtagga agcagggagc aggggaaaga aaattgacaa ctgtacaaga 1140

ttaatcgcgt ctctgagcaa tgaccaaata catctacctc cacggttttc ttccagcccc 1200

ctatctgcga aagcacaaga tattagcaag cgtttcgccc aaattcacat acagctaaca 1260

atccctgatc tcaatgctgg tgaattttct cagttaacaa tcacgcgcca aattcaacaa 1320

gttgccgcaa ttttccctga taattctgaa ccaataacgc tgataggttc tagtttaggc 1380

ggtttaactg ctgcttatct aggacagcga tatttacaag tacaacgctt agttttatta 1440

gcgccagttt ggttttttat cccattggtt gcccaaaatg ggtgaagaag ctgtcacaag 1500

ttggcaacaa acgatatagg ttctctcttc tgccgtta 1538

<210> 168
<211> 1631
<212> DNA
<213> Artificial Sequence

<220>
<223> Synthetic Construct- SEQ ID NO: 168, example 168: a designer
      hox-promoter-controlled 5,10-Methenyl-tetrahydromethanopterin (H4
      methanopterin) cyclohydrolase DNA construct (1631 bp)

<400> 168
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgagtga aagtatgaac atagcagcaa agaaaattgc tgataaaatg 240

gttcaagatg cagaaaaatt aaaagtaatt ccatcaacat tatccaatgg aacatctatt 300

atagattgtg gtgttaatgc taagggaagt attaaaggtg gggaattatt tacaaaagta 360

tgtcttggtg gaatttgtga tgttggaatt tcaatacctg gtgacttaag tgatataatg 420

gctatgcctg ctgttaaggt taaaactgat tttccagcat taaccactct tggttctcag 480

aaagcaggat ggaaaataga tgttaatgga tattatgcta taggttctgg acctgctcag 540

acacataaat ttaaagataa taacatttat aaaataacta actatattga agattcagat 600

attgcagtta ttactcttga agcagataaa ttacctgatg aagatattgc taattatatt 660

gcagatgagt gtggtgtaaa accagaaaat ttaaccatac ttgttgcacc tacatcttct 720

cttgttggat ctattcaaat atctggtaga gctcttgaaa ctggaatata caaaatgtat 780

gaaataatga acttcgatgt tactaaaatt acatatgctg caggtattac ccctattaca 840

ccagttgatc ctgatagtct taaagctatg ggtaaaacta atgatgctat catgtttggt 900

ggaagagcat attattatat tgaacctgat gaaggtgaag atttagaaga attagctgct 960

aatttaccat catctgcttc tgataattat ggtcaaagct ttattgaatt atttaaagaa 1020

gcaaatcagg atttctataa aatgcctaag gatatttttg caccagcaca agttattgtt 1080

aatgatatga taactggtca aatgttccac acaggattta tagatcttaa acgtctaaaa 1140

aaatcttttg aagttaaaga aataatttat gaaaaataat gaagtaagta ggaagcaggg 1200

agcaggggaa agaaaattga caactgtaca agattaatcg cgtctctgag caatgaccaa 1260

atacatctac ctccacggtt ttcttccagc cccctatctg cgaaagcaca agatattagc 1320

aagcgtttcg cccaaattca catacagcta acaatccctg atctcaatgc tggtgaattt 1380

tctcagttaa caatcacgcg ccaaattcaa caagttgccg caattttccc tgataattct 1440

gaaccaataa cgctgatagg ttctagttta ggcggtttaa ctgctgctta tctaggacag 1500

cgatatttac aagtacaacg cttagtttta ttagcgccag tttggttttt tatcccattg 1560

gttgcccaaa atgggtgaag aagctgtcac aagttggcaa caaacgatat aggttctctc 1620

ttctgccgtt a 1631

<210> 169
<211> 1475
<212> DNA
<213> Artificial Sequence

<220>
<223> Synthetic Construct- SEQ ID NO: 169, example 169, a designer
      hox-promoter-controlled 5,10-Methylene-H4-methanopterin
      dehydrogenase DNA construct (1475 bp)

<400> 169
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggttgt aaaaattggt ataataaaat gtggtaacat cgggacctca 240

cctgtgctgg acctgttact tgatgagagg gcagacaggc caaacataga tgtctgtgtc 300

gtgggttcag gtgcaaaaat gaaccccgat gaaatcgaaa gggcagtacc aaccatgctt 360

gaaatggaga gggactttgt tatattcata agcccaaacc ccggtgcacc tggccctgca 420

aaggcaaggg agctcctttc agcggctgat gttccagcca tgatcatagg tgacgcacca 480

ggcctcaggg tcaaggatga aatcgaggag cagggacttg gatacataat agtaaaggcc 540

gacccgatga taggggcaag aagggagttc ctggacccaa cagagatggc atccttcaac 600

tccgacgtta taaaggtcct tgcattcaca ggcgcataca gggttgtgca gaacacaatc 660

gatgcaatga ttgcagatgt tgaagccgga aaggcacctg aacttcctca ggtggtaata 720

gacacagata aggcggttga ggcagcaggc tacaccaacc catatgcaaa ggccaaggcc 780

atggctgcat atgagatagc aaccaaggtg gctgacatag acgtcagggg ctgcttcatg 840

gtacaggacc ctgaccagta cataccaatc gttgcctcag cacacgaaat gctatctgca 900

gctgccaaac ttgcaattga agcaagggaa atcgaaaagg ccaacgacac cgtgctgaga 960

acaccacacg gtaaggaagg caaaacactt agcaagaagg atcttctggc caagccagaa 1020

tagtgaagta agtaggaagc agggagcagg ggaaagaaaa ttgacaactg tacaagatta 1080

atcgcgtctc tgagcaatga ccaaatacat ctacctccac ggttttcttc cagcccccta 1140

tctgcgaaag cacaagatat tagcaagcgt ttcgcccaaa ttcacataca gctaacaatc 1200

cctgatctca atgctggtga attttctcag ttaacaatca cgcgccaaat tcaacaagtt 1260

gccgcaattt tccctgataa ttctgaacca ataacgctga taggttctag tttaggcggt 1320

ttaactgctg cttatctagg acagcgatat ttacaagtac aacgcttagt tttattagcg 1380

ccagtttggt tttttatccc attggttgcc caaaatgggt gaagaagctg tcacaagttg 1440

gcaacaaacg atataggttc tctcttctgc cgtta 1475

<210> 170
<211> 2594
<212> DNA
<213> Artificial Sequence

<220>
<223> Synthetic Construct- SEQ ID NO: 170, example 170: a designer
 hox-promoter-controlled methylenetetrahydrofolate reductase
 and/or methylene-H4-methanopterin reductase DNA construct (2594
 bp)

<400> 170
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgctactcta ccttgggacg gggatacagg ccggcttcct tgacgatggt 240

ggggatgata tcttcccaca tgacggccat gatatggacg ccggctacac cctcgatgga 300

ctgcaggtgt ttaatctgtt ccacacagat ggccacccct tcggccttgg ggtcggaggc 360

tttttccatg cggttgacga tttcgtcggg gacaatcatc ccggccaccg attgttgcat 420

atatttggcg gcccgggcgg atttcagggg catgacgccg gcaataattt tggctctttt 480

atgcaggccg cgctcccgga ccagggccat aaagcgttca aaacgctcca tatcaaaaat 540

gcactgggtt tgaataaagt cggcgccggc attgatcttc ttctccaggc gcatgacccg 600

gaactcaaag gggtcggcaa aggggttggc ggcggcgccg atgaagaagc ggggttcctg 660

gcccttaatc tcttcaccgc aggcgaattt tttctcatcc cggaggtcct tcacgatccg 720

gatcagctgg agggaatcca cgtcatggac gtttttcgcc gtcggatggt taccaaaaga 780

ttgatggtcg ccggacaggc agaggacgtt ccgcatcccc aggctgtagg cacccaggag 840

atcactctgg agggcgatac ggttgcggtc ccgaaccgtc atctggataa tgggttcgcc 900

cccggcctgg aggacgtgga ccccagcggc gatactcgac aggcggacga tggccgtctg 960

gttgtccgtc aggttcatgg catcaacgta gtccttgagg agagcggcgt gcttcttgat 1020

ctctgtagga tcggcgtgct tcggcggtcc aatctcaccg ctgaccagaa attgcccctg 1080

ggagaggatt ttcgccatct tgctttcaac catatgacca ttaaaaaagt tgcaatcttg 1140

ggagcaggat gttacagaac acatgctgca acaggaatca ccaactttgc aagagcatgc 1200

gaagttgcgg aaaaagttgg caaaccggag atagcgatga cgcactccac aattgccatg 1260

gctgcagagt tgaaatacct ggctggaata gacgatatcg tcatctcgga tccgtcattt 1320

gccggtgaat tcactgttgt gaaggacttc gattacaatg aagtcatcaa agcccacaaa 1380

gacaatcccg aaacgattat gccgaagatc agggagaagg ttaacgaact cgccaagaca 1440

gttccaaaac ccccgaaggg cgccatacac tttgttcatc ccgaagacct aggcctgaag 1500

gtaacgactg acgacaggga agcagtacgt gatgcagacc tgataatcac ctggctgcca 1560

aaaggcgaca tgcagaaagg cattatagaa aaattcgcag gagatatcaa agagggagca 1620

ataatcaccc acgcctgtac gattccgaca acccttttct ataagatatt cgaggaactc 1680

ggtatcgcag ataaggtgga agttacatcc tatcaccccg gctcagttcc tgagaacaag 1740

ggccaggtct acatcgcgga aggatatgcc tcagaagaag ctatcaatac aatatacgaa 1800

cttggaaaga aagcccgcgg acatgccttc aaacttcctg cagaacttat cgggccggta 1860

tgcgacatgt gtgcagcact taccgctata acctacgccg gactgcttgt ataccgtgat 1920

gccgttatga atattctcgg tgcacctgcc ggattcagcc agatgatggc gacggaatca 1980

ctggaacaga tcaccgccta tatgaagaag gtaggcataa agaaccttga agagaacctt 2040

gacccgggtg tattccttgg aactgcagac tcaatgaact tcggacctat cgccgagatc 2100

cttcccacag ttcttaaatc acttgagaaa agggcaaaat aatgaagtaa gtaggaagca 2160

gggagcaggg gaaagaaaat tgacaactgt acaagattaa tcgcgtctct gagcaatgac 2220

caaatacatc tacctccacg gttttcttcc agccccctat ctgcgaaagc acaagatatt 2280

agcaagcgtt tcgcccaaat tcacatacag ctaacaatcc ctgatctcaa tgctggtgaa 2340

ttttctcagt taacaatcac gcgccaaatt caacaagttg ccgcaatttt ccctgataat 2400

tctgaaccaa taacgctgat aggttctagt ttaggcggtt taactgctgc ttatctagga 2460

cagcgatatt tacaagtaca acgcttagtt ttattagcgc cagtttggtt ttttatccca 2520

ttggttgccc aaaatgggtg aagaagctgt cacaagttgg caacaaacga tataggttct 2580

ctcttctgcc gtta 2594

<210> 171
<211> 2819
<212> DNA

<213>  Artificial Sequence

<220>
<223>  Synthetic Construct- SEQ ID NO: 171, example 171: a designer
      hox-promoter-controlled
      Methyltetrahydrofolate:corrinoid/iron-sulfur protein
      methyltransferase DNA construct (2819 bp)

<400>  171
agaaaatctg gcaccacacc gcagaaatat aggggctagg agttgagggt actctggttc      60

gtcaagcatt tgggatgatt tcccctcaca agttcctcaa attattctcc tataaacaat     120

agatataagg tcaaaacttg agttatgagt gctgagtaaa aaattactct ccacgcctca     180

gaggtagata tgtcaggcct taatcttccc ggcccgggcg gcagatatat actccaggca     240

gtattcatcc tggccgtaca gggcggtggc ggcccgcaac aggcccatta atggcatatc     300

caggggatca aggataaagg aatccatacc tttagtgagg cacataacca taaaggcccg     360

gttcagcagt cgcctttccg gcaggccata ggaaacgttg gaaaggccgc agatggtgtg     420

aaccccgggg tatttttgcc gcagggcggc aaccgattcc agagcctcca cgccgtactg     480

gctgttaact cccaggggtt taatcagggg atcaaggtag atatcgtctt ccggtatgcc     540

ggcctctacc agcccgggta ccagcttacc agcaacgcga aggcggtcct ctaccgaatc     600

cggcatgccg ccgtcatcca tgcaaagggc gataacctta gccttatatt tctgcactaa     660

aggcaaaact tcctcccagc gctgcttctc ggcggtgatg gagtttacca ttgcctggcc     720

cttatgggct gccaggccgg cagccagggc ctcggcgctg gggctatcaa tacacagggg     780

aacatcaacg acttcctgga cgatattcac gagccaggtc attaccttat tttcttcgcc     840

gatgcttgtc ccgcagttaa catcgataat atcggctccg gcctcggcct gcttcctggc     900

cagttcctgg atataatcct tatcccggtt agcaatagcc tgggcaatgg ctttgcggct     960

ggagttaatt aactctccga caacgagcat ttgcaggtaa ctgccatgga tgtataccga    1020

ttacttccaa aaacaaactg tggtaaatgt gacgagtcat catgcatggc cttcgccaca    1080

aaactcatag aaaaggaact cacactggat gactgcccgc agctcagtgg ggatgaaaga    1140

cagaagctcg aggacctcct tgcgccggct gtgagggaaa tcaccttcgg cccagaaaag    1200

aaccaggtgg tggttggggg tgatgaggtc ctctacaggt ttgaactcac atactataac    1260

cccacagccc tcgtggttga tcttccagat gacctaccat cagaggagat caggaagagg    1320

gccagtgata tcatgaatct taaattcgag cgtaccggtg aggaactgac cctggatgcc    1380

atagcgctca ggaacaaatc aggaagccct gaaaaatttg cagaggcagc agggaccctg    1440

gctgaactta acttccctgt tgttctatgc acattcgatc cagaggcaat gagggctgcc    1500

ctcgaggtcc tgggggatca gaggcccctc atgtatgcgg ctacaaagga caacctccag    1560

gagatggctg atctttccat ttcatacggc tgccccctgg tgctcttctc accgggggac    1620

cttgaggaga tgaagaacct cacaagaaga ctgcgggcaa tgggccttac tgaaattatc 1680

ctggaccccg gaacattcac aggtgaggga ataggcgaca ccatagacaa ctttgtcatg 1740

ataaggcgcc ttgcagttga ggaaagggat gaggacttcc gcttccccat catgggcata 1800

ccagcactct caaggctttc aggaggggat ccagttgagg ataacataaa ggaggccact 1860

gttgcagcca cactcatgaa ccgctacgcc gacattctta tactcggagg aacagatatc 1920

tgggaactta tgcccatact caccctcaga cagggcctct acactgatcc aaggaagcca 1980

cagactgttg atccgggaat atatgagttt ggagacgttg atgagaactc ccctgtgata 2040

ctcacaacca acttctcact cacatactac acagtggagg gtgacctcaa ggcaggtgat 2100

gttacggcat atctccttgt gcttgacaca gagggaaggg ctgtggacgt ttcactggct 2160

ggaggacagc tcacgggaac cgcagtggct gaccttataa aggatagtgg tatagaggat 2220

agggtaaagg atagggttct catcattcct ggacttgcgg cacctgctag tggtgaaata 2280

gaagatgata ctggatggaa ggtgcttgtg gggccaaggg attcctcagg gatacctgac 2340

taccttgaaa aactggcatc ggagtagtga agtaagtagg aagcagggag caggggaaag 2400

aaaattgaca actgtacaag attaatcgcg tctctgagca atgaccaaat acatctacct 2460

ccacggtttt cttccagccc cctatctgcg aaagcacaag atattagcaa gcgtttcgcc 2520

caaattcaca tacagctaac aatccctgat ctcaatgctg gtgaattttc tcagttaaca 2580

atcacgcgcc aaattcaaca agttgccgca attttccctg ataattctga accaataacg 2640

ctgataggtt ctagtttagg cggtttaact gctgcttatc taggacagcg atatttacaa 2700

gtacaacgct tagttttatt agcgccagtt tggtttttta tcccattggt tgcccaaaat 2760

gggtgaagaa gctgtcacaa gttggcaaca aacgatatag gttctctctt ctgccgtta 2819

<210> 172
<211> 2771
<212> DNA
<213> Artificial Sequence

<220>
<223> Synthetic Construct- SEQ ID NO: 172, example 172: a designer
hox-promoter-controlled Corrinoid iron-sulfur protein DNA
construct (2771 bp)

<400> 172
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggccgt ccagatttta cgtgatcgta gccgagctgc cgtccagaaa 240

gttgtcctgg gcgccaccaa agaccagggg ggtacccgca gccataccat cgtcgtcggt 300

ggcgatgctg ccctgccttt ccaccatttc gaaggagaga ttgtcaacag gccggtaatc 360

ggtatggaag tgcaggatat cgtacccgac tggcccgacg ttctcaaaga tcccttcacc 420

gatgttatta atgaaccagg gcgctgggcc caaaagtgcg tagccgagta tggtgctgac 480

cttatctacc tgaaacttga cggggccgac cccgaaggcg ccaaccattc tgtggaccag 540

tgcgtagcta ctgttaaaga ggtcctgcag gccgtggggg tacccctggt agtggtaggt 600

tgcggcgatg tggaaaagga ccatgaggtc ctggaagcag tagccgaggc tgctgccggc 660

gagaatctcc tcctgggtaa cgctgaacag gaaaactata aatccctaac ggcagcctgc 720

atggtccaca agcataatat catcgcccgt tcgcccctgg atattaacat ttgtaaacaa 780

ctcaacatcc tgatcaatga aatgaacctg cccctggatc atatcgtcat cgacccgtcc 840

atcggcggcc tgggttatgg tattgaatac tccttctcga ttatggaacg catccgtctg 900

ggggccctgc agggagataa gatgctctcc atgccggtca tctgcaccgt aggctatgag 960

gcctggcgcg ccaaggaagc ctcggcaccg gtgagcgaat acccgggctg gggtaaggaa 1020

accgagcgtg gcatcctctg ggaagccgtt accgccactg ccctgctcca ggccggcgcc 1080

cacatcctcc tcatgcgcca tccggaagcc gtagccaggg tgaaggagaa tatcgaccag 1140

ttaatggtga gcaacgccta ttaaatggat aaattaacgg aacttctgaa attacttcag 1200

aacacggaat ccatcgaaat aaatgagttc aggattgatg ttgacgaact tgaactctac 1260

ctgatgcctg tggttcagca ggccatccag aaaacagttg aggttaggga ggcagttgag 1320

gcactccctg aggaggaatt tgaaccaccg gtaaagacat accctggtga ggttgcccag 1380

gtgaaactgg gtgagggtac aaggaaacct gtttaccttg gcggtcagaa ggccctctac 1440

agatttgagg aaccccagcc aaacccaccg gtggttacat tcgacgtctt tgacataccc 1500

atgccgggtc tcccaaggcc gataagggag cacttcagcg acgtcatgga ggaccctggc 1560

gactgggcca ggaaggcggt aaaggagtat ggtgccaaca tggtcacaat acacctcatc 1620

ggaacaggac ccaaggtaat ggacaagtca ccgagggagg ctgcaaatga cattgaggag 1680

gtcctccagg ccgttgatgt ccccctggta atcgggggct caggggaccc tgaaaaggat 1740

ccactggttc ttgaaaaggc tgccgaggcc gccgagggcg aaaggtgtct cctggcatca 1800

gcaaacctgg accttgacta cagaaaggtt gcaagggcag cccttgacca caaccacgcg 1860

gtcctatcat gggccataac agacgtcaac atgcagaaaa ccctcaaccg ttacctcatg 1920

aaggagggcc ttagtagaga ggacatagtg atggacccaa caacctgtgc actgggttac 1980

ggtatagaat tttcaataga cgtcatcaca aggacaaggc tcgcggccct caagggtgat 2040

gcagaccttc agatgccaat gtcctctgga acaaccaacg cgtggggttc aagggaggcc 2100

tggatgaaga aggatgaatg gggacccaca gactacaggg gccctctatg ggagatagta 2160

accggactta caatgatgct ctcaggtgtg gatatattca tgatgctcca cccaacatct 2220

gtgaggcttc tgcgggagat aggggaaacc ttcacaaggg aatacatgac gaccgaaaca 2280

cctgatctcc gggaatggat aactgaactt gaatattgat gaagtaagta ggaagcaggg 2340

agcaggggaa agaaaattga caactgtaca agattaatcg cgtctctgag caatgaccaa 2400

atacatctac ctccacggtt ttcttccagc cccctatctg cgaaagcaca agatattagc 2460

aagcgtttcg cccaaattca catacagcta acaatccctg atctcaatgc tggtgaattt 2520

tctcagttaa caatcacgcg ccaaattcaa caagttgccg caattttccc tgataattct 2580

gaaccaataa cgctgatagg ttctagttta ggcggtttaa ctgctgctta tctaggacag 2640

cgatatttac aagtacaacg cttagtttta ttagcgccag tttggttttt tatcccattg 2700

gttgcccaaa atgggtgaag aagctgtcac aagttggcaa caaacgatat aggttctctc 2760

ttctgccgtt a 2771

<210> 173
<211> 7061
<212> DNA
<213> Artificial Sequence

<220>
<223> Synthetic Construct- SEQ ID NO: 173, example 173: a designer
      hox-promoter-controlled CO dehydrogenase /acetyl-CoA synthase DNA
      construct (7061 bp)

<400> 173
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgtcacataa tgggatccat ggtcaaggcc gggtgtccct tttcctccaa 240

gtagggcaag atttcatcca cggtggtacc gatggtctca tcagctattt tatcgataaa 300

gtcctctccc aggccctcct caacactacg acgtacaaag tcgtcgtgga ggaaatcctt 360

cagagatttg ggcatccaga cgatccgggc gataccacca tcggcggaaa taaacttttt 420

gctgacgata taggtgcggc cgatgcccat aaagcccggg gtctgggtgc caccgccgat 480

catcccggcc agggtagaga aggtcatccc cgaaggagtc atgccggcgt gatccctggt 540

ggtaatcatg atgccgttgc actccggcag gatggccata atggcctcaa agcaaccgca 600

ggaggtcatg ggattctcca taagggtgta caggcagacc tgttccaggt tacggttgga 660

agctgtatag agatagtcat ttacactctt ccagataccc ttaatgggat caatttcccc 720

ttctttaggg atgggctggt taggaccggc atggttgatt tcataggacg ccttggcgtc 780

cagccagctt acggctccac acaggcccac ccgttccggg gtgacaatac acacatggtt 840

gggggcaaag gactggcaga ggacgcagga gtaaaaggta tccactgttt catcggtaag 900

gccgcgcatg cggtcgtcac gttccttgta tttctcccgg gcgacctcca tatattcttt 960

gaccttggct tcatcggtaa aaatggttac ctggacccgg tccacaatgg cggggaattc 1020

ttctttcatt ttggctacca ggatttcacc gtagttcttg aaacggaaac ccttggctac 1080

ggcatctttg ctgacccgca accagttgat gttacgctgg ccggtgtgcc agagaccttc 1140

accgtagttg atgaagtcgt ggatgcgccg ttcgaggact ccttcaaaat cggcctgcat 1200

tttacggcca tagatgtcca ccagaatgcc caggggcagt ttgctccctt ccggtatctg 1260

gtcaatatca ggacctatga cttcaatctt accatcagtg atctcggatt ccgaaacggt 1320

gcgtaccagc tcaaaggccg gcgtccggtt gccgcccatt tctacgtaca tatcgccctt 1380

acggatactc tcgccctcaa aggcagggcc aaagttaatg ggcagatcca acttgatctt 1440

ggtgagcttg atcccacggg tctccatggc tatctggact attttatcat agtcctcaac 1500

gctgaagaac caatccggga tctgtttgtc ctccggtagg ggctggtcgg tgattaccgg 1560

aaagccggta aagatggccc cgaaggcggc agccgtcttg accatgtcat gctcgccgag 1620

ataaaggaca aaggcgcgga tacggcggcg ctggtaatcg cgctgttctt cacgggcacc 1680

cggggtaacg ccaccgaaca tcataccagc ccgcagggca tagttggcgg catgaacaat 1740

ctgggtgaag ttccccaggg gataggcgat atagtcaatc cccagtttga cgttttcttc 1800

cagcagctgt tctaccgctt catcacagat gaagagcata aagcccatac ccatgagttc 1860

cttgacgatt ttggccaggg ccttcgagtc tttggctcga cccaggataa tagcttcacc 1920

cggaatggtc cagtcgacca tcttgatacc gaaacggcgg acaaccgggt cgccgatgaa 1980

acccgtccag ggtgggggca ggaggggttc atcaggctta tatttaaggt aacgcagggc 2040

ttcaatgatc tcggccgcat accaggtggc ttccccggcc aggcgggcat tctcgaaatt 2100

caggacaggg cttacctgcg ctcgcttgcg gtttaaaata ggtggtaaat cccccagttt 2160

tttgacctct tccccgctga aacagcgaat aaccggcagg taataggctg tatcaggata 2220

accgacggga tggtcgggac cataggtccg gatggcctgg tttaaaagga tttccgcgta 2280

actggtagct gtaatggcgc cgtggtaaac ctcccggaac agggctaccg gctctttacc 2340

ttctggaata gcaccctcga agattttatc aaaatcagtc atgtggcacc cgaatcaaaa 2400

aaagccaaga accttaaagg ggatttctgg gaccttaaaa atatccagat atccatagga 2460

gaaattataa ctgaggaaaa acctcaggag gaagaggcta ggggtccaaa accccgcccc 2520

catgttaccg acctcaggtc atgggacatg aagctccttg aaagatacga accattctat 2580

gcacctttct gtgacatgtg ctgcctctgc acctacggta aatgcgacct cctcggtaag 2640

aagggagcat gtggtataga cgcagccacc cagcaggcac gcatagtact tctcgcatgt 2700

ctcataggga ccgccgccca tgcgggacac gcaaggcacc ttgtggacca cctcatagag 2760

aaactgggag aggactatga aatagacttg ggcatgaatg tggatataga ggcacccata 2820

acaaggaccg ttatgggaag gaggccgcgg accctgggcg acctcatgga ggttatggac 2880

tacgccgagg aacagatgtc acacctcctc tcggcatgcc acacgggaca ggagggcgac 2940

agcagggact ttgaatcgaa ggcattccat gctggactca tggatgacct cacgagggag 3000

gttgcggata tcgcccagat agttgccctg gatctcccta agggtgatga ggacgcaccc 3060

ctcgttgaac tgggattcgg tacaatagac aacaccaaac ccgtgatact ctgcataggc 3120

cacaacgtcc tccctggagc ggatatagtg gactacgtca ccgagaggga actcgaagac 3180

gaagttgagg tatgcggcat atgctgtgca gcaatagatg ttacaaggta cagcgaggct 3240

gccaaggtcg tcgggcccct ctcaaagcag ctcagattca taagaagtgg cgtggccgat 3300

gtgatagtgg tggacgagca gtgcgtgagg acagacgtgc tggaggaggc cctcaaaaac 3360

aggtcagcgg tgattgcaac aaccgataag atgtgcctgg gactacctga tgtgacagat 3420

gaggaccccg acaaaatcgt gaatgacctc ataaacggta acatagaggg ggcactaatc 3480

cttgaccccc ataaggttgg tgaggtggca gttaaaactg caataaaact ggcaccaatc 3540

aggaaatccc tcaaaaaatt gccagaggtt gatgagataa tccggatggc atcagagtgc 3600

acagattgcg gctggtgcca gagggtctgc cccaacagcc tccctgtcat ggatgcagtt 3660

aagagcgcgg ccgagggtga tctgagcaaa cttgaggaga tggcacttga ggagctctgc 3720

tacacctgtg gacgctgcga acaggagtgt gaaagggaca tacctatagt atccatggtg 3780

acaaaggcag gtgaaaggcg cgtaaaggac gaaaaataca agataagggc tggccgtggc 3840

cctgcccagg atgtggagat aaggagggtc ggcgcaccca tacccctcgg ggacataccc 3900

ggtgtcatag cctttgtggg ttgctcaaac taccctgagg gtggaaagga tgttgccctc 3960

atggcaaagg agttccttga gagaaactac atcgtggtca caacgggatg cggtgcaatg 4020

tccatcgggg aatacaggga cgaggatgga aagacactct atgaaaaata cggtggacag 4080

ttcgatgcaa agggacttgt taacatgggc tcctgcgtat caaacgccca tatatcaggt 4140

gccgccataa agatagccaa catattcgca cagaaaccac tcgaggggaa ctttgaggag 4200

atagcggatt acatacttaa ccgtgtaggg gcatgtggtg tcgcatgggg tgcctactcc 4260

cagaaggcgg cggccatagc aaccggtgtg aaccggtggg gaatacccgt cgtcctggga 4320

ccccacggat cgaagtaccg cagactgttc cttggaaggg cagatgaccc agagaagtgg 4380

aaactgaacg acctcagaac aggtgaggtc atcgatgggg agccagctcc agaacacctt 4440

ctctatgcag cggagaaccg ggaggaagca acggtgatgg tggccaaact ctgcataagg 4500

ccaacagaca cacccaaggg tcgccagatg aaactcagca actacataga cctccacaaa 4560

aaatactttg gcacaattcc agatgacatc gacaggttca taaggacaga gaaggacatc 4620

cccatagtct acaagaggga tatcctgaag atactggagg agaagaactg gaagccaagg 4680

aagcttccaa aggaaccctc actccttgag aggtgaatgt ttgaagacat acccgttgat 4740

gtaagtccca tgcacgaggg ggagcggata agatcagcaa acatgttcgt tgaacttgcc 4800

gggcccaaat ccatcggagc cgaactggtc caggttaagg atagcgttga ggatggaaag 4860

gtggaggtca tagggcccga gataagtgag atggaacagg gccagatcta tccattcgcc 4920

ataaacgtgg agatagcagg aagcgaactc gaggaggaac ttgaaagcgt tatagagcgg 4980

agactccatg aactctgcaa ctacgtccag ggtttcatgc acctcaacca gagggaccag 5040

atatggtgcc gtgtaagcac agaggcaagg gacgcaggat tcatgcttga acaccttgga 5100

aaggcactat cagtcctctt cagggaggag ttcccaataa tcgaatcaat atcagtgaca 5160

atcatgaccg atgaggacgc tgttaaagaa ttccttgaaa cagccaggga aaagtatgag 5220

ataagggatt caagggcaag ggaactctca gacgaggacg ttgacgtatt ctacgggtgc 5280

ctcatgtgcc agtcattcgc cccaacacat gtatgcatcg tcacaccaga ccggacagcc 5340

ctatgtggtg ccataaactg gttcgactgc cgcgcagcct ataagatgga ccccgacggt 5400

ccaatattcg aaattcaaaa gggagatgta attgacccgg agaaggagaa atatgagaat 5460

gttaacgctg cggtggcaga gaactcacag ggtacaacag agaggtttta cctccacagc 5520

gtgttcgggt atccacacac atcatgtggc tgcttcgagg cagtggcatt ctacatacca 5580

gaactggatg gcataggcat cgtgaacagg gacttcaggg gcgaaacacc ccttggaata 5640

ccattctctg caatggcagg gcagtgctca ggcggaaaac aggttgaagg gttttcaggt 5700

ctcagccttg agtacatgag gtcacccaag ttcctgcagg cagatggtgg gtactcaagg 5760

ataatctgga tgccaaagga tctgaaggaa tctgtcattg acttcatccc tgaagacctg 5820

agggataaga tagccacaga ggtggatgca acatcaataa aggaactcag aaggttcctt 5880

agggagaagg agcaccctgt ccttgaaagg gcaccagcag aagttgagga agctgaagtc 5940

gttgaggaag aggaaaccca tgctgaggag gcccccgtca ctgagggaat acctgtaatg 6000

gcgtccccag agatcagcct tccagcagct ggcggattca ggataattct caagaacgca 6060

aagatctacg ctgagaaggt aataataaag cgtaaataac tactgctcca gggcatctat 6120

gagttcatcg agccatctga gccattctgt gtacttcatg ctggggtatg agatgtctgc 6180

atttggatgc atgtacctgc agagggtcac tgttttaagg tgtggtgcaa aggccttgag 6240

ggttgaaagg ccctgtgaac ctatgtaata tgggacacct atgaagcata caaggtcata 6300

gttgccattt ccatcgaagc cctcccagtc aggatttttg aggttgttta caagttcgat 6360

tacccctatt atgttttttc tttccagttt agttaacctg agacctgatc ctccggttag 6420

ggcaacctcc attttccctt tttttattat tttaactatc ctatccacta tatcttgtgg 6480

aaggtcattt aacagcgacc ctaccaccat gagtggtctt tcagactttt ttatggccga 6540

tgcaaccatt tcagggggca tgacggttgc attccttatt gatgttagag ctgggttcca 6600

tgcggtcatt gaagtaagta ggaagcaggg agcaggggaa agaaaattga caactgtaca 6660

agattaatcg cgtctctgag caatgaccaa atacatctac ctccacggtt ttcttccagc 6720

cccctatctg cgaaagcaca agatattagc aagcgtttcg cccaaattca catacagcta 6780

acaatccctg atctcaatgc tggtgaattt tctcagttaa caatcacgcg ccaaattcaa 6840

caagttgccg caattttccc tgataattct gaaccaataa cgctgatagg ttctagttta 6900

ggcggtttaa ctgctgctta tctaggacag cgatatttac aagtacaacg cttagtttta 6960

ttagcgccag tttggttttt tatcccattg gttgcccaaa atgggtgaag aagctgtcac 7020

aagttggcaa caaacgatat aggttctctc ttctgccgtt a 7061

<210> 174
<211> 1847
<212> DNA
<213> Artificial Sequence

<220>
<223> Synthetic Construct- SEQ ID NO: 174, example 174 of a designer
      hox-promoter-controlled Thiolase (07) DNA construct (1847 bp)

<400> 174
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgggcaa agaaagtagt tttagctgtg catgtcgtac agccatcgga 240

acaatgggtg gatctcttag cacaattcct gcagtagatt taggtgctat cgttatcaaa 300

gaggctctta accgcgcagg tgttaaacct gaagatgttg atcacgtata catgggatgc 360

gttattcagg caggacaggg acagaacgtt gctcgtcagg cttctatcaa ggctggtctt 420

cctgtagaag tacctgcagt tacaactaac gttgtatgtg gttcaggtct taactgtgtt 480

aaccaggcag ctcagatgat catggctgga gatgctgata tcgttgttgc cggtggtatg 540

gaaaacatgt cacttgcacc atttgcactt cctaatggcc gttacggata tcgtatgatg 600

tggccaagcc agagccaggg tggtcttgta gacactatgg ttaaggatgc tctttgggat 660

gctttcaatg attatcatat gatccagaca gcagacaaca tctgcacaga gtggggtctt 720

acacgtgaag agctcgatga gtttgcagct aagagccaga acaaggcttg tgcagcaatc 780

gaagctggcg cattcaagga tgagatcgtt cctgtagaga tcaagaagaa gaaagagaca 840

gttatcttcg atacagatga aggcccaaga cagggtgtta cacctgaatc tctttcaaag 900

cttcgtccta tcaacaagga tggattcgtt acagctggta acgcttcagg tatcaacgac 960

ggtgctgcag cactcgtagt tatgtctgaa gagaaggcta aggagctcgg cgttaagcct 1020

atggctacat tcgtagctgg agcacttgct ggtgttcgtc ctgaagttat gggtatcggt 1080

cctgtagcag ctactcagaa ggctatgaag aaggctggta tcgagaacgt atctgagttc 1140

gatatcatcg aggctaacga agcattcgca gctcagtctg tagcagttgg taaggatctt 1200

ggaatcgacg tccacaagca gctcaatcct aacggtggtg ctatcgctct tggacaccca 1260

gttggagctt caggtgctcg tatccttgtt acacttcttc acgagatgca gaagaaagac 1320

gctaagaagg gtcttgctac actttgcatc ggtggcggta tgggatgcgc tactatcgtt 1380

gagaagtacg aataatgaag taagtaggaa gcagggagca ggggaaagaa aattgacaac 1440

tgtacaagat taatcgcgtc tctgagcaat gaccaaatac atctacctcc acggttttct 1500

tccagccccc tatctgcgaa agcacaagat attagcaagc gtttcgccca aattcacata 1560

cagctaacaa tccctgatct caatgctggt gaattttctc agttaacaat cacgcgccaa 1620

attcaacaag ttgccgcaat tttccctgat aattctgaac caataacgct gataggttct 1680

agtttaggcg gtttaactgc tgcttatcta ggacagcgat atttacaagt acaacgctta 1740

gttttattag cgccagtttg gttttttatc ccattggttg cccaaaatgg gtgaagaagc 1800

tgtcacaagt tggcaacaaa cgatataggt tctctcttct gccgtta 1847

<210> 175
<211> 1514
<212> DNA
<213> Artificial Sequence

<220>
<223> Synthetic Construct- SEQ ID NO: 175, example 175: a designer
      hox-promoter-controlled 3-Hydroxybutyryl-CoA dehydrogenase DNA
      construct (1514 bp)

<400> 175
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgcaaaa gatttgtgta ataggtgctg gaacaatggg ctcaggcatc 240

gctcaagtat ttgcacaaaa tggctttgaa gtaattttac gcgatattga tatgaagttc 300

gtagaaaaag gatttggcac aattgaaaaa atttacaaag aaatgttgac aaaggaaaaa 360

ttacagcaga tgagaaaacg aattttaagc agaatcagag gtacaacaaa tttggaagac 420

gcaaaagaag cagattttgt agttgaagcg gctatagaaa atatggatct caagaaacaa 480

atattcaaag agctagatga aatatgcaaa atggaaacaa tccttgcgtc aaatacatca 540

tcactatcca taacagaaat agcaagtgcg acaaaaagac ctgagaaagt cataggaatg 600

catttcttca acccagttcc agtaatgaaa cttgttgaag tcataaaagg attaaagaca 660

tcagagcaaa catttaatgt cgtcagagaa ttggctttaa aagtagacaa aacacctata 720

gaggtcaaag aagcacctgg atttgttgta aataggattt taatcccaat gattaatgaa 780

gcaattggaa tacttgcagt ggtgttggca actgacaaga gcatagatga agctatgaaa 840

cttggtgcaa atcatccaat aggacctttg gcattgtcta gtttgatagg caatgacgtc 900

gttcttgcta taatgaatgt gctttatgaa gagtacggcg attcgaaata cagaccacat 960

ccacttctaa aaaaagtggt aagaggcgga ttgctgggta gaaaaactgg caaaggtttc 1020

tttgaataca aaattaatct tttaaggagg agaatatcat gatgaagtaa gtaggaagca 1080

gggagcaggg gaaagaaaat tgacaactgt acaagattaa tcgcgtctct gagcaatgac 1140

caaatacatc tacctccacg gttttcttcc agccccctat ctgcgaaagc acaagatatt 1200

agcaagcgtt tcgcccaaat tcacatacag ctaacaatcc ctgatctcaa tgctggtgaa 1260

ttttctcagt taacaatcac gcgccaaatt caacaagttg ccgcaatttt ccctgataat 1320

tctgaaccaa taacgctgat aggttctagt ttaggcggtt taactgctgc ttatctagga 1380

cagcgatatt tacaagtaca acgcttagtt ttattagcgc cagtttggtt ttttatccca 1440

ttggttgccc aaaatgggtg aagaagctgt cacaagttgg caacaaacga tataggttct 1500

ctcttctgcc gtta 1514

<210> 176
<211> 1430
<212> DNA
<213> Artificial Sequence

<220>
<223> Synthetic Construct- SEQ ID NO: 176, example 176: a designer
 hox-promoter-controlled Crotonase DNA construct (1430 bp)

<400> 176
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggaatt aaaaaatgtt attcttgaaa aagaagggca tttagctatt 240

gttacaatca atagaccaaa ggcattaaat gcattgaatt cagaaacact aaaagattta 300

aatgttgttt tagatgattt agaagcagac aacaatgtgt atgcagttat agttactggt 360

gctggtgaga aatcttttgt tgctggagca gatatttcag aaatgaaaga tcttaatgaa 420

gaacaaggta aagaatttgg tattttagga aataatgtct tcagaagatt agaaaaattg 480

gataagccag ttatcgcagc tatatcagga tttgctcttg gtggtggatg tgaacttgct 540

atgtcatgtg acataagaat agcttcagtt aaagctaaat ttggtcaacc agaagcagga 600

cttggaataa ctccaggatt tggtggaact caaagattag caagaatagt tggaccagga 660

aaagctaaag aattaattta tacttgtgac cttataaatg cagaagaagc ttatagaata 720

ggcttagtta ataaagtagt tgaattagaa aaattgatgg aagaagcaaa agcaatggct 780

aacaagattg cagctaatgc tccaaaagca gttgcatatt gtaaagatgc tatagacaga 840

ggaatgcaag ttgatataga tgcagctata ttaatagaag cagaagactt tgggaagtgc 900

tttgcaacag aagatcaaac agaaggaatg actgcgttct tagaaagaag agcagaaaag 960

aattttcaaa ataaataatg aagtaagtag gaagcaggga gcaggggaaa gaaaattgac 1020

aactgtacaa gattaatcgc gtctctgagc aatgaccaaa tacatctacc tccacggttt 1080

tcttccagcc ccctatctgc gaaagcacaa gatattagca agcgtttcgc ccaaattcac 1140

atacagctaa caatccctga tctcaatgct ggtgaatttt ctcagttaac aatcacgcgc 1200

caaattcaac aagttgccgc aattttccct gataattctg aaccaataac gctgataggt 1260

tctagtttag gcggtttaac tgctgcttat ctaggacagc gatatttaca agtacaacgc 1320

ttagttttat tagcgccagt ttggtttttt atcccattgg ttgcccaaaa tgggtgaaga 1380

agctgtcaca agttggcaac aaacgatata ggttctctct tctgccgtta 1430

<210> 177
<211> 1784
<212> DNA
<213> Artificial Sequence

<220>
<223> Synthetic Construct- SEQ ID NO: 177, example 177: designer
      hox-promoter-controlled Butyryl-CoA dehydrogenase DNA construct
      (1784 bp)

<400> 177
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgaattt ccaattaact agagaacaac aattagtaca acaaatggtt 240

agagaattcg cagtaaatga agttaagcca atagctgctg aaatcgacga aacagaaaga 300

ttccctatgg aaaacgttga aaaaatggct aagcttaaaa tgatgggtat cccattttct 360

aaagaatttg gtggagcagg cggagatgtt ctttcatata taatagctgt ggaagaatta 420

tcaaaagttt gtggtactac aggagttatt ctttcagcgc atacatcatt atgtgcatca 480

gtaattaatg aaaatggaac taacgaacaa agagcaaaat atttacctga tctttgcagc 540

ggtaaaaaga tcggtgcttt cggattaact gaaccaggtg ctggtacaga tgctgcagga 600

caacaaacaa ctgctgtatt agaaggggat cattatgtat taaatggttc aaaaatcttc 660

ataacaaatg gtggagttgc tgaaactttc ataatatttg ctatgacaga taagagtcaa 720

ggaacaaaag gaatttctgc attcatagta gaaaagtcat tcccaggatt ctcaatagga 780

aaattagaaa ataagatggg gatcagagca tcttcaacta ctgagttagt tatggaaaac 840

tgcatagtac caaaagaaaa cctacttagc aaagaaggta agggatttgg tatagcaatg 900

aaaactcttg atggaggaag aattggtata gctgctcaag ctttaggtat tgcagaagga 960

gcttttgaag aagctgttaa ctatatgaaa gaaagaaaac aatttggtaa accattatca 1020

gcattccaag gattacaatg gtatatagct gaaatggatg ttaaaatcca agctgctaaa 1080

tacttagtat acctagctgc aacaaagaag caagctggtg agccttactc agtagatgct 1140

gcaagagcta aattatttgc tgcagatgtt gcaatggaag ttacaactaa agcagttcaa 1200

atctttggtg gatatggtta cactaaagaa tacccagtag aaagaatgat gagagatgct 1260

aaaatatgcg aaatctacga aggaacttca gaagttcaaa agatggttat cgcaggaagc 1320

attttaagat agtgaagtaa gtaggaagca gggagcaggg gaaagaaaat tgacaactgt 1380

acaagattaa tcgcgtctct gagcaatgac caaatacatc tacctccacg gttttcttcc 1440

agccccctat ctgcgaaagc acaagatatt agcaagcgtt tcgcccaaat tcacatacag 1500

ctaacaatcc ctgatctcaa tgctggtgaa ttttctcagt taacaatcac gcgccaaatt 1560

caacaagttg ccgcaatttt ccctgataat tctgaaccaa taacgctgat aggttctagt 1620

ttaggcggtt taactgctgc ttatctagga cagcgatatt tacaagtaca acgcttagtt 1680

ttattagcgc cagtttggtt ttttatccca ttggttgccc aaaatgggtg aagaagctgt 1740

cacaagttgg caacaaacga tataggttct ctcttctgcc gtta 1784

<210> 178
<211> 2051
<212> DNA
<213> Artificial Sequence

<220>
<223> Synthetic Construct- SEQ ID NO: 178, example 178, a designer
hox-promoter-controlled Butyraldehyde dehydrogenase DNA construct
(2051 bp)

<400> 178
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgattaa agacacgcta gtttctataa caaaagattt aaaattaaaa 240

acaaatgttg aaaatgccaa tctaaagaac tacaaggatg attcttcatg tttcggagtt 300

ttcgaaaatg ttgaaaatgc tataagcaat gccgtacacg cacaaaagat attatccctt 360

cattatacaa aagaacaaag agaaaaaatc ataactgaga taagaaaggc cgcattagaa 420

aataaagaga ttctagctac aatgattctt gaagaaacac atatgggaag atatgaagat 480

aaaatattaa agcatgaatt agtagctaaa tacactcctg ggacagaaga tttaactact 540

actgcttggt caggagataa cgggcttaca gttgtagaaa tgtctccata tggcgttata 600

ggtgcaataa ctccttctac gaatccaact gaaactgtaa tatgtaatag tataggcatg 660

atagctgctg gaaatactgt ggtatttaac ggacatccag gcgctaaaaa atgtgttgct 720

tttgctgtcg aaatgataaa taaagctatt atttcatgtg gtggtcctga gaatttagta 780

acaactataa aaaatccaac tatggactct ctagatgcaa ttattaagca cccttcaata 840

aaactacttt gcggaactgg agggccagga atggtaaaaa ccctcttaaa ttctggtaag 900

aaagctatag gtgctggtgc tggaaatcca ccagttattg tagatgatac tgctgatata 960

gaaaaggctg gtaagagtat cattgaaggc tgttcttttg ataataattt accttgtatt 1020

gcagaaaaag aagtatttgt ttttgagaac gttgcagatg atttaatatc taacatgcta 1080

aaaaataatg ctgtaattat aaatgaagat caagtatcaa agttaataga tttagtatta 1140

caaaaaaata atgaaactca agaatactct ataaataaga aatgggtcgg aaaagatgca 1200

aaattattct tagatgaaat agatgttgag tctccttcaa gtgttaaatg cataatctgc 1260

gaagtaagtg caaggcatcc atttgttatg acagaactca tgatgccaat attaccaatt 1320

gtaagagtta aagatataga tgaagctatt gaatatgcaa aaatagcaga acaaaataga 1380

aaacatagtg cctatattta ttcaaaaaat atagacaacc taaataggtt tgaaagagaa 1440

atcgatacta ctatctttgt aaagaatgct aaatcttttg ccggtgttgg ttatgaagca 1500

gaaggcttta caactttcac tattgctgga tccactggtg aaggaataac ttctgcaaga 1560

aattttacaa gacaaagaag atgtgtactc gccggttaat gaagtaagta ggaagcaggg 1620

agcaggggaa agaaaattga caactgtaca agattaatcg cgtctctgag caatgaccaa 1680

atacatctac ctccacggtt ttcttccagc cccctatctg cgaaagcaca agatattagc 1740

aagcgtttcg cccaaattca catacagcta acaatccctg atctcaatgc tggtgaattt 1800

tctcagttaa caatcacgcg ccaaattcaa caagttgccg caattttccc tgataattct 1860

gaaccaataa cgctgatagg ttctagttta ggcggtttaa ctgctgctta tctaggacag 1920

cgatatttac aagtacaacg cttagtttta ttagcgccag tttggttttt tatcccattg 1980

gttgcccaaa atgggtgaag aagctgtcac aagttggcaa caaacgatat aggttctctc 2040

ttctgccgtt a 2051

<210> 179
<211> 1808
<212> DNA
<213> Artificial Sequence

<220>
<223> Synthetic Construct- SEQ ID NO: 179, example 179: a designer
hox-promoter-controlled NADH-dependent Butanol dehydrogenase DNA
construct (1808 bp)

<400> 179

agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttacaggg aagcgcgcaa aatggcgagc acatcatcgc ggttcaatgt 240

tttgaaacgg ccgaactcgc caaacgccat cgctttatcg gccatcagct ccaagttttc 300

ctcgccgatg ccatagtccg caagccgcga cggcgccccg aggctcgacc agaactcgcg 360

cagccgctcg atgccttcaa gcgccacatc ccgctccgat ttgcccgccg gatcgacatc 420

aaagacgcgc accgcgagct gggcgaaacg gctgacattt tcatcgagca catgcttcat 480

ccagttcggg aacaaaatcg ccaatccccc ggcgtgcggg atgtcataca cggcggaaac 540

ggcatgctca atattgtgcg tcgcccagtc gccgcggacg cccatttgca agaagccgtt 600

taaggcgatc gtgcccgagt acatgatcgt ctcgcgcagc tcgtagtttt ccaaatcgtt 660

gatcagtttg ggcgccgttt cgatcacggt tttcaacacc gcttcacaca tccggtcttg 720

caatggcgtg ttcggcgtat ggtggaaata ttgctcaaac acatgcgaca tcatatcgac 780

aatgccgtaa accgtatggt ctttcggaac ggtcatcgta tacgtcggat ccaaaatcga 840

aaattgcggg aacgtaaacg ggctgcccca gccgtatttt tctttcgtct cccagtttgt 900

gatcaccgaa ccagaattca tctccgaccc ggtcgccgcc agcgtcaaga cgacgccaaa 960

cggcagggcg ccggtgacgg gcgctttttt cgtgataaac tcccacggat cgccatcgaa 1020

tttcgccccg gcggcgatcg ctttcgtgca gtcgatgacg ctgccgccgc caaccgccag 1080

caaaaattcg acgccttccc gcttgcaaat gtccacccct tttcttacgg tcgagacgcg 1140

cgggttcggt tcgacgcctg gcagttcgat gacctcggcg ccaatattcc ccaatatctt 1200

catgacttcc tcatacaatc cgtttcgttt gatgctgccg ccgccataca cgagcagcac 1260

tttcttgccg tagcgcggca cttcttcttt gagacgttcg agctgccctt ttccgaaaat 1320

cagtttcgtc gggttgcgga aaataaactc ttgcattgaa gtaagtagga agcagggagc 1380

aggggaaaga aaattgacaa ctgtacaaga ttaatcgcgt ctctgagcaa tgaccaaata 1440

catctacctc cacggttttc ttccagcccc ctatctgcga aagcacaaga tattagcaag 1500

cgtttcgccc aaattcacat acagctaaca atccctgatc tcaatgctgg tgaattttct 1560

cagttaacaa tcacgcgcca aattcaacaa gttgccgcaa ttttccctga taattctgaa 1620

ccaataacgc tgataggttc tagtttaggc ggtttaactg ctgcttatct aggacagcga 1680

tatttacaag tacaacgctt agttttatta gcgccagttt ggttttttat cccattggtt 1740

gcccaaaatg ggtgaagaag ctgtcacaag ttggcaacaa acgatatagg ttctctcttc 1800

tgccgtta 1808

<210>  180
<211>  10538
<212>  DNA
<213>  Artificial Sequence

<220>
<223>  Synthetic Construct- SEQ ID NO: 180, example 180: a designer
       hox-promoter-controlled Energy converting heydrogenase (Ech) DNA
       construct (10538 bp)

<400>  180
agaaaatctg gcaccacacc gcagaaatat aggggctagg agttgagggt actctggttc      60

gtcaagcatt tgggatgatt tcccctcaca agttcctcaa attattctcc tataaacaat     120

agatataagg tcaaaacttg agttatgagt gctgagtaaa aaattactct ccacgcctca     180

gaggtagata tgttattcta acattttctt aatatatggc tcaagaggga taatatctcc     240

agttgaacga ggtgttaaag ctgaaactgt taaaacacgt tcttcttgat ccatactaac     300

agtgatagta ccaggtgtta atgtgatagt atttgatagt attgcaagag atactggtct     360

ttcaagatca ggtttaatct ttacaactat aggatcaaca tcgcccttga gacagcaaat     420

acttgtgtca agtattgctt taattatttc gactactaat actacgaaaa attctattcc     480

gtagattatt ctagttaaaa acatttataa atcgccccca cctaatacgt aggatacagc     540

tattgttcct acaggactta atataagtat tgcaaatgct atgtctctac agaaatctat     600

tccagtaagt gaagatacca ttgttaaaac aattgcaagt gcaattgtga aacctgctac     660

cccagctaaa gctgaaccaa ttgttcttct tgctgcaatt tttacatttg ctagcatgta     720

aacaattaga cataacatga ggaacaattc agatattgtt aaaaaatcca tttattgctc     780

cccctgttct ccacggaagt gtccacttgc tattgcatga gttgcaaatg gtgctaatat     840

aaagtatgtc attcctaaag ctggttctcc aagaagtatt aatattaaca cgccaagcat     900

atctacaata cctaacattt ctaatcttcc aaatacgaca tatttaacat cgtcatcggt     960

attacataat agtccttttg ctgtaattaa aacgaatata ccgcagatta taaatattaa    1020

ccctataatt aatccaacag gatctacatt taaagtaagt tctaatgctg cttcagtcat    1080

ttaggaatca ccttcctctt ttgatacagg ttgttctctt gttttaagaa ttgtaagagc    1140

tactaatgtt ggtacaatag ctgctcccac tacagcttgt gttaatgcta catcgggtgc    1200

taataaaact tgataaattg ctgctaaaat aaaaccagta atactagtta atacagccat    1260

tttaattaaa tctttttgaa ttaaacaaac aaatgcacct aatattgcaa ataaatataa    1320

aatccaaggt actgcatttt gtattattaa atttatatct gccatctatt cattattatc    1380

ctctccaaat cttttatcat ttaaaagaac ggatgcactt aaagtaccat gtcttttata    1440

taatactact gtcattgcta acattacagc aagtgtactt gctccaatta caatactagt    1500

aagtactaat gcaaatggta atggatatga tgcttgacct aagaagttat ttacttccat    1560

attttgtaaa aagatatatg taattccgtt aactttgtaa cctaatgaaa ttaataataa 1620
atttacacca tcacctataa atgcagcccc aataattttc ttaacaacat tatctagata 1680
aactaatgct actgtaccca taatcattaa aagtgctgct gtaattaaag ctcctaattg 1740
tactgataac atttacatta ttaaagcgga aaaagccatg actccaagtc tatttactac 1800
taaatcagga cataaaccta taattaaaca aataattaag aagaatacca ttattgccat 1860
agttgtttta ggaacttttg cagaagatac ttctaaatca tctggttttg gtctaaggaa 1920
cattgcatat gtgattttta agaatgctaa gaaagttaca atacttaata taatcataat 1980
tattgcaagt tctggtattc ctgcacttaa tgcagcttga cataacatgt atttactttg 2040
gaatacattg aatggaggta ctccagccat tataaatcct gcaagtacaa ttaataatgc 2100
tgctacaggc atatgttcta ataatccacc aagtttactg attttacttg ttcttgtttt 2160
gtataatact aaaccaaatc ctacaaacaa gaatgaagtt actactaatt catttactgc 2220
ttggaataat cctgctgtta tactgtatgg agttcctaat cctagtccta caccaatata 2280
acctaattca ccaacagcta agtatgcgat gatacgtttg taatcatctt gcaccatagc 2340
catacttata cctaaaatca ttgcaagtac agatactgca agtattgcca tttgtactga 2400
tggtaaatat ccaaatattc taattaatac taaacctatg gatatacaac ctattacaga 2460
gaatgattgt aataatactg atccactagg taaaccttta ctgtaaattt ctgatttaat 2520
tgcattgaat ggtggtaatc ctgttgcata taaccaacca taaattaaca tacctgctgc 2580
aaataataat actgggtttg ttggatctac tgctccattg tgtatcatca ttacaatatc 2640
agttatgttt acgttacctg ttaatccaag taataaagca atacccaata acatgaagga 2700
accgcctatt tctccaagta acatgtattt taaaccagtt acataatttc ctcttactct 2760
tgatacgagt attaaaccta cttgaacaag agctgctatt tcaaagaata catataagtg 2820
gaatatatca tctgttaata caaatgccat taatgctgct gagagcatga ataataaata 2880
taagtatact ccagatgttt tcttagtttc tgcaatggat atgaatactg cacacattgc 2940
aactatacct aatataaatg caactacttg ttgtccaggt ccaaatgcat atgtaattgc 3000
tgggtggaat aattctagaa ctgatccaga aattattgct ttagataatg caggtaattg 3060
aactgctaat ccaccagctg ctagaggttt atatcctcca aagaaatggt ttccatatgt 3120
tgctactaat ggtactattg gtaaacaaat agcagtaata actgccaata cttttgttat 3180
tttttcagaa ccatgtaata ggttaactaa tatagcacaa attattggta taataaccat 3240
taatggtaaa aagatagttc catccatcta tattcccccc cagtagaata ctattgatat 3300
tattgctaat gttaaaacag cataaaacat catattattt atatcatcgt tgtgttcttt 3360
gtacaattta ggcatgattg gtgttgcata cataaagatt atgattaagg caacttctac 3420
aattaccatc aatgcaggac ttactaatct aatagtcaaa gcagccacta ataaagatgc 3480

tattaagact aattcagcag acatgactga tgatgttaca gatccattaa ctaatgtttt 3540

cttaggatca tccccaaatg tactttttaa agtttcgaca agtttatcgt acatattcat 3600

tcactcatcg tcgtcgcctc caaggtttac ttttcctcta ccaaatacga gcatagttgt 3660

tactacagca ctaactaaga ttaatgcttc acctaaagta tcgaatcctc tgaaatcaaa 3720

tactatgttt gttaccaagt taggtgctac agatactcct aatccttgat agatatagtt 3780

gataccaggg aatattaagg aactgaagtt atacattgaa cttaaaaata ctgctgagaa 3840

tgctagaaaa gatacagcta atgctatttt tctaatagaa gatttcatct attctatttc 3900

ctcatcatca acgtagtctt ctttgtattt agttacacta tagaaaagaa ctactaatgt 3960

tgttaatcct ataaatactt ttaatcctac cactatgttt aaatatggaa ttattccagc 4020

atgtaatgca tctgggaagt taaatatagc ttctacacca gatggtacta atccatatat 4080

gtttgttcct aagttataaa ggaatgaacc acctgtaaat aatccacata aaccaaggaa 4140

tacataacct aaagctccaa agctttctat tatggacatt ctttcgtgtg aaaaattaaa 4200

tggattttct tttaaaccat atgcaagaat acagagaata gctcctgaag ccataatagc 4260

tcctccctgg aaacctcctc caggtgtaat gtgtccacca agaatagtca tagcaccata 4320

acctattaat ataaaggata atgggtatga tattaattta agaagatcac tcatttaagc 4380

ctcactttta gttcctaata atcctcttcc gactattgca ccaataatag caactacaaa 4440

tcctggtcct aatggtaaac tttcatagta tgggaaagtt ccaaaccagt atgctatgat 4500

gattcctgca attattatag caatccatga ggaagtattg agttttatac cttttgttgg 4560

tgttttatgc ataataactc ctgctaggaa acctataata aaaccaacta ttgttggtcc 4620

agtatataag actccaaaaa ggttaggagc cattgattgt aaaaacggta ctgtactttc 4680

tataaccatt catattgtag ccccaaagtc tctttgtatt tttattgctc tagcaggaca 4740

tgctgtctta caagctccac aataaataca tctgttgtta tcaattttta agttaccttc 4800

ttcatcagga gcaattgctt tgaaaacaca tgcatctata catactccac aacctacaca 4860

taagttattg ttgatcatgg aatatccact ttgtattttc attttaagtg gtgatgtttt 4920

aggtatagca tttactggac agtaaattcc acatttttca caaagaacac atttatcaaa 4980

gttaagtttt acttctttat cttcaatagt taatgcatct tttggacaaa cttctacaca 5040

aataccacat ttaatacatt gtgccccaat ttctccattc catgaagtat ttccaaattt 5100

acgtgcacca tatttacatt caggaataca tcctccacaa cctacacata taccttcaag 5160

tgtaattttt gaatctggaa caaattctaa tacaccttgt ggacaagctg ttacacatgg 5220

agcatgttca tagtctgctg cacaatgttc ttcattatta acattattac ataatgaaca 5280

taaatctgca ctgaatctaa cttttttatt gataatttgt aaaccatttg ttggacatgc 5340

atctgcacat actttacaat caatacatcc aatcattttt gtatcgtttt caacatcata 5400

ttctggttgt tgaatttcaa gttcaaagtc tttttcaatg attgcatcgt ttggacattc 5460

aattaaacat ttgtaacata atgcacattt tttcatgtca acttgatatg ctccaccttc 5520

ttcaataggt ccaattgcat ttcttggaca aacatcaata caagtatcac attttgtaca 5580

aactcctggt tctatgtatg agaacttaat tgagtttgtt ggacagaaat atgcacatct 5640

tccacattca atacaatcat catgatttgt tcctacattg attctgttga ccatttcttt 5700

ttccatttga actggtcttt ttggtggtgc cattctagca ttatttggac atgcaggtac 5760

acatacacca caattagaac ataatcccat gatttttcca tctttaactg tgataacact 5820

tacaggacat acattcatac acattccaca taagttacat tttgttctgt ctactacata 5880

tccaccaaat ttgtttttaa agatagcttt gtttggacag actttttcac acttaccaca 5940

ggttatacaa ctaactgctt tcccattaac tactttaata gcatcagtag ggcaagcatc 6000

tacacaattt cctgagccat tacaattacc tgttgtcata aacatctaat tctcatttcc 6060

agctgcttct tgagctgcta ctttttcttt taattctcta tttttctgaa taattgaatc 6120

atcagttaaa tattgagcta tgtattggat tttcttctca gatacttcac ttggatctaa 6180

taacatactg tttacatcct tatcacattt gtttccaatg tcattaggat gaattgttgc 6240

tgctactcca aataatgcat atactggaca gaaatcgtga cattgataac aatgtacaca 6300

gttaatttca tcaatctcag gaattgctgt tttaactatg ccatcagcta tttctacagg 6360

ttctactgga accataataa tagcttttgt aggacataca ttagaacatc ctccacaacc 6420

aatacattct acttctgcaa ctttaggtga tggtttcaca ttaccattta atacatcttc 6480

acgtaattgt aaatctgttc tacattcact accaaaaatt attcttttaa ggttgttgta 6540

gattccgttg ataaaaactt ttcctaaact catttattta tcttcttttc caagtagtat 6600

tggtattact gatactactc cagctacaac atcttctggc cttactgcac atccaggtat 6660

tttagcatct actggtataa cattatctac aggtccttca atttcttcag aaggaatttc 6720

tccatgaata tttttgtata ctccaccagt taaagcacat gctcctactg caacaactaa 6780

tttaggttta ggtattgctt cgtaaatttt aagaagtggc tctttagtcc agtaagttac 6840

aggaccagat actactaaaa catcagcttc ccttggattc catgttaaat acacattgta 6900

ttgttctata tcgaatttag gagacaatac tgtgttaact atttctatat cgcaaccgtt 6960

acatccacca gtataaacta acataaggtg tattgccttt ttacgggctt gtgattttaa 7020

tcccatctat aatttaatta tttgtatagc tctatcagta caagtgaaac atggatcaca 7080

tgatacaata gcaagttgtg catcctgaat tgggtgtcca atacaagatt cttgcattgc 7140

accaatgtta gacattgatg gagttcttat aatactatgt cttacttttc catcctctaa 7200

accatatgaa tgataacata ctccacgagg tacttctata taacttttat aaactggtga 7260

gtctactaaa tcccaatctc ttgttacaat aggaccttta ggtaagtttt taataacttg 7320

tctaatgatc tttgttgatt caaagatttc agttgctctc ataagaatat tagcccttac 7380

atctccacca tcttgagtga taatatcaaa ttcaaaaggt tcatattcaa acatttcagt 7440

tctaaaatca tattcatatc ctgttgctct taaagatggg ccagttacat gtaaatcaag 7500

tgcttgtttc tgtgataatt caccagtacc agtaatacgg tgcattacca ttggatcagc 7560

tatgaatctt tcagcaaaat ctgctacttt ttcatcgata taatccattg tgtcaagtac 7620

tctttgttgt tctttaagat ttaagtcagc tctaggtctt actccaccaa gaactgatat 7680

tccatattga actctgtttc cacccatcat atataaaaga tccattacag attctcttat 7740

ataaaatatt ctcatagcaa aggtttcatg acataatacc tcagatccat gacctaagta 7800

taacatatga ctatgtaatc tttctaattc ttctgcaaga acacgtaaat atactgctct 7860

ttctggaatt tctataccta aagctccttc tcctacacga caggaattgt atatatgtcc 7920

gttagaacaa ataccacata ctttttctgt taaagcattt gctttttcta caggtagtcc 7980

ttccataatt ctttcaattc ctctatggtt taaacctaca gtaatttcag catcttttac 8040

tatttcatct tctacaaata gtctaactct gtaaggttct aaagctgccg gatgtactgt 8100

acccatattg atttctgttt caaaaacttg tctttctgtt ttgtcacaag ttgttgctcc 8160

atctattttt gtaaccattt ataatacagc aattaaaact gctactacag caacagcact 8220

tgttgctata actgtaggga aaaactcacg gttagtgaat aatggtgaga atgcacttac 8280

tacagctgca gctattgtta ttattataga acatattatt ggtacaatta ttgagatacc 8340

aaatactggt ggcataccta agaataatgt tgaatataca caagctccaa caaatattaa 8400

gaatgcatgt gccattacat atactgctct gtattttgac atgtattcaa gtaaaggacc 8460

ttgaattaca tctgaatgac cttccatgaa tgcaaatgga ttagagttaa gtacaattaa 8520

gaatcctaca aagaacacaa ttgttcctat gatacctgga agtgtgaata atattggacc 8580

tgtaattaat tgatatctta caatgtctat taaattcaaa cttttagcca ttattgcagg 8640

aataaataaa gctatataaa atggtaaact accataagat atgagtttta atgatctttt 8700

tgcacttaat tgttctataa atgattgttt tgcaccaata tgttttccac cttttgcttg 8760

atcagggaaa ggcatggttt ttgataaaag tgattgtgag aatgaactca taaataagta 8820

taaaacttct tccactttta ataaaccaac taatgctact aaactagcaa aagatcccca 8880

agctaccatt acttgtggta tgagtatgag gaatattgct gctactataa tcaaagttat 8940

taaaggtaat atattatata ctctaggcaa tattgcagat ggttctaata cttgtttaaa 9000

gaagaatttt aatgttgcat agaaaccagg acttgaaagt tgaggaccaa gtctttgttg 9060

tactcttgcc tctgctttct tttcaatacc cggtaaccat aaacatacaa tacttgcaat 9120

tataaaagca cctataacat agcctattga tgttactata tccatttaat tactttctat 9180

agcttttctt tcttcatcta atttatgttt agccatttga tacattacta aaaagtcatc 9240

atcatcatta actggaatta cttccaatcc taatgcttca tgttcttcaa tccattcatc 9300

attaagaaca ggaacttcta gtttaattgg ttcatcttca tgattaacta caattaaatt 9360

tctgaaagat gttcttactt ctacaatata acctgctaaa cctattatat gatgtggtct 9420

taatactatt ttcatctaga gtacttcatc aactctatca atatcaaatt tagcagtttt 9480

tgctattgac attacagcca taacacctgc ttgaactgga tctgttacaa ctaaatcagc 9540

atggtctctt acagaaccaa ccatattaag acttattact ttaactccat atttattttt 9600

tatttcatca atagcatcca caatagatcc acccattaat gatccagcaa gtactaaaca 9660

acccactcta ggtaaacatc caacggcacg tacagcttca gttaattttt tttctccagc 9720

cagtggtaat gtatcaacac ttatacgttc accacgaata ttatgacgat ctgcttcggt 9780

aattgcacct aatgctactt gagatacttg tgctccacca ccaactatga tgattctttt 9840

accccatact ttatccatag aaggagatag ttttacttct ttgacttctg gaaattccat 9900

tatttcagaa agtacttctt caatattatc cacatgatct aattctatat atgtagaagc 9960

atgatcatca gattccatat ataaatgggt atagacaata ttaattccat tttttgcaag 10020

ataatcagtt acttttctta aaactcctgg tttatctatt gtcctcatat ttattgcatc 10080

tatcattgaa gtaagtagga agcagggagc aggggaaaga aaattgacaa ctgtacaaga 10140

ttaatcgcgt ctctgagcaa tgaccaaata catctacctc cacggttttc ttccagcccc 10200

ctatctgcga aagcacaaga tattagcaag cgtttcgccc aaattcacat acagctaaca 10260

atccctgatc tcaatgctgg tgaattttct cagttaacaa tcacgcgcca aattcaacaa 10320

gttgccgcaa ttttccctga taattctgaa ccaataacgc tgataggttc tagtttaggc 10380

ggtttaactg ctgcttatct aggacagcga tatttacaag tacaacgctt agttttatta 10440

gcgccagttt ggttttttat cccattggtt gcccaaaatg ggtgaagaag ctgtcacaag 10500

ttggcaacaa acgatatagg ttctctcttc tgccgtta 10538

<210> 181
<211> 3416
<212> DNA
<213> Artificial Sequence

<220>
<223> Synthetic Construct- SEQ ID NO: 181, example 181: a designer
      hox-promoter-controlled [NiFe]-hydrogenase MvhADG DNA construct
      (3416 bp)

<400> 181
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttagatcc tttttacaag gtcgccttca ctgtcgtata cttccaaggt 240

ggcaagtctc atctgactgt caatggtgtg tgttgcgcag gagagacatg ggtcgtatgc 300

ccttatgacc atctccatga ggttgaatat cttatcatcc acttcaacac caggtttgat 360

gtagtcctgg gcaaccttct ggatacccat ctccatggca gggttgttct ggattgttgc 420

aaccacgatg tttgccttgg ttatgaggcc gttctcatca caggtgtagt ggtgtgtgag 480

tgttcccctt ggtgcctcaa ctatacccac accgtcgcct gcctgccttt caagtgaatc 540

agggaatttt tctccggata ggtctccttc aagtgcatcg gctgcacatt cggcacatgc 600

gaggacttct atgagccttg cccagtggta caggagtgtc tgctgtgcgt atccaaattt 660

gtcctggaac tccttgaagt aatcctgtgc ctttggagct gcatcaggca tcttatctgc 720

gacgttgagc cttgagagtg gtgagacacg gtagacacca tcagggtatc caaggtcctt 780

tatgtatggg aatttgagcc aggagtatgg tttaacatgt tcagcgatgg tgtcagcgta 840

gtctgctggt ttgaactccc tgaacaggtt tccttccttg tcctttatcc tcactatacc 900

atcgtagacg tcccatacgc cgtttttaac aagacctgtg tggtatgtct cgatgttgcc 960

cagtgagttc acgaggtcaa tgttctcctc gaatattgga actgcaagtt ccagggtggc 1020

ctctgccagt tcaacgttcc tctgggcctt ctgaaggagg tccttctgtg tttcatcgtc 1080

cagttctgtt gatatacctc ctggtgttga ggaggttggg tgaattggcc ttccgcctgt 1140

ggccctgacg atttccagag catttttacg taactctatg gcctgaagag caacatcagg 1200

ggcgtccttt attatctgga agacgttcct tgtctttctg tccttacctg ctatgaagtc 1260

aggggctgca aggaagtaga agtgcagacc atgggagtgc atgtatgagc cccagttcat 1320

gatctccctc atcttgtagg ctgcaggaag gacatcgtca ggttcgaatc caaagcaggc 1380

gtcaacagcc tttgcggctg ccaggtggtg ctgcacgtca cagataccgc agatccttgg 1440

aactatcctt ggggcttcct ctatgggtct tccctggagg aacttttcga atccacggaa 1500

ttccataaca tggagccttg tgtcctcaac attacctgca tcatcaaggt gtacggtaat 1560

cttggcgtga ccttcaatac gggtcacagg ttccattgtg agtttaacca tttatgcttc 1620

agctagctgt tttttgattg gtgatggacc aagggccctt atcctgtcaa ccatcatctt 1680

gacggtttcg gcgaattttt caccttctga tgcggatatc cagtcgtggt ggatcctttc 1740

cctgccaatt ccaagttcat ctgccagttt gtagatcagt ctcattctcc tgtcgagctt 1800

gtagtttcct gcgtcgtagt ggcagtcacc gtggtggcat cctgttacga gaacaccgtc 1860

agcgccttcc ctgaatgcct tgaaaacaaa ctgtggctct atccttcctg agcacatcac 1920

acggataacc ctaatgtttg tagggtactg catccttgct gttcccgcag tgtccgctcc 1980

accgtaggaa caccagttac aacagaacat cacaattttt atgtcatctt cagccatcta 2040

tttaccctcc ttctttattt tcattggtat gagtgctgct ggaagtgtga atgtgtagaa 2100

tgttccaaca atgtcatcca gctgttcagc cacttcctca gggtcgacgg ttttgtcctc 2160

ttcgaccttg tagtcagagg ctatggcact tatcattttg gcaccctggt cctcgacacg 2220

tgctgttgga ccatagcagc cacggcatgg tatggctatg cttgggcact ctgcaccgca 2280

gattgataca gttgctgggc ccatgcagat gagtccctgt ggtatgagac agaggtcatc 2340

ctctggttta ccaacctcga actgcctctt tatgaagtcc attgcaaggc cttctggtgg 2400

tttttccctt gggcagactt cacagaggtt tgtgcttggc agttctattt cttcaccttt 2460

gaggagtgcc ataacgacct cagcagcaac atctgacctt ggtgggcatc ctggaacttc 2520

aaagtcaacg tctatgactt cacccagtgg tttgaccctt ccctcaaggt gtggtacgtc 2580

ttcagatggt atgacgccct cttcgtttgg tgttgttatg gagttgatgt aggcttcctc 2640

tatgacctca tccttgtccc agaggttcct gagacctggt atgccgccgt atacagcaca 2700

tgtaccgtaa cttatgacaa atttggcctt ttcccttagt tcctctgcga attccctgtt 2760

ttcatcgttt acgattcctc cctcaatgac cacaacatcg agttcaggga tttcatcgta 2820

ttttgtgtcc attaaaacag ggctgaactc gaagtcagcg tgttccatga catcaaggag 2880

cttttcatgg aagtctgcaa tggacaggtg acagccggag catcctccaa gccacattgt 2940

tccgatcttt attttttcag ccattgaagt aagtaggaag cagggagcag gggaaagaaa 3000

attgacaact gtacaagatt aatcgcgtct ctgagcaatg accaaataca tctacctcca 3060

cggttttctt ccagccccct atctgcgaaa gcacaagata ttagcaagcg tttcgcccaa 3120

attcacatac agctaacaat ccctgatctc aatgctggtg aattttctca gttaacaatc 3180

acgcgccaaa ttcaacaagt tgccgcaatt ttccctgata attctgaacc aataacgctg 3240

ataggttcta gtttaggcgg tttaactgct gcttatctag gacagcgata tttacaagta 3300

caacgcttag ttttattagc gccagtttgg ttttttatcc cattggttgc ccaaaatggg 3360

tgaagaagct gtcacaagtt ggcaacaaac gatataggtt ctctcttctg ccgtta 3416

<210> 182
<211> 6695
<212> DNA
<213> Artificial Sequence

<220>
<223> Synthetic Construct- SEQ ID NO: 182, example 182: a designer
      hox-promoter-controlled Heterodisulfide reductases (HdrABC,
      HdrDE) DNA construct (6695 bp)

<400> 182
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgtcaggaac cgcctcctac aggcttcggg agcctgagag cagcgagctc 240

tgatcccgcc ggccccatct ccttcagctc gctgacgaac ttcctgacct cttttgccag 300

gatctcgccc tcactggcgg cgcaccagat gatcttgacc ctcccggggt tgatccccac 360

cttctccagg acccccctga ggacctggac ccgctggtag gcctggtagt tccccttgtt 420

gtagtggcac tcgccgaggc ggcagccgga tatgagaacg ccgtcggcac cccccttcag 480

ggcctcgagg acgaaggctg ggtcgaccct cccggcgcac atcacccgga tcgccctcat 540

gttcgtcgga tactggatcc gggaggtgcc ggccagatcc gctccggcgt agctgcacca 600

gttgcagagg aacccgacga tgaaggggta ctgccccttg accgccgtcg ccgcccggac 660

ctgggccatg atctgctcgt cggagaagag ggtcatatcg atggcgtcga cggggcaggc 720

ggcggcgcag gtcccgcacc ccttgcaggc cgcctcgtcg acgagggcgg tcccgttgac 780

ggagatcgcc ttccccggac aggtatcgac gcagagcttg cagcctatgc acttctcctg 840

gtccaccacg gcgacgacgg ggtcgagggc gagctctcct ctaacaagaa gcctcatcgc 900

cttcgccgcc gccgcctccc cctgggcgat ggagacctgg atctcctttg gtcccgaggc 960

gcagccggcg atgaagaccc cctcgatgtg ggcctccacc ggcctcatct tggggtgggc 1020

gatctcgaag aagccgtcgg ggcgctttgc gaggccgagg aggttgccga cgacgtcggc 1080

atccttattc ggctcgagcc ccgccgagag gacgacgagg tcgtagggct tcgaacggaa 1140

gccgcccagg agggtgtcct cgtagttgac gcgaaggctc ccgtccacct cctccacctc 1200

ggagacccgg ccccggatga agttgatccc gagccgctgg gcccgcatgt agaactcctc 1260

gtacccctcg ccccccgccc ggaggtcgat gtagtggatc gagacctccg tctcgggata 1320

cttctccttg atcagctggg cgttcttgag ggcgtacatg cagcagaccc gggagcagta 1380

ggcgtttccg accgtctcgt cccgggagcc gacgcactgg aggaaggcga cggacttcgc 1440

gacctccccc gtcgatggcc gcaccacctc gccgccggtc ggccctgcgg cgttgaggag 1500

cctctcgacc tggagggcgg atatgacgtc tctatacctg ccgtagccgt actcctcctt 1560

tctggcgggg tcgaaggact gccagccggt ggcgacgatg atggcgccga cgtcgaacct 1620

gaactcctcc tgcctctggt cgtacttgac ggcgtcgacg gggcaggcct cggcgcagag 1680

gccgcagccg atgcaggccg agggatcgat gcaggcgacg agggggaccg cctggggtat 1740

cggcatgtag atcgcctttc ttttgccgag gccgaactcg tagtcgtcgg ggacctccac 1800

gggacagacc ccggcgcact cgttgaggca gcccttgcag agcttctcgt cgacgaaccg 1860

cgccttcctg accccggaga cggtgaacct tccgacagac ccctcgatgt tgaggatctc 1920

ggcgtaggtg acgaggtcga tgagggggtg gttgtagacg tcggtcatct tcggcgcgag 1980

gacgcagatg gagcagtcgt tcgtcggaaa gacgtcggtg aggagggcca tgtaaccccc 2040

gatcgtcggc ctcctctcga cgaggtggac ggagaggccg cagtcggcca gatcgagaga 2100

agcctggatc ccggctacgc ctccaccgat gaccagcacc tcccggctga cgggcatcgt 2160

ctccccctgg agggggttta ggagggccgc cttcgccacc gccatccgga tcaggtcttt 2220

tgctttctcc tgggcctggg ggggctcgtc catgtggacc cacgagcact gctcccggat 2280

gttcgccatc tccagcatga aggggttgat ccccgcctcg gagatcaccc gccggaaggt 2340

cggctcgtgg agccgaggcg agcaggcggc gacgacgatg ctctccagct tatgctctgc 2400

gatgtcctcc ttgatcatat cctggcccgt atcagagcag gtgaactgga ggtcccgggc 2460

gacctcgacc ccagggaggg ttttggcgaa ctcggcgagg gcggagggct ccagaacccc 2520

tgcaatgttc aggccacagt gacagatgta aactccaatt cgcattcagc agacctccgc 2580

cccctcctcc tcagctttct tcccctcgat cttctccagc ttctccagca ccggcctcgt 2640

cgatacggag ttcatcgata ggcccagctc ttctgggcct aggccgaggg cgagccccgc 2700

catctgggtg acgtataata cggggaggct gatctcttcg ccgaacttct tctccaggag 2760

cggctggttg gcgtccagca tcaggtggca tagcgggcat agcgcgacta cgaagtcggc 2820

gccggaggcc ctggcgttcc ggaggatccg gatgagggtc tcggcggccg cctcttcctc 2880

gggcatgaag atggggccgc agcagcactt catctttaat ctgaagtccg taggctccgc 2940

tcccagggtc cggatcaggt cttcgaggat cgaggggttc tcggggctgt cgatcccccc 3000

gccgggcctc gtcaggaggc agccgtagta gggggcggcc ttcaggccgg cgaggggccg 3060

gacgaccagg tcttctgggt cctcagcctc ggcgagggct ttcgtgatca tctcgatgac 3120

gtggaggacc tccttttctt cctcgtactt cttctccagg gcctcgttca cccgctccct 3180

cagttttcgg tccttcaggc ggatcatcgc cgcccggagc ctgctgtagc agatggagca 3240

ggaggccatc actggaagct ccgtcctcac caggtttcgc gccgccaggg ccgtcgagac 3300

gacctcgttc gagacgtggg tggcgccgca gcagttccag tcgtcgatct ccgatagctc 3360

tatgccgagg gctcgggaga ccgccagggt cgatatctcg tactccctcc ccgtcgaccg 3420

ggcgacgcat ccggggtagt aggcgagctt cattcatttt tctccctcct ctttttctcc 3480

ggccttctca gactttcctt ctttcctccc gtcgtcgccg agaagctcga agatccgcct 3540

catctcctcc gtcccctcga tcctctcccc cctcgccgcc tcaaagccga gctcgatctt 3600

ccccttgggg acgagctctg cgccgaaggc catgatctgc gccgccgagc tggggctacg 3660

gatcatgtac tccgccgaca gcaggaactc cgatatcctc ccatagtcgc ggatctggcg 3720

ggcgaagatc ccctcgaaga ccgagtcctt gcacttcaag ccctcctcct cgtagatcgt 3780

cttgagggcg gcgacgaccg ccatcggcct gatcttcctc gggcagcgga cggtgcagag 3840

gaggcaggag gtgcagagcc atatcgacct gctcgccatg gcagcagcct tctccccttc 3900

gaggaccagc tttacgagct ttcgtatggg cacgtccatc agggaggcgg tggggcagct 3960

cgcggagcag gtcccgcact ggatgcaggt tttcacctcg gccccggcga gggcgaggac 4020

cctctcccga aacttctccc cctcggccgt catgatcgcc gtcgtcttca tcgtcgtcac 4080

cgcctccgca atgctagaag cctcctttgt catatggcag aagcaggaaa agagaagaag 4140

cccctcgtat caggattcct catgtccacc cagctgatgg agctggacgg atgcacccga 4200

tgccaggagt gcatgaagtg gtgtccgacc ttcgacgtga ggcaggaccg ccccgagatc 4260

acccccatgt acaagatcgc caagttcagg gaactcctgg gaagccagca cggcctccgg 4320

gcgaagctct tcggccccaa gcccctcaac gaggatgaga tcaacaggtt cacggaagat 4380

acctactact gcaccacctg tggggtctgc ggcaccgtct gcgagtccgg gatcaacacc 4440

gtcgagctct gggaggcgat gaggccaaac ctcgtagctc gggggaacgg cccctacggg 4500

aagcagtcct tcttccccgg cctcctcgga aaggacagga accccttcca ggcaaagcag 4560

gaggagcggc tctgctgggt tcccagtgat gccaccgttc tggagtcgtc ggagatcgcc 4620

tacttcgccg gctgcaccgc tgcctatagg cagcaggccc tcgccgtcgc ctccgtccgg 4680

ctgatgaacg ccctggagat acccttctgc atgctcggaa aggacgagtg gtgctgctct 4740

tcggctctgg tgaggacggg ccagcggaac atcatgaaag aacatgcagt ccacaacgtc 4800

gatgctctaa aggatcgcgg ggtaaggacg gtcctatacg cttgtgcggg ttgtcagaga 4860

accgccacca tcgactggcc taggtggtac gagggctaca tcccctacaa gaacatcccc 4920

ctctccgttt acctgaggga gaagatcagg agcggcgagg tggagtggaa gcggcccctc 4980

aacttcaagg tcacctacca cgacccctgc cacaacggta ggcacctgat gcacgtcaat 5040

gggagggacg tggcctttga ggcccccagg gacatcctga agtccatccc cggggttcag 5100

ttcgaggaca tggcccgttc cagggagttc cagaggtgct gtggcgctgg cggcggcgtc 5160

aaggccggga tccccgatct cgccctcgac tgcgccaagg cgaggatggg cgacgcccag 5220

ctgatagcag cggacgtgat caccagcacc tgtcccttct gcaggcggaa catcatggac 5280

gggagagcag cgatggaagg atgcgacgtc aaggtcttgg acgtagtcga gatgatgact 5340

gcagccatgg gcttggacat cacgatcccc gacaatccct acatgaagtt ccaggagcag 5400

gacgttctgg tatgcgatga ggacgtctgc aaggtcgaga cgatcgagag gaaggctgaa 5460

gggaaggacc tagtaggaga ggcccactga atggcgtttt acacccttgg tctcaccttc 5520

gggatcgtga tagcccttac cgtagcaacc ttggcaatct ggatctacgg cctctacttc 5580

aacttcaaaa agtgggggat ggggtcgacc ggttatcagg acgagcttcg aaacagcttc 5640

tggctattta ttgcaacatg gattcatcag gccttcaaag acggcgtctg ggtatttgtc 5700

aaaaccctca tcctcgacgt cctccttctc aggcggaccc tgaggaggag ccccatcaga 5760

tgggtgatgc acatgaccat attctacggt tttgtcgccc tcgccgccat gtctggcttc 5820

gccctcttta tggacataat tgagcacttc aacctcttgg ggctcgccca cgaggctgag 5880

atggtgaagg aaatgatggc tctccccttc gacatcttcg gctacctcct cctcttcggg 5940

gcgacgatag gggtcgcgag gaggatcttc ctccgagagg tgcggtccag gacctcggcc 6000

tacgacgtgg tcctcctagg cggagtattt ctgatcacca tcacaggctt ctatgcccaa 6060

tggatgcggg gcaactcctt cctcgtcgga gacgtcttcg cgtacccgat atacgctccc 6120

cacttcgccc tagtccatac gatcctggcc ttggccctct tcgtagtgat ccttccctgg 6180

agcaagtaca tgcacataat agctgcaccg atgacgattc tagcaaacag aggaggcgag 6240

taatgaagta agtaggaagc agggagcagg ggaaagaaaa ttgacaactg tacaagatta 6300

atcgcgtctc tgagcaatga ccaaatacat ctacctccac ggttttcttc cagcccccta 6360

tctgcgaaag cacaagatat tagcaagcgt ttcgcccaaa ttcacataca gctaacaatc 6420

cctgatctca atgctggtga attttctcag ttaacaatca cgcgccaaat tcaacaagtt 6480

gccgcaattt tccctgataa ttctgaacca ataacgctga taggttctag tttaggcggt 6540

ttaactgctg cttatctagg acagcgatat ttacaagtac aacgcttagt tttattagcg 6600

ccagtttggt tttttatccc attggttgcc caaaatgggt gaagaagctg tcacaagttg 6660

gcaacaaacg atataggttc tctcttctgc cgtta 6695

<210> 183
<211> 3407
<212> DNA
<213> Artificial Sequence

<220>
<223> Synthetic Construct- SEQ ID NO: 183, example 183: a designer
hox-promoter-controlled Coenzyme F420-reducing hydrogenase (Frh)
DNA construct (3407 bp)

<400> 183
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgttaaa caatgcgatc gcaacgccgg ccctccagcc attccaggaa 240

ctcgagcaga gcgtctggaa gaaggagatg tgcgccggat gccggggctg tatcaccgta 300

tgcccggcca acacgttagc ctatgacctg aagctggcca ggccgtacca gatcactccc 360

tgtgtcgatt gtaaggcctg cctggacgcc tgtcccagga ctccagctaa catggataag 420

ctgtccctgg acattcttgg gccgcatctt gacgtttata atgtgaaggc tactgcgggc 480

aataagcgct accagaacgg cggcgccgtt actgcgttgc tgaagaccgc gctggacgaa 540

ggccttgtgg accgtgtgat cgtcatgggc gcggaccgct gggcacagaa ggcatacgcc 600

cgggtcgtct ccgactcatc gagcctggac agggctgcgg gcagtattta catgaacaac 660

gatgccctgg agacaatgaa ggacatcatg aaggacgact cgatcaggaa cgtggcgatc 720

gtgggaacgc cctgcgcgat acagtcgata ggcctgctcc ggaagtcctc gaacgagtac 780

tccgtaaagc tgacccagaa gatcaggttc gccatcggct tattctgctt tgagtcattc 840

gatgatcggc tcatccccga agttaccaaa cgactcggcg tgccgtcgtg gcgtatcgcg 900

aagatgaacg ccggcgaggg caggctgacc gtcacgctga ggagtggcga ggtaaagacg 960

ctgcccctgt cgagcctggc cgagttcgtc aagcccggat gccgcaagtg caacgacttc 1020

acttcaaagc tcgccgatat ttccgttgga agcgtgggca gtgctgccgg gtccagcgtc 1080

gttattacca ggacgcccga gggtgccggg ctactcgaga tcgcccgcga ggtgggcgct 1140

attgatgtgg ccggcggagt cgatgtcgct gcgatcgaga aggtcggcaa gctgaagctt 1200

aagaaaaatg gcttttaaat gtcattcgga acttacaagg aagtcatggc cctcaaggcc 1260

aaggacggaa agatcggcgg cgtcgcccag gatggcggcg tcgtgaccac gctgctctgc 1320

tacgcgctcg agaagggcgt catcgacggc gccctcgtcg ccggcaagag cgagaccccc 1380

tggatgccta agcccaccat cgcgacgacc aaggaagaga tcatcgcggc ggcaggcacc 1440

aagtacacca tcagcccggt cgtgtccacc atcaaggacg cagcccgcga gtacggcctc 1500

gagaagatcg cggtcgtcgg caccccgtgc cagatctacg cggtccagaa gatgaggctg 1560

tacggcgtcg gcgcgagaca catccccgac aaggtagcca tgaccgtagg catcttctgt 1620

acggagaact tctcttatgc cggcctcagg accgttatcg aggaccactg caaagtgccc 1680

atcgaatcgg tcaccaagat ggagatcggc aagggcaagt tctgggtcaa gggagccaag 1740

gacgtgtcca ttcccatcaa ggagacccac aagtacgagc aggacggctg ccacgtttgc 1800

tccgacctga ccgccgagtt cgcggacatc tccacgggct ctatcggcac tcccgacggc 1860

tggtccacca ccttcgcccg ctcgaccagg ggtaaggacc tcctgagcaa ggccatcgca 1920

gacggcctgt tcgagaagaa gtccatggac gagttcaagt ccgctccgga cgccaagatc 1980

aagagcggcc tcgacctcct gggcaacctg gaaaagggca agaaggacaa ggcaaagaag 2040

catttcgacg agcgcaaggc tctcggcctc ttcgtaaccc cggaaatcct gtactaaatg 2100

gcataccagg gtttcgggaa atataaggaa gtcttgagcg cccggtctgc ggacgaggag 2160

atccgtaaca tcgcccagga cggggggacc acgacggcac tgttgtgttt tgccctcgag 2220

accggcttca tcgacggggc ggtgctgacg aaaaaatcga gcagggagtg ggtgcccgcc 2280

cagtacgtgg cgacgacccg ggacgagatc ctccagtcgg cgaagagcgt ctacgcgctg 2340

tcccccagcc tgtaccggct gaaggaggcg acgagggaaa gggccctgag taaggtgggc 2400

tatgtggggc tgccctgcca gatcgaggcg gtccgcaaga tgcagctgta cccgttcggg 2460

gcacgggata tcgtggagag cctggccctc gtcatcggca tcttttgttt cgagaacttt 2520

tatcccgaga gcctgaaagc cattgtagaa ggcctgggcg aggagcccct ggaggatgtg 2580

gtgcgcatgc ggtgtgcctc cgggaagttc aggacggagg gggagaaggg cattatcgtg 2640

ccgctaaagc aggcgtcccg gtacattcag gacggcgacc gcatctgccc ggacctcgtg 2700

tcggagtggg cggatatctc ggcgggctcc gtgggctcgg accccggatg gaatactgtc 2760

ttcctcagga cgaagaaggg ccacgacttt ttccagcagg ccaccggggc tggcgccata 2820

gagacgaagg agatcagcga ggaagggctt aaggcactcg aaaagctggc cgtagcgaag 2880

aaggaccggg cgaaaaaaca tatcgcaaag cgcgaggagc tcgggcttta cgtgacccgg 2940

gacatctact attgatgaag taagtaggaa gcagggagca ggggaaagaa aattgacaac 3000

tgtacaagat taatcgcgtc tctgagcaat gaccaaatac atctacctcc acggttttct 3060

tccagccccc tatctgcgaa agcacaagat attagcaagc gtttcgccca aattcacata 3120

cagctaacaa tccctgatct caatgctggt gaattttctc agttaacaat cacgcgccaa 3180

attcaacaag ttgccgcaat tttccctgat aattctgaac caataacgct gataggttct 3240

agtttaggcg gtttaactgc tgcttatcta ggacagcgat atttacaagt acaacgctta 3300

gttttattag cgccagtttg gttttttatc ccattggttg cccaaaatgg gtgaagaagc 3360

tgtcacaagt tggcaacaaa cgatataggt tctctcttct gccgtta 3407

<210> 184
<211> 5417
<212> DNA
<213> Artificial Sequence

<220>
<223> Synthetic Construct- SEQ ID NO: 184, example 184: a designer
 hox-promoter-controlled Methyl-H4MPT: coenzyme M
 methyltransferase (MtrA-H) DNA construct (5417 bp)

<400> 184
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggcaga taaaaaagaa gttattcaaa attggccctt agaaactgga 240

gattatgcag tgggaaatgt tgaaagtcca gtagcagtag tctctctagg ttctaatatg 300

aatgatgaat tggtagctgc aggggctgca atttcaggac cattacacac agaaaacttg 360

ggaatagaaa aggttgttgc aaatataata tcaaattcaa acatacgtta tgttcttata 420

tgtggatctg aggttcaagg acatattact ggtaaaacag ttgaagcttt atatgaaaat 480

ggtattgatg aagagaaaaa atcaattatt ggttctcctg gagctattcc attcgttgaa 540

aacttaccag tagaagctgt tgaaagattc caaaaacaag tttctattgt tagtatgata 600

aataatgagg atgttagtga aatatcatct aaaatagatg aatgtattag taatgatcct 660

ggagcatatg atgaagatgc aatgattgtt gaatttaatg aaacacctga agaggagttt 720

gaagttgatg aagtcacatt ttctgatgat tcagcagttg atttagcatc aatagttcta 780

cttgaggttg aaaatcgtat aagtatgatg aataatgaaa taaaacaaat tgcttcttta 840

gaaaaaatat cttcaggata ttatgctgga aaaatagaag gtatagttat tggatttatt 900

ttaacattgg tttttcttat aattattatt caaggtttgt gattggtaga tacaacagca 960

gttataattg atgatgattt agttttagat gttaataatg gtataatagg aacaaaagat 1020

aataatcaaa aacaacatct tccatttgat gatctggtag aaaaaataga tactctggaa 1080

gtggcaatta ataatctaga atcatcatta gatcctagaa caacatctaa tctatcacaa 1140

cctggaagag aaggaatata tgaaaaagca ggattaatta caaacattgt aattggtgtg 1200

attatagcat tagttttcat acttttatta gtataaatgg ctgatgattt aataccttct 1260

cagataataa ttttaagttc tattgttggc ggattaattt gtatagcact aagtagtata 1320

cctgtggttg gtggtatttt ttcaattatt gcaacaatat taggtgttat ttgtggaaca 1380

aatacactta gacacatagg aaaatatagt ttaggtactg gagttccatc aatagtttat 1440

atgttaacag cagcaggact tgttagtatg atatgtggtg taatgatatc aagaataatt 1500

aatcaaaatt tattatatcc aatatttagt atgataatag caatgataat tgcttttgta 1560

atttcactca tgtgtacaca tatcttcaaa atacaagttg aaatattaag aaaatcattc 1620

ataacattaa caattgcaac agtaatatca attattggaa tgtctacatt gatagtttca 1680

tcctctgata ttatgttaat ctattcaaat gtaattaaaa atggacttat aattatatta 1740

atgatattat cagtcatggc aatacaaaat ccatataatt cttgtatggg accaaatgaa 1800

gatcagtata gaacattatc attatcctta tccaatgcat ttttaatgct tataataatt 1860

tcaattatta gtattttaaa tagttcatac tggtatattt acattataat atcaattatt 1920

ggatggttag tttcatttaa attatatatt aaatatacaa aacagcaagc tgcactaata 1980

aaatggtctg gactatggcc taataatgat gaagggggaa tataaatgat atttgaatta 2040

ttaattagta taataatagg atctacatta ataggatttg gagttcattt tatacctgta 2100

ggtggagcac cagcagcatt atctacaact gctggagtac ctacaggagc acctatgata 2160

actattggta tgggaattac aggaatactt tcagctcttt caatgacggg tcagtcagaa 2220

atagtaataa ttctatcagg agcaataggt tcaatgctta tgatggctgt aacaatgttt 2280

ttttcaaata tgatacatgt ttatggggta ggtgttccat tggcatcttc aaactttgaa 2340

agggatccaa ttacaggttt taaacaggaa gaatatgtaa gtcctggaac aacaggtcat 2400

ggaataccaa cagtttcatt tataagtggg gttattggag cattatttgg aggaattggt 2460

ggaagtttgg cattttgggc aatttataat tatatattgg gaaattgtca tctttcatca 2520

atctacacaa attctatttc agcaatactt gcagtaatgt tattttttat tattgcagta 2580

gtagcatcat acaatattgg tggtacaata caaggatttt atgataagaa atttaggaaa 2640

aaaatagttt caggtacatt ttcatgtttt ttaatatcaa tattcttggc tattatttat 2700

atgataatat tgggtggaat ttaaatggtt agcatggggc tcctagcagc tatgggcgcc 2760

cttgcgacag tagccggtgc aagcgaggac ttagagtccg atgtgggttc tcagagcaac 2820

cccaactcac aggtccagtt ggctgcccag gtgggtcaca cccacaggat atacaacaaa 2880

gccatctctg gagagccccc atcgaacgct ctttacgccg cccttgccgc agttacagct 2940

agtgtcttga tgtccgggta tggcatgaac gcgttctttg cgatcgccat cggctcggtt 3000

atggccgcga tcttcaacgg catcttttct gtcaccgcct acatgggcag gaacacgagc 3060

caggcgcggt tcaaccagta cgtctacctg gacgttctgc ggactcacac caccgccatc 3120

atggcacatg cgtacatcac tgccttctgc gtagtcacca tctcctacat catgacagcc 3180

ccgtggcttc cgattcagca cccgttcccg ttccccctcg tcgccatgat atgggggctc 3240

gccatcggag ccataggatc gtctacggga gacgtccact atggaggaga gagggagttt 3300

cagaactaca tcttcggggc ggggctcaac gccgccaact ccggtgacat cgtcaccagg 3360

gccgaggctg gcctgaggag ctccatcgac aacgtctggt tctgcgcccg attcggaggc 3420

cccgtcaccg ggatggcctt cgggctgacg gtcttcctgg acaactggcg gaccaccatc 3480

ttcgatcccg ccacccaggg atggtatgcg gtcctgaccg ggatagtgat cgtcattctg 3540

ttcatcgtct acaacagaat cctggagatc agagcgagaa agaggtacgg gccctatccc 3600

gaatacaagg gggacattga ggcatgatta gatcgccccc agcagcagtg ccggaatcac 3660

aatcagaagc acggctacga tgagcccaat cgcgaatccg aggatcccca tgcccatgat 3720

gcccgactca agtttggtcg tgcgggcaag gatctgtgcc ttgtaccgga tggagtccat 3780

cattctgttg atcgaggtca tccggatggg gcctgcctgt gtaacttctt ctgccatgtg 3840

ggaaatgatt caaaaaagga tattgaagat attaataata gattagatag tctagagcaa 3900

aaattagagg aatctcatgg tgaattttct caaaattatg gaaaaagtat aggacgagat 3960

attggaatat tatatggtgt ggtcttagca ttattagtag ttattatttt aatgaaattt 4020

catataattt agatgtttaa atttgaaaaa aaacaagaag tattcgatat ttatggtgta 4080

aaggtaggtg gacaacctgg tgaatatcca acagtacttg ctggaactat cttttatgct 4140

ggacataata ttattagtga tgagaaaaaa ggaatttttg ataaggaaaa agcagagact 4200

ttattaaata ctatggatga aatgactgat aaaacaggaa atcctaatat tgttcaaata 4260

tttggtgcaa ctagtgatgc aataataaaa tatattgact ttgtaacatc tattagtgaa 4320

tctccatttt taatagattc aacatctgca caggcacgta tagcaggttc aaaatatgta 4380

acagaatctg gtcttgcaga aagaacaatc tacaattcaa ttaatatgtc aattgataat 4440

gaagaaattg atacattaat aaattctgat ataacctctt caataattct tggatttaat 4500

ccaacacaac ctggtgttga tgggaaaata aaactatggg atgatggtgc tggagtttta 4560

gataagggtt tacttgaaat tgcagatgac tgtggaataa ttaaaccatt gatggatgtt 4620

gctgttacac ctctaggtca aggtggaggt tctgcagtaa aaacaacatt taaggaaaaa 4680

agtatatggg gttatcctgc tggtagtgga attcataatg ttccatcagc atgggaatgg 4740

ataaaacaat ataaaaagga atatccagaa gtatggcctg tatgtgatat tggtgcaaat 4800

atagttcagc aaatggttgg tggggacttt gtattatttg gacctattga aaatacaaga 4860

aaagcattta ctgcatgtgc aatgactgat atgtttattg cagaggctaa tgaagctatt 4920

ggaataaatg ctgtagattc tcatccatat aaaaatttat tatgatgaag taagtaggaa 4980

gcagggagca ggggaaagaa aattgacaac tgtacaagat taatcgcgtc tctgagcaat 5040

gaccaaatac atctacctcc acggttttct tccagccccc tatctgcgaa agcacaagat 5100

attagcaagc gtttcgccca aattcacata cagctaacaa tccctgatct caatgctggt 5160

gaattttctc agttaacaat cacgcgccaa attcaacaag ttgccgcaat tttccctgat 5220

aattctgaac caataacgct gataggttct agtttaggcg gtttaactgc tgcttatcta 5280

ggacagcgat atttacaagt acaacgctta gttttattag cgccagtttg gttttttatc 5340

ccattggttg cccaaaatgg gtgaagaagc tgtcacaagt tggcaacaaa cgatataggt 5400

tctctcttct gccgtta 5417

<210> 185
<211> 5042
<212> DNA
<213> Artificial Sequence

<220>
<223> Synthetic Construct- SEQ ID NO: 185, example 185: a designer
 hox-promoter-controlled Methyl-coenzyme M reductase (Mcr) DNA
 construct (5042 bp)

<400> 185
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggataa tgagaaaaaa ttattcttaa aggctttaaa agaaaaattc 240

gatgaagacc ctgaagaaga aaacactaaa ttgcactgct atggtggatg ggaacaatcc 300

gcaagaaaaa gagaatttgc tgaagaagct gaaaaagctg tagaagcacg tggaggatta 360

ccattctaca acccagatat aggtgtacct cttggacaaa gaaaattaat ggcataccaa 420

gtatctggaa cagacacata tgtagaagga gacgaccttc acttctgtaa caactctgca 480

attcaacaat tagtagacga tattaaaaga actgtaattg taggtatgga tacagctcac 540

ggtgtacttg aacaaagatt aggtgtagaa gttactcctg aaacaatcaa cgaatacctt 600

gaaacaatca accacgcact ccctggagga gcagttgtac aagaacacat ggtagaagtt 660

gaccctggat tagtaactga ttcctacgca aaaatattca ctggtgacga cgacttatta 720

gatcaattag atcaaagatt tgtaatcgac atcaacaaag agttccctga agaacaagct 780

gaaatgctca aaaaatacat aggtcagaag acctaccaag taagtagagt acctacatta 840

gtagtacgtg cttgtgatgg aggtacaacc tccagatggt ctgctatgca aattggtatg 900

agtttcattt ctgcatacaa attatgtgca ggagaagcag ctattgctga tttctcatac 960

gcagcaaaac acgcagatgt aatttccatg gctagtgcaa tgccatcaag aagagcaaga 1020

ggtccaaacg aacctggagg tgtaaccttc ggtgcattct ctgatttggt acaaacaagc 1080

agagtaacag atgacccagc tgaagtaact ttagaagtta ttggtggagc agcaccatta 1140

tacgaccaag tatggttagg ttcatacatg tctggtggtg taggattcac acaatatgct 1200

acagcagcat acacagatga aatattagat gacttcattt actatggtaa agactacgta 1260

gaaggtaaat atggtggatt atgtcaagct gaagcaactt ccgaagttgt aaaagacatt 1320

gcaagtgaag taaccttata cggattagaa caatacgaaa tccctgcagc attagaagac 1380

cactttggtg gatcacaaag agcagcagtt cttgcagcag gtgcaggttg ttcagtagca 1440

ttcgcaactg ctaactctaa cgcaggtgta aatggatggt acttaagtca attattacat 1500

aaagaaggtc acagtagatt aggattctat ggttacgact tacaagatca atgtggatca 1560

tccaactcac tctctgtaag aagtgatgaa ggacttattc acgaattaag aggtcctaac 1620

taccctaact acgcaatgaa cgtaggtcac caacctgaat atgcaggtat tgctcaagca 1680

ccacacgcag caagaggaga cgcatttgca cttaacccat taataaaagt agcatttgct 1740

gataaaaact tatccttcga ttgggctcac ccaagagaat gtattgcaaa aggtgctgtt 1800

agagaattta tgcctagcgg tgaacgtgac ttaatcatcc cagctttata aatgccaaca 1860

tatgaagaca aaatagattt gtatggggta gatggaaaac ttttagaaga acaagtacct 1920

ctagaagcaa ttagtccagt aataaaccct acaattaaaa atattataca agagattaag 1980

cgttctgttg ctataaactt agcaggtatt gaaaaatcat tagcaaacgg agcatacggt 2040

ggaaaagcta attttattcc tggaagagaa ttggaattag atattgttga caatgcagat 2100

gcaattgctg acaaaattga aaaaatgtta aaaattagtg atgatgatga tttcaattta 2160

aacttattaa atggaggaaa acaaatatta gttcaagttc cttcagaaag attaagtatt 2220

gcaggtgact actctgtagc accattagca acaggatcag cattaattca agctattctc 2280

gatacttttg acatcaacaa atatcaagct tctgaaatta aaacagcagc tatgggtgga 2340

tacccacaca acgtaaaatt aggcggagca ttaactacct tattaggtca aacaactcac 2400

cttgaaggat taggttacag tctaagaaac attggagcaa accacgtagt agctattaca 2460

aagaaaaata ctcttaacgc agtagcttta tcctctattt tagaacaaac tgcaacattc 2520

gaaatgggtg acacagtagg tgcattcgaa agaagccacc ttttaggatt agcataccaa 2580

ggattaaacg caaacaacat agtttatgac ttagtaaaag aaaatggtaa agcaacactc 2640

ggtgatgtaa ttatttcatt attatctaga gctttagatg atggagtaat cagagtaaaa 2700

gaaacattac cttccggatt caaattatac gaaccagttg actgggcatt atggaatgca 2760

tatgcagcag caggtcttat tgcagctaca attgttaacg taggtgcagc aagagcagct 2820

caaggtgtag catcatctat attatacttt aacgatatct tggaatacga agcaggttta 2880

cctggtgtat actttggtag agtaatgggt acaggtgtag gtatgagttt cttctcacac 2940

ggtatttacg gtggtggagg acctggtgtt ttcaacggta accacgtagt aacaagacac 3000

agtaaaggat atgctatccc atgtaacgca gcagctatgg ctttagatgc aggtacacag 3060

atgttctctg tagaatctac atcaggttta gttggagaag tatatggtag cgtagacaat 3120

ttaagagaac cagtaaaata tgttgctgaa ggagctagtc aagtaaaaga taaactctaa 3180

atgatagaaa tgattggaaa aaatacacat atagttgact gtagggaaac acgcggaata 3240

ggtgaaggtg gtggaattgc tcagcgtgga acatatgcac aatgtgggga tgaattactt 3300

gctgttgcaa tgtctccagg acgtagacat attacaaaac cagtttgtga aataacattt 3360

gctctaagag aatcaaatat tcaaacaagt accttggttc ttaatgcagg ttctggagta 3420

ccaaaagaca cgcctagtgg tggaggacaa ggtttatttg gtgtaactcc taaggaaatt 3480

tcccagatga gtagacataa tattgtattg ttccattttg gaggagtaaa aaatcatata 3540

atatacaagg caaaacatgc tcttaaacat attaataaac ctgttattat tatagcacaa 3600

tatccattgg attttgaaga ttttgcaaaa ataggagtaa atactgttgc agtaaaacct 3660

gaaaaatctg aaacacaggg tactattgtt gaactcataa ctgatgttat tcgtggagaa 3720

tcatgtactc aagagaaatt agatgaaata ataaataaag tggaaacatc cattgtaaaa 3780

tatactaagt aaatggcata tgaagtacaa tattatcctg gagaaagtgt tattgcagaa 3840

aaccgtaaaa aacacatgaa tccagatcat gaacttaaaa aacttagaaa tattgcagat 3900

gacgatatag taaaactttt aagtcacaga aacccaggag aaggttacaa aacagtacac 3960

ccacctcttg atgaaatgga ctttgaagaa gatactatga aagacttcgt agaaccaatc 4020

gacggagcta aaaaaggaat tcgtataaga tacatacaat tcgcagactc aatgtataat 4080

gcacctgctc aaccatacga cagagcaaga tcatacatga gaagattcag aggagtagat 4140

acaggtacac tctcaggaag acaagtaatt gaaatgagag aatctgactt agaagaaact 4200

tccaaattat tattagaatc tgaattcttt gattccgcta aaacaggttt aagaggagca 4260

accgtacacg gtcactcatt aagacttgat gaagacggat taatgtttga tgcattacaa 4320

ttatacgtat tcgataaaga aactggtcac gttgtatacg taaaagatca agtaggagta 4380

gaacttgatg aacctattga tgtaggagaa ccattaccag aagacgaact taagaaaata 4440

acaaccatct acagatctga caacgtcagc ttaagagatg atgaagaagt tattaaagta 4500

gttgaagcta ttcacttcgc aagaactgat ggtggatatg gtttagaagt attcaacaac 4560

gatttaaaaa gtaaattagg tggtaactaa tgaagtaagt aggaagcagg gagcagggga 4620

aagaaaattg acaactgtac aagattaatc gcgtctctga gcaatgacca aatacatcta 4680

cctccacggt tttcttccag ccccctatct gcgaaagcac aagatattag caagcgtttc 4740

gcccaaattc acatacagct aacaatccct gatctcaatg ctggtgaatt ttctcagtta 4800

acaatcacgc gccaaattca acaagttgcc gcaattttcc ctgataattc tgaaccaata 4860

acgctgatag gttctagttt aggcggttta actgctgctt atctaggaca gcgatattta 4920

caagtacaac gcttagtttt attagcgcca gtttggtttt ttatcccatt ggttgcccaa 4980

aatgggtgaa gaagctgtca caagttggca acaaacgata taggttctct cttctgccgt 5040

ta 5042

<210> 186
<211> 5450
<212> DNA
<213> Artificial Sequence

<220>
<223> Synthetic Construct- SEQ ID NO: 186, example 186: a designer
 hox-promoter-controlled Formate dehydrogenase DNA construct (5450
 bp)

<400> 186
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatggttaa cctgaccatt gacggacaaa gggttacggc tccagagggc 240

atgaccatcc tggaggtggc ccgggaaaat ggtatccata tccccaccct gtgccaccat 300

ccaaagttgc ggcccctggg gtattgccgt ctgtgcctgg tcgacatcga gggcgccgca 360

aaacccatga cggcctgcaa tacgccggtc gccgagggta tggtgatccg gaccagcacg 420

cctgttatag aggagatgcg caagggtatt attgaaatgc ttttaagcct ccacccggag 480

gactgcctga cctgcgaaaa agcaggtaac tgccagctcc aggattgcgc ctacacttac 540

ggtgtaaagc atggcgaact gccagtaaaa cgcgaggaat tgcccgtttt gaaggaaaat 600

cccttcattg tgcgggatta taataaatgt atcgtttgcg gccgttgcgt ccgcgcctgc 660

caggaggtgc aggtccagag ggtcgtggac ctggtgggta aaggcagcgc cgcccgggtg 720

ggggcgacga aggccggagc ggaagtaagc ctggaagaag ggggctgcgt cttttgcggt 780

aactgtgtcc aggtctgccc ggtgggagct ctgacggaga aggccggcct gggccagggc 840

cgcgagtggg agttcaaaaa agtccgcagt atttgttctt actgcggcgt gggttgtaat 900

ctcacccttt atgtaaaaga tggtaaggtg gtaaaagtta ggggttacga aaaccctgag 960

gtaaacaacg gctggctgtg cgtaaaaggc cgctttggtt ttgactatat tcacaatcct 1020

gacaggataa ccaggccgtt gatccgggag ggagataggg aaaaaggcta tttccgggag 1080

gcttcctggg aagaagcttt agcccttgta tcccagaaat taactcagat taaaggcagc 1140

tacggctctg aagctctggg ctttctttgt tcagctaaat gtaccaatga agagaattat 1200

ctcttacaaa agctggcccg gggggtactg ggcaccaata atgttgatca ctgtgctcgc 1260

ctctgacact cttctacagt tgccggtctg gcaacaacct ttggcagcgg tgcaatgacc 1320

aattctatcg ctgacatcgc cagcgcagat tgtatctttg tcattggcag caatacaacc 1380

gagaaccatc ctgttattgc ccttaaagta aaagaagctg tccgtcgtgg agccaggctc 1440

attgttgctg atccccggcg tattgaactg gtgaacttca gttacttgtg gttaagacaa 1500

aaacccggaa cagatcttgc tctgctgaat ggactgcttc atgtaatcat caaggaagag 1560

ctttatgaca aagaatttat tgcccagagg acggaaggtt ttgaggctct aaaacttgcc 1620

gtagaggagt atacaccagc aaaggtgtca gaagttacag gtgttccggc aggcgatatt 1680

atcgaggcag caaggactta tgcccggggt ccgagctcta ctattttgta cgcaatggga 1740

ataacccagc atataactgg tacggccaac gtgatggccc tggccaacct ggccatggcc 1800

tgtggtcagg tcggtaaaga aggtaacggc gtaaatcccc tgcgggggca gagcaatgtc 1860

cagggtgcct gcgatatggg tggattaccc aatgtattac cgggatacca accagtaaca 1920

gatccggggg ttcgccataa atttagcgaa acctgggggg taccggactt acccggagaa 1980

cctggcctga cattaatgga gatgatggcg gcagcccaag aaggcaaatt gaaagggatg 2040

tatattttag gagaaaaccc tgtcttgact gatccagatg tctcccatgt aaaagaggcg 2100

ttaaagaacc tggagtttct ggtggtacag gatatttttt tgacggagac agccaggatg 2160

gcggatgttg ttttacctgg agcttccttt gcggaaaagg aaggtacctt taccagtacg 2220

gagcgccggg tgcagctttt gcataaagcc attgaacctc ccggtgaagc acggccggat 2280

tggcttattt taaacgattt gttgctgtta atgggatatc cgcggaaata ttcgtcgcct 2340

ggggagataa tgcaggagat agcagggtta actcccagct atgcgggtat aacttatgag 2400

cgcctggaag ataaagggtt acagtggccg gtgctttccc tcgaacatcc gggtacaccc 2460

gttctccatc gggaaaaatt tagcagaggt tatgggcagt tccaggtagt gcattaccgg 2520

ccgccggccg aagaacctga tgaagagtac ccgttcttat ttaccactgg caggaatttg 2580

tatcactatc atactgttat ttcccgtaag tccagggggc tggaagagat gtgtcctgct 2640

cctgtggtgg agattaatga taacgatgca gcccgtttgg gtatacggga aggagaaatg 2700

attgagattg tttcccgacg tggtaaagta agggttaaag cattggttac ggatcgcata 2760

ccccggggcc aggtatttat gaatttccat ttccatgaag cagcagccaa cctgcttaca 2820

attgctgccc tggatccggt tgctaaaata ccgattataa aacctgtgct gtagttggga 2880

gaggtggtat ttagcacctg gggaggaaaa gttgtcgatc accggggcgg tccgtcgggg 2940

ggaggaccgt cctgggccgg ggaatttggc ggtcggcagt taaaggcctt tattggctgg 3000

gatggtctcg tagtcaccga cccggccgta gacctgctgg ctgctttaca ggcttactac 3060

caggccgtcc agggggaatc ctgcggccgc tgcgtaccct gccgggtagg gaccagggtt 3120

atttacaatg tcctggtacg catcgccggg ggcgaaggcc ttccctccga tttagacctt 3180

ctccgacggg tggcctggat tgtacgggat ggttctctgt gcgagctcgg ccaggctggg 3240

gccaaagctg ttcttgattt tttagattat tatagcgagg ccttgcgacc tttcctggag 3300

gatagcggca gggtcgccgg gggtcaacgg agaccagggc ctggaggccg ggtgcaggtt 3360

ctggcttccg gacgggtcct ggtggggaac gaccgcggaa agggagcggc cgccgcttcc 3420

ccggcagccg gtttaaccta taaacctttt gttactgcgc cctgcctcaa gcggtgcccg 3480

gcccacctgg acatcccggc ttatattgac gccattaagg atggccgcta tgaggaatcc 3540

ctggccatca tccgccagcg gaccgccctg gccggtgtcc tgggacgggt ctgcgtccac 3600

ccgtgtgaag aaaactgccg ccgcggcaat gttgacgaac ccctggccat ccggggcctg 3660

aagcgcttcg tagccgatta tgaggtgaaa cgcggccgcc ggccggtcgc cgtctgcggg 3720

ggtaatctct ttacgggacc ctggcggccg gcaggacagg ccggtgggga agaaacaacg 3780

gccgtcactt caggcaagaa ggtagccatc atcggtgccg gaccggcggg tctcagcgcc 3840

gcctaccagc tcgccggtcg gggctataaa gtgactattt tcgaggcctt gccggtggcc 3900

ggcggtatgc tggcggtggg tattcccagc taccggttgc cacgggatat cctggctgga 3960

gagatcgagg ccatcaaggc tctgggcgta accatcaacc tcaacacccg ggtcggcgtc 4020

gacgtgacca tggaccaatt acagcgcgat tacgacgccg tctttattgc taccggcctc 4080

catgccagct cccgcatggg agtagccggc gaggatgaag gctatggagg atttatcccc 4140

ggggtcaagt tcctgcgaga tttgaacctg gaccggtgtc cttccctgga gggcaaggtg 4200

gtggccgtcg tcggcggcgg caatgtagcg atggattgcg cccgttccgc cctgcgccgg 4260

ggggcccggg aggtgcacct tatttaccgg cgttcccggg cggaaatgcc ggcccatgcg 4320

accgaggtca gggatgccga ggccgagggg gttatttatc acttcctggt taatcccacc 4380

gccctggtag cggaaaaagg caatatcaag ggtatgcagt gcgtccggat gaaacttggc 4440

gagccggacg attctggccg gcgccggccc gtacccgtgc cgggaacgga gtttttcctg 4500

ccctgcgata ttgtggtgcc ggctatcggc caggcggccg acctgtcttt cctggacggc 4560

cggatcgagg tgggcaaacg tggtaccatc agtgttgacc cggtgaccct ggctaccagt 4620

gtccccggcg tcttcgccgg cggcgatatc gtcctggggg ccaggacggt agttgaggcc 4680

gtggcccagg gcaaccgggc ggcagtttcc attgaccagt acctgcgcca ggggaccacc 4740

agtcccacgg tagaggacga gctggacgcg tggctggaaa aggtcggcgt ctatgacccg 4800

gaagaggatg taggaatcta cggcggccgg ccgcggcagg cagaaagggt ggcgccgttg 4860

gccgagcggg taaaggactt ccgggaagtt gaaggcggct tcgatttcta cgcaggcagg 4920

gccgaagccg aacgctgcct gcgctgttac cgggtcggga tgatggtcct ggccggagag 4980

ggggagagta atggttaatg aagtaagtag gaagcaggga gcaggggaaa gaaaattgac 5040

aactgtacaa gattaatcgc gtctctgagc aatgaccaaa tacatctacc tccacggttt 5100

tcttccagcc ccctatctgc gaaagcacaa gatattagca agcgtttcgc ccaaattcac 5160

atacagctaa caatccctga tctcaatgct ggtgaatttt ctcagttaac aatcacgcgc 5220

caaattcaac aagttgccgc aattttccct gataattctg aaccaataac gctgataggt 5280

tctagtttag gcggtttaac tgctgcttat ctaggacagc gatatttaca agtacaacgc 5340

ttagttttat tagcgccagt ttggtttttt atcccattgg ttgcccaaaa tgggtgaaga 5400

agctgtcaca agttggcaac aaacgatata ggttctctct tctgccgtta 5450

<210> 187
<211> 2324
<212> DNA
<213> Artificial Sequence

<220>
<223> Synthetic Construct- SEQ ID NO: 187, example 187: a designer
      hox-promoter-controlled 10-Formyl-H4 folate synthetase DNA
      construct (2324 bp)

<400> 187
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttgtccaa ggtacccagt gatattgaga ttgcccaggc agccaaaatg 240

aaaccggtca tggaactggc ccggggactg ggcatccaag aggacgaggt cgagctttat 300

ggtaagtaca aggccaagat ctccctcgat gtctatcgtc gcctcaaaga caagcctgac 360

gggaaactaa tcctggtaac cgccattacc cctactccgg ccggcgaagg gaaaactact 420

accagtgtcg gtctcaccga tgccctggct cgcctgggga aaagggtgat ggtctgcctg 480

cgggagccct ccctgggacc cagctttggt atcaaaggcg gtgccgccgg cggtggttat 540

gcccaggtag tacccatgga agatatcaac ctgcacttca ccggcgatat ccacgccgtc 600

acctatgccc acaacctgct ggcggccatg gtggataacc acctgcagca gggtaacgtc 660

ctgaatattg atccccgtac catcacctgg cgccgggtca tcgaccttaa tgaccgggct 720

ctgaggaaca tagtcatcgg cctgggtggc aaagccaacg gcgtaccgcg ggagacaggg 780

tttgacatct ccgttgcctc ggaggttatg gcctgcctgt gcctggccag cgacctcatg 840

gatctcaagg aacgtttcag ccgcattgtt gtcggctaca cctatgacgg caaaccggtc 900

accgccggcg atctggaggc ccagggttcc atggctcttc tcatgaagga cgccattaaa 960

cccaacctgg tccaaaccct ggagaatacg ccggccttta tccacggtgg tcccttcgcc 1020

aatatcgccc acggttgcaa cagcattatc gcaaccaaga cggccctgaa actggcggat 1080

tatgtcgtga cggaagccgg tttcggtgcc gacctgggtg ccgagaagtt ctatgacgtt 1140

aaatgccgtt atgccggctt taaacccgat gccacagtca tcgtggctac cgtccgcgcc 1200

ctcaagatgc acggcggcgt acccaaatca gacctggcca ctgaaaacct ggaagccctg 1260

cgggaaggct ttgccaacct ggagaaacac atcgaaaata tcggcaagtt cggcgtaccg 1320

gcagtcgtgg ccatcaatgc cttccccacc gataccgagg ccgagctaaa tctcctctac 1380

gagttgtgcg ccaaagctgg ggccgaagtt gccctctcgg aagtctgggc taagggcggc 1440

gaaggcggtc tggaacttgc ccggaaggtg ttgcagaccc tggagagcag gccatccaac 1500

ttccatgtcc tctacaacct ggacctgagt attaaagaca aaattgccaa aatcgccacc 1560

gagatctacg gggccgacgg cgtcaactat acggccgaag ccgacaaagc tatccagcgt 1620

tatgaatccc tgggctacgg caacctgccg gtggtcatgg ccaagaccca atactccttt 1680

tccgatgaca tgaccaagct cgggcggccg cggaacttta ccatcaccgt gcgcgaggtg 1740

cgcctctcgg ccggagcagg ctttatcgtc cccatcaccg gcgccataat gaccatgccc 1800

gggctgccca aacgcccggc ggcctgcaac atcgacatcg atgccgacgg cgtcattacc 1860

ggtcttttct agtgaagtaa gtaggaagca gggagcaggg gaaagaaaat tgacaactgt 1920

acaagattaa tcgcgtctct gagcaatgac caaatacatc tacctccacg gttttcttcc 1980

agccccctat ctgcgaaagc acaagatatt agcaagcgtt tcgcccaaat tcacatacag 2040

ctaacaatcc ctgatctcaa tgctggtgaa ttttctcagt taacaatcac gcgccaaatt 2100

caacaagttg ccgcaatttt ccctgataat tctgaaccaa taacgctgat aggttctagt 2160

ttaggcggtt taactgctgc ttatctagga cagcgatatt tacaagtaca acgcttagtt 2220

ttattagcgc cagtttggtt ttttatccca ttggttgccc aaaatgggtg aagaagctgt 2280

cacaagttgg caacaaacga tataggttct ctcttctgcc gtta 2324

<210>  188
<211>  1487
<212>  DNA
<213>  Artificial Sequence

<220>
<223>  Synthetic Construct- SEQ ID NO: 188, example 188: a designer
       hox-promoter-controlled 10-Methenyl-H4 folate cyclohydrolase DNA
       construct (1487 bp)

<400>  188
agaaaatctg gcaccacacc gcagaaatat aggggctagg agttgagggt actctggttc      60

gtcaagcatt tgggatgatt tcccctcaca agttcctcaa attattctcc tataaacaat     120

agatataagg tcaaaacttg agttatgagt gctgagtaaa aaattactct ccacgcctca     180

gaggtagata tgctagcgcc gggccgcctc gactgtattc ttcaagagca tggcaatggt     240

catgggacct accccgccgg gtacgggagt gatccaaccg gccttctggg ccgcactctc     300

gaagtggacg tcgcccacca gctttttctc gccgacccgg ttgataccta cgtcgataac     360

taccgcgccc tctttaatca tatccccggt aatcaactcc ggcttcccta cggccgcaat     420

gagaatgtcg gcctgccggc attcggctgc caggtccctg gtgcgggagt gacagatggt     480

caccgtggcg tggcgggcca ggagcatcat tgctactggc ttgccgacga tattgctccg     540

gccgacgacc actgcctttt tacccttggg atcaataccg gctttttcca gcaaaaccat     600

acagccgtgg ggtgtgcatg gatagaagca tttatcgccg ataaccaggt tgccgacgtt     660

ggccgggctg aacccgtcaa catccttttc cagggcgatg gcatcgatga ctttcttctc     720

atcaatatga tcaggcaacg gcaactggac caggatgccg tgaatcttgg gatctttatt     780

gagctggtcg atcagcttta agagttcggc ctggctggtc gccgccggca ggcggtgaac     840

ctcggagtag ataccgacct cctcacaggc gcggtgcttg ttacggacgt acacctggga     900

agccgggtct tcgcccacca ggactacggc caggcctggg ttaatacctt ccgcctttaa     960

ccgggatacc tcctccttta cttcagccct tacttcggca gcaatctttt tcccgtcgag    1020

gatctgggct ggcattgaag taagtaggaa gcagggagca ggggaaagaa aattgacaac    1080

tgtacaagat taatcgcgtc tctgagcaat gaccaaatac atctacctcc acggttttct    1140

tccagccccc tatctgcgaa agcacaagat attagcaagc gtttcgccca aattcacata    1200

cagctaacaa tccctgatct caatgctggt gaattttctc agttaacaat cacgcgccaa    1260

attcaacaag ttgccgcaat tttccctgat aattctgaac caataacgct gataggttct    1320

agtttaggcg gtttaactgc tgcttatcta ggacagcgat atttacaagt acaacgctta    1380

gttttattag cgccagtttg gttttttatc ccattggttg cccaaaatgg gtgaagaagc    1440

tgtcacaagt tggcaacaaa cgatataggt tctctcttct gccgtta                  1487

<210>  189

<211>  1487
<212>  DNA
<213>  Artificial Sequence

<220>
<223>  Synthetic Construct- SEQ ID NO: 189, example 189: a designer
       hox-promoter-controlled 10-Methylene-H4 folate dehydrogenase DNA
       construct (1487 bp)

<400>  189
agaaaatctg gcaccacacc gcagaaatat aggggctagg agttgagggt actctggttc      60

gtcaagcatt tgggatgatt tcccctcaca agttcctcaa attattctcc tataaacaat     120

agatataagg tcaaaacttg agttatgagt gctgagtaaa aaattactct ccacgcctca     180

gaggtagata tgctagcgcc gggccgcctc gactgtattc ttcaagagca tggcaatggt     240

catgggacct accccgccgg gtacgggagt gatccaaccg gccttctggg ccgcactctc     300

gaagtggacg tcgcccacca gctttttctc gccgacccgg ttgataccta cgtcgataac     360

taccgcgccc tctttaatca tatccccggt aatcaactcc ggcttcccta cggccgcaat     420

gagaatgtcg gcctgccggc attcggctgc caggtccctg gtgcgggagt gacagatggt     480

caccgtggcg tggcgggcca ggagcatcat tgctactggc ttgccgacga tattgctccg     540

gccgacgacc actgcctttt tacccttggg atcaataccg gctttttcca gcaaaaccat     600

acagccgtgg ggtgtgcatg gatagaagca tttatcgccg ataaccaggt tgccgacgtt     660

ggccgggctg aacccgtcaa catccttttc cagggcgatg gcatcgatga ctttcttctc     720

atcaatatga tcaggcaacg gcaactggac caggatgccg tgaatcttgg gatctttatt     780

gagctggtcg atcagcttta agagttcggc ctggctggtc gccgccggca ggcggtgaac     840

ctcggagtag ataccgacct cctcacaggc gcggtgcttg ttacggacgt acacctggga     900

agccgggtct tcgcccacca ggactacggc caggcctggg ttaatacctt ccgcctttaa     960

ccgggatacc tcctccttta cttcagccct tacttcggca gcaatctttt tcccgtcgag    1020

gatctgggct ggcattgaag taagtaggaa gcagggagca ggggaaagaa aattgacaac    1080

tgtacaagat taatcgcgtc tctgagcaat gaccaaatac atctacctcc acggttttct    1140

tccagccccc tatctgcgaa agcacaagat attagcaagc gtttcgccca aattcacata    1200

cagctaacaa tccctgatct caatgctggt gaattttctc agttaacaat cacgcgccaa    1260

attcaacaag ttgccgcaat tttccctgat aattctgaac caataacgct gataggttct    1320

agtttaggcg gtttaactgc tgcttatcta ggacagcgat atttacaagt acaacgctta    1380

gttttattag cgccagtttg gttttttatc ccattggttg cccaaaatgg gtgaagaagc    1440

tgtcacaagt tggcaacaaa cgatataggt tctctcttct gccgtta                  1487

<210>  190
<211>  1565

<212>  DNA
<213>  Artificial Sequence

<220>
<223>  Synthetic Construct- SEQ ID NO: 190, example 190: a designer
       hox-promoter-controlled 10-Methylene-H4 folate reductase DNA
       construct (1565 bp)

<400>  190
agaaaatctg gcaccacacc gcagaaatat aggggctagg agttgagggt actctggttc      60

gtcaagcatt tgggatgatt tcccctcaca agttcctcaa attattctcc tataaacaat     120

agatataagg tcaaaacttg agttatgagt gctgagtaaa aaattactct ccacgcctca     180

gaggtagata tgctactcta ccttgggacg gggatacagg ccggcttcct tgacgatggt     240

ggggatgata tcttcccaca tgacggccat gatatggacg ccggctacac cctcgatgga     300

ctgcaggtgt ttaatctgtt ccacacagat ggccacccct tcggccttgg ggtcggaggc     360

tttttccatg cggttgacga tttcgtcggg gacaatcatc ccggccaccg attgttgcat     420

atatttggcg gcccgggcgg atttcagggg catgacgccg gcaataattt tggctctttt     480

atgcaggccg cgctcccgga ccagggccat aaagcgttca aaacgctcca tatcaaaaat     540

gcactgggtt tgaataaagt cggcgccggc attgatcttc ttctccaggc gcatgacccg     600

gaactcaaag gggtcggcaa aggggttggc ggcggcgccg atgaagaagc ggggttcctg     660

gcccttaatc tcttcaccgc aggcgaattt tttctcatcc cggaggtcct tcacgatccg     720

gatcagctgg agggaatcca cgtcatggac gtttttcgcc gtcggatggt taccaaaaga     780

ttgatggtcg ccggacaggc agaggacgtt ccgcatcccc aggctgtagg cacccaggag     840

atcactctgg agggcgatac ggttgcggtc ccgaaccgtc atctggataa tgggttcgcc     900

cccggcctgg aggacgtgga ccccagcggc gatactcgac aggcggacga tggccgtctg     960

gttgtccgtc aggttcatgg catcaacgta gtccttgagg agagcggcgt gcttcttgat    1020

ctctgtagga tcggcgtgct tcggcggtcc aatctcaccg ctgaccagaa attgcccctg    1080

ggagaggatt ttcgccatct tgctttcaac cattgaagta agtaggaagc agggagcagg    1140

ggaaagaaaa ttgacaactg tacaagatta atcgcgtctc tgagcaatga ccaaatacat    1200

ctacctccac ggttttcttc cagcccccta tctgcgaaag cacaagatat tagcaagcgt    1260

ttcgcccaaa ttcacataca gctaacaatc cctgatctca atgctggtga attttctcag    1320

ttaacaatca cgcgccaaat tcaacaagtt gccgcaattt tccctgataa ttctgaacca    1380

ataacgctga taggttctag tttaggcggt ttaactgctg cttatctagg acagcgatat    1440

ttacaagtac aacgcttagt tttattagcg ccagtttggt tttttatccc attggttgcc    1500

caaaatgggt gaagaagctg tcacaagttg gcaacaaacg atataggttc tctcttctgc    1560

cgtta                                                                1565

<210>  191
<211>  1442
<212>  DNA
<213>  Artificial Sequence

<220>
<223>  Synthetic Construct- SEQ ID NO: 191, example 191: a designer
       hox-promoter-controlled Methyl-H4 folate: corrinoid iron-sulfur
       protein Methyltransferase DNA construct (1442 bp)

<400>  191
agaaaatctg gcaccacacc gcagaaatat aggggctagg agttgagggt actctggttc        60

gtcaagcatt tgggatgatt tcccctcaca agttcctcaa attattctcc tataaacaat       120

agatataagg tcaaaacttg agttatgagt gctgagtaaa aaattactct ccacgcctca       180

gaggtagata tgttaatccg ctaacaaatc tttacgatat gcctttaaaa agttcttgcc       240

gaaacggtcg ttacccagca gtaaatcggt ggtggtgact gcagtcatga tttttccatc       300

caagggatcg agaatggccg cgtccatgcc gctcgccata catattgtca ggaagtaacg       360

gttgatgagc ttcctcttcg gcatgttaaa cgagatgttg cttaaaccgg atactgtctt       420

cactttaaat aacctcttga tttccctcag gcactggaaa aacatcaccg cgttggtgtg       480

gtttacggcc agggggagaa ccagcggatc gatatacagg ttgctcaaat cataattctt       540

cctgctcagg gtctctatca gtccctcagt tatcttgatc ctttcttcag ccgttttcgg       600

gataccattg tcgtcaaccg tcaggccgat taccgggcac tggtattcga gtacaagtgg       660

caatacttct tccagccgct ttttttccat ggatatggag ttgattatca ccttgctctt       720

atcgcccttg acggtctcca gacccttttt tattgcctcc cctgctgtgc tatcaatgca       780

taaaggtaca tcagttactt cttgcactgt atgcacaagc cactccatat cggccacttc       840

gtcacagtgg gcggtattta gatccaggta atctgctccc gcttccgcct gcctgcgggc       900

caggtcctgc acggccgcgg cgtttttgtc attaatgatt tgacggacac tggggatcgc       960

gctatttagt ttttcaccga tgattaacat tgaagtaagt aggaagcagg gagcagggga      1020

aagaaaattg acaactgtac aagattaatc gcgtctctga gcaatgacca aatacatcta      1080

cctccacggt tttcttccag ccccctatct gcgaaagcac aagatattag caagcgtttc      1140

gcccaaattc acatacagct aacaatccct gatctcaatg ctggtgaatt ttctcagtta      1200

acaatcacgc gccaaattca acaagttgcc gcaattttcc ctgataattc tgaaccaata      1260

acgctgatag gttctagttt aggcggttta actgctgctt atctaggaca gcgatattta      1320

caagtacaac gcttagtttt attagcgcca gtttggtttt ttatcccatt ggttgcccaa      1380

aatgggtgaa gaagctgtca caagttggca acaaacgata taggttctct cttctgccgt      1440

ta                                                                    1442

<210> 192
<211> 2942
<212> DNA
<213> Artificial Sequence

<220>
<223> Synthetic Construct- SEQ ID NO: 192, example 192: a designer
      hox-promoter-controlled Corrinoid iron-sulfur protein DNA
      construct (2942 bp)

<400> 192
agaaaatctg gcaccacacc gcagaaatat aggggctagg agttgagggt actctggttc      60

gtcaagcatt tgggatgatt tcccctcaca agttcctcaa attattctcc tataaacaat     120

agatataagg tcaaaacttg agttatgagt gctgagtaaa aaattactct ccacgcctca     180

gaggtagata tgatggcttt aacaggatta aatatattta aattaacacc aaaaaagaat     240

tgtaaggatt gtggtttccc tacttgtcta gctttttcaa tgaaagtagc agcaggagct     300

gtggaaatag gaaaatgtcc tcatatgagt gacgaggcaa tggaaaaatt agctgaagct     360

actgcaccaa ttatgaagac aataactatt ggtaagggag ataatgaata taaattaggt     420

ggagaaactg ttttattccg tcatgaaaaa acttttgtaa atagaaatag atttgcagtt     480

gcattttccg atagtatgga tgatgcagaa gtagatgcta agatccaaca tataaaagat     540

gtagattatg ttagaatcgg tgaacaaatg aaaaccgaat ttgctgcaat aaaatatgca     600

ggaaataaag acaaatatct tgctttaata aacaaaataa aagcaagtgg agtaaaagta     660

gcttatgccc tagtttgtga agatgtagca gtaatgaaag aagctcttcc actagttaaa     720

gatgaaaatc cattagttta tggagctaat aaagataatt ttaaagaaat ggttgaactt     780

gtaaaaggag ataaattagc tttaggtgta aaggcagacg gattggaagc tctttatggt     840

ttagtagaag aaatacaaaa attaggatat aagaacttag tacttgatcc aggtggaaaa     900

tccattaaag aagcttttga aaatacagtt caaattagaa gaataaatat tgaaggtcag     960

gatagaactt ttggatatcc ttctataata ttcctagatg aacttactaa agctgataaa    1020

tttatggaag tagctttatc tacattattt actttgaaat atggttcatt acttgtttta    1080

agtgatatgg attattcaag agcacttcct ctttatagta taagacagaa tgtatttaca    1140

gatccacaaa aaccaatgac agttgatttg ggtatacatg gaattaacaa cccagatgaa    1200

aactcaccag tattatgtac tgttgacttt gctcttactt acttcctagt ttcaggagaa    1260

gttgagagat ctaaagttcc agtatggatg gttataccag atgcaggcgg atattctgtt    1320

cttacatctt gggctgcagg taaatttact ggtgctgcaa tagctgatga aataaagaaa    1380

tgtggaatag cagagaagac taagaacaga actcttttaa tcccaggaaa ggttgcagtt    1440

ttgaaaggtg aattagagga acttcttcca gactggaata tagtaattag tagtacagaa    1500

gctatgttta ttcctaagtt attaaaagag ttaactgcta agtaaatgtt caaaaaacca    1560

acacaaaaat tttcaggcaa aattggtgaa gttgaaattg gaacaggaga aaaagcatta 1620

aaattaggag gagaatcagt attaccattt tatacttttg atggagatac aggaaatacc 1680

ccaaaagtag gtatggaaat tttagatgta tatccagaag actggataga tcctttaaag 1740

gacatataca aggatgttgc aaaagaccct gttaaatggg cacaatttgt agaagaaaaa 1800

tatagtccag attttatatg cctaagactt ataagtgctg atccaaacgg tacagatgct 1860

gcaccagaag attgtgctaa aacagctaaa gcagtagttg aagctataaa aactccatta 1920

gtagttgcag gtacaggaaa tcatgaaaaa gatgcaaaat tatttgaaaa agttgctcag 1980

gaaactgaag gacacaatat acttttaatg tcagcagtag aagataacta taagtcagta 2040

ggagctgcag gtgtaatggc ttataatgac aaagttgtag ctgaatcttc agttgatata 2100

aaccttgcaa aacaaataaa tattttaatg aatcaacttg gaatagacaa tacaaagttt 2160

gttgacaacg taggatgtgc agcaggtgga tatggttatg aatatgttat atcaacttta 2220

gacagagtaa agcttgcagc acttggtcaa gatgataaaa ctcttcaagt tcctataata 2280

agccctgttt ctttcgaagc ttgcaaagta aaagaagcaa tggattcaga agaagattca 2340

ccacaatggg gaagtcagga agacagaaca gtttccatgg aagttgcaac agcatccgga 2400

gtattagcat caggaacaga tgctgtaata ttacgtcatc caaaatctgt agaagtaatt 2460

agaaacttta ttaaggaatt attaggttaa tgaagtaagt aggaagcagg gagcagggga 2520

aagaaaattg acaactgtac aagattaatc gcgtctctga gcaatgacca aatacatcta 2580

cctccacggt tttcttccag ccccctatct gcgaaagcac aagatattag caagcgtttc 2640

gcccaaattc acatacagct aacaatccct gatctcaatg ctggtgaatt ttctcagtta 2700

acaatcacgc gccaaattca acaagttgcc gcaattttcc ctgataattc tgaaccaata 2760

acgctgatag gttctagttt aggcggttta actgctgctt atctaggaca gcgatattta 2820

caagtacaac gcttagtttt attagcgcca gtttggtttt ttatcccatt ggttgcccaa 2880

aatgggtgaa gaagctgtca caagttggca acaaacgata taggttctct cttctgccgt 2940

ta 2942

<210> 193
<211> 4859
<212> DNA
<213> Artificial Sequence

<220>
<223> Synthetic Construct- SEQ ID NO: 193, example 193: a designer
      hox-promoter-controlled CO dehydrogenase/acetyl-CoA synthase DNA
      construct (4859 bp)

<400> 193
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgactga ttttgataaa atcttcgagg gtgctattcc agaaggtaaa 240

gagccggtag ccctgttccg ggaggtttac cacggcgcca ttacagctac cagttacgcg 300

gaaatccttt taaaccaggc catccggacc tatggtcccg accatcccgt cggttatcct 360

gatacagcct attacctgcc ggttattcgc tgtttcagcg gggaagaggt caaaaaactg 420

ggggatttac cacctatttt aaaccgcaag cgagcgcagg taagccctgt cctgaatttc 480

gagaatgccc gcctggccgg ggaagccacc tggtatgcgg ccgagatcat tgaagccctg 540

cgttacctta aatataagcc tgatgaaccc ctcctgcccc caccctggac gggtttcatc 600

ggcgacccgg ttgtccgccg tttcggtatc aagatggtcg actggaccat tccgggtgaa 660

gctattatcc tgggtcgagc caaagactcg aaggccctgg ccaaaatcgt caaggaactc 720

atgggtatgg gctttatgct cttcatctgt gatgaagcgg tagaacagct gctggaagaa 780

aacgtcaaac tggggattga ctatatcgcc tatcccctgg ggaacttcac ccagattgtt 840

catgccgcca actatgccct gcgggctggt atgatgttcg gtggcgttac cccgggtgcc 900

cgtgaagaac agcgcgatta ccagcgccgc cgtatccgcg cctttgtcct ttatctcggc 960

gagcatgaca tggtcaagac ggctgccgcc ttcggggcca tctttaccgg ctttccggta 1020

atcaccgacc agcccctacc ggaggacaaa cagatcccgg attggttctt cagcgttgag 1080

gactatgata aaatagtcca gatagccatg gagacccgtg ggatcaagct caccaagatc 1140

aagttggatc tgcccattaa ctttggccct gcctttgagg gcgagagtat ccgtaagggc 1200

gatatgtacg tagaaatggg cggcaaccgg acgccggcct ttgagctggt acgcaccgtt 1260

tcggaatccg agatcactga tggtaagatt gaagtcatag gtcctgatat tgaccagata 1320

ccggaaggga gcaaactgcc cctgggcatt ctggtggaca tctatggccg taaaatgcag 1380

gccgattttg aaggagtcct cgaacggcgc atccacgact tcatcaacta cggtgaaggt 1440

ctctggcaca ccggccagcg taacatcaac tggttgcggg tcagcaaaga tgccgtagcc 1500

aagggtttcc gtttcaagaa ctacggtgaa atcctggtag ccaaaatgaa agaagaattc 1560

cccgccattg tggaccgggt ccaggtaacc atttttaccg atgaagccaa ggtcaaagaa 1620

tatatggagg tcgcccggga gaaatacaag gaacgtgacg accgcatgcg cggccttacc 1680

gatgaaacag tggatacctt ttactcctgc gtcctctgcc agtcctttgc ccccaaccat 1740

gtgtgtattg tcaccccgga acgggtgggc ctgtgtggag ccgtaagctg gctggacgcc 1800

aaggcgtcct atgaaatcaa ccatgccggt cctaaccagc ccatccctaa agaaggggaa 1860

attgatccca ttaagggtat ctggaagagt gtaaatgact atctctatac agcttccaac 1920

cgtaacctgg aacaggtctg cctgtacacc cttatggaga atcccatgac ctcctgcggt 1980

tgctttgagg ccattatggc catcctgccg gagtgcaacg gcatcatgat taccaccagg 2040

gatcacgccg gcatgactcc ttcggggatg accttctcta ccctggccgg gatgatcggc 2100

ggtggcaccc agaccccggg ctttatgggc atcggccgca cctatatcgt cagcaaaaag 2160

tttatttccg ccgatggtgg tatcgcccgg atcgtctgga tgcccaaatc tctgaaggat 2220

ttcctccacg acgagtttgt acgtcgtagt gttgaggagg gcctgggaga ggactttatc 2280

gataaaatag ctgatgagac catcggtacc accgtggatg aaatcttgcc ctacttggag 2340

gaaaagggac acccggcctt gaccatggat cccattatgt gaatgcccag gttccgcgat 2400

ctctcccata attgtaggcc ctcagaggca ccacgggtca tggaacccaa aaacagggac 2460

cgcaccgtag atccggcggt cctggaaatg ctggttaaaa gtaaggatga caaagtcatc 2520

accgcttttg accgcttcgt cgcccagcaa ccccagtgta aaatcgggta tgaaggtatt 2580

tgctgccgtt tctgcatggc cggtccctgc cgtatcaagg caaccgatgg ccctggcagc 2640

cgtggtattt gcggcgcttc tgcctggacc attgtcgccc gtaatgtagg tttaatgatc 2700

cttaccggtg ccgccgccca ctgcgaacac ggcaaccata tagcccatgc cctggtagaa 2760

atggccgaag gtaaagctcc tgattatagc gtcaaggacg aggccaagct caaagaagtc 2820

tgccgacggg tgggtattga ggtagaaggc aaaagcgttt tggaactggc ccaggaggta 2880

ggcgagaagg ccctggaaga cttccgccgc ttgaagggtg aaggtgaagc cacctggctg 2940

atgaccacta ttaatgaggg ccggaaagaa aagttccgta cccacaatgt tgttcccttt 3000

ggtattcatg cctctatttc cgagctggtc aatcaggccc atatgggtat ggataacgac 3060

cctgttaacc tggtcttcag cgccatcagg gtagccctgg ctgactatac gggtgaacat 3120

atagctactg atttctccga cattctcttc ggtactcccc aaccggtggt cagcgaagcc 3180

aacatggggg tcctggatcc ggatcaagtc aacttcgtcc tccatggcca taatcccttg 3240

ttgagtgaga ttattgtcca ggcggcgcgg gagatggaag gagaggccaa ggccgccggt 3300

gccaaaggca tcaacctggt gggtatctgc tgcaccggta acgaagtcct gatgcgccag 3360

ggtatcccct tggttacttc cttcgcctcc caggaactgg ccatctgcac cggagctatt 3420

gacgccatgt gcgtcgacgt ccagtgtatt atgccttcca tcagcgccgt agccgagtgt 3480

tatcataccc ggatcatcac tactgccgat aacgccaaga ttcccggtgc ctaccatatc 3540

gactatcaaa cggctacggc tatcgaaagc gcgaaaaccg ccatccgcat ggccatcgag 3600

gcattcaagg aaagaaaaga aagtaaccgt ccggtttaca tcccccagat taagaaccgg 3660

gtagtcgccg gctggagcct tgaagccctg accaaactcc tggctaccca gaatgctcaa 3720

aatcccatcc gggtactcaa ccaggccatc ctggacggtg aactggctgg cgtagcctta 3780

atctgcgggt gtaacaacct caaagggttc caggataact cccacctgac ggtaatgaaa 3840

gaactgctga aaaataatgt ctttgtggtg gctacgggtt gctccgccca ggccgccgga 3900

aagcttggcc tcctggatcc ggccaatgtg gaaacctact gcggcgatgg tctcaagggc 3960

ttcctgaaac gcctgggtga aggcgccaac atcgaaatcg gcctgccgcc tgtgttccac 4020

atgggttcct gtgtggataa ctcccgggcc gtcgacctct tgatggccat ggccaacgat 4080

ctgggcgtag ataccccgaa ggtgcccttc gtagcctcgg ccccggaagc catgagcggt 4140

aaggctgccg ccatcggcac ctggtgggta tccctcggcg taccgaccca tgtcggcacc 4200

atgcccccgg tagaaggtag cgacctcatt tatagtattc taacccagat agccagcgac 4260

gtttatggtg gttacttcat cttcgaaatg gatccccagg tagctgcccg gaagatcctt 4320

gacgccctgg aataccgcac ctggaagctg ggcgtacaca aagaggtagc tgaacgttat 4380

gaaaccaaac tctgccaggg ttactagtga agtaagtagg aagcagggag caggggaaag 4440

aaaattgaca actgtacaag attaatcgcg tctctgagca atgaccaaat acatctacct 4500

ccacggtttt cttccagccc cctatctgcg aaagcacaag atattagcaa gcgtttcgcc 4560

caaattcaca tacagctaac aatccctgat ctcaatgctg gtgaattttc tcagttaaca 4620

atcacgcgcc aaattcaaca agttgccgca attttccctg ataattctga accaataacg 4680

ctgataggtt ctagtttagg cggtttaact gctgcttatc taggacagcg atatttacaa 4740

gtacaacgct tagttttatt agcgccagtt tggtttttta tcccattggt tgcccaaaat 4800

gggtgaagaa gctgtcacaa gttggcaaca aacgatatag gttctctctt ctgccgtta 4859

<210> 194
<211> 6428
<212> DNA
<213> Artificial Sequence

<220>
<223> Synthetic Construct- SEQ ID NO: 194, example 194: a designer
 hox-promoter-controlled F420 systhesis enzymes DNA construct
 (6428 bp)

<400> 194
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgaaagt attgattgat ggagaggatt ttgacagcgg cgaccattat 240

gaagtaaaaa acccatatga tggtgagctt gtagatactg ttccaatttg ttttaggcaa 300

gatgtagata gggccataga tgcagcaaat agggctaaaa aggaattgca ggatatgtct 360

gcaaaggaag tgtccaataa tctttattct gcttatgaag agctcgaatc aagaagcgaa 420

gaattggcaa agcttatagt cttggaggct ggaaagccaa ttaagcaggc tattggagag 480

cttaacaggt cttgtgagac attgaagttt gcagcagagg aagccaaaag aatctatgga 540

gaatctgtcc ctatagatgc tacaggttct gtagatgagc gttttttggc atttacaaag 600

aaagtacctt taggtgtcgt tggagcaata actccattca attatccggt taatcttgca 660

ttgcataaga tagctccagc cattgcagca aagaatgctg ttgtggtaaa gccttccact 720

gaagctccat tagctgcact taaattggtt gaaattattg ataatcattt cccaaatggg 780

gtgataaact ctgtaactgg ctatggaagt gaagttggag atgcattggt tgtttcagag 840

ggcattgata agatatcatt tactggaagc attgccactg gattgtttat ctcttctcga 900

gcaggaatga agaaattgac tttggagctt ggtggaaacg atccattggt cgttcttgaa 960

gatgcagata ttgaaaaggc agttagtgct gcggtgtccg gcgcatttct attctccgga 1020

caggtttgca taggtgttaa aagaataata ttggataata agattgcaga tgagttcatt 1080

gacttatttg ttaaagaggc aagtaaattg aagatgggca atcctatgga tgagtctaca 1140

gatatcggtc cgcttataaa tgaaaatgct tcaatcaatg ttgaaactgc agtaaacaat 1200

gctataaaag atggggcaga acttattttg ggtggaaacc gtaaggactg cttctatgag 1260

ccaacaatat tggatggtgt caatatgtct atggaccttg ttgcaaatga aacattcgga 1320

ccagttgcac ctatcataag ggtagataga atagatgagg caataaaagt ggcaaatgac 1380

accagatacg gtcttcaagc aggtgttttc actgaaaatg ttcatagtgc attaagatgt 1440

gcaaatgaga tagatgcagg ttcagtctta atcaataagg aatccacttt cagaacagat 1500

gccatgccat ttgggggatt caaatcaagt ggaatgggta aggaaggaat taaatatgca 1560

gttgaggata tgtgcaagtt taaattgatt gcttttaatt gtaggtgaat gaagacctgc 1620

gccataatac ctgtttcaag gtttgcacat gcaaagacac ggctatcccc cacactttca 1680

ccgtcagaac gggaggggct gctcagggcc atgctgatgg atgtttcaga ggccctcagg 1740

agacacgttg accgtgtgct ggtgatcagt gcagatgcag atgtccttga atacgcctac 1800

agccttggag tcgatatact tgaggagagg ggtagaaagg acctcaacgg tgccctggaa 1860

caggctgctg agttctgcat acccgaattt gagagggtca tgataacacc ctcagacata 1920

cccctcatag ggaaaaccga tatcactgaa ctcctccaca gggcatccca ctttgatgtt 1980

gtgatagccc ctgccaaggg tggcgggacc aacaccctga tattcaggcc agggtccatg 2040

aggctgaggt ttggtgactg cagcttcttt gagcatgtga gtgaagctga gagtaggggt 2100

ctcagcgtct cagtgtatga ctcattctac ctttcactgg acgttaacac agcagaggac 2160

ctcggggaga taatactcca tggagaaggg acccatgcaa gggagtacct cagaaagctc 2220

agtttcatcg tgaggccctc aaggggttct gacaggcttg aggtttcaag atcattctag 2280

atgaagataa ctattctttc aggcggaact ggaaccccaa aattaataca gggttttaaa 2340

gaaattcttc caaacgaaga tatttcggtt attgtaaaca ctggtgaaga tacttatatt 2400

ggcgacattt acctgtcccc tgatattgat actgttttgt acacgttttc taatctgata 2460

aatgacgaaa cctggtatgg gctaaaaggg gatacattct tctgccatga acagcttaaa 2520

aattttggat ttgacgaagt tttgaggatt ggagataaag atcgggcatt aaaaatgcat 2580

aaaacatctc ttttgaaaaa aggagtacct atgtctgaaa tcgtagatat cgagagaaaa 2640

tcattatcaa taaactcaaa aatttatcca atgtccgatg aaaaaattga atccaaagtt 2700

ttaatcgaag aaaacaatga aaaaatcctt ttaaagtttc acgattttgg ggtgtcaaga 2760

cgcggaaatg caaaagtttt agatatattc tacgaaaact caaattatgc aaaagcagca 2820

gacggagttt taaaagcaat tgaggaaagc gattttgtga ttattggtcc atcaaacccg 2880

ataacctcaa ttggacctat tttaagcatt tctgaaataa aagatgcatt aaaagaaaag 2940

ctggtttttg cagtttcgcc aatagttggt gaaaatcctg taagtggacc tgctggaacg 3000

ctgatgcatg caaaaggcta ccctgtgaat gcagttggag tttatgaata ttataaagat 3060

attgtagatg ttttagtttt agacaatagt gatattaaca aaaagaaaga aatgaattgt 3120

gaagttttat atgctaatac gataatgaag actattgatg ataaaataac tctcgcaaga 3180

aatattctcg actattataa atccagataa atgtcagttc aggtaacggg aatttccgga 3240

atcccgctca ttcagaaagg cgatgatctc ccggcaataa tctgcagaaa taccacgttc 3300

gaagacgagg atatcctttg tatagcatca acgatcgttt cgaaagcaaa agggtatacc 3360

cgcgctctcg cagatatcac cccttcgccg gatgccctgc ggatctccgg cctcaccaaa 3420

gaagaccccc ggttcatcca ggcgatcctt gattcatcca ccgagatcat ccaggagtat 3480

ccgtttatcc tctccgaagt cccctgcggc cacgtaggtg tgcgcgccgg tgtggacaat 3540

agtaatatag aaggcgaaaa tatcatcatc cttccaaaag acccctcggc cgagtgcaga 3600

gaaatacgtg actcaataaa aaagatcacg ggaaagaatg tcggcgtcat cgtgaccgac 3660

acctgcggca gggcatttcg ccgcggtcag tgcggaaacg ctatcggctg ggcaggcatg 3720

accgcgatcc gtgactttag aggcgaccac gatctgttcg gtctggaact cgaaatcacg 3780

gaagaagcgg tcgtcgatga gatcgccgcc ttctccaact tcattatggg ggagagcaac 3840

aacgggatcc cggccgtgaa attttccggc tgcggtacct ggaaaggcca cgactcgctc 3900

tacttcacca aagaagaaga cctgatcaga aaggctatga aacgataatt aaacacaata 3960

cccctctccg tcagcgtgtt tcctgatgat gggctccagc ctttccgggt accagcccat 4020

tatcacgtac ttcgggtaca gcgggagccg ctcccgcagc ttcaggccca tggcttcgag 4080

ctcgtccagc gtgggccacc tgcactccgg gttgatgtag tcctcggtga ccggcgagat 4140

gccgccgatg tcgcccgcgc cagcctccag gaattcgagc aggtgagacg tcaggttagg 4200

cggcgcctgg agctcgatat cgcccggtag tatctcgcgg gccatcctta aaacacttat 4260

catgtcatca acggtgggcg gttccacgct gcccatccgc gtctcgggct tgggcacgaa 4320

gttctggacg atgacctcct ggatatggcc gtacttttta tgtatatccc ggatggcctc 4380

aagggactgg cggcggtcgt cccaggtctc ccctatgccg accaggatgc cggtagtgaa 4440

cggtatgcgg agcttccccg cgctctcgat gaacccgagg cggagcgagg gctttttcat 4500

ggggctcagc ctgtgggcag gcaggtccgc cgtcgtctcc agcatgaggc ccatgctggc 4560

attgacgtcc tttagtcgtt ccagctcccc gtatggcagc acgccgccgt tcgtgtgggg 4620

tagtatgcca agctcgatgg cgtcgaggca gaggtcgtag acataatcgg tgagcgtctc 4680

atacccgtac ctgttcagct ccttaaagaa aaggggatcc gacggcgagt ccccgtatgt 4740

gaaaagcgcc tccaggcagc ccaggcctgc ggccttcttg agcatggcgt gggcgatctc 4800

gggccggatg acgaaggcct cacgcgatcc gacgtcccgc cggaacccgc agtagccgca 4860

gttgttcctg cacatgttcg tgaccgggac gaacacgttc cgtgaatagg tgatgaattc 4920

tttagacatt cagatatgct cgtaaagcgt attcctctcc acgggcaccc ggtcgatggc 4980

ccgaataagc tggatgaact cttcctttga gaggtactcg ccgtgctcgg agccgccggc 5040

tatggagatc ttgtcctcga tcatggtgcc gccgaagtcg ttggccccgg cgcagagggc 5100

ggcctgcgat agtttgacgc ccatcttgat ccaggagacc tggatgttgg ggatgtcctg 5160

tccgaagatg acccgggcca gcgcgtgcag gcgcaggtcg tccatgccgc tcacgccttt 5220

agactggccg ctgagccggt tgttctcgtg catgaaggtg agaggtatga actcggtgaa 5280

gccgtgagtc tcgcgctgga tgtcccttag aaggaacatg tggtcgatgc ggtcctccca 5340

ggtctccagg gtgccataca tgatggtgga cgtggtcctt atgcccagac ggtgggcgtc 5400

cttgatgatg tcgacccact ctcgcgttga cacctttctg ggacagatct tttcccggac 5460

cgagtcgacc aggatctcgg ccgaggcccc ggtgatcgtg ttcagccccg ccttcttgaa 5520

ggcggccatg gcctctgccg gagtgacgcc cgacactttc gccgcatagt ggacctccat 5580

cggggacagg gcgtggatca tgatatcgta cttcgacttg atggcttcga agaggtcgca 5640

gtagtactcc acggtcatgt agggcatgac acctgagatg aggcacagct cggtggcgcc 5700

gatatccttt gcctcgccga ccctctgcag gacatcatcg gtgctgagct taaagccgat 5760

gttgttcctg aaggagcaga agtcgcaccc gatcatgcac cggtcggtga tctcgacgag 5820

ccggttcacg acgtaggtga tctcatcgcc ggcgatcgtc tttcgcagct cgttcgccgt 5880

gtcgaagagc tcgaatgggt tggctttcac gagctcaagc gcgtccttct tcgtgatatc 5940

ccctgcgagc gagcgctcat agatgtcctt tagcattgaa gtaagtagga agcagggagc 6000

aggggaaaga aaattgacaa ctgtacaaga ttaatcgcgt ctctgagcaa tgaccaaata 6060

catctacctc cacggttttc ttccagcccc ctatctgcga aagcacaaga tattagcaag 6120

cgtttcgccc aaattcacat acagctaaca atccctgatc tcaatgctgg tgaattttct 6180

cagttaacaa tcacgcgcca aattcaacaa gttgccgcaa ttttccctga taattctgaa 6240

ccaataacgc tgataggttc tagtttaggc ggtttaactg ctgcttatct aggacagcga 6300

tatttacaag tacaacgctt agttttatta gcgccagttt ggttttttat cccattggtt 6360

gcccaaaatg ggtgaagaag ctgtcacaag ttggcaacaa acgatatagg ttctctcttc 6420

tgccgtta 6428

<210> 195
<211> 1778
<212> DNA
<213> Artificial Sequence

<220>
<223> Synthetic Construct- SEQ ID NO: 195, example 195: a designer
      hox-promoter-controlled Pyridoxal phosphate-dependent L-tyrosine
      decarboxylase(mfnA for methanofuran synthesis) DNA construct
      (1778 bp)

<400> 195
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttgaggga gcatggggtc gacgaagata cgataatacg ggagctaaag 240

ggcgcctgtg cgaggaacgt cccgtacgag cgcgtgctca gctccatgtg cacgaccccg 300

catcccatcg ccattaaagc ccataaagag ttcatcgtct ctaacctggg cgaccccagg 360

ctttttccgg gtacggcctc gctcgagcat gcctgtatcg gcatgctggg cgagcttttg 420

catttgccat ccgccgtggg ctatatcact accgggggca ccgagagcaa tattcaggcc 480

cttcgtacgg cccgtcagtt aaaacacgtc gacccgggca aggccaacat cgtgctgccc 540

gagtccgcac attattcgtt tgataaggcg gcccagatgc tgggcgtatc cctccggcgc 600

acaccgcttg acgacgaaat gaaagccgac atggacgcca tggccggcct cgtcgataaa 660

aacaccatcg ccctcgtggc cgtcgccggc accacggagt tcgggcaggt cgaccccata 720

ccggccatca gtaagctggc gctcgatgag aacatctttt tacacgtcga cgccgccttc 780

ggcggcttcg tcataccgtt catgaaagac ccgtcgaagt acaggttcga cttcgagctc 840

cccggcgtca tgtccatcgc catcgacccc cacaagatgg gcatgagcac gataccctcc 900

ggagggctgc tttaccggga cgagcgccac atgaagtcgc ttgagatcag cgcccagtac 960

cttacttccc aggtccagtc gtcccttgcc ggcacccgca ccggcgcctc cgctgcggcg 1020

acctatgcgg tcatgcgcca cctggggatg gacggctacc gccgggtcgt gtcagagtgc 1080

atggataaca ccatgttcct ccgggacagc ctggtcgaca tggacattga gctggccctc 1140

gagcccatta tgaacatagt gacggcgaag ctgcccgatg cgcagtcgac ccgcaagaag 1200

ctctgcgaca tgggctggtt cgtctccacg acgtcgaggc ccgaagcgct ccgcatggtc 1260

gtcatgcccc acgtcaccag ggacgtcatc gaggcgttca tggccgacct caaaaaaata 1320

tcgtagtgaa gtaagtagga agcagggagc aggggaaaga aaattgacaa ctgtacaaga 1380

ttaatcgcgt ctctgagcaa tgaccaaata catctacctc cacggttttc ttccagcccc 1440

ctatctgcga aagcacaaga tattagcaag cgtttcgccc aaattcacat acagctaaca 1500

atccctgatc tcaatgctgg tgaattttct cagttaacaa tcacgcgcca aattcaacaa 1560

gttgccgcaa ttttccctga taattctgaa ccaataacgc tgataggttc tagtttaggc 1620

ggtttaactg ctgcttatct aggacagcga tatttacaag tacaacgctt agttttatta 1680

gcgccagttt ggttttttat cccattggtt gcccaaaatg ggtgaagaag ctgtcacaag 1740

ttggcaacaa acgatatagg ttctctcttc tgccgtta 1778

<210> 196
<211> 3215
<212> DNA
<213> Artificial Sequence

<220>
<223> Synthetic Construct- SEQ ID NO: 196, example 196: a designer
 hox-promoter-controlled Methanopterin synthesis enzymes DNA
 construct (3215 bp)

<400> 196
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgacagt ttcagaattt ccagatactc aggataaaca accatccatt 240

ccaatatcac taacaagagt tggagtaaca ggtgtaaaga aattaataaa aataaaaaga 300

gaagataaac gtcctattat tctaatacca acatttgatg cttttgtaga tttacctagt 360

actcagaaag gagtacacat gtctagaaat ccagaagcta tatctgaaat tgttgatgag 420

gctgcaaatc aatcggaaat tcatcttgaa aatatatgtg caaatcttgt aaaaagatta 480

cttgaaaaac atgaatatgc attacatgca gaaacagagg caaggggaga atatattata 540

aataaattat ctccagtatc taaaagaaaa acacaggaaa caacacatat catagcaaga 600

gcaattgcta tgaaagatga tgagggtaat atttctgtta gaaaaatgat tggtgcagaa 660

gttattggaa tgactgtatg tccatgtgca caagaatctg ttgaaaagga ttctaaagat 720

aaattactgg aatttttaga tgaagaaact acacaaaaag tgttggatgt agtaacattt 780

gcttctcata atcaaagggg tgttgggaca atacttcttg aagtaccaga aaaacaagat 840

gttaatgtgg atgatttaat aaaaataata caggatgcta tgagttctcc tgtttgtgaa 900

atattaaaac gtccagatga aaatagaatt gtaacaaatg cacatcaaaa tccagtattt 960

gttgaggatt gtgtaagaaa catggtaata ggattacttg aaaaatatcc taatttacct 1020

gatgattctg tagttacaat taaacaagta aatcaagaaa gtattcatca acataatgct 1080

tatgctgaaa aagttgcatc ttttggtaaa ttacgagaag aaaataagga gtagatgaat 1140

gatctgatcg ctcttgcaga ggatattaag aacgaaaaat taaaaaaaaa ggttgtcgaa 1200

tttataaacg atccgattcc aaaacatcct gaaattgaaa gtactggcat aacacttgaa 1260

acctcccccg caagcgttaa aagacatcac aaatactccg gaggattaat cgaacatacg 1320

atcgcagtta caaaaatggc tgtaaaaatg gctgaagcct tggaagaaac atatggaatt 1380

gaattaaata gagatttgct aatttccgga ggaatactcc acgatcttat gaaaccacaa 1440

aattatcagt taaaagacgg aaaattcgac catctttcaa attttcacct cgaccatttg 1500

acacttggaa ttgcagaact gtacagaagg gactttccac ttgaagttat aaaagttgta 1560

gctagccatc acggagatca tggtcctgta agtccagatt caatagaagc gtggctaatt 1620

catcattcag acaatgtaga cgctgcaatt aatgatatag gaattagaat atgtcaggcg 1680

agagctagag aatttggaat tgatgattca caaatatata aaatcgtcaa tccattaaaa 1740

ttatatgaaa tgcgtaaaaa attgggaaaa gataaagtga aagaattttt aaaggaaaaa 1800

ttggaaataa aggatgaata attgttaagg atacgaacac cttcccgcct ccacatgacg 1860

ctcatcgaca tgaacggaga gatcggcagg atcgacggcg ggatcggaat aacgctcgac 1920

aagccttata tcgagatcac ggcgaaaaag agcgatacgg tcacggttaa gggagaccct 1980

gacctgcgcg agcgcatgcg caaggcatgc gaggcattcg gcccgggata tggcgtggag 2040

atcgacatta aaaagtcgta ctggaaccat atcggcctcg gatcgggtac ccaggccgct 2100

ctggccgcgg gcaccgccgt agcaaaattg tatggcctgg atatgtcgtc cgccgaggtt 2160

gcccttaaag tcggccgcgg cggcctgtcg ggcgtgggca tcggcgcctt cgataaaggc 2220

ggtttcatcc tcgacggggg acataagacg agcgttaaga aggccttcct tcccgattca 2280

ttcgccgacg gagtccctcc cgccccgctg atcatgagct gccccttccc tgactgggac 2340

atcgtgctcg ccacgctgcc gatgaaaggc gcccacgacc tttacgaaaa ggacgtcttc 2400

gcccgaacgt gtcccttacc attaagggag atcgagaggc tcagccacat cattctcatg 2460

cagatgctgc cctcgatcgt agaaaaggac ctggaagcct ttggcacggc gctgaacgcg 2520

atccagggcg tagggttcaa gaagagggaa gtcgaccttc agccgggcgc ctgcgagata 2580

ttgcgtgtca tgacggaagc cggggcgccg ggagcgggca tgagctcgtt cggcccgacc 2640

gtgttcgctg tcactgacag gtccggcccg atcatgtccg aggtcaagag taactgccct 2700

gattgtatgg tcatgaagac aagggcccgt aattcggggg cagatatcga atgcctcccg 2760

tagtgaagta agtaggaagc agggagcagg ggaaagaaaa ttgacaactg tacaagatta 2820

atcgcgtctc tgagcaatga ccaaatacat ctacctccac ggttttcttc cagcccccta 2880

tctgcgaaag cacaagatat tagcaagcgt ttcgcccaaa ttcacataca gctaacaatc 2940

cctgatctca atgctggtga attttctcag ttaacaatca cgcgccaaat tcaacaagtt 3000

gccgcaattt tccctgataa ttctgaacca ataacgctga taggttctag tttaggcggt 3060

ttaactgctg cttatctagg acagcgatat ttacaagtac aacgcttagt tttattagcg 3120

ccagtttggt tttttatccc attggttgcc caaaatgggt gaagaagctg tcacaagttg 3180

gcaacaaacg atataggttc tctcttctgc cgtta 3215

<210> 197
<211> 4226
<212> DNA
<213> Artificial Sequence

<220>
<223> Synthetic Construct- SEQ ID NO: 197, example 197: a designer
      hox-promoter-controlled Coenzyme M synthesis enzymes DNA
      construct (4226 bp)

<400> 197
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttaaagat tcacctttcc gagagtatct cccctcaagc ctcttcttat 240

ggtttcaagg gctgttatct cttcgggggg tatgtttcca aggttaacgt ctggtcctat 300

tttcagtatg aagtaaacct gctggttttt ctggggggcc tcccatatta tcctatcctg 360

cgggagttca ttgaggaggt ggttgaattc ttcctccttt acgtttccat tctcatcgta 420

tatacctata ttatggccgc tctcccgggc ctccatgagc accatgcagg ctccagcttt 480

gaggtcggcc ctgacaagtt caaccctatc ctccgggctg agaagtctgt cccttaccgg 540

gtccttttta ccaacctctg atatgatctg gaacccctcg ttgaccgctt tctctataag 600

ttcacatttt tcatcggttt caatctcaac ggttccattt gatatttcca gtgtttcaaa 660

accaaggctt cttgcctcgt caaagtactc ctgaatttta tcgttgagat aggcgatctc 720

aaatagggtg cccccaggat aggcctccac atcgaatgaa cggtacatct caattttttc 780

catgacagcg tcacgtctgt ggagggggag ggttccccat ccaaacttta tgaaatcaac 840

gtaatctgat gaaatctcca tgaggtcacg ggctgtttta atgcccattc ccttatcgag 900

aaccatggtt attccatttt ttctgggttt cccacgcctt ggaggtgtga gaaagtcaaa 960

ggcgttcata tgagggtaag tctcagcttt gaaaaaccgg agggcagtgg actctgcata 1020

atggtggacc ttctgagggc aagtgcaacc ataacctctg ccctggacag tttcagggag 1080

gtcatccctg ttggggacgt tgaggaggca acggaatact ccaggatggg ctatgtggtg 1140

gcaggggagc gtggtggtga gaccctgcca ggattcctgg ccaactcccc actggaggtg 1200
agggaacatt caggtgatgc ccttgtgcta accaccagta acggtacaag gatccttgag 1260
tcagttgaat cacaggccct cgtgggctgc ctcaacaatc tggatgcagt ggcctcagct 1320
gcaaggaatc tttcggatga agttgaggtg gtcatggccg gtgttaatgg ccgctttgca 1380
atagaggatt tcctctgcgc cggtgaaata atacgtgcca ttggtggtga gtttgaagag 1440
tacgctgagg cctcagtact tgcagttcag gacaggtcca tggttgatga tgccataaga 1500
aaatccaggt cagccaggag gctcagggaa cttggttttg ccggtgatat tgaatactgc 1560
ctcagaagga atataacaga aaacgtaccc gtatacaggg acggcaggat atcgctgatc 1620
taaatgagga taagtgctga agaggaagtt agaatcataa aggaaatact caccgccatg 1680
gaggttccag aggaggtttc agagatagtg gctgatgtta ccctggacgc ggacctgaag 1740
ggtttcagtt cacatggaat cggtcgtttc ccccagtacg ttgaggggct gcgatgtggg 1800
acaataaagc cccatggtga cataaccatt gaaagggaaa ccccctcaac ggccctcata 1860
aacggtaacc acatgttcgg acactttgtt gcctgcaggg caatggaact tgcaattgaa 1920
aaggccaggg ataccggcgt cgggcttgtt ggtgtccatg actcaaacca ctttggtgtt 1980
gccggatact actcggacat ggcggtcatg aacgacatga taggggttgt gatagcaaat 2040
acagagcctg ctgtggcacc cataggtggc aaaacaccca tcctgggaac aaaccccgtg 2100
gccataggga tcccatcaaa caggcactat gtctcggttg acatggcaac ctcagcatcc 2160
gccaggggca aactcctgga ggccgccagg aagggtgaga gtatacccga aaacatagca 2220
atggacgccg atggaaaccc cacaaccgac cctgaacttg cccttaaggg gtccatactc 2280
cccttcggtg ggcataaggg ttatgcgctt tcattcatga tagagatcct agcaggtccc 2340
cttgtggggg ctgcctttgg ggctgcggtg aagggaaccg cgaacccccg ggagatgtgc 2400
accaagggcg acctcatgat ggccatagac ccctcaaaga tggtcgacct ccaggaattc 2460
aaatccagcg tggatgaatt catcgaggag gttaaatcat ccggggacgt cctcataccc 2520
ggtgacattg aggccatgaa cataaagagg gggcgtgaag agggaataga ggtggatgaa 2580
aagctccttc aaaggctgaa tgaaatatca gatgaactcg gcctggacct caagattaag 2640
gggttatgaa tggcaaacgt tgagcaagaa gtcatagaca tcatgaagtc ctccggcatc 2700
gacacagtgc tgacgctgcc gtgcgataaa ataaagaatt tactggcgat ggtcccctcg 2760
aactttaagg agatccccct gaccagggag gagaacggca tcggtatcgc ggccgggctt 2820
tccatggccg gcaaaaggcc ggccctcatt atccagagca cgggaatagg caactcgctg 2880
aacgtgctct catcgctgaa caggacttat gagatacccc tgcccatcct ggccagctgg 2940
aggggctact ataaggaagc catctatgcc cagacggcct tcggcaagtg cctgcccgcc 3000
atacttgaag cctctgacat acagcacatc gagatcggcg ccatgggcga gctcgacctg 3060

atcaagaagg ccatcatcgc ctcgttcaag tcgaacttgc cgaccgtgat actcttatcg 3120

cccaggctgt gggagatctc gacggagagg cactggaacc cggacttcac gccccgggaa 3180

aggcgttttg atatggagtg ccacaccgtt gtcccgaagg ccacgcatac ccgctacgac 3240

atgatcaagg gcatcacttc ataccttagc ggcaaagtcg tggtctccaa catcggcatc 3300

ccgagcaagg agctctatgc cgcccacgac caggacacga acttctacat gacgggcagc 3360

ctgggccttg tttccgctat cggccagggc ctggccatgg gcctgagccg ggaggtcatc 3420

acgctggacg gcgacggcag tattttaatg aacccgaacg tgctggcgag cgtcgcccag 3480

gaaaagcccg agaacctcac catcatttgc ttcgataaca gcgcccacgg ctccactggc 3540

aaccagaaga cctattcgga gagcatggac ctcgagcttc tggcgaaggc gttcggcatc 3600

gagaacacgg cgaaggcatc tacacccgga gagctgcttg aggcgctgga gaaaaggggg 3660

aaagggcccc ggttcatcca cgcgatcatc gaggcgaaga acgccgacgt gccgaatatt 3720

cctttgacac cggtagagat caaggagcgg tttatggggg cggtcacgcg ctgatgaagt 3780

aagtaggaag cagggagcag gggaaagaaa attgacaact gtacaagatt aatcgcgtct 3840

ctgagcaatg accaaataca tctacctcca cggttttctt ccagccccct atctgcgaaa 3900

gcacaagata ttagcaagcg tttcgcccaa attcacatac agctaacaat ccctgatctc 3960

aatgctggtg aattttctca gttaacaatc acgcgccaaa ttcaacaagt tgccgcaatt 4020

ttccctgata attctgaacc aataacgctg ataggttcta gtttaggcgg tttaactgct 4080

gcttatctag gacagcgata tttacaagta caacgcttag ttttattagc gccagtttgg 4140

ttttttatcc cattggttgc ccaaaatggg tgaagaagct gtcacaagtt ggcaacaaac 4200

gatataggtt ctctcttctg ccgtta 4226

<210> 198
<211> 5198
<212> DNA
<213> Artificial Sequence

<220>
<223> Synthetic Construct- SEQ ID NO: 198, example 198: a designer
      hox-promoter-controlled Coenzyme B synthesis enzymes DNA
      construct (5198 bp)

<400> 198
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgttgcccga tcgggtacgg atcttcgaca cgacgctaag agacggtgag 240

cagacaccgg gcgtcagcct cacggtggag gaaaaggtcg agatcgcgag gaagttggac 300

gagttcggcg tggataccat cgaagcgggc ttccccgtag ccagtgaggg tgagttcgag 360

gcagtgaggg cgatagcagg cgaggaactg gacgcggaga tatgcgggtt ggccaggtgc 420

gtgaaggggg acatagacgc ggcgatcgac gccgacgtgg actgcgtgca cgtgttcata 480

gccacgtcgg acatccacct cagatacaag ttggagatgt cccgggaaga ggcattggag 540

cgtgccatag agggcgtgga gtatgccagt gaccacggag tcaccgtgga gttctccgcg 600

gaggacgcca cacggacgga ccgggactac ctactcgagg tctataaagc taccgtggaa 660

gccggcgcgg atcgtgtcaa cgtcccggat actgtgggag ttatgacccc tcccgagatg 720

tatcgactga cggcggaagt tgtcgacgcc gtagacgtgc cggtcagcgt gcactgccac 780

aacgacttcg gcatggccgt ggccaactca ttggccgccg tcgaagccgg ggctgaacag 840

gttcatgtga ccgtgaacgg gatcggcgag cgtgccggta acgcctcgct ggaacaggtc 900

gtaatggccc tcaaagcgct gtacgatatc gagttggacg tgaggaccga gatgctcgtc 960

gagctctcac ggctcgtgga gcgactgacg ggcgtcgtag taccgccgaa caccccgata 1020

gtcggcgaga acgccttcgc ccacgagtcg gggattcact cccacggcgt gatcaagaag 1080

gcggagacgt acgaacctat acggcccgag gacgtcggtc atagacggcg tatagtacta 1140

ggtaagcacg ccggtcggca cgcgatcaag aagaagctcg aggagatggg gatcgaggtc 1200

acggaggaac aactcgacga gatcgttcgg cgcgtcaagg agctcgggga taagggcaag 1260

cgcgtcaccg aagacgatct tgaagccata gcccgagacg tcgtcggcga ggtacccgag 1320

tccgaagctg cggtcaagct ggaagagatc gccgtgatga ccgggaacaa attcacgccg 1380

acggcgtcgg tccgtgtgta cctggacggt gaagagcacg aagcggcatc gaccggtgtg 1440

ggctccgttg acgcggcgat acgggcactg cgcgaagcga tcgaggagct cggtatggac 1500

gtggagctga aggagtaccg cctagaggcc atcaccgggg gcaccgacgc cctcgccgag 1560

gtcacggtga ggttggagga cgaggacggg aacgtcacca cggcccgcgg tgccgccgag 1620

gatatcgtga tggccagcgt gaaggcgttc gttcggggcg tgaatcggct ggcacgtcga 1680

cgtagggact aagtgaacgc gtcgccgacc gtcgtggtaa tccccgggga cgggatcggg 1740

ccggaagtca tcgacgcggc cctgaaggtc gtgcgggcgg ttctgggtga cgaactcgag 1800

atcgtggagg aacaggccgg gtactcgctg tggaagaaga ggggagtcac gatcgaggac 1860

gagacgatcg agcgctgccg cgaggcggac gccatgctct tcggcgcgtg cacgactccg 1920

gaggacccgg aggccaagag tcccatcgtg acgctgcgga aagagctcgg actctacgcc 1980

aacgtccgtc ccgctcgctc ctggcccgta ccgcgacctg tcgacaccga gttcgacctg 2040

gtgatcgtcc gggagaacac cgagggtctc tacaccggat gcgagtgcga gatccacgac 2100

ggtgttaccg tcgccctgcg taagatctcc gaggagggta cgcgcagggt cgctgaggtc 2160

gcctgcgatc tcgccgagga gcgctccggt cgcgtgacga tagtgcacaa ggccaacgtg 2220

ctgaagctca cgtgcggaac gttcaagcgc gtcgcggcgg aaaccgtgga gcgtcgcggt 2280

ctggagtggg acgacgagta cgtggacgcg gcggcgtaca agctggtgcg tgagccggac 2340

agcttcgacg tgatcctcac ctccaacctg ttcggggaca tactctcgga cctggcggcg 2400

ggtctgatgg ggagcctcgg gctcgcaccc agcgcgaacc tgggggacga cgccgcccta 2460

ttcgagcctg tacacgggtc ggcacccgac atcgcgggta aggggatcgc gaacccggtg 2520

gcggcgatcc tctcggccgc catgatgctg gatcacctcg ggtacgggga ggaggctcgg 2580

atcatcgagc gtgccgtcga ggaggtgctg cgcgagggcg tgaggactcc cgacctcggc 2640

ggttccgcga ccacggagga ggtggccgag gcgatcgcgg agcgtgtggc taccgggtga 2700

ctatacctcc ctcacatcgg tgatttcgcc cgccaacgca gccgcagcgg ccatcagcgg 2760

actcatcaga tgagtccgac cacccttgcc ctgcctgcct tcaaagttcc tgttagaagt 2820

ggaggcacag cgctcgccag ggttaagccg atcatcattc ataccgaggc acattgagca 2880

tcccgccgcc cgccactcga aaccagcatt tcgaaaaatc atatccagac cttcccgctc 2940

tgcctgcagg cgaacctggc cagaccctgg aacaatcaga gcctgtttca ccgaccggtg 3000

cactttctgt ccctgcacca gattagcgac ggctctcaag tcctccatgc gggcattggt 3060

gcaagaacca atgaaaacct tgtcgattga caaccccttc agggggcgcc cgggctcaag 3120

ccccatgtag tccagagccc gcctggccga ttcccgcttc tccggcgcca gctgagaaag 3180

atccggggtt ttgccatcaa taccggtgac catctctggg gatgtgcccc aggttacctg 3240

cggcaccagg gcttccagcg gaaaatgctc ctcgcgatca aaatctgcct ccgcatccga 3300

aaccaacgtg cgccaatact cacgagcctg ctccaggcgc tcgccaactg gcgcctgggg 3360

tctgccctcc acgtattgtt ccgtcacccg gtcataagca accatgcctg cccgggcacc 3420

ggcttcaatg ctcatgttgc agatggtcat gcgcccctcc atggacaggt tacgaatggc 3480

tgcaccggcg tattcaatgg catatccgtt gccgccagcg gtccctatac gcccaatcag 3540

cgccagaatc aggtcttttg gggtcacccc gaaacctggc tgaccttcga acaccactcg 3600

catgtttttc tgtttctggg tccgcagggt ctgggtggcc agcacatgct ccacctcgct 3660

ggtaccgatt cccatggcga gcgtcgcgaa agcgccgtga gtggaggtat gagagtcgcc 3720

gcaaacgatg gtcatgcccg gcaaggtcag gccctgctcc ggacccacca cgtgtacgat 3780

cccttgccgg gcatcttcca gttcaatcaa cggaataccg aattcctcgc aattacgcga 3840

cagggtttcc acctgttttc gcgcaacacg gtcggcaata cctgcgacgc cgagttcccg 3900

tttccgggtt ggtacggcat gatcgggcac cgctacattg gccggaattc gccagggctt 3960

tcgaccagcc gagcgcagac cggaaaaagc ctggggcgtg gtcacttcgt gcagcaaatg 4020

acggtcgatg tagagcaacg cggaaccgtc aggcagttca tccaccaggt ggcgctgcca 4080

gagtttgtcg tagagcgtac gcgcagccat ctactcaaaa agccagggtt ccagggttct 4140

tcggttcgcc tcgtaggcct gaatggagcc tgaaaaagtc agggtacgac ctatctcatc 4200

cagaccgctg aggaggttct cccgccgacc cgactcaacc acgaaacgcc aggtcttctt 4260

cggactgcga atctcgcatt gctccaaatc caccgtcagt tccagaccgt ccgtgtcact 4320

ggccaaattg aacaattcgt caatggttga ctcgtcgagt attaccggaa gcaggccatt 4380

gttaaagcag ttgttaaaga aaatatcccc aaaactgctg gcaataaccg ctcgaacgcc 4440

aaagtccttc agggcccaga ccgcgtgttc ccgggatgac ccacagccaa agtttgccct 4500

ggccagcagg atggaagcac ggtcgtaggg cgcccggttc agaatgaagt cgggatccgg 4560

tctgcgctcc gacacgggga tgtccacatc accggggtcc agatatcgct catcatcaaa 4620

gaggaagtct gcaaacccgg ttttctccag cattttaaga tactgcttgg gcagaattgc 4680

gtcggtgtcc acattcgacc ggtcgaaagg aacgaccaga ccggtgtgtg ttttgaatgc 4740

ctccattgaa gtaagtagga agcagggagc aggggaaaga aaattgacaa ctgtacaaga 4800

ttaatcgcgt ctctgagcaa tgaccaaata catctacctc cacggttttc ttccagcccc 4860

ctatctgcga aagcacaaga tattagcaag cgtttcgccc aaattcacat acagctaaca 4920

atccctgatc tcaatgctgg tgaattttct cagttaacaa tcacgcgcca aattcaacaa 4980

gttgccgcaa ttttccctga taattctgaa ccaataacgc tgataggttc tagtttaggc 5040

ggtttaactg ctgcttatct aggacagcga tatttacaag tacaacgctt agttttatta 5100

gcgccagttt ggttttttat cccattggtt gcccaaaatg ggtgaagaag ctgtcacaag 5160

ttggcaacaa acgatatagg ttctctcttc tgccgtta 5198

<210> 199
<211> 1636
<212> DNA
<213> Artificial Sequence

<220>
<223> SEQ ID NO: 199 presents example 199 of a designer Synechocystis
 sp. strain PCC 6803 nirA-promoter-controlled NAD-dependent
 formate dehydrogenase DNA construct (1636 bp)

<400> 199
agaaaatctg gcaccacacc ctaaatgcgt aaactgcata tgccttggct gagtgtaatt 60

tacgttacaa attttaacga aacgggaacc ctatattgat ctctacatga tgaaaatcgt 120

tctcgttttg tactccgctg gtaagcacgc cgccgatgaa ccaaagttgt atggttgtat 180

cgaaaatgaa ttgggtatta gacaatggct tgagaagggc ggccatgaat tggttactac 240

atcagacaaa gagggtgaaa actctgagtt agaaaagcac attcctgacg ctgatgtgat 300

tatttccact ccattccatc cagcctacat cacgaaggag agaatccaaa aagccaagaa 360

gctgaagttg ttggtcgttg ctggtgtcgg ttccgaccac attgacttgg actacattga 420

acaaaatggc ctagatattt cggtcctaga ggttactggt tccaacgttg tttcagtggc 480

tgagcatgtc gttatgacta tattgaactt ggtgagaaac tttgttccag ctcacgagca 540

aattgttaac cacggctggg acgttgctgc catcgccaag gacgcctacg atatcgaagg 600

taagaccatc gcaacaattg gtgctggaag aattggttac agagtcttag agagacttgt 660

ggctttcaac cctaaggaat tgttgtacta cgactaccaa ggtcttccaa aagaggccga 720

ggaaaaagtt ggtgccagaa gagtcgacac tgtcgaggag ctggttgctc aagccgatgt 780

tgttaccgtc aatgccccac tgcacgcagg tactaagggt ttagttaaca aggagcttct 840

gtccaagttc aagaagggtg cttggttggt taacacagcc agaggtgcca tctgcaatgc 900

tcaagatgtc gctgatgccg ttgcatctgg tcaattgaga ggttacggtg gtgacgtctg 960

gttccctcag ccagctccaa aggaccatcc atggagagat atgagaaaca agtacggata 1020

cggaaacgcc atgactcctc attactcagg taccactttg gacgcccagg tcagatatgc 1080

cgaaggtacc aagaacatct tgaactcatt ccttaccaag aagtttgact acagacctca 1140

agatgtcatt cttttgaacg gtaagtacaa gaccaaggct tatggtaatg acaaaaaggt 1200

cgcataatag taatgagtta cagttttggc aattactaaa aaactgactt caattcaatg 1260

ttagcccgct cccgcgggtt ttttgttgct ttttcacagt gactataggt aatcagcaac 1320

acaatacggc cctgttcttt ggacagtttt tgtataatgt tgaccgcatc ctgaccggat 1380

tttttatcta agtggggaat tgtcaattgt caattaaagc taagttctac taatgtttta 1440

gaaggcattg tcgattgaaa ataagggttg aatggagaaa attttgagcc tttgtcaaag 1500

ataaaaattt atttcaacag ttttttaact agccgaacca gagaatgacc cagtggcgct 1560

gactttgctc ccgagttttt gttagaaatt accctcaaga agtaatctaa taataaggtt 1620

ctctcttctg ccgtta 1636

<210> 200
<211> 1567
<212> DNA
<213> Artificial Sequence

<220>
<223> SEQ ID NO: 200 presents example 200 of a designer Synechocystis
 sp. PCC 6803 nirA-promoter-controlled Formaldehyde dehydrogenase
 DNA construct (1567 bp)

<400> 200
agaaaatctg gcaccacacc ctaaatgcgt aaactgcata tgccttggct gagtgtaatt 60

tacgttacaa attttaacga aacgggaacc ctatattgat ctctacatgg tggacatcgt 120

cgtcccggat cccgggcccg gcgaagccgt cgtcgatatc agcgcttgcg gcgtttgtca 180

caccgatctg acctaccgcg agggcggcat caacgacgag tacccgttcc tgttgggcca 240

cgaggccgcg ggcacggtcg aagcggttgg gcccggggta accaacgtgg agccaggcga 300

tttcgtgatc ctgaactggc gtgcggtgtg cggccagtgc cgggcctgca aacggggccg 360

gccgagctac tgtttcgaca cgttcaacgc cgagcagaag atgacactga ccgacggcac 420

cgaactcacc ccggcactgg gcatcggggc attcgccgac aagacgctgg tgcactccgg 480

ccaatgcacc aaggttgatc ccgcggccga ccccgccgtg gcgggcctgt tgggttgcgg 540

ggtgatggcc ggactgggcg ccgcggtcaa caccggtggc gtcacccgcg atgacaccgt 600

cgcggtgatc ggttgtggcg gcgtgggtga tgccgccatc gccggtgcgg cgctggtcgg 660

agccaagaag atcatcgccg tcgacaccga caacaccaaa ctggagtggg cgcgcgcctt 720

cggcgccacc cacaccctca acgcccgcga actcgacgtc gtcgaaacga tccaggacct 780

caccggcggc tttggggtcg acgtggtgat cgacgcggtc ggccggcccg aaacctggaa 840

gcaggcgttc tacgctcgtg atctggccgg aaccgttgtc ctggtggggg ttcccacccc 900

cgacatgcgg ctggacatgc cgctggtgga cttcttctcc cgcggtggcg cgttgaagtc 960

gtcctggtac ggcgattgcc tacccgagcg cgacttcccc accctgatcg acctctacct 1020

gcagggccgg cttccgctgg acaagttcgt ttccgagcag atcggtctgg gcggtatcga 1080

ggaggcattc gagaagatgc acgccggcaa ggtgctgcgc tcggtggtgg tgttgtagta 1140

gtaatgagtt acagttttgg caattactaa aaaactgact tcaattcaat gttagcccgc 1200

tcccgcgggt tttttgttgc tttttcacag tgactatagg taatcagcaa cacaatacgg 1260

ccctgttctt tggacagttt ttgtataatg ttgaccgcat cctgaccgga ttttttatct 1320

aagtggggaa ttgtcaattg tcaattaaag ctaagttcta ctaatgtttt agaaggcatt 1380

gtcgattgaa aataagggtt gaatggagaa aattttgagc ctttgtcaaa gataaaaatt 1440

tatttcaaca gttttttaac tagccgaacc agagaatgac ccagtggcgc tgactttgct 1500

cccgagtttt tgttagaaat taccctcaag aagtaatcta ataataaggt tctctcttct 1560

gccgtta 1567

<210> 201
<211> 1549
<212> DNA
<213> Artificial Sequence

<220>
<223> SEQ ID NO: 201 presents example 201 of a designer Synechocystis
 sp. PCC 6803 nirA-promoter-controlled NAD(P)H-dependent Alcohol
 dehydrogenase DNA construct (1549 bp)

<400> 201
agaaaatctg gcaccacacc ctaaatgcgt aaactgcata tgccttggct gagtgtaatt 60

tacgttacaa attttaacga aacgggaacc ctatattgat ctctacatga tgaaaggtaa 120

atctattgtc gttgagtctg gcggtggttt tgagcgtgtc agagtcagtg atgccgaggc 180

gcaagcgcct aaagcggggg aagttctggt caggatacat gccaattcac tgaattatca 240

tgattatgct gttgttaaag ggttatggcc tgcatctgaa aacagaatac caatggctga 300

tggcgcaggt gaagtgattg aggttggccc aaatgtaact gaatttgcag tgggtgacag 360

cgtcgtgagt acgtttttcc cttcctgggt aaacggtgcc cccatcgttg acggtttctc 420

aacggttccg ggcgatggcg tagatggtta tgcaagagaa tatgtgactg ccagtgctga 480

ggctttcaca aagtcgccca caggatggtc tcacaccgaa gcggcgactt tgaccacagc 540

tgcggtgaca gcttggcgag ctttgtttga ttttaatcca cttaaacccg gtgaaacggt 600

gcttttgcaa ggtacgggtg gagtctctat tttcgcttta caatttgcca aaatggctgg 660

cgccaaagtg atcgtcactt cctccagtga gcaaaaactc gcaaggctca aggagatggg 720

agctgatcat gtgatcaact acaaaaaaga acctaaatgg ggctttttag ccagagaact 780

taatgatgga aaaggggttg atcacgtcgt cgagattgga ggcgctgaaa cactagagca 840

gtctctcaac gcgatacgtg tcgctggcca aatttcacta atcggtatcc tgtctggaca 900

aacttgcgat ataaatattg tgaagtcttt tgtgaaacag gcgaggctgc agggtgttct 960

tgttggcagt agagggcatc agcaggagat gattagagct atcgaggtta ataagctcaa 1020

gccgattatt gatcgtacgt ttgctttgca cgagattgtt caggcatttg agtatcaaga 1080

gacgaatcag catttcggaa aaatttgttt agagttttaa tagtaatgag ttacagtttt 1140

ggcaattact aaaaaactga cttcaattca atgttagccc gctcccgcgg gttttttgtt 1200

gctttttcac agtgactata ggtaatcagc aacacaatac ggccctgttc tttggacagt 1260

ttttgtataa tgttgaccgc atcctgaccg gattttttat ctaagtgggg aattgtcaat 1320

tgtcaattaa agctaagttc tactaatgtt ttagaaggca ttgtcgattg aaaataaggg 1380

ttgaatggag aaaattttga gcctttgtca aagataaaaa tttatttcaa cagtttttta 1440

actagccgaa ccagagaatg acccagtggc gctgactttg ctcccgagtt tttgttagaa 1500

attaccctca agaagtaatc taataataag gttctctctt ctgccgtta 1549

<210> 202
<211> 1180
<212> DNA
<213> Artificial Sequence

<220>
<223> SEQ ID NO: 202 presents example 202 of a designer
 Thermosynechococcus elongatus nirA-promoter-controlled Formate
 dehydrogenase DNA construct (1180 bp)

<400> 202
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac cttgaacgat aaagtaactg cggtcaggga tgtatggcgc tatgaacaag 300

gggaaataag caaagtagaa gaccggatgg tgacggaatt ccctttgaca gtaatactga 360

atggcagtga atttgtgacg ctcgtttgta cgcctgagca tatcgaagag cttgtcatcg 420

gatttctcgc atcagagggt gttatacgat ttcaaaaaga aattaaaagg tttacaatag 480

atgaaagtct cggttttgta tatgtcgacc tcgttcaccc tgagacattg gatcaaaagg 540

attatacgaa acgggtgatc ggctcatgct gcggaaaggg ccgtcatttt tattttcagc 600

aggatgtcaa aacagccaag acggctgtca gccaaatcaa gatatcgcct gaggcatgtc 660

tggcgcttat gaaagacatg cagcaaggga gcgggacgtt tcaggatacg ggaggcgtcc 720

ataatgcggc gctttgcgac acagagaagc ttctgttgat gcgcaccgat atcggtcggc 780

acaacgcact tgataaacta tacggacact gccttctaaa cgggatgtcc gtacgcgaca 840

aactgattgt gtttagcggc agaatctcat cagaggtcct gttaaaagcg gccaaaatcg 900

gcgtgtcgat tgtcatttcc aaatcggccc cgacggagct tgcgattcaa atggctgaag 960

aattgaatat cacagcgatt ggatttgtca gaaacggatc atttaatgta tatacacacc 1020

ctgagagaat cagggagtag taaggctgag atcttcttca gtgcattgta gttgaatgaa 1080

gggttagggg ggaaatgccc ccctattttt tgtctagcca tcctgccacg tttgacaggg 1140

tagcaatttc gacacgatag ggttctctct tctgccgtta 1180

<210> 203
<211> 1432
<212> DNA
<213> Artificial Sequence

<220>
<223> SEQ ID NO: 203 presents example 203 of a designer
      Thermosynechococcus elongatus nirA-promoter-controlled
      formaldehyde dehydrogenase DNA construct (1432 bp)

<400> 203
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgaaagca cttacatacc tagggccagg aaaaaaagaa ttaatggaaa 300

aaccaaagcc aaaaattgaa aaggaaaccg atgcaatcgt caaaataata aaaacaacga 360

tttgtggaac ggatttgcac attctttcag gagatgttcc tactgttgaa gaagggcgga 420

ttttaggaca cgaaggcgtc ggaattattg aagaagttgg ttcggccgta aagaatttta 480

aaaaaggcga cagagtgttg atttcttgca ttacctcttg tggaaaatgc gaaaattgca 540

agaaagggtt atacgcccat tgcgaagatg gcggctggat cttaggccac ttaattgatg 600

gaactcaagc agaatatgta agaattccgc acgcagacaa cagcctttat cctattccgg 660

aaggcgtgga tgaagaggct cttgtcatgc ttagtgacat tcttccaaca ggatttgaaa 720

tcggcgtgtt gaacggcaaa gttcagcctg gacaaaccgt cgccattatc ggagctggtc 780

ccgtaggtat ggcagcgcta ttaacagccc aattttattc accagcagag atcattatgg 840

ttgatttaga cgataaccgt ttagaagttg cgaaaaaatt tggcgcgacc caagtggtga 900

atagcgctga tggcaaggca gtggaaaaaa ttatggaatt aaccggcggg aaaggtgtag 960

acgtcgcgat ggaagccgtc ggaattccgg caacatttga tatttgtcaa gaaattgtca 1020

aaccaggtgg ctatatcgcc aatatcggtg ttcatggaaa aagcgtggaa tttcacattg 1080

aaaaattatg gatacgcaac attacgttga caaccggtct tgtcaacacg acttctacgc 1140

cgatgttatt aaaaacggtg cagtcgaaaa aattgaagcc ggaacaatta attacccatc 1200

gtttcgcctt ttcagacatt atgaaagcgt atgaagtatt tggaaatgca gcaaaagaaa 1260

aagcgttaaa agtcattatt tccaacagtt aataaggctg agatcttctt cagtgcattg 1320

tagttgaatg aagggttagg ggggaaatgc ccccctattt tttgtctagc catcctgcca 1380

cgtttgacag ggtagcaatt tcgacacgat agggttctct cttctgccgt ta 1432

<210> 204
<211> 1579
<212> DNA
<213> Artificial Sequence

<220>
<223> SEQ ID NO: 204 presents example 204 of a designer
      Thermosynechococcus elongatus nirA-promoter-controlled
      thermophilic NADPH-dependent Alcohol Dehydrogenase DNA construct
      (1579 bp)

<400> 204
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggcaggt tattcgaaat ggttcagggt tccgcgggat atagtgttcg 300

gctggggcag cctggaatac cttaaagaac tggaaggcaa aagggccttt atagttaccg 360

acaaaaccat gcaggagctt ggctttgtgg acaaggtgaa gagctacctg gccgaagccg 420

gaattgaaag cgccgtattc aacgaagtag agcccgatcc ttcccggcaa accgtgaaaa 480

aaggcgccag gctgatggag cagtttcagc cggacctgat tgtcgggctg ggcggcggct 540

cttccctgga cgccggcaag ggaatgtgga ttttctacga atatccggat gcaaagtggg 600

aagacatttt cgttcccttc accggcgtgc cgaagctgag aaacaaggcc aggtacgtgg 660

ccattccttc caccagcggc accgccaccg aggtaaccct ggcggcggtt atcaccaacc 720

gcgacgttgt gccgaacgtt aaggacttta ccctttccta cgaggttacg ccggacattg 780

ccatctgcga tccggagttg ccttccacaa tgccacctgc cgtcaccgcc aacaccggct 840

ttgacgtgat cgttcacgcc gttgaggcat acgtttctgt aaacgccacc gacgttaccg 900

acccgattgc cctgcggtct gccgccatgg cttataaatg gttgcctctg gccttcgcca 960

acggcggaaa caaagaagca agggaaaaaa tgcacacggc ctcgctcatg gccggcttta 1020

cctttaccaa caccgccctg gggctggtcc acagcaccgc ccaccagatc ggggcggaat 1080

acggcgtgcc gcacggccgg gccaacgcaa tcatgctgcc gtatgtggtg cagtacaacg 1140

ccccggcggc cgccgcacgt tacgccgata tggcccaggc cttcgggtat gcttccaccg 1200

atccgttcga gggcacggtc aagttcatcg acgccgtccg gaagctgcaa aagattctgg 1260

gcatccctgc ctgtctgaag gatgccggca ttaaagaaga ggacttcctg aagaaagtgg 1320

acatgctcac caagaacgcc atgaacgacg gcagcacgcc ggccaacccg cgggtcccaa 1380

ccctggcgga aatcaagaac atcttcctct gcgcctatta cggaaacgac gtgatttaat 1440

aaggctgaga tcttcttcag tgcattgtag ttgaatgaag ggttaggggg gaaatgcccc 1500

cctatttttt gtctagccat cctgccacgt ttgacagggt agcaatttcg acacgatagg 1560

gttctctctt ctgccgtta 1579

<210> 205
<211> 1822
<212> DNA
<213> Artificial Sequence

<220>
<223> SEQ ID NO: 205 presents example 205 of a designer
 Thermosynechococcus elongatus nirA-promoter-controlled lipase
 (using butanol) DNA construct (1822 bp)

<400> 205
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgggtatc tttgactata aaaaccttgg caccgagggt tccaaaacgt 300

tgttcgccga tgccatggcg atcacgttgt attcctatca caacctggat aacggctttg 360

ccgtgggcta ccagcacaac gggttgggct tggggctacc ggccacgctg gtcggtgcgc 420

tgctcggcag cacggattcc cagggcgtga tccctggcat cccgtggaac ccggattcag 480

aaaaagccgc ccttgaggcg gtgcagaaag ccggttggac accgatcagc gccagtgccc 540

tgggctacgc cggcaaggtc gatgcacgtg gcaccttctt tggggaaaaa gccggctaca 600

ccacggccca ggtcgaggta ctcggcaaat acgatgacgc cggcaagctg ctcgaaatcg 660

gcatcggttt tcgtggcact tcggggccac gggaaacctt gatcagcgac tcgatcggcg 720

acttgatcag cgatctgctc gcggccctgg ggcccaagga ttacgcgaaa aactacgccg 780

gcgaagcctt cggcggcttg ctcaagaatg ttgccgacta cgccggtgcc catggcctga 840

ccggcaagga cgtggtggtc agcggccaca gcctgggcgg gctggcggtc aacagcatgg 900

cggacttgag caactacaaa tgggcggggt tctacaagga cgccaactat gttgcctatg 960

cctcgccgac ccagagtgcc ggcgacaagg tgctcaatat cggttacgaa aacgacccgg 1020

tgttccgcgc gctggacggc tcgtcgttta acctgtcgtc gctgggcgtg cacgacaaac 1080

cccacgagtc caccaccgat aacatcgtca gcttcaacga ccactacgcc tcgacgctgt 1140

ggaatgtgct gccgttttcc atcgtcaacc tgcccacctg ggtctcgcat ttgccgacgg 1200

cgtacggcga tggcatgacg cgcatcctcg agtccggctt ctacgaccag atgacccgtg 1260

actccacggt gattgttgcc aacctgtccg atccggcgcg ggccaacacc tgggtgcagg 1320

acctcaaccg caatgccgag ccccacaagg gcaacacgtt catcatcggc agcgacggca 1380

acgacctgat ccagggcggc aacggtgcgg actttatcga gggtggcaaa ggcaacgaca 1440

cgatccgcga caacagcggg cacaacacct ttttgttcag cggccacttt ggcaatgatc 1500

gcgtgattgg ctaccagccc accgacaaac tggtgttcaa ggacgtgcaa ggaagcaccg 1560

acctgcgtga ccacgcgaag gtggtcggcg ccgatacggt gcttacgttt ggggccgact 1620

cggtgacgct ggtcggcgtg gggcatggcg ggctgtggac ggagggcgtg gtgatcggct 1680

gataaggctg agatcttctt cagtgcattg tagttgaatg aagggttagg ggggaaatgc 1740

ccccctattt tttgtctagc catcctgcca cgtttgacag ggtagcaatt tcgacacgat 1800

agggttctct cttctgccgt ta 1822

<210> 206
<211> 1486
<212> DNA
<213> Artificial Sequence

<220>
<223> SEQ ID NO: 206 presents example 206 of a designer
Thermosynechococcus elongates nirA-promoter-controlled lipase
(using ethanol) DNA construct (1486 bp)

<400> 206
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggccagg acgatgcgtt ccagggtggt ggcaggggca gtggcatgcg 300

cgatgagcat cgcgccgttc gcggggacga ccgcggtgat gacgctcgcg acgacgcacg 360

cggcaatggc ggccaccgcg cccgccgctg gctacgcggc gacgcgttac ccgatcatcc 420

tcgtgcacgg gctctcgggt accgacaagt acgccggcgt gctcgagtat tggtacggca 480

tccaggagga cctgcaacag aacggtgcga ccgtctacgt cgcgaacctg tcgggtttcc 540

agagcgacga cggcccgaac gggcgcggcg aacagttgct cgcttacgtg aagacggtgc 600

tcgcggcgac gggggcgacc aaggtcaatc tcgtcggtca cagccagggc ggcctctcgt 660

cgcgctatgt tgctgccgtc gcgcccgatc tcgttgcgtc ggtgacgacg atcggcacgc 720

cgcatcgcgg ctccgaattc gccgacttcg tgcaggacgt gctcgcgtac gatccgaccg 780

ggctttcgtc atcggtgatc gccgcgttcg tcaatgtgtt cgggatcctg acgagcagca 840

gccacaacac caaccaggac gcgctcgccg cactgcagac gctgaccacc gcacgggccg 900

ccacgtacaa ccagaactat ccgagcgcgg gcctgggtgc gccgggcagt tgccagaccg 960

gtgcgccgac cgaaaccgtc ggcggcaaca cgcacctgct gtattcgtgg gccggcacgg 1020

cgatccagcc gacgctctcc gtgttcggcg tcacgggcgc gacggacacg agcacccttc 1080

cgctcgtcga tccggcgaac gtgctcgacc tgtcgacgct cgcgctgttc ggcaccggca 1140

cggtgatgat caaccgcggc tccgggcaga acgacgggct cgtgtcgaag tgcagtgcgc 1200

tgtacggcaa ggtgctgagc acgagctaca agtggaacca cctcgacgag atcaaccagc 1260

tgctcggcgt gcgcggcgcg tatgcggaag atcccgtcgc ggtgatccgc acgcatgcga 1320

accggctgaa gctggcgggc gtgtaataag gctgagatct tcttcagtgc attgtagttg 1380

aatgaagggt tagggggaa atgcccccct atttttgtc tagccatcct gccacgtttg 1440

acagggtagc aatttcgaca cgatagggtt ctctcttctg ccgtta 1486

<210> 207
<211> 2098
<212> DNA
<213> Artificial Sequence

<220>
<223> SEQ ID NO: 207 presents example 207 of a designer
      Thermosynechococcus elongatus nirA-promoter-controlled Pyruvate
      Decarboxylase DNA construct (2098 bp)

<400> 207
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catgagttat actgtcggta cctatttagc ggagcggctt gtccagattg 300

gtctcaagca tcacttcgca gtcgcgggcg actacaacct cgtccttctt gacaacctgc 360

ttttgaacaa aaacatggag caggtttatt gctgtaacga actgaactgc ggtttcagtg 420

cagaaggtta tgctcgtgcc aaaggcgcag cagcagccgt cgttacctac agcgttggtg 480

cgctttccgc atttgatgct atcggtggcg cctatgcaga aaaccttccg gttatcctga 540

tctccggtgc tccgaacaac aacgaccacg ctgctggtca tgtgttgcat catgctcttg 600

gcaaaaccga ctatcactat cagttggaaa tggccaagaa catcacggcc gccgctgaag 660

cgatttacac cccggaagaa gctccggcta aaatcgatca cgtgatcaaa actgctcttc 720

gcgagaagaa gccggtttat ctcgaaatcg cttgcaacac tgcttccatg ccctgcgccg 780

ctcctggacc ggcaagtgca ttgttcaatg acgaagccag cgacgaagca tccttgaatg 840

cagcggttga cgaaaccctg aaattcatcg ccaaccgcga caaagttgcc gtcctcgtcg 900

gcagcaagct gcgcgctgct ggtgctgaag aagctgctgt taaattcacc gacgctttgg 960

gcggtgcagt ggctactatg gctgctgcca agagcttctt cccagaagaa aatgccaatt 1020

acattggtac ctcatggggc gaagtcagct atccgggcgt tgaaaagacg atgaaagaag 1080

ccgatgcggt tatcgctctg gctcctgtct tcaacgacta ctccaccact ggttggacgg 1140

atatccctga tcctaagaaa ctggttctcg ctgaaccgcg ttctgtcgtt gtcaacggca 1200

ttcgcttccc cagcgttcat ctgaaagact atctgacccg tttggctcag aaagtttcca 1260

agaaaaccgg ttctttggac ttcttcaaat ccctcaatgc aggtgaactg aagaaagccg 1320

ctccggctga tccgagtgct ccgttggtca acgcagaaat cgcccgtcag gtcgaagctc 1380

ttctgacccc gaacacgacg gttattgctg aaaccggtga ctcttggttc aatgctcagc 1440

gcatgaagct cccgaacggt gctcgcgttg aatatgaaat gcagtggggt cacattggtt 1500

ggtccgttcc tgccgccttc ggttatgccg tcggtgctcc ggaacgtcgc aacatcctca 1560

tggttggtga tggttccttc cagctgacgg ctcaggaagt tgctcagatg gttcgcctga 1620

aactgccggt tatcatcttc ttgatcaata actatggtta caccatcgaa gttatgatcc 1680

atgatggtcc gtacaacaac atcaagaact gggattatgc cggtctgatg gaagtgttca 1740

acggtaacgg tggttatgac agcggtgctg ctaaaggcct gaaggctaaa accggtggcg 1800

aactggcaga agctatcaag gttgctctgg caaacaccga cggcccaacc ctgatcgaat 1860

gcttcatcgg tcgtgaagac tgcactgaag aattggtcaa atggggtaag cgcgttgctg 1920

ccgccaacag ccgtaagcct gttaacaagc tcctctagta aggctgagat cttcttcagt 1980

gcattgtagt tgaatgaagg gttagggggg aaatgccccc ctattttttg tctagccatc 2040

ctgccacgtt tgacagggta gcaatttcga cacgataggg ttctctcttc tgccgtta 2098

<210> 208
<211> 1567
<212> DNA
<213> Artificial Sequence

<220>
<223> SEQ ID NO: 208 presents example 208 of a designer Synechocystis
      sp. PCC 6803 nirA-promoter-controlled Lipase DNA construct (1567
      bp)

<400> 208
agaaaatctg gcaccacacc ctaaatgcgt aaactgcata tgccttggct gagtgtaatt 60

tacgttacaa attttaacga aacgggaacc ctatattgat ctctacatga tgaagctact 120

ctctctgacc ggtgtggctg gtgtgcttgc gacttgcgtt gcagccactc ctttggtgaa 180

gcgtctacct tccggttcgg accctgcctt ttcgcagccc aagtcggtgc tcgatgcggg 240

tctgacctgc cagggtgctt cgccatcctc ggtctccaaa cccatccttc tcgtccccgg 300

aaccggcacc acaggtccac agtcgttcga ctcgaactgg atccccctct caacgcagtt 360

gggttacaca ccctgctgga tctcaccccc gccgttcatg ctcaacgaca cccaggtcaa 420

cacggagtac atggtcaacg ccatcaccgc gctctacgct ggttcgggca acaacaagct 480

tcccgtgctt acctggtccc agggtggtct ggttgcacag tggggtctga ccttcttccc 540

cagtatcagg tccaaggtcg atcgacttat ggcctttgcg cccgactaca agggcaccgt 600

cctcgccggc cctctcgatg cactcgcggt tagtgcaccc tccgtatggc agcaaaccac 660

cggttcggca ctcaccaccg cactccgaaa cgcaggtggt ctgacccaga tcgtgcccac 720

caccaacctc tactcggcga ccgacgagat cgttcagcct caggtgtcca actcgccact 780

cgactcatcc tacctcttca acggaaagaa cgtccaggca caggccgtgt gtgggccgct 840

gttcgtcatc gaccatgcag gctcgctcac ctcgcagttc tcctacgtcg tcggtcgatc 900

cgccctgcgc tccaccacgg gccaggctcg tagtgcagac tatggcatta cggactgcaa 960

ccctcttccc gccaatgatc tgactcccga gcaaaaggtc gccgcggctg cgctcctggc 1020

gccggcagct gcagccatcg tggcgggtcc aaagcagaac tgcgagcccg acctcatgcc 1080

ctacgcccgc ccctttgcag taggcaaaag gacctgctcc ggcatcgtca ccccctgata 1140

gtaatgagtt acagttttgg caattactaa aaaactgact tcaattcaat gttagcccgc 1200

tcccgcgggt tttttgttgc tttttcacag tgactatagg taatcagcaa cacaatacgg 1260

ccctgttctt tggacagttt ttgtataatg ttgaccgcat cctgaccgga ttttttatct 1320

aagtggggaa ttgtcaattg tcaattaaag ctaagttcta ctaatgtttt agaaggcatt 1380

gtcgattgaa aataagggtt gaatggagaa aattttgagc ctttgtcaaa gataaaaatt 1440

tatttcaaca gttttttaac tagccgaacc agagaatgac ccagtggcgc tgactttgct 1500

cccgagtttt tgttagaaat taccctcaag aagtaatcta ataataaggt tctctcttct 1560

gccgtta 1567

<210> 209
<211> 2075
<212> DNA
<213> Artificial Sequence

<220>
<223> SEQ ID NO: 209 presents example 209 of a designer Anabaena PCC
      7120 hox-promoter-controlled Lipase DNA construct (2075 bp)

<400> 209
agaaaatctg gcaccacacc gcagaaatat aggggctagg agttgagggt actctggttc 60

gtcaagcatt tgggatgatt tcccctcaca agttcctcaa attattctcc tataaacaat 120

agatataagg tcaaaacttg agttatgagt gctgagtaaa aaattactct ccacgcctca 180

gaggtagata tgatgggtat ctttgactat aaaaaccttg gcaccgaggg ttccaaaacg 240

ttgttcgccg atgccatggc gatcacgttg tattcctatc acaacctgga taacggcttt 300

gccgtgggct accagcacaa cgggttgggc ttggggctac cggccacgct ggtcggtgcg 360

ctgctcggca gcacggattc ccagggcgtg atccctggca tcccgtggaa cccggattca 420

gaaaaagccg cccttgaggc ggtgcagaaa gccggttgga caccgatcag cgccagtgcc 480

ctgggctacg ccggcaaggt cgatgcacgt ggcaccttct ttggggaaaa agccggctac 540

accacggccc aggtcgaggt actcggcaaa tacgatgacg ccggcaagct gctcgaaatc 600

ggcatcggtt ttcgtggcac ttcggggcca cgggaaacct tgatcagcga ctcgatcggc 660

gacttgatca gcgatctgct cgcggccctg gggcccaagg attacgcgaa aaactacgcc 720

ggcgaagcct tcggcggctt gctcaagaat gttgccgact acgccggtgc ccatggcctg 780

accggcaagg acgtggtggt cagcggccac agcctgggcg ggctggcggt caacagcatg 840

gcggacttga gcaactacaa atgggcgggg ttctacaagg acgccaacta tgttgcctat 900

gcctcgccga cccagagtgc cggcgacaag gtgctcaata tcggttacga aaacgacccg 960

gtgttccgcg cgctggacgg ctcgtcgttt aacctgtcgt cgctgggcgt gcacgacaaa 1020

ccccacgagt ccaccaccga taacatcgtc agcttcaacg accactacgc ctcgacgctg 1080

tggaatgtgc tgccgttttc catcgtcaac ctgcccacct gggtctcgca tttgccgacg 1140

gcgtacggcg atggcatgac gcgcatcctc gagtccggct tctacgacca gatgacccgt 1200

gactccacgg tgattgttgc caacctgtcc gatccggcgc gggccaacac ctgggtgcag 1260

gacctcaacc gcaatgccga gccccacaag ggcaacacgt tcatcatcgg cagcgacggc 1320

aacgacctga tccagggcgg caacggtgcg gactttatcg agggtggcaa aggcaacgac 1380

acgatccgcg acaacagcgg gcacaacacc tttttgttca gcggccactt tggcaatgat 1440

cgcgtgattg gctaccagcc caccgacaaa ctggtgttca aggacgtgca aggaagcacc 1500

gacctgcgtg accacgcgaa ggtggtcggc gccgatacgg tgcttacgtt tggggccgac 1560

tcggtgacgc tggtcggcgt ggggcatggc gggctgtgga cggagggcgt ggtgatcggc 1620

tgatgaagta agtaggaagc agggagcagg ggaaagaaaa ttgacaactg tacaagatta 1680

atcgcgtctc tgagcaatga ccaaatacat ctacctccac ggttttcttc cagcccccta 1740

tctgcgaaag cacaagatat tagcaagcgt ttcgcccaaa ttcacataca gctaacaatc 1800

cctgatctca atgctggtga attttctcag ttaacaatca cgcgccaaat tcaacaagtt 1860

gccgcaattt tccctgataa ttctgaacca ataacgctga taggttctag tttaggcggt 1920

ttaactgctg cttatctagg acagcgatat ttacaagtac aacgcttagt tttattagcg 1980

ccagtttggt tttttatccc attggttgcc caaaatgggt gaagaagctg tcacaagttg 2040

gcaacaaacg atataggttc tctcttctgc cgtta 2075

<210> 210
<211> 2290
<212> DNA
<213> Artificial Sequence

<220>
<223> SEQ ID NO: 210 presents example 210 of a designer Synechococcus
sp. strain PCC 7942 nirA-promoter-controlled Lipase gene DNA
construct (2290 base pairs (bp))

<400> 210
agaaaatctg gcaccacacc cttcttgcag aacatgcatg atttacaaaa agttgtagtt 60

tctgttacca attgcgaatc gagaactgcc taatctgccg agtatatgat gggtgtgtat 120

gactacaaaa acttcggcac ggcggattcc aaggcgttgt tcagcgatgc catggcgatc 180

acgctgtatt cctaccacaa cctcgataac ggttttgccg ccggttatca gcacaacggt 240

tttggccttg gcctgccggc gacgctggtc acggcgttgc tcggcggtac cgattcccag 300

ggcgtcatcc ccggcattcc gtggaatccc gattcggaaa aactcgccct cgaagccgtg 360

aaaaaggccg gctggacgcc gatcacggcc tcgcaactgg gctacgacgg caagaccgac 420

gcacgcggaa ccttctttgg cgagaaggcc ggttactcga cagcgcaggt cgagattctc 480

ggcaagtacg acgcccaggg ccatctcaca gaaatcggca tcgcctttcg cggcaccagc 540

ggcccgcgcg agaacctgat ccttgattcc atcggcgacg tgatcaacga cttgctcgcc 600

gcgttcggcc ccaaggatta cgccaagaac tacgtcggcg aagcgttcgg caacctgctc 660

aatgacgtgg tcgcctttgc caaggccaat ggcctcagcg gcaaggacgt gctggtcagc 720

ggccacagcc tcggcgggct ggcggtcaac agcatggcgg atttgagcgg cggcaagtgg 780

ggcgggttct tcgccgactc caactacatc gcctatgcct cgccgaccca gagcagcacc 840

gacaaagtgc tcaacgtcgg ctacgagaac gacccggtgt tccgcgccct cgacggttcg 900

aatttcaccg gcgcctcgat tggcgtgcac gacgcgccga aggaatcggc caccgacaac 960

atcgtcagct tcaacgatca ctacgcctcg acggcgtgga atctgctgcc gttctccatc 1020

ctcaacatcc cgacctggat ctcgcacctg ccaaccgctt acggcgacgg catgaaccgg 1080

gtgatcgagt cgaagttcta cgacctgacc agcaaggact cgacgatcat cgtcgccaac 1140

ctgtcggatc cggcgcgggc caacacctgg gtgcaggatc tcaaccgcaa cgccgaaacc 1200

cacaagggca gcaccttcat catcggcagc gacgccaacg atctgattca gggtggcagc 1260

ggcaatgact atctggaagg tcgcgccggc aacgacacct ttcgcgacag cggcggctac 1320

aacatcatcc tgggcgggca gggcagcaat acgctggact tgcagaagtc ggtgaatacc 1380

ttcgacttcg ccaacgacgg cgccggcaat ctgtacattc gcgatgccaa cggcgggatc 1440

agcatcaccc gcgacatcgg cgccatcgtc accaaagagc cgggcttcct ctggggtctg 1500

ttcaaggacg acgtgaccca cagcgtcacg gccagtggct tgaaggtcgg caacaacctg 1560

accgcctacg agtcgagcgt gaagggcagc aacggcgccg acacgctcaa ggcgcatgcc 1620

ggcggcgact ggttgttcgg cctcgacggc aacgatcatc tgatcggcgg ggcgggcaac 1680

gatgtgtttg ttggcggcgc cggtaacgat ctgatggagt ccgggggcgg ggcggatacg 1740

ttcctgttca acggcgcgtt cggccaggat cgggtggtgg gattcacgtc caacgacaaa 1800

ctggtgtttc tcggcgtgca gggtgtgttg cctggcgatg acttccgagc gcatgcctcg 1860

gcagccgggc aggataccgt gctgaagttc ggcggcgatt cggtgacatt ggttggcgtt 1920

tcgctgggga gtttgagtgg cgatggaatt gtgatcgcct gataatagtg atcccggccg 1980

ctactaaagc ctgatttgtc ttgatagctg ctcctgcctt tgggcagggg cttttttctg 2040

tctgccattc ttgaggatgg cggactcttt cccttttgct ctacgcccat gaatgcgatc 2100

gcagtctccc ctgtccagca cgttggagtg attggtggtg gccagttagc ttggatgctg 2160

gcaccagcag cgcaacagtt ggggatgtcg ctgcacgttc aaacacccaa tgatcacgac 2220

ccagcagtag cgatcgcgga tcaaaccgta ttagcagcag ttgctgacgc ggttctctct 2280

tctgccgtta 2290

<210> 211
<211> 1231
<212> DNA
<213> Artificial Sequence

<220>
<223> SEQ ID NO:211 presents example 211 for a designer Prochlorococcus
 marinus MIT9313 groE-promoter-controlled Lipase DNA construct
 (1231 bp)

<400> 211

agaaaatctg gcaccacacc ccctttcaga gcggcgcaac attaccactg catggcgaga 60

tcttctcagg gttcggtgac ccgcacaggt atccactagt cggcacagca tcaacacaca 120

tagggttggc actcaatggc cacgagtgct actcatggtg ctggggctgt tcgccgcgcc 180

ccggctgaat atgcgggagg actacgagaa ggtccgtcat ctgcagcggc agcttgccgc 240

ccgacccggg cagcggttcc gggccgcctc ctggcggacc atcgccgagg acggcgacag 300

tcatgtgccg gtccgggtct ttacgccgaa ggagcgtcgg cgcgaggacc tgctcctgtt 360

cttccatggg ggcggctggg tcaccggcga tatcgagagc tacaccccgg cctgctccac 420

gatggcggac ctcaccggct gcgtcgtcat ctccgtcgac taccggctcg cccccgagca 480

cccgtacccc gcgggtctcg aggactgcta ccagatcgtg cagcgactgc tggatgaacc 540

ggagctggcc cagctcgacg atccgcatcg gatcgtcctg gtgggcgact ccgcgggcgg 600

caacctcgcg gccgccgtct ccctgctgct gcgcgagcgc ggccatcgtg gggtggaccg 660

gcagatcctg ctgtacccgg tgacccattg ggatcatgac ccggagacct cccccttcgc 720

ctccgtgcgc caccacggtg aggactatcg cctgaccaac accgaggtcc aggactacat 780

ggagctctat gtccccgatc cgaccatgcg ccgcgacccg ctggtgtccc cgctgatggc 840

gacggacctc tcctcccaac cggcgacgct gctgatcagc gcagagctcg acctgctgtg 900

cgacgagggc gaggcctacg gtcgggctct cgaggaggcc gggaacaccg tgcgcatcca 960

ccgcgtggag ggggctctgc acggcttcat ctccctgccc cggttctccc ggtcgctccg 1020

agacgcctac gaggtgatca acgcgttcct cgacgaggac ggggccgccc atccggagga 1080

gacgacgtga tgatttagtt tcggtgtcta tctcttaata gcctcgattt attttcgggg 1140

ctattaatca actctcagag gcgacaagct tcttcttccc ttacgacgtt tttattggtt 1200

ggacatggca aggttctctc ttctgccgtt a 1231

<210> 212
<211> 1386
<212> DNA
<213> Artificial Sequence

<220>
<223> SEQ ID NO:212 presents example 212 for a designer Cyanothece sp.
ATCC 51142 nirA-promoter-controlled Lipase DNA construct (1386
bp)

<400> 212
agaaaatctg gcaccacacc tattaaatct aaaatagctg ttttagctaa aatagtcaat 60

agcaagtctt ataggtaatc aaacgcaact aaaatgcaaa aaatccataa ttaaaatgca 120

aaaaacggat ttttaataca attttgttac attagctaca aaatatctca aatggtagag 180

gttaaatagg tacaactcga ccagatggag ggttttccct gtggtgagca cgttcacact 240

tccgctgccc gtggtcgcat tcttgttgaa gccgtacttc cgtctggctt tcaactcgcg 300

actgccgtac cggattcaac gtccgttgct cgacgccggg gcaccgctac agcaggttcc 360

agcgggcgct gtcgtgcgcc cgctggagct cgcctgccga ccggcggaga agatcaccgt 420

cggcgcaacc gagcgcccca ccgcagttct gtacctgcac ggcggggcgt acacgcttgg 480

ctcgctggca acccaccgtt ctctcgcagc gcatctcgcg cgtgaatcgt ccagtgcggt 540

cttcaccttg gactaccgcc tcgcaccgga gaacccgtat cccgcagctc tcgacgacgc 600

ggtctccgcc ttcatgcagt tggtcaccga gttcgactat tcggaagacc gcgttgccat 660

cgccggcgat tcggccggtg gcggtttggc cgccgcgacc gctctgcgtc tgatcgaacg 720

tcacggtgtg acccccggtg cgcttgctct gatctcgccg tgggtcgatc ccggcgatcg 780

aggtgccacc aagaaggccg acctggtcgt caatacagcc tggtccttcg atgccgccga 840

cgcttacctt gccggcggcg atcgcacaga tcccggctac gcaccgatgc acgggaagct 900

cgacgggttg ccgcccacca ggattcacgt cggttccgaa gaagttctct acccgcagat 960

catgacgttc accgacaagc tctccgctgc cggcgtcgat gtgtccctgg tcgagtacgc 1020

aagactgtgg cacgtggctc acgcacaggc gtcactggtg aaggaagcag cagacgccgt 1080

cgccgatctg gggggcttca tcgcatcgaa actgattcgc gccaagaatg attcgagttc 1140

agcagtaggc tcgaacagtc actgataagc tgttttagag aaatttgttc ggtaaatatt 1200

agcctaccta cagttgttgt gggtaggcta atattatgaa ttgagtccta ctgaaccaat 1260

gattatcgtt acgactaaaa gtaataaatg tcatcagcag gataggggtt gataggaaaa 1320

gttttttaat cggatggttt tcgagttaga ggttagggtt tctttaggtt ctctcttctg 1380

ccgtta 1386

<210> 213
<211> 2420
<212> DNA
<213> Artificial Sequence

<220>
<223> SEQ ID NO. 213 presents example 213 of a designer Chlamydomonas
reinhardtii Nial-promoter-controlled chloroplast-targeted Lipase
DNA construct (2420 bp)

<400> 213
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat ggccgccgtc attgccaagt cctccgtctc cgcggccgtg gctcgcccgg 240

cccgctccag cgtgcgcccc atggccgcgc tgaagcccgc cgtcaaggct gcccccgtgg 300

ctgccccggc tcaggccaac cagatgggtg tgtatgacta caagaacttc ggcacagccg 360

attccaaagc gttattcacc gacgctatgg cgatcacgct gtattcctat cacaacctcg 420

acaatggctt tgccaccggc taccagcaca acggcttcgg cctgggcttg ccggcgactt 480

tggttaccgc gctgctgggc ggcaccgact ctcaaggcgt gatccccggc attccctgga 540

accccgattc ggaaaaagcc gccctggagg ccgtgcaaaa agccggctgg acgcccatca 600

cggcctcgca actgggttat gacggcaagg tcgacgctcg cggaacgttc ttcggcgaga 660

aggccgggta caccagcgcg caggtcgaaa tcctcggcaa gtacgatgct caaggccatc 720

tcagcgaaat cggcatcgcc tttcgcggca ccagcggccc gcgggaaatc ttgatcggcg 780

attccatcgg cgacgtgatc aacgatctgc tggcggcgct aggtcccaag gattatgcga 840

aaaactatgt gggtgaagcc ttcggcaatc tgctcggcga cgtcatggcg tttgcccagg 900

ccaatggcct gtcgggaaaa aacgtgctgg tcagcggcca cagcctcggc gggctggcgg 960

tcaacagcct ggcggacttg agcgcggaga agtggtccgg gttctaccag gattccaact 1020

acatcgccta cgcgtccccg acccagagca gcaccgacaa agtgctcaat gtcggctatg 1080

aaaacgaccc ggtttttcgg gctctcgacg gttcatcctt caacctttcg tcggtgggtg 1140

tgcacgatgc cgccaaggcc tcggcgaccg ataacatcgt cagcttcaac gatcactacg 1200

cctcgaccgc atggaatgtg ctgccgttct ccatcctcaa catcccgacc tggatctcgc 1260

acttgccgac cgcctatggc gacggcatga accgggtgat cgaatcgaag ttctacgacc 1320

tcaccagcaa ggactcgacg atcgtcgtcg ccaatctctc ggacccggca cgcgccaata 1380

cctgggttca ggacctcaat cgcaacgccg aaacccacaa gggcagtacc ttcatcatcg 1440

gcagcgacgg caacgacctg attcagggtg gcaagggtaa cgactacctg gaggggcgtg 1500

acggtaacga caccttccgt gacggcggcg gctacaacat cgtattgggt ggcaagggca 1560

gcaacgtgct ggacttgcag cagtcggtga aaaatttcaa ctttgccaac gatggcgccg 1620

gcaccctcta tgttcgtgac gccaatggcg gtatcagcat cacccgagac atcggcagta 1680

ttgtcaccaa ggaaccgggg ttcttgtggg ggctgttcaa ggatgatgtg acgcacagtg 1740

tgacggccaa tggacttgcc gtcggcaaca acctgacgtc atacgcgtca tcggtgaagg 1800

gcggcacggg ggccgatacg ctcaaggcgc atacgacggg cgattggttg ttcggtctgg 1860

acggcaacga tcatttgatc ggcggccagg gcaacgatgt gtttgtgggc ggggcgggga 1920

atgacctgat ggaatcgggg ggcgggattg atacgttcct gttcagcggt gcgtttggtc 1980

aggaccgggt ggtggggtat caggcaaacg acaagctggt attcctcggg gttcaggggg 2040

tcgcgccgaa tgatgactat cgggctcatg ccacgacggt ggggcaggat acggtgctga 2100

cgtttggcgg ggattcggtg acgttggttg gggtggcgct caatagcctc agtgctgatg 2160

gaatcgtgat cgcctgataa atggaggcgc tcgttgatct gagccttgcc ccctgacgaa 2220

cggcggtgga tggaagatac tgctctcaag tgctgaagcg gtagcttagc tccccgtttc 2280

gtgctgatca gtctttttca acacgtaaaa agcggaggag ttttgcaatt ttgttggttg 2340

taacgatcct ccgttgattt tggcctcttt ctccatgggc gggctgggcg tatttgaagc 2400

ggttctctct tctgccgtta 2420

<210> 214
<211> 6498
<212> DNA
<213> Artificial Sequence

<220>
<223> SEQ ID NO: 214 presents example 214 of a designer Synechocystis
      sp. PCC 6803 transcription factor-arginine-based
      alcohol-selection DNA construct (6498 bp)

<400> 214
agaaaatctg gcaccacacc tctaccacat agtccgccaa gcgccgatcc cggttgagcg 60

ccagggccac atcccggacc agatcctcga cgaatttcgg attgtcatag gcccgctcgg 120

taacgaattt ctcgtccggc cgcttgagaa gcccgtacac ctcgcaggag gcgctctctt 180

cggcgacggc gatcaattcc tcgatcagca tgtcgccagc cagcctcacc tcgatgccga 240

tatgggagcg ctggttgtgg gcgccgtagt cggagattcc cttggagcag gggcacaggc 300

tcgtcgccgg caccaggacg cgcaaggaaa aggaatactc cccggcatcg gataccgagc 360

cctgccagca gacctcgtag tcgaggaggc tccgcacacc cgacaccggc gccgtcttgc 420

tcacgaaata gggaaagcgc atctcgatcg tgcccctcct cgcctccagc ctgtccaaca 480

ttgcgagggc cagggctttg aacgccgcct gatccagcgg gccggtctgc tcctccatca 540

gttcgacgaa gcgggacatg agggttccct tgacgtgcgc cgccaaaccg acggtgagcg 600

acagggtggc aacggtcgcc agccgctcgg cgcccgaccg gatggtggca gggtatctga 660

ggcccttgac cccgaccgca tgtatgggta ggtcacggcc atccagttgc gactggacgt 720

ccggcatcag agttgctctg tcaggggcat tcatctcggt ccttcctcgg gaatggggtc 780

gaacaaaacg gagaactggc cgcactagag taaagtagat aaaatccttt ctcaatggtt 840

ttatgtataa agccccagag ccaccgaccg tgtcgatgcg gagcgcgtca ggaggcgagt 900

ccaggacagt cggagagcgt gacatgcttg ccatattggg aacgcgccag cgggagttgc 960

tgaagctcct cctgaggagc aagggcggtt tgacggtggt cgagctctcg gcgcggctgg 1020

gcatctccag caacgcggtt cgccagcatc ttgccgccct ggagaacgag gggctggtcg 1080

cccacggcga gtcccgtcct tccggcgggc ggccggagca actctacgtg ctcactgcgg 1140

cgggcaagga gctttttccc cgtcagtatt cctggttcgc ccagttggtg gtcgagtcca 1200

tgaagcagga agccggcgcc gaggcgacgc gggaacgcct cgcggccatg ggatcgggca 1260

ttgcccgctt gttgctccgt cagcatggcg ggctcgaagg ccggcggcag cgcgtggcca 1320

agctcgccga actcatggag gcgctgggtt acgagatcgg cagcgtcgat gccgaaggca 1380

aagagccggt gattgaggcc aacaactgcg ttttccacaa gctcgccatg gacgacccgg 1440

atatctgcga gttcgacctg gccctgctgt ccacgtttac ggacagcaag gtcgaccatc 1500

aggagtgcat cgccaagggc ggcaatgtct gtcgtttccg gttctcgcga aaacgctaat 1560

acatccggta atgaacgtgc gctaccacac tttgatggct caccccacag acacgctgcg 1620

tggcagaccg tgcaccaggg cgtcgtgccg gcgcactgca actggattat gatatcgcca 1680

tcgaccgccg ccgacggcaa cgcccggtag acgaacgtgt acgccgccgg cgacggcatg 1740

aacgccaagc atcgtcctca atcaggtacg agatccggag aacctgagtg cggagcgacg 1800

ttctattttc gtattcagta acaatttgtc gagaatcggt ctgatccgcg gcttcacgca 1860

cctgcccgag gggcttgcgc ggaccttgag gtgaccttga gcgggcagat accaccaaaa 1920

tttcccacgt gctattatgg ttttgctaaa gctctcgaca gcgaggagag actcgcgaag 1980

ataagcaatt cgcccgacag aggtgaatga ggagacggta tgtccaagat gcaagagttc 2040

gcgcggctgg agacagtcgc gtcgatgcgc agagcggtct gggacggcaa cgagtgtcag 2100

ccggggaaag tggctgatgt cgttttgcgc tcgtggaccc ggtgtcgtgc tgaaggtgtc 2160

gttcccaatg cccgccagga gttcgacccg atcccgcgaa cggcgcttga cgaaacggtt 2220

gaggccaagc gggcgctgat ccttgctgcc gagccggtcg tcgacgcgtt gatggagcag 2280

atgaacgacg cccccaggat gatcatcctg aacgacgaac ggggcgtcgt gctgctgaac 2340

cagggaaacg acaccctcct tgaagacgcc cgccgccggg ccgtgcgggt gggcgtctgc 2400

tgggacgaac acgcccgagg caccaatgcc atgggaaccg cgctcgcgga gaggaggccc 2460

gtagcgatcc acggcgcaga gcactacctc gagtcgaata cgattttcac ctgcaccgcg 2520

gcgccgatct acgatccgtt cggcgagttc accggaattc tggatatcag cggatatgcg 2580

ggggacatgg gcccggttcc gattcccttt gttcagatgg cggtgcaatt catcgagaat 2640

cagttgttcc gccagacctt tgccgattgc attctgctgc actttcatgt gcgccccgac 2700

ttcgtcggaa cgatgcgcga agggatagcc gtgctgtcgc gcgagggaac catcgtctcg 2760

atgaaccgtg ctgggctcaa gatcgcaggg ctcaacctgg aggccgtcgc cgatcaccgt 2820

ttcgattccg tcttcgactt gaatttcggg gcctttctcg accacgtgcg gcagtccgcc 2880

ttcggtctcg tccgcgtctc gctctacggc ggcgttcagg tctacgcccg agtggaaccg 2940

ggcctgcgtg ttccgccacg tccggccgcc cacgcccgcc ctcctcggcc ggcaccgcgg 3000

cctctggatt cgctggacac gggcgacgca gcagtccgcc tcgcgattga ccgcgcccgc 3060

cgcgcgatcg gccgcaacct cagcatcctc atccagggcg agacgggtgc cggcaaggaa 3120

gtgttcgcca agcatctgca tgccgagagc ccgagaagca aggggccgtt cgttgccgtc 3180

aattgcgccg ccatacccga gggtttgatc gagtccgagc ttttcggata cgaagaaggg 3240

gccttcactg gcggacggcg caaaggcaac atcggcaagg tcgcccaggc ccacggcggc 3300

acgttgttcc tcgacgagat cggagacatg gcgccggggc tgcagaccag actgctgcgg 3360

gttctacagg accgggcagt gatgcccctc ggcggtcgcg aacctatgcc ggtcgacatt 3420

gcgctggtat gcgcgacgca ccgcaatttg cgcagcctga tagcgcaggg ccagttccgc 3480

gaagacctct actaccgcct gaacgggctg gcgatctcgc tgccacccct gcgtcaacgt 3540

agcgaccttg ccgccctggt taaccatatc ctctttcagt gttgcggggg cgagccgcac 3600

tacagcgtga gcccagaagt gatgacgctc ttcaagcggc atgcgtggcc cggcaaccta 3660

cgccaactac ataacgttct cgatgcagcg cttgccatgc tcgacgacgg ccatgtcatt 3720

gagccccatc acctccccga agacttcgtc atggaggtcg attcgggcct ccgaccgatc 3780

gaggaagacg gttcgacggc ggcgcatcgc gcgcgacagc cggcgtcggg aagcggtcct 3840

gccaaaaagt tgcaagatct cgcgttggat gccatcgagc aggcgatcga gcaaaacgag 3900

ggaaatatat cggtcgccgc acggcagttg ggggtcagcc ggaccacgat ctaccgcaag 3960

ctgaggcaac tttcaccaac cggttgtcac cgaccggcac attggagcca gtcgcggatc 4020

ggcacataga ggccagcgcc ggatcggcgt aatggagcca cttcccctct gacggacgtc 4080

tcgaccttga ccggtccatc gctaccctgc tgcctttttg ttcggcagga ggtaggcggt 4140

ggcccgcagg agcatcgagc cccccaacga cgtccgtcag agcccggttc gagtggcttc 4200

tatatgccga tcatcggtgg ctctattgtg gcggtcagtg acaccggtcg ccttcacccc 4260

cacagatagt aggtgctgcg gctgctcatg ctcctgtcgc ggtagcgcgc tgttacgcga 4320

ccgcccccgg acctcggcgg acagcgcgga agattggaaa cagcccgagc gtgcgtgcct 4380

cgggctgcat ccttgccaca cccaaccgga ttcgtcggac cgctcgacat tcgcgttcgc 4440

tcccgcggcg ccgcgggtgt accgttgcgt tacagatgta cccttcttta acgtgtaaca 4500

cacgcctgga gcggccaaga gccccgcacc ttgcggcgcg tcttccccag gggcccaccg 4560

gttgcggcct tttgctgcga ccgtccatgc tggcacgaca cttgctgaaa gcgttagagc 4620

ggaatcggtc cgatggagca ttcgaagccg ctaccgacag cagaacacac aaaggaggaa 4680

gtgatgacaa aaaaaacctg gagcgatcgt tttgaaggca cattgcaccc cgccattgcc 4740

ctgtttaatg ccagcattgg ttttgacatt gaactaatcg aatatgacct ggatggctcg 4800

atcgcccatg gcaaaatgtt ggccaagacg ggcattatca gcccagggga agcagaacag 4860

ctagtgcagg gcttggagca aattcgccag gaataccgag ccggtaattt caatccaggg 4920

gtagaccagg aagacgttca ctttgctgtg gaacgccgat taacagaatt ggtgggggat 4980

gtgggcaaaa aactccacac tgcccgttcc cgcaatgacc aagtaggaac cgatgttcgt 5040

ctttacctgc gtgcccaaat tgatgacatc cgccaacggt taagggattt tcaagcagtg 5100

ttgctccaac tggcggaaac caatgtggaa accttaattc ccggctacac ccacctacaa 5160

cgggcccaac cggtcagttt ggctcaccat ctactggcct atttccagat ggcccaacgg 5220

gattggcaac ggttagggga aattcgagct agaactaacg tttctcccct cggttctggg 5280

gctttagcag gaacaacttt tcccatcgat cgccattaca gtgccgaatt gttggggttt 5340

gcaggggttt acgccaacag cttagatggg gtgagcgatc gggattttgc cattgaattt 5400

ctcaacgccg ccagtttgat catggtgcac ctcagtcggc tgagcgaaga aatgatcctc 5460

tgggcttccc aggaatttag ctttatcagt ttgaccgata gctgtgccac aggatcaagc 5520

attatgcccc aaaagaaaaa tccagacgta ccagagttaa ttcggggtaa agccgggcgg 5580

gtgatgggtc atctgcaagg aatgctagta ctgatgaaag gtttgcccct ggcctataac 5640

aaggatttgc aggaagacaa ggaagccctg tttgatgcag taaaaacggt gcaagtgagc 5700

ctggaagcca tgaccatttt gcttgatgaa ggtattgttt ttcgtcaaga acggttagcg 5760

gaggcggtgg cggaagattt ttccaatgcg acggatgtgg cggattactt ggcggctaag 5820

ggagtcccct tccgggaagc ttataatctg gtgggcaagg tggttaaaac cagtttggcg 5880

gcagggaaat tgctcaagga tttaaccctg gcggaatggc aagcgctcca tcccgctttt 5940

gaagaggata tttaccaagc tattaccccc cagcaagtgg tggcggcacg caatagttac 6000

gggggcacgg gctttgaaca ggtgaaaatg gcgatcgcca atgccaaagc tgaattaagc 6060

cagacttaat agtaatgagt tacagttttg gcaattacta aaaaactgac ttcaattcaa 6120

tgttagcccg ctcccgcggg ttttttgttg ctttttcaca gtgactatag gtaatcagca 6180

acacaatacg gccctgttct ttggacagtt tttgtataat gttgaccgca tcctgaccgg 6240

attttttatc taagtgggga attgtcaatt gtcaattaaa gctaagttct actaatgttt 6300

tagaaggcat tgtcgattga aaataagggt tgaatggaga aaattttgag cctttgtcaa 6360

agataaaaat ttatttcaac agttttttaa ctagccgaac cagagaatga cccagtggcg 6420

ctgactttgc tcccgagttt ttgttagaaa ttaccctcaa gaagtaatct aataataagg 6480

ttctctcttc tgccgtta 6498

<210> 215
<211> 7034
<212> DNA
<213> Artificial Sequence

<220>
<223> SEQ ID NO: 215 presents example 215 of a designer
      Thermosynechococcus elongatus transcription factor and nitrate
      reductase-based alcohol-selection DNA construct (7034 bp)

<400> 215
agaaaatctg gcaccacacc tctaccacat agtccgccaa gcgccgatcc cggttgagcg 60

ccagggccac atcccggacc agatcctcga cgaatttcgg attgtcatag gcccgctcgg 120

taacgaattt ctcgtccggc cgcttgagaa gcccgtacac ctcgcaggag gcgctctctt 180

cggcgacggc gatcaattcc tcgatcagca tgtcgccagc cagcctcacc tcgatgccga 240

tatgggagcg ctggttgtgg gcgccgtagt cggagattcc cttggagcag gggcacaggc 300

tcgtcgccgg caccaggacg cgcaaggaaa aggaatactc cccggcatcg gataccgagc 360

cctgccagca gacctcgtag tcgaggaggc tccgcacacc cgacaccggc gccgtcttgc 420

tcacgaaata gggaaagcgc atctcgatcg tgcccctcct cgcctccagc ctgtccaaca 480

ttgcgagggc cagggctttg aacgccgcct gatccagcgg gccggtctgc tcctccatca 540

gttcgacgaa gcgggacatg agggttccct tgacgtgcgc cgccaaaccg acggtgagcg 600

acagggtggc aacggtcgcc agccgctcgg cgcccgaccg gatggtggca gggtatctga 660

ggcccttgac cccgaccgca tgtatgggta ggtcacggcc atccagttgc gactggacgt 720

ccggcatcag agttgctctg tcaggggcat tcatctcggt ccttcctcgg gaatggggtc 780

gaacaaaacg gagaactggc cgcactagag taaagtagat aaaatccttt ctcaatggtt 840

ttatgtataa agccccagag ccaccgaccg tgtcgatgcg gagcgcgtca ggaggcgagt 900

ccaggacagt cggagagcgt gacatgcttg ccatattggg aacgcgccag cgggagttgc 960

tgaagctcct cctgaggagc aagggcggtt tgacggtggt cgagctctcg gcgcggctgg 1020

gcatctccag caacgcggtt cgccagcatc ttgccgccct ggagaacgag gggctggtcg 1080

cccacggcga gtcccgtcct tccggcgggc ggccggagca actctacgtg ctcactgcgg 1140

cgggcaagga gctttttccc cgtcagtatt cctggttcgc ccagttggtg gtcgagtcca 1200

tgaagcagga agccggcgcc gaggcgacgc gggaacgcct cgcggccatg ggatcgggca 1260

ttgcccgctt gttgctccgt cagcatggcg ggctcgaagg ccggcggcag cgcgtggcca 1320

agctcgccga actcatggag gcgctgggtt acgagatcgg cagcgtcgat gccgaaggca 1380

aagagccggt gattgaggcc aacaactgcg ttttccacaa gctcgccatg gacgacccgg 1440

atatctgcga gttcgacctg gccctgctgt ccacgtttac ggacagcaag gtcgaccatc 1500

aggagtgcat cgccaagggc ggcaatgtct gtcgtttccg gttctcgcga aaacgctaat 1560

acatccggta atgaacgtgc gctaccacac tttgatggct caccccacag acacgctgcg 1620

tggcagaccg tgcaccaggg cgtcgtgccg gcgcactgca actggattat gatatcgcca 1680

tcgaccgccg ccgacggcaa cgcccggtag acgaacgtgt acgccgccgg cgacggcatg 1740

aacgccaagc atcgtcctca atcaggtacg agatccggag aacctgagtg cggagcgacg 1800

ttctattttc gtattcagta acaatttgtc gagaatcggt ctgatccgcg gcttcacgca 1860

cctgcccgag gggcttgcgc ggaccttgag gtgaccttga gcgggcagat accaccaaaa 1920

tttcccacgt gctattatgg ttttgctaaa gctctcgaca gcgaggagag actcgcgaag 1980

ataagcaatt cgcccgacag aggtgaatga ggagacggta tgtccaagat gcaagagttc 2040

gcgcggctgg agacagtcgc gtcgatgcgc agagcggtct gggacggcaa cgagtgtcag 2100

ccggggaaag tggctgatgt cgttttgcgc tcgtggaccc ggtgtcgtgc tgaaggtgtc 2160

gttcccaatg cccgccagga gttcgacccg atcccgcgaa cggcgcttga cgaaacggtt 2220

gaggccaagc gggcgctgat ccttgctgcc gagccggtcg tcgacgcgtt gatggagcag 2280

atgaacgacg cccccaggat gatcatcctg aacgacgaac ggggcgtcgt gctgctgaac 2340

cagggaaacg acaccctcct tgaagacgcc cgccgccggg ccgtgcgggt gggcgtctgc 2400

tgggacgaac acgcccgagg caccaatgcc atgggaaccg cgctcgcgga gaggaggccc 2460

gtagcgatcc acggcgcaga gcactacctc gagtcgaata cgattttcac ctgcaccgcg 2520

gcgccgatct acgatccgtt cggcgagttc accggaattc tggatatcag cggatatgcg 2580

ggggacatgg gcccggttcc gattcccttt gttcagatgg cggtgcaatt catcgagaat 2640

cagttgttcc gccagacctt tgccgattgc attctgctgc actttcatgt gcgccccgac 2700

ttcgtcggaa cgatgcgcga agggatagcc gtgctgtcgc gcgagggaac catcgtctcg 2760

atgaaccgtg ctgggctcaa gatcgcaggg ctcaacctgg aggccgtcgc cgatcaccgt 2820

ttcgattccg tcttcgactt gaatttcggg gcctttctcg accacgtgcg gcagtccgcc 2880

ttcggtctcg tccgcgtctc gctctacggc ggcgttcagg tctacgcccg agtggaaccg 2940

ggcctgcgtg ttccgccacg tccggccgcc cacgcccgcc ctcctcggcc ggcaccgcgg 3000

cctctggatt cgctggacac gggcgacgca gcagtccgcc tcgcgattga ccgcgcccgc 3060

cgcgcgatcg gccgcaacct cagcatcctc atccagggcg agacgggtgc cggcaaggaa 3120

gtgttcgcca agcatctgca tgccgagagc ccgagaagca aggggccgtt cgttgccgtc 3180

aattgcgccg ccatacccga gggtttgatc gagtccgagc ttttcggata cgaagaaggg 3240

gccttcactg gcggacggcg caaaggcaac atcggcaagg tcgcccaggc ccacggcggc 3300

acgttgttcc tcgacgagat cggagacatg gcgccggggc tgcagaccag actgctgcgg 3360

gttctacagg accgggcagt gatgcccctc ggcggtcgcg aacctatgcc ggtcgacatt 3420

gcgctggtat gcgcgacgca ccgcaatttg cgcagcctga tagcgcaggg ccagttccgc 3480

gaagacctct actaccgcct gaacgggctg gcgatctcgc tgccacccct gcgtcaacgt 3540

agcgaccttg ccgccctggt taaccatatc ctctttcagt gttgcggggg cgagccgcac 3600

tacagcgtga gcccagaagt gatgacgctc ttcaagcggc atgcgtggcc cggcaaccta 3660

cgccaactac ataacgttct cgatgcagcg cttgccatgc tcgacgacgg ccatgtcatt 3720

gagccccatc acctccccga agacttcgtc atggaggtcg attcgggcct ccgaccgatc 3780

gaggaagacg gttcgacggc ggcgcatcgc gcgcgacagc cggcgtcggg aagcggtcct 3840

gccaaaaagt tgcaagatct cgcgttggat gccatcgagc aggcgatcga gcaaaacgag 3900

ggaaatatat cggtcgccgc acggcagttg ggggtcagcc ggaccacgat ctaccgcaag 3960

ctgaggcaac tttcaccaac cggttgtcac cgaccggcac attggagcca gtcgcggatc 4020

ggcacataga ggccagcgcc ggatcggcgt aatggagcca cttcccctct gacggacgtc 4080

tcgaccttga ccggtccatc gctaccctgc tgcctttttg ttcggcagga ggtaggcggt 4140

ggcccgcagg agcatcgagc cccccaacga cgtccgtcag agcccggttc gagtggcttc 4200

tatatgccga tcatcggtgg ctctattgtg gcggtcagtg acaccggtcg ccttcacccc 4260

cacagatagt aggtgctgcg gctgctcatg ctcctgtcgc ggtagcgcgc tgttacgcga 4320

ccgcccccgg acctcggcgg acagcgcgga agattggaaa cagcccgagc gtgcgtgcct 4380

cgggctgcat ccttgccaca cccaaccgga ttcgtcggac cgctcgacat tcgcgttcgc 4440

tcccgcggcg ccgcgggtgt accgttgcgt tacagatgta cccttcttta acgtgtaaca 4500

cacgcctgga gcggccaaga gccccgcacc ttgcggcgcg tcttccccag gggcccaccg 4560

gttgcggcct tttgctgcga ccgtccatgc tggcacgaca cttgctgaaa gcgttagagc 4620

ggaatcggtc cgatggagca ttcgaagccg ctaccgacag cagaacacac aaaggaggaa 4680

gtgatgggca caccacagat tagcgcattg gcggttcagt ctggtcctta cctccaccac 4740

aaccacaata cgagtgagcg gatgcagcca acagagactg tgtcgtccct gtgtccttac 4800

tgtggggtgg gctgtggcct agaggcagtg ccccaaggca agggaaccta taaaattcgt 4860

ggcgatcgcc aacatccctc gtcccaaggc atggtctgtg tcaaaggggc aacgattttg 4920

gaagccattg atcgcgatcg cttggcctat cccctttggc gagaacggtt agatcatccc 4980

tttacggtga tttcttggga tgaagctcta actcgtcttg tggaacgcat ccaatgggtg 5040

cgccatgagc agggggccga tgccatttgt atgtacggtt cggggcaatt tcacaccgag 5100

gactactacg tcgcacaaaa gctcatgaag gggtgcctcg gtacgaacaa ttttgatgct 5160

aattctcggt tgtgcatgtc ctcggcagtc tctgcctata tggctagctt cggtgctgac 5220

ggccccccct gctgctatga cgatttggag gctaccgact gtgcttttct cattggtacg 5280

aataccgctg agtgccatcc cattgtcttt aaccgcttac gcaatcacca caagcgggat 5340

cgccgtgtca agctaattgt cgtcgatcct cggcgcacac cgaccgcaga agtggcggat 5400

ctgcaccttg ccatccgccc cggcacggat attgatctgc tccacggcat tggctatctg 5460

ctcttgcagt ggggacacat tgatgtggac ttcattgaga agtgtactga gggctttgcc 5520

gattatgtaa agctgctggc ggcctatacc ccagaatggg tggcgcaacg gtgtggcatt 5580

cctgaaagtg atctagagca ggcggctcgc tactgggggc atgctcaagc agtactctct 5640

ctttggtcaa tgggcatcaa tcagtccgct gaaggtactg ccaaagctcg ctgtctcatt 5700

aatttgcatc tgctcacggg gcagattggc cgtccgggga gtggtccttt ttcccttaca 5760

ggacaaccca acgcgatggg aggtcgcgaa gccggggggt taagccatct gttgccgggc 5820

tatcgccaag tcaccaatcc ccagcaccgc caagaagtgg aggacttttg gggacttcct 5880

cgcggtcaaa tcagcgatcg ccccggtcgc acggcatggc aaattatcga gggcttagaa 5940

cagggagaag tgggtttgct gtggattgca gccaccaatc ccgcggttag ttttccccat 6000

ttagaacgta ccaaggcggc ctttctcaaa tcccccttta ccgtctatca ggatgcctac 6060

tttcccactg aaacagcagc ctatgcccat ctgattttgc ctgcagctca atggagcgag 6120

aaaacaggtg tgatgaccaa ctctgaacgc cgcgtcaccc ttgcccctgc ttttcgagtg 6180

ccccccggtc ttgcccgctc cgattgggaa atttttgctg aggtggggcg ccgcttggga 6240

tatgggcatc tcttctcctt tgccgatgcc gctgctgtct atgccgaatt tgtgcagctc 6300

actgccggtc gcccctgcga tcaatcggga ctgagccacg aacgtcttgc ccgtttaggt 6360

gccctgcagt ggccctgtcc tgccagcatg gatgacgaag aagcccgtaa gcccaaacgc 6420

ctctatactg actggcgatt tcacaccccc aatcaacggg catatttttg ccttgcccac 6480

agtcaagggg tggctgaacc caccgatcac atctataact ttaccctgac aacaggtcgt 6540

ctctacggcc actggcacac ccaaacccgt accggtcgca ttgccaaaat tcaaaaaatg 6600

caccccaatc cctttgtgga aatgcatccc gaggatgccg caaacttgca tctccaagat 6660

ggggattggg tggaggtgcg atcgcgccgc ggtcaagtgt gcctacctgt cacgctcacc 6720

aaggccattc gcccaggaac actctttgta cccatgcact ggggctttct ctttgccgat 6780

agcggtgagg tgaaccagct gacacatcct attgcctgtc cagtctcaaa gcagccagaa 6840

ctcaaggcct gtgctgtaca ggtgttgccc ctgaagctgc agcagacgtc ctagtaaggc 6900

tgagatcttc ttcagtgcat tgtagttgaa tgaagggtta ggggggaaat gcccccctat 6960

tttttgtcta gccatcctgc cacgtttgac agggtagcaa tttcgacacg atagggttct 7020

ctcttctgcc gtta 7034

<210> 216
<211> 7006
<212> DNA
<213> Artificial Sequence

<220>
<223> SEQ ID NO: 216 presents example 216 of a designer Synechococcus
 sp. strain PCC 7942 transcription factor and Tetracycline
 resistance-based alcohol-selection and Lipase DNA construct (7006
 base pairs (bp))

<400> 216
agaaaatctg gcaccacacc tctaccacat agtccgccaa gcgccgatcc cggttgagcg 60

ccagggccac atcccggacc agatcctcga cgaatttcgg attgtcatag gcccgctcgg 120

taacgaattt ctcgtccggc cgcttgagaa gcccgtacac ctcgcaggag gcgctctctt 180

cggcgacggc gatcaattcc tcgatcagca tgtcgccagc cagcctcacc tcgatgccga 240

tatgggagcg ctggttgtgg gcgccgtagt cggagattcc cttggagcag gggcacaggc 300

tcgtcgccgg caccaggacg cgcaaggaaa aggaatactc cccggcatcg gataccgagc 360

cctgccagca gacctcgtag tcgaggaggc tccgcacacc cgacaccggc gccgtcttgc 420

tcacgaaata gggaaagcgc atctcgatcg tgcccctcct cgcctccagc ctgtccaaca 480

ttgcgagggc cagggctttg aacgccgcct gatccagcgg gccggtctgc tcctccatca 540

gttcgacgaa gcgggacatg agggttccct tgacgtgcgc cgccaaaccg acggtgagcg 600

acagggtggc aacggtcgcc agccgctcgg cgcccgaccg gatggtggca gggtatctga 660

ggcccttgac cccgaccgca tgtatgggta ggtcacggcc atccagttgc gactggacgt 720

ccggcatcag agttgctctg tcaggggcat tcatctcggt ccttcctcgg gaatggggtc 780

gaacaaaacg gagaactggc cgcactagag taaagtagat aaaatccttt ctcaatggtt 840

ttatgtataa agccccagag ccaccgaccg tgtcgatgcg gagcgcgtca ggaggcgagt 900

ccaggacagt cggagagcgt gactacatcc ggtaatgaac gtgcgctacc acactttgat 960

ggctcacccc acagacacgc tgcgtggcag accgtgcacc agggcgtcgt gccggcgcac 1020

tgcaactgga ttatgatatc gccatcgacc gccgccgacg gcaacgcccg gtagacgaac 1080

gtgtacgccg ccggcgacgg catgaacgcc aagcatcgtc ctcaatcagg tacgagatcc 1140

ggagaacctg agtgcggagc gacgttctat tttcgtattc agtaacaatt tgtcgagaat 1200

cggtctgatc cgcggcttca cgcacctgcc cgagggcctt gcgcggacct tgaggtgacc 1260

ttgagcgggc agataccacc aaaatttccc acgtgctatt atggttttgc taaagctctc 1320

gacagcgagg agagactcgc gaagataagc aattcgcccg acagaggtga atgaggagac 1380

ggtatgtcca agatgcaaga gttcgcgcgg ctggagacag tcgcgtcgat gcgcagagcg 1440

gtctgggacg gcaacgagtg tcagccgggg aaagtggctg atgtcgtttt gcgctcgtgg 1500

acccggtgtc gtgctgaagg tgtcgttccc aatgcccgcc aggagttcga cccgatcccg 1560

cgaacggcgc ttgacgaaac ggttgaggcc aagcgggcgc tgatccttgc tgccgagccg 1620

gtcgtcgacg cgttgatgga gcagatgaac gacgccccca ggatgatcat cctgaacgac 1680

gaacggggcg tcgtgctgct gaaccaggga aacgacaccc tccttgaaga cgcccgccgc 1740

cgggccgtgc gggtgggcgt ctgctgggac gaacacgccc gaggcaccaa tgccatggga 1800

accgcgctcg cggagaggag gcccgtagcg atccacggcg cagagcacta cctcgagtcg 1860

aatacgattt tcacctgcac cgcggcgccg atctacgatc cgttcggcga gttcaccgga 1920

attctggata tcagcggata tgcgggggac atgggcccgg ttccgattcc ctttgttcag 1980

atggcggtgc aattcatcga gaatcagttg ttccgccaga cctttgccga ttgcattctg 2040

ctgcactttc atgtgcgccc cgacttcgtc ggaacgatgc gcgaagggat agccgtgctg 2100

tcgcgcgagg gaaccatcgt ctcgatgaac cgtgctgggc tcaagatcgc agggctcaac 2160

ctggaggccg tcgccgatca ccgtttcgat tccgtcttcg acttgaattt cggggccttt 2220

ctcgaccacg tgcggcagtc cgccttcggt ctcgtccgcg tctcgctcta cggcggcgtt 2280

caggtctacg cccgagtgga accgggcctg cgtgttccgc cacgtccggc cgcccacgcc 2340

cgccctcctc ggccggcacc gcggcctctg gattcgctgg acacgggcga cgcagcagtc 2400

cgcctcgcga ttgaccgcgc ccgccgcgcg atcggccgca acctcagcat cctcatccag 2460

ggcgagacgg gtgccggcaa ggaagtgttc gccaagcatc tgcatgccga gagcccgaga 2520

agcaaggggc cgttcgttgc cgtcaattgc gccgccatac ccgagggttt gatcgagtcc 2580

gagcttttcg gatacgaaga aggggccttc actggcggac ggcgcaaagg caacatcggc 2640

aaggtcgccc aggcccacgg cggcacgttg ttcctcgacg agatcggaga catggcgccg 2700

gggctgcaga ccagactgct gcgggttcta caggaccggg cagtgatgcc cctcggcggt 2760

cgcgaaccta tgccggtcga cattgcgctg gtatgcgcga cgcaccgcaa tttgcgcagc 2820

ctgatagcgc agggccagtt ccgcgaagac ctctactacc gcctgaacgg gctggcgatc 2880

tcgctgccac ccctgcgtca acgtagcgac cttgccgccc tggttaacca tatcctcttt 2940

cagtgttgcg gggcgagccg gcactacagc gtgagcccag aagtgatgac gctcttcaag 3000

cggcatgcgt ggcccggcaa cctacgccaa ctacataacg ttctcgatgc agcgcttgcc 3060

atgctcgacg acggccatgt cattgagccc catcacctcc ccgaagactt cgtcatggag 3120

gtcgattcgg gcctccgacc gatcgaggaa gacggttcga cggcggcgca tcgcgcgcga 3180

cagccggcgt cgggaagcgg tcctgccaaa aagttgcaag atctcgcgtt ggatgccatc 3240

gagcaggcga tcgagcaaaa cgagggaaat atatcggtcg ccgcacggca gttgggggtc 3300

agccggacca cgatctaccg caagctgagg caactttcac caaccggttg tcaccgaccg 3360

gcacattgga gccagtcgcg gatcggcaca tagaggccag cgccggatcg gcgtaatgga 3420

gccacttccc ctctgacgga cgtctcgacc ttgaccggtc catcgctacc ctgctgcctt 3480

tttgttcggc aggaggtagg cggtggcccg caggagcatc gagcccccca acgacgtccg 3540

tcagagcccg gttcgagtgg cttctatatg ccgatcatcg gtggctctat tgtggcggtc 3600

agtgacaccg gtcgccttca cccccacaga tagtaggtgc tgcggctgct catgctcctg 3660

tcgcggtagc gcgctgttac gcgaccgccc ccggacctcg gcggacagcg cggaagattg 3720

gaaacagccc gagcgtgcgt gcctcgggct gcatccttgc cacacccaac cggattcgtc 3780

ggaccgctcg acattcgcgt tcgctcccgc ggcgccgcgg gtgtaccgtt gcgttacaga 3840

tgtacccttc tttaacgtgt aacacacgcc tggagcggcc aagagccccg caccttgcgg 3900

cgcgtcttcc ccaggggccc accggttgcg gccttttgct gcgaccgtcc atgctggcac 3960

gacacttgct gaaagcgtta gagcggaatc ggtccgatgg agcattcgaa gccgctaccg 4020

acagcagaac acacaaagga ggaagtgatg aaacccaaca tacccctgat cgtaattctg 4080

agcactgtcg cgctcgacgc tgtcggcatc ggcctgatta tgccggtgct gccgggcctc 4140

ctgcgcgatc tggttcactc gaacgacgtc accgcccact atggcattct gctggcgctg 4200

tatgcgttgg tgcaatttgc ctgcgcacct gtgctgggcg cgctgtcgga tcgtttcggg 4260

cggcggccaa tcttgctcgt ctcgctggcc ggcgccactg tcgactacgc catcatggcg 4320

acagcgcctt tcctttgggt tctctatatc gggcggatcg tggccggcat caccggggcg 4380

actggggcgg tagccggcgc ttatattgcc gatatcactg atggcgatga gcgcgcgcgg 4440

cacttcggct tcatgagcgc ctgtttcggg ttcgggatgg tcgcgggacc tgtgctcggt 4500

gggctgatgg gcggtttctc cccccacgct ccgttcttcg ccgcggcagc cttgaacggc 4560

ctcaatttcc tgacgggctg tttccttttg ccggagtcgc acaaaggcga acgccggccg 4620

ttacgccggg aggctctcaa cccgctcgct tcgttccggt gggcccgggg catgaccgtc 4680

gtcgccgccc tgatggcggt cttcttcatc atgcaacttg tcggacaggt gccggccgcg 4740

ctttgggtca ttttcggcga ggatcgcttt cactgggacg cgaccacgat cggcatttcg 4800

cttgccgcat ttggcattct gcattcactc gcccaggcaa tgatcaccgg ccctgtagcc 4860

gcccggctcg gcgaaaggcg ggcactcatg ctcggaatga ttgccgacgg cacaggctac 4920

atcctgcttg ccttcgcgac acggggatgg atggcgttcc cgatcatggt cctgcttgct 4980

tcgggtggca tcggaatgcc ggcgctgcaa gcaatgttgt ccaggcaggt ggatgaggaa 5040

cgtcaggggc agctgcaagg ctcactggcg gcgctcacca gcctgacctc gatcgtcgga 5100

cccctcctct tcacggcgat ctatgcggct tctataacaa cgtggaacgg gtgggcatgg 5160

attgcaggcg ctgccctcta cttgctctgc ctgccggcgc tgcgtcgcgg gctttggagc 5220

ggcgcagggc aacgagccga tcgctgaatg ggtatctttg actataaaaa cctcggcacc 5280

gagggctcca aaacgctgtt cgccgatgcc atcgcgatca cgctgtattc ctaccacaac 5340

ctggataacg gctttgccgt gggttaccag cacaatggcc tggggcttgg cttgccggcc 5400

acgctggtgg gtgcgttgct gggcagcacg gattcacaag gtgtgattcc cggtattccc 5460

tggaaccccg actcggaaaa agctgcgctg gaagcggtgc aaaaagccgg ctggacgccc 5520

atcagcgcca gtgacctggg ctatggcggc aaggtcgatg ggcgcggcac tttctttggc 5580

gaaaaggccg gctacaccac ggcccaggtc gaggtgctgg gcaagtacga tgacgccggc 5640

aaactgctcg aaatcggcat cggctttcgt ggcacctcgg ggccacggga aagcttgatc 5700

accgactcca tcggcgacgt gatcagcgac ctgctggcgg cctttggccc caaggattat 5760

gccaagaact acgcgggcga agcctttggc ggcttgctca aaaatgtcgc cgactatgcc 5820

ggcgcccatg gcctgagcgg caaggacgtg gtggtcagcg gccacagcct gggcgggctg 5880

gcggtcaaca gcatggcgga tttaagcaac aacaaatggt cggggttcta caaggacgcc 5940

aactacgtgg cctatgcctc accgacccag agcgcgggcg ataaagtgtt gaacatcggc 6000

tacgaaaacg acccggtgtt ccgcgcgctt gacggctcat ccttcaactt gtcgtccctg 6060

ggcgtgcacg acaaacccca cgagtcgacc accgacaata tcgtcagctt caacgaccac 6120

tacgcctcga cgctgtggaa tatcctgccg ttttcgatcg tcaacctgcc gacctgggtc 6180

tcgcatttgc ccaccggcta tggcgacggc atgacgcgca tcctcgagtc cggcttctac 6240

gaccagatga cccgcgactc cacggtgatc gtcgccaacc tgtccgaccc ggcgcgggcc 6300

accacctggg tgcaagacct caaccgcaat gccgaacccc acaagagcaa caccttcatc 6360

atcggcagcc acggcaatga cctgatccag ggcggcaagg gcgcagactt tatcgaaggc 6420

ggcaaaggca atgacacgat ccgcgacaac agtgggcata acaccttcct gttcagcggt 6480

aattttggca atgatcgggt gatcggctac caaaccacgg acaagctggt gttccagaac 6540

gtggagggca gcaccgacct gcgcgaccat gccaaagtgg tcggtgccga cacggtgctc 6600

acttttggcg ccgactcggt gaccctggtc ggcgttgggc atggcggcct gtgggcggac 6660

ggggtcagca tcggctgata atagtgatcc cggccgctac taaagcctga tttgtcttga 6720

tagctgctcc tgcctttggg caggggcttt tttctgtctg ccattcttga ggatggcgga 6780

ctctttccct tttgctctac gcccatgaat gcgatcgcag tctcccctgt ccagcacgtt 6840

ggagtgattg gtggtggcca gttagcttgg atgctggcac cagcagcgca acagttgggg 6900

atgtcgctgc acgttcaaac acccaatgat cacgacccag cagtagcgat cgcggatcaa 6960

accgtattag cagcagttgc tgacgcggtt ctctcttctg ccgtta 7006

<210> 217
<211> 5556
<212> DNA
<213> Artificial Sequence

<220>
<223> SEQ ID NO:217 presents example 217 for a designer Prochlorococcus
 marinus MIT9313 transcription factor and glyphosate tolerant
 protein-based alcohol-selection DNA construct (5556 bp)

<400> 217
agaaaatctg gcaccacacc tctaccacat agtccgccaa gcgccgatcc cggttgagcg 60

ccagggccac atcccggacc agatcctcga cgaatttcgg attgtcatag gcccgctcgg 120

taacgaattt ctcgtccggc cgcttgagaa gcccgtacac ctcgcaggag gcgctctctt 180

cggcgacggc gatcaattcc tcgatcagca tgtcgccagc cagcctcacc tcgatgccga 240

tatgggagcg ctggttgtgg gcgccgtagt cggagattcc cttggagcag gggcacaggc 300

tcgtcgccgg caccaggacg cgcaaggaaa aggaatactc cccggcatcg gataccgagc 360

cctgccagca gacctcgtag tcgaggaggc tccgcacacc cgacaccggc gccgtcttgc 420

tcacgaaata gggaaagcgc atctcgatcg tgcccctcct cgcctccagc ctgtccaaca 480

ttgcgagggc cagggctttg aacgccgcct gatccagcgg gccggtctgc tcctccatca 540

gttcgacgaa gcgggacatg agggttccct tgacgtgcgc cgccaaaccg acggtgagcg 600

acagggtggc aacggtcgcc agccgctcgg cgcccgaccg gatggtggca gggtatctga 660

ggcccttgac cccgaccgca tgtatgggta ggtcacggcc atccagttgc gactggacgt 720

ccggcatcag agttgctctg tcaggggcat tcatctcggt ccttcctcgg gaatggggtc 780

gaacaaaacg gagaactggc cgcactagag taaagtagat aaaatccttt ctcaatggtt 840

ttatgtataa agccccagag ccaccgaccg tgtcgatgcg gagcgcgtca ggaggcgagt 900

ccaggacagt cggagagcgt gactacatcc ggtaatgaac gtgcgctacc acactttgat 960

ggctcacccc acagacacgc tgcgtggcag accgtgcacc agggcgtcgt gccggcgcac 1020

tgcaactgga ttatgatatc gccatcgacc gccgccgacg gcaacgcccg gtagacgaac 1080

gtgtacgccg ccggcgacgg catgaacgcc aagcatcgtc ctcaatcagg tacgagatcc 1140

ggagaacctg agtgcggagc gacgttctat tttcgtattc agtaacaatt tgtcgagaat 1200

cggtctgatc cgcggcttca cgcacctgcc cgaggggctt gcgcggacct tgaggtgacc 1260

ttgagcgggc agataccacc aaaatttccc acgtgctatt atggttttgc taaagctctc 1320

gacagcgagg agagactcgc gaagataagc aattcgcccg acagaggtga atgaggagac 1380

ggtatgtcca agatgcaaga gttcgcgcgg ctggagacag tcgcgtcgat gcgcagagcg 1440

gtctgggacg gcaacgagtg tcagccgggg aaagtggctg atgtcgtttt gcgctcgtgg 1500

acccggtgtc gtgctgaagg tgtcgttccc aatgcccgcc aggagttcga cccgatcccg 1560

cgaacggcgc ttgacgaaac ggttgaggcc aagcgggcgc tgatccttgc tgccgagccg 1620

gtcgtcgacg cgttgatgga gcagatgaac gacgccccca ggatgatcat cctgaacgac 1680

gaacggggcg tcgtgctgct gaaccaggga aacgacaccc tccttgaaga cgcccgccgc 1740

cgggccgtgc gggtgggcgt ctgctgggac gaacacgccc gaggcaccaa tgccatggga 1800

accgcgctcg cggagaggag gcccgtagcg atccacggcg cagagcacta cctcgagtcg 1860

aatacgattt tcacctgcac cgcggcgccg atctacgatc cgttcggcga gttcaccgga 1920

attctggata tcagcggata tgcgggggac atgggcccgg ttccgattcc ctttgttcag 1980

atggcggtgc aattcatcga gaatcagttg ttccgccaga cctttgccga ttgcattctg 2040

ctgcactttc atgtgcgccc cgacttcgtc ggaacgatgc gcgaagggat agccgtgctg 2100

tcgcgcgagg gaaccatcgt ctcgatgaac cgtgctgggc tcaagatcgc agggctcaac 2160

ctggaggccg tcgccgatca ccgtttcgat tccgtcttcg acttgaattt cggggccttt 2220

ctcgaccacg tgcggcagtc cgccttcggt ctcgtccgcg tctcgctcta cggcggcgtt 2280

caggtctacg cccgagtgga accgggcctg cgtgttccgc cacgtccggc cgcccacgcc 2340

cgccctcctc ggccggcacc gcggcctctg gattcgctgg acacgggcga cgcagcagtc 2400

cgcctcgcga ttgaccgcgc ccgccgcgcg atcggccgca acctcagcat cctcatccag 2460

ggcgagacgg gtgccggcaa ggaagtgttc gccaagcatc tgcatgccga gagcccgaga 2520

agcaaggggc cgttcgttgc cgtcaattgc gccgccatac ccgagggttt gatcgagtcc 2580

gagcttttcg gatacgaaga aggggccttc actggcggac ggcgcaaagg caacatcggc 2640

aaggtcgccc aggcccacgg cggcacgttg ttcctcgacg agatcggaga catggcgccg 2700

gggctgcaga ccagactgct gcgggttcta caggaccggg cagtgatgcc cctcggcggt 2760

cgcgaaccta tgccggtcga cattgcgctg gtatgcgcga cgcaccgcaa tttgcgcagc 2820

ctgatagcgc agggccagtt ccgcgaagac ctctactacc gcctgaacgg gctggcgatc 2880

tcgctgccac ccctgcgtca acgtagcgac cttgccgccc tggttaacca tatcctcttt 2940

cagtgttgcg gggcgagccc gcactacagc gtgagcccag aagtgatgac gctcttcaag 3000

cggcatgcgt ggcccggcaa cctacgccaa ctacataacg ttctcgatgc agcgcttgcc 3060

atgctcgacg acggccatgt cattgagccc catcacctcc ccgaagactt cgtcatggag 3120

gtcgattcgg gcctccgacc gatcgaggaa gacggttcga cggcggcgca tcgcgcgcga 3180

cagccggcgt cgggaagcgg tcctgccaaa aagttgcaag atctcgcgtt ggatgccatc 3240

gagcaggcga tcgagcaaaa cgagggaaat atatcggtcg ccgcacggca gttgggggtc 3300

agccggacca cgatctaccg caagctgagg caactttcac caaccggttg tcaccgaccg 3360

gcacattgga gccagtcgcg gatcggcaca tagaggccag cgccggatcg gcgtaatgga 3420

gccacttccc ctctgacgga cgtctcgacc ttgaccggtc catcgctacc ctgctgcctt 3480

tttgttcggc aggaggtagg cggtggcccg caggagcatc gagcccccca acgacgtccg 3540

tcagagcccg gttcgagtgg cttctatatg ccgatcatcg gtggctctat tgtggcggtc 3600

agtgacaccg gtcgccttca cccccacaga tagtaggtgc tgcggctgct catgctcctg 3660

tcgcggtagc gcgctgttac gcgaccgccc ccggacctcg gcggacagcg cggaagattg 3720

gaaacagccc gagcgtgcgt gcctcgggct gcatccttgc cacacccaac cggattcgtc 3780

ggaccgctcg acattcgcgt tcgctcccgc ggcgccgcgg gtgtaccgtt gcgttacaga 3840

tgtacccttc tttaacgtgt aacacacgcc tggagcggcc aagagccccg caccttgcgg 3900

cgcgtcttcc ccagggcccc accggttgcg gccttttgct gcgaccgtcc atgctggcac 3960

gacacttgct gaaagcgtta gagcggaatc ggtccgatgg agcattcgaa gccgctaccg 4020

acagcagaac acacaaagga ggaagtgatg cttcatggtg cttcctcaag gccagccact 4080

gcacgcaaat ccagtgggct cagcggaacc gttcgtatac ccggagataa gagcatatct 4140

cacaggtcct tcatgttcgg tggcttggca tctggtgaaa ccagaataac tggcctattg 4200

gagggtgagg acgtcataaa cacaggtaag gcgatgcaag ctatgggagc cagaattcgc 4260

aaagagggag atacgtggat cattgacggg gttggaaacg gtgggctact tgccccagag 4320

gcaccacttg acttcggaaa tgcagccacc ggttgtcgtc tgactatggg ccttgttggc 4380

gtttacgact tcgactccac attcatcgga gatgctagcc ttactaagcg tcctatggga 4440

cgcgttttga accctcttag ggagatgggt gttcaggtca agtcagaaga tggcgataga 4500

ctgccggtaa ctctcagagg tcccaaaaca ccaacaccca tcacatacag ggtgccaatg 4560

gctagtgccc aagtgaagag tgcagttttg ttggcaggac tgaacacacc ggggataacg 4620

acagtcatcg aacctatcat gactagggat cacaccgaaa agatgcttca gggatttggc 4680

gccaatttga ccgtggagac tgatgcagac ggtgtcagga ccattaggct tgaaggcaga 4740

ggtaagttga ctggtcaagt gatcgatgtg ccaggtgacc cttcttctac cgcctttcca 4800

ctcgtcgctg ctctcttggt acctggtagc gacgttacca tcctaaacgt tctaatgaac 4860

cctaccagaa ctggccttat tctgaccctt caggagatgg gtgctgatat tgaggtgatt 4920

aaccctagat tggctggagg ggaagatgtg gctgacctcc gtgttcgctc aagtactctt 4980

aaaggcgtca ctgttccgga agatagagcg cctagcatga ttgatgagta tcccatcctt 5040

gcagtagccg cagcatttgc tgaaggcgca accgtgatga acggccttga agaactcagg 5100

gttaaggagt ctgataggct tagcgctgtt gcaaacggtc ttaagctgaa tggcgtggat 5160

tgcgacgaag gagagacatc tctcgtcgta aggggaaggc ctgatggaaa gggacttggg 5220

aatgcctcag gagcagcagt ggctacacat ctcgatcaca ggattgccat gtcattcctc 5280

gttatgggct tggttagcga aaatccagtg actgtggatg atgctaccat gattgctacg 5340

tcctttccag agttcatgga cctcatggct gggttgggag cgaaaatcga gctcagcgac 5400

acaaaagcgg cctaatgatt tagtttcggt gtctatctct taatagcctc gatttatttt 5460

cggggctatt aatcaactct cagaggcgac aagcttcttc ttcccttacg acgtttttat 5520

tggttggaca tggcaaggtt ctctcttctg ccgtta 5556

<210> 218
<211> 6500
<212> DNA
<213> Artificial Sequence

<220>
<223> SEQ ID NO:218 presents example 218 for a designer Cyanothece sp.
ATCC 51142 transcription factor and nitrate reductase-based
alcohol-selection DNA construct (6500 bp)

<400> 218
agaaaatctg gcaccacacc tctaccacat agtccgccaa gcgccgatcc cggttgagcg 60

ccagggccac atcccggacc agatcctcga cgaatttcgg attgtcatag gcccgctcgg 120

taacgaattt ctcgtccggc cgcttgagaa gcccgtacac ctcgcaggag gcgctctctt 180

cggcgacggc gatcaattcc tcgatcagca tgtcgccagc cagcctcacc tcgatgccga 240

tatgggagcg ctggttgtgg gcgccgtagt cggagattcc cttggagcag gggcacaggc 300

tcgtcgccgg caccaggacg cgcaaggaaa aggaatactc cccggcatcg gataccgagc 360

cctgccagca gacctcgtag tcgaggaggc tccgcacacc cgacaccggc gccgtcttgc 420

tcacgaaata gggaaagcgc atctcgatcg tgcccctcct cgcctccagc ctgtccaaca 480

ttgcgagggc cagggctttg aacgccgcct gatccagcgg gccggtctgc tcctccatca 540

gttcgacgaa gcgggacatg agggttccct tgacgtgcgc cgccaaaccg acggtgagcg 600

acagggtggc aacggtcgcc agccgctcgg cgcccgaccg gatggtggca gggtatctga 660

ggcccttgac cccgaccgca tgtatgggta ggtcacggcc atccagttgc gactggacgt 720

ccggcatcag agttgctctg tcaggggcat tcatctcggt ccttcctcgg gaatggggtc 780

gaacaaaacg gagaactggc cgcactagag taaagtagat aaaatccttt ctcaatggtt 840

ttatgtataa agccccagag ccaccgaccg tgtcgatgcg gagcgcgtca ggaggcgagt 900

ccaggacagt cggagagcgt gactacatcc ggtaatgaac gtgcgctacc acactttgat 960

ggctcacccc acagacacgc tgcgtggcag accgtgcacc agggcgtcgt gccggcgcac 1020

tgcaactgga ttatgatatc gccatcgacc gccgccgacg gcaacgcccg gtagacgaac 1080

gtgtacgccg ccggcgacgg catgaacgcc aagcatcgtc ctcaatcagg tacgagatcc 1140

ggagaacctg agtgcggagc gacgttctat tttcgtattc agtaacaatt tgtcgagaat 1200

cggtctgatc cgcggcttca cgcacctgcc cgaggggctt gcgcggacct tgaggtgacc 1260

ttgagcgggc agataccacc aaaatttccc acgtgctatt atggttttgc taaagctctc 1320

gacagcgagg agagactcgc gaagataagc aattcgcccg acagaggtga atgaggagac 1380

ggtatgtcca agatgcaaga gttcgcgcgg ctggagacag tcgcgtcgat gcgcagagcg 1440

gtctgggacg gcaacgagtg tcagccgggg aaagtggctg atgtcgtttt gcgctcgtgg 1500

acccggtgtc gtgctgaagg tgtcgttccc aatgcccgcc aggagttcga cccgatcccg 1560

cgaacggcgc ttgacgaaac ggttgaggcc aagcgggcgc tgatccttgc tgccgagccg 1620

gtcgtcgacg cgttgatgga gcagatgaac gacgccccca ggatgatcat cctgaacgac 1680

gaacggggcg tcgtgctgct gaaccaggga aacgacaccc tccttgaaga cgcccgccgc 1740

cgggccgtgc gggtgggcgt ctgctgggac gaacacgccc gaggcaccaa tgccatggga 1800

accgcgctcg cggagaggag gcccgtagcg atccacggcg cagagcacta cctcgagtcg 1860

aatacgattt tcacctgcac cgcggcgccg atctacgatc cgttcggcga gttcaccgga 1920

attctggata tcagcggata tgcgggggac atgggcccgg ttccgattcc ctttgttcag 1980

atggcggtgc aattcatcga gaatcagttg ttccgccaga cctttgccga ttgcattctg 2040

ctgcactttc atgtgcgccc cgacttcgtc ggaacgatgc gcgaagggat agccgtgctg 2100

tcgcgcgagg gaaccatcgt ctcgatgaac cgtgctgggc tcaagatcgc agggctcaac 2160

ctggaggccg tcgccgatca ccgtttcgat tccgtcttcg acttgaattt cggggccttt 2220

ctcgaccacg tgcggcagtc cgccttcggt ctcgtccgcg tctcgctcta cggcggcgtt 2280

caggtctacg cccgagtgga accgggcctg cgtgttccgc cacgtccggc cgcccacgcc 2340

cgccctcctc ggccggcacc gcggcctctg gattcgctgg acacgggcga cgcagcagtc 2400

cgcctcgcga ttgaccgcgc ccgccgcgcg atcggccgca acctcagcat cctcatccag 2460

ggcgagacgg gtgccggcaa ggaagtgttc gccaagcatc tgcatgccga gagcccgaga 2520

agcaaggggc cgttcgttgc cgtcaattgc gccgccatac ccgagggttt gatcgagtcc 2580

gagcttttcg gatacgaaga aggggccttc actggcggac ggcgcaaagg caacatcggc 2640

aaggtcgccc aggcccacgg cggcacgttg ttcctcgacg agatcggaga catggcgccg 2700

gggctgcaga ccagactgct gcgggttcta caggaccggg cagtgatgcc cctcggcggt 2760

cgcgaaccta tgccggtcga cattgcgctg gtatgcgcga cgcaccgcaa tttgcgcagc 2820

ctgatagcgc agggccagtt ccgcgaagac ctctactacc gcctgaacgg gctggcgatc 2880

tcgctgccac ccctgcgtca acgtagcgac cttgccgccc tggttaacca tatcctcttt 2940

cagtgttgcg gggcgagccg gcactacagc gtgagcccag aagtgatgac gctcttcaag 3000

cggcatgcgt ggcccggcaa cctacgccaa ctacataacg ttctcgatgc agcgcttgcc 3060

atgctcgacg acggccatgt cattgagccc catcacctcc ccgaagactt cgtcatggag 3120

gtcgattcgg gcctccgacc gatcgaggaa gacggttcga cggcggcgca tcgcgcgcga 3180

cagccggcgt cgggaagcgg tcctgccaaa aagttgcaag atctcgcgtt ggatgccatc 3240

gagcaggcga tcgagcaaaa cgagggaaat atatcggtcg ccgcacggca gttgggggtc 3300

agccggacca cgatctaccg caagctgagg caactttcac caaccggttg tcaccgaccg 3360

gcacattgga gccagtcgcg gatcggcaca tagaggccag cgccggatcg gcgtaatgga 3420

gccacttccc ctctgacgga cgtctcgacc ttgaccggtc catcgctacc ctgctgcctt 3480

tttgttcggc aggaggtagg cggtggcccg caggagcatc gagcccccca acgacgtccg 3540

tcagagcccg gttcgagtgg cttctatatg ccgatcatcg gtggctctat tgtggcggtc 3600

agtgacaccg gtcgccttca cccccacaga tagtaggtgc tgcggctgct catgctcctg 3660

tcgcggtagc gcgctgttac gcgaccgccc ccggacctcg gcggacagcg cggaagattg 3720

gaaacagccc gagcgtgcgt gcctcgggct gcatccttgc cacacccaac cggattcgtc 3780

ggaccgctcg acattcgcgt tcgctcccgc ggcgccgcgg gtgtaccgtt gcgttacaga 3840

tgtacccttc tttaacgtgt aacacacgcc tggagcggcc aagagccccg caccttgcgg 3900

cgcgtcttcc ccaggggccc accggttgcg gccttttgct gcgaccgtcc atgctggcac 3960

gacacttgct gaaagcgtta gagcggaatc ggtccgatgg agcattcgaa gccgctaccg 4020

acagcagaac acacaaagga ggaagtgatg accaactcta ctaaaactct ctgtccttat 4080

tgtggtgtcg gttgcggttt agaagtctta cccccggccc aacctggaaa accggtaaac 4140

cgagatagtg aaggaacccc catctggcaa gtgagaggcg atcgcaacca tccttctagt 4200

ttagggaaag tctgtgttaa aggggcaacg gttagtgagt cgttagaaaa atctcgtctt 4260

aaatatccca tgatgcgaga cagtttaacc gatgagtttc gtcgagtgag ttgggatgaa 4320

gcgatggaag ccattgttaa ccgtatgcaa accctacgcc taacccaagg accagacgct 4380

ttatgtatgt atggttctgg acagtttcaa acagaagatt attatattgc tcaaaaacta 4440

tttaaagggt tcctgggaac caataacttt gacgcgaatt ctcgtctgtg tatgtcttct 4500

gcagtggcag gttataccca aagtttcggc agtgatgggc caccctgttg ttacgaagac 4560

ttagagttaa ccgactgtgc ctttttaata gggacgaata cagcagaatg tcatcccatc 4620

gtctttaacc gtctacgagt tcatcacaaa aagaaccgta aagttaaaat ggtggtagtt 4680

gaccccagac gaacccaaac cgcagaagca gcggacttac acttagctat taaaccagga 4740

accgatatcg atctctttaa cggtattgcc tatttactca tgcgttgggg tcgtatcgat 4800

acctttttca tcgatgaatg tacccaaaac ttttcagcct atgctgatgt tatccgtcat 4860

tatcccccag aattggtagc tcgtaactgt ggtattcgta tcgatgagtt agaaaaagcg 4920

gcccaatatt ggggagactc agaacgggtg ttatccttat ggtctatggg gatcaatcaa 4980

tcttctgaag gaacagccaa agttcgcacc ctgatcaact tacatttgat gacgggagat 5040

atcggtaaac ctggggccgg accattctcc ctcaccggac aacccaatgc catgggcggc 5100

cgagaagccg gaggactttg taatatctta cctgggtatc gagtcattaa taaccctcaa 5160

catcgggccg aagttgaaca gttttggaag cttcctcaag gaagtattca accgcaacgg 5220

ggccgtaccg cttgggaaat gattcaaggg ttagaaacgg gagaggtagg ctttttatgg 5280

atcgcagcca ctaaccctgc ggtgagtatg cccgatatta aacggactca agcagcctta 5340

aagcgatcgc ctttcaccgt ttatcaagac gcttattatc ctacagaaac ctcagcctat 5400

gcccatattt tactcccagc agcccaatgg agtgaaaaaa caggggtcat gaccaactca 5460

gaacgaatgg tgaccttatg tccccagttt cgtgatcccc caggggaagc aaggcccgac 5520

tgggaggtat ttgcagaagt gggacgacgg ttagggttta cagaggggtt taattttaaa 5580

acttccgccg aggtgtatgc agagtttgtg cagataactg aaggccgtcc ctgcgatagt 5640

tcgggtttaa gtcacgacaa gttacgcacc tatggccctc tacaatggcc ctatcccgat 5700

gcttggcctt ggttagaagg agaggactta gagcaacaaa accgcaccaa taagcgactt 5760

tataccgatt ttcgcttcaa taccccagat ggacgggcta ggttcgcagc ctaccattct 5820

aaagggctgg ctgaacccac tgatgaagct taccctttcg tgttaaccac cggaagatta 5880

tacggccatt ggcacaccca aacccgcacc ggacgcattc ctaaaattca aaagatgcac 5940

cccgatccct ttatggaaat tcatcctcaa gatgctgcaa aattgaacat tgaagagaat 6000

acgttcgttg aagtgcgatc gcgtcgagga aaggctcgtt ttctggcaaa aatcactaag 6060

gcgatcgctc ctgggactct gtttatcccc atgcactggg gcgcactttg ggctgaggat 6120

gccgaagcga acgctttaac tcatcctaat gcctgtccta cttctttgca accagagtta 6180

aaagcttgtg cggtacaggt aaaggttgct actgaagaag aatgggttat tacgagttca 6240

gagtccgaag ttcagagttc ggagttaaga gttatctaat aagctgtttt agagaaattt 6300

gttcggtaaa tattagccta cctacagttg ttgtgggtag gctaatatta tgaattgagt 6360

cctactgaac caatgattat cgttacgact aaaagtaata aatgtcatca gcaggatagg 6420

ggttgatagg aaaagttttt taatcggatg gttttcgagt tagaggttag ggtttcttta 6480

ggttctctct tctgccgtta 6500

<210> 219
<211> 7187
<212> DNA
<213> Artificial Sequence

<220>
<223> SEQ ID NO: 219 presents example 219 of a designer Anabaena PCC
 7120 transcription factor and ketol-acid
 reductoisomerase/dihydroxy-acid dehydratase-based
 alcohol-selection DNA construct (7187 bp)

<400> 219
agaaaatctg gcaccacacc tctaccacat agtccgccaa gcgccgatcc cggttgagcg 60

ccagggccac atcccggacc agatcctcga cgaatttcgg attgtcatag gcccgctcgg 120

taacgaattt ctcgtccggc cgcttgagaa gcccgtacac ctcgcaggag gcgctctctt 180

cggcgacggc gatcaattcc tcgatcagca tgtcgccagc cagcctcacc tcgatgccga 240

tatgggagcg ctggttgtgg gcgccgtagt cggagattcc cttggagcag gggcacaggc 300

tcgtcgccgg caccaggacg cgcaaggaaa aggaatactc cccggcatcg gataccgagc 360

cctgccagca gacctcgtag tcgaggaggc tccgcacacc cgacaccggc gccgtcttgc 420

tcacgaaata gggaaagcgc atctcgatcg tgcccctcct cgcctccagc ctgtccaaca 480

ttgcgagggc cagggctttg aacgccgcct gatccagcgg gccggtctgc tcctccatca 540

gttcgacgaa gcgggacatg agggttccct tgacgtgcgc cgccaaaccg acggtgagcg 600

acagggtggc aacggtcgcc agccgctcgg cgcccgaccg gatggtggca gggtatctga 660

ggcccttgac cccgaccgca tgtatgggta ggtcacggcc atccagttgc gactggacgt 720

ccggcatcag agttgctctg tcaggggcat tcatctcggt ccttcctcgg gaatggggtc 780

gaacaaaacg gagaactggc cgcactagag taaagtagat aaaatccttt ctcaatggtt 840

ttatgtataa agccccagag ccaccgaccg tgtcgatgcg gagcgcgtca ggaggcgagt 900

ccaggacagt cggagagcgt gactacatcc ggtaatgaac gtgcgctacc acactttgat 960

ggctcacccc acagacacgc tgcgtggcag accgtgcacc agggcgtcgt gccggcgcac 1020

tgcaactgga ttatgatatc gccatcgacc gccgccgacg gcaacgcccg gtagacgaac 1080

gtgtacgccg ccggcgacgg catgaacgcc aagcatcgtc ctcaatcagg tacgagatcc 1140

ggagaacctg agtgcggagc gacgttctat tttcgtattc agtaacaatt tgtcgagaat 1200

cggtctgatc cgcggcttca cgcacctgcc cgaggggctt gcgcggacct tgaggtgacc 1260

ttgagcgggc agataccacc aaaatttccc acgtgctatt atggttttgc taaagctctc 1320

gacagcgagg agagactcgc gaagataagc aattcgcccg acagaggtga atgaggagac 1380

ggtatgtcca agatgcaaga gttcgcgcgg ctggagacag tcgcgtcgat gcgcagagcg 1440

gtctgggacg gcaacgagtg tcagccgggg aaagtggctg atgtcgtttt gcgctcgtgg 1500

acccggtgtc gtgctgaagg tgtcgttccc aatgcccgcc aggagttcga cccgatcccg 1560

cgaacggcgc ttgacgaaac ggttgaggcc aagcgggcgc tgatccttgc tgccgagccg 1620

gtcgtcgacg cgttgatgga gcagatgaac gacgccccca ggatgatcat cctgaacgac 1680

gaacggggcg tcgtgctgct gaaccaggga aacgacaccc tccttgaaga cgcccgccgc 1740

cgggccgtgc gggtgggcgt ctgctgggac gaacacgccc gaggcaccaa tgccatggga 1800

accgcgctcg cggagaggag gcccgtagcg atccacggcg cagagcacta cctcgagtcg 1860

aatacgattt tcacctgcac cgcggcgccg atctacgatc cgttcggcga gttcaccgga 1920

attctggata tcagcggata tgcgggggac atgggcccgg ttccgattcc ctttgttcag 1980

atggcggtgc aattcatcga gaatcagttg ttccgccaga cctttgccga ttgcattctg 2040

ctgcactttc atgtgcgccc cgacttcgtc ggaacgatgc gcgaagggat agccgtgctg 2100

tcgcgcgagg gaaccatcgt ctcgatgaac cgtgctgggc tcaagatcgc agggctcaac 2160

ctggaggccg tcgccgatca ccgtttcgat tccgtcttcg acttgaattt cggggccttt 2220

ctcgaccacg tgcggcagtc cgccttcggt ctcgtccgcg tctcgctcta cggcggcgtt 2280

caggtctacg cccgagtgga accgggcctg cgtgttccgc cacgtccggc cgcccacgcc 2340

cgccctcctc ggccggcacc gcggcctctg gattcgctgg acacggcgac cgcagcagtc 2400

cgcctcgcga ttgaccgcgc ccgccgcgcg atcggccgca acctcagcat cctcatccag 2460

ggcgagacgg gtgccggcaa ggaagtgttc gccaagcatc tgcatgccga gagcccgaga 2520

agcaaggggc cgttcgttgc cgtcaattgc gccgccatac ccgagggttt gatcgagtcc 2580

gagcttttcg gatacgaaga aggggccttc actggcggac ggcgcaaagg caacatcggc 2640

aaggtcgccc aggcccacgg cggcacgttg ttcctcgacg agatcggaga catggcgccg 2700

gggctgcaga ccagactgct gcgggttcta caggaccggg cagtgatgcc cctcggcggt 2760

cgcgaaccta tgccggtcga cattgcgctg gtatgcgcga cgcaccgcaa tttgcgcagc 2820

ctgatagcgc agggccagtt ccgcgaagac ctctactacc gcctgaacgg gctggcgatc 2880

tcgctgccac ccctgcgtca acgtagcgac cttgccgccc tggttaacca tatcctcttt 2940

cagtgttgcg gggcgagccg gcactacagc gtgagcccag aagtgatgac gctcttcaag 3000

cggcatgcgt ggcccggcaa cctacgccaa ctacataacg ttctcgatgc agcgcttgcc 3060

atgctcgacg acggccatgt cattgagccc catcacctcc ccgaagactt cgtcatggag 3120

gtcgattcgg gcctccgacc gatcgaggaa gacggttcga cggcggcgca tcgcgcgcga 3180

cagccggcgt cgggaagcgg tcctgccaaa aagttgcaag atctcgcgtt ggatgccatc 3240

gagcaggcga tcgagcaaaa cgagggaaat atatcggtcg ccgcacggca gttgggggtc 3300

agccggacca cgatctaccg caagctgagg caactttcac caaccggttg tcaccgaccg 3360

gcacattgga gccagtcgcg gatcggcaca tagaggccag cgccggatcg gcgtaatgga 3420

gccacttccc ctctgacgga cgtctcgacc ttgaccggtc catcgctacc ctgctgcctt 3480

tttgttcggc aggaggtagg cggtggcccg caggagcatc gagcccccca acgacgtccg 3540

tcagagcccg gttcgagtgg cttctatatg ccgatcatcg gtggctctat tgtggcggtc 3600

agtgacaccg gtcgccttca cccccacaga tagtaggtgc tgcggctgct catgctcctg 3660

tcgcggtagc gcgctgttac gcgaccgccc ccggacctcg gcggacagcg cggaagattg 3720

gaaacagccc gagcgtgcgt gcctcgggct gcatccttgc cacacccaac cggattcgtc 3780

ggaccgctcg acattcgcgt tcgctcccgc ggcgccgcgg gtgtaccgtt gcgttacaga 3840

tgtacccttc tttaacgtgt aacacacgcc tggagcggcc aagagccccg caccttgcgg 3900

cgcgtcttcc ccagggcccc accggttgcg gccttttgct gcgaccgtcc atgctggcac 3960

gacacttgct gaaagcgtta gagcggaatc ggtccgatgg agcattcgaa gccgctaccg 4020

acagcagaac acacaaagga ggaagtgatg gcccggatgt actatgacga agatgccaat 4080

ttagaccttt tggctggaaa aaccattgca attatcggct atggttctca aggtcatgcc 4140

cacgctttaa atttaaaaga tagtggtttg aatgtgattg tggggctata tccgggtagt 4200

aagtcagcag aaaaagccca agcagctggg ttaacagtga aaaatgtggc ggatgctgct 4260

aacattgctg acttcatcat gattttgtta cccgatgaag tgcagaaaac agtttacaaa 4320

aacgaaatcg aaccaaatct gcaagaagga aacactttag cttttgctca cgggtttaat 4380

attcactttg ggcaagtcgt accacctgcg aacgtagacg tagttatggt agcacccaaa 4440

ggccctgggc atttggtacg acgcacttac gaacaaggtc aaggtgtacc agcgctgttt 4500

gccgtttatc aagatgctag tggaaaagca cgcgatcgcg ccctatctta tgctaaaggt 4560

atcggtggca ctcgtggcgg tgttttggaa acaactttcc gtgaagaaac tgaaaccgat 4620

ttattcggtg aacaagccgt attatgcggt ggtttgagtg ctttaattaa agctggtttt 4680

gaaaccttgg tagaagcagg ttatcaacca gaactagctt attttgaatg tctacacgaa 4740

gttaaattaa ttgttgactt ggtagtagaa ggcggtttag ccaaaatgcg cgacagcatt 4800

tctaacactg ctgaatatgg tgattacact cgcgggcctc ggattgtcaa cgaacaaacc 4860

aaagccgaaa tgcggaaggt tctcagcgaa attcaatcag gacaatttgc acgggaattt 4920

gtgttagaaa accaatctgg taaacccgga tttactgcaa tgcgtcgtca agaagctgaa 4980

cacccaattg aagaagttgg taaagattta cgggcaatgt ttagctggtt gaagaaagtt 5040

taaatgtcgg agaattttag aagcaaggct atcacacaag gggtacagcg atcgcctaac 5100

agagcaatgc tgcgggctgt tggttttcag gatgcagatt ttaccaaagc cattgtaggt 5160

gttgccaatg gttacagcac gattaccccg tgtaatatgg ggataaatca actagcacaa 5220

agggcagaag ctggtataaa tactgctgga gcaaagccgc aaatattcgg tacaattacg 5280

attagtgatg ggatttcgat gggaaccgaa gggatgaaat attccctggt atcacgagaa 5340

gtaatcgctg actccattga aaccgtttgt aatgggcaaa gtttagacgg ggtaattgcc 5400

attggtggct gtgataagaa tatgccaggg gcaatgattg cgatcgcccg gataaatatc 5460

cctgctatct ttgtttacgg tggcacaatt aaacccggac attacaacgg taaagattta 5520

actgttgtca gttcttttga ggctgttggt gagtacagcg ccggtaaaat tgacgaaaat 5580

gaactgttag cagtagaacg caatgcttgt cctggtgcag gttcctgcgg tgggatgtac 5640

acagcaaata ccatgtcctc tgcttttgaa gcactgggga tgagtttgcc ctattcgtct 5700

acaatggcgg cagaagatga cgaaaaagct gatagtacgg aagaatcagc caaggtattg 5760

gtagaggcga ttcgtcatca attattaccc agacagatta tcactcgtaa gtccatagaa 5820

aacgccatat ctgtaattat ggctgtgggt ggttctacta atgcggtgtt acattttcta 5880

gcgatcgccc gtgcggctgg tgtagaacta aatctggacg actttgaaac tattcgtggt 5940

cgtgtccccg ttttgtgcga cttgaaacca agcggtagat atgtggctac agacctgcac 6000

aaagctggtg gtatacccca agtcatgaaa atgttactcg tgcatggttt actccacggc 6060

gactgtatga ccatcacagg taaaaccatt gccgaagttt tagcagatat cccagaagaa 6120

ccatcgccta atcaagatgt aattcgtcct tggaataacc ccatgtatgc ccaaggccac 6180

ttggctatac tcaaaggtaa tttggcaaca gaaggcgcag ttgccaaaat tactggtgtg 6240

aaaaatcctg tgattacagg gccagccaaa gtatttgaat cagaagagga ttgtttagat 6300

gcaattttgg caggtaaaat taaagccggt gacgtgattg tcatccgcta cgaaggcccc 6360

aaaggcggcc ctgggatgcg agaaatgtta gcccccacct cagctattat cggtgcaggt 6420

ttaggcgact cagtgggact aattaccgat ggacgcttct ccggtggtac ttacggtatg 6480

gttgtgggac acgttgcacc agaagcagcc gttggtgggg cgatcgctct ggtacaagaa 6540

ggtgatagca tcacaattga tgcccacgcc cgttctttgc agttgaacat atcagacgaa 6600

gaattaaccc gtcgtcgtgc caattggcaa ccccgtcccc cacgttacac tacaggcata 6660

ctggcaaaat acgccaagtt ggtagcttct agtagtgtgg gtgctgtcac cgatttagac 6720

ttattcaata aatagtgaag taagtaggaa gcagggagca ggggaaagaa aattgacaac 6780

tgtacaagat taatcgcgtc tctgagcaat gaccaaatac atctacctcc acggtttctt 6840

tccagccccc tatctgcgaa agcacaagat attagcaagc gtttcgccca aattcacata 6900

cagctaacaa tccctgatct caatgctggt gaattttctc agttaacaat cacgcgccaa 6960

attcaacaag ttgccgcaat tttccctgat aattctgaac caataacgct gataggttct 7020

agtttaggcg gtttaactgc tgcttatcta ggacagcgat atttacaagt acaacgctta 7080

gttttattag cgccagtttg gttttttatc ccattggttg cccaaaatgg gtgaagaagc 7140

tgtcacaagt tggcaacaaa cgatataggt tctctcttct gccgtta 7187

<210> 220
<211> 5885
<212> DNA
<213> Artificial Sequence

<220>
<223> SEQ ID NO: 220 presents example 220 of a designer Anabaena PCC
 7120 transcription factor and argininosuccinate lyase-based
 alcohol-selection lipase DNA construct (5885 bp)

<400> 220
agaaaatctg gcaccacacc tctaccacat agtccgccaa gcgccgatcc cggttgagcg 60

ccagggccac atcccggacc agatcctcga cgaatttcgg attgtcatag gcccgctcgg 120

taacgaattt ctcgtccggc cgcttgagaa gcccgtacac ctcgcaggag gcgctctctt 180

cggcgacggc gatcaattcc tcgatcagca tgtcgccagc cagcctcacc tcgatgccga 240

tatgggagcg ctggttgtgg gcgccgtagt cggagattcc cttggagcag gggcacaggc 300

tcgtcgccgg caccaggacg cgcaaggaaa aggaatactc cccggcatcg gataccgagc 360

cctgccagca gacctcgtag tcgaggaggc tccgcacacc cgacaccggc gccgtcttgc 420

tcacgaaata gggaaagcgc atctcgatcg tgcccctcct cgcctccagc ctgtccaaca 480

ttgcgagggc cagggctttg aacgccgcct gatccagcgg gccggtctgc tcctccatca 540

gttcgacgaa gcgggacatg agggttccct tgacgtgcgc cgccaaaccg acggtgagcg 600

acagggtggc aacggtcgcc agccgctcgg cgcccgaccg gatggtggca gggtatctga 660

ggcccttgac cccgaccgca tgtatgggta ggtcacggcc atccagttgc gactggacgt 720

ccggcatcag agttgctctg tcaggggcat tcatctcggt ccttcctcgg gaatggggtc 780

gaacaaaacg gagaactggc cgcactagag taaagtagat aaaatccttt ctcaatggtt 840

ttatgtataa agccccagag ccaccgaccg tgtcgatgcg gagcgcgtca ggaggcgagt 900

ccaggacagt cggagagcgt gactacatcc ggtaatgaac gtgcgctacc acactttgat 960

ggctcacccc acagacacgc tgcgtggcag accgtgcacc agggcgtcgt gccggcgcac 1020

tgcaactgga ttatgatatc gccatcgacc gccgccgacg gcaacgcccg gtagacgaac 1080

gtgtacgccg ccggcgacgg catgaacgcc aagcatcgtc ctcaatcagg tacgagatcc 1140

ggagaacctg agtgcggagc gacgttctat tttcgtattc agtaacaatt tgtcgagaat 1200

cggtctgatc cgcggcttca cgcacctgcc cgaggggctt gcgcggacct tgaggtgacc 1260

ttgagcgggc agataccacc aaaatttccc acgtgctatt atggttttgc taaagctctc 1320

gacagcgagg agagactcgc gaagataagc aattcgcccg acagaggtga atgaggagac 1380

ggtatgtcca agatgcaaga gttcgcgcgg ctggagacag tcgcgtcgat gcgcagagcg 1440

gtctgggacg gcaacgagtg tcagccgggg aaagtggctg atgtcgtttt gcgctcgtgg 1500

acccggtgtc gtgctgaagg tgtcgttccc aatgcccgcc aggagttcga cccgatcccg 1560

cgaacggcgc ttgacgaaac ggttgaggcc aagcgggcgc tgatccttgc tgccgagccg 1620

gtcgtcgacg cgttgatgga gcagatgaac gacgccccca ggatgatcat cctgaacgac 1680

gaacggggcg tcgtgctgct gaaccaggga aacgacaccc tccttgaaga cgcccgccgc 1740

cgggccgtgc gggtgggcgt ctgctgggac gaacacgccc gaggcaccaa tgccatggga 1800

accgcgctcg cggagaggag gcccgtagcg atccacggcg cagagcacta cctcgagtcg 1860

aatacgattt tcacctgcac cgcggcgccg atctacgatc cgttcggcga gttcaccgga 1920

attctggata tcagcggata tgcgggggac atgggcccgg ttccgattcc ctttgttcag 1980

atggcggtgc aattcatcga gaatcagttg ttccgccaga cctttgccga ttgcattctg 2040

ctgcactttc atgtgcgccc cgacttcgtc ggaacgatgc gcgaagggat agccgtgctg 2100

tcgcgcgagg gaaccatcgt ctcgatgaac cgtgctgggc tcaagatcgc agggctcaac 2160

ctggaggccg tcgccgatca ccgtttcgat tccgtcttcg acttgaattt cggggccttt 2220

ctcgaccacg tgcggcagtc cgccttcggt ctcgtccgcg tctcgctcta cggcggcgtt 2280

caggtctacg cccgagtgga accgggcctg cgtgttccgc cacgtccggc cgcccacgcc 2340

cgccctcctc ggccggcacc gcggcctctg gattcgctgg acacggcgac cgcagcagtc 2400

cgcctcgcga ttgaccgcgc ccgccgcgcg atcggccgca acctcagcat cctcatccag 2460

ggcgagacgg gtgccggcaa ggaagtgttc gccaagcatc tgcatgccga gagcccgaga 2520

agcaaggggc cgttcgttgc cgtcaattgc gccgccatac ccgagggttt gatcgagtcc 2580

gagcttttcg gatacgaaga aggggccttc actggcggac ggcgcaaagg caacatcggc 2640

aaggtcgccc aggcccacgg cggcacgttg ttcctcgacg agatcggaga catggcgccg 2700

gggctgcaga ccagactgct gcgggttcta caggaccggg cagtgatgcc cctcggcggt 2760

cgcgaaccta tgccggtcga cattgcgctg gtatgcgcga cgcaccgcaa tttgcgcagc 2820

ctgatagcgc agggccagtt ccgcgaagac ctctactacc gcctgaacgg gctggcgatc 2880

tcgctgccac ccctgcgtca acgtagcgac cttgccgccc tggttaacca tatcctcttt 2940

cagtgttgcg ggggcgagcc gcactacagc gtgagcccag aagtgatgac gctcttcaag 3000

cggcatgcgt ggcccggcaa cctacgccaa ctacataacg ttctcgatgc agcgcttgcc 3060

atgctcgacg acggccatgt cattgagccc catcacctcc ccgaagactt cgtcatggag 3120

gtcgattcgg gcctccgacc gatcgaggaa gacggttcga cggcggcgca tcgcgcgcga 3180

cagccggcgt cgggaagcgg tcctgccaaa aagttgcaag atctcgcgtt ggatgccatc 3240

gagcaggcga tcgagcaaaa cgagggaaat atatcggtcg ccgcacggca gttgggggtc 3300

agccggacca cgatctaccg caagctgagg caactttcac caaccggttg tcaccgaccg 3360

gcacattgga gccagtcgcg gatcggcaca tagaggccag cgccggatcg gcgtaatgga 3420

gccacttccc ctctgacgga cgtctcgacc ttgaccggtc catcgctacc ctgctgcctt 3480

tttgttcggc aggaggtagg cggtggcccg caggagcatc gagcccccca acgacgtccg 3540

tcagagcccg gttcgagtgg cttctatatg ccgatcatcg gtggctctat tgtggcggtc 3600

agtgacaccg gtcgccttca cccccacaga tagtaggtgc tgcggctgct catgctcctg 3660

tcgcggtagc gcgctgttac gcgaccgccc ccggacctcg gcggacagcg cggaagattg 3720

gaaacagccc gagcgtgcgt gcctcgggct gcatccttgc cacacccaac cggattcgtc 3780

ggaccgctcg acattcgcgt tcgctcccgc ggcgccgcgg gtgtaccgtt gcgttacaga 3840

tgtacccttc tttaacgtgt aacacacgcc tggagcggcc aagagccccg caccttgcgg 3900

cgcgtcttcc ccagggcccc accggttgcg gccttttgct gcgaccgtcc atgctggcac 3960

gacacttgct gaaagcgtta gagcggaatc ggtccgatgg agcattcgaa gccgctaccg 4020

acagcagaac acacaaagga ggaagtgatg accgaaaaac aaacttggag tcagcgattt 4080

gaatctgctc tacatccagc gatcgctctt tttaatgcca gtataagttt tgacatagaa 4140

ttaatagaat acgaccttac aggctctcaa gctcatgccc agatgttggc gcatacgggg 4200

attatttcgc cggcagaagg agaacaacta gttgcaggtt tagaacaaat tcgtgaagaa 4260

tatcgccagg gcaaatttca acctggtatc gatgcggaag atgtccactt tgcagttgag 4320

cgacggttaa cagaaattgt tggagatgta ggtaagaaat tacatactgc gcgatcgcgt 4380

aatgatcaag tcggtacgga tacaagactt tacctacgtg accaaatttt acaaatcaaa 4440

acccaattac gggaatttca acgggtctta ttagatatag ctgaaaaaaa cgtggaaact 4500

ctaattcctg gttacactca cctacaacgc gcccagcctg tgagtttagc tcatcacttg 4560

ttggcttact tccaaatggc acaacgggac tgggaacgct taggggatgt ttatcgccgg 4620

gtgaatattt cgcctttggg gtgtggtgct ttagctggta cgacttttcc cattgaccgc 4680

cactacacag ccgaattgtt gcagtttgag cgtatttacg agaatagctt ggatggagtg 4740

agcgatcgcg attttgccat tgaatttttg tgtgcggcta gtctgattat ggttcacctc 4800

agccgtcttt ctgaagaaat tattctttgg gcatcggaag aatttcgctt cgtcatcctc 4860

aaagatagtt gtgcaacagg ttccagcatt atgccccaaa agaaaaaccc cgacgtaccg 4920

gaattagtca ggggaaaaac tgggcgtgta tttggtcatc tccaagcaat gttggtaatc 4980

atgaaaggat tacccttggc atataacaaa gacctgcaag aagacaaaga aggtttattt 5040

gacagcgtta acacagttaa ggcttgtctg gaagccatga caatcttgct acaggaaggc 5100

ttggaatttc ggactcagcg cctagctgaa gctgtcacac aagatttttc taacgctacc 5160

gatgtcgcag attatctagc agccaggggt gttcccttcc gcgaagctta taatatcgtg 5220

ggtaaagtcg ttaaaaccag tattgctgct ggcaaacttt taaaggattt aaccttagaa 5280

gaatggcaac aaatacaccc aaaatttgcc gacgatattt atgaagccat ctcccctcgt 5340

caagttgtag ctgcacgtaa cagttacggt ggtactggtt ttgcccaggt gagtaaagcc 5400

gtcagcgcgg cgcgtaagga aattggagaa tagtgaagta agtaggaagc agggagcagg 5460

ggaaagaaaa ttgacaactg tacaagatta atcgcgtctc tgagcaatga ccaaatacat 5520

ctacctccac ggttttcttc cagcccccta tctgcgaaag cacaagatat tagcaagcgt 5580

ttcgcccaaa ttcacataca gctaacaatc cctgatctca atgctggtga attttctcag 5640

ttaacaatca cgcgccaaat tcaacaagtt gccgcaattt tccctgataa ttctgaacca 5700

ataacgctga taggttctag tttaggcggt ttaactgctg cttatctagg acagcgatat 5760

ttacaagtac aacgcttagt tttattagcg ccagtttggt tttttatccc attggttgcc 5820

caaaatgggt gaagaagctg tcacaagttg gcaacaaacg atataggttc tctcttctgc 5880

cgtta 5885

<210> 221
<211> 1479
<212> DNA
<213> Artificial Sequence

<220>
<223> SEQ ID NO. 221 presents example 221 of a designer Chlamydomonas
      reinhardtii Nial-promoter-controlled cell surface ZYS1-like
      protein-polyamine DNA construct (1479 bp)

<400> 221
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat gctggaagcc agcagcgtcc cagcggagag cccgcagggt tctgacgcca 240

tggagactga tgacatgtgc tggatttgcc ttgatgacac caaggaccgt gacgtactag 300

tcagcccctg ccgttgcccg cgcaaggtgc acccgcgctg cctggcgcgg tggcagctgc 360

agcaggccgg caggctggag gagaccaatt gcaggttttg ccagcacaac ctggcggact 420

ggaaggccag cctgacgccc gagaacctca agcccgacgt gcagcgcgtg cagcccatca 480

tggtggtcta cttcgagggc cagatccacc gcatccccgt caagcagggg ccgggcggcc 540

tcaaggagtt cacgcagcgc attcgcgacc tgttccggct gcccgaggac gttgacatct 600

cgctgacgtt tggctgcaag gagccgcatg ttggtcattt tgccaacctg ggtcaatctc 660

cttctgaaag cctatgcttt tatggggatt tttggtcaac aaggcggtat caatgccttt 720

ctcggtttct ttgggattgc acccatgcaa attctcttta cggatttctc ctttattttc 780

gtagctagtt acattgaaat tccctttatg attctaccga ttttcaatgt actagatgat 840

ttggatgaaa atattgtgaa tgcaagtaaa gatttgggag ccaatgattg gcagaccttt 900

accaaggtta tttttccctt atcgcttaat ggggtacgct ctggcgtcca gtcggtcttt 960

attcctagtc tcagcctctt tatgctgaca cgtttgattg gtggtaaccg agttatcacc 1020

ttagggacag ctatcgaaca gcacttcctc acgacacaaa actggggaat gggggcaacc 1080

attggtgttg ttcttattgc tgctatgatt gcgacaatgt ggttaacaaa ggagagacgt 1140

caaaaaaaga aaaagaaaaa gaaaaagaaa aagaaaaaga aaaagaaaaa gaaaaagaaa 1200

aagaaaaaga aaaagaaaaa gaaaaagaaa aagtgataaa tggaggcgct cgttgatctg 1260

agccttgccc cctgacgaac ggcggtggat ggaagatact gctctcaagt gctgaagcgg 1320

tagcttagct ccccgtttcg tgctgatcag tctttttcaa cacgtaaaaa gcggaggagt 1380

tttgcaattt tgttggttgt aacgatcctc cgttgatttt ggcctctttc tccatgggcg 1440

ggctgggcgt atttgaagcg gttctctctt ctgccgtta 1479

<210> 222
<211> 2717
<212> DNA
<213> Artificial Sequence

<220>
<223> SEQ ID NO. 222 presents example 222 of a designer Chlamydomonas
 reinhardtii Nial-promoter-controlled cell surface ZYS1-like
 protein-polyamine-linked Lipase-polylysine DNA construct (2717
 bp)

<400> 222
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat gggccataca caccgtgcct ccgcgtcggc ggcggctgca gtgctgctgg 240

cgctgctgct agcagggtgc tccgttggga caaccgcagc tgccgggcgt cgcgcgctcc 300

tgcagggccc cgttgctgac gtgcctgctg ccaacctcac gtccgaggtt cctgctccta 360

acgcgactgt tgctattgac gcctacaatg tcacgggcgc tgctcagaag gtcgccgcca 420

acaagaccgc cgccatctcc accatcttcg cccccacgga cgcggccttt gcggaacttg 480

ctgcttctct gggcctggcc tcgccagtgg acttgttcaa cgccacgctg aacgcaacgg 540

ccgccaacat caccgccctg cacgtcatcc cgggagtggc cctgaccgcc gccaacatca 600

cggccgctgg cagcatcacc gccacctccc tgctgggata caatatcact gtgacagcgc 660

tgccgaacgg cacgatcacc gccacaattc cgggcagcaa catcgttgcc gacgtcatca 720

cccctgatgt gcccttcaac gggagcattg tgcatgtgat caacaaggtc ctgctgccgc 780

cgctgaacga gactgtgggc tgggctgtgc ccattgccaa cgccactgcc gcgggcgtaa 840

tgttggtcat tttgccaacc tgggtcaatc tccttctgaa agcctatgct tttatgggga 900

tttttggtca acaaggcggt atcaatgcct ttctcggttt ctttgggatt gcacccatgc 960

aaattctctt tacggatttc tcctttattt tcgtagctag ttacattgaa attcccttta 1020

tgattctacc gattttcaat gtactagatg atttggatga aaatattgtg aatgcaagta 1080

aagatttggg agccaatgat tggcagacct ttaccaaggt tatttttccc ttatcgctta 1140

atggggtacg ctctggcgtc cagtcggtct ttattcctag tctcagcctc tttatgctga 1200

cacgtttgat tggtggtaac cgagttatca ccttagggac agctatcgaa cagcacttcc 1260

tcacgacaca aaactgggga atgggggcaa ccattggtgt tgttcttatt gctgctatga 1320

ttgcgacaat gtggttaaca aaggagagac gtcaaatgaa gctactctct ctgaccggtg 1380

tggctggtgt gcttgcgact tgcgttgcag ccactccttt ggtgaagcgt ctaccttccg 1440

gttcggaccc tgccttttcg cagcccaagt cggtgctcga tgcgggtctg acctgccagg 1500

gtgcttcgcc atcctcggtc tccaaaccca tccttctcgt ccccggaacc ggcaccacag 1560

gtccacagtc gttcgactcg aactggatcc ccctctcaac gcagttgggt tacacaccct 1620

gctggatctc acccccgccg ttcatgctca acgacaccca ggtcaacacg gagtacatgg 1680

tcaacgccat caccgcgctc tacgctggtt cgggcaacaa caagcttccc gtgcttacct 1740

ggtcccaggg tggtctggtt gcacagtggg gtctgacctt cttccccagt atcaggtcca 1800

aggtcgatcg acttatggcc tttgcgcccg actacaaggg caccgtcctc gccggccctc 1860

tcgatgcact cgcggttagt gcaccctccg tatggcagca aaccaccggt tcggcactca 1920

ccaccgcact ccgaaacgca ggtggtctga cccagatcgt gcccaccacc aacctctact 1980

cggcgaccga cgagatcgtt cagcctcagg tgtccaactc gccactcgac tcatcctacc 2040

tcttcaacgg aaagaacgtc caggcacagg ccgtgtgtgg gccgctgttc gtcatcgacc 2100

atgcaggctc gctcacctcg cagttctcct acgtcgtcgg tcgatccgcc ctgcgctcca 2160

ccacgggcca ggctcgtagt gcagactatg gcattacgga ctgcaaccct cttcccgcca 2220

atgatctgac tcccgagcaa aaggtcgccg cggctgcgct cctggcgccg gcagctgcag 2280

ccatcgtggc gggtccaaag cagaactgcg agcccgacct catgccctac gcccgcccct 2340

ttgcagtagg caaaaggacc tgctccggca tcgtcacccc caaaaagaaa aagaaaaaga 2400

aaaagaaaaa gaaaaagaaa aagaaaaaga aaaagaaaaa gaaaaagaaa aagaaaaaga 2460

aaaagaaaaa gtgataaatg gaggcgctcg ttgatctgag ccttgccccc tgacgaacgg 2520

cggtggatgg aagatactgc tctcaagtgc tgaagcggta gcttagctcc ccgtttcgtg 2580

ctgatcagtc tttttcaaca cgtaaaaagc ggaggagttt tgcaattttg ttggttgtaa 2640

cgatcctccg ttgattttgg cctctttctc catgggcggg ctgggcgtat ttgaagcggt 2700

tctctcttct gccgtta 2717

<210> 223
<211> 3617
<212> DNA
<213> Artificial Sequence

<220>
<223> SEQ ID NO. 223 presents example 223 of a designer Chlamydomonas
 reinhardtii Nial-promoter-controlled cell surface ZYS1-like
 protein-polyamine DNA construct (3617 bp)

<400> 223
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat gggccataca caccgtgcct ccgcgtcggc ggcggctgca gtgctgctgg 240

cgctgctgct agcagggtgc tccgttggga caaccgcagc tgccgggcgt cgcgcgctcc 300

tgcagggccc cgttgctgac gtgcctgctg ccaacctcac gtccgaggtt cctgctccta 360

acgcgactgt tgctattgac gcctacaatg tcacgggcgc tgctcagaag gtcgccgcca 420

acaagaccgc cgccatctcc accatcttcg cccccacgga cgcggccttt gcggaacttg 480

ctgcttctct gggcctggcc tcgccagtgg acttgttcaa cgccacgctg aacgcaacgg 540

ccgccaacat caccgccctg cacgtcatcc cgggagtggc cctgaccgcc gccaacatca 600

cggccgctgg cagcatcacc gccacctccc tgctgggata caatatcact gtgacagcgc 660

tgccgaacgg cacgatcacc gccacaattc cgggcagcaa catcgttgcc gacgtcatca 720

cccctgatgt gcccttcaac gggagcattg tgcatgtgat caacaaggtc ctgctgccgc 780

cgctgaacga gactgtgggc tgggctgtgc ccattgccaa cgccactgcc gcgggcgtaa 840

tgttggtcat tttgccaacc tgggtcaatc tccttctgaa agcctatgct tttatgggga 900

tttttggtca acaaggcggt atcaatgcct ttctcggttt ctttgggatt gcacccatgc 960

aaattctctt tacggatttc tcctttattt tcgtagctag ttacattgaa attcccttta 1020

tgattctacc gattttcaat gtactagatg atttggatga aaatattgtg aatgcaagta 1080

aagatttggg agccaatgat tggcagacct ttaccaaggt tatttttccc ttatcgctta 1140

atggggtacg ctctggcgtc cagtcggtct ttattcctag tctcagcctc tttatgctga 1200

cacgtttgat tggtggtaac cgagttatca ccttagggac agctatcgaa cagcacttcc 1260

tcacgacaca aaactgggga atgggggcaa ccattggtgt tgttcttatt gctgctatga 1320

ttgcgacaat gtggttaaca aaggagagac gtcaaatggc caggacgatg cgttccaggg 1380

tggtggcagg ggcagtggca tgcgcgatga gcatcgcgcc gttcgcgggg acgaccgcgg 1440

tgatgacgct cgcgacgacg cacgcggcaa tggcggccac cgcgcccgcc gctggctacg 1500

cggcgacgcg ttacccgatc atcctcgtgc acgggctctc gggtaccgac aagtacgccg 1560

gcgtgctcga gtattggtac ggcatccagg aggacctgca acagaacggt gcgaccgtct 1620

acgtcgcgaa cctgtcgggt ttccagagcg acgacggccc gaacgggcgc ggcgaacagt 1680

tgctcgctta cgtgaagacg gtgctcgcgg cgacgggggc gaccaaggtc aatctcgtcg 1740

gtcacagcca gggcggcctc tcgtcgcgct atgttgctgc cgtcgcgccc gatctcgttg 1800

cgtcggtgac gacgatcggc acgccgcatc gcggctccga attcgccgac ttcgtgcagg 1860

acgtgctcgc gtacgatccg accgggcttt cgtcatcggt gatcgccgcg ttcgtcaatg 1920

tgttcgggat cctgacgagc agcagccaca acaccaacca ggacgcgctc gccgcactgc 1980

agacgctgac caccgcacgg gccgccacgt acaaccagaa ctatccgagc gcgggcctgg 2040

gtgcgccggg cagttgccag accggtgcgc cgaccgaaac cgtcggcggc aacacgcacc 2100

tgctgtattc gtgggccggc acggcgatcc agccgacgct ctccgtgttc ggcgtcacgg 2160

gcgcgacgga cacgagcacc cttccgctcg tcgatccggc gaacgtgctc gacctgtcga 2220

cgctcgcgct gttcggcacc ggcacggtga tgatcaaccg cggctccggg cagaacgacg 2280

ggctcgtgtc gaagtgcagt gcgctgtacg gcaaggtgct gagcacgagc tacaagtgga 2340

accacctcga cgagatcaac cagctgctcg gcgtgcgcgg cgcgtatgcg gaagatcccg 2400

tcgcggtgat ccgcacgcat gcgaaccggc tgaagctggc gggcgtgatg aattcaggaa 2460

ttaaagcatc tttaggtctt aacagaagat taatattcct gcttccttac atttttatag 2520

ccattctttt agtgttttta ccaattattt taataattgt gaactcagtt attcatatag 2580

ctgatccaac ttttgattcg tttaaattgg ttaaggattc aaaaacctga accaaaattt 2640

gaagaagctt atttattgga atcatatctg ctttattgtg tctagttata ggattgccgt 2700

atgcatattt tgtagctaga agcaaaaaca aaattatgcc aatttatgct atgagtctta 2760

ttttaagtcc attagtaata tttactattg caaaaattta tgcagtacgc ggctttttct 2820

taactattgt ttcgaaagaa aacgatctta atgcaatttg atttatgatt tttggcttaa 2880

cctacttaaa cttgccatat atgataatgc cactttattc agtctttaga gatatgcccg 2940

aaaatattgt tgaagcttct catgacttag gtaacggacc aattagaaca ctatttagag 3000

taattttgcc atacagcagt aaagcaatat taagtggcct aggtattatt ttcttatcta 3060

gtgctaccaa ctttgttata agtgataaat tacttccaga tggctcgcaa caccaattaa 3120

ttggtacagt aattaataat tacaccaacc ctgcaaacca atatgaagta gcaattggca 3180

ctacattagt tattgtcgta tcagtcatat ttataggtac attcgcattg ataagcttct 3240

tgccaagagt attcaaaaga agaaagagag caagatatga gaaaaagaaa aagaaaaaga 3300

aaaagaaaaa gaaaaagaaa aagaaaaaga aaaagaaaaa gaaaaagaaa aagaaaaaga 3360

aaaagaaaaa gtaataaatg gaggcgctcg ttgatctgag ccttgccccc tgacgaacgg 3420

cggtggatgg aagatactgc tctcaagtgc tgaagcggta gcttagctcc ccgtttcgtg 3480

ctgatcagtc tttttcaaca cgtaaaaagc ggaggagttt tgcaattttg ttggttgtaa 3540

cgatcctccg ttgattttgg cctctttctc catgggcggg ctgggcgtat ttgaagcggt 3600

tctctcttct gccgtta 3617

<210> 224
<211> 2687
<212> DNA
<213> Artificial Sequence

<220>
<223> SEQ ID NO. 224 presents example 224 of a designer Chlamydomonas
      reinhardtii Nial-promoter-controlled cell surface ZYS1-like
      protein-polyamine DNA construct (2687 bp)

<400> 224
agaaaatctg gcaccacacc atggtagggt gcgagtgacc ccgcgcgact tggaagggtt 60

caaacgaccc cgccgtacga acttttgtcg gggggcgctc ccggatggta gggtgcgagt 120

gaccccgcgc gacttggaag ggttcaaacg accccgccgt acgaactttt gtcggggggc 180

gctcccggat gggccataca caccgtgcct ccgcgtcggc ggcggctgca gtgctgctgg 240

cgctgctgct agcagggtgc tccgttggga caaccgcagc tgccgggcgt cgcgcgctcc 300

tgcagggccc cgttgctgac gtgcctgctg ccaacctcac gtccgaggtt cctgctccta 360

acgcgactgt tgctattgac gcctacaatg tcacgggcgc tgctcagaag gtcgccgcca 420

acaagaccgc cgccatctcc accatcttcg cccccacgga cgcggccttt gcggaacttg 480

ctgcttctct gggcctggcc tcgccagtgg acttgttcaa cgccacgctg aacgcaacgg 540

ccgccaacat caccgccctg cacgtcatcc cgggagtggc cctgaccgcc gccaacatca 600

cggccgctgg cagcatcacc gccacctccc tgctgggata caatatcact gtgacagcgc 660

tgccgaacgg cacgatcacc gccacaattc cgggcagcaa catcgttgcc gacgtcatca 720

cccctgatgt gcccttcaac gggagcattg tgcatgtgat caacaaggtc ctgctgccgc 780

cgctgaacga gactgtgggc tgggctgtgc ccattgccaa cgccactgcc gcgggcgtaa 840

tggccaggac gatgcgttcc agggtggtgg caggggcagt ggcatgcgcg atgagcatcg 900

cgccgttcgc ggggacgacc gcggtgatga cgctcgcgac gacgcacgcg gcaatggcgg 960

ccaccgcgcc cgccgctggc tacgcggcga cgcgttaccc gatcatcctc gtgcacgggc 1020

tctcgggtac cgacaagtac gccggcgtgc tcgagtattg gtacggcatc caggaggacc 1080

tgcaacagaa cggtgcgacc gtctacgtcg cgaacctgtc gggtttccag agcgacgacg 1140

gcccgaacgg gcgcggcgaa cagttgctcg cttacgtgaa gacggtgctc gcggcgacgg 1200

gggcgaccaa ggtcaatctc gtcggtcaca gccagggcgg cctctcgtcg cgctatgttg 1260

ctgccgtcgc gcccgatctc gttgcgtcgg tgacgacgat cggcacgccg catcgcggct 1320

ccgaattcgc cgacttcgtg caggacgtgc tcgcgtacga tccgaccggg ctttcgtcat 1380

cggtgatcgc cgcgttcgtc aatgtgttcg ggatcctgac gagcagcagc cacaacacca 1440

accaggacgc gctcgccgca ctgcagacgc tgaccaccgc acgggccgcc acgtacaacc 1500

agaactatcc gagcgcgggc ctgggtgcgc cgggcagttg ccagaccggt gcgccgaccg 1560

aaaccgtcgg cggcaacacg cacctgctgt attcgtgggc cggcacggcg atccagccga 1620

cgctctccgt gttcggcgtc acgggcgcga cggacacgag cacccttccg ctcgtcgatc 1680

cggcgaacgt gctcgacctg tcgacgctcg cgctgttcgg caccggcacg gtgatgatca 1740

accgcggctc cgggcagaac gacgggctcg tgtcgaagtg cagtgcgctg tacggcaagg 1800

tgctgagcac gagctacaag tggaaccacc tcgacgagat caaccagctg ctcggcgtgc 1860

gcggcgcgta tgcggaagat cccgtcgcgg tgatccgcac gcatgcgaac cggctgaagc 1920

tggcgggcgt gatgaatgct gtatatttct tgcttgtctt atccgccggg ttgttgctaa 1980

cccaagcaga tgatgcaaat gaggaaggtg ctgaagatgg tacacagggt ggagaggaga 2040

agaaggagaa ggagaagaag aagaagaaga agaagaagga gaagaaaaag aagaagaaga 2100

agaagaagaa gaagaagaag aagaagaaga aggtggcgaa gaagaaggag gcggcaaaga 2160

agaagaagtc gaaagagaga aagataaagg cgaaagagag aaaggtaaag gcgaaaaaac 2220

taaaggcgaa aaaggtagag gcgaaaaagg ccaacgcgaa aaaggcaaag aagaaggcga 2280

agaagaggta cctggaaaaa agaaaaagcc tggaaagggt aaaaaaggtg aagaagacaa 2340

atatcgtaga aaaaaagaaa aagaaaaaga aaaagaaaaa gaaaaagaaa aagaaaaaga 2400

aaaagaaaaa gaaaaagaaa aagaaaaaga aaaagaaaaa gtagtaaatg gaggcgctcg 2460

ttgatctgag ccttgccccc tgacgaacgg cggtggatgg aagatactgc tctcaagtgc 2520

tgaagcggta gcttagctcc ccgtttcgtg ctgatcagtc tttttcaaca cgtaaaaagc 2580

ggaggagttt tgcaattttg ttggttgtaa cgatcctccg ttgattttgg cctctttctc 2640

catgggcggg ctgggcgtat ttgaagcggt tctctcttct gccgtta 2687

<210> 225
<211> 3205
<212> DNA
<213> Artificial Sequence

<220>
<223> SEQ ID NO: 225 presents example 225 of a designer Synechocystis
 sp. strain PCC 6803 nirA-promoter-controlled Lipase DNA construct
 (3205 bp)

<400> 225
agaaaatctg gcaccacacc ctaaatgcgt aaactgcata tgccttggct gagtgtaatt 60

tacgttacaa attttaacga aacgggaacc ctatattgat ctctacatga tgcctaacat 120

tgttgaaatt gcggtgtctg acgagcgatt ctccaccttg gtgaccgctg tcactgcggc 180

caatttggtt gacgtgttgc aaagccctgg gccctttacg gttttcgctc ccacggatac 240

ggcctttgcc aaactccccc ccggtaccat taccaccctg gtacaaaata ttccccaatt 300

ggctcggatt ttaacctacc acgtggttgc cgggaagttt acccaagctg atttatgtcg 360

cctatcaact gttgattcag tagagggctc cccgatcgcc atcgactgta cggaggggtt 420

tgaagtgaaa aatgccacgg taatcatccc cgatatcgaa gcggacaacg gcattatcca 480

tgtgattgac aacgtgattt taatgggaat gagtaaagga gaagaacttt tcactggagt 540

tgttccaatt cttgttgaat tagatggtga tgttaatggg cacaaatttt ctgtcagtgg 600

agagggtgaa ggtgatgcaa catacggaaa acttaccctt aaatttattt gcactactgg 660

aaaactacct gttccatggc caacacttgt cactactttc gcctatggtg ttcaatgctt 720

ttcaagatac ccagatcata tgaaacggca tgactttttc aagagtgcca tgcccgaagg 780

ttatgtacag gaaagaacta tatttttcaa agatgacggg aactacaaga cacgtgctga 840

agtcaagttt gaaggtgata cccttgttaa tagaatcgag ttaaaaggta ttgattttaa 900

agaagatgga aacattcttg gacacaaatt ggaatacaac tataactcac acaatgtata 960

catcatggca gacaaacaaa agaatggaat caaagttaac ttcaaaatta gacacaacat 1020

tgaagatgga agcgttcaac tagcagacca ttatcaacaa aatactccaa ttggcgatgg 1080

ccctgtcctt ctaccagaca accattacct gtccacacaa tctgtccttt cgaaagaccc 1140

caacgaaaag agagaccaca tggtccttct tgagtttgta acagctgctg ggattacaca 1200

tggcatggat gaactataca aaatggtttc attcatttcc atttctcaag gtgttagtct 1260

ttgtcttctt gtctcttcca tgatgctcgg ttcatctgct gttcctgttt ctggtaaatc 1320

tggatcttcc actaccgccg tctctgcatc tgacaattct gccctccctc ctctcatttc 1380

cagccgttgt gctcctcctt ctaacaaggg aagtaaaagc gatcttcaag ctgaacctta 1440

ctacatgcaa aagaatacag aatggtatga gtcccatggt ggcaacctga catccatcgg 1500

aaagcgagat gacaatttgg ttggtggcat gactttggat ttacctagcg atgctcctcc 1560

tatcagcctc tctggatcta ccaacagcgc ctctgatggt ggtaaggttg ctgctgctac 1620

tactgctcaa attcaagagt tcaccaagta tgctggtatc gctgccactg cctactgtcg 1680

ttctgttgtc cctggtaaca agtgggactg tgtccaatgt caaaagtggg ttcctgatgg 1740

caagatcatc actaccttta cctccttgct ttccgacaca aatggttacg tcttgagaag 1800

tgataaacaa aagaccattt atcttgtttt ccgtggtacc aactccttca gaagtgccat 1860

cactgatatt gtcttcaact tttccgacta caagcctgtc aagggcgcca aggttcatgc 1920

tggtttcctt tcctcttatg agcaagttgt caatgactat ttccctgtca tccaagaaca 1980

actgaccgct aaccctactt acaaggtcat cgttaccggt cactcactcg gtggtgcaca 2040

agctttgctt gccggtatgg atctctacca acgtgaacca agattgtctc ccaagaattt 2100

gagcatcttc actgttggtg gtcctcgtgt tggtaacccc acctttgctt actatgttga 2160

atctaccggt attcctttcc aacgtaccgt tcacaagaga gatatcgttc ctcacgttcc 2220

tcctcaatcc ttcggattcc ttcaccccgg tgttgaatct tggatcaagt ccggtacctc 2280

caacgttcaa atctgtactt ctgaaattga aaccaaggat tgcagtaact ctatcgttcc 2340

tttcacctct ctccttgatc acttgagtta ctttgatatc aacgaaggaa gctgtttgat 2400

gactcagaca agtcatatgg acaagaccca aggggtcata gatgttgaag cattgaagaa 2460

agaaattcgc gagcaaattt tatccgaact gaaggaacaa aaacaagaac aaaagccaga 2520

gagaccaaag agaaagttaa gtgaaaaaca acttgctgca ttagctgctg gaagacagaa 2580

gaacccacgt ttgctggcga agaaagctag agaagaagca aaagcaaagg aagaacaaaa 2640

agcaaaggaa gctgaagcaa agaaagctga agcaaagaaa gagaaaaaga aaaagaaaaa 2700

gaaaaagaaa aagaaaaaga aaaagaaaaa gaaaaagaaa aagaaaaaga aaaagaaaaa 2760

gaaaaagaaa aagtaatagt aatgagttac agttttggca attactaaaa aactgacttc 2820

aattcaatgt tagcccgctc ccgcgggttt tttgttgctt tttcacagtg actataggta 2880

atcagcaaca caatacggcc ctgttctttg gacagttttt gtataatgtt gaccgcatcc 2940

tgaccggatt ttttatctaa gtggggaatt gtcaattgtc aattaaagct aagttctact 3000

aatgttttag aaggcattgt cgattgaaaa taagggttga atggagaaaa ttttgagcct 3060

ttgtcaaaga taaaaattta tttcaacagt tttttaacta gccgaaccag agaatgaccc 3120

agtggcgctg actttgctcc cgagtttttg ttagaaatta ccctcaagaa gtaatctaat 3180

aataaggttc tctcttctgc cgtta 3205

<210> 226
<211> 2062
<212> DNA
<213> Artificial Sequence

<220>
<223> SEQ ID NO: 226 presents example 226 of a designer
Thermosynechococcus elongatus nirA-promoter-controlled cell
surface-linked lipase (using butanol) DNA construct (2062 bp)

<400> 226
agaaaatctg gcaccacacc tgacccccat cgagagactc cgaacgtggc aaatggaggg 60

accagagttg ttcagttcac aggtagataa tgtcgcgggt cttgatagtt agcaataaat 120

acagtttcag aatatctgta atacaaaaac tgtatcgaga caagaaaaaa gtagcaaaat 180

ttacaaatgt tcatgattca tctggctaaa ttggatgttc aactgaccca ttgaagacaa 240

gggcaacaac catggccatt accctctgca tgattgtgcg ggatgaggcg acacgcttac 300

cccagtgcct agccagtgtt gccggcgttg ttgatgaagc ggtgattgtg gatacggggt 360

cgcaggatga aacggtggcg atcgcccgcg attggggggc acgggtctat gaaattcctt 420

ggcaagatga ttttgccgct gcccgcaatg ttgctctagg gtatgtgacc actgagtggg 480

tcttagttct cgatgctgat gaaaccctca cccctgcctt tgctgctttg ctgcctcaaa 540

tttgccagca catgaggagc tcccttgtgc tgttcttcct ctctgcgtgg acggccttgg 600

cgcggcctgt tcgacgagcg gttccgcaag atctgctcga ccagtttgaa ctcttttcac 660

aatattcggc ggccgcatac tgtgcggcaa acaatcatgc tccagtgggc tcagacgtaa 720

cgtgctcgga gaatgtctgc cctgaggtag atgcggcgga cgcaacgttt ctctattctt 780

ttgaagattc tggattaggc gatgttaccg gccttctcgc tctcgacaac acgaataaac 840

tgatcgtcct ctctttccgc ggctctcgtt cagtagagaa ctggatcgcg aacctcgccg 900

ccgacctgac agaaatatct gacatctgct ccggctgcga ggggcatgtc ggcttcgtta 960

cttcttggag gtctgtagcc gacactataa gggagcaggt gcagaatgcc gtgaacgagc 1020

atcccgatta ccgcgtggtc tttaccggac atagcttggg aggcgcactg gcaactattg 1080

ccgcagcagc tctgcgagga aatggataca atatcgacgt gttctcatat ggcgcgcccc 1140

gcgtcggtaa cagggcattt gcagaattcc tgaccgcaca gacgggcggc accctgtatc 1200

gcatcaccca taccaatgat atcgtcccta gactccctcc tcgagactgg ggttacagcc 1260

actctagccc ggagtactgg gtcacgtctg gtaacgacgt cccagtgacc gcaaacgaca 1320

tcaccgtcgt ggagggcatc gattccaccg acgggaacaa ccaggggaat atcccagaca 1380

tcccttcgca tctatggtat ttcggtccca tttcagagtg tgatatgact cagacaagtc 1440

atatggacaa gacccaaggg gtcatagatg ttgaagcatt gaagaaagaa attcgcgagc 1500

aaattttatc cgaactgaag gaacaaaaac aagaacaaaa gccagagaga ccaaagagaa 1560

agttaagtga aaaacaactt gctgcattag ctgctggaag acagaagaac ccacgtttgc 1620

tggcgaagaa agctagagaa gaagcaaaag caaaggaaga acaaaaagca aaggaagctg 1680

aagcaaagaa agctgaagca aagaaagaga tgatgattcg gaggctactg tcgccgaatc 1740

ggattcgttt taaatatgtc ttacagtatc actactccat ctcagttcgt gttcttgtca 1800

tcagcgtggg ccgacccaat agagttaata aaaagaaaaa gaaaaagaaa aagaaaaaga 1860

aaaagaaaaa gaaaaagaaa aagaaaaaga aaaagaaaaa gaaaaagaaa aagaaaaagt 1920

aataaggctg agatcttctt cagtgcattg tagttgaatg aagggttagg ggggaaatgc 1980

ccccctattt tttgtctagc catcctgcca cgtttgacag ggtagcaatt tcgacacgat 2040

agggttctct cttctgccgt ta 2062

<210> 227
<211> 2053
<212> DNA
<213> Artificial Sequence

<220>
<223> SEQ ID NO: 227 presents example 227 of a designer Arthrospira
 platensis cell surface-linked Lipase-polylysine DNA construct
 (2053 bp)

<400> 227
agaaaatctg gcaccacacc atgactttga tggctaaacg gcttttgatc ctgttagtta 60

ccctaggtgg tacagcattt ttggcagctt gtggcgaacc tactgccacc gaaacccaag 120

gcgaacccac cgaacaaatt gccgaaacca gtgaaccagt aaccgctgaa accgtttctg 180

atgaaactat cgaagcccaa gcagaacccg ctggggaaga aatggctgct gtagttgact 240

cagaccccac agaaaccatt gtcggtattg ctagtggcga gagccaattc agcactctgg 300

tagcggcttt agaaaccgcc gaattagccg aaatcctttc tggggaagga cctttcaccg 360

tttttgctcc cactgacgaa gcatttgcgg ctctaccaga aggcacagtt gaagaacttc 420

tcaaacccga aaatcgtgat caactggttc aaattcttac ctatcacgtt gtccctcccc 480

aagttctgtc tgctaacatc agtgatgggt ctgttgaaac cgttgccgga atgcccttaa 540

ctattactgt tatggacggt actgtaatgg tgaatgaagc cagtgtcatt caagctgaca 600

ttttaggcag caatggggtt attcatgccg ttgacacggt aattttacct ggtgtggtcg 660

aaatgaggag ctcccttgtg ctgttcttcc tctctgcgtg gacggccttg gcgcggcctg 720

ttcgacgagc ggttccgcaa gatctgctcg accagtttga actcttttca caatattcgg 780

cggccgcata ctgtgcggca aacaatcatg ctccagtggg ctcagacgta acgtgctcgg 840

agaatgtctg ccctgaggta gatgcggcgg acgcaacgtt tctctattct tttgaagatt 900

ctggattagg cgatgttacc ggccttctcg ctctcgacaa cacgaataaa ctgatcgtcc 960

tctctttccg cggctctcgt tcagtagaga actggatcgc gaacctcgcc gccgacctga 1020

cagaaatatc tgacatctgc tccggctgcg aggggcatgt cggcttcgtt acttcttgga 1080

ggtctgtagc cgacactata agggagcagg tgcagaatgc cgtgaacgag catcccgatt 1140

accgcgtggt ctttaccgga catagcttgg gaggcgcact ggcaactatt gccgcagcag 1200

ctctgcgagg aaatggatac aatatcgacg tgttctcata tggcgcgccc cgcgtcggta 1260

acagggcatt tgcagaattc ctgaccgcac agacgggcgg caccctgtat cgcatcaccc 1320

ataccaatga tatcgtccct agactccctc ctcgagactg gggttacagc cactctagcc 1380

cggagtactg ggtcacgtct ggtaacgacg tcccagtgac cgcaaacgac atcaccgtcg 1440

tggagggcat cgattccacc gacgggaaca accaggggaa tatcccagac atcccttcgc 1500

atctatggta tttcggtccc atttcagagt gtgatatgac tcagacaagt catatggaca 1560

agacccaagg ggtcatagat gttgaagcat tgaagaaaga aattcgcgag caaattttat 1620

ccgaactgaa ggaacaaaaa caagaacaaa agccagagag accaaagaga aagttaagtg 1680

aaaaacaact tgctgcatta gctgctggaa gacagaagaa cccacgtttg ctggcgaaga 1740

aagctagaga agaagcaaaa gcaaaggaag aacaaaaagc aaaggaagct gaagcaaaga 1800

aagctgaagc aaagaaagag atgatgattc ggaggctact gtcgccgaat cggattcgtt 1860

ttaaatatgt cttacagtat cactactcca tctcagttcg tgttcttgtc atcagcgtgg 1920

gccgacccaa tagagttaat aaaaagaaaa agaaaaagaa aaagaaaaag aaaaagaaaa 1980

agaaaaagaa aaagaaaaag aaaaagaaaa agaaaaagaa aaagaaaaag taaggttctc 2040

tcttctgccg tta 2053

<210> 228
<211> 2971
<212> DNA
<213> Artificial Sequence

<220>
<223> SEQ ID NO: 228 presents example 228 of a designer Arthrospira
      platensis cell surface-linked Lipase-GFP-polylysine DNA construct
      (2971 bp)

<400> 228
agaaaatctg gcaccacacc atggcggata ttgtagatat agcagtgagt gcggggtgtt 60

tcaccacctt ggtggcagcc gtacaggcag cgggattagt ggaaaccctg aaaactcccg 120

gtccttttac cgtttttgcg ccccatgatg atgcttttgc gaaactcccc cccggaactg 180

ttcagacctt agtgcagaac ccaccccagt tagcgaggat tttaacatat catgtggctg 240

ctggcaagtt cctgaaggca gacctagaaa aactggatta tttaacttcg gtggaaggtg 300

cgcctatttc tctgaatttt accgagggat ttgaggttaa aaatgccaca gttatcaacc 360

ccgatattga agcagacaat ggggtgattc atgtgattga tacagtcatt ttaatgggga 420

tgacgttgga aaaaggtatt gcttcgctgg ttgaggagtt tattgctgcg gggcggccat 480

cagcacgaga acagagtatt gatgaccgga gaaacggcta tctcgccagc gccgttctcg 540

ctggggcaac ggaaacgcgt gtacaggttg aagacatcac gctcgatggc atgaccttgc 600

gcgtcgtctc gccgccaaaa agcaacggct tgctacccac cattgtgtat taccacggcg 660

gctgttttgt gagcggcgac tttgccaccc atgacaacca attgcgccag cttgcctggt 720

tcagtgggtg ccgggtgatc gccgtgcagt accgtctggc gccggagtac acttttccgg 780

cggcgcacga tgatgcggaa cgaggcgcca acattatcta tcggtatgcg cagcggcttg 840

gcgttgacgc cgcgcgcatc acgctggccg gcgacagcgc cggcggccac ctcgccctgg 900

tcacagcgct acgcctgaaa gcggcgggag tatggcaacc tgcgcagctt atcctgattt 960

accctatgct cgacgctacc gccagcatgg caagctacgc cagcaacggc gaggattaca 1020

tcattacccg ggatacgctg cttagcggct acgaaatgta tttagcggcc acacccgcta 1080

cccatccgga agccagtccg ctgtggcgcg aagattttaa cgggctgccg ccggttcata 1140

tcctgacggc cgaatttgat cctttgcgcg atgagggaga agcgctgtac cggcggttaa 1200

tagcacaagg gatagaaagc acctgccagc gctatctggg cgtcattcac ggtttctttc 1260

agttaggcgg gataagcctc gcggcaaagc aggcgatgcg cgatatcgcc tggcgtacga 1320

tgtctaaagg tgaagaatta ttcactggtg ttgtcccaat tttggttgaa ttagatggtg 1380

atgttaatgg tcacaaattt tctgtctccg gtgaaggtga aggtgatgct acttacggta 1440

aattgacctt aaaatttatt tgtactactg gtaaattgcc ggttccatgg ccaaccttag 1500

tcactacttt cggttatggt gttcaatgtt ttgctagata cccagatcat atgaaacaac 1560

atgacttttt caagtctgcc atgccagaag gttatgttca agaaagaact atttttttca 1620

aagatgacgg taactacaag accagagctg aagtcaagtt tgaaggtgat accttagtta 1680

atagaatcga attaaaaggt attgatttta aagaagatgg taacatttta ggtcacaaat 1740

tggaatacaa ctataactct cacaatgttt acatcatggc tgacaaacaa aagaatggta 1800

tcaaagttaa cttcaaaatt agacacaaca ttgaagatgg ttctgttcaa ttagctgacc 1860

attatcaaca aaatactcca attggtgatg gtccagtctt gttaccagac aaccattact 1920

tatccactca atctgcctta tccaaagatc caaacgaaaa gagagaccac atggtcttgt 1980

tagaatttgt tactgctgct ggtattaccc atggtatgga tgaattgtac aaaatgaatg 2040

ctgtatattt cttgcttgtc ttatccgccg ggttgttgct aacccaagca gatgatgcaa 2100

atgaggaagg tgctgaagat ggtacacagg gtggagagga gaagaaggag aaggagaaga 2160

agaagaagaa gaagaagaag gagaagaaaa agaagaagaa gaagaagaag aagaagaaga 2220

agaagaagaa gaaggtggcg aagaagaagg aggcggcaaa gaagaagaag tcgaaagaga 2280

gaaagataaa ggcgaaagag agaaaggtaa aggcgaaaaa actaaaggcg aaaaaggtag 2340

aggcgaaaaa ggccaacgcg aaaaaggcaa agaagaaggc gaagaagagg tacctggaaa 2400

aaaagaaaag cctggaaagg gtaaaaaagg tgaagaagac aaatatcgta gaaatgactc 2460

agacaagtca tatggacaag acccaagggg tcatagatgt tgaagcattg aagaaagaaa 2520

ttcgcgagca aattttatcc gaactgaagg aacaaaaaca agaacaaaag ccagagagac 2580

caaagagaaa gttaagtgaa aaacaacttg ctgcattagc tgctggaaga cagaagaacc 2640

cacgtttgct ggcgaagaaa gctagagaag aagcaaaagc aaaggaagaa caaaaagcaa 2700

aggaagctga agcaaagaaa gctgaagcaa agaaagagat gatgattcgg aggctactgt 2760

cgccgaatcg gattcgtttt aaatatgtct tacagtatca ctactccatc tcagttcgtg 2820

ttcttgtcat cagcgtgggc cgacccaata gagttaataa aaagaaaaag aaaaagaaaa 2880

agaaaaagaa aaagaaaaag aaaaagaaaa agaaaaagaa aaagaaaaag aaaaagaaaa 2940

agaaaaagta aggttctctc ttctgccgtt a 2971

<210> 229
<211> 2524
<212> DNA
<213> Artificial Sequence

<220>
<223> SEQ ID NO: 229 presents example 229 of a designer Phaeodactylum
tricornutum cell surface-linked Lipase-polylysine DNA construct
(2524 bp)

<400> 229
agaaaatctg gcaccacacc atgaaggggc tttcatctgt tgtcgtcttc aaatacttga 60

cgatcttcat agtggtcgcc agcgctcgtc gcttggagga tggcggttcg aattttgccg 120

gcgaatttct ttctgatgcg gaagtatgcg aaatgcaaga ggaagcatgg ccgagtcagc 180

tgggggaatg gttcggagcg cagcattcgt gcgaatgcaa tggtgcttcc gatgaagaag 240

gcgttgacgt cgtcgagacg aaatgtaaag caaccgagca attttgttgc cctgatacag 300

gccgatgcca cgaagcccgt cgagagcact acaacatgaa agccaaagac gtcaaccgga 360

ctaccgatca agggggtgac tggttttgga catccggagt ggaagcaaat atgtactgct 420

acacatactt gggtgacgat ctaccaaacg gacgggtaga gatttgcgaa gacaccgatt 480

ttgatcccga cgcgcctgga aacgagtgga gctgcgacat gatcaacaat ggcgaaaaat 540

gtcaagagtg ttcccagtgt gaagatgacg acgctaccac caactttgat tgttccaata 600

ttgccggcat agatgacatt cctgccgata tctttcccaa aactggaatc tgctatcgct 660

tcaatggagc ggagtgtgct tccgccttta tcaccaagcc cttatcggtg caagcgacaa 720

atggcggaga ttccggcagt acgaacgccg ttctgtcagg tctgtgtttt gttattgcga 780

ttttgatggt ttttggtaag gacgttatga cgttggaaaa aggtattgct tcgctggttg 840

aggagtttat tgctgcgggg cggccatcag cacgagaaca gagtattgat gaccggagaa 900

acggctatct cgccagcgcc gttctcgctg ggcaacggaa aacgcgtgta caggttgaag 960

acatcacgct cgatggcatg accttgcgcg tcgtctcgcc gccaaaaagc aacggcttgc 1020

tacccaccat tgtgtattac cacggcggct gttttgtgag cggcgacttt gccacccatg 1080

acaaccaatt gcgccagctt gcctggttca gtgggtgccg ggtgatcgcc gtgcagtacc 1140

gtctggcgcc ggagtacact tttccggcgg cgcacgatga tgcggaacga ggcgccaaca 1200

ttatctatcg gtatgcgcag cggcttggcg ttgacgccgc gcgcatcacg ctggccggcg 1260

acagcgccgg cggccacctc gccctggtca cagcgctacg cctgaaagcg gcgggagtat 1320

ggcaacctgc gcagcttatc ctgatttacc ctatgctcga cgctaccgcc agcatggcaa 1380

gctacgccag caacggcgag gattacatca ttacccggga tacgctgctt agcggctacg 1440

aaatgtattt agcggccaca cccgctaccc atccggaagc cagtccgctg tggcgcgaag 1500

attttaacgg gctgccgccg gttcatatcc tgacggccga atttgatcct ttgcgcgatg 1560

agggagaagc gctgtaccgg cggttaatag cacaagggat agaaagcacc tgccagcgct 1620

atctgggcgt cattcacggt ttctttcagt taggcgggat aagcctcgcg gcaaagcagg 1680

cgatgcgcga tatcgcctgg cgtacgatga atgctgtata tttcttgctt gtcttatccg 1740

ccgggttgtt gctaacccaa gcagatgatg caaatgagga aggtgctgaa gatggtacac 1800

agggtggaga ggagaagaag gagaaggaga agaagaagaa gaagaagaag aaggagaaga 1860

aaaagaagaa gaagaagaag aagaagaaga agaagaagaa gaagaaggtg gcgaagaaga 1920

aggaggcggc aaagaagaag aagtcgaaag agagaaagat aaaggcgaaa gagagaaagg 1980

taaaggcgaa aaaactaaag gcgaaaaagg tagaggcgaa aaaggccaac gcgaaaaagg 2040

caaagaagaa ggcgaagaag aggtacctgg aaaaaagaaa aagcctggaa agggtaaaaa 2100

aggtgaagaa gacaaatatc gtagaaatga ctcagacaag tcatatggac aagacccaag 2160

gggtcataga tgttgaagca ttgaagaaag aaattcgcga gcaaatttta tccgaactga 2220

aggaacaaaa acaagaacaa aagccagaga gaccaaagag aaagttaagt gaaaaacaac 2280

ttgctgcatt agctgctgga agacagaaga acccacgttt gctggcgaag aaagctagag 2340

aagaagcaaa agcaaaggaa gaacaaaaag caaaggaagc tgaagcaaag aaagctgaag 2400

caaagaaaga gaaaaagaaa aagaaaaaga aaaagaaaaa gaaaaagaaa aagaaaaaga 2460

aaaagaaaaa gaaaaagaaa aagaaaaaga aaaagaaaaa gtaaggttct ctcttctgcc 2520

gtta 2524

<210>  230
<211>  2317
<212>  DNA
<213>  Artificial Sequence

<220>
<223>  SEQ ID NO: 230 presents example 230 of a designer Microcoleus
       vaginatu cell surface-linked Lipase-polylysine DNA construct
       (2317 bp)

<400>  230
agaaaatctg gcaccacacc atgagcaaat tttacaattc tcaaggatgg cgcgatcggg      60

tggcggtgct agcgggaatt tttggcctca gcagtctctt gattttaccc gtcttagcgc     120

aatctaaccg cgaatcccag agcacttttc agccctcttc cgtacttgac tccccccagc     180

gatcgggcgg caacattgca gatactctca agagagaaag cgaattccaa actcttgctt     240

ctgtgttggc tgctgctggt ttgacggatc tgctcagaca agaaggtcgt cctttaacaa     300

ttttcgctcc taccaatcaa gcttttcgta aaaatgccgc tgtttacaat cagctactcc     360

agccgcaaaa caaggagaaa ttagcgagag ttttgaaata tcatttaatt gctggccgtg     420

tcactcctga agatgtgaat aaaggggaaa ttaaaacagt tgaaggcggc acagtgaaaa     480

ttcgcgtggc acctgatgga gtgattatga atgataatgt taaaggagtt caaccgtcta     540

ttgaggctaa aaacggggta attgtgcggg tagataattt acttttaccc ccggatttaa     600

tgacgttgga aaaaggtatt gcttcgctgg ttgaggagtt tattgctgcg gggcggccat      660

cagcacgaga acagagtatt gatgaccgga gaaacggcta tctcgccagc gccgttctcg     720

ctggggcaac ggaaacgcgt gtacaggttg aagacatcac gctcgatggc atgaccttgc     780

gcgtcgtctc gccgccaaaa agcaacggct tgctacccac cattgtgtat taccacggcg     840

gctgttttgt gagcggcgac tttgccaccc atgacaacca attgcgccag cttgcctggt     900

tcagtgggtg ccgggtgatc gccgtgcagt accgtctggc gccggagtac acttttccgg     960

cggcgcacga tgatgcggaa cgaggcgcca acattatcta tcggtatgcg cagcggcttg    1020

gcgttgacgc cgcgcgcatc acgctggccg gcgacagcgc cggcggccac ctcgccctgg    1080

tcacagcgct acgcctgaaa gcggcgggag tatggcaacc tgcgcagctt atcctgattt    1140

accctatgct cgacgctacc gccagcatgg caagctacgc cagcaacggc gaggattaca    1200

tcattacccg ggatacgctg cttagcggct acgaaatgta tttagcggcc acacccgcta    1260

cccatccgga agccagtccg ctgtggcgcg aagattttaa cgggctgccg ccggttcata    1320

tcctgacggc cgaatttgat cctttgcgcg atgagggaga agcgctgtac cggcggttaa    1380

tagcacaagg gatagaaagc acctgccagc gctatctggg cgtcattcac ggtttctttc    1440

agttaggcgg gataagcctc gcggcaaagc aggcgatgcg cgatatcgcc tggcgtacga    1500

tgaatgctgt atatttcttg cttgtcttat ccgccgggtt gttgctaacc caagcagatg    1560

atgcaaatga ggaaggtgct gaagatggta cacagggtgg agaggagaag aaggagaagg 1620

agaagaagaa gaagaagaag aagaaggaga agaaaaagaa gaagaagaag aagaagaaga 1680

agaagaagaa gaagaagaag gtggcgaaga agaaggaggc ggcaaagaag aagaagtcga 1740

aagagagaaa gataaaggcg aaagagagaa aggtaaaggc gaaaaaacta aaggcgaaaa 1800

aggtagaggc gaaaaaggcc aacgcgaaaa aggcaaagaa gaaggcgaag aagaggtacc 1860

tggaaaaaag aaaaagcctg gaaagggtaa aaaaggtgaa gaagacaaat atcgtagaaa 1920

tgactcagac aagtcatatg gacaagaccc aaggggtcat agatgttgaa gcattgaaga 1980

aagaaattcg cgagcaaatt ttatccgaac tgaaggaaca aaaacaagaa caaaagccag 2040

agagaccaaa gagaaagtta agtgaaaaac aacttgctgc attagctgct ggaagacaga 2100

agaacccacg tttgctggcg aagaaagcta gagaagaagc aaaagcaaag gaagaacaaa 2160

aagcaaagga agctgaagca aagaaagctg aagcaaagaa agagaaaaag aaaaagaaaa 2220

agaaaaagaa aaagaaaaag aaaaagaaaa agaaaaagaa aaagaaaaag aaaaagaaaa 2280

agaaaaagaa aaagtaaggt tctctcttct gccgtta 2317

<210> 231
<211> 4552
<212> DNA
<213> Artificial Sequence

<220>
<223> SEQ ID NO: 231 presents example 231 of a designer Methanosarcina
barkeri cell surface-linked Lipase-polylysine DNA construct (4552
bp)

<400> 231
agaaaatctg gcaccacacc atgaaagata ggggaaaatt gcattcagta cctttagtgt 60

taatggcatt atttttcgta tttttggttt caaactcagc tgtaacgtta gctgcccagg 120

aaattaagct ctcagaggat attggactaa atggagagac ttctatttac ggtgataaag 180

tggtgtggtc atattgggac aaaattcatc tttatgacct aaaaaccgga aatgatacta 240

taattaccat gcctgaacat tatgtatcac atcctgctat atatgataat aaaattgtat 300

actgtcttta taatgtaaat gagaatccag ttagggtcag actttacgtg tatgatatac 360

ttaattctac aagttcacta atcacagaga atttgtccca ttgtgttcct gatatttacg 420

gtgatagaat tgtgtgggct gcggaacgcg actacaagac cgatgtctac atgtacaata 480

tttccactca tacacaaact cagataacca caagaggatc tgcaagtaac ccttcaatat 540

atgaaaacaa aatagtatgg gtgggttacg gggacaatgg aactattata gataaaaata 600

acaattccat tagcagagat agtatttgca tttatgacct ttccactaag aaggaaacta 660

tgatcaacac tatcggaaga gcatatgctc ccgtgatcta cagtgatagg atagtatgga 720

aggatagtcg caattcagac tttactggga gaagagggga tgtctacatg tataacctct 780

caactgagaa agaaagccgg atctcttaca gcggacagtc ctcgttaggt tcgtctccag 840

ctatctatgg ggacaggata gtatgggatg ataatcgcaa tggaaaatgg gatatttaca 900

tgtacaatct ctcaactaag aaggagactc agattaccac cagtggaagt gcaatgggac 960

ctgatatcta cggagacagg atagtgtata gtgataatcg tcaaagattg acccctggtc 1020

ctggtgatgt ttatttttgt gatatctatg tgtatgatct cactgctaga cccatagaac 1080

cacaagctgg gtttacatcc aatgtgactt ccggaatagc acctctaacg gtattattca 1140

ctgataccag cactggtgga gtaccaactt cctggcattg ggacactggt gacggtattt 1200

actcaaaaca tgccatgaat gcaacccata cgtttacaaa cccaggggtg tataacgtta 1260

ctttaacagt tacaaatgaa gcaggcaata gcacggtaac gaaaccgaat tatattactg 1320

taactccccc acaaccacca gtggcagatt tttatgctaa tgtaacctct ggaaaagcac 1380

ctctaatggt gtctttccat ggtagaatta ttggcaaagc tacaagtaaa ggcgaagaac 1440

ctatttcctg gtattgggat tttggcgatg gcatttattc aaaacacacc acgaatgcaa 1500

tccacacatt tcacaccacg aatgcaatcc acacatttac aaaaccaggg atttacacag 1560

taaatcttac tgtaggaaat ttcgtgggta atagtacagc gacaaagcca gattacatag 1620

ttgttatcga tccaaaagct cccgatgcaa attttagtag taacattatc gagggttatg 1680

ttcccctgac ggtacagttt aatgacactt ctcaaaatgc aacgtcaagg gtatgggact 1740

tcgacaataa tggaaaaaca gactcaaccg atgtaaaccc tgtttataca tttacaacac 1800

caggaatcca tactgttaac ctgacagtaa gaaatgctta cggcactacc tcaaaaactg 1860

atacgataat tgtacttaca gaaaatgaga tccggattac aaccagcgaa ttagcatccc 1920

atcctgatat ctatggtaat agaatagtat ggcaagacct tcgcaacgga aactacgata 1980

tctacatgta cgatctctcc acttccaggg aaaccaggat cactgctaat gaatcaaatc 2040

agacatatcc ttcactctac tgtgatagaa tagtgtggca agatgatcgt aatggacagt 2100

atgatatcta tatgtacaat atatctactt caaaagaaac tcaaataagc actagcgggc 2160

gcgctcagag acctaaaatc tatggtgaca ggataatgtg gatggattat cgaactagaa 2220

ataaaggtat ttacgtgcac gacctatcca cttctaagga aactcggata atttcaaaag 2280

gaatacctca atatattaat atgcaaggta acttgatagt ctggcatgat cttcgataca 2340

taagtccgga tatttacatg tacgatctct ccacttcaaa ggaaactcaa ataacctcca 2400

acggagaagc aacattccct aacgtctatg gtaacaggat agtgtggaaa gagtggcaca 2460

gagaagatgt caccaccgat atctacatgt acgatttatc cacttccaag aagactcaga 2520

ttactaccaa taaatcaagt caatactatc ctgctattta tggtaataag atagtatggg 2580

aagatttccg caacgaatac atcaatattt acatgtacga tctctcaact cagaaggaga 2640

ctcagattac caccagcgga ggtgcacacg atcctgatat ctatggagac aggattgtat 2700

ataggaagta tagttatcat agatcacccg ttgatcatgt tttttttagt gatatctata 2760

tgtatgatct cactgctaga cccatggaac cacaaactgg acttacatcc aatgtgactc 2820

tagaacaaca cctcatgacg ttggaaaaag gtattgcttc gctggttgag gagtttattg 2880

ctgcggggcg gccatcagca cgagaacaga gtattgatga ccggagaaac ggctatctcg 2940

ccagcgccgt tctcgctggg gcaacggaaa cgcgtgtaca ggttgaagac atcacgctcg 3000

atggcatgac cttgcgcgtc gtctcgccgc caaaaagcaa cggcttgcta cccaccattg 3060

tgtattacca cggcggctgt tttgtgagcg gcgactttgc cacccatgac aaccaattgc 3120

gccagcttgc ctggttcagt gggtgccggg tgatcgccgt gcagtaccgt ctggcgccgg 3180

agtacacttt tccggcggcg cacgatgatg cggaacgagg cgccaacatt atctatcggt 3240

atgcgcagcg gcttggcgtt gacgccgcgc gcatcacgct ggccggcgac agcgccggcg 3300

gccacctcgc cctggtcaca gcgctacgcc tgaaagcggc gggagtatgg caacctgcgc 3360

agcttatcct gatttaccct atgctcgacg ctaccgccag catggcaagc tacgccagca 3420

acggcgagga ttacatcatt acccgggata cgctgcttag cggctacgaa atgtatttag 3480

cggccacacc cgctacccat ccggaagcca gtccgctgtg gcgcgaagat tttaacgggc 3540

tgccgccggt tcatatcctg acggccgaat ttgatccttt gcgcgatgag ggagaagcgc 3600

tgtaccggcg gttaatagca caagggatag aaagcacctg ccagcgctat ctgggcgtca 3660

ttcacggttt ctttcagtta ggcgggataa gcctcgcggc aaagcaggcg atgcgcgata 3720

tcgcctggcg tacgatgaat gctgtatatt tcttgcttgt cttatccgcc gggttgttgc 3780

taacccaagc agatgatgca aatgaggaag gtgctgaaga tggtacacag ggtggagagg 3840

agaagaagga gaaggagaag aagaagaaga agaagaagaa ggagaagaaa aagaagaaga 3900

agaagaagaa gaagaagaag aagaagaaga agaaggtggc gaagaagaag gaggcggcaa 3960

agaagaagaa gtcgaaagag agaaagataa aggcgaaaga gagaaaggta aaggcgaaaa 4020

aactaaaggc gaaaaaggta gaggcgaaaa aggccaacgc gaaaaaggca aagaagaagg 4080

cgaagaagag gtacctggaa aaaagaaaaa gcctggaaag ggtaaaaaag gtgaagaaga 4140

caaatatcgt agaaatgact cagacaagtc atatggacaa gacccaaggg gtcatagatg 4200

ttgaagcatt gaagaaagaa attcgcgagc aaattttatc cgaactgaag gaacaaaaac 4260

aagaacaaaa gccagagaga ccaaagagaa agttaagtga aaaacaactt gctgcattag 4320

ctgctggaag acagaagaac ccacgtttgc tggcgaagaa agctagagaa gaagcaaaag 4380

caaaggaaga acaaaaagca aaggaagctg aagcaaagaa agctgaagca aagaaagaga 4440

aaaagaaaaa gaaaaagaaa aagaaaaaga aaaagaaaaa gaaaaagaaa aagaaaaaga 4500

aaaagaaaaa gaaaaagaaa aagaaaaagt aaggttctct cttctgccgt tg          4552

<210>  232
<211>  2533
<212>  DNA
<213>  Artificial Sequence

<220>
<223>  SEQ ID NO: 232 presents example 232 of a designer Synechococcus
       sp. strain PCC 7942 nirA-promoter-controlled cell surface-linked
       Lipase-polylysine DNA construct (2533 base pairs (bp))

<400>  232
agaaaatctg gcaccacacc cttcttgcag aacatgcatg atttacaaaa agttgtagtt      60

tctgttacca attgcgaatc gagaactgcc taatctgccg agtatatgat ggccaaaatc     120

cttgaggttg cccgcgaggc gggttgtttt cagacgctac tcacagccgt tgaagtcgct     180

ggcttagtcg atgcactcaa tagcgacggt ccttttaccg tctttgcacc taccgatgat     240

gcttttgctg cactaccacc gggcacagtg acaacgctgg ttcagaaccc gccgcaacta     300

gctcgaattc tcaagtttca cgttacggca ggtgctctat ccaaagcaga cctgatcgat     360

cgcccgtcag ttgattcgtt agaaggcgca ccaattccca ttggacgagc agagccgttt     420

gaaatcaaaa atgcgacagt tttgtctgct gatattgcct gtgataacgg cattgttcat     480

gtcttgaatc gcgtcatttt aatgggcatg acgttggaaa aaggtattgc ttcgctggtt     540

gaggagttta ttgctgcggg gcggccatca gcacgagaac agagtattga tgaccggaga     600

aacggctatc tcgccagcgc cgttctcgct ggggcaacgg aaacgcgtgt acaggttgaa     660

gacatcacgc tcgatggcat gaccttgcgc gtcgtctcgc cgccaaaaag caacggcttg     720

ctacccacca ttgtgtatta ccacggcggc tgttttgtga gcggcgactt tgccacccat     780

gacaaccaat tgcgccagct tgcctggttc agtgggtgcc gggtgatcgc cgtgcagtac     840

cgtctggcgc cggagtacac ttttccggcg gcgcacgatg atgcggaacg aggcgccaac     900

attatctatc ggtatgcgca gcggcttggc gttgacgccg cgcgcatcac gctggccggc     960

gacagcgccg gcggccacct cgccctggtc acagcgctac gcctgaaagc ggcgggagta    1020

tggcaacctg cgcagcttat cctgatttac cctatgctcg acgctaccgc cagcatggca    1080

agctacgcca gcaacggcga ggattacatc attacccggg atacgctgct tagcggctac    1140

gaaatgtatt tagcggccac acccgctacc catccggaag ccagtccgct gtggcgcgaa    1200

gattttaacg ggctgccgcc ggttcatatc ctgacggccg aatttgatcc tttgcgcgat    1260

gagggagaag cgctgtaccg gcggttaata gcacaaggga tagaaagcac ctgccagcgc    1320

tatctgggcg tcattcacgg tttctttcag ttaggcggga taagcctcgc ggcaaagcag    1380

gcgatgcgcg atatcgcctg gcgtacgatg aatgctgtat atttcttgct tgtcttatcc    1440

gccgggttgt tgctaaccca agcagatgat gcaaatgagg aaggtgctga agatggtaca    1500

cagggtggag aggagaagaa ggagaaggag aagaagaaga agaagaagaa gaaggagaag 1560

aaaaagaaga agaagaagaa gaagaagaag aagaagaaga agaagaaggt ggcgaagaag 1620

aaggaggcgg caaagaagaa gaagtcgaaa gagagaaaga taaaggcgaa agagagaaag 1680

gtaaaggcga aaaaactaaa ggcgaaaaag gtagaggcga aaaaggccaa cgcgaaaaag 1740

gcaaagaaga aggcgaagaa gaggtacctg gaaaaaagaa aaagcctgga aagggtaaaa 1800

aaggtgaaga agacaaatat cgtagaaatg actcagacaa gtcatatgga caagacccaa 1860

ggggtcatag atgttgaagc attgaagaaa gaaattcgcg agcaaatttt atccgaactg 1920

aaggaacaaa aacaagaaca aaagccagag agaccaaaga gaaagttaag tgaaaaacaa 1980

cttgctgcat tagctgctgg aagacagaag aacccacgtt tgctggcgaa gaaagctaga 2040

gaagaagcaa aagcaaagga agaacaaaaa gcaaaggaag ctgaagcaaa gaaagctgaa 2100

gcaaagaaag agaaaaagaa aaagaaaaag aaaaagaaaa agaaaaagaa aaagaaaaag 2160

aaaaagaaaa agaaaaagaa aaagaaaaag aaaaagaaaa agtaataata gtgatcccgg 2220

ccgctactaa agcctgattt gtcttgatag ctgctcctgc ctttgggcag ggcttttttt 2280

ctgtctgcca ttcttgagga tggcggactc tttccctttt gctctacgcc catgaatgcg 2340

atcgcagtct cccctgtcca gcacgttgga gtgattggtg gtggccagtt agcttggatg 2400

ctggcaccag cagcgcaaca gttggggatg tcgctgcacg ttcaaacacc caatgatcac 2460

gacccagcag tagcgatcgc ggatcaaacc gtattagcag cagttgctga cgcggttctc 2520

tcttctgccg tta 2533